(19) Europäisches Patentamt
European Patent Office
Office européen des brevets

(11) EP 3 980 410 B1

(12) **EUROPEAN PATENT SPECIFICATION**

(45) Date of publication and mention of the grant of the patent:
**19.02.2025 Bulletin 2025/08**

(21) Application number: **20750715.3**

(22) Date of filing: **04.06.2020**

(51) International Patent Classification (IPC):
***C07D 401/12*** (2006.01) ***C07D 403/12*** (2006.01)
***C07D 401/14*** (2006.01) ***C07D 407/14*** (2006.01)
***C07D 405/14*** (2006.01) ***C07D 417/12*** (2006.01)
***C07D 417/14*** (2006.01) ***C07D 277/52*** (2006.01)
***A61K 31/506*** (2006.01) ***A61K 31/497*** (2006.01)
***A61K 31/444*** (2006.01) ***A61K 31/351*** (2006.01)
***A61K 31/4545*** (2006.01) ***A61K 31/427*** (2006.01)
***A61P 35/00*** (2006.01) ***A61P 35/02*** (2006.01)
***A61P 17/06*** (2006.01) ***A61P 37/00*** (2006.01)
***A61P 25/00*** (2006.01)

(52) Cooperative Patent Classification (CPC):
**C07D 401/14; A61P 17/06; A61P 25/00; A61P 35/00; A61P 35/02; A61P 37/00; C07D 277/52; C07D 401/12; C07D 403/12; C07D 405/14; C07D 407/14; C07D 417/12; C07D 417/14**

(86) International application number:
**PCT/IB2020/000560**

(87) International publication number:
**WO 2020/245665 (10.12.2020 Gazette 2020/50)**

(54) **N-(4-(5-CHLOROPYRIDIN-3-YL)PHENYL)-2-(2-(CYCLOPROPANESULFONAMIDO) PYRIMIDIN-4-YL)BUTANAMIDE DERIVATIVES AND RELATED COMPOUNDS AS HUMAN CTPS1 INHIBITORS FOR THE TREATMENT OF PROLIFERATIVE DISEASES**

N-(4-(5-CHLOROPYRIDIN-3-YL)PHENYL)-2-(2-(CYCLOPROPANESULFONAMIDO) PYRIMIDIN-4-YL)BUTANAMID-DERIVATE UND ÄHNLICHE VERBINDUNGEN ALS MENSCHLICHE CTPS1 INHIBITOREN ZUR BEHANDLUNG VON PROLIFERATIVEN ERKRANKUNGEN

DÉRIVÉS DE N-(4-(5-CHLOROPYRIDIN-3-YL)PHÉNYL)-2-(2-(CYCLOPROPANESULFONAMIDO) PYRIMIDIN-4-YL)BUTANAMIDE ET COMPOSÉS SIMILAIRES EN TANT QU'INHIBITEURS DE LA CTPS1 HUMAINE POUR LE TRAITEMENT DE MALADIES PROLIFÉRATIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.06.2019 EP 19305714**
**04.06.2019 EP 19305717**
**04.06.2019 EP 19305715**

(43) Date of publication of application:
**13.04.2022 Bulletin 2022/15**

(73) Proprietor: **Step Pharma S.A.S.**
**75272 Paris, Cedex 06 (FR)**

(72) Inventors:
- **QUDDUS, Abdul**
  **Nottingham, Nottinghamshire NG1 1GR (GB)**
- **NOVAK, Andrew**
  **Nottingham, Nottinghamshire NG1 1GR (GB)**
- **COUSIN, David**
  **Nottingham, Nottinghamshire NG1 1GR (GB)**
- **CHATZOPOULOU, Elli**
  **Nottingham, Nottinghamshire NG1 1GR (GB)**
- **BLACKHAM, Emma**
  **Nottingham, Nottinghamshire NG1 1GR (GB)**
- **JONES, Geraint**
  **Nottingham, Nottinghamshire NG1 1GR (GB)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 980 410 B1

- **THOMAS, Jennifer**
  **Nottingham, Nottinghamshire NG1 1GR (GB)**
- **WRIGGLESWORTH, Joseph**
  **Nottingham, Nottinghamshire NG1 1GR (GB)**
- **DUFFY, Lorna**
  **Nottingham, Nottinghamshire NG1 1GR (GB)**
- **BIRCH, Louise**
  **Nottingham, Nottinghamshire NG1 1GR (GB)**
- **GEORGE, Pascal**
  **75272 Paris Cedex 06 (FR)**
- **AHMED, Saleh**
  **Nottingham, Nottinghamshire NG1 1GR (GB)**
- **TOSCHI, Gianna**
  **Nottingham, Nottinghamshire NG1 1GR (GB)**

(74) Representative: **Sagittarius IP**
**Marlow International**
**Parkway**
**Marlow SL7 1YL (GB)**



(56) References cited:

| | |
|---|---|
| **EP-A1- 3 492 454** | **EP-A1- 3 543 232** |
| **EP-A1- 3 578 551** | **WO-A1-02/16318** |
| **WO-A1-02/24665** | **WO-A1-2019/106146** |
| **WO-A1-2019/106156** | **WO-A1-2019/179652** |
| **WO-A1-2019/180244** | **WO-A1-2020/083975** |
| **WO-A2-2014/170435** | **CN-A- 104 262 071** |
| **GB-A- 1 555 007** | **GB-A- 1 575 803** |
| **US-A1- 2003 158 218** | |

- **JESSIE SANDOSHAM ET AL: "Synthesis of Pyrimidinyl Triflates and Palladium-catalyzed Coupling with Organotion and Organozic Reagents", HETEROCYCLES, vol. 37, no. 1, 1994, pages 501 - 514, XP055732636, ISSN: 0385-5414, DOI: 10.3987/COM-93-S39**

## Description

### Field of the invention

The invention relates to novel compounds, processes for the manufacture of such compounds, related intermediates, compositions comprising such compounds and the use of such compounds as cytidine triphosphate synthase 1 inhibitors, particularly in the treatment or prophylaxis of disorders associated with cell proliferation.

### Background of the invention

Nucleotides are a key building block for cellular metabolic processes such as deoxyribonucleic acid (DNA) and ribonucleic acid (RNA) synthesis. There are two classes of nucleotides, that contain either purine or pyrimidine bases, both of which are important for metabolic processes. Based on this, many therapies have been developed to target different aspects of nucleotide synthesis, with some inhibiting generation of purine nucleotides and some pyrimidine nucleotides or both.

The pyrimidine nucleotide cytidine 5' triphosphate (CTP) is a precursor required not just for the anabolism of DNA and RNA but also phospholipids and sialyation of proteins. CTP originates from two sources: a salvage pathway and a *de novo* synthesis pathway that depends on two enzymes, the CTP synthases (or synthetases) 1 and 2 (CTPS1 and CTPS2) (Evans and Guy 2004; Higgins, *et al.* 2007; Ostrander, *et al.* 1998).

CTPS1 and CTPS2 catalyse the conversion of uridine triphosphate (UTP) and glutamine into cytidine triphosphate (CTP) and L-glutamate:

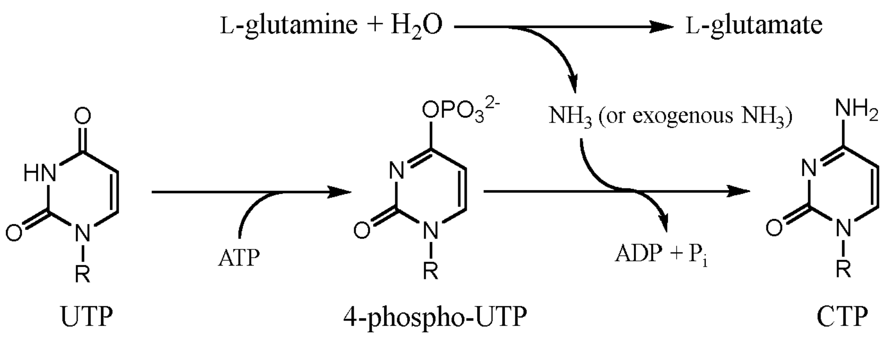


Both enzymes have two domains, an N-terminal synthetase domain and a C-terminal glutaminase domain (Kursula, *et al.* 2006). The synthetase domain transfers a phosphate from adenosine triphosphate (ATP) to the 4-position of UTP to create an activated intermediate, 4-phospho-UTP. The glutaminase domain generates ammonia from glutamine, via a covalent thioester intermediate with a conserved active site cysteine, generating glutamate. This ammonium is transferred from the glutaminase domain to the synthetase domain *via* a tunnel or can be derived from external ammonium. This ammonium is then used by the synthetase domain to generate CTP from the 4-phospho-UTP (Lieberman, 1956).

Although CTPS exists as two isozymes in humans and other eukaryotic organisms, CTPS1 and CTPS2, functional differences between the two isozymes are not yet fully elucidated (van Kuilenburg, *et al.* 2000).

The immune system provides protection from infections and has therefore evolved to rapidly respond to the wide variety of pathogens that the individual may be exposed to. This response can take many forms, but the expansion and differentiation of immune populations is a critical element and is hence closely linked to rapid cell proliferation. Within this, CTP synthase activity appears to play an important role in DNA synthesis and the rapid expansion of lymphocytes following activation (Fairbanks, *et al.* 1995; van den Berg, *et al.* 1995).

Strong clinical validation that CTPS1 is the critical enzyme in human lymphocyte proliferation came with the identification of a loss-of-function homozygous mutation (rs145092287) in this enzyme that causes a distinct and life-threatening immunodeficiency, characterized by an impaired capacity of activated T- and B-cells to proliferate in response to antigen receptor-mediated activation. Activated CTPS1-deficient cells were shown to have decreased levels of CTP. Normal T-cell proliferation was restored in CTPS1-deficient cells by expressing wild-type CTPS1 or by addition of cytidine. CTPS1 expression was found to be low in resting lymphocytes, but rapidly upregulated following activation of these cells. Expression of CTPS1 in other tissues was generally low. CTPS2 seems to be ubiquitously expressed in a range of cells and tissues but at low levels, and the failure of CTPS2, which is still intact in the patients, to compensate for the mutated CTPS1, supports CTPS1 being the critical enzyme for the immune populations affected in the patients (Martin, *et al.* 2014).

Overall, these findings suggest that CTPS1 is a critical enzyme necessary to meet the demands for the supply of

CTP required by several important immune cell populations.

Normally the immune response is tightly regulated to ensure protection from infection, whilst controlling any response targeting host tissues. In certain situations, the control of this process is not effective, leading to immune-mediated pathology. A wide range of human diseases are thought to be due to such inappropriate responses mediated by different elements of the immune system.

Given the role that cell populations, such as T and B lymphocytes, are thought to play in a wide range of autoimmune and other diseases, CTPS1 represents a target for a new class of immunosuppressive agents. Inhibition of CTPS1 therefore provides a novel approach to the inhibition of activated lymphocytes and selected other immune cell populations such as Natural Killer cells, Mucosal-Associated Invariant T (MAIT) and Invariant Natural Killer T cells, highlighted by the phenotype of the human mutation patients (Martin, *et al.* 2014).

Cancer can affect multiple cell types and tissues but the underlying cause is a breakdown in the control of cell division. This process is highly complex, requiring careful coordination of multiple pathways, many of which remain to be fully characterised. Cell division requires the effective replication of the cell's DNA and other constituents. Interfering with a cell's ability to replicate by targeting nucleic acid synthesis has been a core approach in cancer therapy for many years. Examples of therapies acting in this way are 6-thioguanine, 6-mecaptopurine, 5-fluorouracil, cytarabine, gemcitabine and pemetrexed.

As indicated above, pathways involved in providing the key building blocks for nucleic acid replication are the purine and pyrimidine synthesis pathways, and pyrimidine biosynthesis has been observed to be up-regulated in tumors and neoplastic cells.

CTPS activity is upregulated in a range of tumour types of both haematological and non-haematological origin, although heterogeneity is observed among patients. Linkages have also been made between high enzyme levels and resistance to chemotherapeutic agents.

Currently, the precise role that CTPS1 and CTPS2 may play in cancer is not completely clear. Several non-selective CTPS inhibitors have been developed for oncology indications up to phase I/II clinical trials, but were stopped due to toxicity and efficacy issues.

Most of the developed inhibitors are nucleoside-analogue prodrugs (3-deazauridine, CPEC, carbodine), which are converted to the active triphosphorylated metabolite by the kinases involved in pyrimidine biosynthesis: uridine/cytidine kinase, nucleoside monophosphate-kinase (NMP-kinase) and nucleoside diphosphatekinase (NDP-kinase). The remaining inhibitors (acivicin, DON) are reactive analogues of glutamine, which irreversibly inhibit the glutaminase domain of CTPS. Gemcitibine is also reported to have some inhibitory activity against CTPS (McClusky *et al.,* 2016).

CTPS therefore appears to be an important target in the cancer field. The nature of all of the above compounds is such that effects on other pathways are likely to contribute to the efficacy they show in inhibiting tumours.

Selective CTPS inhibitors therefore offer an attractive alternative approach for the treatment of tumours. Compounds with different potencies against CTPS1 and CTPS2 may offer important opportunities to target different tumours depending upon their relative dependence on these enzymes.

CTPS1 has also been suggested to play a role in vascular smooth muscle cell proliferation following vascular injury or surgery (Tang, *et al.* 2013).

As far as is known to date, no selective CTPS1 inhibitors have been developed. Recently, the CTPS1 selective inhibitory peptide CTpep-3 has been identified. The inhibitory effects of CTpep-3 however, were seen in cell free assays but not in the cellular context. This was not unexpected though, since the peptide is unlikely to enter the cell and hence is not easily developable as a therapeutic (Sakamoto, *et al.* 2017).

In summary, the available information and data strongly suggest that inhibitors of CTPS1 will reduce the proliferation of a number of immune and cancer cell populations, with the potential for an effect on other selected cell types such as vascular smooth muscle cells as well. Inhibitors of CTPS1 may therefore be expected to have utility for treatment or prophylaxis in a wide range of indications where the pathology is driven by these populations.

CTPS1 inhibitors represent a novel approach for inhibiting selected components of the immune system in various tissues, and the related pathologies or pathological conditions such as, in general terms, rejection of transplanted cells and tissues, Graft-related diseases or disorders, allergies and autoimmune diseases. In addition, CTPS1 inhibitors offer therapeutic potential in a range of cancer indications and in enhancing recovery from vascular injury or surgery and reducing morbidity and mortality associated with neointima and restenosis.

International patent applications WO2019/106156, WO2019/106146, WO2019/179652 and WO2019/180244 and WO2020/083975 disclose CTPS1 inhibitors. Patent applications EP3543232, EP3492454 and EP3578551 disclose CTPS1 inhibitors. International patent application WO2014/170435 discloses methods and pharmaceutical composition for inhibiting lymphocyte proliferation. International patent application WO02/24665 discloses aryl-alkane sulfonamides and their use as endothelin receptor antagonists. International patent application WO02/16318 discloses thiourea derivatives as vanilloid receptor modulators. US2003/158218 discloses thrombin inhibitors. Sandosham et al. "Heterocycles, vol. 37, mo. 1, 1994, pages 501-514" discloses the synthesis of pyrmidinyl triflates and palladium catalyzed coupling with organotin and organozinc reagents. CN104262071 discloses a photocatalysis synthesis method of biphenyl

compounds. GB1575803 discloses 3,7-disubstituted-3-cephem-4-carboxylic acid compounds which have antimicrobial activity. GB155007 discloses intermediates used in the preparation of substituted cephem carboxylic acid derivatives.

**Summary of the Invention**

The invention provides a compound of formula (I):

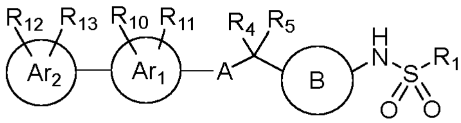

(I)

wherein ring B is selected from the group consisting of:

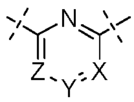

(B-a)

wherein X, Y and Z are as defined below; and

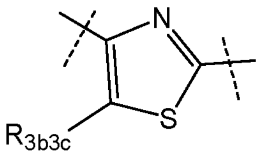

(B-bc);

wherein $R_{3b3c}$ is $R_{3b}$ or $R_{3c}$ as defined below;
wherein when B is (B-a) the compound of formula (I) is a compound of formula (I-a):

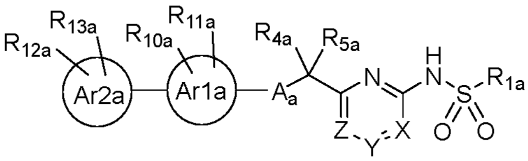

(I-a)

wherein:

$A_a$ is $A_{aa}$ or $A_{ba}$; wherein:

$A_{aa}$ is an amine linker having the following structure: -NH-, $-CH_2NH-$ or $-NHCH_2-$;
$A_{ba}$ is an amide linker having the following structure: -C(=O)NH- or -NHC(=O)-;

X is N or CH;

Y is N or $CR_{2a}$;

Z is N or $CR_{3a}$;
with the proviso that when at least one of X or Z is N, Y cannot be N;

$R_{2a}$ is H, halo, $C_{1-2}$alkyl, $OC_{1-2}$alkyl, $C_{1-2}$haloalkyl or $OC_{1-2}$haloalkyl; and

$R_{3a}$ is H, halo, $CH_3$, $OCH_3$, $CF_3$ or $OCF_3$;
wherein at least one of $R_{2a}$ and $R_{3a}$ is H;

$R_{1a}$ is $R_{1aa}$ or $R_{1ba}$; wherein:

$R_{1aa}$ is $NR_{32a}R_{33a}$;

$R_{1ba}$ is $C_{1-5}$alkyl, $C_{0-2}$alkylene$C_{3-5}$cycloalkyl which cycloalkyl is optionally substituted by $CH_3$, or $CF_3$;

$R_{4a}$ and $R_{5a}$ are $R_{4aa}$ and $R_{5aa}$, or $R_{4ba}$ and $R_{5ba}$;
wherein:

$R_{4aa}$ and $R_{5aa}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and $NR_{21a}R_{22a}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4aa}$ and $R_{5aa}$ together with the carbon atom to which they are attached may be substituted by one or two substituents, each substituent being independently selected from the group consisting of $C_{1-3}$alkyl or O$C_{1-3}$alkyl; or

$R_{4aa}$ and $R_{5aa}$ together with the carbon atom to which they are attached form a $C_{3-6}$heterocycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heterocycloalkyl formed by $R_{4aa}$ and $R_{5aa}$ together with the carbon atom to which they are attached may be substituted by one or two substituents, each substituent being independently selected from the group consisting of $C_{1-3}$alkyl or O$C_{1-3}$alkyl; or

$R_{4aa}$ and $R_{5aa}$ together with the carbon atom to which they are attached form a $C_{3-6}$heterocycloalkyl comprising one nitrogen atom, wherein said nitrogen atom is substituted by $-S(O)_2R_{29a}$; or

$R_{4ba}$ and $R_{5ba}$ are each independently H, $C_{1-6}$alkyl, $C_{1-6}$alkylOH, $C_{1-6}$haloalkyl, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, or $R_{4ba}$ and $R_{5ba}$ together with the carbon atom to which they are attached form a $C_{3-6}$cycloalkyl or $C_{3-6}$heterocycloalkyl; and

when $A_a$ is -NHC(=O)- or $-NHCH_2-$:
$R_{4ba}$ and $R_{5ba}$ may additionally be selected from halo, O$C_{1-6}$haloalkyl, O$C_{0-2}$alkylene$C_{3-6}$cycloalkyl, O$C_{0-2}$alkylene$C_{3-6}$heterocycloalkyl, O$C_{1-6}$alkyl and $NR_{21a}R_{22a}$;

Ar1a is a 6-membered aryl or heteroaryl;

Ar2a is a 6-membered aryl or heteroaryl and is attached to Ar1a in the para position relative to group $A_a$;

$R_{10a}$ is H, halo, $C_{1-3}$alkyl, $C_{1-2}$haloalkyl, O$C_{1-2}$alkyl, O$C_{1-2}$haloalkyl or CN;

$R_{11a}$ is H, F, Cl, $C_{1-2}$alkyl, $CF_3$, $OCH_3$ or CN;

$R_{12a}$ is attached to Ar2 in the ortho or meta position relative to Ar1a and $R_{12a}$ is H, halo, $C_{1-4}$alkyl, $C_{2-4}$alkenyl, $C_{0-2}$alkylene$C_{3-5}$cycloalkyl, O$C_{1-4}$alkyl, O$C_{0-2}$alkylene$C_{3-5}$cycloalkyl, $C_{1-4}$haloalkyl, O$C_{1-4}$haloalkyl, hydroxy, $C_{1-4}$alkylOH, $SO_2C_{1-2}$alkyl, C(O)N($C_{1-2}$alkyl$)_2$, NHC(O)$C_{1-3}$alkyl or $NR_{23a}R_{24a}$; and
when $A_a$ is -NHC(=O)-, -NH- or $-NHCH_2-$:
$R_{12a}$ may additionally be selected from CN, $OCH_2CH_2N(CH_3)_2$ and a $C_{3-6}$heterocycloalkyl comprising one nitrogen located at the point of attachment to Ar2a, or $R_{12a}$ together with a nitrogen atom to which it is attached forms an N-oxide ($N^+$-$O^-$);

$R_{13a}$ is H or halo;

$R_{21a}$ is H, $C_{1-5}$alkyl, C(O)$C_{1-5}$alkyl, C(O)O$C_{1-5}$alkyl, $C_{1-3}$alkylO$C_{1-2}$alkyl, $C_{1-4}$haloalkyl, or $C_{4-6}$heterocycloalkyl;

$R_{22a}$ is H or $CH_3$;

$R_{23a}$ is H or $C_{1-2}$alkyl; and

$R_{24a}$ is H or $C_{1-2}$alkyl

$R_{29a}$ is $C_{1-3}$alkyl, $C_{0-2}$alkylene$C_{3-5}$cycloalkyl which cycloalkyl is optionally substituted by $CH_3$, $CF_3$, $N(C_{1-3}alkyl)_2$, or a 5 or 6 membered heteroaryl wherein the 5 or 6 membered heteroaryl is optionally substituted by methyl;

$R_{32a}$ is $C_{1-3}$alkyl and $R_{33a}$ is $C_{1-3}$alkyl; or

$R_{32a}$ and $R_{33a}$ together with the nitrogen atom to which they are attached form a $C_{3-5}$heterocycloalkyl; wherein:

$R_{1a}$ is $R_{1aa}$; and/or

$R_{4a}$ and $R_{5a}$ are $R_{4aa}$ and $R_{5aa}$; and/or

$A_a$ is $A_{aa}$; and

wherein when B is (B-bc) and $R_{3b3c}$ is $R_{3b}$, the compound of formula (I) is a compound of formula (I-b):

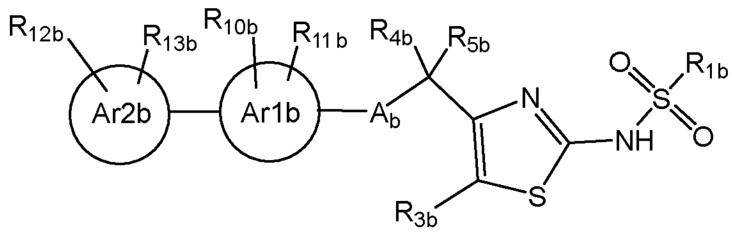

(I-b)

$A_b$ is $A_{ab}$ or $A_{bb}$;
wherein:

$A_{ab}$ is $-NR_{6b}CH_2-$ or $-NR_{6b}-$;

$A_{bb}$ is $-NR_{6b}C(=O)-$;

$R_{1b}$ is $R_{1ab}$ or $R_{1bb}$;
wherein:

$R_{1ab}$ is $NR_{32b}R_{33b}$;

$R_{1bb}$ is $C_{1-5}$alkyl, $C_{0-2}$alkylene$C_{3-5}$cycloalkyl which cycloalkyl is optionally substituted by $CH_3$, $C_{1-3}$alkyleneO$C_{1-2}$alkyl, or $CF_3$;

$R_{3b}$ is H, halo, $CH_3$, O$C_{1-2}$alkyl or $CF_3$;

or $R_{3b}$ together with $R_{5bb}$ forms a 5- or 6-membered cycloalkyl or 5 or 6 membered oxygen-containing heterocycloalkyl;

$R_{4b}$ and $R_{5b}$ are either $R_{4ab}$ and $R_{5ab}$ or $R_{4bb}$ and $R_{5bb}$;
wherein:

$R_{4ab}$ and $R_{5ab}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-

$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and $NR_{21b}R_{22b}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ab}$ and $R_{5ab}$ together with the carbon atom to which they are attached may be substituted by one or two substituents, each substituent being independently selected from the group consisting of $C_{1-3}$alkyl or O$C_{1-3}$alkyl; or

$R_{4ab}$ and $R_{5ab}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ab}$ and $R_{5ab}$ together with the carbon atom to which they are attached may be substituted by one or two substituents, each substituent being independently selected from the group consisting of $C_{1-3}$alkyl or O$C_{1-3}$alkyl; or

$R_{4ab}$ and $R_{5ab}$ together with the carbon atom to which they are attached form a $C_{3-6}$heterocycloalkyl comprising one nitrogen atom, wherein said nitrogen atom is substituted by $-S(O)_2R_{29b}$; or

$R_{4bb}$ and $R_{5bb}$ are each independently H, halo, $C_{1-6}$alkyl, $C_{0-2}$alkylene$C_{3-6}$cycloalkyl, $C_{0-2}$alkylene$C_{3-6}$heterocycloalkyl, O$C_{1-6}$alkyl, O$C_{0-2}$alkylene$C_{3-6}$cycloalkyl, $C_{1-3}$alkyleneO$C_{1-3}$alkyl, $C_{1-6}$alkylOH, $C_{1-6}$haloalkyl, O$C_{1-6}$haloalkyl or $NR_{21b}R_{22b}$,

or $R_{4bb}$ is H and $R_{5bb}$ together with $R_{3b}$ form a 5- or 6-membered cycloalkyl or 5 or 6 membered oxygen-containing heterocycloalkyl,

or $R_{4bb}$ and $R_{5bb}$ together with the carbon atom to which they are attached form a $C_{3-6}$cycloalkyl or $C_{3-6}$heterocycloalkyl,

or $R_{4bb}$ is H and $R_{5bb}$ and $R_{6b}$ are a $C_{2-3}$alkylene chain forming a 5- or 6-membered ring;

or $R_{4bb}$ is O and $R_{5bb}$ is absent;

$R_{6b}$ is H or $C_{1-3}$alkyl,

or $R_{6b}$ together with $R_{11b}$ when in the ortho-position to group $A_b$ are a $C_2$alkylene chain forming a 5-membered ring,

or $R_{5bb}$ and $R_{6b}$ are a $C_{2-3}$alkylene chain forming a 5- or 6-membered ring and $R_{4bb}$ is H;

Ar1b is 6-membered aryl or heteroaryl;

Ar2b is a 6-membered aryl or heteroaryl and is attached to Ar1b in the para position relative to group $A_b$;

$R_{10b}$ is H, halo, $C_{1-3}$alkyl, O$C_{1-2}$alkyl, $C_{1-2}$haloalkyl, O$C_{1-2}$haloalkyl or CN;

$R_{11b}$ is H, F, Cl, $CH_3$, ethyl, $OCH_3$, $CF_3$, $OCF_3$ or CN,
or $R_{11b}$, when in the ortho-position to group $A_b$, together with $R_{6b}$ are a $C_2$alkylene chain forming a 5-membered ring;

$R_{12b}$ is attached to Ar2b in the ortho or meta position relative to Ar1b and $R_{12b}$ is H, halo, $C_{1-4}$alkyl, $C_{2-4}$alkynyl, $C_{0-2}$alkylene$C_{3-5}$cycloalkyl, O$C_{1-4}$alkyl, O$C_{0-2}$alkylene$C_{3-5}$cycloalkyl, $OCH_2CH_2N(CH_3)_2$, OH, $C_{1-4}$alkylOH, CN, $C_{1-3}$alkyleneO$C_{1-3}$alkyl, $C_{1-4}$haloalkyl, O$C_{1-4}$haloalkyl, C(=O)$C_{1-2}$alkyl, $NR_{23b}R_{24b}$, $SO_2C_{1-4}$alkyl, SO$C_{1-4}$alkyl, S$C_{1-4}$alkyl, SH, $C(O)N(CH_3)_2$, NHC(O)$C_{1-3}$alkyl, $C_{3-6}$heterocycloalkyl comprising one nitrogen located at the point of attachment to Ar2b, or $R_{12b}$ together with a nitrogen atom to which it is attached forms an N-oxide ($N^+$-$O^-$);

$R_{13b}$ is H, halo, $CH_3$ or $OCH_3$;

$R_{21b}$ is H, $C_{1-5}$alkyl, C(O)$C_{1-5}$alkyl, C(O)O$C_{1-5}$alkyl, $C_{1-3}$alkylO$C_{1-2}$alkyl, $C_{1-4}$haloalkyl, or $C_{4-6}$heterocycloalkyl;

$R_{22b}$ is H or $CH_3$;

$R_{23b}$ is H or $C_{1-2}$alkyl;

$R_{24b}$ is H or $C_{1-2}$alkyl;

$R_{29b}$ is $C_{1-3}$alkyl, $C_{0-2}$alkylene$C_{3-5}$cycloalkyl which cycloalkyl is optionally substituted by $CH_3$, $CF_3$, N($C_{1-3}$alkyl)$_2$, or a 5 or 6 membered heteroaryl wherein the 5 or 6 membered heteroaryl is optionally substituted by methyl; and

$R_{32b}$ is $C_{1-3}$alkyl and $R_{33b}$ is $C_{1-3}$alkyl; or

$R_{32b}$ and $R_{33b}$ together with the nitrogen atom to which they are attached form a $C_{3-5}$heterocycloalkyl; wherein:

$R_{1b}$ is $R_{1ab}$; and/or

$R_{4b}$ and $R_{5b}$ are $R_{4ab}$ and $R_{5ab}$; and/or

$A_b$ is $A_{ab}$; or

wherein when B is (B-bc) and $R_{3b3c}$ is $R_{3c}$, the compound of formula (I) is a compound of formula (I-c):

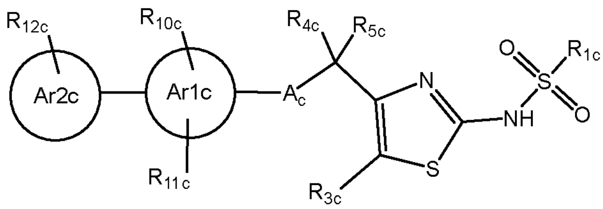

(I-c)

wherein:

$A_c$ is $A_{ac}$ or $A_{bc}$;
wherein:

$A_{ac}$ is -$CH_2NR_{6c}$-;
$A_{bc}$ is -C(=O)$NR_{6c}$-;

$R_{1c}$ is $R_{1ac}$ or $R_{1bc}$;
wherein:
$R_{1ac}$ is $NR_{32c}R_{33c}$;

$R_{1bc}$ is $C_{1-5}$alkyl, $C_{0-2}$alkylene$C_{3-5}$cycloalkyl which cycloalkyl is optionally substituted by $CH_3$, $C_{1-3}$alkyleneO-$C_{1-2}$alkyl, or $CF_3$;

$R_{3c}$ is H, $CH_3$, halo, O$C_{1-2}$alkyl or $CF_3$;

$R_{4c}$ and $R_{5c}$ are either $R_{4ac}$ and $R_{5aa}$ or $R_{4bc}$ and $R_{5bc}$;
wherein:

$R_{4ac}$ and $R_{5aa}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and $NR_{21c}R_{22c}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ac}$ and $R_{5ac}$ together with the carbon atom to which they are attached may be substituted by one or two substituents, each substituent being independently selected from the group consisting of $C_{1-3}$alkyl or $OC_{1-3}$alkyl; or

$R_{4ac}$ and $R_{5aa}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ac}$ and $R_{5ac}$ together with the carbon atom to which they are attached may be substituted by one or two substituents, each substituent being independently selected from the group consisting of $C_{1-3}$alkyl or $OC_{1-3}$alkyl; or

$R_{4ac}$ and $R_{5aa}$ together with the carbon atom to which they are attached form a $C_{3-6}$heterocycloalkyl comprising one nitrogen atom, wherein said nitrogen atom is substituted by $-S(O)_2R_{29c}$; or

$R_{4bc}$ and $R_{5bc}$ are each independently H, $C_{1-6}$alkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, $C_{1-6}$alkylOH or $C_{1-6}$haloalkyl,

or $R_{4bc}$ and $R_{5bc}$ together with the carbon atom to which they are attached form a $C_{3-6}$cycloalkyl or $C_{3-6}$heterocycloalkyl ring;

$R_{6c}$ is H or $C_{1-3}$alkyl;

Ar1c is a 6-membered aryl or heteroaryl;

Ar2c is a 6-membered aryl or heteroaryl and is attached to Ar1c in the para position relative to group $A_c$;

$R_{10c}$ is H, halo, $C_{1-3}$alkyl, $OC_{1-2}$alkyl, $C_{1-2}$haloalkyl, $OC_{1-2}$haloalkyl or CN;

$R_{11c}$ is H, F, Cl, $CH_3$, ethyl, $OCH_3$, $CF_3$, $OCF_3$ or CN;

$R_{12c}$ is attached to Ar2c in the meta or ortho position relative to Ar1c and $R_{12c}$ is H, halo, $C_{1-4}$alkyl, $C_{2-4}$alkynyl, $C(=O)C_{1-2}$alkyl, $C_{0-2}$alkylene$C_{3-5}$cycloalkyl, $OC_{1-4}$alkyl, $C_{1-3}$alkylene$OC_{1-3}$alkyl, $C_{1-4}$haloalkyl, $OC_{1-4}$haloalkyl, CN, $OC_{0-2}$alkylene$C_{3-5}$cycloalkyl, $OCH_2CH_2N(CH_3)_2$, OH, $C_{1-4}$alkylOH, $NR_{23c}R_{24c}$, $SO_2CH_3$, $C(O)N(CH_3)_2$, $NHC(O)C_{1-3}$alkyl, or a $C_{3-6}$heterocycloalkyl comprising one nitrogen located at the point of attachment to Ar2c, or $R_{12c}$ together with a nitrogen atom to which it is attached forms an N-oxide ($N^+$-$O^-$);

$R_{21c}$ is H, $C_{1-5}$alkyl, $C(O)C_{1-5}$alkyl, $C(O)OC_{1-5}$alkyl, $C_{1-3}$alkyl$OC_{1-2}$alkyl, $C_{1-4}$haloalkyl, or $C_{4-6}$heterocycloalkyl;

$R_{22c}$ is H or $CH_3$;

$R_{23c}$ is H or $C_{1-2}$alkyl;

$R_{24c}$ is H or $C_{1-2}$alkyl;

$R_{29c}$ is $C_{1-3}$alkyl, $C_{0-2}$alkylene$C_{3-5}$cycloalkyl which cycloalkyl is optionally substituted by $CH_3$, $CF_3$, $N(C_{1-3}alkyl)_2$, or a 5 or 6 membered heteroaryl wherein the 5 or 6 membered heteroaryl is optionally substituted by methyl; and

$R_{32c}$ is $C_{1-3}$alkyl and $R_{33c}$ is $C_{1-3}$alkyl; or

$R_{32c}$ and $R_{33c}$ together with the nitrogen atom to which they are attached form a $C_{3-5}$heterocycloalkyl;

wherein:

$R_{1c}$ is $R_{1ac}$; and/or

$R_{4c}$ and $R_{5c}$ are $R_{4ac}$ and $R_{5ac}$; and/or

$A_c$ is $A_{ac}$.

In one embodiment, the compound formula (I) is a compound of formula (I-a).

In another embodiment, the compound formula (I) is a compound of formula (I-b).

In another embodiment, the compound formula (I) is a compound of formula (I-c).

A compound of formula (I) may be provided in the form of a salt and/or solvate thereof.. In particular, the compound of formula (I) may be provided in the form of a pharmaceutically acceptable salt and/or solvate, such as a pharmaceutically acceptable salt.

Also provided is a compound of formula (I), or a pharmaceutically acceptable salt and/or solvate thereof, for use as a medicament, in particular for use in the inhibition of CTPS1 in a subject or the prophylaxis or treatment of associated diseases or disorders, such as those in which a reduction in T-cell and/or B-cell proliferation would be beneficial.

Suitably the disease or disorder is selected from: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; and transplantation. In addition, the disease or disorder may be selected from myasthenia gravis, multiple sclerosis, and scleroderma/systemic sclerosis.

Also provided is a compound of formula (I), or a pharmaceutically acceptable salt and/or solvate thereof, for use in the treatment of cancer.

Also provided is a compound of formula (I), or a pharmaceutically acceptable salt and/or solvate thereof, for use in enhancing recovery from vascular injury or surgery and reducing morbidity and mortality associated with neointima and restenosis in a subject.

Also provided are pharmaceutical compositions containing a compound of formula (I), or a pharmaceutically acceptable salt and/or solvate thereof, and a pharmaceutically acceptable carrier or excipient.

## Detailed description of the Invention

In one embodiment there is provided a compound of formula (I) as described above, or a salt such as a pharmaceutically acceptable salt and/or solvate thereof.

Suitably, the invention provides a compound of formula (I):

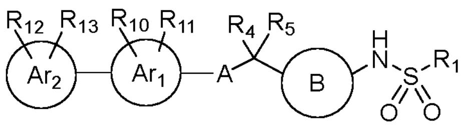
(I)

wherein ring B is selected from the group consisting of:

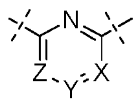
(B-a)

wherein X, Y and Z are as defined below; and

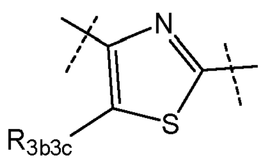
(B-bc);

wherein $R_{3b3c}$ is $R_{3b}$ or $R_{3c}$ as defined below;
wherein when B is (B-a) the compound of formula (I) is a compound of formula (I-a):

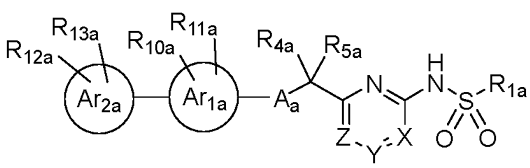
(I-a)

wherein:

$A_a$ is $A_{aa}$ or $A_{ba}$;
wherein:

$A_{aa}$ is an amine linker having the following structure: -NH-, $-CH_2NH-$ or $-NHCH_2-$;
$A_{ba}$ is an amide linker having the following structure: -C(=O)NH- or -NHC(=O)-;

X is N or CH;

Y is N or $CR_{2a}$;

Z is N or $CR_{3a}$;
with the proviso that when at least one of X or Z is N, Y cannot be N;

$R_{2a}$ is H, halo, $C_{1-2}$alkyl, $OC_{1-2}$alkyl, $C_{1-2}$haloalkyl or $OC_{1-2}$haloalkyl; and

$R_{3a}$ is H, halo, $CH_3$, $OCH_3$, $CF_3$ or $OCF_3$;
wherein at least one of $R_{2a}$ and $R_{3a}$ is H;

$R_{1a}$ is $R_{1aa}$ or $R_{1ba}$;
wherein:

$R_{1aa}$ is $NR_{32a}R_{33a}$;
$R_{1ba}$ is $C_{1-5}$alkyl, $C_{0-2}$alkylene$C_{3-5}$cycloalkyl which cycloalkyl is optionally substituted by $CH_3$, or $CF_3$;

$R_{4a}$ and $R_{5a}$ are $R_{4aa}$ and $R_{5aa}$, or $R_{4ba}$ and $R_{5ba}$;
wherein:

$R_{4aa}$ and $R_{5aa}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21a}R_{22a}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or

$R_{4aa}$ and $R_{5aa}$ together with the carbon atom to which they are attached form a $C_{3-6}$heterocycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heterocycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or

$R_{4aa}$ and $R_{5aa}$ together with the carbon atom to which they are attached form a $C_{3-6}$heterocycloalkyl comprising one nitrogen atom, wherein said nitrogen atom is substituted by $-S(O)_2R_{29a}$; or

$R_{4ba}$ and $R_{5ba}$ are each independently H, $C_{1-6}$alkyl, $C_{1-6}$alkylOH, $C_{1-6}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, or $R_{4ba}$ and $R_{5ba}$ together with the carbon atom to which they are attached form a $C_{3-6}$cycloalkyl or $C_{3-6}$heterocycloalkyl; and

when $A_a$ is -NHC(=O)- or $-NHCH_2-$:

$R_{4ba}$ and $R_{5ba}$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl and $NR_{21a}R_{22a}$;

Ar1a is a 6-membered aryl or heteroaryl;

Ar2a is a 6-membered aryl or heteroaryl and is attached to Ar1a in the para position relative to group $A_a$;

$R_{10a}$ is H, halo, $C_{1-3}$alkyl, $C_{1-2}$haloalkyl, $OC_{1-2}$alkyl, $OC_{1-2}$haloalkyl or CN;

$R_{11a}$ is H, F, Cl, $C_{1-2}$alkyl, $CF_3$, $OCH_3$ or CN;

$R_{12a}$ is attached to Ar2 in the ortho or meta position relative to Ar1a and $R_{12a}$ is H, halo, $C_{1-4}$alkyl, $C_{2-4}$alkenyl, $C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, $NHC(O)C_{1-3}$alkyl or $NR_{23a}R_{24a}$; and
when $A_a$ is -NHC(=O)-, -NH- or -$NHCH_2$-:
$R_{12a}$ may additionally be selected from CN, $OCH_2CH_2N(CH_3)_2$ and a $C_{3-6}$heterocycloalkyl comprising one nitrogen located at the point of attachment to Ar2a, or $R_{12a}$ together with a nitrogen atom to which it is attached forms an N-oxide ($N^+$-$O^-$);

$R_{13a}$ is H or halo;

$R_{21a}$ is H, $C_{1-5}$alkyl, $C(O)C_{1-5}$alkyl, $C(O)OC_{1-5}$alkyl;

$R_{22a}$ is H or $CH_3$;

$R_{23a}$ is H or $C_{1-2}$alkyl; and

$R_{24a}$ is H or $C_{1-2}$alkyl

$R_{29a}$ is $C_{1-3}$alkyl, $C_{0-2}$alkylene$C_{3-5}$cycloalkyl which cycloalkyl is optionally substituted by $CH_3$, or $CF_3$;

$R_{32a}$ is $C_{1-3}$alkyl and $R_{33a}$ is $C_{1-3}$alkyl; or

$R_{32a}$ and $R_{33a}$ together with the nitrogen atom to which they are attached form a $C_{3-5}$heterocycloalkyl;

wherein

$R_{1a}$ is $R_{1aa}$; and/or

$R_{4a}$ and $R_{5a}$ are $R_{4aa}$ and $R_{5aa}$; and/or

$A_a$ is $A_{aa}$; and

wherein when B is (B-bc) and $R_{3b3c}$ is $R_{3b}$, the compound of formula (I) is a compound of formula (I-b):

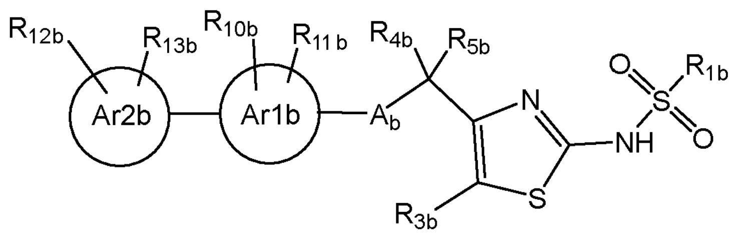

(I-b)

wherein:

$A_b$ is $A_{ab}$ or $A_{bb}$;
wherein:

$A_{ab}$ is $-NR_{6b}CH_2-$ or $-NR_{6b}-$;

$A_{bb}$ is $-NR_{6b}C(=O)-$;

$R_{1b}$ is $R_{1ab}$ or $R_{1bb}$;

wherein:

$R_{1ab}$ is $NR_{32b}R_{33b}$;

$R_{1bb}$ is $C_{1-5}$alkyl, $C_{0-2}$alkylene$C_{3-5}$cycloalkyl which cycloalkyl is optionally substituted by $CH_3$, $C_{1-3}$alkyleneO$C_{1-2}$alkyl, or $CF_3$;

$R_{3b}$ is H, halo, $CH_3$, O$C_{1-2}$alkyl or $CF_3$;

or $R_{3b}$ together with $R_{5bb}$ forms a 5- or 6-membered cycloalkyl or 5 or 6 membered oxygen-containing heterocycloalkyl;

$R_{4b}$ and $R_{5b}$ are either $R_{4ab}$ and $R_{5ab}$ or $R_{4bb}$ and $R_{5bb}$;
wherein:

$R_{4ab}$ and $R_{5ab}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and $NR_{21b}R_{22b}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ab}$ and $R_{5ab}$ together with the carbon atom to which they are attached may be substituted by one or two substituents, each substituent being independently selected from the group consisting of $C_{1-3}$alkyl or O$C_{1-3}$alkyl; or

$R_{4ab}$ and $R_{5ab}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ab}$ and $R_{5ab}$ together with the carbon atom to which they are attached may be substituted by one or two substituents, each substituent being independently selected from the group consisting of $C_{1-3}$alkyl or O$C_{1-3}$alkyl; or

$R_{4ab}$ and $R_{5ab}$ together with the carbon atom to which they are attached form a $C_{3-6}$heterocycloalkyl comprising one nitrogen atom, wherein said nitrogen atom is substituted by $-S(O)_2R_{29b}$; or

$R_{4bb}$ and $R_{5bb}$ are each independently H, halo, $C_{1-6}$alkyl, $C_{0-2}$alkylene$C_{3-6}$cycloalkyl, $C_{0-2}$alkylene$C_{3-6}$heterocycloalkyl, O$C_{1-6}$alkyl, O$C_{0-2}$alkylene$C_{3-6}$cycloalkyl, $C_{1-3}$alkyleneO$C_{1-3}$alkyl, $C_{1-6}$alkylOH, $C_{1-6}$haloalkyl, O$C_{1-6}$haloalkyl or $NR_{21b}R_{22b}$,

or $R_{4bb}$ is H and $R_{5bb}$ together with $R_{3b}$ form a 5- or 6-membered cycloalkyl or 5 or 6 membered oxygen-containing heterocycloalkyl,

or $R_{4bb}$ and $R_{5bb}$ together with the carbon atom to which they are attached form a $C_{3-6}$cycloalkyl or $C_{3-6}$heterocycloalkyl,

or $R_{4bb}$ is H and $R_{5bb}$ and $R_{6b}$ are a $C_{2-3}$alkylene chain forming a 5- or 6-membered ring;

or $R_{4bb}$ is O and $R_{5bb}$ is absent;

$R_{6b}$ is H or $C_{1-3}$alkyl,

or $R_{6b}$ together with $R_{11b}$ when in the ortho-position to group $A_b$ are a $C_2$alkylene chain forming a 5-membered ring,

or $R_{5bb}$ and $R_{6b}$ are a $C_{2-3}$alkylene chain forming a 5- or 6-membered ring and $R_{4bb}$ is H;

Ar1b is 6-membered aryl or heteroaryl;

Ar2b is a 6-membered aryl or heteroaryl and is attached to Ar1b in the para position relative to group $A_b$;

$R_{10b}$ is H, halo, $C_{1-3}$alkyl, $OC_{1-2}$alkyl, $C_{1-2}$haloalkyl, $OC_{1-2}$haloalkyl or CN;

$R_{11b}$ is H, F, Cl, $CH_3$, ethyl, $OCH_3$, $CF_3$, $OCF_3$ or CN,
or $R_{11b}$, when in the ortho-position to group $A_b$, together with $R_{6b}$ are a $C_2$alkylene chain forming a 5-membered ring;

$R_{12b}$ is attached to Ar2b in the ortho or meta position relative to Ar1b and $R_{12b}$ is H, halo, $C_{1-4}$alkyl, $C_{2-4}$alkynyl, $C_{0-2}$alkylene$C_{3-5}$cycloalkyl, $OC_{1-4}$alkyl, $OC_{0-2}$alkylene$C_{3-5}$cycloalkyl, $OCH_2CH_2N(CH_3)_2$, OH, $C_{1-4}$alkylOH, CN, $C_{1-3}$alkylene$OC_{1-3}$alkyl, $C_{1-4}$haloalkyl, $OC_{1-4}$haloalkyl, $C(=O)C_{1-2}$alkyl, $NR_{23b}R_{24b}$, $SO_2C_{1-4}$alkyl, $SOC_{1-4}$alkyl, $SC_{1-4}$alkyl, SH, $C(O)N(CH_3)_2$, $NHC(O)C_{1-3}$alkyl, $C_{3-6}$heterocycloalkyl comprising one nitrogen located at the point of attachment to Ar2b, or $R_{12b}$ together with a nitrogen atom to which it is attached forms an N-oxide ($N^+$-$O^-$);

$R_{13b}$ is H, halo, $CH_3$ or $OCH_3$;

$R_{21b}$ is H, $C_{1-5}$alkyl, $C(O)C_{1-5}$alkyl, $C(O)OC_{1-5}$alkyl;

$R_{22b}$ is H or $CH_3$;

$R_{23b}$ is H or $C_{1-2}$alkyl;

$R_{24b}$ is H or $C_{1-2}$alkyl;

$R_{29b}$ is $C_{1-3}$alkyl, $C_{0-2}$alkylene$C_{3-5}$cycloalkyl which cycloalkyl is optionally substituted by $CH_3$, or $CF_3$; and

$R_{32b}$ is $C_{1-3}$alkyl and $R_{33b}$ is $C_{1-3}$alkyl; or

$R_{32b}$ and $R_{33b}$ together with the nitrogen atom to which they are attached form a $C_{3-5}$heterocycloalkyl;

wherein:

$R_{1b}$ is $R_{1ab}$; and/or

$R_{4b}$ and $R_{5b}$ are $R_{4ab}$ and $R_{5ab}$; and/or

$A_b$ is $A_{ab}$; or

wherein when B is (B-bc) and $R_{3b3c}$ is $R_{3c}$, the compound of formula (I) is a compound of formula (I-c):

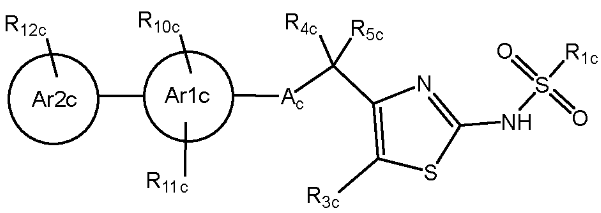

(I-c)

wherein:

$A_c$ is $A_{ac}$ or $A_{bc}$;

$A_{ac}$ is $-CH_2NR_{6c}-$;

$A_{bc}$ is $-C(=O)NR_{6c}-$;

$R_{1c}$ is $R_{1ac}$ or $R_{1bc}$;
wherein:
$R_{1ac}$ is $NR_{32c}R_{33c}$;

$R_{1bc}$ is $C_{1-5}$alkyl, $C_{0-2}$alkylene$C_{3-5}$cycloalkyl which cycloalkyl is optionally substituted by $CH_3$, $C_{1-3}$alkyleneO-$C_{1-2}$alkyl, or $CF_3$;

$R_{3c}$ is H, $CH_3$, halo, $OC_{1-2}$alkyl or $CF_3$;

$R_{4c}$ and $R_{5c}$ are either $R_{4ac}$ and $R_{5aa}$ or $R_{4bc}$ and $R_{5bc}$;

wherein:

$R_{4ac}$ and $R_{5ac}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21c}R_{22c}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ac}$ and $R_{5ac}$ together with the carbon atom to which they are attached may be substituted by one or two substituents, each substituent being independently selected from the group consisting of $C_{1-3}$alkyl or $OC_{1-3}$alkyl; or

$R_{4ac}$ and $R_{5ac}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ac}$ and $R_{5ac}$ together with the carbon atom to which they are attached may be substituted by one or two substituents, each substituent being independently selected from the group consisting of $C_{1-3}$alkyl or $OC_{1-3}$alkyl; or

$R_{4ac}$ and $R_{5ac}$ together with the carbon atom to which they are attached form a $C_{3-6}$heterocycloalkyl comprising one nitrogen atom, wherein said nitrogen atom is substituted by $-S(O)_2R_{29c}$; or

$R_{4bc}$ and $R_{5bc}$ are each independently H, $C_{1-6}$alkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, $C_{1-6}$alkylOH or $C_{1-6}$haloalkyl,

or $R_{4bc}$ and $R_{5bc}$ together with the carbon atom to which they are attached form a $C_{3-6}$cycloalkyl or $C_{3-6}$heterocycloalkyl ring;

$R_{6c}$ is H or $C_{1-3}$alkyl;

Ar1c is a 6-membered aryl or heteroaryl;

Ar2c is a 6-membered aryl or heteroaryl and is attached to Ar1c in the para position relative to group $A_c$;

$R_{10c}$ is H, halo, $C_{1-3}$alkyl, $OC_{1-2}$alkyl, $C_{1-2}$haloalkyl, $OC_{1-2}$haloalkyl or CN;

$R_{11c}$ is H, F, Cl, $CH_3$, ethyl, $OCH_3$, $CF_3$, $OCF_3$ or CN;

$R_{12c}$ is attached to Ar2c in the meta or ortho position relative to Ar1c and $R_{12c}$ is H, halo, $C_{1-4}$alkyl, $C_{2-4}$alkynyl, $C(=O)C_{1-2}$alkyl, $C_{0-2}$alkylene$C_{3-5}$cycloalkyl, $OC_{1-4}$alkyl, $C_{1-3}$alkylene$OC_{1-3}$alkyl, $C_{1-4}$haloalkyl, $OC_{1-4}$haloalkyl, CN, $OC_{0-2}$alkylene$C_{3-5}$cycloalkyl, $OCH_2CH_2N(CH_3)_2$, OH, $C_{1-4}$alkylOH, $NR_{23c}R_{24c}$, $SO_2CH_3$, $C(O)N(CH_3)_2$, $NHC(O)C_{1-3}$alkyl, or a $C_{3-6}$heterocycloalkyl comprising one nitrogen located at the point of attachment to Ar2c, or $R_{12c}$ together with a nitrogen atom to which it is attached forms an N-oxide ($N^+$-$O^-$);

$R_{21c}$ is H, $C_{1-5}$alkyl, $C(O)C_{1-5}$alkyl, $C(O)OC_{1-5}$alkyl;

$R_{22c}$ is H or $CH_3$;

$R_{23c}$ is H or $C_{1-2}$alkyl;

$R_{24c}$ is H or $C_{1-2}$alkyl;

$R_{29c}$ is $C_{1-3}$alkyl, $C_{0-2}$alkylene$C_{3-5}$cycloalkyl which cycloalkyl is optionally substituted by $CH_3$, or $CF_3$; and

$R_{32c}$ is $C_{1-3}$alkyl and $R_{33c}$ is $C_{1-3}$alkyl; or

$R_{32c}$ and $R_{33c}$ together with the nitrogen atom to which they are attached form a $C_{3-5}$heterocycloalkyl;

wherein:

$R_{1c}$ is $R_{1ac}$; and/or

$R_{4c}$ and $R_{5c}$ are $R_{4ac}$ and $R_{5ac}$; and/or

$A_c$ is $A_{ac}$.

More suitably, there is provided a compound of formula (I-a).

Alternatively, there is provided a compound of formula (I-b).

Alternatively, there is provided a compound of formula (I-c).

Compounds of formula (I-a)

The invention provides a compound of formula (I-a):

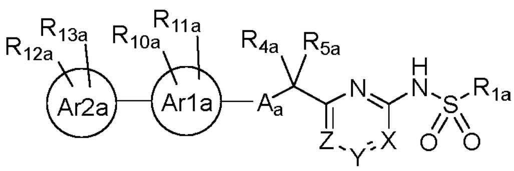

(I-a)

wherein:

$A_a$ is $A_{aa}$ or $A_{ba}$;
wherein:

$A_{aa}$ is an amine linker having the following structure: -NH-, -$CH_2$NH- or -NH$CH_2$-;

$A_{ba}$ is an amide linker having the following structure: -C(=O)NH- or -NHC(=O)-;

X is N or CH;

Y is N or $CR_{2a}$;

Z is N or $CR_{3a}$;
with the proviso that when at least one of X or Z is N, Y cannot be N;

$R_{1a}$ is $R_{1aa}$ or $R_{1ba}$;
wherein:

$R_{1aa}$ is $NR_{32a}R_{33a}$;

$R_{1ba}$ is $C_{1-5}$alkyl, $C_{0-2}$alkylene$C_{3-5}$cycloalkyl which cycloalkyl is optionally substituted by $CH_3$, or $CF_3$;

$R_{2a}$ is H, halo, $C_{1-2}$alkyl, $OC_{1-2}$alkyl, $C_{1-2}$haloalkyl or $OC_{1-2}$haloalkyl;

$R_{3a}$ is H, halo, $CH_3$, $OCH_3$, $CF_3$ or $OCF_3$;
wherein at least one of $R_{2a}$ and $R_{3a}$ is H;

$R_{4a}$ and $R_{5a}$ are $R_{4aa}$ and $R_{5aa}$, or $R_{4ba}$ and $R_{5ba}$;
wherein:

$R_{4aa}$ and $R_{5aa}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21a}R_{22a}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or

$R_{4aa}$ and $R_{5aa}$ together with the carbon atom to which they are attached form a $C_{3-6}$heterocycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heterocycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or

$R_{4aa}$ and $R_{5aa}$ together with the carbon atom to which they are attached form a $C_{3-6}$heterocycloalkyl comprising one nitrogen atom, wherein said nitrogen atom is substituted by $-S(O)_2R_{29a}$; or

$R_{4ba}$ and $R_{5ba}$ are each independently H, $C_{1-6}$alkyl, $C_{1-6}$alkylOH, $C_{1-6}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, or $R_{4ba}$ and $R_{5ba}$ together with the carbon atom to which they are attached form a $C_{3-6}$cycloalkyl or $C_{3-6}$heterocycloalkyl; and

when $A_a$ is -NHC(=O)- or $-NHCH_2-$:
$R_{4ba}$ and $R_{5ba}$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl and $NR_{21a}R_{22a}$;

Ar1a is a 6-membered aryl or heteroaryl;

Ar2a is a 6-membered aryl or heteroaryl and is attached to Ar1a in the para position relative to group $A_a$;

$R_{10a}$ is H, halo, $C_{1-3}$alkyl, $C_{1-2}$haloalkyl, $OC_{1-2}$alkyl, $OC_{1-2}$haloalkyl or CN;

$R_{11a}$ is H, F, Cl, $C_{1-2}$alkyl, $CF_3$, $OCH_3$ or CN;

$R_{12a}$ is attached to Ar2a in the ortho or meta position relative to Ar1a and $R_{12a}$ is H, halo, $C_{1-4}$alkyl, $C_{2-4}$alkenyl,

$C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, $NHC(O)C_{1-3}$alkyl or $NR_{23a}R_{24a}$; and

when $A_a$ is -NHC(=O)-, -NH- or $-NHCH_2-$:

$R_{12a}$ may additionally be selected from CN, $OCH_2CH_2N(CH_3)_2$ and a $C_{3-6}$heterocycloalkyl comprising one nitrogen located at the point of attachment to Ar2a, or $R_{12a}$ together with a nitrogen atom to which it is attached forms an N-oxide ($N^+$-$O^-$);

$R_{13a}$ is H or halo;

$R_{21a}$ is H, $C_{1-5}$alkyl, $C(O)C_{1-5}$alkyl, $C(O)OC_{1-5}$alkyl, $C_{1-3}$alkyl$OC_{1-2}$alkyl, $C_{1-4}$haloalkyl, or $C_{4-6}$heterocycloalkyl;

$R_{22a}$ is H or $CH_3$;

$R_{23a}$ is H or $C_{1-2}$alkyl; and

$R_{24a}$ is H or $C_{1-2}$alkyl;

$R_{29a}$ is $C_{1-3}$alkyl, $C_{0-2}$alkylene$C_{3-5}$cycloalkyl which cycloalkyl is optionally substituted by $CH_3$, $CF_3$, $N(C_{1-3}alkyl)_2$, or a 5 or 6 membered heteroaryl wherein the 5 or 6 membered heteroaryl is optionally substituted by methyl;

$R_{32a}$ is $C_{1-3}$alkyl and $R_{33a}$ is $C_{1-3}$alkyl; or

$R_{32a}$ and $R_{33a}$ together with the nitrogen atom to which they are attached form a $C_{3-5}$heterocycloalkyl;

or a salt and/or solvate thereof.

Suitably, $R_{1c}$ is $R_{1ac}$; and/or $R_{4c}$ and $R_{5c}$ are $R_{4ac}$ and $R_{5ac}$; and/or $A_c$ is $A_{ac}$.

The invention also provides a compound of formula (I-a):

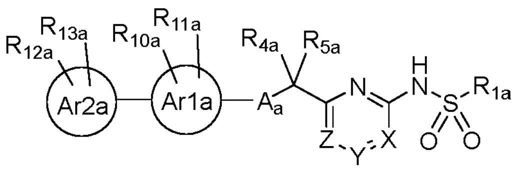

(I-a)

wherein:

$A_a$ is $A_{aa}$ or $A_{ba}$;
wherein:

$A_{aa}$ is an amine linker having the following structure: -NH-, $-CH_2NH-$ or $-NHCH_2-$;

$A_{ba}$ is an amide linker having the following structure: -C(=O)NH- or -NHC(=O)-;

X is N or CH;

Y is N or $CR_{2a}$;

Z is N or $CR_{3a}$;
with the proviso that when at least one of X or Z is N, Y cannot be N;

$R_{1a}$ is $R_{1aa}$ or $R_{1ba}$;
wherein:

$R_{1aa}$ is $NR_{32a}R_{33a}$;

$R_{1ba}$ is $C_{1-5}$alkyl, $C_{0-2}$alkylene$C_{3-5}$cycloalkyl which cycloalkyl is optionally substituted by $CH_3$, or $CF_3$;

$R_{2a}$ is H, halo, $C_{1-2}$alkyl, $OC_{1-2}$alkyl, $C_{1-2}$haloalkyl or $OC_{1-2}$haloalkyl;

$R_{3a}$ is H, halo, $CH_3$, $OCH_3$, $CF_3$ or $OCF_3$;
wherein at least one of $R_{2a}$ and $R_{3a}$ is H;

$R_{4a}$ and $R_{5a}$ are $R_{4aa}$ and $R_{5aa}$, or $R_{4ba}$ and $R_{5ba}$;
wherein:

$R_{4aa}$ and $R_{5aa}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21a}R_{22a}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or

$R_{4aa}$ and $R_{5aa}$ together with the carbon atom to which they are attached form a $C_{3-6}$heterocycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heterocycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or

$R_{4aa}$ and $R_{5aa}$ together with the carbon atom to which they are attached form a $C_{3-6}$heterocycloalkyl comprising one nitrogen atom, wherein said nitrogen atom is substituted by $-S(O)_2R_{29a}$; or

$R_{4ba}$ and $R_{5ba}$ are each independently H, $C_{1-6}$alkyl, $C_{1-6}$alkylOH, $C_{1-6}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, or $R_{4ba}$ and $R_{5ba}$ together with the carbon atom to which they are attached form a $C_{3-6}$cycloalkyl or $C_{3-6}$heterocycloalkyl; and

when $A_a$ is -NHC(=O)- or $-NHCH_2-$:
$R_{4ba}$ and $R_{5ba}$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl and $NR_{21a}R_{22a}$;

Ar1a is a 6-membered aryl or heteroaryl;

Ar2a is a 6-membered aryl or heteroaryl and is attached to Ar1a in the para position relative to group $A_a$;

$R_{10a}$ is H, halo, $C_{1-3}$alkyl, $C_{1-2}$haloalkyl, $OC_{1-2}$alkyl, $OC_{1-2}$haloalkyl or CN;

$R_{11a}$ is H, F, Cl, $C_{1-2}$alkyl, $CF_3$, $OCH_3$ or CN;

$R_{12a}$ is attached to Ar2a in the ortho or meta position relative to Ar1a and $R_{12a}$ is H, halo, $C_{1-4}$alkyl, $C_{2-4}$alkenyl, $C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, $NHC(O)C_{1-3}$alkyl or $NR_{23a}R_{24a}$; and
when $A_a$ is -NHC(=O)-, -NH- or $-NHCH_2-$:
$R_{12a}$ may additionally be selected from CN, $OCH_2CH_2N(CH_3)_2$ and a $C_{3-6}$heterocycloalkyl comprising one nitrogen located at the point of attachment to Ar2a, or $R_{12a}$ together with a nitrogen atom to which it is attached forms an N-oxide ($N^+-O^-$);

$R_{13a}$ is H or halo;

$R_{21a}$ is H, $C_{1-5}$alkyl, $C(O)C_{1-5}$alkyl, $C(O)OC_{1-5}$alkyl;

$R_{22a}$ is H or $CH_3$;

$R_{23a}$ is H or $C_{1-2}$alkyl; and

$R_{24a}$ is H or $C_{1-2}$alkyl;

$R_{29a}$ is $C_{1-3}$alkyl, $C_{0-2}$alkylene$C_{3-5}$cycloalkyl which cycloalkyl is optionally substituted by $CH_3$, or $CF_3$;

$R_{32a}$ is $C_{1-3}$alkyl and $R_{33a}$ is $C_{1-3}$alkyl; or

$R_{32a}$ and $R_{33a}$ together with the nitrogen atom to which they are attached form a $C_{3-5}$heterocycloalkyl;

or a salt and/or solvate thereof .

Suitably, $R_{1c}$ is $R_{1ac}$; and/or $R_{4c}$ and $R_{5c}$ are $R_{4ac}$ and $R_{5ac}$; and/or $A_c$ is $A_{ac}$.

The phrase '$A_{ba}$ is an amide linker having the following structure: -C(=O)NH- or -NHC(=O)-' means the following structures form:

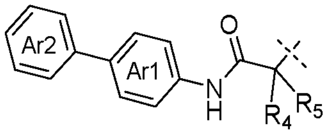

and

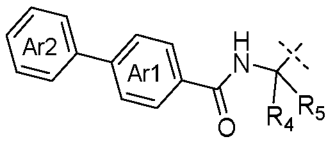

-NH(C=O)- -(C=O)NH-

Reference to $R_4$ and $R_5$ above includes variables $R_{4a}$, $R_{5a}$, $R_{4aa}$, $R_{5aa}$, $R_{4ba}$ and $R_{5ba}$, and reference to Ar1 and Ar2 includes variables Ar1a and Ar2a.

The phrase '$A_{aa}$ is an amine linker having the following structure: $-CH_2NH-$ or $-NHCH_2-$' means the following structures form:

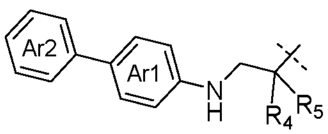

and

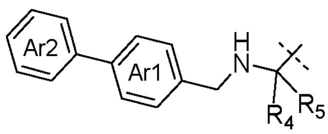

$-NHCH_2-$ $-CH_2NH-$

Reference to $R_4$ and $R_5$ above includes variables $R_{4a}$, $R_{5a}$, $R_{4aa}$, $R_{5aa}$, $R_{4ba}$ and $R_{5ba}$, and reference to Ar1 and Ar2 includes variables Ar1a and Ar2a.

In one embodiment, $A_{ba}$ is -C(=O)NH-. In another embodiment, $A_{ba}$ is -NHC(=O)-. In an additional embodiment, $A_{aa}$ is -NH-. In a further embodiment, $A_{aa}$ is $-CH_2NH-$. In another embodiment, $A_{aa}$ is $-NHCH_2-$.

In one embodiment X is N. In another embodiment, X is CH.

In one embodiment, Y is N. In another embodiment, Y is $CR_{2a}$.

In one embodiment, Z is N. In another embodiment, Z is $CR_{3a}$.

Suitably, X is N, Y is $CR_{2a}$ and Z is $CR_{3a}$. Alternatively, X is CH, Y is N and Z is $CR_{3a}$. Alternatively, X is CH, Y is $CR_{2a}$ and Z is $CR_{3a}$. Alternatively, X is CH, Y is $CR_{22}$ and Z is N. Alternatively, X is N, Y is $CR_{2a}$ and Z is N.

In one embodiment of the invention, $R_{1a}$ is $R_{1aa}$, i.e. is $NR_{32a}R_{33a}$. In an embodiment $R_{32a}$ is $C_{1-3}$alkyl, such as methyl or ethyl, e.g. methyl. In an embodiment, $R_{33a}$ is $C_{1-3}$alkyl, such as methyl or ethyl, e.g. methyl. Suitably, $R_{32a}$ and $R_{33a}$ are both methyl. Suitably, $R_{32a}$ and $R_{33a}$ are both ethyl. Suitably, $R_{32a}$ is methyl and $R_{33a}$ is ethyl.

In another embodiment, $R_{32a}$ and $R_{33a}$ together with the nitrogen atom to which they are attached form a $C_{3-5}$heterocycloalkyl. Suitably, the $C_{3-5}$heterocycloalkyl is aziridinyl, azetidinyl or pyrrolidinyl.

Suitably, $R_{1a}$ is $R_{1ba}$.

In one embodiment of the invention $R_{1ba}$ is $C_{1-5}$alkyl. When $R_{1ba}$ is $C_{1-5}$alkyl, $R_{1ba}$ may be methyl, ethyl, propyl (n-propyl or isopropyl), butyl (n-butyl, isobutyl, sec-butyl or *tert*-butyl) or pentyl (e.g. n-pentyl, sec-pentyl or 3-pentyl).

In a second embodiment of the invention $R_{1ba}$ is $C_{0-2}$alkylene$C_{3-5}$cycloalkyl which cycloalkyl is optionally substituted by $CH_3$. In some embodiments, $R_{1ba}$ is $C_{0-2}$alkylene$C_{3-5}$cycloalkyl. In other embodiments, $R_{1ba}$ is $C_{0-2}$alkylene$C_{3-5}$cycloalkyl which cycloalkyl is substituted by $CH_3$. $R_{1ba}$ may be $C_{3-5}$cycloalkyl, which cycloalkyl is optionally

substituted by $CH_3$. $R_{1ba}$ may be $C_1$alkylene$C_{3-5}$cycloalkyl, which cycloalkyl is optionally substituted by $CH_3$. $R_{1ba}$ may be $C_2$alkylene$C_{3-5}$cycloalkyl, which cycloalkyl is optionally substituted by $CH_3$. $R_{1ba}$ may be $C_{0-2}$alkylene$C_3$cycloalkyl, which cycloalkyl is optionally substituted by $CH_3$. $R_{1ba}$ may be $C_{0-2}$alkylene$C_4$cycloalkyl, which cycloalkyl is optionally substituted by $CH_3$. $R_{1ba}$ may be $C_{0-2}$alkylene$C_5$cycloalkyl, which cycloalkyl is optionally substituted by $CH_3$. Suitably, where $C_{0-2}$alkylene$C_{3-5}$cycloalkyl is optionally substituted by $CH_3$, the $CH_3$ is at the point of attachment of the $C_{3-5}$cycloalkyl to the $C_{0-2}$alkylene.

In a third embodiment, $R_{1ba}$ is $CF_3$.

Suitably $R_{1ba}$ is cyclopropyl, cyclopropyl substituted by $CH_3$ at the point of attachment, cyclobutyl, $CH_3$ or $CH_2CH_3$. In particular $R_{1ba}$ is cyclopropyl, cyclobutyl, $CH_3$ or $CH_2CH_3$, especially cyclopropyl.

In one embodiment, $R_{2a}$ is H. In a second embodiment, $R_{2a}$ is halo such as F, Cl or Br, e.g. Cl or Br. In a third embodiment, $R_{2a}$ is $C_{1-2}$alkyl. When $R_{2a}$ is $C_{1-2}$alkyl, $R_{2a}$ may be methyl or ethyl, such as methyl. In a fourth embodiment, $R_{2a}$ is $OC_{1-2}$alkyl. When $R_{2a}$ is $OC_{1-2}$alkyl, may be $OCH_3$ or OEt, such as $OCH_3$. In a fifth embodiment, $R_{2a}$ is $C_{1-2}$haloalkyl. When $R_{2a}$ is $C_{1-2}$haloalkyl, $R_{2a}$ may be $CF_3$ or $CH_2CF_3$, such as $CF_3$. In a sixth embodiment, $R_{2a}$ is $OC_{1-2}$haloalkyl. When $R_{2a}$ is $OC_{1-2}$haloalkyl, $R_{2a}$ may be $OCF_3$ or $OCH_2CF_3$, such as $OCF_3$.

Suitably, $R_{2a}$ is H, $CH_3$ or $CF_3$, such as H or $CH_3$, in particular H.

In one embodiment $R_{3a}$ is H. In a second embodiment $R_{3a}$ is halo, in particular chloro or fluoro, especially fluoro. In a third embodiment, $R_{3a}$ is $CH_3$. In a fourth embodiment, $R_{3a}$ is $OCH_3$. In a fifth embodiment, $R_{3a}$ is $CF_3$. In a sixth embodiment, $R_{3a}$ is $OCF_3$.

Suitably, $R_{3a}$ is H, halo in particular chloro or fluoro, especially fluoro, $CH_3$ or $CF_3$. More suitably, $R_{3a}$ is H or F, such as H.

Suitably, at least one of $R_{2a}$ and $R_{3a}$ is H.

In one embodiment, $R_{4a}$ and $R_{5a}$ are $R_{4aa}$ and $R_{5aa}$.

Suitably, $R_{4aa}$ and $R_{5aa}$ together with the carbon atom to which they are attached form a $C_{3-6}$cycloalkyl which is substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21a}R_{22a}$.

In one embodiment, the $C_{3-6}$cycloalkyl is cyclopropyl. In another embodiment, the $C_{3-6}$cycloalkyl is cyclobutyl. In another embodiment, the $C_{3-6}$cycloalkyl is cyclopentyl. In another embodiment, the $C_{3-6}$cycloalkyl is cyclohexyl.

In one embodiment the $C_{3-6}$cycloalkyl is substituted by one substituent. In a second embodiment the $C_{3-6}$cycloalkyl is substituted by two substituents.

In one embodiment, the substituent is $C_{1-3}$alkyl. Suitably, the substituent is methyl. Suitably, the substituent is ethyl. Suitably, the substituent is n-propyl. Suitably, the substituent is iso-propyl.

In a second embodiment, the substituent is $C_{1-3}$alkylOH. Suitably, the substituent is $CH_2OH$. Suitably, the substituent is $CH_2CH_2OH$. Suitably, the substituent is $CH_2CH_2CH_2OH$.

In a third embodiment, the substituent is $C_{1-3}$haloalkyl. Suitably the $C_{1-3}$alkyl group is substituted by one, two or three, such as one, halogen atom. Suitably, the halogen atom is fluoro or chloro such as fluoro. Suitably, the substituent is $C_1$haloalkyl such as $CF_3$. Suitably, the substituent is $C_2$haloalkyl such as $CH_2CF_3$.

In a fourth embodiment, the substituent is $C_{0-2}$alkylene$C_{3-6}$cycloalkyl, in particular $C_{0-2}$alkylene$C_{3-5}$cycloalkyl, such as $C_{3-5}$cycloalkyl, $C_1$alkylene$C_{3-5}$cycloalkyl or $C_2$alkylene$C_{3-5}$cycloalkyl.

In a fifth embodiment, the substituent is $C_{0-2}$alkylene$C_{3-6}$heterocycloalkyl such as $C_{0-2}$alkylene$C_3$heterocycloalkyl, $C_{0-2}$alkylene$C_4$heterocycloalkyl, $C_{0-2}$alkylene$C_5$heterocycloalkyl, $C_{0-2}$alkylene$C_6$heterocycloalkyl, $C_0$alkylene-$C_{3-6}$heterocycloalkyl, $C_1$alkylene$C_{3-6}$heterocycloalkyl and $C_2$alkylene$C_{3-6}$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) in the $C_{3-6}$heterocycloalkyl ring may be substituted (such as one nitrogen atom is substituted), for example by $C_{1-4}$alkyl, C(O)H, $C(O)C_{1-4}$alkyl, $C(O)OC_{1-4}$alkyl, $C(O)OC_{1-4}$alkylaryl such as C(O)OBz, $C(O)NHC_{1-4}$alkyl, $C(O)NHC_{1-4}$alkylaryl such as C(O)NHBz, an Fmoc group, $C(O)C_{1-4}$haloalkyl, $C(O)OC_{1-4}$haloalkyl or $C(O)NHC_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, $C(O)C_{1-3}$alkyl$OC_{1-2}$alkyl such as $C(O)CH_2OCH_3$, and $C_{1-2}$alkylC(O)$OC_{1-4}$alkyl such as $CH_2C(O)OCH_2CH_3$. Suitably, any nitrogen atom in the $C_{3-6}$heterocycloalkyl ring is not substituted.

In a sixth embodiment, the substituent is $C_{1-3}$alkylene$OC_{1-3}$alkyl, in particular $C_{1-2}$alkylene$OC_{1-2}$alkyl such as $C_1$alkylene$OC_1$alkyl, $C_2$alkylene$OC_1$alkyl, $C_1$alkylene$OC_2$alkyl or $C_2$alkylene$OC_2$alkyl.

In a seventh embodiment, the substituent is halo, in particular fluoro or chloro such as chloro.

In an eighth embodiment, the substituent is $OC_{1-3}$haloalkyl. Suitably the $OC_{1-3}$alkyl group is substituted by one two or three, such as one, halogen atom. Suitably, the halogen atom is fluoro or chloro such as fluoro. Suitably, the substituent is $OC_1$haloalkyl such as $OCF_3$. Suitably, the substituent is $OC_2$haloalkyl such as $OCH_2CF_3$.

In a ninth embodiment, the substituent is $OC_{0-2}$alkylene$C_{3-6}$cycloalkyl, such as $OC_{3-6}$cycloalkyl, $OC_1$alkyle-

ne$C_{3-6}$cycloalkyl or $OC_2$alkylene$C_{3-6}$cycloalkyl.

In a tenth embodiment, the substituent is $OC_{0-2}$alkylene$C_{3-6}$heterocycloalkyl such as $OC_{0-2}$alkylene$C_3$heterocycloalkyl, $OC_{0-2}$alkylene$C_4$heterocycloalkyl, $OC_{0-2}$alkylene$C_5$heterocycloalkyl, $OC_{0-2}$alkylene$C_6$heterocycloalkyl, $OC_0$alkylene$C_{3-6}$heterocycloalkyl, $OC_1$alkylene$C_{3-6}$heterocycloalkyl and $OC_2$alkylene$C_{3-6}$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) (such as one nitrogen atom) in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted.

In an eleventh embodiment, the substituent is $OC_{1-3}$alkyl, such as $OCH_3$ or $OCH_2CH_3$.

In a twelfth embodiment, the substituent is $NR_{21a}R_{22a}$ wherein $R_{21a}$ and $R_{22a}$ are defined elsewhere herein.

In an embodiment the substituent is oxo.

In another embodiment the substituent is OH.

Suitably, the one or two substituents, in particular one substituent, are independently selected from the group consisting of $C_{1-3}$alkyl, oxo, OH, $C_{1-3}$alkylOH, $C_{1-3}$haloalkyl, halo, $OC_{1-3}$haloalkyl, $OC_{1-3}$alkyl and $NR_{21a}R_{22a}$.

More suitably, the substituent is independently selected from the group consisting of oxo, OH, halo, $OC_{1-3}$alkyl and $NR_{21a}R_{22a}$.

Most suitably, the substituent is independently selected from the group consisting of oxo, OH, fluoro and $NR_{21a}R_{22a}$.

When the substituent is $NR_{21a}R_{22a}$, in one embodiment $R_{21a}$ is H. In a second embodiment $R_{21a}$ is $C_{1-5}$alkyl, such as methyl, ethyl or propyl, especially methyl. In a third embodiment $R_{21a}$ is C(O)$C_{1-5}$alkyl, such as C(O)$CH_3$. In a fourth embodiment $R_{21a}$ is C(O)O$C_{1-5}$alkyl, such as C(O)$OCH_3$ or C(O)Otert-butyl. In a fifth embodiment $R_{21a}$ is $C_{1-3}$alkylO$C_{1-2}$alkyl such as $C_1$alkylO$C_1$alkyl, $C_2$alkylO$C_1$alkyl or $C_3$alkylO$C_1$alkyl e.g. $C_2$alkylO$C_1$alkyl. In a sixth embodiment, $R_{21a}$ is $C_{1-4}$haloalkyl, such as $CF_3$, $CH_2CF_3$ or $CH_2CHF_2$ e.g. $CH_2CHF_2$. In a seventh embodiment $R_{21a}$ is $C_{4-6}$heterocycloalkyl, such as oxetanyl, tetrahydrofuranyl or tetrahydropyranyl e.g. oxetanyl, in particular 3-oxetanyl.

When the substituent is $NR_{21a}R_{22a}$, in one embodiment $R_{22a}$ is H. In a second embodiment $R_{22a}$ is methyl.

Suitably, $R_{21a}$ is C(O)$OCH_3$ and $R_{22a}$ is H. Suitably, $R_{21a}$ is C(O)$CH_3$ and $R_{22a}$ is H. Suitably, $R_{21a}$ and $R_{22a}$ are both $CH_3$. Suitably, $R_{21a}$ and $R_{22a}$ are both H.

Alternatively, $R_{4aa}$ and $R_{5aa}$ suitably together with the carbon atom to which they are attached form a $C_{3-6}$cycloalkyl and one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl.

In one embodiment the $C_{3-6}$cycloalkyl formed by $R_{4aa}$ and $R_{5aa}$ together with the carbon atom to which they are attached is unsubstituted. In a second embodiment the $C_{3-6}$cycloalkyl formed by $R_{4aa}$ and $R_{5aa}$ together with the carbon atom to which they are attached is substituted by one or two substituents, in particular one substituent. Suitably, each substituent being independently selected from the group consisting of $C_{1-2}$alkyl or $OCH_3$.

Suitably one of the carbons of the $C_{3-6}$cycloalkyl formed by $R_{4aa}$ and $R_{5aa}$ together with the carbon atom to which they are attached is a $C_{4-6}$cycloalkyl. Suitably the further $C_{3-6}$heterocycloalkyl is an oxygen containing $C_{3-6}$heterocycloalkyl.

Alternatively, $R_{4aa}$ and $R_{5aa}$ suitably together with the carbon atom to which they are attached form a $C_{3-6}$heterocycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heterocycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl.

In one embodiment the $C_{3-6}$heterocycloalkyl formed by $R_{4aa}$ and $R_{5aa}$ together with the carbon atom to which they are attached is unsubstituted. In a second embodiment the $C_{3-6}$heterocycloalkyl formed by $R_{4aa}$ and $R_{5aa}$ together with the carbon atom to which they are attached is substituted by one or two substituents, in particular one substituent. Suitably, each substituent being independently selected from the group consisting of $C_{1-2}$alkyl or $OCH_3$.

In an embodiment, $R_{4aa}$ and $R_{5aa}$ together with the carbon atom to which they are attached form a $C_{3-6}$heterocycloalkyl comprising one nitrogen atom, wherein said nitrogen atom is substituted by $-S(O)_2R_{29a}$.

Suitably, the $C_{3-6}$heterocycloalkyl is selected from the group consisting of aziridinyl, azetidinyl, pyrrolidinyl and

piperidinyl such as piperidinyl.

Suitably, when the $C_{3-6}$heterocycloalkyl is piperidinyl, the nitrogen atom is in the 4-position relative to the quaternary carbon:

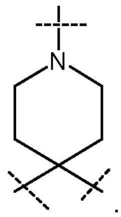

The $C_{3-6}$heterocycloalkyl may be other groups as defined elsewhere herein.

In an embodiment, $R_{29a}$ is $C_{1-3}$alkyl, $C_{0-2}$alkylene$C_{3-5}$cycloalkyl which cycloalkyl is optionally substituted by $CH_3$, or $CF_3$. In one embodiment, $R_{29a}$ is $C_{1-3}$alkyl such as methyl. In another embodiment, $R_{29a}$ is $C_{0-2}$alkylene$C_{3-5}$cycloalkyl which cycloalkyl is optionally substituted by $CH_3$. In some embodiments, $R_{29a}$ is $C_{0-2}$alkylene$C_{3-5}$cycloalkyl. In other embodiments, $R_{29a}$ is $C_{0-2}$alkylene$C_{3-5}$cycloalkyl which cycloalkyl is substituted by $CH_3$. $R_{29a}$ may be $C_{3-5}$cycloalkyl, which cycloalkyl is optionally substituted by $CH_3$. $R_{29a}$ may be $C_1$alkylene$C_{3-5}$cycloalkyl, which cycloalkyl is optionally substituted by $CH_3$. $R_{29a}$ may be $C_2$alkylene$C_{3-5}$cycloalkyl, which cycloalkyl is optionally substituted by $CH_3$. $R_{29a}$ may be $C_{0-2}$alkylene$C_3$cycloalkyl, which cycloalkyl is optionally substituted by $CH_3$. $R_{29a}$ may be $C_{0-2}$alkylene$C_4$cycloalkyl, which cycloalkyl is optionally substituted by $CH_3$. $R_{29a}$ may be $C_{0-2}$alkylene$C_5$cycloalkyl, which cycloalkyl is optionally substituted by $CH_3$. Suitably, where $C_{0-2}$alkylene$C_{3-5}$cycloalkyl is optionally substituted by $CH_3$, the $CH_3$ is at the point of attachment of the $C_{3-5}$cycloalkyl to the $C_{0-2}$alkylene. In another embodiment, $R_{29a}$ is $CF_3$. In another embodiment, $R_{29a}$ is $N(C_{1-3}alkyl)_2$ such as $N(CH_3)_2$. In another embodiment, $R_{29a}$ is a 5 or 6 membered heteroaryl wherein the 5 or 6 membered heteroaryl is optionally substituted by methyl. In one embodiment, the 5 or 6 membered heteroaryl is substituted by methyl. In one embodiment, the 5 or 6 membered heteroaryl is not substituted by methyl. In one embodiment, $R_{29a}$ is a 5-membered heteroaryl such as pyrrolyl, furanyl, thiophenyl, imidazolyl, pyrazolyl, oxazolyl, isoxazolyl, thiazolyl, isothiazolyl, such as pyrazolyl. Suitably the pyrazolyl is substituted by methyl. In another embodiment, $R_{29a}$ is a 6-membered heteroaryl such as pyridinyl, pyridazinyl, pyrimidinyl or pyrazinyl.

In another embodiment, $R_{4a}$ and $R_{5a}$ are $R_{4ba}$ and $R_{5ba}$.

In one embodiment, $R_{4ba}$ and $R_{5ba}$ together with the carbon atom to which they are attached form a $C_{3-6}$cycloalkyl, such as cyclopropyl, cyclobutyl or cyclopentyl in particular cyclopropyl or cyclopentyl. In a second embodiment, $R_{4ba}$ and $R_{5ba}$ together with the carbon atom to which they are attached form a $C_{3-6}$heterocycloalkyl, such as a heterocyclohexyl, in particular a tetrahydropyranyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted. In a third embodiment, $R_{4ba}$ is $C_{1-6}$alkyl, in particular $C_{1-4}$alkyl such as methyl, ethyl, propyl (n-propyl or isopropyl) or butyl (n-butyl, isobutyl, sec-butyl or *tert*-butyl). In a fourth embodiment, $R_{4ba}$ is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a fifth embodiment, $R_{4ba}$ is H. In a sixth embodiment, $R_{4ba}$ is halo, such as chloro or fluoro, especially fluoro. In a seventh embodiment, $R_{4ba}$ is $C_{1-6}$haloalkyl, such as $CF_3$ or $CH_2CF_3$. In an eighth embodiment, $R_{4ba}$ is $C_{0-2}$alkylene$C_{3-6}$cycloalkyl such as $C_{3-6}$cycloalkyl, $C_1$alkylene$C_{3-6}$cycloalkyl, $C_2$alkylene$C_{3-6}$cycloal kyl, $C_{0-2}$alkylene$C_3$cycloalkyl, $C_{0-2}$alkylene$C_4$cycloalkyl, $C_{0-2}$alkylene$C_5$cycloalkyl or $C_{0-2}$alkylene$C_6$cycloalkyl. In a ninth embodiment, $R_{4ba}$ is $C_{0-2}$alkylene$C_{3-6}$heterocycloalkyl such as $C_{3-6}$heterocycloalkyl, $C_1$alkylene$C_{3-6}$heterocycloalkyl, $C_2$alkylene$C_{3-6}$heterocycloalkyl, $C_{0-2}$alkylene$C_3$heterocycloalkyl, $C_{0-2}$alkylene$C_4$hetero-cycloalkyl, $C_{0-2}$alkylene$C_5$heterocycloalkyl or $C_{0-2}$alkylene$C_6$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted. In a tenth embodiment, $R_{4ba}$ is $C_{1-6}$alkylOH, such as $CH_2OH$ or $CH_2CH_2OH$. In an eleventh embodiment, $R_{4ba}$ is O$C_{1-6}$haloalkyl, such as O$C_{1-4}$haloalkyl, such as $OCF_3$ or $OCHF_2$. In a twelfth embodiment,

$R_{4ba}$ is $OC_{0-2}$alkylene$C_{3-6}$cycloalkyl such as $OC_{3-6}$cycloalkyl, $OC_1$alkylene$C_{3-6}$cycloalkyl, $OC_2$alkylene$C_{3-6}$cycloalkyl, $OC_{0-2}$alkylene$C_3$cycloalkyl, $OC_{0-2}$alkylene$C_4$cycloalkyl, $OC_{0-2}$alkylene$C_5$cycloalkyl or $OC_{0-2}$alkylene$C_6$cycloalkyl. In a thirteenth embodiment, $R_{4ba}$ is $OC_{1-6}$alkyl, in particular $OC_{1-4}$alkyl such as methoxy, ethoxy, propoxy (n-propoxy or isopropoxy) or butoxy (n-butoxy, isobutoxy, sec-butoxy or tert-butoxy). In a fourteenth embodiment, $R_{4ba}$ is $OC_{0-2}$alkylene$C_{3-6}$heterocycloalkyl such as $OC_{3-6}$heterocycloalkyl, $OC_1$alkylene$C_{3-6}$heterocycloalkyl, $OC_2$alkylene$C_{3-6}$heterocycloalkyl, $OC_{0-2}$alkylene$C_3$heterocycloalkyl, $OC_{0-2}$alkylene$C_4$hetero-cycloalkyl, $OC_{0-2}$alkylene$C_5$heterocycloalkyl or $OC_{0-2}$alkylene$C_6$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)$OC_{1-4}$alkyl, C(O)$OC_{1-4}$alkylaryl such as C(O)OBz, C(O)$NHC_{1-4}$alkyl, C(O)$NHC_{1-4}$alkylaryl such as C(O)NHBz, an Fmoc group, C(O)$C_{1-4}$haloalkyl, C(O)$OC_{1-4}$haloalkyl or C(O)$NHC_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkyl$OC_{1-2}$alkyl such as C(O)$CH_2OCH_3$, and $C_{1-2}$alkylC(O)$OC_{1-4}$alkyl such as $CH_2C(O)OCH_2CH_3$. Suitably, any nitrogen atom in the $C_{3-6}$heterocycloalkyl ring is not substituted. In a fifteenth embodiment, $R_{4ba}$ is $NR_{21a}R_{22a}$.

When $A_a$ is -NHC(=O)- or -C(=O)NH-, suitably, $R_{4ba}$ is H, $C_{1-6}$alkyl, $C_{1-6}$haloalkyl, $C_{1-6}$alkylOH, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, or $R_{4ba}$ and $R_{5ba}$ together with the carbon atom to which they are attached form a $C_{3-6}$cycloalkyl or $C_{3-6}$heterocycloalkyl. When $A_a$ is -NHC(=O)-, suitably $R_{4ba}$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl or $NR_{21a}R_{22a}$.

When $A_a$ is -NH-, -$CH_2$NH- or -NH$CH_2$-, suitably, $R_{4ba}$ is H, $C_{1-6}$alkyl, $C_{1-6}$haloalkyl, $C_{1-6}$alkylOH, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, or $R_{4ba}$ and $R_{5ba}$ together with the carbon atom to which they are attached form a $C_{3-6}$cycloalkyl or $C_{3-6}$heterocycloalkyl. When $A_a$ is -NH$CH_2$-, suitably $R_{4ba}$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl or $NR_{21a}R_{22a}$.

Suitably $R_{4ba}$ is H, fluoro, $CH_3$, ethyl, $OCH_3$ or $CH_2CH_2OCH_3$, such as fluoro, ethyl, $OCH_3$ or $CH_2CH_2OCH_3$.

Suitably $R_{4ba}$ is H, $CH_3$, ethyl or $CH_2CH_2OCH_3$, in particular $CH_3$ or ethyl.

Suitably $R_{4ba}$ and $R_{5ba}$ together with the carbon atom to which they are attached form a cyclopropyl or cyclopentyl, in particular a cyclopentyl.

Suitably $R_{4ba}$ and $R_{5ba}$ together with the carbon atom to which they are attached form a heterocyclohexyl, such as tetrahydropyranyl or piperidinyl, especially tetrahydropyranyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)$OC_{1-4}$alkyl, C(O)$OC_{1-4}$alkylaryl such as C(O)OBz, C(O)$NHC_{1-4}$alkyl, C(O)$NHC_{1-4}$alkylaryl such as C(O)NHBz, an Fmoc group, C(O)$C_{1-4}$haloalkyl, C(O)$OC_{1-4}$haloalkyl or C(O)$NHC_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkyl$OC_{1-2}$alkyl such as C(O)$CH_2OCH_3$, and $C_{1-2}$alkylC(O)$OC_{1-4}$alkyl such as $CH_2C(O)OCH_2CH_3$. Suitably, any nitrogen atom in the $C_{3-6}$heterocycloalkyl ring is not substituted.

Suitably $R_{4ba}$ and $R_{5ba}$ together with the carbon atom to which they are attached form a heterocyclobutyl, such as azetidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)$OC_{1-4}$alkyl, C(O)$OC_{1-4}$alkylaryl such as C(O)OBz, C(O)$NHC_{1-4}$alkyl, C(O)$NHC_{1-4}$alkylaryl such as C(O)NHBz, an Fmoc group, C(O)$C_{1-4}$haloalkyl, C(O)$OC_{1-4}$haloalkyl or C(O)$NHC_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkyl$OC_{1-2}$alkyl such as C(O)$CH_2OCH_3$, and $C_{1-2}$alkylC(O)$OC_{1-4}$alkyl such as $CH_2C(O)OCH_2CH_3$. Suitably, any nitrogen atom in the $C_{3-6}$heterocycloalkyl ring is not substituted.

When $R_{4ba}$ is $NR_{21a}R_{22a}$, in one embodiment $R_{21a}$ is H. In a second embodiment $R_{21a}$ is $C_{1-5}$alkyl, such as methyl, ethyl or propyl, especially methyl. In a third embodiment $R_{21a}$ is C(O)$C_{1-5}$alkyl, such as C(O)$CH_3$. In a fourth embodiment $R_{21a}$ is C(O)$OC_{1-5}$alkyl, such as C(O)$OCH_3$ or C(O)O*tert*-butyl. In a fifth embodiment $R_{21a}$ is $C_{1-3}$alkylO-$C_{1-2}$alkyl such as $C_1$alkyl$OC_1$alkyl, $C_2$alkyl$OC_1$alkyl or $C_3$alkyl$OC_1$alkyl e.g. $C_2$alkyl$OC_1$alkyl. In a sixth embodiment, $R_{21a}$ is $C_{1-4}$haloalkyl, such as $CF_3$, $CH_2CF_3$ or $CH_2CHF_2$ e.g. $CH_2CHF_2$. In a seventh embodiment $R_{21a}$ is $C_{4-6}$heterocycloalkyl, such as oxetanyl, tetrahydrofuranyl or tetrahydropyranyl e.g. oxetanyl, in particular 3-oxetanyl.

When $R_{4ba}$ is $NR_{21a}R_{22a}$, in one embodiment $R_{22a}$ is H. In a second embodiment $R_{22a}$ is methyl.

For example, $R_{4ba}$ is $NH_2$, $N(CH_3)_2$, NHC(O)$CH_3$, NHC(O)$OCH_3$, NHC(O)Otert-butyl and $CH_2CH_2OH$, especially, $N(CH_3)_2$, NHC(O)$CH_3$, NHC(O)$OCH_3$.

Suitably, $R_{21a}$ is C(O)$OCH_3$ and $R_{22a}$ is H. Suitably, $R_{21a}$ is C(O)$CH_3$ and $R_{22a}$ is H. Suitably, $R_{21a}$ and $R_{22a}$ are both $CH_3$. Suitably, $R_{21a}$ and $R_{22a}$ are both H.

In one embodiment $R_{5ba}$ is $C_{1-6}$alkyl, in particular $C_{1-4}$alkyl such as methyl, ethyl, propyl (n-propyl or isopropyl) or butyl (n-butyl, isobutyl, sec-butyl or *tert*-butyl). In a second embodiment $R_{5b}$, is $C_{1-3}$alkylene$OC_{1-3}$alkyl, in particular $C_{1-2}$alkylene$OC_{1-2}$alkyl such as $C_1$alkylene$OC_1$alkyl, $C_2$alkylene$OC_1$alkyl, $C_1$alkylene$OC_2$alkyl or $C_2$alkylene$OC_2$alkyl.

In a third embodiment $R_{5ba}$ is H. In a fourth embodiment, $R_{5ba}$ is halo, such as chloro or fluoro, especially fluoro. In a fifth embodiment, $R_{5ba}$ is $C_{1-6}$haloalkyl, such as $CF_3$ or $CH_2CF_3$. In a sixth embodiment, $R_{5ba}$ is $C_{0-2}$alkylene$C_{3-6}$cycloalkyl such as $C_{3-6}$cycloalkyl, $C_1$alkylene$C_{3-6}$cycloalkyl, $C_2$alkylene$C_{3-6}$cycloalkyl, $C_{0-2}$alkylene$C_3$cycloalkyl, $C_{0-2}$alkylene$C_4$cycloalkyl, $C_{0-2}$alkylene$C_5$cycloalkyl or $C_{0-2}$alkylene$C_6$cycloalkyl. In a seventh embodiment, $R_{5ba}$ is $C_{0-2}$alkylene-$C_{3-6}$heterocycloalkyl such as $C_{3-6}$heterocycloalkyl, $C_1$alkylene$C_{3-6}$heterocycloalkyl, $C_2$alkylene$C_{3-6}$heterocycloalkyl, $C_{0-2}$alkylene$C_3$heterocycloalkyl, $C_{0-2}$alkylene$C_4$hetero-cycloalkyl, $C_{0-2}$alkylene$C_5$heterocycloalkyl or $C_{0-2}$alkylene$C_6$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted. In an eighth embodiment, $R_{5ba}$ is $C_{1-6}$alkylOH, such as $CH_2OH$ or $CH_2CH_2OH$. In a ninth embodiment, $R_{5ba}$ is O$C_{1-6}$haloalkyl, such as O$C_{1-4}$haloalkyl, such as $OCF_3$ or $OCHF_2$. In a tenth embodiment, $R_{5ba}$ is O$C_{0-2}$alkylene$C_{3-6}$cycloalkyl such as O$C_{3-6}$cycloalkyl, O$C_1$alkylene$C_{3-6}$cycloalkyl, O$C_2$alkylene$C_{3-6}$cycloalkyl, O$C_{0-2}$alkylene$C_3$cycloalkyl, O$C_{0-2}$alkylene$C_4$cycloalkyl, O$C_{0-2}$alkylene$C_5$cycloalkyl or O$C_{0-2}$alkylene$C_6$cycloalkyl. In an eleventh embodiment, $R_{5ba}$ is O$C_{1-6}$alkyl, in particular O$C_{1-4}$alkyl such as methoxy, ethoxy, propoxy (n-propoxy or isopropoxy) or butoxy (n-butoxy, isobutoxy, sec-butoxy or tert-butoxy). In a twelfth embodiment, $R_{5ba}$ is O$C_{0-2}$alkylene$C_{3-6}$heterocycloalkyl such as O$C_{3-6}$heterocycloalkyl, O$C_1$alkylene$C_{3-6}$heterocycloalkyl, O$C_2$alkylene$C_{3-6}$heterocycloalkyl, O$C_{0-2}$alkylene$C_3$heterocycloalkyl, O$C_{0-2}$alkylene$C_4$hetero-cycloalkyl, O$C_{0-2}$alkylene$C_5$heterocycloalkyl or O$C_{0-2}$alkylene$C_6$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted. In a thirteenth embodiment, $R_{5ba}$ is $NR_{21a}R_{22a}$.

When $A_a$ is -NHC(=O)- or -C(=O)NH-, suitably, $R_{5ba}$ is H, $C_{1-6}$alkyl, $C_{1-6}$haloalkyl, $C_{1-6}$alkylOH, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, or $R_{4ba}$ and $R_{5ba}$ together with the carbon atom to which they are attached form a $C_{3-6}$cycloalkyl or $C_{3-6}$heterocycloalkyl. When $A_a$ is -NHC(=O)-, suitably $R_{5ba}$ may additionally be selected from halo, O$C_{1-6}$haloalkyl, O$C_{0-2}$alkylene$C_{3-6}$cycloalkyl, O$C_{0-2}$alkylene$C_{3-6}$heterocycloalkyl, O$C_{1-6}$alkyl or $NR_{21a}R_{22a}$.

When $A_a$ is -NH-, -$CH_2$NH- or -NH$CH_2$-, suitably, $R_{5ba}$ is H, $C_{1-6}$alkyl, $C_{1-6}$haloalkyl, $C_{1-6}$alkylOH, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, or $R_{4ba}$ and $R_{5ba}$ together with the carbon atom to which they are attached form a $C_{3-6}$cycloalkyl or $C_{3-6}$heterocycloalkyl. When $A_a$ is -NH$CH_2$-, suitably $R_{5ba}$ may additionally be selected from halo, O$C_{1-6}$haloalkyl, O$C_{0-2}$alkylene$C_{3-6}$cycloalkyl, O$C_{0-2}$alkylene$C_{3-6}$heterocycloalkyl, O$C_{1-6}$alkyl or $NR_{21a}R_{22a}$.

When $R_{5ba}$ is $NR_{21a}R_{22a}$, in one embodiment $R_{21a}$ is H. In a second embodiment $R_{21a}$ is $C_{1-5}$alkyl, such as methyl, ethyl or propyl, especially methyl. In a third embodiment $R_{21a}$ is C(O)$C_{1-5}$alkyl, such as C(O)$CH_3$. In a fourth embodiment $R_{21a}$ is C(O)O$C_{1-5}$alkyl, such as C(O)$OCH_3$ or C(O)O*tert*-butyl. In a fifth embodiment $R_{21a}$ is $C_{1-3}$alkylO-$C_{1-2}$alkyl such as $C_1$alkylO$C_1$alkyl, $C_2$alkylO$C_1$alkyl or $C_3$alkylO$C_1$alkyl e.g. $C_2$alkylO$C_1$alkyl. In a sixth embodiment, $R_{21a}$ is $C_{1-4}$haloalkyl, such as $CF_3$, $CH_2CF_3$ or $CH_2CHF_2$ e.g. $CH_2CHF_2$. In a seventh embodiment $R_{21a}$ is $C_{4-6}$heterocycloalkyl, such as oxetanyl, tetrahydrofuranyl or tetrahydropyranyl e.g. oxetanyl, in particular 3-oxetanyl.

When $R_{5ba}$ is $NR_{21a}R_{22a}$, in one embodiment $R_{22a}$ is H. In a second embodiment $R_{22a}$ is methyl.

For example, $R_{5ba}$ is $NH_2$, $N(CH_3)_2$, NHC(O)$CH_3$, NHC(O)$OCH_3$, NHC(O)Otert-butyl and $CH_2CH_2OH$, especially, $N(CH_3)_2$, NHC(O)$CH_3$, NHC(O)$OCH_3$.

Suitably, $R_{21a}$ is C(O)$OCH_3$ and $R_{22a}$ is H. Suitably, $R_{21a}$ is C(O)$CH_3$ and $R_{22a}$ is H. Suitably, $R_{21a}$ and $R_{22a}$ are both $CH_3$. Suitably, $R_{21a}$ and $R_{22a}$ are both H.

Suitably $R_{5ba}$ is H, F, $CH_3$ or ethyl such as H, $CH_3$ or ethyl.

Suitably $R_{4ba}$ is H, $CH_3$, ethyl or $CH_2CH_2OCH_3$ and $R_{5ba}$ is H, $CH_3$ or ethyl, in particular $R_{4ba}$ is $CH_3$, or ethyl and $R_{5ba}$ is H, methyl or ethyl. For example, $R_{4ba}$ and $R_{5ba}$ are H, $R_{4ba}$ and $R_{5ba}$ are methyl, $R_{4ba}$ and $R_{5ba}$ are ethyl or $R_{4ba}$ is $CH_2CH_2OCH_3$ and $R_{5ba}$ is H.

Suitably, $R_{4ba}$ is F and $R_{5ba}$ is ethyl.

Suitably, $R_{4ba}$ is F and $R_{5ba}$ is F.

Suitably, $R_{4ba}$ is ethyl and $R_{5ba}$ is H.

Suitably $R_{4ba}$ and $R_{5ba}$ are arranged in the following configuration:

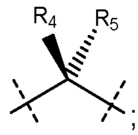

wherein $R_4$ and $R_5$ include variables $R_{4a}$, $R_{5a}$, $R_{4aa}$, $R_{5aa}$, $R_{4ba}$ and $R_{5ba}$.

In one embodiment Ar1a is a 6-membered aryl, i.e. phenyl. In a second embodimentAr1a is a 6-membered heteroaryl, in particular containing one nitrogen atom (pyridyl) or two nitrogen atoms (pyridazinyl, pyrimidinyl or pyrazinyl).

In particular Ar1a is phenyl, 2-pyridyl or 3-pyridyl, such as phenyl or 2-pyridyl.

In one embodiment $R_{10a}$ is H. In a second embodiment $R_{10a}$ is halo, for example fluoro or chloro. In a third embodiment $R_{10a}$ is $C_{1-3}$alkyl such as $C_{1-2}$alkyl, such as $CH_3$ or ethyl. In a fourth embodiment $R_{10a}$ is $OC_{1-2}$alkyl, such as $OCH_3$ or ethoxy. In a fifth embodiment $R_{10a}$ is $OC_{1-2}$haloalkyl, such as $OCF_3$. In a sixth embodiment $R_{10a}$ is CN. In a seventh embodiment, $R_{10a}$ is $C_{1-2}$haloalkyl such as $CF_3$.

Suitably $R_{10a}$ is H, fluoro, chloro, $CH_3$, $CF_3$, $OCH_3$, $OCF_3$ or CN, such as H, fluoro, chloro, $CH_3$, $OCH_3$, $OCF_3$ or CN, in particular H, fluoro, chloro, $OCH_3$, $OCF_3$ or CN especially H or fluoro.

Suitably, $R_{10a}$ is H, F or $CH_3$.

In one embodiment $R_{11a}$ is H. In a second embodiment $R_{11a}$ is F. In a third embodiment, $R_{11a}$ is $C_{1-2}$alkyl such as $CH_3$ or Et, such as $CH_3$. In a fourth embodiment $R_{11a}$ is $OCH_3$. In a fifth embodiment, $R_{11a}$ is Cl. In a sixth embodiment, $R_{11a}$ is Et. In a seventh embodiment, $R_{11a}$ is $CF_3$. In an eighth embodiment, $R_{11a}$ is CN.

Suitably, $R_{11a}$ is H, F, $CH_3$ or $OCH_3$, such as H, For $CH_3$, such as H or F, such as H.

In one embodiment, $R_{10a}$ is in the ortho position with respect to group Aa. In another embodiment, $R_{10a}$ is in the meta position with respect to group Aa. Suitably $R_{10a}$ is in the ortho position with respect to group Aa.

In one embodiment, $R_{11a}$ is in the ortho position with respect to group Aa. In another embodiment, $R_{11a}$ is in the meta position with respect to group Aa. Suitably $R_{11a}$ is in the ortho position with respect to group Aa.

In one embodiment Ar2a is a 6-membered aryl, i.e. phenyl. In a second embodiment Ar2a is a 6-membered heteroaryl, in particular containing one nitrogen atom (pyridyl) or two nitrogen atoms (pyridazinyl, pyrimidinyl or pyrazinyl).

In particular Ar2a is 3-pyridyl or 2,5-pyrazinyl, especially 2,5-pyrazinyl.

In one embodiment $R_{12a}$ is H. In a second embodiment $R_{12a}$ is halo, for example fluoro or chloro. In a third embodiment $R_{12a}$ is $C_{1-4}$alkyl, such as methyl, ethyl, propyl (n-propyl or isopropyl) or butyl (n-butyl, isobutyl, sec-butyl or *tert*-butyl). In a fourth embodiment $R_{12a}$ is $OC_{1-4}$alkyl, such as $OCH_3$, ethoxy, isopropoxy or n-propoxy. In a fifth embodiment $R_{12a}$ is $OC_{0-2}$alkylene$C_{3-5}$cycloalkyl, such as $OC_{3-5}$cycloalkyl (e.g. cyclopropoxy or cyclobutoxy), $OC_1$alkylene$C_{3-5}$cycloalkyl or $OC_2$alkylene$C_{3-5}$cycloalkyl. In a sixth embodiment $R_{12a}$ is CN. In a seventh embodiment $R_{12a}$ is $C_{1-4}$haloalkyl, such as $CF_3$. In an eighth embodiment $R_{12a}$ is $OC_{1-4}$haloalkyl, such as $OCF_3$, $OCHF_2$ or $OCH_2CF_3$. In a ninth embodiment, $R_{12a}$ is $C_{2-4}$alkenyl such as $C(=CH_2)CH_3$. In a tenth embodiment, $R_{12a}$ is $C_{0-2}$alkylene$C_{3-5}$cycloalkyl such as $C_{3-5}$cycloalkyl, $C_1$alkylene$C_{3-5}$cycloalkyl, $C_2$alkylene$C_{3-5}$cycloalkyl, $C_{0-2}$alkylene$C_3$cycloalkyl, $C_{0-2}$alkylene$C_4$cycloalkyl or $C_{0-2}$alkylene$C_5$cycloalkyl. In an eleventh embodiment, $R_{12a}$ is hydroxy. In a twelfth embodiment, $R_{12a}$ is $C_{1-4}$alkylOH such as $CH_2OH$. In a thirteenth embodiment, $R_{12a}$ is $SO_2C_{1-2}$alkyl such as $SO_2CH_3$. In a fourteenth embodiment, $R_{12a}$ is $C(O)N(C_{1-2}alkyl)_2$ such as $C(O)N(CH_3)_2$. In a fifteenth embodiment, $R_{12a}$ is $NHC(O)C_{1-3}$alkyl. In a sixteenth embodiment, $R_{12a}$ is $NR_{23a}R_{24a}$. In a seventeenth embodiment, $R_{12a}$ is $OCH_2CH_2N(CH_3)_2$. In an eighteenth embodiment, $R_{12a}$ is a $C_{3-6}$heterocycloalkyl comprising one nitrogen located at the point of attachment to Ar2a. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is pyrrolidinyl. Suitably, the heterocyclohexyl ring is piperidinyl or piperazinyl. Any nitrogen atom such as one nitrogen atom in the $C_{3-6}$heterocycloalkyl ring may be substituted, for example by $C_{1-4}$alkyl, C(O)H, $C(O)C_{1-4}$alkyl, $C(O)OC_{1-4}$alkyl, $C(O)OC_{1-4}$alkylaryl such as C(O)OBz, $C(O)NHC_{1-4}$alkyl, $C(O)NHC_{1-4}$alkylaryl such as C(O)NHBz, an Fmoc group, $C(O)C_{1-4}$haloalkyl, $C(O)OC_{1-4}$haloalkyl or $C(O)NHC_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, $C(O)C_{1-3}$alkyl$OC_{1-2}$alkyl such as $C(O)CH_2OCH_3$, and $C_{1-2}$alkyl$C(O)OC_{1-4}$alkyl such as $CH_2C(O)OCH_2CH_3$. Suitably, any nitrogen atom in the $C_{3-6}$heterocycloalkyl ring is not substituted. In a nineteenth embodiment, $R_{12a}$ together with a nitrogen atom to which it is attached forms an N-oxide ($N^+$-$O^-$).

When Aa is -NHC(=O)- or -C(=O)NH-, suitably, $R_{12a}$ is attached to Ar2a in the ortho or meta position relative to Ar1a and $R_{12a}$ is H, halo, $C_{1-4}$alkyl, $C_{2-4}$alkenyl, $C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, $NHC(O)C_{1-3}$alkyl or $NR_{23a}R_{24a}$.

When Aa is -NHC(=O)-, suitably $R_{12a}$ may additionally be selected from CN, $OCH_2CH_2N(CH_3)_2$ and a $C_{3-6}$heterocycloalkyl comprising one nitrogen located at the point of attachment to Ar2a, or $R_{12a}$ together with a nitrogen atom to which it is attached forms an N-oxide ($N^+$-$O^-$).

When Aa is -NH-, -$CH_2$NH- or -NH$CH_2$-, suitably, $R_{12a}$ is attached to Ar2a in the ortho or meta position relative to Ar1a and $R_{12a}$ is H, halo, $C_{1-4}$alkyl, $C_{2-4}$alkenyl, $C_{0-2}$alkylene$C_{3-5}$cycloalkyl, O$C_{1-4}$alkyl, O$C_{0-2}$alkylene$C_{3-5}$cycloalkyl, $C_{1-4}$haloalkyl, O$C_{1-4}$haloalkyl, hydroxy, $C_{1-4}$alkylOH, $SO_2C_{1-2}$alkyl, C(O)N($C_{1-2}$alkyl)$_2$, NHC(O)$C_{1-3}$alkyl or $NR_{23a}R_{24a}$.

When Aa is -NH- or -NH$CH_2$-, suitably $R_{12a}$ may additionally be selected from CN, $OCH_2CH_2N(CH_3)_2$ and a $C_{3-6}$heterocycloalkyl comprising one nitrogen located at the point of attachment to Ar2a, or $R_{12a}$ together with a nitrogen atom to which it is attached forms an N-oxide ($N^+$-$O^-$).

$R_{12a}$ is suitably H, F, Cl, $CH_3$, $OCH_3$, OEt, OiPr, OCyclopropyl, CN, $CF_3$, $OCHF_2$ or $OCH_2CF_3$. In particular, $R_{12a}$ is Cl, CN, $CF_3$, $OCHF_2$, $OCH_2CF_3$, $OCH_3$, OEt, OiPr, OCyclopropyl, such as $CF_3$, $OCHF_2$, $OCH_2CF_3$, $OCH_3$, OEt, OiPr, OCyclopropyl, e.g. OEt.

$R_{12a}$ is suitably H, F, Cl, $CH_3$, iPr, $OCH_3$, OEt, OiPr, OCyclopropyl, CN, $CF_3$, $OCHF_2$, $OCH_2CF_3$, $C_3$cycloalkyl or C(=$CH_2$)$CH_3$. In particular, $R_{12a}$ is Cl, iPr, $OCH_3$, OEt, OiPr, OCyclopropyl, CN, $CF_3$, $OCHF_2$, $OCH_2CF_3$, $C_3$cycloalkyl or C(=$CH_2$)$CH_3$, such as Cl, $OCH_3$, OEt, OiPr, OCyclopropyl, $CF_3$, $OCHF_2$, $OCH_2CF_3$ or $C_3$cycloalkyl, e.g. OEt.

When Aa is -C(=O)NH-, suitably $R_{12a}$ is $CF_3$, OEt or OiPr, such as OEt or OiPr.

Suitably $R_{12a}$ is in the meta position of Ar2a. Alternatively, $R_{12a}$ is in the ortho position of Ar2a.

In one embodiment, $R_{13a}$ is H. In another embodiment, $R_{13a}$ is halo such as F or Cl, suitably F.

In one embodiment, $R_{13a}$ is in the ortho position with respect to Ar1a. In another embodiment, $R_{13a}$ is in the para position with respect to Ar1a. In another embodiment, $R_{13a}$ is in the meta position with respect to Ar1a.

In one embodiment, $R_{23a}$ is H. In another embodiment, $R_{23a}$ is $C_{1-2}$alkyl such as methyl.

In one embodiment, $R_{24a}$ is H. In another embodiment $R_{24a}$ is $C_{1-2}$alkyl such as methyl.

Suitably, $R_{23a}$ is H and $R_{24a}$ is ethyl. Suitably, $R_{23a}$ is $CH_3$ and $R_{24a}$ is $CH_3$.

Desirably, a compound of formula (I) does not include 2-(6-(methylsulfonamido)pyrazin-2-yl)-N-(4-(pyridin-3-yl) phenyl)acetamide.

In one embodiment, at least one of $R_{10a}$, $R_{11a}$, $R_{12a}$ and $R_{13a}$ is other than H.

Suitably, at least one of $R_{4a}$, $R_{5a}$, $R_{10a}$, $R_{11a}$, $R_{12a}$ and $R_{13a}$ is other than H.

More suitably, when $R_{1a}$ is methyl, at least one of $R_{4a}$, $R_{5a}$, $R_{10a}$, $R_{11a}$, $R_{12a}$ and $R_{13a}$ is other than H.

The present invention provides the compounds described in any one of Examples P226, P227, P228, P229, P230, P235, P242, P244, P248, P251, P254, P255, P256, P258, P260 and P261.

The present invention also provides the compounds described in any one of Examples P288, P289, P290, P291, P292, P293, P294, P295, P296, P297, P298, P299, P300, P301, P302, P303, P304, P305, P306, P307, P308, P309, P310, P311, P312, P313, P314, P315, P316, P317 and P318.

The present invention provides the following compounds:

1-(2-(cyclopropanesulfonamido)pyrimidin-4-yl)-*N*-(5-(6-ethoxypyrazin-2-yl)pyridin-2-yl)-4-oxocyclohexanecarboxamide;

1-(2-(cyclopropanesulfonamido)pyrimidin-4-yl)-*N*-(5-(6-ethoxypyrazin-2-yl)pyridin-2-yl)-4-hydroxycyclohexanecarboxamide;

1-(2-(cyclopropanesulfonamido)pyrimidin-4-yl)-*N*-(5-(6-ethoxypyrazin-2-yl)pyridin-2-yl)-4-hydroxycyclohexanecarboxamide (diastereomer 1);

1-(2-(cyclopropanesulfonamido)pyrimidin-4-yl)-*N*-(5-(6-ethoxypyrazin-2-yl)pyridin-2-yl)-4-hydroxycyclohexanecarboxamide (diastereomer 2);

1-(2-(cyclopropanesulfonamido)pyrimidin-4-yl)-4-(dimethylamino)-*N*-(5-(6-ethoxypyrazin-2-yl)pyridin-2-yl)cyclohexane-1-carboxamide;

1-(2-(cyclopropanesulfonamido)pyrimidin-4-yl)-4-(dimethylamino)-*N*-(5-(6-ethoxypyrazin-2-yl)pyridin-2-yl)cyclohexane-1-carboxamide (diastereomer 1);

1-(2-(cyclopropanesulfonamido)pyrimidin-4-yl)-4-(dimethylamino)-*N*-(5-(6-ethoxypyrazin-2-yl)pyridin-2-yl)cyclohexane-1-carboxamide (diastereomer 2);

*N*-(4-(1-((4-(6-Ethoxypyrazin-2-yl)-2-fluorobenzyl)amino)propyl)pyrimidin-2-yl)cyclopropanesulfonamide;

1-(2-(cyclopropanesulfonamido)pyrimidin-4-yl)-*N*-(5-(6-ethoxypyrazin-2-yl)pyridin-2-yl)-4,4-difluorocyclohexane-1-carboxamide;

8-(2-(cyclopropanesulfonamido)pyrimidin-4-yl)-*N*-(5-(6-ethoxypyrazin-2-yl)pyridin-2-yl)-1,4-dioxaspiro[4.5]decane-8-carboxamide;

4-(2-((*N,N*-dimethylsulfamoyl)amino)pyrimidin-4-yl)-N-(5-(6-ethoxypyrazin-2-yl)pyridin-2-yl)tetrahydro-2*H*-pyran-4-carboxamide;

4-(2-(cyclopropanesulfonamido)pyrimidin-4-yl)-*N*-(5-(6-ethoxypyrazin-2-yl)pyridin-2-yl)-1-(methylsulfonyl)piperidine-4-carboxamide;

N-(4-(1-(((5-(6-ethoxypyrazin-2-yl)pyridin-2-yl)methyl)amino)cyclopropyl)pyrimidin-2-yl)cyclopropanesulfonamide;

N-(4-(1-((4-(6-ethoxypyrazin-2-yl)-2-fluorobenzyl)amino)cyclopropyl)pyrimidin-2-yl)cyclopropanesulfonamide;

N-(4-(4-(((4-(6-ethoxypyrazin-2-yl)phenyl)amino)methyl)tetrahydro-2H-pyran-4-yl)pyrimidin-2-yl)cyclopropanesulfonamide;

2-(2-(cyclopropanesulfonamido)pyrimidin-4-yl)-*N*-(5-(6-ethoxypyrazin-2-yl)pyridin-2-yl)-5,8-dioxaspiro[3.4]octane-2-carboxamide;

1-(2-(cyclopropanesulfonamido)pyrimidin-4-yl)-*N*-(5-(6-ethoxypyrazin-2-yl)pyridin-2-yl)-4-methoxycyclohexane-1-carboxamide; and

*N*-(4-(1-((4-(6-ethoxypyrazin-2-yl)phenyl)amino)propyl)pyrimidin-2-yl)cyclopropanesulfonamidearboxamide.

The present invention also provides the following compounds:

4-(2-(Cyclopropanesulfonamido)pyrimidin-4-yl)-*N*-(5-(6-ethoxypyrazin-2-yl)pyridin-2-yl)-1-(2-methoxyacetyl)piperidine-4-carboxamide;

4-(2-(Cyclopropanesulfonamido)pyrimidin-4-yl)-*N*-(5-(6-ethoxypyrazin-2-yl)pyridin-2-yl)-1-(ethylsulfonyl)piperidine-4-carboxamide;

4-(2-(Cyclopropanesulfonamido)pyrimidin-4-yl)-1-(cyclopropylsulfonyl)-*N*-(5-(6-ethoxypyrazin-2-yl)pyridin-2-yl)piperidine-4-carboxamide;

4-(2-(Cyclopropanesulfonamido)pyrimidin-4-yl)-1-(*N,N*-dimethylsulfamoyl)-*N*-(5-(6-ethoxypyrazin-2-yl)pyridin-2-yl) piperidine-4-carboxamide;

4-(2-(Cyclopropanesulfonamido)pyrimidin-4-yl)-*N*-(5-(6-ethoxypyrazin-2-yl)pyridin-2-yl)-1-((trifluoromethyl)sulfonyl)piperidine-4-carboxamide;

4-(2-(Cyclopropanesulfonamido)pyrimidin-4-yl)-*N*-(5-(6-ethoxypyrazin-2-yl)pyridin-2-yl)-1-((1-methyl-1*H*-pyrazol-3-yl)sulfonyl)piperidine-4-carboxamide;

1-(cyanomethyl)-4-(2-(cyclopropanesulfonamido)pyrimidin-4-yl)-N-(5-(6-ethoxypyrazin-2-yl)pyridin-2-yl)piperidine-4-carboxamide;

ethyl 2-(4-(2-(cyclopropanesulfonamido)pyrimidin-4-yl)-4-((5-(6-ethoxypyrazin-2-yl)pyridin-2-yl)carbamoyl)piperidin-1-yl)acetate;

*N*-(5-(6-ethoxypyrazin-2-yl)pyridin-2-yl)-4-(2-(ethylsulfonamido)pyrimidin-4-yl)-1-(2-methoxyacetyl)piperidine-4-carboxamide;

*N*-(5-(6-ethoxypyrazin-2-yl)pyridin-2-yl)-4-(2-(ethylsulfonamido)pyrimidin-4-yl)-1-(methylsulfonyl)piperidine-4-carboxamide;

*N*-(5-(6-ethoxypyrazin-2-yl)pyridin-2-yl)-4-(2-(ethylsulfonamido)pyrimidin-4-yl)-1-(ethylsulfonyl)piperidine-4-carboxamide;

1-(Cyclopropylsulfonyl)-*N*-(5-(6-ethoxypyrazin-2-yl)pyridin-2-yl)-4-(2-(ethylsulfonamido)pyrimidin-4-yl)piperidine-4-carboxamide;

*N*-(5-(6-ethoxypyrazin-2-yl)pyridin-2-yl)-4-(2-(ethylsulfonamido)pyrimidin-4-yl)-1-((1-methyl-1*H*-pyrazol-3-yl)sulfonyl)piperidine-4-carboxamide;

1-(2-(Cyclopropanesulfonamido)pyrimidin-4-yl)-N-(5-(6-cyclopropylpyrazin-2-yl)pyridin-2-yl)-4-methoxycyclohexane-1-carboxamide (diastereomer 1);

1-(2-(Cyclopropanesulfonamido)pyrimidin-4-yl)-N-(5-(6-cyclopropylpyrazin-2-yl)pyridin-2-yl)-4-methoxycyclohexane-1-carboxamide (diastereomer 2);

1-(2-(Cyclopropanesulfonamido)pyrimidin-4-yl)-*N*-(5-(6-ethoxypyrazin-2-yl)pyridin-2-yl)-4-(pyrrolidin-1-yl)cyclohexane-1-carboxamide (diastereomer 1);

1-(2-(Cyclopropanesulfonamido)pyrimidin-4-yl)-*N*-(5-(6-ethoxypyrazin-2-yl)pyridin-2-yl)-4-(pyrrolidin-1-yl)cyclohexane-1-carboxamide (diastereomer 2);

4-amino-1-(2-(cyclopropanesulfonamido)pyrimidin-4-yl)-N-(5-(6-ethoxypyrazin-2-yl)pyridin-2-yl)cyclohexane-1-carboxamide (diastereomer 1);

1-(2-(cyclopropanesulfonamido)pyrimidin-4-yl)-N-(5-(6-ethoxypyrazin-2-yl)pyridin-2-yl)-4-morpholinocyclohexane-1-carboxamide (diastereomer 1);

1-(2-(cyclopropanesulfonamido)pyrimidin-4-yl)-N-(5-(6-ethoxypyrazin-2-yl)pyridin-2-yl)-4-morpholinocyclohexane-1-carboxamide (diastereomer 2);

1-(2-(cyclopropanesulfonamido)pyrimidin-4-yl)-N-(5-(6-ethoxypyrazin-2-yl)pyridin-2-yl)-4-(methyl(oxetan-3-yl)amino)cyclohexane-1-carboxamide (diastereomer 1);

1-(2-(cyclopropanesulfonamido)pyrimidin-4-yl)-N-(5-(6-ethoxypyrazin-2-yl)pyridin-2-yl)-4-((2-methoxyethyl)(methyl)amino)cyclohexane-1-carboxamide (diastereomer 1);

1-(2-(cyclopropanesulfonamido)pyrimidin-4-yl)-N-(5-(6-ethoxypyrazin-2-yl)pyridin-2-yl)-4-((2-methoxyethyl)(methyl)amino)cyclohexane-1-carboxamide (diastereomer 2);

1-(2-(cyclopropanesulfonamido)pyrimidin-4-yl)-4-((2,2-difluoroethyl)(methyl)amino)-N-(5-(6-ethoxypyrazin-2-yl)pyridin-2-yl)cyclohexane-1-carboxamide (diastereomer 1);

1-(2-(cyclopropanesulfonamido)pyrimidin-4-yl)-N-(5-(6-ethoxypyrazin-2-yl)pyridin-2-yl)-4-(4-methylpiperazin-1-yl)cyclohexane-1-carboxamide (diastereomer 1);

1-(2-(cyclopropanesulfonamido)pyrimidin-4-yl)-N-(5-(6-ethoxypyrazin-2-yl)pyridin-2-yl)-4-(4-methylpiperazin-1-yl)cyclohexane-1-carboxamide (diastereomer 2);

4-(6-(cyclopropanesulfonamido)pyrazin-2-yl)-N-(5-(6-ethoxypyrazin-2-yl)pyridin-2-yl)-1-(methylsulfonyl)piperidine-4-carboxamide;

4-(4-(cyclopropanesulfonamido)pyrimidin-2-yl)-N-(5-(6-cyclopropylpyrazin-2-yl)pyridin-2-yl)-1-(methylsulfonyl)piperidine-4-carboxamide;

4-(4-(cyclopropanesulfonamido)pyrimidin-2-yl)-N-(5-(6-ethoxypyrazin-2-yl)pyridin-2-yl)-1-(methylsulfonyl)piperidine-4-carboxamide;

4-(4-(cyclopropanesulfonamido)pyrimidin-2-yl)-N-(5-(6-cyclopropylpyrazin-2-yl)pyridin-2-yl)-1-(ethylsulfonyl)piperidine-4-carboxamide; and

4-(4-(cyclopropanesulfonamido)pyrimidin-2-yl)-N-(5-(6-ethoxypyrazin-2-yl)pyridin-2-yl)-1-(ethylsulfonyl)piperidine-4-carboxamide.

Compounds of formula (I-b)

The invention provides a compound of formula (I-b):

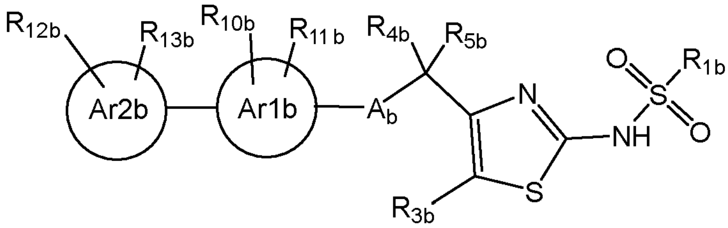

(I-b)

wherein

$A_b$ is $A_{ab}$ or $A_{bb}$;

$A_{ab}$ is $-NR_{6b}CH_2-$ or $-NR_{6b}-$;
$A_{bb}$ is $-NR_{6b}C(=O)-$;

$R_{1b}$ is $R_{1ab}$ or $R_{1bb}$,

wherein:
$R_{1ab}$ is $NR_{32b}R_{33b}$;

$R_{1bb}$ is $C_{1-5}$alkyl, $C_{0-2}$alkylene$C_{3-5}$cycloalkyl which cycloalkyl is optionally substituted by $CH_3$, $C_{1-3}$alkylene$OC_{1-2}$alkyl, or $CF_3$;

$R_{3b}$ is H, halo, $CH_3$, $OC_{1-2}$alkyl or $CF_3$;
or $R_{3b}$ together with $R_{5bb}$ forms a 5- or 6-membered cycloalkyl or 5 or 6 membered oxygen-containing heterocycloalkyl;

$R_{4b}$ and $R_{5b}$ are either $R_{4ab}$ and $R_{5ab}$ or $R_{4bb}$ and $R_{5bb}$;

wherein:

$R_{4ab}$ and $R_{5ab}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21b}R_{22b}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ab}$ and $R_{5ab}$ together with the carbon atom to which they are attached may be substituted by one or two substituents, each substituent being independently selected from the group consisting of $C_{1-3}$alkyl or $OC_{1-3}$alkyl; or

$R_{4ab}$ and $R_{5ab}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ab}$ and $R_{5ab}$ together with the carbon atom to which they are attached may be substituted by one or two substituents, each substituent being independently selected from the group consisting of $C_{1-3}$alkyl or $OC_{1-3}$alkyl; or

$R_{4ab}$ and $R_{5ab}$ together with the carbon atom to which they are attached form a $C_{3-6}$heterocycloalkyl comprising one nitrogen atom, wherein said nitrogen atom is substituted by $-S(O)_2R_{29b}$; or

$R_{4bb}$ and $R_{5bb}$ are each independently H, halo, $C_{1-6}$alkyl, $C_{0-2}$alkylene$C_{3-6}$cycloalkyl, $C_{0-2}$alkylene$C_{3-6}$heterocycloalkyl, $OC_{1-6}$alkyl, $OC_{0-2}$alkylene$C_{3-6}$cycloalkyl, $OC_{0-2}$alkylene$C_{3-6}$heterocycloalkyl, $C_{1-3}$alkyleneO-$C_{1-3}$alkyl, $C_{1-6}$alkylOH, $C_{1-6}$haloalkyl, $OC_{1-6}$haloalkyl or $NR_{21b}R_{22b}$,

or $R_{4bb}$ is H and $R_{5bb}$ together with $R_{3b}$ form a 5- or 6-membered cycloalkyl or 5 or 6 membered oxygen-containing heterocycloalkyl,

or $R_{4bb}$ and $R_{5bb}$ together with the carbon atom to which they are attached form a $C_{3-6}$cycloalkyl or $C_{3-6}$heterocycloalkyl,

or $R_{4bb}$ is H and $R_{5bb}$ and $R_{6b}$ are a $C_{2-3}$alkylene chain forming a 5- or 6-membered ring;

or $R_{4bb}$ is O and $R_{5bb}$ is absent;

$R_{6b}$ is H or $C_{1-3}$alkyl,

or $R_{6b}$ together with $R_{11b}$ when in the ortho-position to group $A_b$ are a $C_2$alkylene chain forming a 5-membered ring,

or $R_{5bb}$ and $R_{6b}$ are a $C_{2-3}$alkylene chain forming a 5- or 6-membered ring and $R_{4bb}$ is H;

Ar1b is 6-membered aryl or heteroaryl;

Ar2b is a 6-membered aryl or heteroaryl and is attached to Ar1b in the para position relative to group $A_b$;

$R_{10b}$ is H, halo, $C_{1-3}$alkyl, $OC_{1-2}$alkyl, $C_{1-2}$haloalkyl, $OC_{1-2}$haloalkyl or CN;

$R_{11b}$ is H, F, Cl, $CH_3$, ethyl, $OCH_3$, $CF_3$, $OCF_3$ or CN,
or $R_{11b}$, when in the ortho-position to group $A_b$, together with $R_{6b}$ are a $C_2$alkylene chain forming a 5-membered ring;

$R_{12b}$ is attached to Ar2b in the ortho or meta position relative to Ar1b and $R_{12b}$ is H, halo, $C_{1-4}$alkyl, $C_{2-4}$alkynyl, $C_{0-2}$alkylene$C_{3-5}$cycloalkyl, $OC_{1-4}$alkyl, $OC_{0-2}$alkylene$C_{3-5}$cycloalkyl, $OCH_2CH_2N(CH_3)_2$, OH, $C_{1-4}$alkylOH, CN, $C_{1-3}$alkylene$OC_{1-3}$alkyl, $C_{1-4}$haloalkyl, $OC_{1-4}$haloalkyl, $C(=O)C_{1-2}$alkyl, $NR_{23b}R_{24b}$, $SO_2C_{1-4}$alkyl, $SOC_{1-4}$alkyl, $SC_{1-4}$alkyl, SH, $C(O)N(CH_3)_2$, $NHC(O)C_{1-3}$alkyl, $C_{3-6}$heterocycloalkyl comprising one nitrogen located at the point of attachment to Ar2b, or $R_{12b}$ together with a nitrogen atom to which it is attached forms an N-oxide ($N^+$-$O^-$);

$R_{13b}$ is H, halo, $CH_3$ or $OCH_3$;

$R_{21b}$ is H, $C_{1-5}$alkyl, $C(O)C_{1-5}$alkyl, $C(O)OC_{1-5}$alkyl, $C_{1-3}$alkyl$OC_{1-2}$alkyl, $C_{1-4}$haloalkyl, or $C_{4-6}$heterocycloalkyl;

$R_{22b}$ is H or $CH_3$;

$R_{23b}$ is H or $C_{1-2}$alkyl;

$R_{24b}$ is H or $C_{1-2}$alkyl;

$R_{29b}$ is $C_{1-3}$alkyl, $C_{0-2}$alkylene$C_{3-5}$cycloalkyl which cycloalkyl is optionally substituted by $CH_3$, $CF_3$, $N(C_{1-3}alkyl)_2$, or a 5 or 6 membered heteroaryl wherein the 5 or 6 membered heteroaryl is optionally substituted by methyl; and

$R_{32b}$ is $C_{1-3}$alkyl and $R_{33b}$ is $C_{1-3}$alkyl; or

$R_{32b}$ and $R_{33b}$ together with the nitrogen atom to which they are attached form a $C_{3-5}$heterocycloalkyl;

or a salt and/or solvate thereof.

Suitably, $R_{1b}$ is $R_{1ab}$; and/or $R_{4b}$ and $R_{5b}$ are $R_{4ab}$ and $R_{5ab}$; and/or A is $A_{ab}$.

The invention also provides a compound of formula (I-b):

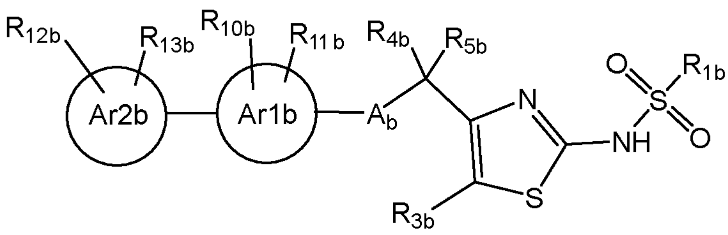

(I-b)

wherein

$A_b$ is $A_{ab}$ or $A_{bb}$;

$A_{ab}$ is $-NR_{6b}CH_2-$ or $-NR_{6b}-$;
$A_{bb}$ is $-NR_{6b}C(=O)-$;

$R_{1b}$ is $R_{1ab}$ or $R_{1bb}$,

wherein:
$R_{1ab}$ is $NR_{32b}R_{33b}$;

$R_{1bb}$ is $C_{1-5}$alkyl, $C_{0-2}$alkylene$C_{3-5}$cycloalkyl which cycloalkyl is optionally substituted by $CH_3$, $C_{1-3}$alkylene$OC_{1-2}$alkyl, or $CF_3$;

$R_{3b}$ is H, halo, $CH_3$, $OC_{1-2}$alkyl or $CF_3$;
or $R_{3b}$ together with $R_{5bb}$ forms a 5- or 6-membered cycloalkyl or 5 or 6 membered oxygen-containing heterocycloalkyl;

$R_{4b}$ and $R_{5b}$ are either $R_{4ab}$ and $R_{5ab}$ or $R_{4bb}$ and $R_{5bb}$;

wherein:

$R_{4ab}$ and $R_{5ab}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21b}R_{22b}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ab}$ and $R_{5ab}$ together with the carbon atom to which they are attached may be substituted by one or two substituents, each substituent being independently selected from the group consisting of $C_{1-3}$alkyl or $OC_{1-3}$alkyl; or

$R_{4ab}$ and $R_{5ab}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ab}$ and $R_{5ab}$ together with the carbon atom to which they are attached may be substituted by one or two substituents, each substituent being independently selected from the group consisting of $C_{1-3}$alkyl or $OC_{1-3}$alkyl; or

$R_{4ab}$ and $R_{5ab}$ together with the carbon atom to which they are attached form a $C_{3-6}$heterocycloalkyl comprising one nitrogen atom, wherein said nitrogen atom is substituted by $-S(O)_2R_{29b}$; or

$R_{4bb}$ and $R_{5bb}$ are each independently H, halo, $C_{1-6}$alkyl, $C_{0-2}$alkylene$C_{3-6}$cycloalkyl, $C_{0-2}$alkylene$C_{3-6}$heterocycloalkyl, $OC_{1-6}$alkyl, $OC_{0-2}$alkylene$C_{3-6}$cycloalkyl, $OC_{0-2}$alkylene$C_{3-6}$heterocycloalkyl, $C_{1-3}$alkyleneO-$C_{1-3}$alkyl, $C_{1-6}$alkylOH, $C_{1-6}$haloalkyl, $OC_{1-6}$haloalkyl or $NR_{21b}R_{22b}$,

or $R_{4bb}$ is H and $R_{5bb}$ together with $R_{3b}$ form a 5- or 6-membered cycloalkyl or 5 or 6 membered oxygen-containing heterocycloalkyl,

or $R_{4bb}$ and $R_{5bb}$ together with the carbon atom to which they are attached form a $C_{3-6}$cycloalkyl or $C_{3-6}$heterocycloalkyl,

or $R_{4bb}$ is H and $R_{5bb}$ and $R_{6b}$ are a $C_{2-3}$alkylene chain forming a 5- or 6-membered ring;

or $R_{4bb}$ is O and $R_{5bb}$ is absent;

$R_{6b}$ is H or $C_{1-3}$alkyl,

or $R_{6b}$ together with $R_{11b}$ when in the ortho-position to group $A_b$ are a $C_2$alkylene chain forming a 5-membered ring,

or $R_{5pp}$ and $R_{6b}$ are a $C_{2-3}$alkylene chain forming a 5- or 6-membered ring and $R_{4bb}$ is H;

Ar1b is 6-membered aryl or heteroaryl;

Ar2b is a 6-membered aryl or heteroaryl and is attached to Ar1b in the para position relative to group $A_b$;

$R_{10b}$ is H, halo, $C_{1-3}$alkyl, $OC_{1-2}$alkyl, $C_{1-2}$haloalkyl, $OC_{1-2}$haloalkyl or CN;

$R_{11b}$ is H, F, Cl, $CH_3$, ethyl, $OCH_3$, $CF_3$, $OCF_3$ or CN,
or $R_{11b}$, when in the ortho-position to group $A_b$, together with $R_{6b}$ are a $C_2$alkylene chain forming a 5-membered ring;

$R_{12b}$ is attached to Ar2b in the ortho or meta position relative to Ar1b and $R_{12b}$ is H, halo, $C_{1-4}$alkyl, $C_{2-4}$alkynyl, $C_{0-2}$alkylene$C_{3-5}$cycloalkyl, $OC_{1-4}$alkyl, $OC_{0-2}$alkylene$C_{3-5}$cycloalkyl, $OCH_2CH_2N(CH_3)_2$, OH, $C_{1-4}$alkylOH, CN, $C_{1-3}$alkylene$OC_{1-3}$alkyl, $C_{1-4}$haloalkyl, $OC_{1-4}$haloalkyl, $C(=O)C_{1-2}$alkyl, $NR_{23b}R_{24b}$, $SO_2C_{1-4}$alkyl, $SOC_{1-4}$alkyl, $SC_{1-4}$alkyl, SH, $C(O)N(CH_3)_2$, $NHC(O)C_{1-3}$alkyl, $C_{3-6}$heterocycloalkyl comprising one nitrogen located at the point of attachment to Ar2b, or $R_{12b}$ together with a nitrogen atom to which it is attached forms an N-oxide ($N^+$-$O^-$);

$R_{13b}$ is H, halo, $CH_3$ or $OCH_3$;

$R_{21b}$ is H, $C_{1-5}$alkyl, $C(O)C_{1-5}$alkyl, $C(O)OC_{1-5}$alkyl;

$R_{22b}$ is H or $CH_3$;

$R_{23b}$ is H or $C_{1-2}$alkyl;

$R_{24b}$ is H or $C_{1-2}$alkyl;

$R_{29b}$ is $C_{1-3}$alkyl, $C_{0-2}$alkylene$C_{3-5}$cycloalkyl which cycloalkyl is optionally substituted by $CH_3$, or $CF_3$; and

$R_{32b}$ is $C_{1-3}$alkyl and $R_{33b}$ is $C_{1-3}$alkyl; or

$R_{32b}$ and $R_{33b}$ together with the nitrogen atom to which they are attached form a $C_{3-5}$heterocycloalkyl;

or a salt and/or solvate thereof.

Suitably, $R_{1b}$ is $R_{1ab}$; and/or $R_{4b}$ and $R_{5b}$ are $R_{4ab}$ and $R_{5ab}$; and/or A is $A_{ab}$.

In one embodiment of the invention, $R_{1b}$ is $R_{1ab}$, i.e. is $NR_{32b}R_{33b}$. In an embodiment, $R_{32b}$ is $C_{1-3}$alkyl, such as methyl or ethyl, e.g. methyl. In an embodiment, $R_{33b}$ is $C_{1-3}$alkyl, such as methyl or ethyl, e.g. methyl. Suitably, $R_{32b}$ and $R_{33b}$ are both methyl. Suitably, $R_{32b}$ and $R_{33b}$ are both ethyl. Suitably, $R_{32b}$ is methyl and $R_{33b}$ is ethyl.

In another embodiment, $R_{32b}$ and $R_{33b}$ together with the nitrogen atom to which they are attached form a $C_{3-5}$heterocycloalkyl. Suitably, the $C_{3-5}$heterocycloalkyl is aziridinyl, azetidinyl or pyrrolidinyl.

Suitably, $R_{1b}$ is $R_{1bb}$.

In one embodiment of the invention $R_{1bb}$ **is** $C_{1-5}$alkyl. When $R_{1bb}$ **is** $C_{1-5}$alkyl, $R_{1bb}$ may be methyl, ethyl, propyl (n-propyl or isopropyl), butyl (n-butyl, isobutyl, sec-butyl or tert-butyl) or pentyl (e.g. n-pentyl, sec-pentyl or 3-pentyl). Suitably, when $R_{1bb}$ **is** $C_{1-5}$alkyl, $R_{1bb}$ may be methyl, ethyl, propyl (e.g. isopropyl) or butyl (e.g. sec-butyl or tert-butyl), especially methyl, ethyl or isopropyl and in particular methyl.

In a second embodiment of the invention $R_{1bb}$ is $C_{0-2}$alkylene$C_{3-5}$cycloalkyl which cycloalkyl is optionally substituted by $CH_3$. In some embodiments, $R_{1bb}$ is $C_{0-2}$alkylene$C_{3-5}$cycloalkyl. In other embodiments, $R_{1bb}$ is $C_{0-2}$alkylene$C_{3-5}$cycloalkyl which cycloalkyl is substituted by $CH_3$. $R_{1bb}$ may be $C_{3-5}$cycloalkyl, which cycloalkyl is optionally substituted by $CH_3$. $R_{1bb}$ may be $C_1$alkylene$C_{3-5}$cycloalkyl, which cycloalkyl is optionally substituted by $CH_3$. $R_{1bb}$ may be $C_2$alkylene$C_{3-5}$cycloalkyl, which cycloalkyl is optionally substituted by $CH_3$. $R_{1bb}$ may be $C_{0-2}$alkylene$C_3$cycloalkyl, which cycloalkyl is optionally substituted by $CH_3$. $R_{1bb}$ may be $C_{0-2}$alkylene$C_4$cycloalkyl, which cycloalkyl is optionally substituted by $CH_3$. $R_{1bb}$ may be $C_{0-2}$alkylene$C_5$cycloalkyl, which cycloalkyl is optionally substituted by $CH_3$. Suitably, where $C_{0-2}$alkylene$C_{3-5}$cycloalkyl is optionally substituted by $CH_3$, the $CH_3$ is at the point of attachment of the $C_{3-5}$cycloalkyl to the $C_{0-2}$alkylene.

In a third embodiment of the invention $R_{1bb}$ is $CF_3$.

In a fourth embodiment of the invention $R_{1bb}$ is $C_{1-3}$alkylene$OC_{1-2}$alkyl such as $C_{1-2}$alkylene$OC_{1-2}$alkyl. When $R_{1bb}$ is $C_{1-3}$alkylene$OC_{1-2}$alkyl, $R_{1bb}$ may be methoxymethyl, methoxyethyl, ethoxymethyl, ethoxyethyl, propoxymethyl or propoxyethyl. When $R_{1bb}$ is $C_{1-2}$alkylene$OC_{1-2}$alkyl, $R_{1bb}$ may be methoxymethyl, methoxyethyl, ethoxymethyl or ethoxyethyl.

Suitably $R_{1bb}$ is cyclopropyl, cyclopropyl substituted by $CH_3$ at the point of attachment, cyclobutyl, $CH_3$, isopropyl, sec-butyl, tert-butyl or $CF_3$. In particular $R_{1bb}$ is cyclopropyl, cyclopropyl substituted by $CH_3$ at the point of attachment, cyclobutyl, $CH_3$, isopropyl, sec-butyl or tert-butyl, especially cyclopropyl, cyclopropyl substituted by $CH_3$ at the point of attachment, cyclobutyl or isopropyl, such as cyclopropyl or cyclopropyl substituted by $CH_3$ at the point of attachment.

Additionally of interest is when $R_{1bb}$ is cyclopentyl, methyl, ethyl, cyclopropylmethylene and methoxyethyl, in particular cyclopentyl, methyl, ethyl and cyclopropylmethylene, especially ethyl and methyl, such as methyl.

Consequently, suitably $R_{1bb}$ is cyclopropyl, cyclopropyl substituted by $CH_3$ at the point of attachment, cyclopropylmethylene, cyclobutyl, cyclopentyl, $CH_3$, ethyl, isopropyl, sec-butyl, tert-butyl, methoxyethyl or $CF_3$. In particular $R_{1bb}$ is cyclopropyl, cyclopropyl substituted by $CH_3$ at the point of attachment, cyclopropylmethylene, cyclobutyl, cyclopentyl, $CH_3$, ethyl, isopropyl, sec-butyl or tert-butyl, especially cyclopropyl, cyclopropyl substituted by $CH_3$ at the point of attachment, cyclobutyl, $CH_3$, ethyl or isopropyl, such as cyclopropyl, cyclopropyl substituted by $CH_3$ at the point of attachment, ethyl or methyl such as cyclopropyl, cyclopropyl substituted by $CH_3$ at the point of attachment or methyl.

In one embodiment $R_{3b}$ is H. In a second embodiment $R_{3b}$ is halo, in particular chloro or fluoro, especially chloro. In a third embodiment $R_{3b}$ is $CH_3$. In a fourth embodiment $R_{3b}$ is $CF_3$. In a fifth embodiment $R_{3b}$ together with $R_{5bb}$ forms a 5- or 6-membered cycloalkyl, in particular a 5-membered cycloalkyl. In a sixth embodiment $R_{3b}$ is $OC_{1-2}$alkyl such as $OCH_3$. In a seventh embodiment $R_{3b}$ together with $R_{5bb}$ forms a 5- or 6-membered oxygen-containing heterocycloalkyl, in particular a 5-membered heterocycloalkyl.

The phrase '$R_{3b}$ together with $R_{5bb}$ forms a 5- or 6-membered cycloalkyl' means that compounds with the following exemplary substructure are formed:

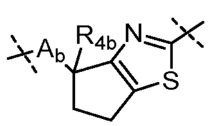 or 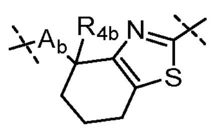 .

The phrase '$R_{3b}$ together with $R_{5bb}$ forms a 5- or 6-membered oxygen containing heterocycloalkyl' means that compounds with the following substructure are formed:

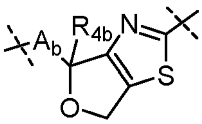 ; 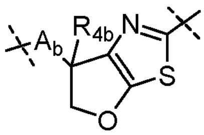

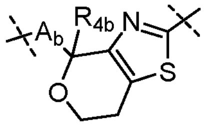 ; 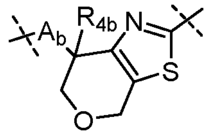 ; or 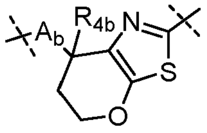 .

In particular $R_{3b}$ is H, $CH_3$ or $R_{3b}$ together with $R_{5bb}$ forms a 5- or 6-membered cycloalkyl, especially H, $CH_3$ or $R_{3b}$ together with $R_{5bb}$ forms a 5-membered cycloalkyl, such as $R_{3b}$ is H or $CH_3$, e.g. H.

In one embodiment, $R_{4b}$ and $R_{5b}$ are $R_{4ab}$ and $R_{5ab}$.

Suitably, $R_{4ab}$ and $R_{5ab}$ together with the carbon atom to which they are attached form a $C_{3-6}$cycloalkyl which is: substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and $NR_{21b}R_{22b}$.

In one embodiment, the $C_{3-6}$cycloalkyl is cyclopropyl. In another embodiment, the $C_{3-6}$cycloalkyl is cyclobutyl. In another embodiment, the $C_{3-6}$cycloalkyl is cyclopentyl. In another embodiment, the $C_{3-6}$cycloalkyl is cyclohexyl.

In one embodiment the $C_{3-6}$cycloalkyl is substituted by one substituent. In a second embodiment the $C_{3-6}$cycloalkyl is substituted by two substituents.

In one embodiment, the substituent is $C_{1-3}$alkyl. Suitably, the substituent is methyl. Suitably, the substituent is ethyl. Suitably, the substituent is n-propyl. Suitably, the substituent is iso-propyl.

In a second embodiment, the substituent is $C_{1-3}$alkylOH. Suitably, the substituent is $CH_2OH$. Suitably, the substituent is $CH_2CH_2OH$. Suitably, the substituent is $CH_2CH_2CH_2OH$.

In a third embodiment, the substituent is $C_{1-3}$haloalkyl. Suitably the $C_{1-3}$alkyl group is substituted by one two or three, such as one, halogen atom. Suitably, the halogen atom is fluoro or chloro such as fluoro. Suitably, the substituent is $C_1$haloalkyl such as $CF_3$. Suitably, the substituent is $C_2$haloalkyl such as $CH_2CF_3$.

In a fourth embodiment, the substituent is $C_{0-2}$alkylene$C_{3-6}$cycloalkyl, in particular $C_{0-2}$alkylene$C_{3-5}$cycloalkyl, such as $C_{3-5}$cycloalkyl, $C_1$alkylene$C_{3-5}$cycloalkyl or $C_2$alkylene$C_{3-5}$cycloalkyl.

In a fifth embodiment, the substituent is $C_{0-2}$alkylene$C_{3-6}$heterocycloalkyl such as $C_{0-2}$alkylene$C_3$heterocycloalkyl, $C_{0-2}$alkylene$C_4$heterocycloalkyl, $C_{0-2}$alkylene$C_5$heterocycloalkyl, $C_{0-2}$alkylene$C_6$heterocycloalkyl, $C_0$alkylene-$C_{3-6}$heterocycloalkyl, $C_1$alkylene$C_{3-6}$heterocycloalkyl and $C_2$alkylene$C_{3-6}$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) in the $C_{3-6}$heterocycloalkyl ring may be substituted (such as one nitrogen atom is substituted), for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted.

In a sixth embodiment, the substituent is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl.

In a seventh embodiment, the substituent is halo, in particular fluoro or chloro such as chloro.

In an eighth embodiment, the substituent is O$C_{1-3}$haloalkyl. Suitably the O$C_{1-3}$alkyl group is substituted by one two or three, such as one, halogen atom. Suitably, the halogen atom is fluoro or chloro such as fluoro. Suitably, the substituent is O$C_1$haloalkyl such as $OCF_3$. Suitably, the substituent is O$C_2$haloalkyl such as $OCH_2CF_3$.

In a ninth embodiment, the substituent is O$C_{0-2}$alkylene$C_{3-6}$cycloalkyl, such as O$C_{3-6}$cycloalkyl, O$C_1$alkylene$C_{3-6}$cycloalkyl or O$C_2$alkylene$C_{3-6}$cycloalkyl.

In a tenth embodiment, the substituent is O$C_{0-2}$alkylene$C_{3-6}$heterocycloalkyl such as O$C_{0-2}$alkylene$C_3$heterocycloalkyl, O$C_{0-2}$alkylene$C_4$heterocycloalkyl, O$C_{0-2}$alkylene$C_5$heterocycloalkyl, O$C_{0-2}$alkylene$C_6$heterocycloalkyl, O$C_0$alkylene$C_{3-6}$heterocycloalkyl, O$C_1$alkylene$C_{3-6}$heterocycloalkyl and O$C_2$alkylene$C_{3-6}$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) (such as one nitrogen atom) in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted.

In an eleventh embodiment, the substituent is $OC_{1-3}$alkyl, such as $OCH_3$ or $OCH_2CH_3$.

In a twelfth embodiment, the substituent is $NR_{21b}R_{22b}$.

In one embodiment $R_{21b}$ is H. In a second embodiment $R_{21b}$ is $C_{1-5}$alkyl, such as methyl, ethyl or propyl, especially methyl. In a third embodiment $R_{21b}$ is $C(O)C_{1-5}$alkyl, such as $C(O)CH_3$. In a fourth embodiment $R_{21b}$ is $C(O)OC_{1-5}$alkyl, such as $C(O)OCH_3$ or C(O)Otert-butyl. In a fifth embodiment $R_{21b}$ is $C_{1-3}$alkyl$OC_{1-2}$alkyl such as $C_1$alkyl$OC_1$alkyl, $C_2$alkyl$OC_1$alkyl or $C_3$alkyl$OC_1$alkyl e.g. $C_2$alkyl$OC_1$alkyl. In a sixth embodiment, $R_{21b}$ is $C_{1-4}$haloalkyl, such as $CF_3$, $CH_2CF_3$ or $CH_2CHF_2$ e.g. $CH_2CHF_2$. In a seventh embodiment $R_{21b}$ is $C_{4-6}$heterocycloalkyl, such as oxetanyl, tetrahydrofuranyl or tetrahydropyranyl e.g. oxetanyl, in particular 3-oxetanyl.

When the substituent is $NR_{21b}R_{22b}$, in one embodiment $R_{22b}$ is H. In a second embodiment $R_{22b}$ is methyl.

Suitably, $R_{21b}$ is $C(O)OCH_3$ and $R_{22b}$ is H. Suitably, $R_{21b}$ is $C(O)CH_3$ and $R_{22b}$ is H. Suitably, $R_{21b}$ and $R_{22b}$ are both $CH_3$. Suitably, $R_{21b}$ and $R_{22b}$ are both H.

In a thirteenth embodiment, the substituent is oxo.

In a fourteenth embodiment, the substituent is OH.

Suitably, the one or two substituents, in particular one substituent, are independently selected from the group consisting of $C_{1-3}$alkyl, oxo, OH, $C_{1-3}$alkylOH, $C_{1-3}$haloalkyl, halo, $OC_{1-3}$haloalkyl, $OC_{1-3}$alkyl and $NR_{21b}R_{22b}$.

More suitably, the substituent is independently selected from the group consisting of oxo, OH, halo, $OC_{1-3}$alkyl and $NR_{21b}R_{22b}$.

Most suitably, the substituent is independently selected from the group consisting of oxo, OH, fluoro, $NR_{21b}R_{22b}$.

Alternatively, $R_{4ab}$ and $R_{5ab}$ together with the carbon atom to which they are attached form a $C_{3-6}$cycloalkyl and one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ab}$ and $R_{5ab}$ together with the carbon atom to which they are attached may be substituted by one or two substituents, each substituent being independently selected from the group consisting of $C_{1-3}$alkyl or $OC_{1-3}$alkyl.

In one embodiment the $C_{3-6}$cycloalkyl formed by $R_{4ab}$ and $R_{5ab}$ together with the carbon atom to which they are attached is unsubstituted. In a second embodiment the $C_{3-6}$cycloalkyl formed by $R_{4ab}$ and $R_{5ab}$ together with the carbon atom to which they are attached is substituted by one or two substituents, in particular one substituent. Suitably, each substituent is independently selected from the group consisting of $C_{1-2}$alkyl or $OCH_3$.

Suitably one of the carbons of the $C_{3-6}$cycloalkyl which is formed by $R_{4ab}$ and $R_{5ab}$ is a spiro centre such that a spirocyclic ring system is formed, wherein the $C_{3-6}$cycloalkyl which is formed by $R_{4ab}$ and $R_{5ab}$ is a $C_{4-6}$cycloalkyl. Suitably the $C_{3-6}$heterocycloalkyl is an oxygen containing $C_{3-6}$heterocycloalkyl. Suitably, the $C_{3-6}$heterocycloalkyl is an oxygen comprising, such as containing, $C_{3-6}$heterocycloalkyl ring, such as a $C_5$cycloalkyl ring.

In an embodiment, $R_{4ab}$ and $R_{5ab}$ together with the carbon atom to which they are attached form a $C_{3-6}$heterocycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ab}$ and $R_{5ab}$ together with the carbon atom to which they are attached may be substituted by one or two substituents, each substituent being independently selected from the group consisting of $C_{1-3}$alkyl or $OC_{1-3}$alkyl. Suitably, each substituent is independently selected from the group consisting of $C_{1-2}$alkyl or $OCH_3$.

Suitably one of the carbons of the $C_{3-6}$heterocycloalkyl is a spiro centre such that a spirocyclic ring system is formed containing further ring C, wherein C is a $C_{4-6}$heterocycloalkyl. Suitably the $C_{4-6}$heterocycloalkyl is an oxygen containing $C_{4-6}$heterocycloalkyl such as tetrahydropyranyl or 1,3-dioxolanyl.

In an embodiment, $R_{4ab}$ and $R_{5ab}$ together with the carbon atom to which they are attached form a $C_{3-6}$heterocycloalkyl comprising one nitrogen atom, wherein said nitrogen atom is substituted by $-S(O)_2R_{29b}$.

Suitably, the $C_{3-6}$heterocycloalkyl is selected from the group consisting of aziridinyl, azetidinyl, pyrrolidinyl and piperidinyl such as piperidinyl.

Suitably, when the $C_{3-6}$heterocycloalkyl is piperidinyl, the nitrogen atom is in the 4-position relative to the quaternary carbon:

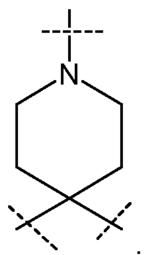

The $C_{3-6}$heterocycloalkyl may be other groups as defined elsewhere herein.

In an embodiment, $R_{29b}$ is $C_{1-3}$alkyl, $C_{0-2}$alkylene$C_{3-5}$cycloalkyl which cycloalkyl is optionally substituted by $CH_3$,

or $CF_3$. In one embodiment, $R_{29b}$ is $C_{1-3}$alkyl such as methyl. In another embodiment, $R_{29b}$ is $C_{0-2}$alkylene$C_{3-5}$cycloalkyl which cycloalkyl is optionally substituted by $CH_3$. In some embodiments, $R_{29b}$ is $C_{0-2}$alkylene$C_{3-5}$cycloalkyl. In other embodiments, $R_{29b}$ is $C_{0-2}$alkylene$C_{3-5}$cycloalkyl which cycloalkyl is substituted by $CH_3$. $R_{29b}$ may be $C_{3-5}$cycloalkyl, which cycloalkyl is optionally substituted by $CH_3$. $R_{29b}$ may be $C_1$alkylene$C_{3-5}$cycloalkyl, which cycloalkyl is optionally substituted by $CH_3$. $R_{29b}$ may be $C_2$alkylene$C_{3-5}$cycloalkyl, which cycloalkyl is optionally substituted by $CH_3$. $R_{29b}$ may be $C_{0-2}$alkylene$C_3$cycloalkyl, which cycloalkyl is optionally substituted by $CH_3$. $R_{29b}$ may be $C_{0-2}$alkylene$C_4$cycloalkyl, which cycloalkyl is optionally substituted by $CH_3$. $R_{29b}$ may be $C_{0-2}$alkylene$C_5$cycloalkyl, which cycloalkyl is optionally substituted by $CH_3$. Suitably, where $C_{0-2}$alkylene$C_{3-5}$cycloalkyl is optionally substituted by $CH_3$, the $CH_3$ is at the point of attachment of the $C_{3-5}$cycloalkyl to the $C_{0-2}$alkylene. In another embodiment, $R_{29b}$ is $CF_3$. In another embodiment, $R_{29b}$ is $N(C_{1-3}alkyl)_2$ such as $N(CH_3)_2$. In another embodiment, $R_{29b}$ is a 5 or 6 membered heteroaryl wherein the 5 or 6 membered heteroaryl is optionally substituted by methyl. In one embodiment, the 5 or 6 membered heteroaryl is substituted by methyl. In one embodiment, the 5 or 6 membered heteroaryl is not substituted by methyl. In one embodiment, $R_{29b}$ is a 5-membered heteroaryl such as pyrrolyl, furanyl, thiophenyl, imidazolyl, pyrazolyl, oxazolyl, isoxazolyl, thiazolyl, isothiazolyl, such as pyrazolyl. Suitably the pyrazolyl is substituted by methyl. In another embodiment, $R_{29b}$ is a 6-membered heteroaryl such as pyridinyl, pyridazinyl, pyrimidinyl or pyrazinyl.

In another embodiment, $R_{4b}$ and $R_{5b}$ are $R_{4bb}$ and $R_{5bb}$.

In one embodiment $R_{4bb}$ is O and $R_{5bb}$ is absent. The person skilled in the art will appreciate that in this embodiment, the following moiety forms, in order to retain the correct carbon valency of 4:

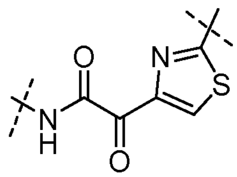

$R_{4bb}$ = O
$R_{5bb}$ = absent

.

In a second embodiment, $R_{4bb}$ and $R_{5bb}$ together with the carbon atom to which they are attached form a $C_{3-6}$cycloalkyl, such as cyclopropyl, cyclobutyl or cyclopentyl. In a third embodiment $R_{4bb}$ is $C_{1-6}$alkyl, in particular $C_{1-4}$alkyl such as methyl, ethyl, propyl (n-propyl or isopropyl) or butyl (n-butyl, isobutyl, sec-butyl or tert-butyl). In a fourth embodiment $R_{4bb}$ is $C_{0-2}$alkylene$C_{3-6}$cycloalkyl, in particular $C_{0-2}$alkylene$C_{3-5}$cycloalkyl, such as $C_{3-5}$cycloalkyl, $C_1$alkylene$C_{3-5}$cycloalkyl or $C_2$alkylene$C_{3-5}$cycloalkyl. In a fifth embodiment $R_{4bb}$ is $OC_{1-6}$alkyl, in particular $OC_{1-4}$alkyl, such as methoxy, ethoxy, propoxy (n-propoxy or isopropoxy) or butoxy (n-butoxy, isobutoxy, sec-butoxy or tert-butoxy). In a sixth embodiment $R_{4bb}$ is $OC_{0-2}$alkylene$C_{3-6}$cycloalkyl, such as $OC_{3-6}$cycloalkyl, $OC_1$alkylene$C_{3-6}$cycloalkyl or $OC_2$alkylene$C_{3-6}$cycloalkyl. In a seventh embodiment $R_{4bb}$ is $C_{1-3}$alkylene$OC_{1-3}$alkyl, in particular $C_{1-2}$alkylene$OC_{1-2}$alkyl such as $C_1$alkylene$OC_1$alkyl, $C_2$alkylene$OC_1$alkyl, $C_1$alkylene$OC_2$alkyl or $C_2$alkylene$OC_2$alkyl. In an eighth embodiment $R_{4bb}$ is $C_{1-6}$haloalkyl, in particular $C_{1-4}$haloalkyl. In a ninth embodiment $R_{4bb}$ is $OC_{1-6}$haloalkyl, in particular $OC_{1-4}$haloalkyl. In a tenth embodiment $R_{4bb}$ is H. In an eleventh embodiment $R_{4bb}$ is halo such as fluoro. In a twelfth embodiment $R_{4bb}$ is $C_{1-6}$alkylOH, such as $CH_2OH$ or $CH_2CH_2OH$, in particular $CH_2CH_2OH$. In a thirteenth embodiment $R_{4bb}$ is $NR_{21b}R_{22b}$. In a fourteenth embodiment, $R_{4bb}$ is $C_{0-2}$alkylene$C_{3-6}$heterocycloalkyl such as $C_{0-2}$alkylene$C_3$heterocycloalkyl, $C_{0-2}$alkylene$C_4$heterocycloalkyl, $C_{0-2}$alkylene$C_5$heterocycloalkyl, $C_{0-2}$alkylene$C_6$heterocycloalkyl, $C_0$alkylene$C_{3-6}$heterocycloalkyl, $C_1$alkylene$C_{3-6}$heterocycloalkyl and $C_2$alkylene$C_{3-6}$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) (such as one nitrogen atom) in the $C_{3-6}$heterocycloalkyl ring may be substituted, for example by $C_{1-4}$alkyl, C(O)H, C(O)$C_{1-4}$alkyl, C(O)$OC_{1-4}$alkyl, C(O)$OC_{1-4}$alkylaryl such as C(O)OBz, C(O)$NHC_{1-4}$alkyl, C(O)$NHC_{1-4}$alkylaryl such as C(O)NHBz, an Fmoc group, C(O)$C_{1-4}$haloalkyl, C(O)$OC_{1-4}$haloalkyl or C(O)$NHC_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkyl$OC_{1-2}$alkyl such as $C(O)CH_2OCH_3$, and $C_{1-2}$alkylC(O)$OC_{1-4}$alkyl such as $CH_2C(O)OCH_2CH_3$. Suitably, any nitrogen atom in the $C_{3-6}$heterocycloalkyl ring is not substituted. In a fifteenth embodiment, $R_{4bb}$ and $R_{5bb}$ together with the carbon atom to which they are attached form a $C_{3-6}$heterocycloalkyl, such as tetrahydrofuranyl, tetrahydropyranyl, pyrrolidinyl or piperidinyl, such as tetrahydrofuranyl or piperidinyl. If the $C_{3-6}$heterocycloalkyl group comprises (e.g. contains) a nitrogen atom, independently the nitrogen atom(s) may be unsubstituted (NH) or the nitrogen atom(s) may be substituted, for example substituted by a group selected from the following: $C_{1-4}$alkyl, C(O)H, C(O)$C_{1-4}$alkyl, C(O)$OC_{1-4}$alkyl, C(O)$OC_{1-4}$alkylaryl such as C(O)OBz, C(O)$NHC_{1-4}$alkyl, C(O)$NHC_{1-4}$alkylaryl such as C(O)NHBz, an Fmoc group, C(O)$C_{1-4}$haloalkyl, C(O)$OC_{1-4}$haloalkyl or C(O)$NHC_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkyl$OC_{1-2}$alkyl such as $C(O)CH_2OCH_3$, and $C_{1-2}$alkylC(O)$OC_{1-4}$alkyl such as $CH_2C(O)OCH_2CH_3$. In a fifteenth embodiment, $R_{4bb}$ is H and $R_{5bb}$ and $R_{6b}$ are a $C_{2-3}$alkylene chain forming a 5- or 6-membered ring. In a sixteenth

embodiment, $R_{4bb}$ is $OC_{0-2}$alkylene$C_{3-6}$heterocycloalkyl such as $OC_{0-2}$alkylene$C_3$heterocycloalkyl, $OC_{0-2}$alkylene$C_4$heterocycloalkyl, $OC_{0-2}$alkylene$C_5$heterocycloalkyl, $OC_{0-2}$alkylene$C_6$heterocycloalkyl, $OC_0$alkylene$C_{3-6}$heterocycloalkyl, $OC_1$alkylene$C_{3-6}$heterocycloalkyl and $OC_2$alkylene$C_{3-6}$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) (such as one nitrogen atom) in the $C_{3-6}$heterocycloalkyl ring may be substituted, for example by $C_{1-4}$alkyl, C(O)H, C(O)$C_{1-4}$alkyl, C(O)$OC_{1-4}$alkyl, C(O)$OC_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)$OC_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkyl$OC_{1-2}$alkyl such as C(O)$CH_2OCH_3$, and $C_{1-2}$alkylC(O)$OC_{1-4}$alkyl such as $CH_2C(O)OCH_2CH_3$. Suitably, any nitrogen atom in the $C_{3-6}$heterocycloalkyl ring is not substituted.

When $R_{4bb}$ is H and $R_{5bb}$ and $R_{6b}$ are a $C_{2-3}$alkylene chain forming a 5- or 6-membered ring, suitably a 5-membered ring, compounds comprising one of the following moieties are formed:

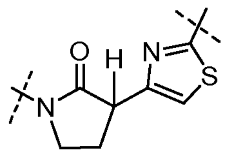 or 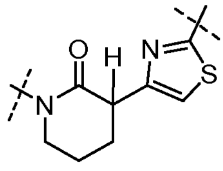 .

When $R_{4bb}$ is $NR_{21b}R_{22b}$, in one embodiment $R_{21b}$ is H. In a second embodiment $R_{21b}$ is $C_{1-5}$alkyl, such as methyl, ethyl or propyl, especially methyl. In a third embodiment $R_{21b}$ is C(O)$C_{1-5}$alkyl, such as C(O)$CH_3$. In a fourth embodiment $R_{21b}$ is C(O)$OC_{1-5}$alkyl, such as C(O)$OCH_3$ or C(O)Otert-butyl. In a fifth embodiment $R_{21b}$ is $C_{1-3}$alkylO-$C_{1-2}$alkyl such as $C_1$alkyl$OC_1$alkyl, $C_2$alkyl$OC_1$alkyl or $C_3$alkyl$OC_1$alkyl e.g. $C_2$alkyl$OC_1$alkyl. In a sixth embodiment, $R_{21b}$ is $C_{1-4}$haloalkyl, such as $CF_3$, $CH_2CF_3$ or $CH_2CHF_2$ e.g. $CH_2CHF_2$. In a seventh embodiment $R_{21b}$ is $C_{4-6}$heterocycloalkyl, such as oxetanyl, tetrahydrofuranyl or tetrahydropyranyl e.g. oxetanyl, in particular 3-oxetanyl.

When $R_{4bb}$ is $NR_{21b}R_{22b}$, in one embodiment $R_{22b}$ is H. In a second embodiment $R_{22b}$ is methyl.

Suitably $R_{4bb}$ is H, $CH_3$, ethyl, isopropyl, fluoro, $OCH_3$, isopropoxy or $CH_2CH_2OCH_3$, in particular H, $CH_3$, ethyl, fluoro, $OCH_3$, isopropoxy or $CH_2CH_2OCH_3$, especially H, $CH_3$, ethyl, $OCH_3$ or $CH_2CH_2OCH_3$.

Additionally of interest is when $R_{4bb}$ is $NH_2$, $N(CH_3)_2$, NHC(O)$CH_3$, NHC(O)$OCH_3$, NHC(O)Otert-butyl and $CH_2CH_2OH$, especially, $N(CH_3)_2$, NHC(O)$CH_3$, NHC(O)$OCH_3$.

Suitably, $R_{21b}$ is C(O)$OCH_3$ and $R_{22b}$ is H. Suitably, $R_{21b}$ is C(O)$CH_3$ and $R_{22b}$ is H. Suitably, $R_{21b}$ and $R_{22b}$ are both $CH_3$. Suitably, $R_{21b}$ and $R_{22b}$ are both H.

Consequently, suitably $R_{4bb}$ is H, $CH_3$, ethyl, isopropyl, fluoro, $OCH_3$, isopropoxy, $CH_2CH_2OCH_3$, $NH_2$, $N(CH_3)_2$, NHC(O)$CH_3$, NHC(O)$OCH_3$, NHC(O)Otert-butyl or $CH_2CH_2OH$, in particular H, $CH_3$, ethyl, fluoro, $OCH_3$, isopropoxy, $CH_2CH_2OCH_3$, $NH_2$, $N(CH_3)_2$, NHC(O)$CH_3$, NHC(O)$OCH_3$, NHC(O)Otert-butyl or $CH_2CH_2OH$, especially H, $CH_3$, ethyl, $OCH_3$, $CH_2CH_2OCH_3$, $N(CH_3)_2$, NHC(O)$CH_3$ or NHC(O)$OCH_3$.

Suitably $R_{4bb}$ may be C=O and $R_{5bb}$ is absent.

Suitably $R_{4bb}$ and $R_{5bb}$ together with the carbon atom to which they are attached form a cyclopropyl or cyclopentyl, in particular a cyclopentyl.

Suitably $R_{4bb}$ is H and $R_{3b}$ together with $R_{5bb}$ forms a 5- or 6-membered cycloalkyl, in particular a 5-membered cycloalkyl, especially $R_{4bb}$ is H and $R_{3b}$ together with $R_{5bb}$ forms a 5- or 6-membered cycloalkyl, such as a 5-membered cycloalkyl.

In one embodiment $R_{5bb}$ is $C_{1-6}$alkyl, in particular $C_{1-4}$alkyl, such as methyl, ethyl, propyl (n-propyl or isopropyl) or butyl (n-butyl, isobutyl, sec-butyl or tert-butyl). In a second embodiment $R_{5bb}$ is $C_{0-2}$alkylene$C_{3-6}$cycloalkyl, in particular $C_{0-2}$alkylene$C_{3-5}$cycloalkyl, such as $C_{3-5}$cycloalkyl, $C_1$alkylene$C_{3-5}$cycloalkyl or $C_2$alkylene$C_{3-5}$cycloalkyl. In a third embodiment $R_{5bb}$ is $OC_{1-6}$alkyl, in particular $OC_{1-4}$alkyl, such as methoxy, ethoxy, propoxy (n-propoxy or isopropoxy) or butoxy (n-butoxy, isobutoxy, sec-butoxy or tert-butoxy). In a fourth embodiment $R_{5bb}$ is $OC_{0-2}$alkylene$C_{3-6}$cycloalkyl, such as $OC_{3-6}$cycloalkyl, $OC_1$alkylene$C_{3-6}$cycloalkyl or $OC_2$alkylene$C_{3-6}$cycloalkyl. In a fifth embodiment $R_{5bb}$ is $C_{1-3}$alkylene$OC_{1-3}$alkyl, in particular $C_{1-2}$alkylene$OC_{1-2}$alkyl such as $C_1$alkylene$OC_1$alkyl, $C_2$alkylene$OC_1$alkyl, $C_1$alkylene$OC_2$alkyl or $C_2$alkylene$OC_2$alkyl. In a sixth embodiment $R_{5bb}$ is $C_{1-6}$haloalkyl, in particular $C_{1-4}$haloalkyl. In a seventh embodiment $R_{5bb}$ is $OC_{1-6}$haloalkyl, in particular $OC_{1-4}$haloalkyl. In an eighth embodiment $R_{5bb}$ is H. In a ninth embodiment $R_{5bb}$ is halo such as fluoro. In a tenth embodiment $R_{5bb}$ is $C_{1-6}$alkylOH, such as $CH_2OH$ or $CH_2CH_2OH$, in particular $CH_2CH_2OH$. In an eleventh embodiment $R_{5bb}$ is $NR_{21b}R_{22b}$. In a twelfth embodiment, $R_{5bb}$ is $C_{0-2}$alkylene$C_{3-6}$heterocycloalkyl such as $C_{0-2}$alkylene$C_3$heterocycloalkyl, $C_{0-2}$alkylene$C_4$heterocycloalkyl, $C_{0-2}$alkylene$C_5$heterocycloalkyl, $C_{0-2}$alkylene$C_6$heterocycloalkyl, $C_0$alkylene$C_{3-6}$heterocycloalkyl, $C_1$alkylene$C_{3-6}$heterocycloalkyl and $C_2$alkylene$C_{3-6}$heterocycloalkyl. Suitably the heterocycloalkyl is a heterocyclopropyl, heterocyclobutyl, heterocyclopentyl or

heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) in the $C_{3-6}$heterocycloalkyl ring may be substituted (such as one nitrogen atom is substituted), for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted. In a thirteenth embodiment, $R_{5bb}$ is O$C_{0-2}$alkylene-$C_{3-6}$heterocycloalkyl such as O$C_{0-2}$alkylene$C_3$heterocycloalkyl, O$C_{0-2}$alkylene$C_4$heterocycloalkyl, O$C_{0-2}$alkylene$C_5$heterocycloalkyl, O$C_{0-2}$alkylene$C_6$heterocycloalkyl, O$C_0$alkylene$C_{3-6}$heterocycloalkyl, O$C_1$alkylene$C_{3-6}$heterocycloalkyl and O$C_2$alkylene$C_{3-6}$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) (such as one nitrogen atom) in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO-$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted.

Suitably $R_{5bb}$ is H, $CH_3$, ethyl, isopropyl or fluoro, in particular $R_{5bb}$ is H, methyl or ethyl.

Suitably $R_{4bb}$ is H, $CH_3$, ethyl, fluoro, $OCH_3$, propoxy or $CH_2CH_2OCH_3$ and $R_{5bb}$ is H, $CH_3$, ethyl or fluoro, in particular $R_{4bb}$ is H, $CH_3$, ethyl or $OCH_3$ and $R_{5bb}$ is H, methyl or ethyl. For example, $R_{4bb}$ and $R_{5bb}$ are H, $R_{4bb}$ and $R_{5bb}$ are methyl, $R_{4bb}$ and $R_{5bb}$ are ethyl, $R_{4bb}$ is $CH_2CH_2OCH_3$ and $R_{5bb}$ is H or $R_{4bb}$ and $R_{5bb}$ are fluoro.

Suitably, when $R_{4bb}$ is other than H, methyl, ethyl or fluoro, then $R_{5bb}$ is H.

In one embodiment, $A_b$ is $A_{ab}$. Suitably, $A_{ab}$ is -$NR_{6b}CH_2$-. Alternatively, $A_{ab}$ is -$NR_{6b}$-.

In another embodiment, $A_b$ is $A_{bb}$ i.e. -$NR_{6b}$C(=O)-.

In one embodiment $R_{6b}$ is H. In a second embodiment $R_{6b}$ is $C_{1-3}$alkyl, in particular methyl. In a third embodiment $R_{6b}$ together with $R_{11b}$ in the ortho-position to group $A_b$ are a $C_2$alkylene chain forming a 5-membered ring. In a fourth embodiment, $R_{4bb}$ is H and $R_{5bb}$ and $R_{6b}$ are a $C_{2-3}$alkylene chain forming a 5- or 6-membered ring in particular a 5-membered ring.

Suitably $R_{6b}$ is H, methyl or $R_{6b}$ together with $R_{11b}$ when in the ortho-position to group $A_b$ are a $C_2$alkylene chain forming a 5-membered ring. In particular $R_{6b}$ is H or $R_{6b}$ together with $R_{11b}$ in the ortho-position to group $A_b$ are a $C_2$alkylene chain forming a 5-membered ring, especially $R_{6b}$ is H.

The term '$R_{6b}$ together with $R_{11b}$ in the ortho-position to group $A_b$ are a $C_2$alkylene chain forming a 5-membered ring' as used herein means that compounds with the following exemplary substructure are formed:

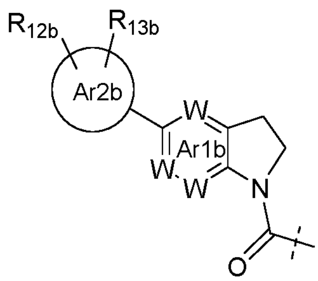

wherein W may be N or $CR_{10b}$.

In one embodiment Ar1b is a 6-membered aryl, i.e. phenyl. In a second embodiment Ar1b is a 6-membered heteroaryl, in particular containing one nitrogen atom (pyridyl) or two nitrogen atoms (pyridazinyl, pyrimidinyl or pyrazinyl).

In particular Ar1b is phenyl, 2-pyridyl, 3-pyridyl or 2,6-pyrimidinyl, especially phenyl, 2-pyridyl or 3-pyridyl, such as phenyl or 2-pyridyl.

In one embodiment $R_{10b}$ is H. In a second embodiment $R_{10b}$ is halo, for example fluoro or chloro. In a third embodiment $R_{10b}$ is $C_{1-3}$alkyl, such as $CH_3$, ethyl or isopropyl, in particular $C_{1-2}$alkyl, such as $CH_3$ or ethyl. In a fourth embodiment $R_{10b}$ is O$C_{1-2}$alkyl, such as $OCH_3$ or ethoxy. In a fifth embodiment $R_{10b}$ is $C_{1-2}$haloalkyl, such as $CF_3$. In a sixth embodiment $R_{10b}$ is O$C_{1-2}$haloalkyl, such as $OCF_3$. In a seventh embodiment $R_{10b}$ is CN.

Suitably $R_{10b}$ is H, fluoro, chloro, $CH_3$, $OCH_3$, ethoxy, $OCF_3$ or CN, in particular H, fluoro, chloro, $CH_3$, $OCH_3$, ethoxy or $OCF_3$, especially or H, fluoro, chloro, $CH_3$, $OCH_3$ or $OCF_3$, such as H, fluoro or $CH_3$.

Additionally of interest are compounds wherein $R_{10b}$ is ethyl, isopropyl and $CF_3$, in particular isopropyl and $CF_3$.

Additionally of interest are compounds when $R_{10b}$ is CN.

Consequently, suitably $R_{10b}$ is H, fluoro, chloro, $CH_3$, ethyl, isopropyl, $OCH_3$, ethoxy, $OCF_3$, $CF_3$ or CN, in particular H, fluoro, chloro, $CH_3$, isopropyl, $OCH_3$, ethoxy, $OCF_3$ or $CF_3$, especially or H, fluoro, chloro, $CH_3$, isopropyl, $OCH_3$, $OCF_3$ or $CF_3$, such as H, fluoro or $CH_3$.

In one embodiment $R_{11b}$ is H. In a second embodiment $R_{11b}$ is F. In a third embodiment, $R_{11b}$ is $CH_3$. In a fourth embodiment $R_{6b}$ together with $R_{11b}$ in the ortho-position to group $A_b$ are a $C_2$alkylene chain forming a 5-membered ring. In a fifth embodiment $R_{11b}$ is ethyl. In a sixth embodiment $R_{11b}$ is Cl. In a seventh embodiment $R_{11b}$ is $OCH_3$. In an eighth embodiment, $R_{11b}$ is $CF_3$. In a ninth embodiment, $R_{11b}$ is $OCF_3$. In a tenth embodiment, $R_{11b}$ is CN. In an eleventh embodiment $R_{6b}$ together with $R_{11b}$ in the ortho-position to group $A_b$ are a $C_2$alkylene chain forming a 5-membered ring.

Suitably, $R_{10b}$ and $R_{11b}$ are both $CH_3$. Suitably, $R_{10b}$ and $R_{11b}$ are both H. Suitably, $R_{10b}$ and $R_{11b}$ are both fluoro.

In one embodiment, $R_{10b}$ is in the ortho position with respect to group $A_b$. In another embodiment, $R_{10b}$ is in the meta position with respect to group $A_b$. Suitably $R_{10b}$ is in the ortho position with respect to group $A_b$.

In one embodiment, $R_{11b}$ is in the ortho position with respect to group $A_b$. In another embodiment, $R_{11b}$ is in the meta position with respect to group $A_b$. Suitably $R_{11b}$ is in the ortho position with respect to group $A_b$.

In one embodiment Ar2b is a 6-membered aryl, i.e. phenyl. In a second embodiment Ar2b is a 6-membered heteroaryl, in particular containing one nitrogen atom (pyridyl) or two nitrogen atoms (pyridazinyl, pyrimidinyl or pyrazinyl).

In particular Ar2b is phenyl, 2-pyridyl, 3-pyridyl, 4-pyridyl, 2,3-pyridazinyl, 3,4-pyridazinyl, 3,5-pyrimidinyl or 2,5-pyrazinyl, especially 3-pyridyl, 3,5-pyrimidinyl or 2,5-pyrazinyl, such as 3-pyridyl or 2,5-pyrazinyl.

In one embodiment $R_{12b}$ is H. In a second embodiment $R_{12b}$ is halo, for example fluoro or chloro. In a third embodiment $R_{12b}$ is $C_{1-4}$alkyl, such as methyl, ethyl, propyl (n-propyl or isopropyl) or butyl (n-butyl, isobutyl, sec-butyl or tert-butyl). In a fourth embodiment $R_{12b}$ is $C_{2-4}$alkynyl, such as C≡CH. In a fifth embodiment $R_{12b}$ is $C_{0-2}$alkylene$C_{3-5}$cycloalkyl, such as $C_{3-5}$cycloalkyl (e.g. cyclopropyl), $C_1$alkylene$C_{3-5}$cycloalkyl or $C_2$alkylene$C_{3-5}$cycloalkyl. In a sixth embodiment $R_{12b}$ is $OC_{1-4}$alkyl, such as $OCH_3$, ethoxy, isopropoxy or n-propoxy. In a seventh embodiment $R_{12b}$ is $OC_{0-2}$alkylene$C_{3-5}$cycloalkyl, such as $OC_{3-5}$cycloalkyl (e.g. cyclopropoxy or cyclobutoxy), $OC_1$alkylene$C_{3-5}$cycloalkyl or $OC_2$alkylene$C_{3-5}$cycloalkyl. In an eighth embodiment $R_{12b}$ is $OCH_2CH_2N(CH_3)_2$. In a ninth embodiment $R_{12b}$ is $C_{1-4}$alkylOH, such as $CH_2OH$ or $C(CH_3)_2OH$. In a tenth embodiment $R_{12b}$ is CN. In an eleventh embodiment $R_{12b}$ is $C_{1-3}$alkylene$OC_{1-3}$alkyl. In a twelfth embodiment $R_{12b}$ is $C_{1-4}$haloalkyl, such as $CF_3$. In a thirteenth embodiment $R_{12b}$ is $OC_{1-4}$haloalkyl, such as $OCF_3$, $OCHF_2$ or $OCH_2CF_3$. In a fourteenth embodiment $R_{12b}$ is $NR_{23b}R_{24b}$ such as $N(CH_3)_2$. In a fifteenth embodiment $R_{12b}$ is $S(O)_2C_{1-4}$alkyl such as $SO_2CH_3$. In a sixteenth embodiment $R_{12b}$ is $C(O)N(CH_3)_2$. In a seventeenth embodiment $R_{12b}$ is $NHC(O)C_{1-3}$alkyl such as $NHC(O)CH_3$. In an eighteenth embodiment $R_{12b}$ is a $C_{3-6}$heterocycloalkyl comprising one nitrogen located at the point of attachment to Ar2b, such as a $C_5$heterocycloalkyl, in particular pyrrolidinyl, or a $C_6$heterocycloalkyl such as morpholinyl. In a nineteenth embodiment $R_{12b}$ is OH. In a twentieth embodiment $R_{12b}$ is $C(=O)C_{1-2}$alkyl. In a twentyfirst embodiment $R_{12b}$ is $S(O)C_{1-4}$alkyl. In a twentysecond embodiment $R_{12b}$ is $SC_{1-4}$alkyl. In a twentythird embodiment $R_{12b}$ is SH. In a twentyfourth embodiment, $R_{12b}$ together with a nitrogen atom to which it is attached forms an N-oxide ($N^+$-$O^-$).

$R_{12b}$ is suitably H, fluoro, chloro, $CH_3$, cyclopropyl, C≡CH, $OCH_3$, ethoxy, n-propoxy, isopropoxy, cyclopropoxy, cyclobutoxy, CN, $CF_3$, $OCF_3$, $OCHF_2$, $OCH_2CF_3$, $CH_2OH$, $N(CH_3)_2$, $NHC(O)CH_3$, $SO_2CH_3$, $C(O)N(CH_3)_2$ or pyrrolidinyl, in particular H, fluoro, chloro, $CH_3$, cyclopropyl, C≡CH, $OCH_3$, ethoxy, n-propoxy, isopropoxy, cyclopropoxy, cyclobutoxy, CN, $CF_3$, $OCF_3$, $OCHF_2$, $OCH_2CF_3$, $CH_2OH$, $C(O)N(CH_3)_2$ or pyrrolidinyl, especially H, fluoro, chloro, $CH_3$, cyclopropyl, C≡CH, $OCH_3$, ethoxy, n-propoxy, isopropoxy, cyclopropoxy, CN, $CF_3$, $OCHF_2$, $OCH_2CF_3$ or pyrrolidinyl, such as H, fluoro, chloro, $CH_3$, C≡CH, $OCH_3$, ethoxy, n-propoxy, isopropoxy, cyclopropoxy, CN, $CF_3$, $OCHF_2$ or $OCH_2CF_3$.

Additionally of interest are ethyl, 2-methoxyisopropyl and OH, especially ethyl.

In one embodiment, $R_{23b}$ is H. In another embodiment, $R_{23b}$ is $C_{1-2}$alkyl such as methyl.

In one embodiment, $R_{24b}$ is H. In another embodiment $R_{24b}$ is $C_{1-2}$alkyl such as methyl.

Suitably, $R_{23b}$ is H and $R_{24b}$ is ethyl. Suitably, $R_{23b}$ is $CH_3$ and $R_{24b}$ is $CH_3$.

Consequently, suitably $R_{12b}$ is H, fluoro, chloro, $CH_3$, ethyl, cyclopropyl, C≡CH, $OCH_3$, ethoxy, *n*-propoxy, isopropoxy, cyclopropoxy, cyclobutoxy, CN, $CF_3$, $OCF_3$, $OCHF_2$, $OCH_2CF_3$, OH, $CH_2OH$, $N(CH_3)_2$, $NHC(O)CH_3$, $SO_2CH_3$, $C(O)N(CH_3)_2$ or pyrrolidinyl, in particular H, fluoro, chloro, $CH_3$, ethyl, cyclopropyl, C≡CH, $OCH_3$, ethoxy, n-propoxy, isopropoxy, cyclopropoxy, cyclobutoxy, CN, $CF_3$, $OCF_3$, $OCHF_2$, $OCH_2CF_3$, $CH_2OH$, $C(O)N(CH_3)_2$ or pyrrolidinyl, especially H, fluoro, chloro, $CH_3$, ethyl, cyclopropyl, C≡CH, $OCH_3$, ethoxy, n-propoxy, isopropoxy, cyclopropoxy, CN, $CF_3$, $OCHF_2$, $OCH_2CF_3$ or pyrrolidinyl, such as H, fluoro, chloro, $CH_3$, C≡CH, $OCH_3$, ethoxy, n-propoxy, isopropoxy, cyclopropoxy, CN, $CF_3$, $OCHF_2$ or $OCH_2CF_3$.

Suitably $R_{12b}$ is suitably in the meta position of Ar2b. Alternatively, $R_{12b}$ is in the ortho position of Ar2b.

In one embodiment $R_{13b}$ is methyl. In a second embodiment $R_{13b}$ is H. In a third embodiment $R_{13b}$ is methoxy. In a fourth embodiment $R_{13b}$ is halo such as fluoro.

In one embodiment, $R_{13b}$ is in the ortho position with respect to Ar1b. In another embodiment, $R_{13b}$ is in the para position with respect to Ar1b.

The present invention provides compound T466.

The present invention provides the following compound: N-(4-(1-((2-fluoro-4-(pyridin-3-yl)phenyl)amino)-2-methylpropan-2-yl)thiazol-2-yl)cyclopropanesulfonamide.

Compounds of formula (I-c)

The invention provides a compound of formula (I-c):

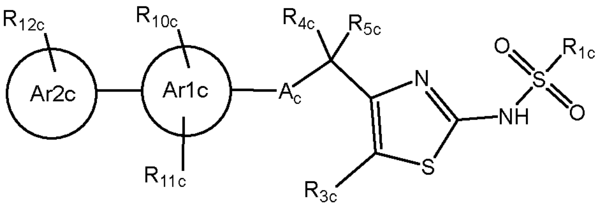

(I-c)

wherein

$A_c$ is $A_{ac}$ or $A_{bc}$;

$A_{ac}$ is $-CH_2NR_{6c}-$;

$A_{bc}$ is $-C(=O)NR_{6c}-$;

$R_{1c}$ is $R_{1ac}$ or $R_{1bc}$;

wherein:
$R_{1ac}$ is $NR_{32c}R_{33c}$;

$R_{1bc}$ is $C_{1-5}$alkyl, $C_{0-2}$alkylene$C_{3-5}$cycloalkyl which cycloalkyl is optionally substituted by $CH_3$, $C_{1-3}$alkyleneO$C_{1-2}$alkyl, or $CF_3$;

$R_{3c}$ is H, $CH_3$, halo, O$C_{1-2}$alkyl or $CF_3$;

$R_{4c}$ and $R_{5c}$ are either $R_{4ac}$ and $R_{5ac}$ or $R_{4bc}$ and $R_{5bc}$;

wherein:

$R_{4ac}$ and $R_{5ac}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and $NR_{21c}R_{22c}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ac}$ and $R_{5ac}$ together with the carbon atom to which they are attached may be substituted by one or two substituents, each substituent being independently selected from the group consisting of $C_{1-3}$alkyl or O$C_{1-3}$alkyl; or

$R_{4ac}$ and $R_{5ac}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ac}$ and $R_{5ac}$ together with the carbon atom to which they are attached may be substituted by one or two substituents, each substituent being independently selected from the group consisting of $C_{1-3}$alkyl or O$C_{1-3}$alkyl; or

$R_{4ac}$ and $R_{5ac}$ together with the carbon atom to which they are attached form a $C_{3-6}$heterocycloalkyl comprising

one nitrogen atom, wherein said nitrogen atom is substituted by $-S(O)_2R_{29c}$; or

$R_{4bc}$ and $R_{5bc}$ are each independently H, $C_{1-6}$alkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, $C_{1-6}$alkylOH or $C_{1-6}$haloalkyl,

or $R_{4bc}$ and $R_{5bc}$ together with the carbon atom to which they are attached form a $C_{3-6}$cycloalkyl or $C_{3-6}$heterocycloalkyl ring;

$R_{6c}$ is H or $C_{1-3}$alkyl;

Ar1c is a 6-membered aryl or heteroaryl;

Ar2c is a 6-membered aryl or heteroaryl and is attached to Ar1c in the para position relative to group $A_c$;

$R_{10c}$ is H, halo, $C_{1-3}$alkyl, $OC_{1-2}$alkyl, $C_{1-2}$haloalkyl, $OC_{1-2}$haloalkyl or CN;

$R_{11c}$ is H, F, Cl, $CH_3$, ethyl, $OCH_3$, $CF_3$, $OCF_3$ or CN;

$R_{12c}$ is attached to Ar2c in the meta or ortho position relative to Ar1c and $R_{12c}$ is H, halo, $C_{1-4}$alkyl, $C_{2-4}$alkynyl, C(=O)$C_{1-2}$alkyl, $C_{0-2}$alkylene$C_{3-5}$cycloalkyl, $OC_{1-4}$alkyl, $C_{1-3}$alkylene$OC_{1-3}$alkyl, $C_{1-4}$haloalkyl, $OC_{1-4}$haloalkyl, CN, $OC_{0-2}$alkylene$C_{3-5}$cycloalkyl, $OCH_2CH_2N(CH_3)_2$, OH, $C_{1-4}$alkylOH, $NR_{23c}R_{24c}$, $SO_2CH_3$, $C(O)N(CH_3)_2$, NHC(O)$C_{1-3}$alkyl, or a $C_{3-6}$heterocycloalkyl comprising one nitrogen located at the point of attachment to Ar2c, or $R_{12c}$ together with a nitrogen atom to which it is attached forms an N-oxide ($N^+$-$O^-$);

$R_{21c}$ is H, $C_{1-5}$alkyl, C(O)$C_{1-5}$alkyl, C(O)$OC_{1-5}$alkyl, $C_{1-3}$alkyl$OC_{1-2}$alkyl, $C_{1-4}$haloalkyl, or $C_{4-6}$heterocycloalkyl;

$R_{22c}$ is H or $CH_3$;

$R_{23c}$ is H or $C_{1-2}$alkyl;

$R_{24c}$ is H or $C_{1-2}$alkyl;

$R_{29c}$ is $C_{1-3}$alkyl, $C_{0-2}$alkylene$C_{3-5}$cycloalkyl which cycloalkyl is optionally substituted by $CH_3$, $CF_3$, $N(C_{1-3}alkyl)_2$, or a 5 or 6 membered heteroaryl wherein the 5 or 6 membered heteroaryl is optionally substituted by methyl; and

$R_{32c}$ is $C_{1-3}$alkyl and $R_{33c}$ is $C_{1-3}$alkyl; or

$R_{32c}$ and $R_{33c}$ together with the nitrogen atom to which they are attached form a $C_{3-5}$heterocycloalkyl;

or a salt and/or solvate thereof.

Suitably, $R_{1c}$ is $R_{1ac}$; and/or $R_{4c}$ and $R_{5c}$ are $R_{4ac}$ and $R_{5ac}$; and/or $A_c$ is $A_{ac}$.

The invention also provides a compound of formula (I-c):

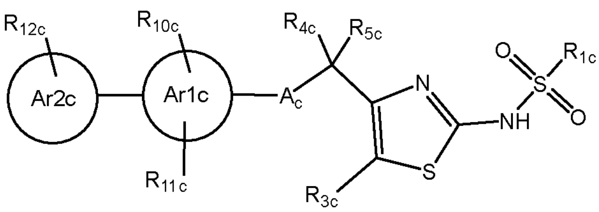

(I-c)

wherein

$A_c$ is $A_{ac}$ or $A_{bc}$;

$A_{ac}$ is $-CH_2NR_{6c}-$;
$A_{bc}$ is $-C(=O)NR_{6c}-$;

$R_{1c}$ is $R_{1ac}$ or $R_{1bc}$;

wherein:
$R_{1ac}$ is $NR_{32c}R_{33c}$;

$R_{1bc}$ is $C_{1-5}$alkyl, $C_{0-2}$alkylene$C_{3-5}$cycloalkyl which cycloalkyl is optionally substituted by $CH_3$, $C_{1-3}$alkylene$OC_{1-2}$alkyl, or $CF_3$;

$R_{3c}$ is H, $CH_3$, halo, $OC_{1-2}$alkyl or $CF_3$;

$R_{4c}$ and $R_{5c}$ are either $R_{4ac}$ and $R_{5ac}$ or $R_{4bc}$ and $R_{5bc}$;

wherein:

$R_{4ac}$ and $R_{5ac}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene-$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21c}R_{22c}$; or

one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ac}$ and $R_{5ac}$ together with the carbon atom to which they are attached may be substituted by one or two substituents, each substituent being independently selected from the group consisting of $C_{1-3}$alkyl or $OC_{1-3}$alkyl; or

$R_{4ac}$ and $R_{5ac}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ac}$ and $R_{5ac}$ together with the carbon atom to which they are attached may be substituted by one or two substituents, each substituent being independently selected from the group consisting of $C_{1-3}$alkyl or $OC_{1-3}$alkyl; or

$R_{4ac}$ and $R_{5ac}$ together with the carbon atom to which they are attached form a $C_{3-6}$heterocycloalkyl comprising one nitrogen atom, wherein said nitrogen atom is substituted by $-S(O)_2R_{29c}$; or

$R_{4bc}$ and $R_{5bc}$ are each independently H, $C_{1-6}$alkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, $C_{1-6}$alkylOH or $C_{1-6}$haloalkyl,

or $R_{4bc}$ and $R_{5bc}$ together with the carbon atom to which they are attached form a $C_{3-6}$cycloalkyl or $C_{3-6}$heterocycloalkyl ring;

$R_{6c}$ is H or $C_{1-3}$alkyl;

Ar1c is a 6-membered aryl or heteroaryl;

Ar2c is a 6-membered aryl or heteroaryl and is attached to Ar1c in the para position relative to group $A_c$;

$R_{10c}$ is H, halo, $C_{1-3}$alkyl, $OC_{1-2}$alkyl, $C_{1-2}$haloalkyl, $OC_{1-2}$haloalkyl or CN;

$R_{11c}$ is H, F, Cl, $CH_3$, ethyl, $OCH_3$, $CF_3$, $OCF_3$ or CN;

$R_{12c}$ is attached to Ar2c in the meta or ortho position relative to Ar1c and $R_{12c}$ is H, halo, $C_{1-4}$alkyl, $C_{2-4}$alkynyl, C(=O)$C_{1-2}$alkyl, $C_{0-2}$alkylene$C_{3-5}$cycloalkyl, $OC_{1-4}$alkyl, $C_{1-3}$alkylene$OC_{1-3}$alkyl, $C_{1-4}$haloalkyl, $OC_{1-4}$haloalkyl, CN, $OC_{0-2}$alkylene$C_{3-5}$cycloalkyl, $OCH_2CH_2N(CH_3)_2$, OH, $C_{1-4}$alkylOH, $NR_{23c}R_{24c}$, $SO_2CH_3$, $C(O)N(CH_3)_2$, NHC(O)$C_{1-3}$alkyl, or a $C_{3-6}$heterocycloalkyl comprising one nitrogen located at the point of attachment to Ar2c, or $R_{12c}$ together with a nitrogen atom to which it is attached forms an N-oxide ($N^+$-$O^-$);

$R_{21c}$ is H, $C_{1-5}$alkyl, C(O)$C_{1-5}$alkyl, C(0)O$C_{1-5}$alkyl;

$R_{22c}$ is H or $CH_3$;

$R_{23c}$ is H or $C_{1-2}$alkyl;

$R_{24c}$ is H or $C_{1-2}$alkyl;

$R_{29c}$ is $C_{1-3}$alkyl, $C_{0-2}$alkylene$C_{3-5}$cycloalkyl which cycloalkyl is optionally substituted by $CH_3$, or $CF_3$; and

$R_{32c}$ is $C_{1-3}$alkyl and $R_{33c}$ is $C_{1-3}$alkyl; or

$R_{32c}$ and $R_{33c}$ together with the nitrogen atom to which they are attached form a $C_{3-5}$heterocycloalkyl;

or a salt and/or solvate thereof.

Suitably, $R_{1c}$ is $R_{1ac}$; and/or $R_{4c}$ and $R_{5c}$ are $R_{4ac}$ and $R_{5ac}$; and/or $A_c$ is $A_{ac}$.

When $R_{4bc}$ and/or $R_{5bc}$ is $C_0$alkylene$C_{3-6}$heterocycloalkyl, any heteroatom in the heterocycloalkyl may not be directly connected to the carbon to which $R_{4bc}$ and $R_{5bc}$ are connected.

In one embodiment of the invention, $R_{1c}$ is $R_{1ac}$, i.e. is $NR_{32c}R_{33c}$. In an embodiment, $R_{32c}$ is $C_{1-3}$alkyl, such as methyl or ethyl, e.g. methyl. In an embodiment, $R_{33c}$ is $C_{1-3}$alkyl, such as methyl or ethyl, e.g. methyl. Suitably, $R_{32c}$ and $R_{33c}$ are both methyl. Suitably, $R_{32c}$ and $R_{33c}$ are both ethyl. Suitably, $R_{32c}$ is methyl and $R_{33c}$ is ethyl.

In another embodiment, $R_{32c}$ and $R_{33c}$ together with the nitrogen atom to which they are attached form a $C_{3-5}$heterocycloalkyl. Suitably, the $C_{3-5}$heterocycloalkyl is aziridinyl, azetidinyl or pyrrolidinyl.

Suitably, $R_{1c}$ is $R_{1bc}$.

In one embodiment of the invention $R_{1bc}$ is $C_{1-5}$alkyl such as $C_{1-4}$alkyl. When $R_{1bc}$ is $C_{1-5}$alkyl, $R_{1bc}$ is methyl, ethyl, propyl (n-propyl or isopropyl), butyl (n-butyl, isobutyl, sec-butyl or tert-butyl) or pentyl (e.g. n-pentyl, sec-pentyl, 3-pentyl, sec-isopentyl or active pentyl). When $R_{1bc}$ is $C_{1-4}$alkyl, $R_{1bc}$ is methyl, ethyl, propyl (n-propyl or isopropyl) or butyl (n-butyl, isobutyl, sec-butyl or tert-butyl).

In a second embodiment of the invention $R_{1bc}$ is $C_{1-3}$alkyleneO$C_{1-2}$alkyl such as $C_{1-2}$alkyleneO$C_{1-2}$alkyl. $R_{1bc}$ may be $C_1$alkyleneO$C_1$alkyl. $R_{1bc}$ may be $C_1$alkyleneO$C_2$alkyl. $R_{1bc}$ may be $C_2$alkyleneO$C_1$alkyl. $R_{1bc}$ may be $C_2$alkyleneO$C_2$alkyl. $R_{1bc}$ may be $C_3$alkyleneO$C_1$alkyl. $R_{1bc}$ may be $C_3$alkyleneO$C_2$alkyl.

In a third embodiment of the invention $R_{1bc}$ is $C_{0-2}$alkylene$C_{3-5}$cycloalkyl which cycloalkyl is optionally substituted by $CH_3$ such as $C_{0-1}$alkylene$C_{3-4}$cycloalkyl which cycloalkyl is optionally substituted by $CH_3$. In some embodiments, $R_{1bc}$ is $C_{0-2}$alkylene$C_{3-5}$cycloalkyl such as $C_{0-1}$alkylene$C_{3-4}$cycloalkyl. In other embodiments, $R_{1bc}$ is $C_{0-2}$alkylene$C_{3-5}$cycloalkyl which cycloalkyl is substituted by $CH_3$ such as $C_{0-1}$alkylene$C_{3-4}$cycloalkyl which cycloalkyl is substituted by $CH_3$. $R_{1bc}$ may be $C_{3-5}$cycloalkyl, which cycloalkyl is optionally substituted by $CH_3$ such as $C_{3-4}$cycloalkyl, which cycloalkyl is optionally substituted by $CH_3$. $R_{1bc}$ may be $C_1$alkylene$C_{3-5}$cycloalkyl, which cycloalkyl is optionally substituted by $CH_3$. $R_{1bc}$ may be $C_1$alkylene$C_{3-4}$cycloalkyl, which cycloalkyl is optionally substituted by $CH_3$. $R_{1bc}$ may be $C_2$alkylene$C_{3-5}$cycloalkyl, which cycloalkyl is optionally substituted by $CH_3$. $R_{1bc}$ may be $C_2$alkylene$C_{3-4}$cycloalkyl, which cycloalkyl is optionally substituted by $CH_3$. $R_{1bc}$ may be $C_{0-2}$alkylene$C_3$cycloalkyl, which cycloalkyl is optionally substituted by $CH_3$. $R_{1bc}$ may be $C_{0-1}$alkylene$C_3$cycloalkyl, which cycloalkyl is optionally substituted by $CH_3$. $R_{1bc}$ may be $C_{0-2}$alkylene$C_4$cycloalkyl, which cycloalkyl is optionally substituted by $CH_3$. $R_{1bc}$ may be $C_{0-1}$alkylene$C_4$cycloalkyl, which cycloalkyl is optionally substituted by $CH_3$. $R_{1bc}$ may be $C_{0-2}$alkylene$C_5$cycloalkyl, which cycloalkyl is optionally substituted by $CH_3$. $R_{1bc}$ may be $C_{0-1}$alkylene$C_5$cycloalkyl, which cycloalkyl is optionally substituted by $CH_3$. Suitably, where $C_{0-2}$alkylene$C_{3-5}$cycloalkyl such as $C_{0-1}$alkylene$C_{3-4}$cycloalkyl is optionally substituted by $CH_3$, the $CH_3$ is at the point of attachment of the $C_{3-5}$cycloalkyl to the $C_{0-2}$alkylene such as at the point of attachment of the $C_{3-4}$cycloalkyl to the $C_{0-1}$alkylene.

Suitably $R_{1bc}$ is cyclopropyl.

In a fourth embodiment of the invention, $R_{1bc}$ is $CF_3$.

In one embodiment $R_{3c}$ is H. In a second embodiment $R_{3c}$ is $CH_3$. In a third embodiment, $R_{3c}$ is halo. In an example, $R_{3c}$ is F. In a second example, $R_{3c}$ is Cl. In a fourth embodiment, $R_{3c}$ is O$C_{1-2}$alkyl. Suitably $R_{3c}$ is $OCH_3$. Suitably, $R_{3c}$ is $OCH_2CH_3$. In a fifth embodiment, $R_{3c}$ is $CF_3$.

Suitably, $R_{3c}$ is H.

In one embodiment, $R_{4c}$ and $R_{5c}$ are $R_{4ac}$ and $R_{5ac}$.

Suitably, $R_{4ac}$ and $R_{5ac}$ together with the carbon atom to which they are attached form a $C_{3-6}$cycloalkyl which is: substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and

$NR_{21c}R_{22c}$.

In one embodiment, the $C_{3-6}$cycloalkyl is cyclopropyl. In another embodiment, the $C_{3-6}$cycloalkyl is cyclobutyl. In another embodiment, the $C_{3-6}$cycloalkyl is cyclopentyl. In another embodiment, the $C_{3-6}$cycloalkyl is cyclohexyl.

In one embodiment the $C_{3-6}$cycloalkyl is substituted by one substituent. In a second embodiment the $C_{3-6}$cycloalkyl is substituted by two substituents.

In one embodiment, the substituent is $C_{1-3}$alkyl. Suitably, the substituent is methyl. Suitably, the substituent is ethyl. Suitably, the substituent is n-propyl. Suitably, the substituent is iso-propyl.

In a second embodiment, the substituent is $C_{1-3}$alkylOH. Suitably, the substituent is $CH_2OH$. Suitably, the substituent is $CH_2CH_2OH$. Suitably, the substituent is $CH_2CH_2CH_2OH$.

In a third embodiment, the substituent is $C_{1-3}$haloalkyl. Suitably the $C_{1-3}$alkyl group is substituted by one two or three, such as one, halogen atom. Suitably, the halogen atom is fluoro or chloro such as fluoro. Suitably, the substituent is $C_1$haloalkyl such as $CF_3$. Suitably, the substituent is $C_2$haloalkyl such as $CH_2CF_3$.

In a fourth embodiment, the substituent is $C_{0-2}$alkylene$C_{3-6}$cycloalkyl, in particular $C_{0-2}$alkylene$C_{3-5}$cycloalkyl, such as $C_{3-5}$cycloalkyl, $C_1$alkylene$C_{3-5}$cycloalkyl or $C_2$alkylene$C_{3-5}$cycloalkyl.

In a fifth embodiment, the substituent is $C_{0-2}$alkylene$C_{3-6}$heterocycloalkyl such as $C_{0-2}$alkylene$C_3$heterocycloalkyl, $C_{0-2}$alkylene$C_4$heterocycloalkyl, $C_{0-2}$alkylene$C_5$heterocycloalkyl, $C_{0-2}$alkylene$C_6$heterocycloalkyl, $C_0$alkylene-$C_{3-6}$heterocycloalkyl, $C_1$alkylene$C_{3-6}$heterocycloalkyl and $C_2$alkylene$C_{3-6}$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) in the $C_{3-6}$heterocycloalkyl ring may be substituted (such as one nitrogen atom is substituted), for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted.

In a sixth embodiment, the substituent is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl.

In a seventh embodiment, the substituent is halo, in particular fluoro or chloro such as chloro.

In an eighth embodiment, the substituent is O$C_{1-3}$haloalkyl. Suitably the O$C_{1-3}$alkyl group is substituted by one two or three, such as one, halogen atom. Suitably, the halogen atom is fluoro or chloro such as fluoro. Suitably, the substituent is O$C_1$haloalkyl such as $OCF_3$. Suitably, the substituent is O$C_2$haloalkyl such as $OCH_2CF_3$.

In a ninth embodiment, the substituent is O$C_{0-2}$alkylene$C_{3-6}$cycloalkyl, such as O$C_{3-6}$cycloalkyl, O$C_1$alkylene$C_{3-6}$cycloalkyl or O$C_2$alkylene$C_{3-6}$cycloalkyl.

In a tenth embodiment, the substituent is O$C_{0-2}$alkylene$C_{3-6}$heterocycloalkyl such as O$C_{0-2}$alkylene$C_3$heterocycloalkyl, O$C_{0-2}$alkylene$C_4$heterocycloalkyl, O$C_{0-2}$alkylene$C_5$heterocycloalkyl, O$C_{0-2}$alkylene$C_6$heterocycloalkyl, O$C_0$alkylene$C_{3-6}$heterocycloalkyl, O$C_1$alkylene$C_{3-6}$heterocycloalkyl and O$C_2$alkylene$C_{3-6}$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom(s) (such as one nitrogen atom) in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted.

In an eleventh embodiment, the substituent is O$C_{1-3}$alkyl, such as $OCH_3$ or $OCH_2CH_3$.

In a twelfth embodiment, the substituent is $NR_{21c}R_{22c}$. In one embodiment $R_{21c}$ is H. In a second embodiment $R_{21c}$ is $C_{1-5}$alkyl, such as methyl, ethyl or propyl, especially methyl. In a third embodiment $R_{21c}$ is C(O)$C_{1-5}$alkyl, such as C(O)$CH_3$. In a fourth embodiment $R_{21c}$ is C(O)O$C_{1-5}$alkyl, such as C(O)$OCH_3$ or C(O)Otert-butyl. In a fifth embodiment $R_{21c}$ is $C_{1-3}$alkylO$C_{1-2}$alkyl such as $C_1$alkylO$C_1$alkyl, $C_2$alkylO$C_1$alkyl or $C_3$alkylO$C_1$alkyl e.g. $C_2$alkylO$C_1$alkyl. In a sixth embodiment, $R_{21c}$ is $C_{1-4}$haloalkyl, such as $CF_3$, $CH_2CF_3$ or $CH_2CHF_2$ e.g. $CH_2CHF_2$. In a seventh embodiment $R_{21c}$ is $C_{4-6}$heterocycloalkyl, such as oxetanyl, tetrahydrofuranyl or tetrahydropyranyl e.g. oxetanyl, in particular 3-oxetanyl.

When the substituent is $NR_{21c}R_{22c}$, in one embodiment $R_{22c}$ is H. In a second embodiment $R_{22c}$ is methyl.

Suitably, $R_{21c}$ is C(O)$OCH_3$ and $R_{22c}$ is H. Suitably, $R_{21c}$ is C(O)$CH_3$ and $R_{22c}$ is H. Suitably, $R_{21c}$ and $R_{22c}$ are both $CH_3$. Suitably, $R_{21c}$ and $R_{22c}$ are both H.

In a thirteenth embodiment, the substituent is oxo.

In a fourteenth embodiment, the substituent is OH.

Suitably, the one or two substituents, in particular one substituent, are independently selected from the group

consisting of $C_{1-3}$alkyl, oxo, OH, $C_{1-3}$alkylOH, $C_{1-3}$haloalkyl, halo, $OC_{1-3}$haloalkyl, $OC_{1-3}$alkyl and $NR_{21c}R_{22c}$.

More suitably, the substituent is independently selected from the group consisting of oxo, OH, halo, $OC_{1-3}$alkyl and $NR_{21c}R_{22c}$.

Most suitably, the substituent is independently selected from the group consisting of oxo, OH, fluoro, $NR_{21c}R_{22c}$.

Alternatively, $R_{4ac}$ and $R_{5ac}$ together with the carbon atom to which they are attached form a $C_{3-6}$cycloalkyl and one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ac}$ and $R_{5ac}$ together with the carbon atom to which they are attached may be substituted by one or two substituents, each substituent being independently selected from the group consisting of $C_{1-3}$alkyl or $OC_{1-3}$alkyl.

In one embodiment the $C_{3-6}$cycloalkyl formed by $R_{4ac}$ and $R_{5ac}$ together with the carbon atom to which they are attached is unsubstituted. In a second embodiment the $C_{3-6}$cycloalkyl formed by $R_{4ac}$ and $R_{5ac}$ together with the carbon atom to which they are attached is substituted by one or two substituents, in particular one substituent. Suitably, each substituent is independently selected from the group consisting of $C_{1-2}$alkyl or $OCH_3$.

Suitably one of the carbons of the $C_{3-6}$cycloalkyl which is formed by $R_{4ac}$ and $R_{5ac}$ is a spiro centre such that a spirocyclic ring system is formed, wherein the $C_{3-6}$cycloalkyl which is formed by $R_{4ac}$ and $R_{5ac}$ is a $C_{4-6}$cycloalkyl. Suitably the $C_{3-6}$heterocycloalkyl is an oxygen containing $C_{3-6}$heterocycloalkyl. Suitably, the $C_{3-6}$heterocycloalkyl is an oxygen comprising, such as containing, $C_{3-6}$heterocycloalkyl ring, such as a $C_5$cycloalkyl ring.

In an embodiment, $R_{4ac}$ and $R_{5ac}$ together with the carbon atom to which they are attached form a $C_{3-6}$heterocycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ac}$ and $R_{5ac}$ together with the carbon atom to which they are attached may be substituted by one or two substituents, each substituent being independently selected from the group consisting of $C_{1-3}$alkyl or $OC_{1-3}$alkyl. Suitably, each substituent is independently selected from the group consisting of $C_{1-2}$alkyl or $OCH_3$.

Suitably one of the carbons of the $C_{3-6}$heterocycloalkyl is a spiro centre such that a spirocyclic ring system is formed containing further ring C, wherein C is a $C_{4-6}$heterocycloalkyl. Suitably the $C_{4-6}$heterocycloalkyl is an oxygen containing $C_{4-6}$heterocycloalkyl such as tetrahydropyranyl or 1,3-dioxolanyl.

As stated above, when a heterocycloalkyl is formed from $R_{4c}$ and $R_{5c}$ together with the carbon atom to which they are attached, suitably any heteroatom is not directly connected to the carbon to which $R_{4c}$ and $R_{5c}$ are attached.

In an embodiment, $R_{4ac}$ and $R_{5ac}$ together with the carbon atom to which they are attached form a $C_{3-6}$heterocycloalkyl comprising one nitrogen atom, wherein said nitrogen atom is substituted by $-S(O)_2R_{29c}$.

Suitably, the $C_{3-6}$heterocycloalkyl is selected from the group consisting of aziridinyl, azetidinyl, pyrrolidinyl and piperidinyl such as piperidinyl.

Suitably, when the $C_{3-6}$heterocycloalkyl is piperidinyl, the nitrogen atom is in the 4-position relative to the quaternary carbon:

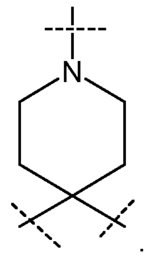

The $C_{3-6}$heterocycloalkyl may be other groups as defined elsewhere herein.

In an embodiment, $R_{29c}$ is $C_{1-3}$alkyl, $C_{0-2}$alkylene$C_{3-5}$cycloalkyl which cycloalkyl is optionally substituted by $CH_3$, or $CF_3$. In one embodiment, $R_{29c}$ is $C_{1-3}$alkyl such as methyl. In another embodiment, $R_{29c}$ is $C_{0-2}$alkylene$C_{3-5}$cycloalkyl which cycloalkyl is optionally substituted by $CH_3$. In some embodiments, $R_{29c}$ is $C_{0-2}$alkylene$C_{3-5}$cycloalkyl. In other embodiments, $R_{29c}$ is $C_{0-2}$alkylene$C_{3-5}$cycloalkyl which cycloalkyl is substituted by $CH_3$. $R_{29c}$ may be $C_{3-5}$cycloalkyl, which cycloalkyl is optionally substituted by $CH_3$. $R_{29c}$ may be $C_1$alkylene$C_{3-5}$cycloalkyl, which cycloalkyl is optionally substituted by $CH_3$. $R_{29c}$ may be $C_2$alkylene$C_{3-5}$cycloalkyl, which cycloalkyl is optionally substituted by $CH_3$. $R_{29c}$ may be $C_{0-2}$alkylene$C_3$cycloalkyl, which cycloalkyl is optionally substituted by $CH_3$. $R_{29c}$ may be $C_{0-2}$alkylene$C_4$cycloalkyl, which cycloalkyl is optionally substituted by $CH_3$. $R_{29c}$ may be $C_{0-2}$alkylene$C_5$cycloalkyl, which cycloalkyl is optionally substituted by $CH_3$. Suitably, where $C_{0-2}$alkylene$C_{3-5}$cycloalkyl is optionally substituted by $CH_3$, the $CH_3$ is at the point of attachment of the $C_{3-5}$cycloalkyl to the $C_{0-2}$alkylene. In another embodiment, $R_{29c}$ is $CF_3$. In another embodiment, $R_{29c}$ is $N(C_{1-3}alkyl)_2$ such as $N(CH_3)_2$. In another embodiment, $R_{29c}$ is a 5 or 6 membered heteroaryl wherein the 5 or 6 membered heteroaryl is optionally substituted by methyl. In one embodiment, the 5 or 6 membered heteroaryl is substituted by methyl. In one embodiment, the 5 or 6 membered heteroaryl is not substituted by methyl. In one embodiment, $R_{29c}$ is a 5-membered heteroaryl such as pyrrolyl, furanyl, thiophenyl, imidazolyl, pyrazolyl, oxazolyl, isoxazolyl, thiazolyl, isothiazolyl, such as pyrazolyl. Suitably the pyrazolyl is substituted by methyl. In another embodiment, $R_{29c}$ is a 6-membered heteroaryl such as pyridinyl, pyridazinyl, pyrimidinyl or pyrazinyl.

In another embodiment, $R_{4c}$ and $R_{5c}$ are $R_{4bc}$ and $R_{5bc}$.

In one embodiment, $R_{4bc}$ is H. In a second embodiment $R_{4bc}$ is $C_{1-6}$alkyl such as $C_{1-4}$alkyl, i.e. methyl, ethyl, propyl (n-propyl or isopropyl) or butyl (n-butyl, isobutyl, sec-butyl or tert-butyl). $R_{4bc}$ may also be pentyl (e.g. n-pentyl, sec-pentyl, 3-pentyl, sec-isopentyl or active pentyl) or hexyl (e.g. n-hexyl, 2-methylpentyl, 3-methylpentyl, 2,2-dimethylbutyl and 2,3-dimethylbutyl). In a third embodiment, $R_{4bc}$ is $C_{0-2}$alkylene$C_{3-6}$cycloalkyl such as $C_{0-2}$alkylene$C_{3-5}$cycloalkyl, such as $C_{0-2}$alkylene$C_3$cycloalkyl, $C_{0-2}$alkylene$C_4$cycloalkyl, $C_{0-2}$alkylene$C_5$cycloalkyl, $C_0$alkylene$C_{3-5}$cycloalkyl, $C_1$alkylene$C_{3-5}$cycloalkyl and $C_2$alkylene$C_{3-5}$cycloalkyl. $R_{4bc}$ may also be $C_{0-2}$alkylene$C_6$cycloalkyl, $C_0$alkylene$C_{3-6}$cycloalkyl, $C_1$alkylene$C_{3-6}$cycloalkyl and $C_2$alkylene$C_{3-6}$cycloalkyl. In a fourth embodiment $R_{4bc}$ is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a fifth embodiment $R_{4bc}$ is $C_{1-6}$alkylOH such as $C_{1-4}$alkylOH such as $C_1$alkylOH, $C_2$alkylOH, $C_3$alkylOH or $C_4$alkylOH wherein $C_{1-4}$alkyl is methyl, ethyl, propyl (n-propyl or isopropyl) and butyl (n-butyl, isobutyl, sec-butyl or tert-butyl). $R_{4bc}$ may also be $C_5$alkylOH or $C_6$alkylOH. In a sixth embodiment, $R_{4bc}$ is $C_{1-6}$haloalkyl such as $C_{1-4}$haloalkyl such as $C_1$haloalkyl (e.g. $CF_3$), $C_2$haloalkyl (e.g. $CH_2CF_3$), $C_3$haloalkyl (e.g. $CH_2CH_2CF_3$) or $C_4$haloalkyl (e.g. $CH_2CH_2CH_2CF_3$). $R_{4bc}$ may also be $C_5$haloalkyl (e.g. $CH_2CH_2CH_2CH_2CF_3$) or $C_6$haloalkyl (e.g. $CH_2CH_2CH_2CH_2CH_2CF_3$). In a seventh embodiment, $R_{4bc}$ is $C_{0-2}$alkylene$C_{3-6}$heterocycloalkyl such as $C_{0-2}$alkylene$C_3$heterocycloalkyl, $C_{0-2}$alkylene$C_4$heterocycloalkyl, $C_{0-2}$alkylene$C_5$heterocycloalkyl, $C_{0-2}$alkylene$C_6$heterocycloalkyl, $C_0$alkylene$C_{3-6}$heterocycloalkyl, $C_1$alkylene$C_{3-6}$heterocycloalkyl and $C_2$alkylene$C_{3-6}$heterocycloalkyl. Suitably the heterocycloalkyl of a $C_{0-2}$alkylene$C_{3-6}$heterocycloalkyl group is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted. In an eighth embodiment, $R_{4bc}$ and $R_{5b}$, together with the carbon atom to which they are attached form a $C_{3-6}$cycloalkyl or $C_{3-6}$heterocycloalkyl ring. Suitably $R_{4bc}$ and $R_{5b}$, together with the carbon atom to which they are attached form a $C_{3-6}$cycloalkyl ring, such as a cyclopropyl, cyclobutyl, cyclopentyl or cyclohexyl ring. Suitably $R_{4bc}$ and $R_{5b}$, together with the carbon atom to which they are attached form a $C_{3-6}$heterocycloalkyl ring, such as a heterocyclopropyl, heterocyclobutyl, heterocyclopentyl or heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted.

Suitably $R_{4bc}$ is H, $CH_3$ or ethyl, in particular $CH_3$ or ethyl. Suitably, $R_{4bc}$ and $R_{5b}$, together with the carbon atom to which they are attached form a $C_{3-6}$cycloalkyl ring, such as a cyclopropyl ring.

In one embodiment, $R_{5b}$, is H. In a second embodiment $R_{5bc}$ is $C_{1-6}$alkyl such as $C_{1-4}$alkyl, i.e. methyl, ethyl, propyl (n-propyl or isopropyl) or butyl (n-butyl, isobutyl, sec-butyl or tert-butyl). $R_{5b}$, may also be pentyl (e.g. n-pentyl, sec-pentyl, 3-pentyl, sec-isopentyl and active pentyl) or hexyl (e.g. n-hexyl, 2-methylpentyl, 3-methylpentyl, 2,2-dimethylbutyl and 2,3-dimethylbutyl). In a third embodiment, $R_{5b}$, is $C_{0-2}$alkylene$C_{3-6}$cycloalkyl such as $C_{0-2}$alkylene$C_{3-5}$cycloalkyl, such as $C_{0-2}$alkylene$C_3$cycloalkyl, $C_{0-2}$alkylene$C_4$cycloalkyl, $C_{0-2}$alkylene$C_5$cycloalkyl, $C_0$alkylene$C_{3-5}$cycloalkyl, $C_1$alkylene$C_{3-5}$cycloalkyl and $C_2$alkylene$C_{3-5}$cycloalkyl. $R_{5b}$, may also be $C_{0-2}$alkylene$C_6$cycloalkyl, $C_0$alkylene$C_{3-6}$cycloalkyl, $C_1$alkylene$C_{3-6}$cycloalkyl and $C_2$alkylene$C_{3-6}$cycloalkyl. In a fourth embodiment $R_{5bc}$ is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a fifth embodiment $R_{5b}$, is $C_{1-6}$alkylOH such as $C_{1-4}$alkylOH such as $C_1$alkylOH, $C_2$alkylOH, $C_3$alkylOH or $C_4$alkylOH wherein $C_{1-4}$alkyl is methyl, ethyl, propyl (n-propyl or isopropyl) and butyl (n-butyl, isobutyl, sec-butyl or tert-butyl). $R_{5bc}$ may also be $C_5$alkylOH or $C_6$alkylOH. In a sixth embodiment, $R_{5bc}$ is $C_{1-6}$haloalkyl such as $C_{1-4}$haloalkyl such as $C_1$haloalkyl (e.g. $CF_3$), $C_2$haloalkyl (e.g. $CH_2CF_3$), $C_3$haloalkyl (e.g. $CH_2CH_2CF_3$), $C_4$haloalkyl (e.g. $CH_2CH_2CH_2CF_3$). $R_{5bc}$ may also be $C_5$haloalkyl (e.g. $CH_2CH_2CH_2CH_2CF_3$) or $C_6$haloalkyl (e.g. $CH_2CH_2CH_2CH_2CH_2CF_3$). In a seventh embodiment, $R_{5b}$, is $C_{0-2}$alkylene$C_{3-6}$heterocycloalkyl such as $C_{0-2}$alkylene$C_3$heterocycloalkyl, $C_{0-2}$alkylene$C_4$heterocycloalkyl, $C_{0-2}$alkylene$C_5$heterocycloalkyl, $C_{0-2}$alkylene$C_6$heterocycloalkyl, $C_0$alkylene$C_{3-6}$heterocycloalkyl, $C_1$alkylene$C_{3-6}$heterocycloalkyl and $C_2$alkylene$C_{3-6}$heterocycloalkyl. Suitably the heterocycloalkyl is a heterocyclopropyl,

heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Suitably, any nitrogen atom in the $C_{3-6}$heterocycloalkyl ring is not substituted.

Suitably $R_{5bc}$ is H, $CH_3$ or ethyl, in particular $CH_3$ or ethyl. Suitably, $R_{4bc}$ and $R_{5b}$, together with the carbon atom to which they are attached form a $C_{3-6}$cycloalkyl ring, such as a cyclopropyl ring.

Suitably $R_{4bc}$ is H, $CH_3$ or ethyl and $R_{5b}$, is H, $CH_3$ or ethyl, in particular $R_{4bc}$ is $CH_3$ or ethyl and $R_{5b}$, is $CH_3$ or ethyl. For example, $R_{4bc}$ and $R_{5b}$, are H, $R_{4bc}$ and $R_{5bc}$ are methyl or $R_{4bc}$ and $R_{5bc}$ are ethyl.

Suitably, $R_{4bc}$ is $CH_2CH_2OCH_3$ and $R_{5bc}$ is H.

In one embodiment, $A_c$ is $A_{ac}$ i.e. -$CH_2NR_{6c}$-.

In another embodiment, $A_c$ is $A_{bc}$ i.e. -C(=O)$NR_{6c}$-.

In one embodiment, $R_{6c}$ is H. In another embodiment, $R_{6c}$ is $C_{1-3}$alkyl, in particular $CH_3$.

In one embodiment Ar1c is a 6-membered aryl, i.e. phenyl. In a second embodiment Ar1c is a 6-membered heteroaryl, in particular containing one nitrogen atom (pyridyl) or two nitrogen atoms (pyridazinyl, pyrimidinyl or pyrazinyl).

In particular Ar1c is phenyl or 2-pyridyl, such as phenyl.

In one embodiment $R_{10c}$ is H. In a second embodiment $R_{10c}$ is halo, for example fluoro or chloro. In a third embodiment $R_{10c}$ is $C_{1-3}$alkyl, i.e. $CH_3$, ethyl or propyl (e.g. *n*-propyl or *iso*-propyl). In a fourth embodiment $R_{10c}$ is O$C_{1-2}$alkyl, such as $OCH_3$ or ethoxy. In a fifth embodiment, $R_{10c}$ is $C_{1-2}$haloalkyl, such as $CF_3$ or $CH_2CF_3$. In a sixth embodiment $R_{10c}$ is O$C_{1-2}$haloalkyl, such as $OCF_3$. In a seventh embodiment $R_{10c}$ is CN.

Suitably $R_{10c}$ is H, fluoro, $OCH_3$, $CH_3$ or $CF_3$, in particular H or fluoro, especially H.

Suitably $R_{10c}$ is attached at the ortho position of Ar1c relative to group $A_c$ (i.e. proximal to group $A_c$).

In one embodiment $R_{11c}$ is H. In a second embodiment $R_{11c}$ is F. In a third embodiment, $R_{11c}$ is Cl. In a fourth embodiment $R_{11c}$ is $CH_3$. In a fifth embodiment $R_{11c}$ is $CH_2CH_3$. In a sixth embodiment $R_{11c}$ is $OCH_3$. In a seventh embodiment $R_{11c}$ is $CF_3$. In an eighth embodiment $R_{11c}$ is $OCF_3$. In a ninth embodiment $R_{11c}$ is CN.

In one embodiment, $R_{11c}$ is in the ortho position relative to group $A_c$. In another embodiment, $R_{11c}$ is in the meta position relative to group $A_c$.

In one embodiment Ar2c is a 6-membered aryl, i.e. phenyl. In a second embodiment Ar2c is a 6-membered heteroaryl, in particular containing one nitrogen atom (pyridyl) or two nitrogen atoms (pyridazinyl, pyrimidinyl or pyrazinyl).

In particular Ar2c is 3-pyridyl or 2,5-pyrazinyl, especially 2,5-pyrazinyl.

In one embodiment $R_{12c}$ is H. In a second embodiment $R_{12c}$ is halo, for example fluoro or chloro. In a third embodiment $R_{12c}$ is $C_{1-4}$alkyl, i.e. methyl, ethyl, propyl (n-propyl or isopropyl) or butyl (n-butyl, isobutyl, sec-butyl or tert-butyl). In a fourth embodiment, $R_{12c}$ is $C_{2-4}$alkynyl such as $C_2$alkynyl (i.e. C≡CH). In a fifth embodiment, $R_{12c}$ is C(=O)$C_{1-2}$alkyl, such as C(=O)$C_1$alkyl or C(=O)$C_2$alkyl. In a sixth embodiment $R_{12c}$ is O$C_{0-2}$alkylene$C_{3-5}$cycloalkyl, such as O$C_{3-5}$cycloalkyl (e.g. cyclopropoxy or cyclobutoxy), O$C_1$alkylene$C_{3-5}$cycloalkyl or O$C_2$alkylene$C_{3-5}$cycloalkyl. In a seventh embodiment $R_{12c}$ is O$C_{1-4}$alkyl, such as $OCH_3$, ethoxy, *iso*-propoxy or *n*-propoxy. In an eighth embodiment, $R_{12c}$ is $C_{1-3}$alkyleneO$C_{1-3}$alkyl in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a ninth embodiment $R_{12c}$ is $C_{1-4}$haloalkyl, such as $CF_3$. In a tenth embodiment $R_{12c}$ is O$C_{1-4}$haloalkyl, such as $OCF_3$, $OCHF_2$ or $OCH_2CF_3$. In an eleventh embodiment $R_{12c}$ is CN. In an eleventh embodiment $R_{12c}$ is O$C_{0-2}$alkylene$C_{3-5}$cycloalkyl, such as O$C_{3-5}$cycloalkyl (e.g. cyclopropoxy or cyclobutoxy), O$C_1$alkylene$C_{3-5}$cycloalkyl or O$C_2$alkylene$C_{3-5}$cycloalkyl. In a twelfth embodiment $R_{12c}$ is $OCH_2CH_2N(CH_3)_2$. In a thirteenth embodiment $R_{12c}$ is OH. In a fourteenth embodiment $R_{12c}$ is $C_{1-4}$alkylOH, such as $CH_2OH$ or $C(CH_3)_2OH$. In a fifteenth embodiment $R_{12c}$ is $NR_{23c}R_{24c}$. In a sixteenth embodiment $R_{12c}$ is $SO_2CH_3$. In a seventeenth embodiment $R_{12c}$ is $C(O)N(CH_3)_2$. In an eighteenth embodiment $R_{12c}$ is NHC(O)$C_{1-3}$alkyl such as NHC(O)$CH_3$. In a nineteenth embodiment $R_{12c}$ is a $C_{3-6}$heterocycloalkyl comprising (such as containing) one nitrogen located at the point of attachment to Ar2c, such as a $C_5$heterocycloalkyl, in particular pyrrolidinyl, or a $C_6$heterocycloalkyl such as morpholinyl. In a twentieth embodiment, $R_{12c}$ together with a nitrogen atom to which it is attached forms an N-oxide ($N^+$-$O^-$).

In one embodiment, $R_{23c}$ is H. In another embodiment, $R_{23c}$ is $C_{1-2}$alkyl i.e. $CH_3$ or $CH_2CH_3$.

In one embodiment, $R_{24c}$ is H. In another embodiment, $R_{24c}$ is $C_{1-2}$alkyl i.e. $CH_3$ or $CH_2CH_3$.

$R_{12c}$ is suitably H, fluoro, chloro, $CH_3$, Et, $OCH_3$, OEt, OiPr, $CF_3$ or $OCH_2CF_3$. In particular, $R_{12c}$ is fluoro, chloro, $CH_3$, $OCH_3$, OEt, OiPr or $CF_3$, for example chloro, OEt, OiPr or $CF_3$ such as chloro, OEt or $CF_3$.

$R_{12c}$ is suitably attached at the meta position of Ar2c. Alternatively, $R_{12c}$ is attached at the ortho position of Ar2c.

The present invention provides compounds R94 and R95.

The present invention provides the following compounds:

*N*-(4-(2-((4-(6-Ethoxypyrazin-2-yl)-2-fluorobenzyl)amino)propan-2-yl)thiazol-2-yl)cyclopropanesulfonamide; and

*N*-(4-(2-(((5-(6-ethoxypyrazin-2-yl)pyridin-2-yl)methyl)amino)propan-2-yl)thiazol-2-yl)cyclopropanesulfonamide.

## General Terms and Definitions

In this section reference to variables and compounds of formula (I) are taken to generically cover variables wherein the suffix "a", "b" or "c" has been added. For example, reference to $R_4$ is taken to include reference to $R_{4a}$, $R_{4b}$ and $R_{4c}$ as well as sub-groups within these variables. The same applies for all other variables discussed in this section. Similarly, reference to compounds of formula (I) is taken to include compounds of formula (I-a), (I-b) and/or (I-c).

The term 'alkyl' as used herein, such as in $C_{1-3}$alkyl, $C_{1-4}$alkyl, $C_{1-5}$alkyl or $C_{1-6}$alkyl e.g. $C_{1-3}$alkyl, $C_{1-4}$alkyl or $C_{1-5}$alkyl, or such as in $C_{1-2}$alkyl, $C_{1-3}$alkyl or $C_{1-4}$alkyl whether alone or forming part of a larger group such as an Oalkyl group (e.g. $OC_{1-3}$alkyl, $OC_{1-4}$alkyl and $OC_{1-5}$alkyl or $OC_{1-2}$alkyl, $OC_{1-3}$alkyl or $OC_{1-4}$alkyl), is a straight or a branched fully saturated hydrocarbon chain containing the specified number of carbon atoms. Examples of alkyl groups include the $C_{1-5}$alkyl groups methyl, ethyl, *n*-propyl, *iso*-propyl, *n*-butyl, *iso*-butyl, *sec*-butyl, *tert*-butyl and *n*-pentyl, *sec*-pentyl and 3-pentyl, in particular the $C_{1-3}$alkyl groups methyl, ethyl, *n*-propyl and *iso*-propyl. Examples of alkyl groups also include the $C_{1-4}$alkyl groups methyl, ethyl, *n*-propyl, *iso*-propyl, *n*-butyl, *iso*-butyl, *sec*-butyl and *tert*-butyl, in particular the $C_{1-3}$alkyl groups methyl, ethyl, *n*-propyl and *iso*-propyl such as $C_{1-2}$alkyl groups methyl and ethyl. Reference to "propyl" includes *n*-propyl and *iso*-propyl, and reference to "butyl" includes *n*-butyl, isobutyl, *sec*-butyl and *tert*-butyl. Examples of Oalkyl groups include the $OC_{1-4}$alkyl groups methoxy, ethoxy, propoxy (which includes *n*-propoxy and *iso*-propoxy) and butoxy (which includes *n*-butoxy, *iso*-butoxy, *sec*-butoxy and *tert*-butoxy). $C_5$alkyl groups as used herein, whether alone or forming part of a larger group such as an $OC_5$alkyl group is a straight or a branched fully saturated hydrocarbon chain containing five carbon atoms. Examples of $C_5$alkyl groups include n-pentyl, sec-pentyl, 3-pentyl, sec-isopentyl and active pentyl. $C_6$alkyl groups as used herein, whether alone or forming part of a larger group such as an $OC_6$alkyl group is a straight or a branched fully saturated hydrocarbon chain containing six carbon atoms. Examples of $C_6$alkyl groups include n-hexyl, 2-methylpentyl, 3-methylpentyl, 2,2-dimethylbutyl and 2,3-dimethylbutyl.

The term 'alkylene' as used herein, such as in $C_{0-2}$alkylene$C_{3-5}$cycloalkyl, $C_{1-3}$alkylene$OC_{1-2}$alkyl $C_{1-2}$alkylene$OC_{1-2}$alkyl or $OC_{0-2}$alkylene$C_{3-5}$cycloalkyl is a bifunctional straight or a branched fully saturated hydrocarbon chain containing the specified number of carbon atoms. Examples of $C_{0-2}$alkylene groups are where the group is absent (i.e. $C_0$), methylene ($C_1$) and ethylene ($C_2$). Examples of $C_{1-3}$alkylene groups are where the group is methylene ($C_1$), ethylene ($C_2$) and propylene ($C_3$). Examples of $C_{1-2}$alkylene groups are where the group is methylene ($C_1$) and ethylene ($C_2$). Examples of $C_{0-1}$alkylene groups are where the group is absent ($C_0$) and methylene ($C_1$).

The term 'alkenyl' as used herein, such as in $C_{2-4}$alkenyl, is a straight or branched hydrocarbon chain containing the specified number of carbon atoms and a carbon-carbon double bond.

The term 'alkynyl' as used herein, such as in $C_{2-4}$alkynyl such as in $C_2$alkynyl is an unbranched hydrocarbon chain containing the specified number of carbons (e.g. 2, 3 or 4 carbons, such as two carbons), two of which carbon atoms are linked by a carbon-carbon triple bond.

The term 'cycloalkyl' as used herein, such as in $C_{3-4}$cycloalkyl, $C_{3-5}$cycloalkyl or $C_{3-6}$cycloalkyl, whether alone or forming part of a larger group such as $OC_{3-5}$cycloalkyl, $C_{0-2}$alkylene$C_{3-6}$cycloalkyl, $C_{0-2}$alkylene$C_{3-5}$cycloalkyl or $C_{0-1}$alkylene$C_{3-4}$cycloalkyl is a fully saturated hydrocarbon ring containing the specified number of carbon atoms. Examples of cycloalkyl groups include the $C_{3-6}$cycloalkyl groups cyclopropyl, cyclobutyl, cyclopentyl and cyclohexyl, in particular the $C_{3-5}$cycloalkyl groups cyclopropyl, cyclobutyl and cyclopentyl:

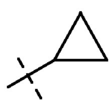
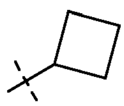
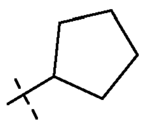
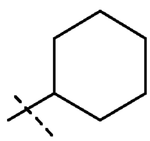

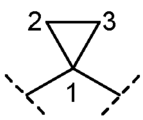
$C_3$ cycloalkyl

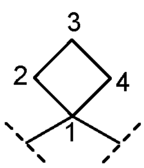
$C_4$ cycloalkyl

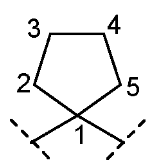
$C_5$ cycloalkyl

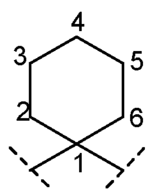
$C_6$ cycloalkyl

When B is B-a, the term 'heterocycloalkyl' as used herein, such as in $C_{3-6}$heterocycloalkyl or $C_{0-2}$alkylene-

$C_{3-6}$heterocycloalkyl is a fully saturated hydrocarbon ring containing the specified number of carbon atoms, wherein at least one of the carbon atoms in the ring is replaced by a heteroatom such as N, S or O. When B is B-bc, the term 'heterocycloalkyl' as used herein, such as in $C_{3-6}$heterocycloalkyl or $C_{0-2}$alkylene$C_{3-6}$heterocycloalkyl is a fully saturated hydrocarbon ring containing the specified number of ring atoms and includes the ring atom through which the heterocycloalkyl group is attached, wherein at least one of the atoms in the ring is a heteroatom such as O, N or S.

As required by valency, the nitrogen atom(s) may be connected to a hydrogen atom to form an NH group. Alternatively the nitrogen atom(s) may be substituted (such as one nitrogen atom is substituted), for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. Wherein a ring heteroatom is S, the term 'heterocycloalkyl' includes wherein the S atom(s) is substituted (such as one S atom is substituted) by one or two oxygen atoms (i.e. S(O) or $S(O)_2$). Alternatively, any sulphur atom(s) in the $C_{3-6}$heterocycloalkyl ring is not substituted.

Examples of $C_{3-6}$heterocycloalkyl groups include those comprising one heteroatom such as containing one heteroatom (e.g. oxygen) or containing two heteroatoms (e.g. two oxygen atoms or one oxygen atom and one nitrogen atom). Other examples of $C_{3-6}$heterocycloalkyl include those comprising one heteratom atom such as containing one heteroatom (e.g. one oxygen atom or one nitrogen atom) or containing two heteroatoms (e.g. two nitrogen atoms or one nitrogen atom and one oxygen atom). Particular examples of $C_{3-6}$heterocycloalkyl comprising one nitrogen atom include pyrrolidinyl, pyrazolidinyl, piperidinyl, piperazinyl, morpholinyl and thiomorpholinyl, especially piperidinyl, pyrrolidinyl and morpholinyl. Particular examples of $C_{3-6}$heterocycloalkyl comprising one oxygen atom include oxiranyl, oxetanyl, 3-dioxolanyl, morpholinyl, 1,4-oxathianyl, tetrahydropyranyl, 1,4-thioxanyl and 1,3,5-trioxanyl. Examples of $C_{3-6}$heterocycloalkyl include those comprising one oxygen atom such as containing one oxygen atom, or containing two oxygen atoms. Particular examples of $C_{3-6}$heterocycloalkyl comprising one oxygen atom include oxiranyl, oxetanyl, 3-dioxolanyl, morpholinyl, 1,4-oxathianyl, tetrahydropyranyl, 1,4-thioxanyl and 1,3,5-trioxanyl. Particular examples of $C_{3-6}$heterocycloalkyl comprising one nitrogen atom include piperidinyl. Other examples of $C_{3-6}$heterocycloalkyl comprising one nitrogen atom include pyrrolidinyl, pyrazolidinyl, imidazolidinyl, thiazolidinyl, piperidinyl, piperazinyl, morpholinyl and thiomorpholinyl, such as piperidinyl.

In one embodiment and when B is B-a, the term 'heterocycloalkyl' as used herein, such as in $C_{3-6}$heterocycloalkyl is a fully saturated hydrocarbon ring containing the specified number of carbon atoms, wherein at least one of the carbon atoms in the ring is replaced by a heteroatom such as N, S or O. Examples of $C_{3-6}$heterocycloalkyl groups include those comprising one heteroatom such as containing one heteroatom (e.g. oxygen) or containing two heteroatoms (e.g. two oxygen atoms or one oxygen atom and one nitrogen atom).

When B is B-bc and the compound is a compound of formula (I-b), the term 'heterocycloalkyl' as used herein, such as in $C_{3-6}$heterocycloalkyl is a fully saturated hydrocarbon ring containing the specified number of ring atoms and includes the ring atom through which the heterocycloalkyl group is attached, wherein at least one of the atoms in the ring is a heteroatom such as O, N or S. Examples of $C_{3-6}$heterocycloalkyl include those comprising one nitrogen atom such as containing one heteroatom (i.e. nitrogen) or containing two heteroatoms (e.g. two nitrogen atoms or one nitrogen atom and one oxygen atom).

The term '5- or 6-membered oxygen-containing heterocycloalkyl' as used herein, is a fully saturated hydrocarbon ring containing the specified number of ring atoms (i.e. 5 or 6), wherein at least one ring atom is an oxygen atom and the ring does not contain heteroatoms other than oxygen. Examples of oxygen-containing heterocycloalkyl groups are oxiranyl, oxetanyl, tetrahydrofuranyl, 3-dioxolanyl, tetrahydropyranyl, and 1,3,5-trioxanyl, such as tetrahydrofuranyl and tetrahydropyranyl. An example of a nitrogen-containing heterocycloalkyl group is piperidinyl.

The heterocycloalkyl groups may have any one of the following structures:

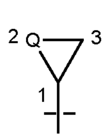
$C_3$ heterocycloalkyl

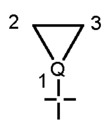
$C_3$ heterocycloalkyl

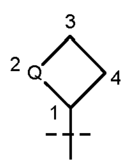
$C_4$ heterocycloalkyl

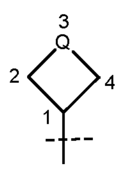
$C_4$ heterocycloalkyl

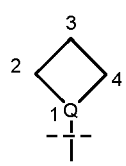
$C_4$ heterocycloalkyl

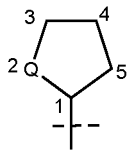
$C_5$ heterocycloalkyl
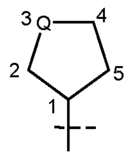
$C_5$ heterocycloalkyl
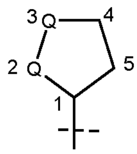
$C_5$ heterocycloalkyl
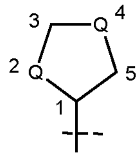
$C_5$ heterocycloalkyl
;

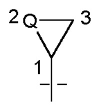
$C_3$ heterocycloalkyl
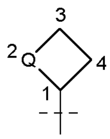
$C_4$ heterocycloalkyl
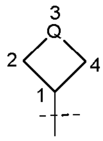
$C_4$ hetero-cycloalkyl
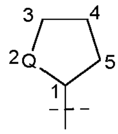
$C_5$ heterocycloalkyl

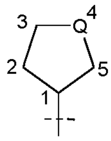
$C_5$ heterocycloalkyl
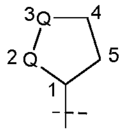
$C_5$ heterocycloalkyl
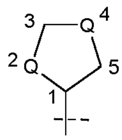
$C_5$ heterocycloalkyl

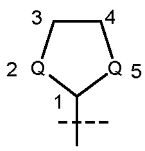
$C_5$ heterocycloalkyl
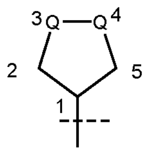
$C_5$ heterocycloalkyl
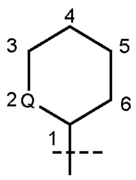
$C_6$ heterocycloalkyl
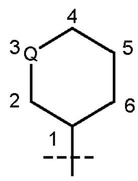
$C_6$ heterocycloalkyl
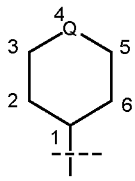
$C_6$ heterocycloalkyl

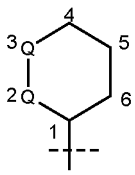
$C_6$ heterocycloalkyl
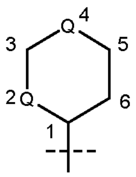
$C_6$ heterocycloalkyl
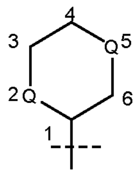
$C_6$ heterocycloalkyl
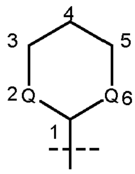
$C_6$ heterocycloalkyl
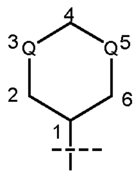
$C_6$ heterocycloalkyl
;

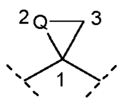
$C_3$ heterocycloalkyl
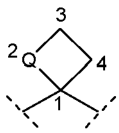
$C_4$ heterocycloalkyl
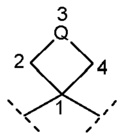
$C_4$ hetero-cycloalkyl
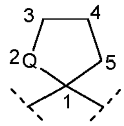
$C_5$ heterocycloalkyl

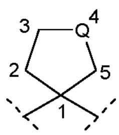
$C_5$ heterocycloalkyl
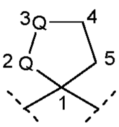
$C_5$ heterocycloalkyl
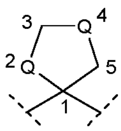
$C_5$ heterocycloalkyl

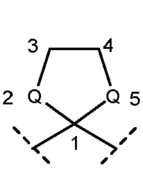
$C_5$ heterocycloalkyl
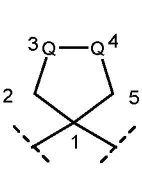
$C_5$ heterocycloalkyl
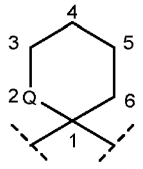
$C_6$ heterocycloalkyl
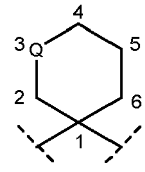
$C_6$ heterocycloalkyl
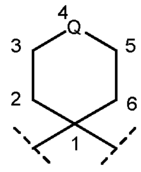
$C_6$ heterocycloalkyl

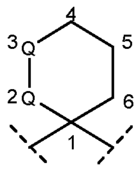
$C_6$ heterocycloalkyl
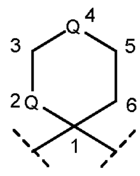
$C_6$ heterocycloalkyl
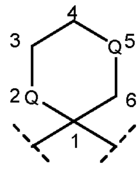
$C_6$ heterocycloalkyl
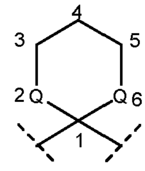
$C_6$ heterocycloalkyl
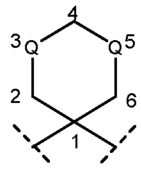
$C_6$ heterocycloalkyl

wherein each Q is independently selected from O, N or S, such as O or N. When Q is N, as required by valency, the nitrogen atom(s) may be connected to a hydrogen atom to form an NH group. Alternatively the nitrogen atom(s) may be substituted (such as one nitrogen atom is substituted), for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. When any Q is S, the S atoms can be substituted (such as one S atom is substituted) by one or two oxygen atoms (i.e. S(O) or $S(O)_2$). When $R_4$ and $R_5$ are $R_{4a}$ and $R_{5a}$, Q is N substituted by $S(O)_2R_{29}$. Alternatively, any sulphur atom(s) in the $C_{3-6}$heterocycloalkyl ring is not substituted.

When $A_a$ is -C(=O)NH-, -NH- or -$CH_2$NH- and $R_{4a}$ and/or $R_{5a}$ is $C_0$alkylene$C_{3-6}$heterocycloalkyl, or when $R_{4a}$ and $R_{5a}$ together with the carbon atom to which they are attached form a $C_{3-6}$heterocycloalkyl, any heteroatom in the heterocycloalkyl may not be directly connected to the carbon to which $R_{4a}$ and $R_{5a}$ are connected.

When B is B-a, suitably, heterocycloalkyl is a fully saturated hydrocarbon ring containing the specified number of carbon atoms wherein at least one of the carbon atoms is replaced by a heteroatom such as N, S or O wherein as required by valency, any nitrogen atom is connected to a hydrogen atom, and wherein the S atom is not present as an oxide.

When B is B-bc and the compound is a compound of formula (I-b), suitably, heterocycloalkyl is a fully saturated hydrocarbon ring containing the specified number of ring atoms and includes the ring atom through which the heterocycloalkyl group is attached, wherein at least one of the atoms in the ring is a heteroatom such as O, N or S. Suitably, as required by valency, any nitrogen atom is connected to a hydrogen atom. Suitably any S atom is not present as an oxide. In particular, any nitrogen atom is connected to a hydrogen atom and any S atom is not present as an oxide.

When B is B-bc and the compound is a compound of formula (I-c), and when the heterocycloalkyl is formed from $R_4$ and $R_5$ together with the carbon atom to which they are attached, suitably any heteroatom is not directly connected to the carbon to which $R_4$ and $R_5$ are attached. Thus suitably, when the heterocycloalkyl is formed from $R_4$ and $R_5$ together with the carbon atom to which they are attached, the heterocycloalkyl may be:

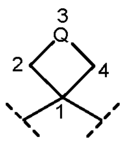
$C_4$ heterocycloalkyl
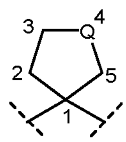
$C_5$ heterocycloalkyl
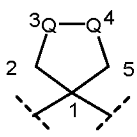
$C_5$ heterocycloalkyl

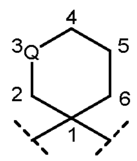
$C_6$ heterocycloalkyl
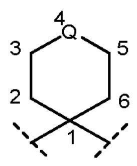
$C_6$ heterocycloalkyl
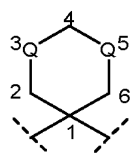
$C_6$ heterocycloalkyl

wherein each Q is independently O, N or S such as O or N. When Q is N, as required by valency, the nitrogen atom(s) may be connected to a hydrogen atom to form an NH group. Alternatively the nitrogen atom (s) may be substituted (such as one

nitrogen atom is substituted), for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Additional substituent groups on any nitrogen atom(s) in the $C_{3-6}$heterocycloalkyl ring include $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$. When any Q is S, the S atom(s) can be substituted (such as one S atom is substituted) by one or two oxygen atoms (i.e. S(O) or $S(O)_2$). When $R_4$ and $R_5$ are $R_{4a}$ and $R_{5a}$, Q is N substituted by - $S(O)_2R_{29}$. Alternatively, any sulphur atom(s) in the $C_{3-6}$heterocycloalkyl ring is not substituted.

The term 'halo' or 'halogen' as used herein, refers to fluorine, chlorine, bromine or iodine. Particular examples of halo are fluorine and chlorine, especially fluorine.

The term 'haloalkyl' as used herein, such as in $C_{1-6}$haloalkyl, such as in $C_{1-4}$haloalkyl or $C_{1-2}$haloalkyl, whether alone or forming part of a larger group such as an Ohaloalkyl group, such as in O$C_{1-6}$haloalkyl,such as in O$C_{1-4}$haloalkyl or O$C_{1-2}$haloalkyl, is a straight or a branched fully saturated hydrocarbon chain containing the specified number of carbon atoms and at least one halogen atom, such as fluoro or chloro, especially fluoro. An example of haloalkyl is $CF_3$. Further examples of haloalkyl are $CHF_2$ and $CH_2CF_3$. Another example of haloalkyl is $CH_2CHF_2$. Examples of Ohaloalkyl include $OCF_3$, $OCHF_2$ and $OCH_2CF_3$.

The term '6-membered aryl' as used herein refers to a phenyl ring.

The term '6-membered heteroaryl' as used herein refers to 6-membered aromatic rings containing at least one heteroatom (e.g. nitrogen). Exemplary 6-membered heteroaryls include one nitrogen atom (pyridinyl), two nitrogen atoms (pyridazinyl, pyrimidinyl or pyrazinyl) and three nitrogen atoms (triazinyl).

The phrase 'in the para position relative to group A' as used herein, such as in relation to the position of Ar2, means that compounds with the following substructure are formed:

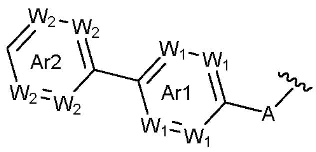

wherein when B is B-a, $W_1$ may be N, CH, $CR_{10a}$ or $CR_{11a}$, and $W_2$ may be N, CH or $CR_{12a}$ as allowed by the definitions provided for compounds of formula (I). $W_2$ may also be $CR_{13a}$ as allowed by the definitions provided for compounds of formula (I-a); and

when B is B-bc and the compound is a compound of formula (I-b), compounds with the following substructure are formed:

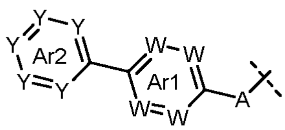

wherein W may be N, CH, $CR_{10b}$ or $CR_{11b}$, and Y may be N, CH, $CR_{12b}$ or $CR_{13b}$ as allowed by the definitions provided for compounds of formula (I-b);

and when B is B-bc and the compound is a compound of formula (I-c), compounds with the following substructure are formed:

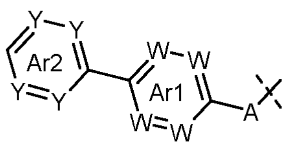

wherein W may be N, CH or $CR_{10c}$, and Y may be N, CH or $CR_{12c}$ as required by the definitions provided for compounds of formula (I-c). W may also be $CR_{11c}$ as allowed by the definitions provided for compounds of formula (I-c).

The terms 'ortho' and 'meta' as used herein, such as when used in respect of defining the position of $R_{12}$ on Ar2 is with respect to Ar1, means that the following structures may form:

when B is B-a:

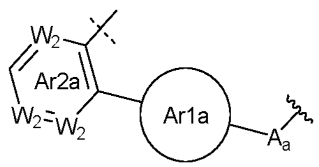

*ortho*

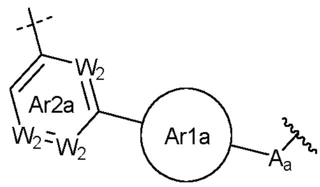

*meta* ;

when B is B-bc and the compound is a compound of formula (I-b):

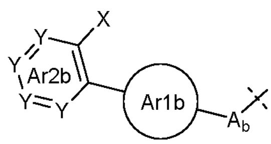

*ortho*

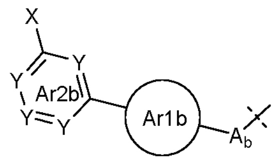

*meta*

wherein X represents a substituent e.g. $R_{12b}$; and

when B is B-bc and the compound is a compound of formula (I-c):

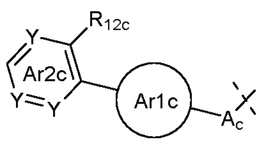

*ortho*

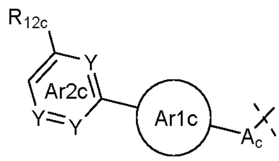

*meta* ;

wherein all variables listed above are as defined elsewhere herein.

When a spirocyclic ring system is said to form, e.g. when $R_{4aa}$ and $R_{5aa}$, $R_{4ab}$ and $R_{5ab}$, or $R_{4ac}$ and $R_{5ac}$, suitably together with the carbon atom to which they are attached form a $C_{3-6}$cycloalkyl and one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, the following spirocyclic groups are encompassed (which may optionally be substituted as mentioned above):

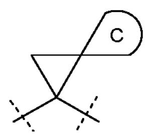
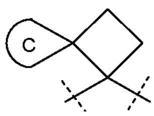
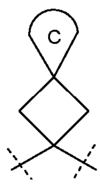
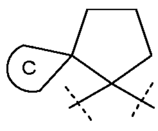

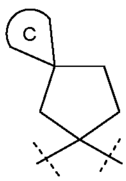
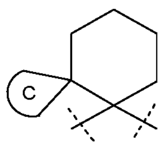
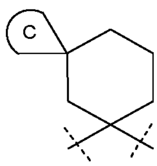
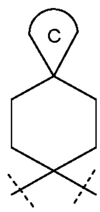 ;

wherein C is a $C_{3-6}$cycloalkyl ring or a $C_{3-6}$heterocycloalkyl ring, as defined elsewhere herein. In one embodiment C is a $C_{3-6}$cycloalkyl ring. In a second embodiment C is a $C_{3-6}$heterocycloalkyl ring.

For example, one of the carbons is quaternary and is attached to a 5-membered dioxalane ring to form the following structure:

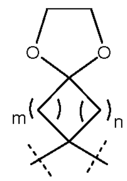

wherein m is 1 or 2 and n is 0, 1 or 2. Suitably m is 2 and n is 2.

Alternatively, when a spirocyclic ring system is said to form, e.g. when $R_{4aa}$ and $R_{5aa}$, $R_{4ab}$ and $R_{5ab}$, or $R_{4ac}$ and $R_{5ac}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, the following spirocyclic groups are encompassed (which may optionally be substituted as mentioned above):

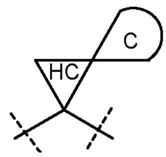
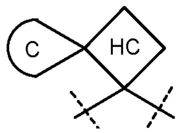
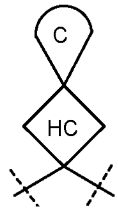
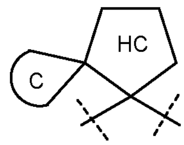

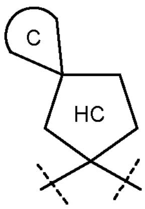
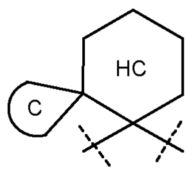
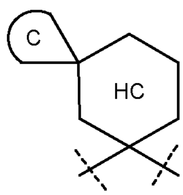
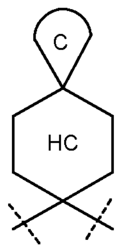;

wherein C is a $C_{3-6}$cycloalkyl ring or a $C_{3-6}$heterocycloalkyl ring, as defined elsewhere herein, and HC is a $C_{3-6}$heterocycloalkyl ring formed by $R_{4a}$ and $R_{5a}$ (i.e. $R_{4aa}$ and $R_{5aa}$, $R_{4ab}$ and $R_{5ab}$, or $R_{4ac}$ and $R_{5ac}$) as defined elsewhere herein. In one embodiment C is a $C_{3-6}$cycloalkyl ring. In a second embodiment C is a $C_{3-6}$heterocycloalkyl ring.

Throughout the specification Ar1 and Ar2 may be depicted as follows:

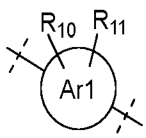 ; 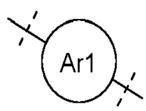 ; 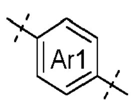 ; Ar1

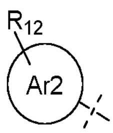 ; 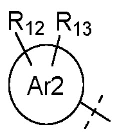 ; 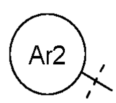 ; 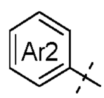 ; Ar2

All depictions with respect to Ar1 are equivalent and all depictions with respect to Ar2 are equivalent, unless the context requires otherwise, depictions of Ar1 and Ar2 should not be taken to exclude the presence of heteroatoms or substitutions. Ar1 encompasses the variables Ar1a, Ar1b and Ar1c. Ar2 encompasses the variables Ar2a, Ar2b and Ar2c.

The present invention provides N-oxides of the compound of formula (I). Suitably, when $R_{12}$ together with a nitrogen atom to which it is attached forms an N-oxide ($N^+$-$O^-$), the example following structures are formed:

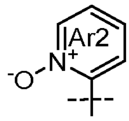 or 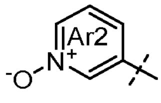.

The position numbering for Ar1 is in respect of group A, with the carbon at the point of attachment designated position 1 and other numbers providing the relative location of the nitrogen atoms, for example:

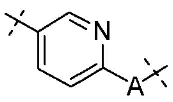 ; 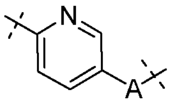 ; 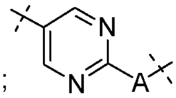 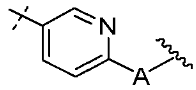 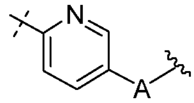

2-pyridyl 3-pyridyl 2,6-pyrimidinyl e.g. 2-pyridyl ; 3-pyridyl ;

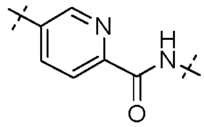

e.g. 2-pyridyl

The position numbering for Ar2 is in respect of the point of attachment to Ar1, for example:

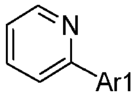 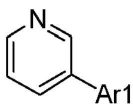 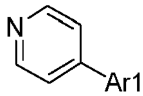 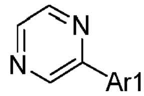

2-pyridyl 3-pyridyl 4-pyridyl 2,5-pyrazinyl

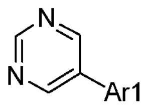 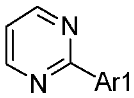 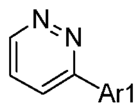 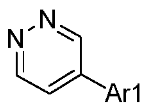 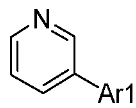 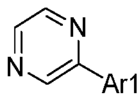

3,5-pyrimidinyl 2,6-pyrimidinyl 2,3-pyridazinyl 3,4-pyridazinyl e.g. 3-pyridyl 2,5-pyrazinyl .

Reference to compounds of formula (I) throughout the application is intended to encompass reference to compounds of formulae (I-a), (I-b) and (I-c).

The compounds of the invention may be provided in the form of a pharmaceutically acceptable salt and/or solvate thereof. In particular, a pharmaceutically acceptable salt.

Compounds of the invention of particular interest are those demonstrating an $IC_{50}$ of 1 uM or lower, especially 100nM or lower, in respect of CTPS1 enzyme, using the methods of the examples (or comparable methods).

Compounds of the invention of particular interest are those demonstrating a selectivity for CTPS1 over CTPS2 of 2-30 fold, suitably >30-60 fold or more suitably >60 fold, using the methods of the examples (or comparable methods). Desirably the selectivity is for human CTPS1 over human CTPS2.

It will be appreciated that for use in medicine the salts of the compounds of formula (I) should be pharmaceutically acceptable. Non-pharmaceutically acceptable salts of the compounds of formula (I) may be of use in other contexts such as during preparation of the compounds of formula (I). Suitable pharmaceutically acceptable salts will be apparent to those skilled in the art. Pharmaceutically acceptable salts include those described by Berge et al. (1977). Such pharmaceutically acceptable salts include acid and base addition salts. Pharmaceutically acceptable acid additional salts may be formed with inorganic acids e.g. hydrochloric, hydrobromic, sulphuric, nitric or phosphoric acid and organic acids e.g. succinic, maleic, acetic, fumaric, citric, tartaric, benzoic, p-toluenesulfonic, methanesulfonic or naphthalenesulfonic acid. Other salts e.g. oxalates or formates, may be used, for example in the isolation of compounds of formula (I) and are included within the scope of this invention.

Certain of the compounds of formula (I) may form acid or base addition salts with one or more equivalents of the acid or base. The present invention includes within its scope all possible stoichiometric and non-stoichiometric forms.

The compounds of formula (I) may be prepared in crystalline or non-crystalline form and, if crystalline, may optionally be solvated, e.g. as the hydrate. This invention includes within its scope stoichiometric solvates (e.g. hydrates) as well as compounds containing variable amounts of solvent (e.g. water).

It is to be understood that the present invention encompasses all isomers of formula (I) including all geometric, tautomeric and optical forms, and mixtures thereof (e.g. racemic mixtures). Where additional chiral centres are present in compounds of formula (I), the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The present disclosure includes all isotopic forms of the compounds of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exist as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%, >50%, >75%, >90%, >95% or >99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium ($^{2}H$ or D), carbon-11 ($^{11}C$), carbon-13 ($^{13}C$), carbon-14 ($^{14}C$) , nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-37 ($^{31}Cl$), fluorine-18 ($^{18}F$) iodine-123 ($^{123}I$), iodine-125 ($^{125}I$) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. $^{3}H$, and carbon-14, i.e. $^{14}C$, are particularly useful for this purpose in view of their ease of incorporation and ready means of detection. Unnatural variant isotopic forms which incorporate deuterium i.e. $^{2}H$ or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as $^{11}C$, $^{18}F$, $^{15}O$ and $^{13}N$, and would be useful in Positron Emission Topography (PET) studies for examining substrate receptor occupancy.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. $^{2}H$ or D) is incorporated where hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the Examples. Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

In general, the compounds of formula (I) may be made according to the organic synthesis techniques known to those skilled in this field, as well as by the representative methods set forth below, those in the Examples, and modifications thereof.

**General Routes:**

In this section reference to variables and compounds of formula (I) are taken to generically cover variables wherein the suffix "a", "b" or "c" has been added. For example, reference to $R_4$ is taken to include reference to $R_{4a}$, $R_{4b}$ and $R_{4c}$ as well as sub-groups within these variables. The same applies for all other variables discussed in this section. Reference to compounds of formula (I) is taken to include compounds of formula (I-a), (I-b) and/or (I-c).

Generic and specific routes by which compounds disclosed herein and compound examples of the invention may be conveniently prepared are disclosed in WO2019/106156, WO2019/106146, WO2019/179652, WO2019/180244 and WO2020/083975, as well as those routes summarised below, and adaptations thereof.

Compounds of formula (I-a)

Compounds of formula (I-a) may be synthesised according to general and specific methods disclosed in WO2019/179652, WO2019/180244 and WO2020/083975. For the avoidance of doubt, reference to variables in each of the schemes in this section encompasses variables specific to compounds of formula (I-a). For example, $R_1$ includes $R_{1a}$. Furthermore, reference to intermediates in each of the schemes in this section encompasses corresponding intermediates for compounds of formula (I-a). For example, reference to compounds of formula (II) encompasses compounds of formula (II-a).

## Scheme 1a

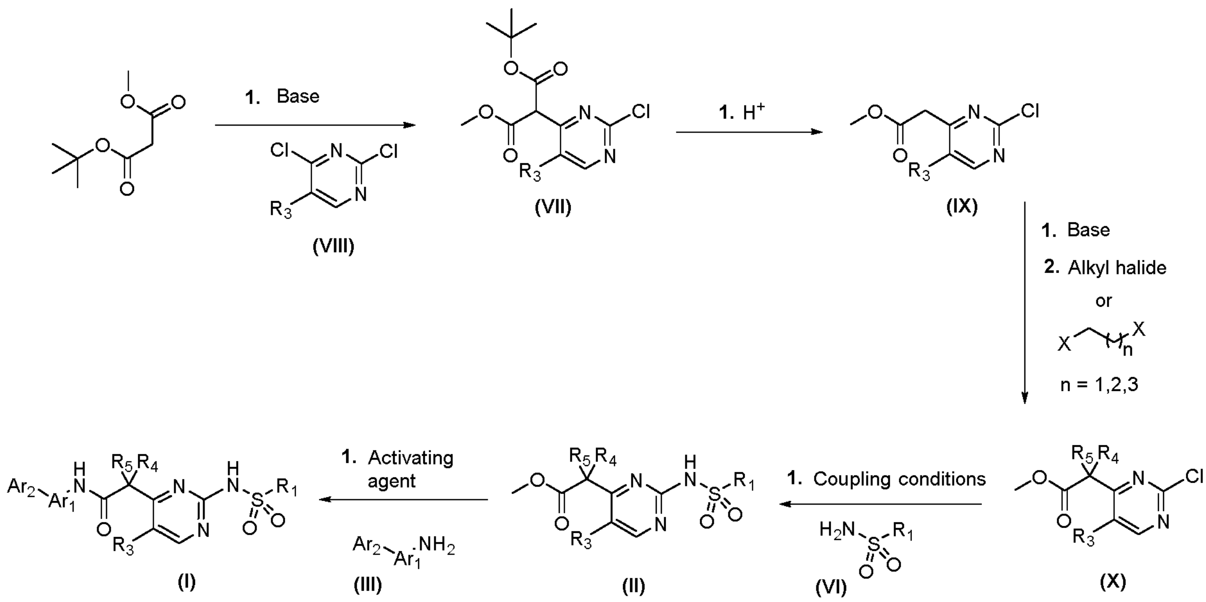


## Scheme 1b

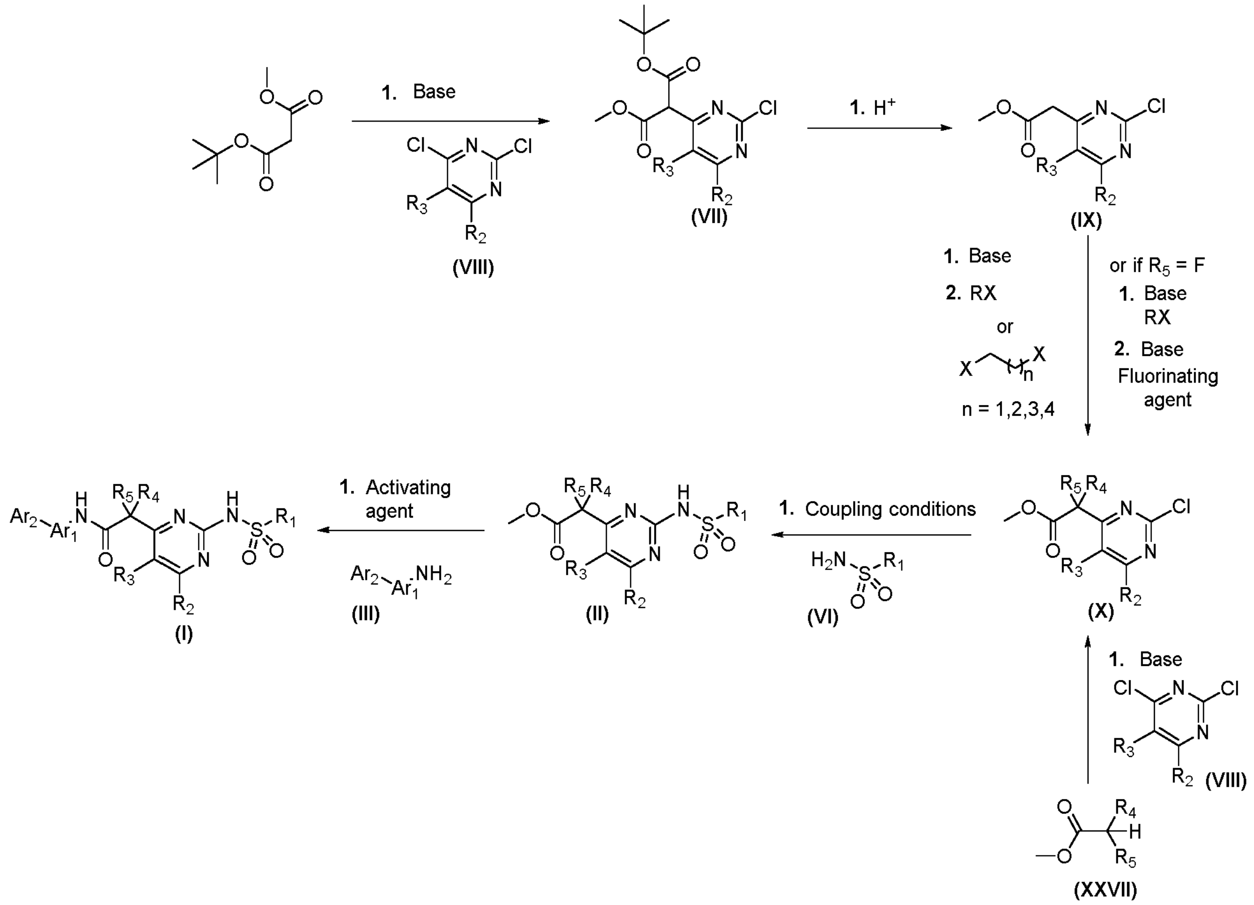


Suitably, $R_2$ is H, **(IX)** is converted to **(X)** using a base and alkyl halide or X-$CH_2$-$(CH_2)$n-X wherein n = 1,2,3 and the compounds of general formula **(I)** are obtained by a five step process.

In general and as illustrated in Schemes 1a and 1b, compounds of general formula **(I)** may be obtained by a five or six step process from a 2,4-dichloropyrimidine derivative of general formula **(VIII)**. Firstly, the derivative **(VIII)** can be reacted with an unsymmetrical malonate ester as shown in Schemes 1a, 1b of WO2019/179652, or 1a or 1b herein. For example, the unsymmetrical malonate ester can be treated with a base such as $Cs_2CO_3$ in the presence of dichloropyrimidine **(VIII)** in a solvent such as DMF and heated to an elevated temperature such as 80 °C, followed by an aqueous work-up to obtain compounds of formula **(VII)**. This intermediate compound can then be deprotected at this stage *via* a decarboxylation, initiated by the use of a strong acid such as TFA to yield intermediate derivative **(IX)**. Certain intermediates such as **(IX)** where $R_3$ = H, are commercially available. Reaction of a methyl 2-(2-chloropyrimidin-4-yl) acetate derivative of general formula **(IX)** with an inorganic base such as potassium carbonate, in the presence of an alkylating agent leads to alkylation alpha to the ester. It will be understood by persons skilled in the art that both mono- and dialkylation may be achieved with careful control of the reaction conditions, but for a more reliable synthesis of the monoalkylated product, an alternative procedure should be considered (as in Scheme 1a of WO2019179652). $R_4$ and $R_5$ can be connected to form a $C_{3-6}$cycloalkyl ring as defined above (**(IX)** to **(X)**). Such compounds may be prepared by double alkylation with a dihaloalkane, such as 1,2-dibromoethane or 1,3-dibromobutane in the presence of an inorganic base such as sodium hydroxide. For compounds of general formula **(I)** wherein $R_4$ and $R_5$ together with the carbon to which they are attached form a $C_{3-6}$heterocycloalkyl, double alkylation of intermediates **(IX)** using a di-haloheteroalkane (such as $BrCH_2CH_2OCH_2CH_2Br$) in the presence of a base such as $Cs_2CO_3$ in a solvent such as MeCN at an elevated temperature such as 60 °C followed by direct column chromatography can be used to provide compounds of formula **(X)**.

Palladium catalysed sulfamination of intermediate **(X)** may be achieved using a catalyst such as [*t*-BuXPhosPd(allyl)]OTf or *t*-BuXPhos-Pd-G3 and substituted sulfonamide nucleophile **(VI)**, in the presence of an inorganic base, for example potassium carbonate to form intermediate derivative **(II)**. Alternatively, sulfamination of intermediate **(X)** may be achieved using a substituted sulfonamide nucleophile **(VI)**, in the presence of an inorganic base, for example $Cs_2CO_3$ and a solvent such as N-methyl pyrrolidinone to form intermediates **(II)** which may be obtained by precipitation following dilution in aqueous 4M HCl.

Final transformation to compounds of general formula **(I)** can be prepared by conversion of intermediate **(II)** by activation of the ester moiety using trimethylaluminium (usually a 2.0 M solution in toluene or heptane) and addition of

amine **(III)** (commercially available or prepared as in Schemes 6a, 6b, 7a or 7b of WO2019179652). Alternatively, compounds of formula **(I)** may be obtained by a strong base-mediated amide formation between compounds **(II)** and **(III)** at room temperature using bases such as iPrMgCl, LiHMDS or KOtBu.

Compounds of the general formula **(VII)** where $R_2$ is O-alkyl may be accessed in two steps from commercial 2,4,6-trichloropyrimidine derivatives such as **(VIII)** where $R_2$ is Cl. Reaction of an unsymmetrical malonate ester can yield compounds such as **(VII)** which can then be treated with an alkoxide base such as sodium methoxide to displace the more reactive chloride to give compounds of general formula **(VII)** where $R_2$ = O-alkyl. Such compounds can then be progressed to final compounds of formula **(I)** following the steps previously described in Schemes 1a or 1b.

Compounds of general formula **(I)** where $R_1$, Ar1 and Ar2 are defined above and $R_4$ and $R_5$ together with the carbon to which they are attached form a $C_{3-6}$heterocycloalkyl, may be prepared in five steps starting from intermediate of general formula **(VIII)**. Firstly, alkyl esters of general formula **(XXVII)** can be treated with a strong base such as LiHMDS then reacted with 2,4-dichloropyrimidines such as derivative **(VIII)**. Such compounds can then be converted to final compounds using the methods described in Scheme 1b. If any protecting groups remain after amide coupling, treatment with a strong acid such as TFA may yield final compounds of formula **(I)**.

Following deprotection compounds of general formula **(I)** where $R_1$, Ar1 and Ar2 are defined above and $R_4$ and $R_5$ together with the carbon to which they are attached form a $C_{3-6}$ aminocycloalkyl, may be further elaborated by treatment with a suitable electrophile such as an acid chloride or an isocyanate, to yield the corresponding amide or urea. Such compounds may also undergo reductive amination in the presence of a suitable aldehyde or ketone followed by treatment with sodium triacetoxyborohydride.

## Scheme 2

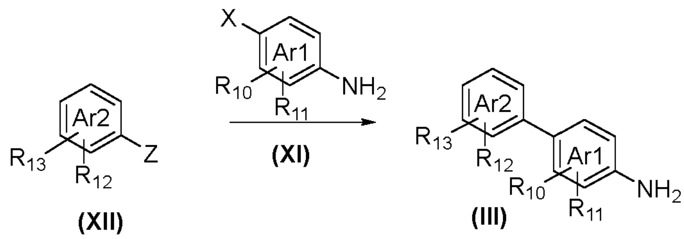

(XII)

(XI)

(III)

Z = $B(OH)_2$, B(pin)
X = Br, Cl

Intermediates of formula **(III)** wherein Ar1, $R_{10}$, $R_{11}$, $R_{12}$ and $R_{13}$ are defined above and Ar2 is an unsubstituted or substituted 3-pyridyl ring, may be synthesised by coupling under Suzuki conditions of a boronate of general formula **(XII)**, wherein $R_{12}$ and $R_{13}$ are defined above and Z represents a dihydroxyboryl or dialkyloxyboryl group, usually a 4,4,5,5-tetramethyl-1,3,3,2-dioxaborolan-2-yl group, to a substituted pyridine of formula **(XI)** where X denotes a halide. The couplings according to the Suzuki method are performed, for example, by heating in the presence of a catalyst such as [1,1'-bis(diphenylphosphino)ferrocene]dichloropalladium(II) complex with dichloromethane and an inorganic base such as potassium carbonate in a solvent mixture of dioxane and water.

## Scheme 3

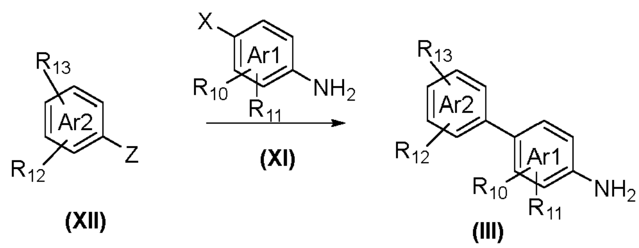

(XII)

(XI)

(III)

Z = Br, Cl
X = $B(OH)_2$, B(pin)

Intermediates of formula **(III)** wherein Ar1, $R_{10}$, $R_{11}$, $R_{12}$ and $R_{13}$ are defined above and Ar2 is an unsubstituted or substituted 2,5-pyrazinyl ring, may be synthesised by coupling under Suzuki conditions of an aromatic halide of general formula **(XII)** and Z represents a halide, to a boronate of general formula **(XI)** where X denotes a dihydroxyboryl or

dialkyloxyboryl group, usually a 4,4,5,5-tetramethyl-1,3,3,2-dioxaborolan-2-yl group. The couplings according to the Suzuki method are performed, for example, by heating in the presence of a catalyst such as tetrakis(triphenylphosphine) palladium or [1,1'-bis(diphenylphosphino)ferrocene]dichloro palladium(II) and an inorganic base such as potassium carbonate in a solvent mixture of dioxane and water.

**Benzamide pyrimidines**

## Scheme 4

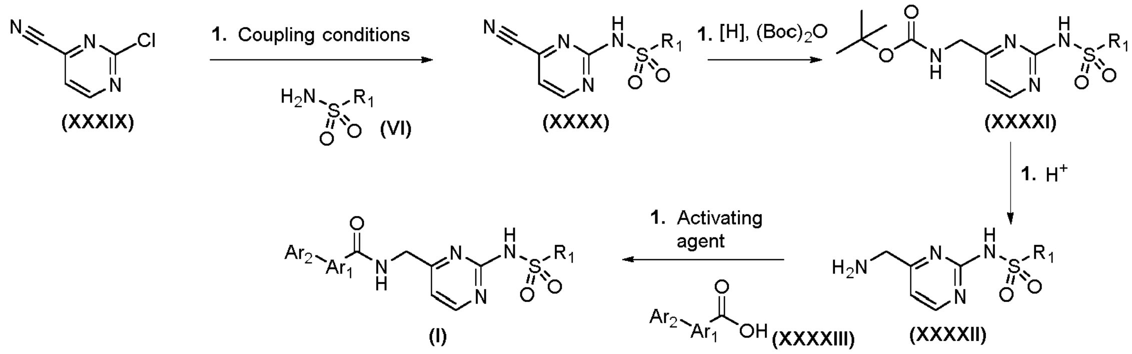


Compounds of general formula (I) may be obtained by a four step process, as shown in Scheme 4. 2-Chloropyrimidine-4-carbonitrile **(XXXIX)** can be converted to the corresponding sulfonamide **(XXXX)** using palladium catalysed sulfamination conditions previously reported in Scheme 1a and 1b of WO2019179652. Reduction of the nitrile group using sodium borohydride in the presence of nickel (II) chloride and di-*tert*-butyl dicarbonate may yield the protected benzylamine derivative of general formula **(XXXXI).** Deprotection can be carried out by acid hydrolysis using HCl in dioxane to yield benzylamine derivative of general formula **(XXXXII).** Amide coupling conditions may then be employed to convert the benzylamine derivative **(XXXXII)** to amides of general formula **(I)** by employing a coupling reagent together with a biaryl carboxylic acid **(XXXXIII)** (commercially available or prepared as in Scheme 19 of WO2019179652 or Scheme 6 herein).

Compounds of general formula **(I)** where A is an amine linker such as -$CH_2NH$-, where $R_1$, $Ar_1$ and $Ar_2$ are defined above, $R_4$ is $C_{1-6}$alkyl and $R_5$ is H or $C_{1-6}$alkyl or $R_4$ and $R_5$ together with the carbon to which they are attached form a $C_{3-6}$cycloalkyl or $C_{3-6}$heterocycloalkyl may be accessed in one step from benzyl amines such as **(XXXXII).** Reaction of **(XXXXII)** with aromatic aldehydes **(LXXII)** in the presence of a hydride source such as sodium triacetoxyborohydride may yield amines of formula **(I).**

## Scheme 5

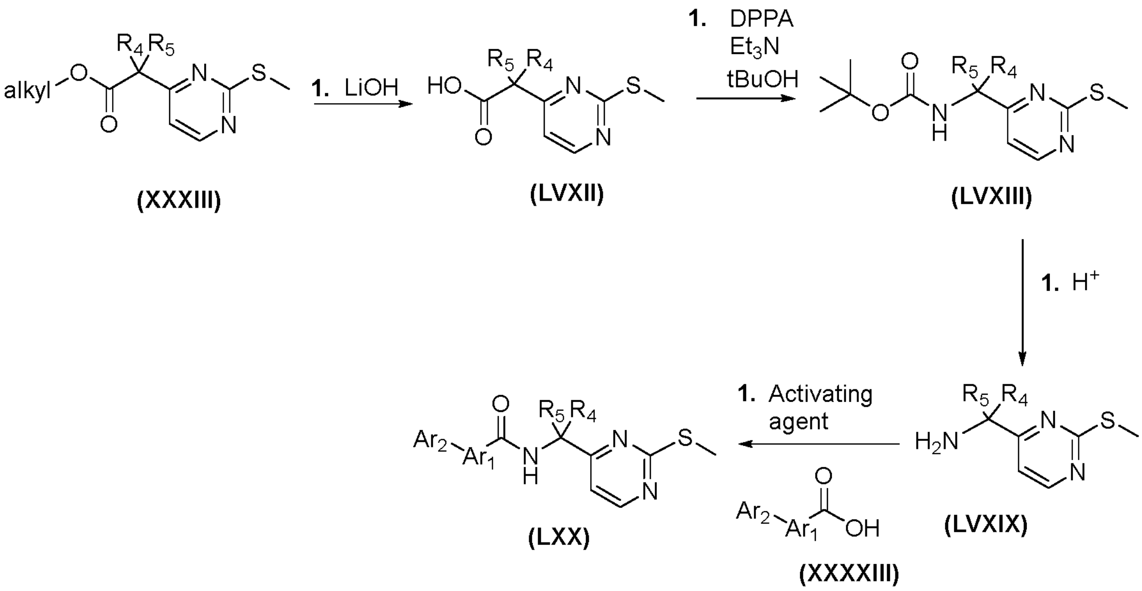


(XXXIII) (LVXII) (LVXIII) (LVXIX) (LXX) (XXXXIII)

Compounds of general formula **(I)** where $R_1$, $Ar_1$ and $Ar_2$ are defined above, $R_4$ is $C_{1-6}$alkyl and $R_5$ is H or $C_{1-6}$alkyl or $R_4$ and $R_5$ together with the carbon to which they are attached form a $C_{3-6}$cycloalkyl or $C_{3-6}$heterocycloalkyl may be obtained by a six step process, as shown in Scheme 5. Firstly, the carboxylic acid **(LVXII)** can be obtained by hydrolysis of methyl ester **(XXXIII)** using an alkali metal base such as lithium hydroxide. Curtius rearrangement can be carried out, for example, using diphenylphosphoryl azide in the presence of propylphosphonic anhydride, triethylamine and tert-butanol to yield carbamates such as **(LVXIII).** Deprotection can be carried out by acid hydrolysis using HCl in dioxane to yield benzylamine derivative of general formula **(LVXIX).** Amide coupling conditions may then be employed to convert the benzylamine derivative **(LVXIX)** to amides of general formula **(LXX)** by employing a coupling reagent together with a biaryl carboxylic acid **(XXXXIII)** (commercially available or prepared as in Scheme 19 of WO2019179652). Compound of formula **(LXX)** can then be progressed to compounds of formula **(I)** following the oxidation, displacement sequence described in Scheme 9a.

## Scheme 6

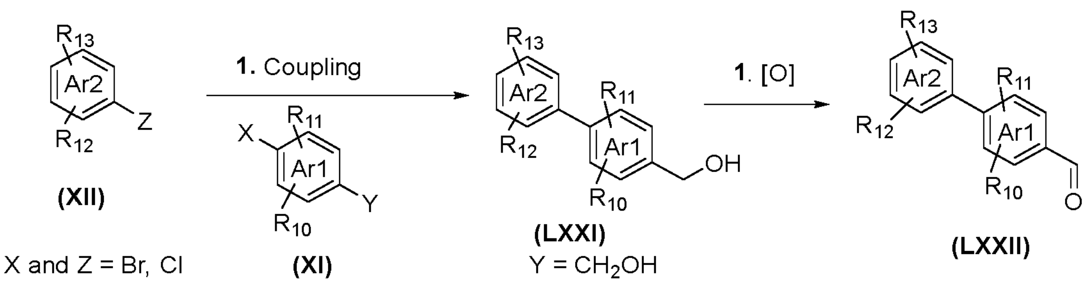


(XII) X and Z = Br, Cl

(XI)

(LXXI) Y = $CH_2OH$

(LXXII)

Intermediates of formula **(LXXII)** where $Ar_2$ is an unsubstituted or substituted 2-pyrazine ring or 3-pyridyl ring, may be synthesised as shown in Scheme 6, in a one-pot, two step procedure starting with borylation of **(XI)**, where X denotes a halogen such as Br or Cl. followed by coupling under Suzuki conditions with an aromatic halide of general formula **(XII)**, of which $R_{12}$ and $R_{13}$ are defined above and Z represents Br or Cl. Initially compounds such as **(XI)**, can be converted to the corresponding boronate using a catalyst such as [1,1'-bis(diphenylphosphino)ferrocene]dichloropalladium(II).$CH_2Cl_2$ adduct and an inorganic base such as potassium acetate in a solvent such as dioxane. Aromatic halide **(XII)** may then be added to the reaction mixture along with an aqueous solution of an inorganic base such as caesium carbonate to yield alcohols of formula **(LXXI)**. The aldehydes of general formula **(LXXII)** are obtained by treatment with an oxidant such as manganese dioxide.

## Scheme 7

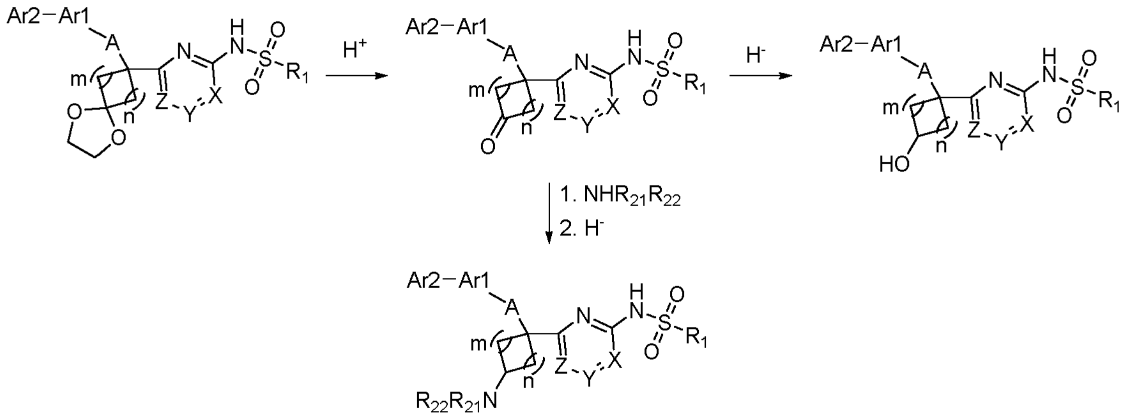


In general compounds of formula **(I)** where $R_4$ and $R_5$ together with the carbon to which they are attached form a 1,4-dioxaspiro[4.5]decane (i.e. m and n are 2) may be treated with a strong acid, such as HCl, to yield cyclic ketones of formula **(I)**. Such ketones may then be treated with a hydride source, such as sodium borohydride, to yield the corresponding exocyclic alcohol or reacted with an amine, such as dimethylamine, followed by sodium triacetoxyborohydride to yield exocyclic amines of formula **(I)**.

## Scheme 8

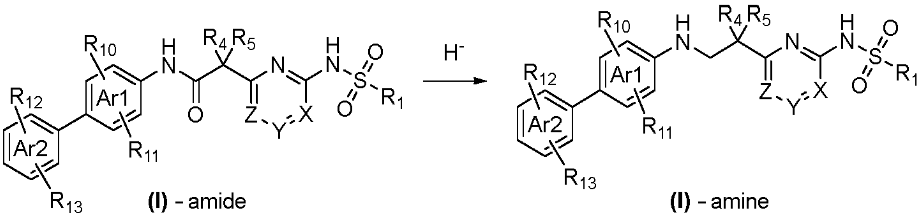


Compounds of formula **(I)** wherein A is $-NR_6CH_2-$ can be obtained from compounds of formula **(I)** wherein A is $-NR_6C(=O)-$, by the reduction of the amide to the amine using a reducing agent such as $LiAlH_4$ in a solvent such as THF.

## Scheme 9a

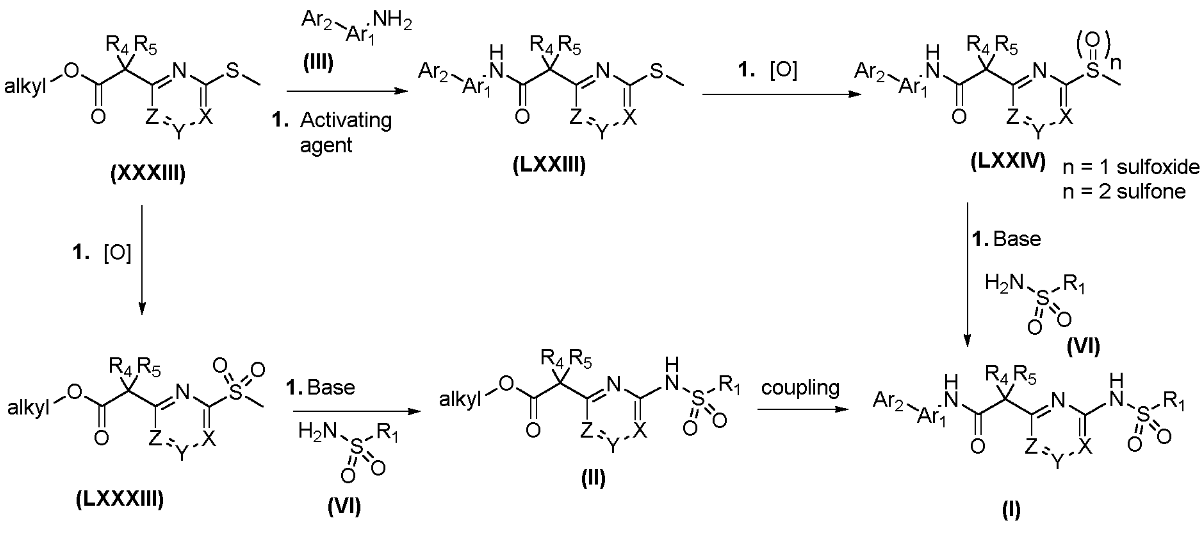


In general and as illustrated in Scheme 9a, compounds of formula **(I)** wherein $R_1$, Ar1 and Ar2 are as defined above, alkyl is $C_{1-4}$alkyl such as methyl or ethyl, e.g. methyl, and for example, $R_4$ and $R_5$ together with the carbon atom to

which they are attached form a $C_{3-6}$heterocycloalkyl ring may be prepared starting from a general intermediate of formula **(XXXIII).** Intermediates such as **(LXXIV)** may be obtained by subjecting compounds such as **(XXXIII)** to amide coupling conditions such as those described in Scheme 9 of WO2019179652 using iPrMgCl. Thioethers of the general formula **(LXXIII)** may be transformed to sulfoxides or sulfones **(LXXIV)** in the presence of an oxidising agent such as *m*CPBA. Displacement of the sulfone group with a primary sulphonamide **(VI)** in the presence of a base such as $Cs_2CO_3$ and a solvent such as *N*-methyl pyrrolidone gives compounds of formula **(I).**

Compounds of formula **(I)** may also be accessed by oxidation of **(XXXIII)** to form sulphone **(LXXXIII),** which can be coupled with **(VI),** and then **(III)** using standard conditions disclosed elsewhere herein to give compounds of formula **(I).**

## Scheme 9b

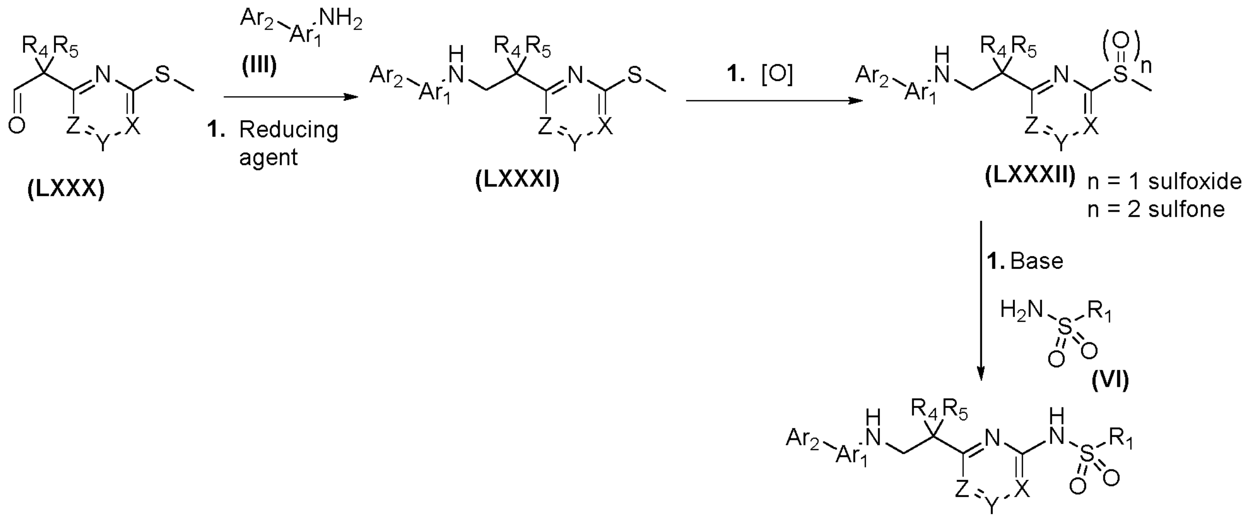


(LXXX) (LXXXI) (LXXXII) (I)

In general and as illustrated in Scheme 9b, compounds of formula **(I)** wherein $R_1$, Ar1 and Ar2 are as defined above, alkyl is $C_{1-4}$alkyl such as methyl or ethyl, e.g. methyl, and for example, $R_4$ and $R_5$ together with the carbon atom to which they are attached form a $C_{3-6}$heterocycloalkyl ring may be prepared starting from a general intermediate of formula **(LXXX)**. Intermediates such as **(LXXXI)** may be obtained by subjecting compounds such as **(LXXX)** and **(III)** to reductive amination conditions such as those described in Scheme 7. Thioethers of the general formula **(LXXXI)** may be transformed to sulfoxides or sulfones **(LXXXII)** in the presence of an oxidising agent such as *m*CPBA. Displacement of the sulfone group with a primary sulphonamide **(VI)** in the presence of a base such as $Cs_2CO_3$ and a solvent such as *N*-methyl pyrrolidone gives compounds of formula (I).

## Scheme 9c

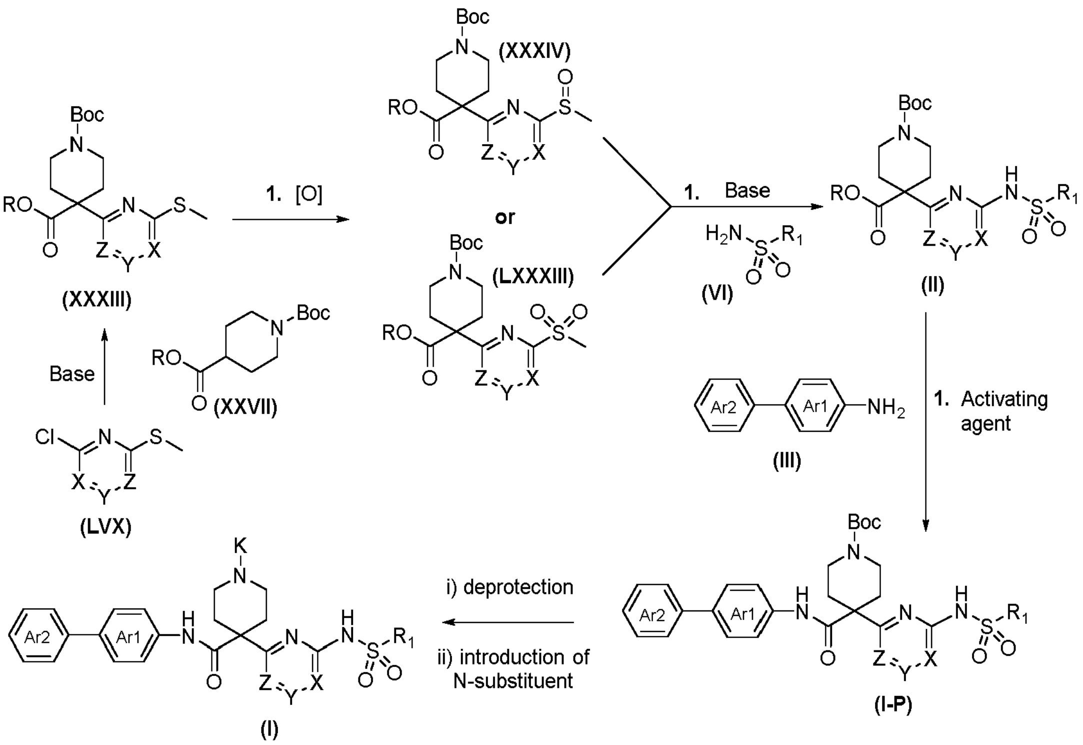


wherein R is H, $C_{1-4}$alkyl (e.g. methyl and ethyl) or benzyl. In general and as illustrated in Scheme 9c, compounds of formula **(I)** wherein $R_1$, X, Y, Z, Ar1 and Ar2 are as defined above and K is $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu, $C_{1-4}$alkylCN such as $CH_2CN$, C(O)$C_{1-3}$alkylO$C_{1-2}$alkyl such as C(O)$CH_2OCH_3$, and $C_{1-2}$alkylC(O)O$C_{1-4}$alkyl such as $CH_2C(O)OCH_2CH_3$, may be prepared in five steps from compounds of formula **(LVX).** An intermediate of formula **(XXXVII)** may be coupled to a compound of formula **(LVX)** in the presence of a base such as LiHMDS to give a compound of formula **(XXXIII).** Thioethers of the general formula **(XXXIII)** may be transformed to a sulfoxide of formula **(XXXIV)** or a sulfone of formula **(LXXXIII)** in the presence of an oxidising agent such as mCPBA. The skilled person will appreciate that formation of the sulfoxide or sulfone can be controlled by varying the number of equivalents of oxidising agent used, the length of reaction and/or the temperature of the reaction. Displacement of the sulfoxide group or the sulfone group with a primary sulfonamide (**VI**) in the presence of a base such as $Cs_2CO_3$ and a solvent such as N-methyl pyrrolidone gives compounds of formula **(II)**. Compounds of formula **(I)** may be obtained by a strong base-mediated amide formation between compounds **(II)** and **(III)** at room temperature using bases such as iPrMgCl, LiHMDS or KOtBu, to give compounds of formula **(I-P),** followed by removal of the Boc group using a strong acid such as TFA, and introduction of the N-substituent K. Introduction of K may be performed by reaction of the free NH group with K-LG wherein LG is a leaving group such as halo e.g. chloro or bromo, under standard conditions known to the skilled person, or by any other N-substituent forming conditions known to the skilled person (such as Mitsunobu conditions, reductive amination or N-acylation), to give a compound of formula **(I).**

## Scheme 10

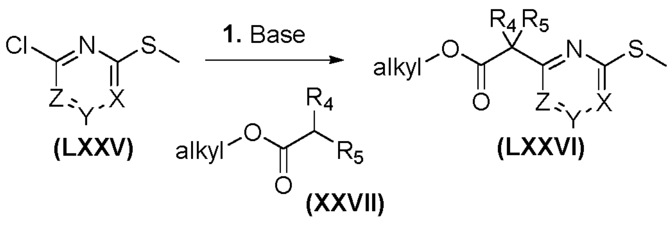


In general and as illustrated in Scheme 10, compounds of formula **(I)** wherein $R_1$, Ar1 and Ar2 are as defined

above, alkyl is $C_{1-4}$alkyl such as methyl or ethyl, e.g. methyl, and for example, $R_4$ and $R_5$ together with the carbon atom to which they are attached form a $C_{3-6}$heterocycloalkyl ring may be prepared starting from chloro-pyrimidine **(LXXV)**. Intermediates **(XXXVII)** are coupled to chloro-pyrimidine **(LXXV)** in the presence of a base such as LiHMDS to give intermediates **(LXXVI).** Thioethers of the general formula **(LXXVI)** may then be transformed to compounds of formula (I) following the route described in Scheme 9a of WO2019179652.

## Scheme 11

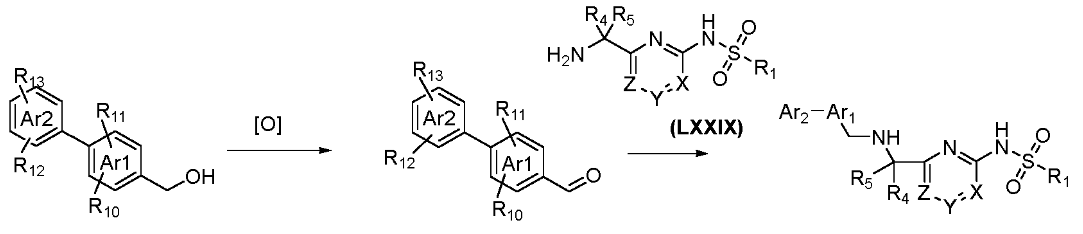


**(LXXI)** **(LXXII)** **(I)**

In general and as illustrated in Scheme 11, compounds of formula **(I)** wherein $R_1$, Ar1 and Ar2 are as defined above, $R_4$ and $R_5$ together with the carbon atom to which they are attached form a $C_{3-6}$heterocycloalkyl ring may be prepared starting from alcohols **(LXXI),** which as oxidised to aldehydes **(LXXII)** in the presence of $MnO_2$ in a non-protic solvent such as DCM. Reductive coupling of amine **(LXXIX)** and aldehyde **(LXXII)** in the presence of a hydride source such as sodium triacetoxyborohydride in an aprotic solvent such as DCM in the presence of a proton source such as acetic acid affords compounds of formula **(I)** following the route described in Scheme 11.

## Scheme 12

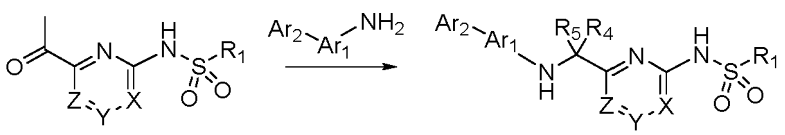

Compounds of formula **(I)** wherein A is -NH- and $R_4$ or $R_5$ is H may be prepared by reductive coupling of the appropriate amine and aldehyde in the presence of a hydride source such as sodium triacetoxyborohydride.

Compounds of formula (I-b)

Compounds of formula (I-b) may be synthesised by general and specific methods disclosed in WO2019/106156 and those disclosed below. For the avoidance of doubt, reference to variables in each of the schemes in this section encompasses variables specific to compounds of formula (I-b). For example, $R_1$ includes $R_{1b}$. Furthermore, reference to intermediates in each of the schemes in this section encompasses corresponding intermediates for compounds of formula (I-b). For example, reference to compounds of formula (II) encompasses compounds of formula (II-b).

## Scheme 13

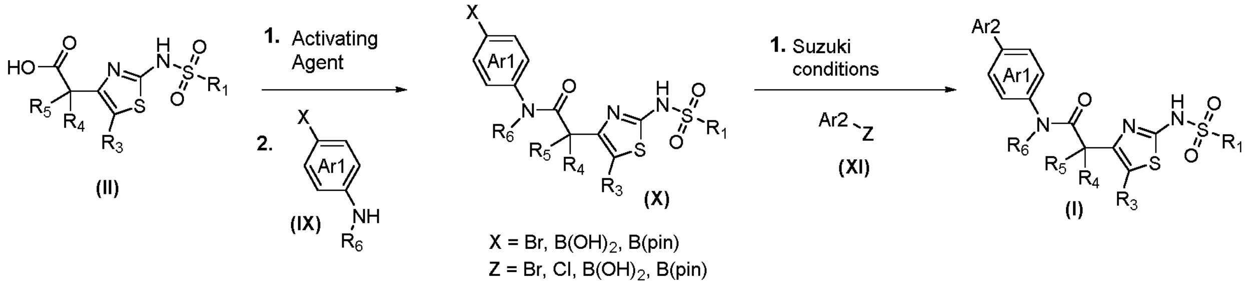


**(II)** **(IX)** **(X)** **(XI)** **(I)**

Compounds of formula **(I)** may be obtained by a general process whereby a carboxylic acid precursor **(II)**, or a suitably protected derivative thereof, is reacted with an activating agent, to generate a reactive, electrophilic carboxylic

acid derivative, followed by subsequent reaction with an amine of formula **(IX)**. Intermediates of formula **(X)** are then converted to a compound of the invention of general formula **(I)** by coupling under Suzuki conditions with an aromatic halide or boronate of general formula **(XI)**, of which X is defined above and represents usually a bromide, a dihydroxyboryl or dialkyloxyboryl group, usually a 4,4,5,5-tetramethyl-1,3,3,2-dioxaborolan-2-yl group. The couplings according to the Suzuki method are performed, for example, by heating in the presence of a catalyst such as bis(diphenylphosphino) ferrocene]dichloropalladium(II) and an inorganic base such as potassium carbonate in a solvent mixture of dioxane and water. It will be understood by persons skilled in the art that many catalysts and conditions can be employed for such couplings.

## Scheme 14

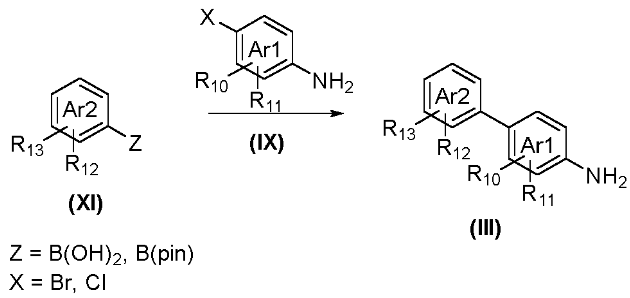


Intermediates of formula **(III)** where Ar2 is an unsubstituted or substituted 3-pyridyl ring, may be synthesised by coupling under Suzuki conditions of a boronate of general formula **(XI)**, of which $R^{12}$ and $R^{13}$ are defined above and **Z** represents a dihydroxyboryl or dialkyloxyboryl group, usually a 4,4,5,5-tetramethyl-1,3,3,2-dioxaborolan-2-yl group, to a substituted pyridine of formula **(IX)** of which $R^{10}$ and $R^{11}$ are defined above and where **X** denotes a halide. The couplings according to the Suzuki method are performed, for example, by heating in the presence of a catalyst such as [1,1'-bis(diphenylphosphino)ferrocene]dichloropalladium(II) complex with dichloromethane and an inorganic base such as potassium carbonate in a solvent mixture of 1,4-dioxane and water.

## Scheme 15

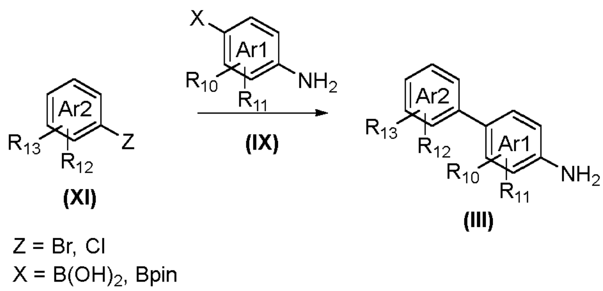


Intermediates of formula **(III)** where Ar2 is an unsubstituted or substituted 2-pyrazine ring, may be synthesised by coupling under Suzuki conditions of an aromatic halide of general formula **(XI)**, of which $R^{12}$ and $R^{13}$ are defined above and **Z** represents a halide, to a boronate of general formula **(IX)** of which $R^{10}$ and $R^{11}$ are defined above and where **X** denotes a dihydroxyboryl or dialkyloxyboryl group, usually a 4,4,5,5-tetramethyl-1,3,3,2-dioxaborolan-2-yl group. The couplings according to the Suzuki method are performed, for example, by heating in the presence of a catalyst such as tetrakis(triphenylphosphine)palladium or [1,1'-bis(diphenylphosphino) ferrocene]dichloropalladium(II) and an inorganic base such as potassium carbonate in a solvent mixture of dioxane and water.

## Scheme 16

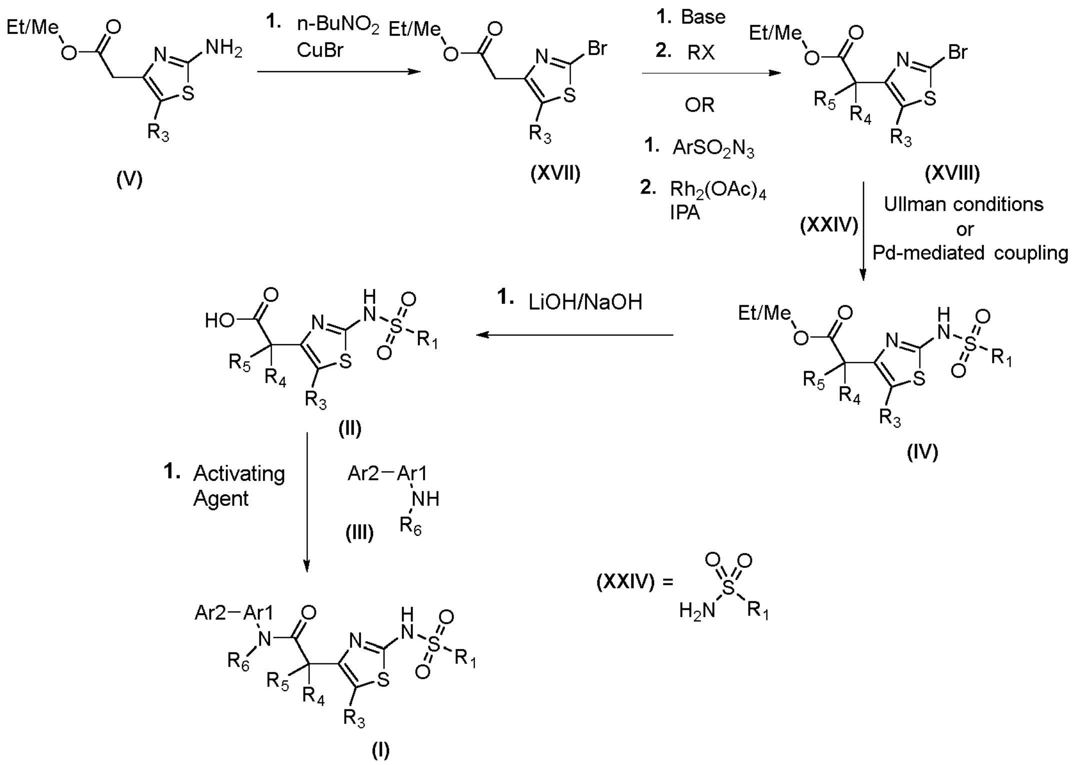

(V) (XVII) (XVIII) (IV) (II) (III) (I)

Compound of general formula **(XVII)** may be prepared by conversion of a suitable 2-(2-aminothiazol-4-yl)acetate derivative, such as a methyl or ethyl derivative, by a Sandmeyer type reaction using an organic nitrite, such as n-butylnitrite, in the presence of a halide source, such as Cu(I)Br in acetonitrile. Such reactions can be undertaken at temperatures of RT to 60 °C. Introduction of $R_4/R_5$ can be undertaken by two alternative methods at this stage. Firstly, alkylation of compounds of general formula **(XVII)** can be undertaken by addition of a suitable base, for example, LiHMDS, together with an alkylating agent, such as iodomethane which results in dialkylation alpha to the ester moiety to yield compounds of formula **(XVIII),** where $R_{4b} = R_{5b}$ = Me. Secondly, diazotisation of compounds of general formula **(XVII)** with the use of an diazo transfer reagent, such as 4-acetamidobenzenesulfonyl azide, under basic conditions, followed by treatment with rhodium and subsequent insertion of the corresponding nucleophile, such as isopropyl alcohol, gives intermediates of general formula **(XVIII)** where $R_{4b}$ = Oisopropoxy and $R_{5b}$ = H.

Introduction of the sulfonamide group in the preparation of compounds of formula **(IV)** may be achieved by an Ullmann coupling reaction i.e. Cu mediated coupling conditions using amines of formula **(XXIV)** and a copper catalyst, such as Cu(I)I, in the presence of an inorganic base, potassium carbonate, and a diamine ligand in dioxane. Such reactions are typically carried out at elevated temperatures such as 80 °C. Alternatively, conversion of compounds of formula **(XVIII)** to **(IV)** can be achieved via a palladium mediated coupling, for example using a catalyst such as [t-BuXPhos Pd(allyl)]OTf and substituted sulfonamide nucleophile **(XXIV),** in the presence of an inorganic base, for example potassium carbonate to form compounds of formula **(IV)**. Palladium mediatiated coupling conditions are particularly useful when $R_4$ and $R_5$ together with the carbon atom to which they are attached form a 5- or 6-membered heterocycloalkyl, such as a tetrahydropyranyl. The alkyl esters of formula (IV) may be conveniently converted to compounds of formula **(I)** according to synthetic steps reported in Scheme 13.

## Scheme 17

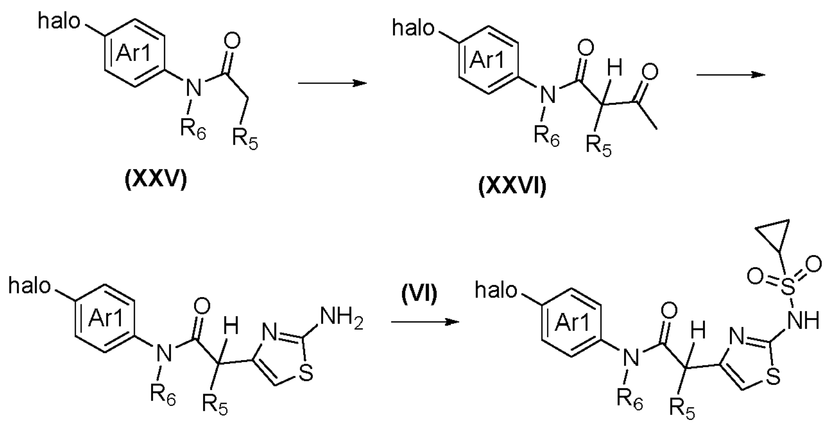


(XXV) (XXVI) (XXVII) (XXVIII)

wherein halo is, for example, bromo. Compounds of formula **(XXVII),** for example, when $R_{4b}$ is H and $R_{5b}$ and $R_6$ are a $C_{2-3}$alkylene chain forming a 5- or 6-membered ring, can be accessed in three steps from compounds of formula **(XXV)**. Acylation of compounds of formula **(XXV)** in the presence of a strong base such as LDA followed by a quench with an $^{-}$OAc source such as EtOAc provides compounds of formula **(XXVI).** Compounds of formula **(XXVII)** can be made from **(XXVI)** following addition of bromine and quench with a suitable thiourea, before a coupling with sulfonyl chlorides of formula **(VI)** to give compounds of formula **(XXVIII).** Compounds of formula **(I)** may be accessed using conditions set out in Scheme 14.

## Scheme 18

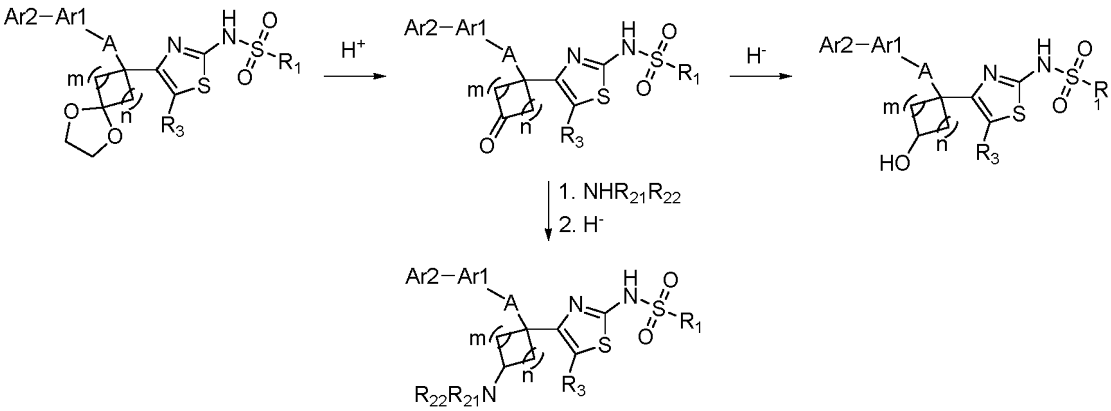


In general compounds of formula **(I)** where $R_4$ and $R_5$ together with the carbon to which they are attached form a 1,4-dioxaspiro[4.5]decane (i.e. m is 2 and n is 2) may be treated with a strong acid such as HCl to yield cyclic ketones of formula **(I)**. Such ketones may then be treated with a hydride source such as sodium borohydride to yield the corresponding exocyclic alcohol or reacted with an amine such as dimethylamine followed by sodium triacetoxyborohydride to yield exocyclic amines of formula **(I).**

## Scheme 19

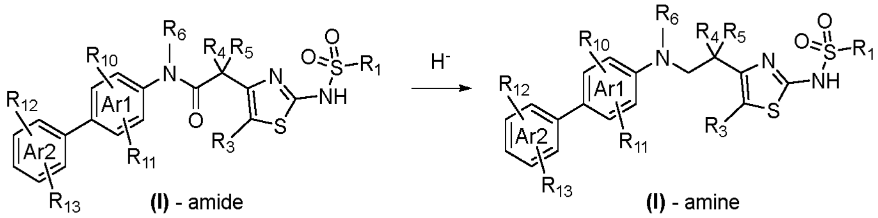


**(I)** - amide **(I)** - amine

Compounds of formula (I) wherein A is -$NR_6CH_2$- can be obtained from compounds of formula **(I)** wherein A is -$NR_6C$(=O)-, by the reduction of the amide to the amine using a reducing agent such as $LiAlH_4$ in a solvent such as THF.

## Scheme 20

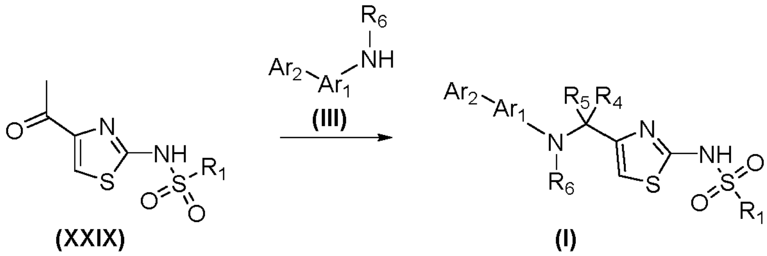


(XXIX) (I)

Compounds of formula **(I)** wherein A is -$NR_6$- and $R_5$ or $R_4$ are H can be obtained in a single step by a reductive amination between ketones of formula **(XXIX)** and amines of formula **(III)** in the presence of a hydride source such as sodium triacetoxyborohydride.

Compounds of formula (I-c)

Compounds of formula (I-c) may be synthesised by general and specific methods disclosed in WO2019/106146 and those disclosed below. For the avoidance of doubt, reference to variables in each of the schemes in this section encompasses variables specific to compounds of formula (I-c). For example, $R_1$ includes $R_{1c}$. Furthermore, reference to intermediates in each of the schemes in this section encompasses corresponding intermediates for compounds of formula (I-c). For example, reference to compounds of formula (II) encompasses compounds of formula (II-c).

## Scheme 21

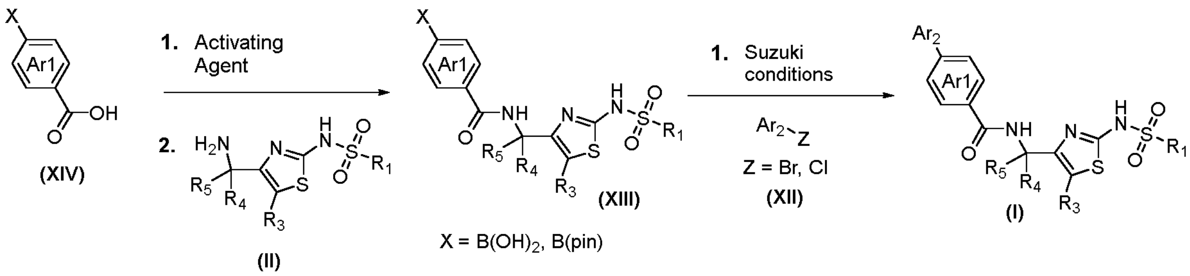


(XIV) (II) (XIII) (I)

Compounds of formula **(XIII)** may be obtained by a general process as shown in Scheme 21 whereby a carboxylic acid precursor **(XIV)** is reacted with an activating agent such as HATU, T3P or Ghosez's reagent, to generate a reactive, electrophilic carboxylic acid derivative, followed by subsequent reaction with an amine of formula **(II)**. Intermediates of formula **(XIII)** are then converted to a compound of general formula **(I)** by coupling under Suzuki conditions with an aromatic halide of general formula **(XII),** of which X is defined in Scheme 21 and represents a dihydroxyboryl or dialkyloxyboryl group, such as a 4,4,5,5-tetramethyl-1,3,3,2-dioxaborolan-2-yl group. The couplings according to the Suzuki method are performed, for example, by heating in the presence of a catalyst such as bis(diphenylphosphino) ferrocene]dichloropalladium(II).$CH_2Cl_2$ adduct and an inorganic base such as potassium carbonate in a solvent mixture of dioxane and water under an inert atmosphere such as a nitrogen atmosphere. It will be understood by persons skilled in the art that many catalysts and conditions can be employed for such couplings.

## Scheme 22

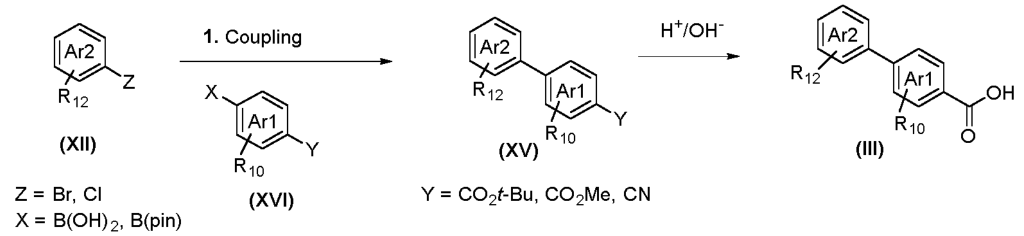


(XII) (XVI) (XV) (III)

Intermediates of formula **(III)** where $Ar_2$ is an unsubstituted or substituted 2-pyrazine ring or 3-pyridyl ring, may be synthesised as shown in Scheme 22 by coupling under Suzuki conditions of an aromatic halide of general formula **(XII)**, of which $R_{10}$ and $R_{12}$ are defined above and Z represents a halide such as Br or Cl, to a boronate of general formula **(XVI)** where X denotes a dihydroxyboryl or dialkyloxyboryl group, such as a 4,4,5,5-tetramethyl-1,3,3,2-dioxaborolan-2-yl group. The couplings according to the Suzuki method are performed, for example, by heating in the presence of a catalyst such as [1,1'-bis(diphenylphosphino)ferrocene]dichloropalladium(II).$CH_2Cl_2$ adduct and an inorganic base such as cesium carbonate in a solvent mixture of dioxane and water under an inert atmosphere such as a nitrogen atmosphere. The carboxylic acids of general formula **(III)** are obtained by either deprotection of the t-butyl ester using a strong acid, such as TFA in a solvent of $CH_2Cl_2$, hydrolysis of the methyl ester using an alkali metal hydroxide such as NaOH in a solvent mixture such as THF/MeOH or hydrolysis of the nitrile using a strong acid such as concentrated HCl. Compounds of formula **(III-A)** may also be made using this method.

## Scheme 23

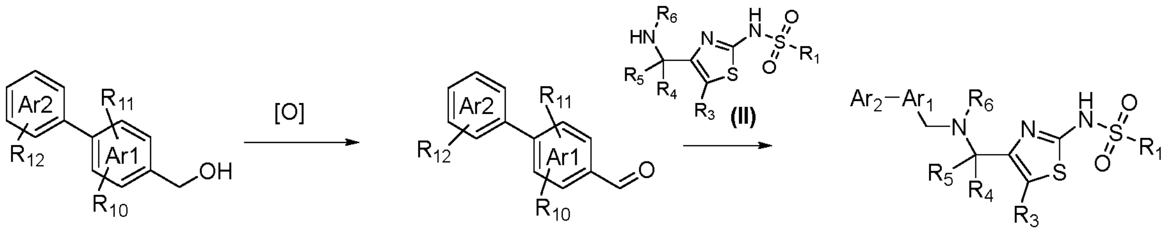


(II)

(XXII) (XXIII) (I)

Compounds of formula **(I)** wherein $A_a$ is $-CH_2NR_6-$ can be accessed in two steps from compounds of formula **(XXII)**. Oxidation of the alcohol **(XXII)** under standard oxidation conditions such as $MnO_2$ gives aldehyde **(XXIII)**. A reductive amination using a hydride source such as sodium triacetoxyborohydride between aldehyde **(XXIII)** and amine **(II)** gives compounds of formula **(I)**.

## Scheme 24

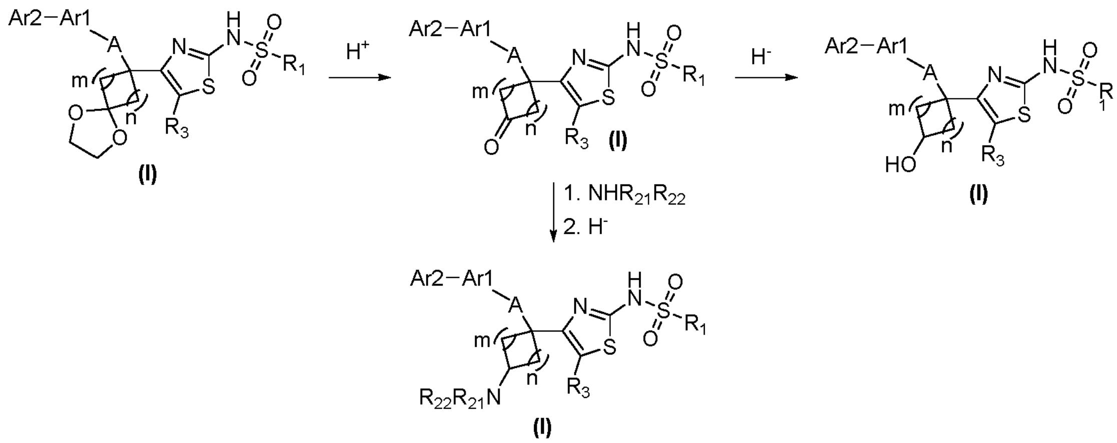


(I) (I) (I) (I)

In general compounds of formula **(I)** where $R_{4a}$ and $R_{5a}$ together with the carbon to which they are attached form a $C_{3-6}$cycloalkyl wherein one of the carbons of the $C_{3-6}$cycloalkyl is a spiro centre such that a spirocyclic ring system is formed by the $C_{3-6}$cycloalkyl ring and a $C_{3-6}$heterocycloalkyl ring, such as a 1,4-dioxaspiro[4.5]decane moiety (m is 2 and n is 2), may be treated with a strong acid such as HCl to yield cyclic ketones of formula **(I)**. Such ketones may then be treated with a hydride source such as sodium borohydride to yield the corresponding exocyclic alcohol or reacted with an amine such as dimethylamine followed by sodium triacetoxyborohydride to yield exocyclic amines of formula **(I)**.

**Intermediates of the Invention**

<u>Compounds of formula (I-a)</u>

The present invention provides intermediates of the following general formulae, wherein unless otherwise stated, the variable groups and associated preferences are as defined previously for compounds of formula **(I-a):**

- a compound of formula **(II-a):**

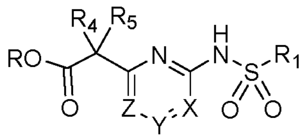

**(II-a)**

wherein X, Y and Z are as defined herein, R is H, $C_{1-6}$alkyl (e.g. methyl and ethyl) or benzyl, $R_1$ is $R_{1aa}$ and/or $R_4$ and $R_5$ are $R_{4aa}$ and $R_{5aa}$;

- a compound of formula **(XX-a):**

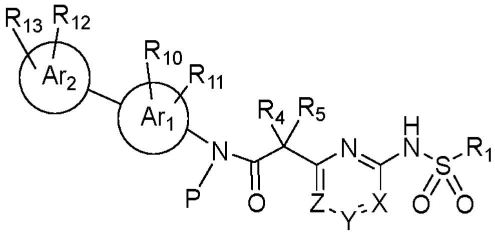

**(XX-a)**

wherein X, Y, Z, $R_{10}$, $R_{11}$, $R_{12}$, $R_{13}$, $Ar_1$ and $Ar_2$ are as defined herein, P is a nitrogen protecting group such as para-methoxybenzyl, $R_1$ is $R_{1aa}$ and/or $R_4$ and $R_5$ are $R_{4aa}$ and $R_{5aa}$;

- a compound of formula **(XXIV-a):**

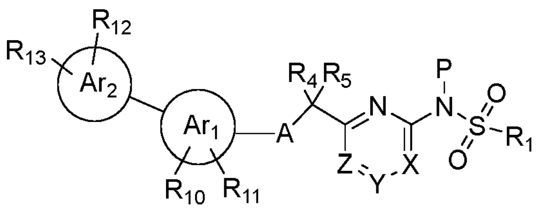

**(XXIV-a)**

wherein X, Y, Z, $R_{10}$, $R_{11}$, $R_{12}$, $R_{13}$, A, $Ar_1$ and $Ar_2$ are as defined herein, P is a nitrogen protecting group such as para-methoxybenzyl, $R_1$ is $R_{1aa}$ and/or $R_4$ and $R_5$ are $R_{4aa}$ and $R_{5aa}$;

- a compound of formula **(XXXI-a):**

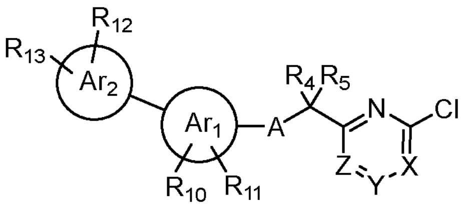

**(XXXI-a)**

wherein X, Y, Z, $R_{10}$, $R_{11}$, $R_{12}$, $R_{13}$, $Ar_1$ and $A_2$ are as defined herein, A is $A_a$ and/or $R_4$ and $R_5$ are $R_{4aa}$ and $R_{5aa}$;

- a compound of formula **(XXXXII-a):**

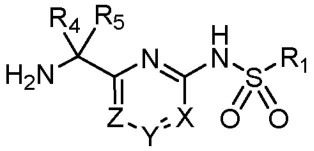

**(XXXXII-a)**

wherein X, Y and Z are as defined herein, $R_1$ is $R_{1aa}$ and/or $R_4$ and $R_5$ are $R_{4aa}$ and $R_{5aa}$;

- a compound of formula **(LI-a):**

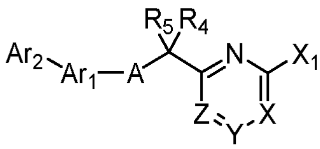

**(LI-a)**;

wherein X, Y, Z, $Ar_1$ and $Ar_2$ are as defined herein, $X_1$ is Cl or Br, A is $A_{aa}$ and/or $R_4$ and $R_5$ are $R_{4aa}$ and $R_{5aa}$;

- a compound of formula **(LVIII-a):**

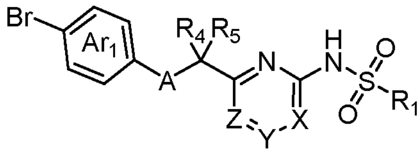

**(LVIII-a)**

wherein X, Y, Z, and $Ar_1$ are as defined herein, A is $A_a$ and/or $R_1$ is $R_{1aa}$ and/or $R_4$ and $R_5$ are $R_{4aa}$ and $R_{5aa}$;

- a compound of formula **(XXXIII-a):**

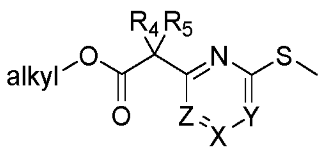

**(XXXIII-a)**;

wherein X, Y and Z are as defined herein, alkyl is $C_{1-4}$alkyl such as methyl or ethyl, e.g. methyl and $R_4$ and $R_5$ are $R_{4aa}$ and $R_{5aa}$;

- a compound of formula **(LXXIII-a):**

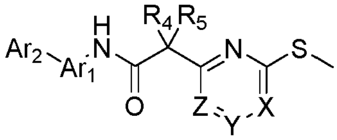

**(LXXIII-a)**

wherein X, Y, Z, $A_1$, $Ar_2$ are as defined herein and $R_4$ and $R_5$ are $R_{4aa}$ and $R_{5aa}$;

- a compound of formula **(LXXIV-a):**

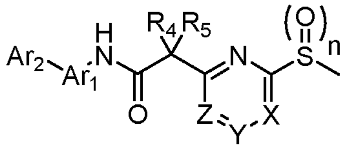

**(LXXIV-a)** n = 1 sulfoxide
n = 2 sulfone

wherein X, Y, Z, $Ar_1$ and $Ar_2$ are as defined herein, and $R_4$ and $R_5$ are $R_{4aa}$ and $R_{5aa}$;

- a compound of formula **(LXXXIII-a):**

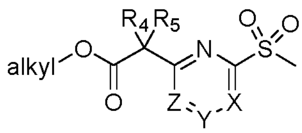

**(LXXXIII-a)**

wherein X, Y and Z are as defined herein, alkyl is $C_{1-4}$alkyl such as methyl or ethyl, e.g. methyl and $R_4$ and $R_5$ are $R_{4aa}$ and $R_{5aa}$;

- a compound of formula **(XXXIV-a):**

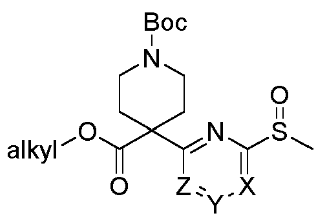

**(XXXIV-a)**

wherein X, Y and Z are as defined herein, and alkyl is $C_{1-4}$alkyl such as methyl or ethyl, e.g. methyl;

Compounds of formula (I) may be considered intermediates for further compounds of formula (I), as described in the Examples below.

Included as an aspect of the invention are all novel intermediates described in the examples, including:

- Intermediates INTC186 to INTC218; and

- Intermediates INTC232 to INTC247.

Included as an aspect of the invention are salts such as pharmaceutically acceptable salts of any one of the intermediates claimed herein.

Compounds of formula (I-b)

The present invention also provides intermediates of the following general formulae, wherein unless otherwise stated, the variable groups and associated preferences are as defined previously for compounds of formula **(I-b):**

- Compounds of formula **(II-b)**

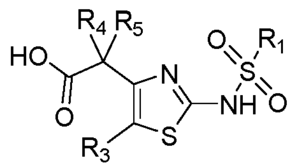

wherein $R_3$, is as defined herein $R_1$ is $R_{1ab}$ and/or $R_4$ and $R_5$ are $R_{4ab}$ and $R_{5ab}$;

- Compounds of formula **(IV-b)**

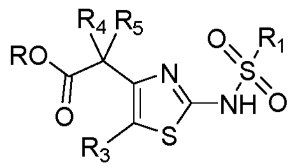

wherein $R_3$ is as defined herein, R is $C_{1-6}$alkyl (e.g. methyl, ethyl) or benzyl, $R_1$ is $R_{1ab}$ and/or $R_4$ and $R_5$ are $R_{4ab}$ and $R_{5ab}$.

Included as an aspect of the invention are salts such as pharmaceutically acceptable salts of any one of the intermediates claimed herein.

Compounds of formula (I-c)

The present invention also provides intermediates of the following general formulae, wherein unless otherwise stated, the variable groups and associated preferences are as defined previously for compounds of formula **(I-c):**

- a compound of formula **(II-c):**

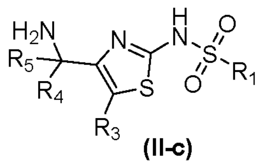

**(II-c)**

wherein $R_3$ is as defined herein, $R_1$ is $R_{1ac}$ and/or $R_4$ and $R_5$ are $R_{4ac}$ and $R_{5ac}$;

- a compound of formula **(VIII-c)**:

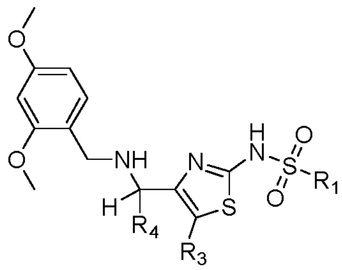

**(VIII-c)**

wherein $R_3$ and $R_4$ are as defined herein and $R_1$ is $R_{1ac}$.

Included as an aspect of the invention are salts such as pharmaceutically acceptable salts of any one of the intermediates claimed herein.

**Therapeutic Methods**

In the following section, reference to compounds of formula (I) encompasses compounds of formula (I-a), (I-b) and (I-c).

Compounds of formula (I) of the present invention have utility as inhibitors of CTPS1.

Therefore, the invention also provides a compound of formula (I), or a pharmaceutically acceptable salt and/or solvate (e.g. salt), for use as a medicament, in particular in the treatment or prophylaxis of a disease or disorder wherein an inhibitor of CTPS1 is beneficial, for example those diseases and disorders mentioned herein below.

More suitably, the disease or disorder wherein an inhibitor of CTPS1 is beneficial is a disease or disorder wherein a reduction in T-cell and/or B-cell proliferation would be beneficial.

The invention also provides a compound of formula (I), or a pharmaceutically acceptable salt and/or solvate (e.g. salt) thereof, for use in the inhibition of CTPS1 in a subject.

The invention also provides a compound of formula (I), or a pharmaceutically acceptable salt and/or solvate (e.g. salt) thereof, for use in the reduction of T-cell and/or B-cell proliferation in a subject.

More suitably, the disease or disorder wherein an inhibitor of CTPS1 is beneficial is a disease or disorder wherein a reduction in T-cell and/or B-cell proliferation would be beneficial.

The term 'treatment' or 'treating' as used herein includes the control, mitigation, reduction, or modulation of the disease state or its symptoms.

The term 'prophylaxis' or 'preventing' is used herein to mean preventing symptoms of a disease or disorder in a subject or preventing recurrence of symptoms of a disease or disorder in an afflicted subject and is not limited to complete prevention of an affliction.

Suitably, the disease or disorder is selected from rejection of transplanted cells and tissues, Graft-related diseases or disorders, allergies and autoimmune diseases.

In one embodiment the disease or disorder is the rejection of transplanted cells and tissues. The subject may have been transplanted with a graft selected from the group consisting of heart, kidney, lung, liver, pancreas, pancreatic islets, brain tissue, stomach, large intestine, small intestine, cornea, skin, trachea, bone, bone marrow (or any other source of hematopoietic precursor cells and stem cells including hematopoietic cells mobilized from bone marrow into peripheral blood or umbilical cord blood cells), muscle, or bladder. The compounds of the invention may be of use in preventing or suppressing an immune response associated with rejection of a donor tissue, cell, graft or organ transplant in a subject.

In a further embodiment the disease or disorder is a Graft-related disease or disorder. Graft-related diseases or disorders include graft versus host disease (GVHD), such as GVHD associated with bone marrow transplantation, and immune disorders resulting from or associated with rejection of organ, tissue, or cell graft transplantation (e.g., tissue or cell allografts or xenografts), including, e.g., grafts of skin, muscle, neurons, islets, organs, parenchymal cells of the liver, etc, and Host-Versus-Graft-Disease (HVGD). The compounds of the invention may be of use in preventing or suppressing acute rejection of such transplant in the recipient and/or for long-term maintenance therapy to prevent rejection of such transplant in the recipient (e.g., inhibiting rejection of insulin-producing islet cell transplant from a donor in the subject recipient suffering from diabetes). Thus the compounds of the invention have utility in preventing Host-Versus-Graft-Disease (HVGD) and Graft-Versus-Host-Disease (GVHD).

A CTPS1 inhibitor may be administered to the subject before, after transplantation and/or during transplantation. In some embodiments, the CTPS1 inhibitor may be administered to the subject on a periodic basis before and/or after transplantation.

In another embodiment, the disease or disorder is an allergy.

In additional embodiments the immune related disease or disorder is an autoimmune disease. As used herein, an "autoimmune disease" is a disease or disorder directed at a subject's own tissues. Examples of autoimmune diseases include, but are not limited to Addison's Disease, Adult-onset Still's disease, Alopecia Areata, Alzheimer's disease, Anti-neutrophil Cytoplasmic Antibodies (ANCA)-Associated Vasculitis, Ankylosing Spondylitis, Anti-phospholipid Syndrome (Hughes' Syndrome), Aplastic Anemia, Arthritis, Asthma, Atherosclerosis, Atherosclerotic plaque, Atopic Dermatitis, Autoimmune Hemolytic Anemia, Autoimmune Hepatitis, Autoimmune Hypophysitis (Lymphocytic Hypophysitis), Autoimmune Inner Ear Disease, Autoimmune Lymphoproliferative Syndrome, Autoimmune Myocarditis, Autoimmune Neutropenia, Autoimmune Oophoritis, Autoimmune Orchitis, Auto-Inflammatory Diseases requiring an immunosuppressive treatment, Azoospermia, Bechet's Disease, Berger's Disease, Bullous Pemphigoid, Cardiomyopathy, Cardiovascular disease, Celiac disease including Refractory Celiac Disease (type I and type II), Chronic Fatigue Immune Dysfunction Syndrome (CFIDS), Chronic Idiopathic Polyneuritis, Chronic Inflammatory Demyelinating Polyneuropathy (CIPD),

Chronic Relapsing Polyneuropathy (Guillain-Barré syndrome), Churg-Strauss Syndrome (CSS), Cicatricial Pemphigoid, Cold Agglutinin Disease (CAD), chronic obstructive pulmonary disease (COPD), CREST Syndrome, Cryoglobulin Syndromes, Cutaneous Lupus, Dermatitis Herpetiformis, Dermatomyositis, Eczema, Epidermolysis Bullosa Acquisita, Essential Mixed Cryoglobulinemia, Evan's Syndrome, Exophthalmos, Fibromyalgia, Goodpasture's Syndrome, Grave's disease, Hemophagocytic Lymphohistiocytosis (HLH) (including Type 1 Hemophagocytic Lymphohistiocytosis), Histiocytosis/Histiocytic Disorders, Hashimoto's Thyroiditis, Idiopathic Pulmonary Fibrosis, Idiopathic Thrombocytopenia Purpura (ITP), IgA Nephropathy, Immunoproliferative Diseases or Disorders, Inflammatory Bowel Disease (IBD), Interstitial Lung Disease, Juvenile Arthritis, Juvenile Idiopathic Arthritis (JIA), Kawasaki's Disease, Lambert-Eaton Myasthenic Syndrome, Lichen Planus, Localized Scleroderma, Lupus Nephritis, Ménière's Disease, Microangiopathic Hemoytic Anemia, Microscopic Polyangitis, Miller Fischer Syndrome/Acute Disseminated Encephalomyeloradiculopathy, Mixed Connective Tissue Disease, Multiple Sclerosis (MS), Muscular Rheumatism, Myalgic Encephalomyelitis (ME), Myasthenia Gravis, Ocular Inflammation, Pemphigus Foliaceus, Pemphigus Vulgaris, Pernicious Anemia, Polyarteritis Nodosa, Polychondritis, Polyglandular Syndromes (Whitaker's syndrome), Polymyalgia Rheumatica, Polymyositis, Primary Agammaglobulinemia, Primary Biliary Cirrhosis/Autoimmune Cholangiopathy, Primary Glomerulonephritis, Primary Sclerosing Cholangitis, Psoriasis, Psoriatic Arthritis, Pure Red Cell Anemia, Raynaud's Phenomenon, Reiter's Syndrome/Reactive Arthritis, Relapsing Polychondritis, Restenosis, Rheumatic Fever, Rheumatic Disease, Rheumatoid Arthritis, Sarcoidosis, Schmidt's Syndrome, Scleroderma/Systemic Sclerosis, Sjörgen's Syndrome, Stiff-Man Syndrome, The Sweet Syndrome (Febrile Neutrophilic Dermatosis), Systemic Lupus Erythematosus (SLE), Systemic Scleroderma, Takayasu Arteritis, Temporal Arteritis/Giant Cell Arteritis, Thyroiditis, Type 1 diabetes, Type 2 diabetes, Uveitis, Vasculitis, Vitiligo, Wegener's Granulomatosis, and X-linked lymphoproliferative disease.

Of particular interest are diseases and disorders which are mainly driven by T-cell activation and proliferation, including:

- diseases and disorders which are not linked to alloreactivity including:
    - Alopecia areata, atopic dermatitis, eczema, psoriasis, lichen planus, psoriatic arthritis, vitiligo;
    - Uveitis;
    - Ankylosing spondylitis, Reiter's syndrome/reactive arthritis;
    - Aplastic anemia, autoimmune lymphoproliferative syndrome/disorders, hemophagocytic lymphohistiocytosis;
    - Type 1 diabetes; and
    - Refractory celiac disease;
- Acute rejection of grafted tissues and transplanted organs; acute graft versus host disease (GVHD) after transplantation of bone marrow cells or any other source of allogenic cells including hematopoietic precursors cells and/or stem cells.

Also of interest are diseases and disorders which are driven by both T- and B-cell activation and proliferation, with an important involvement of B-cells, including:

- diseases and disorders for which the involvement of pathogenic auto-antibodies is well characterized, including:
    - Allergy;
    - Cicatricial pemphigoid, bullous pemphigoid, epidermolysis bullosa acquisita, pemphigus foliaceus, pemphigus vulgaris, dermatitis herpetiformis;
    - ANCA-associated vasculitis and microscopic polyangitis, vasculitis, Wegener's granulomatosis; Churg-Strauss syndrome (CSS), polyarteritis nodosa, cryoglobulin syndromes and essential mixed cryglobulinemia;
    - Systemic lupus erythematosus (SLE), antiphospholipid syndrome (Hughes' syndrome), cutaneous lupus, lupus nephritis, mixed connective tissue disease;

- Thyroiditis, Hashimoto thyroiditis, Grave's disease, exophthalmos;
- Autoimmune hemolytic anemia, autoimmune neutropenia, ITP, pernicious anaemia, pure red cell anaemia, micro-angiopathic hemolytic anemia;
- Primary glomerulonephritis, Berger's disease, Goodpasture's syndrome, IgA nephropathy; and
- Chronic idiopathic polyneuritis, chronic inflammatory demyelinating polyneuropathy (CIPD), chronic relapsing polyneuropathy (Guillain-Barré syndrome), Miller Fischer syndrome, Stiff man syndrome, Lambert-Eaton myasthenic syndrome, myasthenia gravis.

- diseases and disorders for which the involvement of B-cells is less clearly characterized (although sometimes illustrated by the efficacy of anti-CD20 monoclonal antibodies or intravenous immunoglobulin infusions) and may not correspond or be limited to the production of pathogenic antibodies (nevertheless, non-pathogenic antibodies are sometimes described or even often present and used as a diagnosis biomarker), including:

  - Addison's disease, autoimmune oophoritis and azoospermia, polyglandular syndromes (Whitaker's syndrome), Schmidt's syndrome;
  - Autoimmune myocarditis, cardiomyopathy, Kawasaki's disease;
  - Rheumatoid arthritis, Sjögren's syndrome, mixed connective tissue disease, polymyositis and dermatomyositis; polychondritis;
  - Primary glomerulonephritis;
  - Multiple sclerosis;
  - Autoimmune hepatitis, primary biliary cirrhosis/ autoimmune cholangiopathy,

    - Hyper acute rejection of transplanted organs;
    - Chronic rejection of graft or transplants;
    - Chronic Graft versus Host reaction / disease after transplantation of bone marrow cells or hematopoietic precursor cells.

Additionally of interest are diseases and disorders for which the mechanism is shared between activation/proliferation of T-cells and activation/proliferation of innate immune cells and other inflammatory cellular subpopulations (including myeloid cells such as macrophages or granulocytes) and resident cells (such as fibroblasts and endothelial cells), including:

- COPD, idiopathic pulmonary fibrosis, interstitial lung disease, sarcoidosis;
- Adult onset Still's disease, juvenile idiopathic arthritis, Systemic sclerosis, CREST syndrome where B cells and pathogen antibodies may also play a role; the Sweet syndrome; Takayasu arteritis, temporal arteritis/ giant cell arteritis;
- Ulcerative cholangitis, inflammatory bowel disease (IBD) including Crohn's disease and ulcerative colitis, primary sclerosing cholangitis.

Also of interest are diseases and disorders for which the mechanism remains poorly characterized but involves the activation and proliferation of T-cells, including:

- Alzheimer's disease, cardiovascular syndrome, type 2 diabetes, restenosis, chronic fatigue immune dysfunction syndrome (CFIDS).

- Autoimmune Lymphoproliferative disorders, including:

- Autoimmune Lymphoproliferative Syndrome and X-linked lymphoproliferative disease.

Suitably the disease or disorder is selected from: inflammatory skin diseases such as psoriasis or lichen planus; acute and/or chronic GVHD such as steroid resistant acute GVHD; acute lymphoproliferative syndrome; systemic lupus erythematosus, lupus nephritis or cutaneous lupus; or transplantation. In addition, the disease or disorder may be selected from myasthenia gravis, multiple sclerosis, and scleroderma/systemic sclerosis.
The compounds of formula (I) may be used in the treatment of cancer.
Thus, in one embodiment there is provided a compound of formula (I), or a pharmaceutically acceptable salt and/or solvate thereof, for use in the treatment of cancer.
Suitably the cancer is a haematological cancer, such as Acute myeloid leukemia, Angioimmunoblastic T-cell lymphoma, B-cell acute lymphoblastic leukemia, Sweet Syndrome, T-cell Non-Hodgkins lymphoma (including natural killer/T-cell lymphoma, adult T-cell leukaemia/lymphoma, enteropathy type T-cell lymphoma, hepatosplenic T-cell lymphoma and cutaneous T-cell lymphoma), T-cell acute lymphoblastic leukemia, B-cell Non-Hodgkins lymphoma (including Burkitt lymphoma, diffuse large B-cell lymphoma, Follicular lymphoma, Mantle cell lymphoma, Marginal Zone lymphoma), Hairy Cell Leukemia, Hodgkin lymphoma, Lymphoblastic lymphoma, Lymphoplasmacytic lymphoma, Mucosa-associated lymphoid tissue lymphoma, Multiple myeloma, Myelodysplastic syndrome, Plasma cell myeloma, Primary mediastinal large B-cell lymphoma, chronic myeloproliferative disorders (such as chronic myeloid leukemia, primary myelofibrosis, essential thrombocytemia, polycytemia vera) or chronic lymphocytic leukemia.
Alternatively, the cancer is a non-haematological cancer, such as selected from the group consisting of bladder cancer, breast, melanoma, neuroblastoma, malignant pleural mesothelioma, and sarcoma.
In addition, compounds of formula (I) may be used in enhancing recovery from vascular injury or surgery and reducing morbidity and mortality associated with neointima and restenosis in a subject. For example, the compounds of formula (I) may be used in preventing, reducing, or inhibiting neointima formation. A medical device may be treated prior to insertion or implantation with an effective amount of a composition comprising a compound of formula (I) in order to prevent, reduce, or inhibit neointima formation following insertion or implantation of the device or graft into the subject. The device can be a device that is inserted into the subject transiently, or a device that is implanted permanently. In some embodiments, the device is a surgical device. Examples of medical devices include, but are not limited to, needles, cannulas, catheters, shunts, balloons, and implants such as stents and valves.
Suitably the subject is a mammal, in particular the subject is a human.

### Pharmaceutical Compositions

In the following section, reference to compounds of formula (I) encompasses compounds of formula (I-a), (I-b) and (I-c).
For use in therapy the compounds of the invention are usually administered as a pharmaceutical composition. The invention also provides a pharmaceutical composition comprising a compound of formula (I), or a pharmaceutically acceptable salt and/or solvate (e.g. salt) thereof, and a pharmaceutically acceptable carrier or excipient.
In one embodiment, there is provided a pharmaceutical composition comprising a compound of formula (I), or a pharmaceutically acceptable salt and/or solvate (e.g. salt) thereof, for use in the treatment or prophylaxis of a disease or disorder as described herein.
The compounds of formula (I) or their pharmaceutically acceptable salts and/or solvates thereof may be administered by any convenient method, e.g. by oral, parenteral, buccal, sublingual, nasal, rectal or transdermal administration, and the pharmaceutical compositions adapted accordingly.
The compounds of formula (I) or their pharmaceutically acceptable salts and/or solvates thereof may be administered topically, for example to the eye, gut or skin. Thus, in an embodiment there is provided a pharmaceutical composition comprising a compound of the invention optionally in combination with one or more topically acceptable diluents or carriers.
A pharmaceutical composition of the invention may be delivered topically to the skin. Compositions suitable for transdermal administration include ointments, gels and patches. Such a pharmaceutical composition may also suitably be in the form of a cream, lotion, foam, powder, paste or tincture.
The pharmaceutical composition may suitably include vitamin D3 analogues (e.g. calcipotriol and maxacalcitol), steroids (e.g. fluticasone propionate, betamethasone valerate and clobetasol propionate), retinoids (e.g. tazarotene), coal tar and dithranol. Topical medicaments are often used in combination with each other (e.g. a vitamin D3 and a steroid) or with further agents such as salicylic acid.
A pharmaceutical composition of the invention may be delivered topically to the eye. Such a pharmaceutical composition may suitably be in the form of eye drops or an ointment.
A pharmaceutical composition of the invention may be delivered topically to the gut. Such a pharmaceutical composition may suitably be delivered orally, such as in the form of a tablet or a capsule, or rectally, such as in the form of a

suppository.

Suitably, delayed release formulations are in the form of a capsule.

The compounds of formula (I) or their pharmaceutically acceptable salts and/or solvates thereof which are active when given orally can be formulated as liquids or solids, e.g. as syrups, suspensions, emulsions, tablets, capsules or lozenges.

A liquid formulation will generally consist of a suspension or solution of the active ingredient (such as a compound of formula (I) or a pharmaceutically acceptable salt and/or solvate (e.g. salt) thereof) in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.

A composition in the form of a tablet can be prepared using any suitable pharmaceutical carrier(s) routinely used for preparing solid formulations, such as magnesium stearate, starch, lactose, sucrose and cellulose.

A composition in the form of a capsule can be prepared using routine encapsulation procedures, e.g. pellets containing the active ingredient (such as a compound of formula (I) or a pharmaceutically acceptable salt and/or solvate (e.g. salt) thereof) can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.

Typical parenteral compositions consist of a solution or suspension of the active ingredient (such as a compound of formula (I) or a pharmaceutically acceptable salt and/or solvate (e.g. salt) thereof) in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively the sealed container may be a disposable dispensing device such as a single dose nasal inhaler or an aerosol dispenser fitted with a metering valve. Where the dosage form comprises an aerosol dispenser, it will contain a propellant which can be a compressed gas e.g. air, or an organic propellant such as a fluoro-chloro-hydrocarbon or hydrofluorocarbon. Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatin and glycerin.

Compositions for rectal administration are conveniently in the form of suppositories containing a conventional suppository base such as cocoa butter.

Suitably, the composition is in unit dose form such as a tablet, capsule or ampoule.

The composition may for example contain from 0.1% to 100% by weight, for example from 10 to 60% by weight, of the active material, depending on the method of administration. The composition may contain from 0% to 99% by weight, for example 40% to 90% by weight, of the carrier, depending on the method of administration. The composition may contain from 0.05 mg to 2000 mg, for example from 1.0 mg to 500 mg, of the active material, depending on the method of administration. The composition may contain from 50 mg to 1000 mg, for example from 100 mg to 400 mg of the carrier, depending on the method of administration. The dose of the compound used in the treatment or prophylaxis of the aforementioned disorders will vary in the usual way with the seriousness of the disorders, the weight of the sufferer, and other similar factors. However, as a general guide suitable unit doses may be 0.05 mg to 1000 mg, more suitably 1.0 mg to 500 mg, and such unit doses may be administered more than once a day, for example two or three a day. Such therapy may extend for a number of weeks or months.

The invention provides, in a further aspect, a combination comprising a compound of formula (I) or a pharmaceutically acceptable salt and/or solvate thereof together with a further pharmaceutically acceptable active ingredient or ingredients.

The invention provides a compound of formula (I), for use in combination with a further pharmaceutically acceptable active ingredient or ingredients.

When the compounds are used in combination with other therapeutic agents, the compounds may be administered separately, sequentially or simultaneously by any convenient route.

Optimal combinations may depend on the disease or disorder. Possible combinations include those with one or more active agents selected from the list consisting of: 5-aminosalicylic acid, or a prodrug thereof (such as sulfasalazine, olsalazine or bisalazide); corticosteroids (e.g. prednisolone, methylprednisolone, or budesonide); immunosuppressants (e.g. cyclosporin, tacrolimus, sirolimus, methotrexate, azathioprine mycophenolate mofetil, leflunomide, cyclophosphamide, 6-mercaptopurine or anti-lymphocyte (or thymocyte) globulins); anti-TNF-alpha antibodies (e.g., infliximab, adalimumab, certolizumab pegol or golimumab); anti-IL12/IL23 antibodies (e.g., ustekinumab); anti-IL6 or anti-IL6R antibodies, anti-IL17 antibodies or small molecule IL12/IL23 inhibitors (e.g., apilimod); Anti-alpha-4-beta-7 antibodies (e.g., vedolizumab); MAdCAM-1 blockers (e.g., PF-00547659); antibodies against the cell adhesion molecule alpha-4-integrin (e.g., natalizumab); antibodies against the IL2 receptor alpha subunit (e.g., daclizumab or basiliximab); JAK inhibitors including JAK1 and JAK3 inhibitors (e.g., tofacitinib, baricitinib, R348); Syk inhibitors and prodrugs thereof (e.g., fostamatinib and R-406); Phosphodiesterase-4 inhibitors (e.g., tetomilast); HMPL-004; probiotics; Dersalazine; semapimod/CPSI-2364; and protein kinase C inhibitors (e.g. AEB-071).

For cancer, the further pharmaceutically acceptable active ingredient may be selected from anti-mitotic agents such as vinblastine, paclitaxel and docetaxel; alkylating agents, for example cisplatin, carboplatin, dacarbazine and cyclophosphamide; antimetabolites, for example 5-fluorouracil, cytosine arabinoside and hydroxyurea; intercalating agents for example adriamycin and bleomycin; topoisomerase inhibitors for example etoposide, topotecan and irinotecan; thymidylate synthase inhibitors for example raltitrexed; PI3 kinase inhibitors for example idelalisib; mTor inhibitors for example everolimus and temsirolimus; proteasome inhibitors for example bortezomib; histone deacetylase inhibitors for example panobinostat or vorinostat; and hedgehog pathway blockers such as vismodegib.

The further pharmaceutically acceptable active ingredient may be selected from tyrosine kinase inhibitors such as, for example, axitinib, dasatinib, erlotinib, imatinib, nilotinib, pazopanib and sunitinib.

Anticancer antibodies may be included in a combination therapy and may be selected from the group consisting of olaratumab, daratumumab, necitumumab, dinutuximab, traztuzumab emtansine, pertuzumab, obinutuzumab, brentuximab, ofatumumab, panitumumab, catumaxomab, bevacizumab, cetuximab, tositumomab, traztuzumab, gentuzumab ozogamycin and rituximab.

Compounds or pharmaceutical compositions of the invention may also be used in combination with radiotherapy.

Some of the combinations referred to above may conveniently be presented for use in the form of a pharmaceutical formulation and thus pharmaceutical formulations comprising a combination as defined above together with a pharmaceutically acceptable carrier or excipient comprise a further aspect of the invention. The individual components of such combinations may be administered either sequentially or simultaneously in separate or combined pharmaceutical formulations. The individual components of combinations may also be administered separately, through the same or different routes.

When a compound of formula (I) or a pharmaceutically acceptable salt and/or solvate thereof is used in combination with a second therapeutic agent active against the same disease state the dose of each compound may differ from that when the compound is used alone. Appropriate doses will be readily appreciated by those skilled in the art.

**Medical Devices**

In the following section, reference to compounds of formula (I) encompasses compounds of formula (I-a), (I-b) and (I-c).

In an embodiment, compounds of the invention or pharmaceutical compositions comprising said compounds may be formulated to permit incorporation into the medical device, thus providing application of the compound or composition directly to the site to prevent or treat conditions disclosed herein.

In an embodiment, the compounds of the invention or pharmaceutical composition thereof is formulated by including it within a coating onto the medical device. There are various coatings that can be utilized such as, for example, polymer coatings that can release the compound over a prescribed time period. The compound, or a pharmaceutical composition thereof, can be embedded directly within the medical device. In some embodiments, the compound is coated onto or within the device in a delivery vehicle such as a microparticle or liposome that facilitates its release and delivery. In some embodiments, the compound or pharmaceutical composition is miscible in the coating.

In some embodiments, the medical device is a vascular implant such as a stent. Stents are utilized in medicine to prevent or eliminate vascular restrictions. The implants may be inserted into a restricted vessel whereby the restricted vessel is widened. Excessive growth of the adjacent cells following vascular implantation results in a restriction of the vessel particularly at the ends of the implants which results in reduced effectiveness of the implants. If a vascular implant is inserted into a human artery for the elimination of for example an arteriosclerotic stenosis, intima hyperplasia can occur within a year at the ends of the vascular implant and results in renewed stenosis ("restenosis").

Accordingly, in some embodiments, the stents are coated or loaded with a composition including a compound of the invention or pharmaceutical composition thereof and optionally a targeting signal, a delivery vehicle, or a combination thereof. Many stents are commercially available or otherwise know in the art.

In some embodiments, the stent is a drug-eluting stent. Various drug eluting stents that simultaneously deliver a therapeutic substance to the treatment site while providing artificial radial support to the wall tissue are known in the art. Endoluminal devices including stents are sometimes coated on their outer surfaces with a substance such as a drug releasing agent, growth factor, or the like. Stents have also been developed having a hollow tubular structure with holes or ports cut through the sidewall to allow drug elution from a central lumen. Although the hollow nature of the stent allows the central lumen to be loaded with a drug solution that is delivered via the ports or holes in the sidewall of the stent, the hollow tubular structure may not have suitable mechanical strength to provide adequate scaffolding in the vessel.

In some embodiments, the devices are also coated or impregnated with a compound of the invention, or

pharmaceutical composition thereof and one or more additional therapeutic agents, including, but not limited to, antiplatelet agents, anticoagulant agents, anti-inflammatory agents, antimicrobial agents, antimetabolic agents, additional anti-neointima agents, additional antiproliferative agents, immunomodulators, antiproliferative agents, agents that affect migration and extracellular matrix production, agents that affect platelet deposition or formation of thrombis, and agents that promote vascular healing and re-endothelialization, such as those and others described in Sousa *et al.* (2003) and Salu *et al.* (2004).

Examples of antithrombin agents include, but are not limited to, Heparin (including low molecular heparin), R-Hirudin, Hirulog, Argatroban, Efegatran, Tick anticoagulant peptide, and Ppack.

Examples of antiproliferative agents include, but are not limited to, Paclitaxel (Taxol), QP-2 Vincristin, Methotrexat, Angiopeptin, Mitomycin, BCP 678, Antisense c-myc, ABT 578, Actinomycin-D, RestenASE, 1 -Chlor- deoxyadenosin, PCNA Ribozym, and Celecoxib.

Examples of anti-restenosis agents include, but are not limited to, immunomodulators such as Sirolimus (Rapamycin), Tacrolimus, Biorest, Mizoribin, Cyclosporin, Interferon-γ Ib, Leflunomid, Tranilast, Corticosteroide, Mycophenolic acid and Biphosphonate.

Examples of anti-migratory agents and extracellular matrix modulators include, but are not limited to Halofuginone, Propyl-hydroxylase-Inhibitors, C- Proteinase-Inhibitors, MMP-Inhibitors, Batimastat, Probucol.

Examples of antiplatelet agents include, but are not limited to, heparin.

Examples of wound healing agents and endothelialization promoters include vascular epithelial growth factor ("VEGF"), 17 -Estradiol, Tkase- Inhibitors, BCP 671, Statins, nitric oxide ("NO")-Donors, and endothelial progenitor cell ("EPC")-antibodies.

Besides coronary applications, drugs and active agents may be incorporated into the stent or stent coating for other indications. For example, in urological applications, antibiotic agents may be incorporated into the stent or stent coating for the prevention of infection. In gastroenterological and urological applications, active agents may be incorporated into the stent or stent coating for the local treatment of carcinoma. It may also be advantageous to incorporate in or on the stent a contrast agent, radiopaque markers, or other additives to allow the stent to be imaged in vivo for tracking, positioning, and other purposes. Such additives could be added to the absorbable composition used to make the stent or stent coating, or absorbed into, melted onto, or sprayed onto the surface of part or all of the stent. Preferred additives for this purpose include silver, iodine and iodine labelled compounds, barium sulfate, gadolinium oxide, bismuth derivatives, zirconium dioxide, cadmium, tungsten, gold tantalum, bismuth, platinum, iridium, and rhodium. These additives may be, but are not limited to, micro- or nano-sized particles or nano particles. Radio-opacity may be determined by fluoroscopy or by x-ray analysis.

A compound of the invention and one or more additional agents, or pharmaceutical composition thereof, can be incorporated into the stent, either by loading the compound and one or more additional agents, or pharmaceutical composition thereof into the absorbable material prior to processing, and/or coating the surface of the stent with the agent(s). The rate of release of agent may be controlled by a number of methods including varying the following: the ratio of the absorbable material to the compound and one or more additional agents, or pharmaceutical composition, the molecular weight of the absorbable material, the composition of the compound and one or more additional agents, or pharmaceutical composition, the composition of the absorbable polymer, the coating thickness, the number of coating layers and their relative thicknesses, and/or the compound and one or more additional agents, or pharmaceutical composition concentration. Top coats of polymers and other materials, including absorbable polymers, may also be applied to active agent coatings to control the rate of release. For example, P4HB can be applied as a top coat on a metallic stent coated with P4HB including an active agent to retard the release of the active agent.

The invention is further exemplified by the following non-limiting examples.

## EXAMPLES

Abbreviations used herein are defined below. Any abbreviations not defined are intended to convey their generally accepted meaning.

### Abbreviations

| | |
|---|---|
| Ac | acetyl ($C(O)CH_3$) |
| AcOH | glacial acetic acid |
| $AlMe_3$ | trimethylaluminium |
| aq | aqueous |
| Ar | Aromatic ring |

| | |
|---|---|
| BEH | ethylene bridged hybrid |
| Bispin | Bis(pinacolato)diboron; 4,4,4',4',5,5,5',5'-Octamethyl-2,2'-bi-1,3,2-dioxaborolane |
| Bz | benzyl ($CH_2$-phenyl) |
| Boc | *tert*-butyloxycarbonyl protecting group |
| $Cs_2CO_3$ | Cesium carbonate |
| CSH | charged surface hybrid |
| d | doublet |
| DABAL-$Me_3$ | adduct of trimethylaluminum and 1,4-diazabicyclo[2.2.2]octane |
| DCM | dichloromethane |
| DIPEA | N,N-diisopropylethylamine |
| dioxane | 1,4-dioxane |
| DMAP | 4-dimethylaminopyridine |
| DME | dimethoxyethane |
| DMF | N,N-dimethylformamide |
| DMSO | dimethyl sulfoxide |
| DMP | Dess-Martin Periodinane |
| DPPA | diphenylphosphoryl azide |
| dppf | 1,1'-bis(diphenylphosphino)ferrocene |
| ($ES^+$) | electrospray ionisation, positive mode |
| ($ES^-$) | electrospray ionisation, negative mode |
| ESI | electrospray ionisation |
| Et | ethyl |
| EtI | Ethyl iodide |
| EtOAc | ethyl acetate |
| EtOH | ethanol |
| g | grams |
| Hal | halogen |
| HATU | 1-[bis(dimethylamino)methylene]-1*H*-1,2,3-triazolo[4,5-*b*]pyridinium 3-oxid hexafluorophosphate |
| HPLC | high performance liquid chromatography |
| hr(s) | hour(s) |
| $IC_{50}$ | 50% inhibitory concentration |
| iPr | *iso*-propyl |
| iPrMgCl | *iso*-propyl magnesium chloride |
| $K_2CO_3$ | potassium carbonate |
| LCMS | liquid chromatography-mass spectrometry |
| LiHMDS | lithium hexamethyldisilazide |
| LiOH | lithium hydroxide |
| $(M+H)^+$ | protonated molecular ion |
| $(M-H)^-$ | unprotonated molecular ion |
| M | molar concentration |
| *m*CPBA | Meta-chloroperoxybenzoic acid |
| mL | millilitre |
| mm | millimiter |
| mmol | millimole |
| Me | methyl |
| MeCN | acetonitrile |
| MeI | iodomethane |
| MeOH | methanol |
| MesCl | methanesulfonyl chloride |
| MHz | megahertz |
| min(s) | minute(s) |
| MSD | mass selective detector |
| m/z | mass-to-charge ratio |
| $N_2$ | nitrogen gas |
| $NH_3$ | ammonia |
| $NH_4Cl$ | ammonium chloride |
| NaH | sodium hydride |
| $NaHCO_3$ | sodium bicarbonate |

| | |
|---|---|
| $NaBH(OAc)_3$ | Sodium triacetoxyborohydride |
| nm | nanometre |
| NMR | nuclear magnetic resonance (spectroscopy) |
| NSFI | *N*-fluorobenzenesulfonimide |
| P4HB | poly-4-hydroxybutyrate |
| PDA | photodiode array |
| Pd 170 | chloro(crotyl)(2-dicyclohexylphosphino-2',4',6'-triisopropybiphenyl)palladium(II) or XPhos Pd(crotyl) Cl |
| Pd 174 | allyl(2-di-*tert*-butylphosphino-2',4',6'-triisopropyl-1,1'-biphenyl)palladium(II) triflate or [tBuXPhosP-d(allyl)]OTf |
| $[Pd(allyl)Cl_2]_2$ | bis(allyl)dichlorodipalladium |
| $PdCl_2$(dppf) | [1,1'-bis(diphenylphosphino)ferrocene]dichloropalladium(II) |
| $Pd(PPh_3)_4$ | tetrakis(triphenylphosphine)palladium(0) |
| PMB | 4-methoxybenzyl |
| prep HPLC | preparative high performance liquid chromatography |
| Ph | phenyl |
| pos/neg | positive/negative |
| q | quartet |
| RF/MS | RapidFire Mass Spectrometry |
| RT | room temperature |
| Rt | retention time |
| RP | reverse phase |
| s | singlet |
| $S_NAr$ | nucleophilic aromatic substitution |
| sat | saturated |
| SCX | solid supported cation exchange (resin) |
| Selectfluor | *N*-chloromethyl-*N'*-fluorotriethylenediammonium bis(tetrafluoroborate) |
| t | triplet |
| tBu | *tert-butyl* |
| T3P | 2,4,6-tripropyl-1,3,5,2,4,6-trioxatriphosphorinane-2,4,6-trioxide |
| TBME | *tert-butyl* methyl ether |
| TFA | Trifluoroacetic acid |
| [*t*-BuXPhos Pd(allyl)]OTf | allyl(2-di-*tert*-butylphosphino-2',4',6'-triisopropyl-1,1'-biphenyl)palladium(II) triflate |
| THF | tetrahydrofuran |
| TMP | 2,2,6,6-tetramethylpiperidinyl |
| TMSOK | potassium trimethylsilanolate |
| TTIP | titanium tetraisopropoxide |
| UPLC | ultra performance liquid chromatography |
| UV | ultraviolet |
| v/v | volume/volume |
| VWD | variable wave detector |
| wt | weight |
| um | micrometre |
| uL | microlitre |
| °C | degrees Celsius |

**General Procedures**

All starting materials and solvents were obtained either from commercial sources or prepared according to the literature. Unless otherwise stated all reactions were stirred. Organic solutions were routinely dried over anhydrous magnesium sulfate. Hydrogenations were performed on a Thales H-cube flow reactor under the conditions stated.

Column chromatography was performed on pre-packed silica (230-400 mesh, 40-63 um) cartridges using the amount indicated. SCX was purchased from Supelco and treated with 1M hydrochloric acid prior to use. Unless stated otherwise the reaction mixture to be purified was first diluted with MeOH and made acidic with a few drops of AcOH. This solution was loaded directly onto the SCX and washed with MeOH. The desired material was then eluted by washing with 0.7 M $NH_3$ in MeOH.

*Preparative Reverse Phase High Performance Liquid Chromatography*

**Prep HPLC**

***Acidic prep***

Waters X-Select CSH column C18, 5 um (19 x 50 mm), flow rate 28 mL $min^{-1}$ eluting with a $H_2O$-MeCN gradient containing 0.1 % v/v formic acid over 6.5 min using UV detection at 254 nm.

***Basic prep***

Waters X-Bridge Prep column C18, 5 um (19 x 50 mm), flow rate 28 mL $min^{-1}$ eluting with a 10 mM $NH_4HCO_3$-MeCN gradient over 6.5 min using UV detection at 254 nm.

**Analytical Methods**

*Reverse Phase HPLC Conditions for the LCMS Analytical Methods*

**HPLC acidic:** Acidic LCMS 4 minute (5-95%)

Analytical LCMS was carried out using a Waters X-Select CSH C18, 2.5 um, 4.6x30 mm column eluting with a gradient of 0.1 % Formic acid in MeCN in 0.1 % Formic acid in water. The gradient from 5-95 % 0.1 % Formic acid in MeCN occurs between 0.00-3.00 minutes at 2.5 mL/min with a flush from 3.01-3.5 minutes at 4.5 mL/min. A column re-equilibration to 5% MeCN is from 3.60-4.00 minutes at 2.5 mL/min. UV spectra of the eluted peaks were measured using an Agilent 1260 Infinity VWD at 254 nm. Mass spectra were measured using an Agilent 6120 MSD running with positive/-negative switching.

**HPLC basic:** Basic LCMS 4 minute (5-95%)

Analytical LCMS was carried out using a Waters X-Select BEH C18, 2.5 um, 4.6x30 mm column eluting with a gradient of MeCN in aqueous 10mM ammonium bicarbonate. The gradient from 5-95% MeCN occurs between 0.00-3.00 minutes at 2.5mL/min with a flush from 3.01-3.5 minutes at 4.5 mL/min. A column re-equilibration to 5% MeCN is from 3.60-4.00 minutes at 2.5mL/min. UV spectra of the eluted peaks were measured using an Agilent 1260 Infinity VWD at 254nm. Mass spectra were measured using an Agilent 6120 MSD running with positive/negative switching.

*Reverse Phase HPLC Conditions for the UPLC Analytical Methods*

**UPLC acidic:** Acidic UPLC 3 minute

Analytical UPLC/MS was carried out using a Waters Acquity CSH C18, 1.7 um, 2.1x30 mm column eluting with a gradient of 0.1% Formic acid in MeCN in 0.1% Formic acid in water. The gradient is structured with a starting point of 5% MeCN held from 0.0-0.11 minutes. The gradient from 5-95% occurs between 0.11-2.15 minutes with a flush from 2.15-2.56 minutes. A column re-equilibration to 5% MeCN is from 2.56-2.83 minutes. UV spectra of the eluted peaks were measured using an Acquity PDA and mass spectra were recorded using an Acquity QDa detector with ESI pos/neg switching.

**Acidic UPLC 2** Acidic UPLC 1 minute

Analytical UPLC/MS was carried out using a Waters Acquity CSH C18, 1.7 um, 2.1x30 mm column eluting with a gradient of 0.1% Formic acid in MeCN in 0.1% Formic acid in water. The gradient is structured with a starting point of 5% MeCN held from 0.0-0.08 minutes. The gradient from 5-95% occurs between 0.08-0.70 minutes with a flush from 0.7-0.8 minutes. A column re-equilibration to 5% MeCN is from 0.8-0.9 minutes. UV spectra of the eluted peaks were measured using an Acquity PDA and mass spectra were recorded using an Acquity QDa detector with ESI pos/neg switching.

**UPLC basic:** Basic UPLC 3 minute

Analytical UPLC/MS was carried out using a Waters Acquity BEH C18, 1.7 um, 2.1x30 mm column eluting with a gradient of MeCN in aqueous 10 mM Ammonium Bicarbonate. The gradient is structured with a starting point of 5% MeCN held from 0.0-0.11 minutes. The gradient from 5-95% occurs between 0.11-2.15 minutes with a flush from 2.15-2.56

minutes. A column re-equilibration to 5% MeCN is from 2.56-2.83 minutes. UV spectra of the eluted peaks were measured using an Acquity PDA and mass spectra were recorded using an Acquity QDa detector with ESI pos/neg switching.

**Basic UPLC 2** Basic UPLC 1 minute

Analytical UPLC/MS was carried out using a Waters Acquity BEH C18, 1.7 um, 2.1x30 mm column eluting with a gradient of MeCN in aqueous 10 mM Ammonium Bicarbonate. The gradient is structured with a starting point of 5% MeCN held from 0.0-0.08 minutes. The gradient from 5-95% occurs between 0.08-0.70 minutes with a flush from 0.7-0.8 minutes. A column re-equilibration to 5% MeCN is from 0.8-0.9 minutes. UV spectra of the eluted peaks were measured using an Acquity PDA and mass spectra were recorded using an Acquity QDa detector with ESI pos/neg switching.

Column temperature is 40 °C in all runs. Injection volume is 3 uL and the flow rate is 0.77 mL/min. PDA scan from 210-400 nm on all runs.

*Normal Phase HPLC Conditions for the Chiral Analytical Methods*

**Chiral IC3 method:** Chiral HPLC (Diacel Chiralpak IC, 5 um, 4.6x250 mm, 1.0 mL/min, 25-70% EtOH (0.2% TFA) in iso-hexane (0.2% TFA)

**Chiral IC4 method:** Chiral HPLC (Diacel Chiralpak IC, 5 um, 4.6x250 mm, 1.0 mL/min, 40% EtOH (0.2% TFA) in 4:1 heptane/chloroform (0.2 % TFA).

**Chiral IC5 method:** Chiral HPLC (Diacel Chiralpak IC, 5 um, 4.6x250 mm, 1.0 mL/min, 20% EtOH (0.2% TFA) in iso-hexane (0.2% TFA).

*Reverse Phase HPLC Conditions for the Chiral Analytical Methods*

**Chiral IC6 method:** Chiral HPLC (Diacel Chiralpak IC, 5 um, 4.6x250 mm, 1.0 mL/min, 50% MeCN (0.1 % formic acid) in water (0.1 % formic acid).

**Chiral IC7 method:** Chiral HPLC (Diacel Chiralpak IC, 5 um, 4.6x250 mm, 1.0 mL/min, 5-95% MeCN (0.1 % formic acid) in water (0.1 % formic acid).

*$^1$H NMR Spectroscopy*

$^1$H NMR spectra were acquired on a Bruker Avance III spectrometer at 400 MHz or Bruker Avance III HD spectrometer at 500 MHz using residual undeuterated solvent as reference and unless specified otherwise were run in DMSO-d6.

**Preparation of Intermediates**

Known synthetic intermediates were procured from commercial sources or were obtained using published literature procedures. Additional intermediates were prepared by the representative synthetic processes described herein.

**Compounds of formula (I-a) - Intermediates**

Any one of Methods 1-1q (referred to later herein) or A-N and Q-R may be used in the synthesis of the compounds of formula (I). For example, a scheme which is shown using a compound wherein X = N, Y = $CR_2$ and Z = $CR_3$ may also be used in the synthesis of compounds wherein X, Y and Z are as defined in the claims.

The synthesis of INTC1 to INTC179 and INTD1 to INTD86 is disclosed in at least one of WO2019/179652, WO2019/180244 and WO2020/083975.

**Method A: Decarboxylation of chloro-heterocycles such as chloro-pyrimidines**

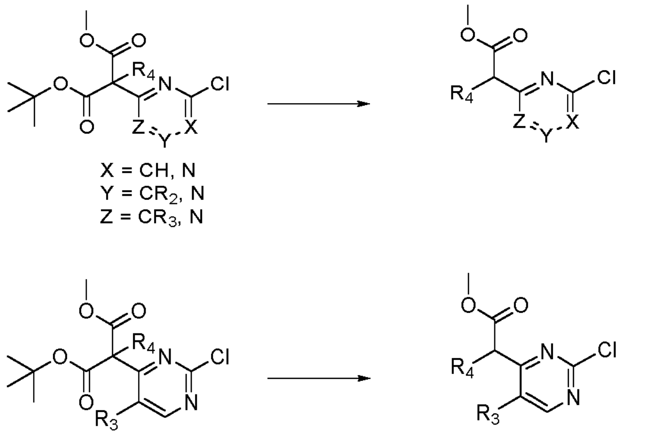


TFA (10 eq) was added dropwise to an ice-cooled, stirred solution of malonate derivative (1 eq) in DCM (15 volumes). The reaction vessel was stirred at RT for 18 hrs and then concentrated. The crude product was purified by normal phase chromatography.

**Method B: Alkylation**

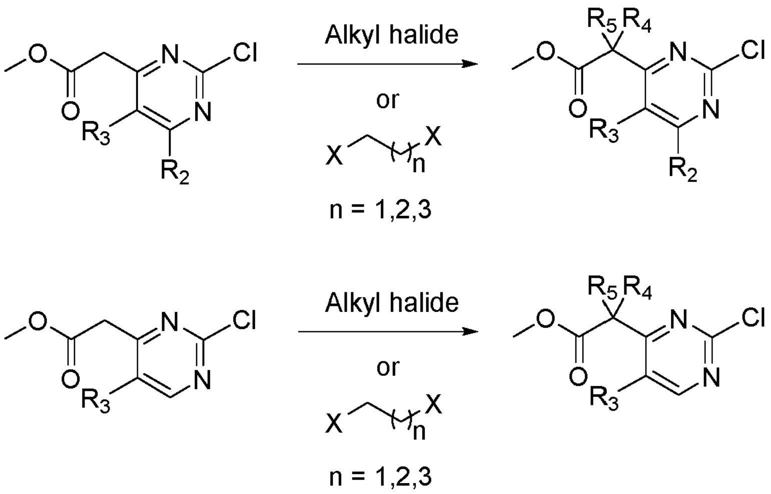


Base (2.5 - 5 eq) was added to an ice-cooled, stirred mixture of methyl 2-(2-chloropyrimidin-4-yl)acetate (1 eq) in appropriate polar aprotic solvent such as DMF or acetone (10 volumes). After 20 min, alkyl halide (1-5 eq) was added. The reaction vessel was stirred at 0 °C for 30 mins then at RT for 2 hrs. The reaction was quenched with $NH_4Cl$ (aq) or 1M HCl (aq), stirred for 20 mins then extracted with EtOAc. The organic phases were dried (phase separator) and concentrated. The crude product was purified by normal phase chromatography.

**Method C: Formation of sulfonamides from aromatic halides**

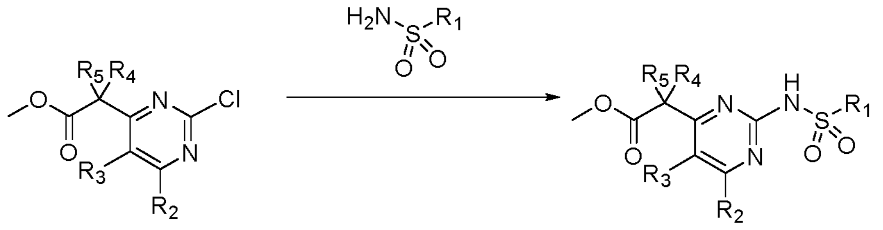

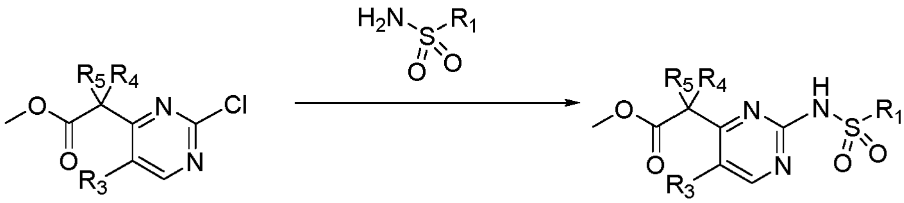

2-Chloropyrimidine intermediate (1 eq), sulfonamide (1.2 eq) and base (2 eq) were dissolved in dioxane (40 volumes). The mixture was degassed ($N_2$, 5 mins) then catalyst (5 mol%) was added. The resulting mixture was heated under nitrogen at 90 °C for 2 hrs. The mixture was filtered, washing with EtOAc or DCM and the resulting filtrate was concentrated. The crude product was purified by normal phase chromatography or trituration using a suitable solvent.

**Method D: Decarboxylation of pyrimidines bearing sulfonamides**

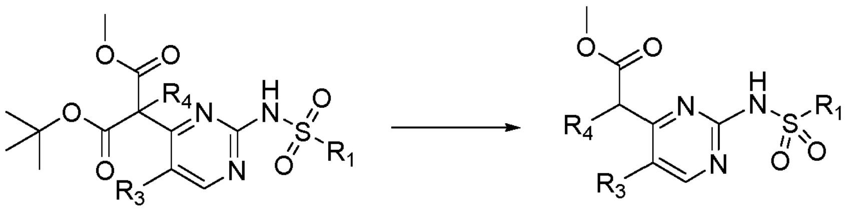

TFA (10 eq) was added dropwise to an ice-cooled, stirred solution of malonate derivative (1 eq) in DCM (15 volumes). The reaction vessel was stirred at RT for 18 hrs and then concentrated. The crude product was purified by normal phase chromatography.

**Method H: Benzylic fluorination of hetero-aromatic esters**

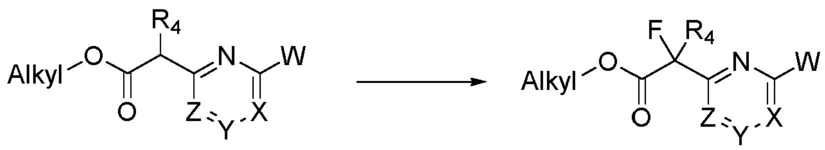

X = CH, N
Y = $CR_2$, N
Z = $CR_3$, N
W = Hal, $N(PMB)SO_2Alkyl$

A solution of hetero-aromatic ester (1 eq) in THF (10 volumes) was cooled to -78 °C to which was added LiHMDS (1.25 eq 1M in THF). The reaction mixture was then warmed to RT for 1 hr. The solution was cooled to -78 °C and a solution (in THF) of, or solid, NSFI (1.25 eq) was added dropwise then warmed to RT for 2 hrs. The solution was diluted with sat. $NaHCO_3$ (aq) and the product was extracted into EtOAc. The crude product was purified by normal phase chromatography.

**Method I: Buchwald coupling - sulfonylation**

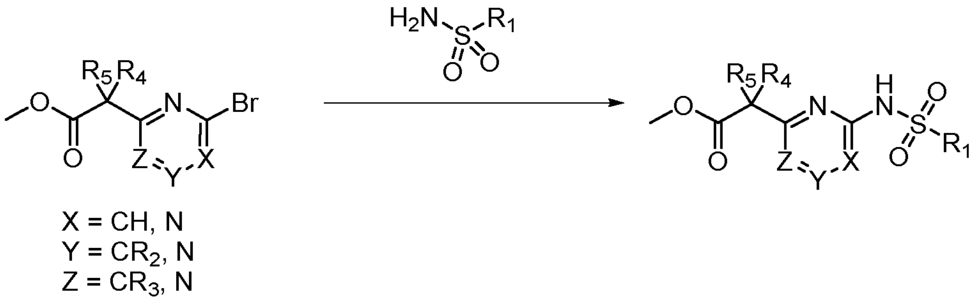

2-Bromopyridine intermediate (1 eq), sulfonamide (1.2 eq) and base (2 eq) were dissolved in dioxane (40 volumes). The mixture was degassed ($N_2$, 5 mins) then catalyst (5 mol%) was added. The resulting mixture was heated under nitrogen at 90 °C for 2 hrs. The mixture was filtered, washing with EtOAc or DCM and the resulting filtrate was concentrated. The crude product was purified by normal phase chromatography.

**Method J: Hydrolysis**

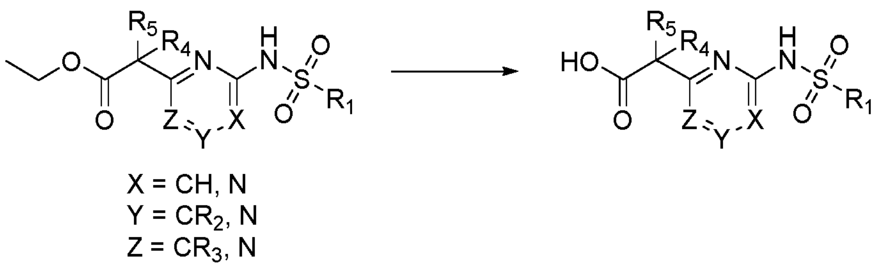

2M LiOH (aq, 2 eq) was added into a solution of ester (1 eq) in MeOH (3 volumes) and THF (3 volumes) and the resulting reaction mixture was stirred at 50 °C for 2 hrs. The solvent was removed under reduced pressure and then was acidified with 1M HCl (aq) until pH 3. The solution was extracted with EtOAc, the organic phase was passed through a phase separator and the solvent was removed. The compound was used crude or purified by reverse phase chromatography.

**Amine intermediate preparation**

**Method E: Suzuki coupling of halo anilines with heteroaromatic boronates**

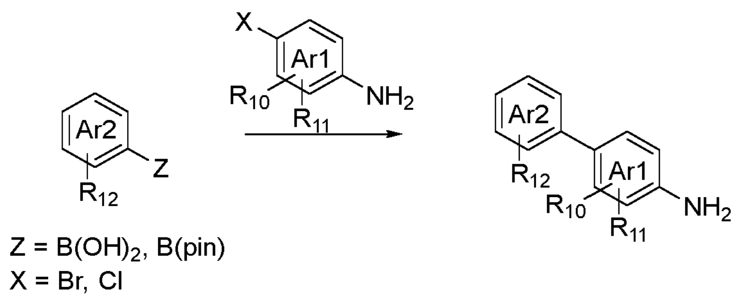

A solution of Ar1-X (1 eq) and Ar2-Z (1 eq) in solvent (3 volumes) and base (2.5 eq) was degassed ($N_2$, 5 min) and heated to 40 °C whereupon Pd catalyst (3 mol%) was added and the reaction mixture further degassed ($N_2$, 5 min) before

being heated to 90 °C for 90 mins. The reaction mixture was allowed to cool to RT. In general, the desired compound was purified by column chromatography.

**Method F: Suzuki coupling of heteroaromatic halides with aniline boronates**

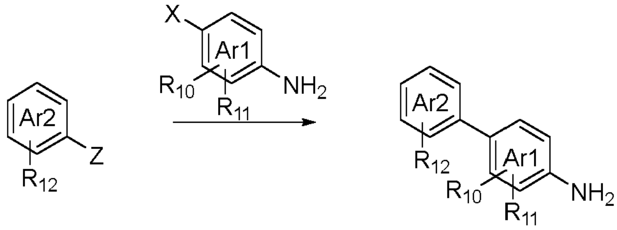

Z = Br, Cl
X = $B(OH)_2$, B(pin)

Pd catalyst (5 mol%) was added to a degassed ($N_2$, 5 mins) solution of Ar1-X (1 eq), Ar2-Z (1 eq) and base (3 eq, 6.85 mmol) in solvent (3 volumes). The solution was then degassed further ($N_2$, 5 mins) and then heated to 90 °C for 2 hrs then allowed to cool to RT. In general, the desired compound was purified by column chromatography.

**Method G: Telescoped boronate formation and Suzuki coupling**

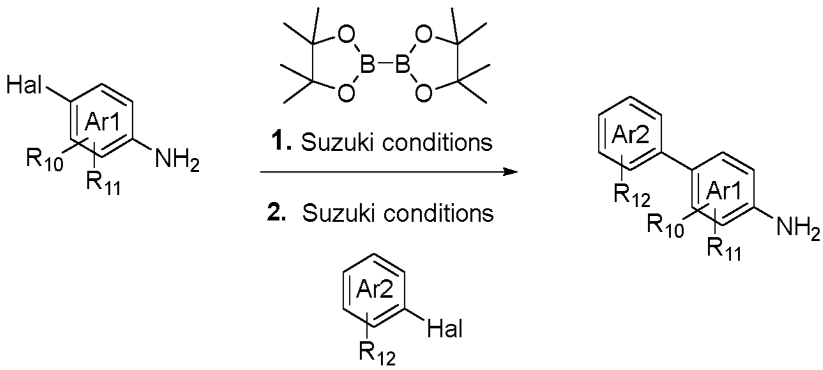


Bispin (1.1 eq) and KOAc (4 eq) were added to Ar1-Hal (1 eq) in dioxane (5 volumes). The reaction was heated to 60 °C and degassed ($N_2$, 5 mins). $PdCl_2$(dppf) (5 mol%) was added to the reaction mixture and the temperature was increased to 90 °C for 1 hr. The reaction mixture was then cooled to RT and a solution of Ar2-Hal (1 eq) in dioxane (3 volumes) was added, followed by a solution of $K_2CO_3$ (4 eq) in water (2 volumes). The temperature was then increased to 90 °C for 18 hrs. The reaction was cooled to RT, an aqueous work up was performed and the crude compound was purified by normal phase chromatography.

**Anilines**

**Method K: Suzuki coupling**

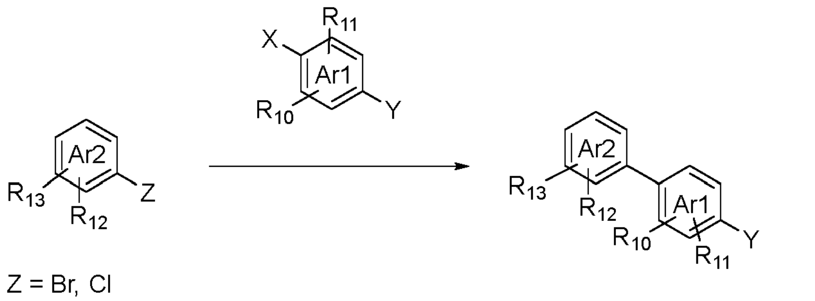

Z = Br, Cl
X = $B(OH)_2$, Bpin

Y = $CO_2t$-Bu, $CO_2Me$, CN

A solution of boronic acid (1 eq), aryl halide (1.05 eq.) and $Cs_2CO_3$ (3 eq.) in a mixture of dioxane (40 volumes) and water (6 volumes) was degassed ($N_2$, 5 mins). $PdCl_2$(dppf).$CH_2Cl_2$ (5 mol%) was added and the reaction was further degassed ($N_2$) before being heated to 90 °C for 18 hrs. The reaction mixture was filtered through celite before an aqueous workup was undertaken, followed by purification by normal phase chromatography.

**Method L: Ester deprotection with TFA**

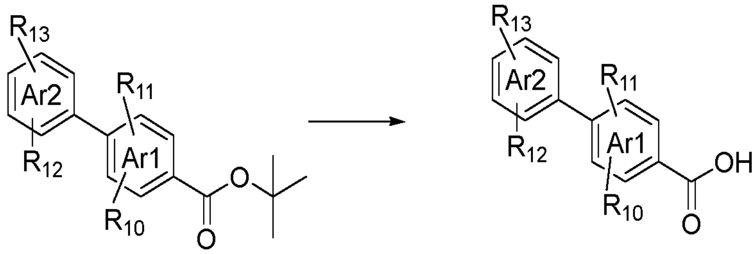

A solution of the ester (1 eq) in DCM (20 volumes) was treated with TFA (10 eq.) and stirred at RT for 3 hrs. The reaction mixture was then concentrated and azeotroped with MeOH and MeCN. No further purification was undertaken.

**Method M: Ester deprotection with base**

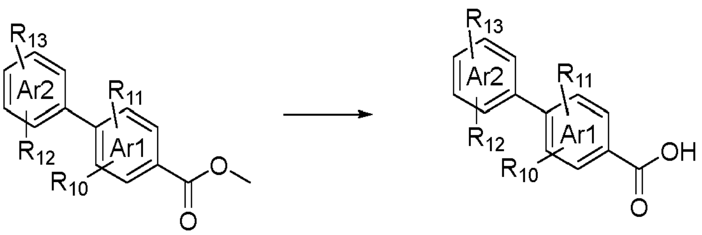

A solution of the ester (1 eq) in a mixture of THF/MeOH (4/1 volumes) was treated with LiOH (2.2-6 eq.) and stirred between RT and 50 °C for between 3 hrs and 18 hrs. The organic solvents were removed *in vacuo* then acidified with 1 M HCl and extracted with EtOAc. The organic phases were combined, dried ($Na_2SO_4$), filtered and concentrated. The products were used directly in the next step with no further purification undertaken.

**Method N: Potassium salt formation**

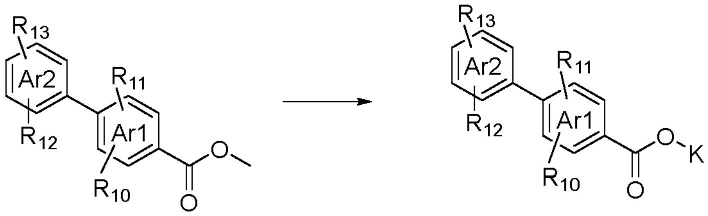

A solution of the ester (1 eq.) in THF (4 volumes) was treated with TMSOK (1 eq.) and stirred at RT for 2 hrs before the reaction mixtures were filtered and washed with iso-hexanes. The products were used directly in the next step with no further purification undertaken.

**Method P: SNAR using 4-chloro-2-(methylthio)-heterocycles**

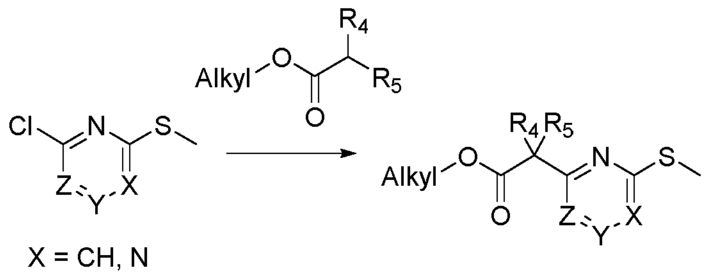

X = CH, N
Y = $CR_2$, N
Z = $CR_3$, N

A solution of hetero-aromatic chloride (1 eq) and ester (1 eq) in THF (5-20 volumes) was warmed to 30 °C to which was added LiHMDS (1.25 eq 1-1.5M in THF). The reaction mixture was stirred at this temperature for up to 3 hrs, then was poured into water and extracted with EtOAc. The organic extract was washed with brine, dried ($MgSO_4$), filtered and the solvent removed in vacuo to afford the desired compound. If required, the crude product was purified by normal phase chromatography.

**Table 1:** The following intermediates were made according to Method P.

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **INTC186** | methyl 8-(2-(methylthio)pyrimidin-4-yl)-1,4-dioxaspiro[4.5]decane-8-carboxylate 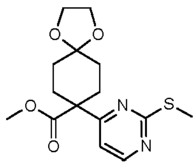 | Method P, [HPLC acidic], 325 (1.99). | 8.60 (d, J = 5.3 Hz, 1H), 7.24 (d, J = 5.3 Hz, 1H), 3.88-3.86 (m, 4H), 3.65 (s, 3H), 2.49 (s, 3H), 2.30-2.24 (m, 2H), 2.16-2.07 (m, 2H), 1.65 - 1.56 (m, 4H). |

(continued)

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| INTC187 | *1-(tert-butyl)* 4-ethyl 4-(2-(methylthio)pyrimidin-4-yl)piperidine-1,4-dicarboxylate 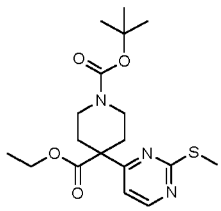 | Method P, [HPLC acidic], 382 (2.54). | 8.61 (d, J = 5.3 Hz, 1H), 7.25 (d, J = 5.3 Hz, 1H), 4.14 (q, J = 7.1 Hz, 2H), 3.73 - 3.65 (m, 2H), 3.32 (s, 1H), 2.49 (s, 3H), 2.26 - 2.20 (m, 1H), 2.02 - 1.93 (m, 2H), 1.40 (s, 9H), 1.14 (t, J = 7.1 Hz, 3H), 1H obscured by DMSO and 1H by obscured by water |
| **INTC188** | methyl 2-(2-(methylthio)pyrimidin-4-yl)-5,8-dioxaspiro[3.4]octane-2-carboxylate 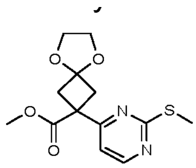 | Method P, [HPLC acidic], 297 (1.76). | No data collected |
| **INTC190** | methyl 4-methoxy-1-(2-(methylthio) pyrimidin-4-yl)cyclohexane-1-carboxylate 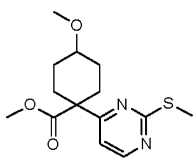 | Method P, [HPLC acidic], 297 (2.00). | 8.63 - 8.56 (m, 1H), 7.22 (d, J = 5.2 Hz, 1H), 3.65 (s, 3H), 3.24 (s, 3H), 3.23 - 3.20 (m, 1H), 2.48 (s, 3H), 2.40 - 2.28 (m, 2H), 1.93-1.80 (m, 4H), 1.36 - 1.24 (m, 2H). |
| **INTC236** | 1-(tert-Butyl) 4-methyl 4-(6-(methylthio)pyrazin-2-yl)piperidine-1,4-dicarboxylate 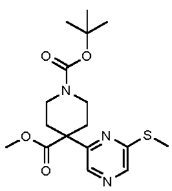 | Method P, [UPLC acidic], (M-Boc)+H 268, (1.58) | 8.53 (s, 1H), 8.40 (s, 1H), 3.72 - 3.59 (m, 2H), 3.66 (s, 3H), 3.24 - 3.00 (m, 2H), 2.52 (s, 3H), 2.25-2.30 (m, 2H), 2.00-2.10 (m, 2H), 1.40 (s, 9H). |

(continued)

| INTC | Name/Structure (All examples containing chiral centres are racemates unless stated) | Synthesis Method, [LCMS Method], m/z (M+H)+, (Rt/min) | $^1$H NMR Chemical Shift Data (DMSO-d6 unless stated) |
|---|---|---|---|
| INTC241 | 1-(tert-Butyl) 4-methyl 4-(4-(methylthio)pyrimidin-2-yl)piperidine-1,4-dicarboxylate 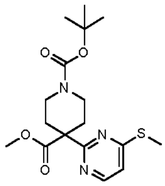 | Method P, [UPLC Acidic], (M-tBu)+H 312, (1.57) | 8.45 (d, J = 5.5 Hz, 1H), 7.35 (d, J = 5.5 Hz, 1H), 3.64 (s, 3H), 3.62 - 3.55 (m, 2H), 2.53 - 2.47 (m, 2H), 2.21 - 2.09 (m, 4H), 1.40 (s, 9H). 3H not observed, obscured by DMSO peak. |

Ethyl 4-(2-(methylthio)pyrimidin-4-yl)piperidine-4-carboxylate INTC191

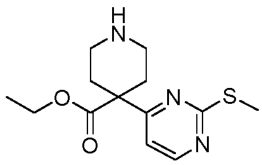

To a solution of 1-*tert*-butyl 4-ethyl 4-(2-(methylthio)pyrimidin-4-yl)piperidine-1,4-dicarboxylate (4 g, 9.44 mmol) **INTC187** in DCM (30 mL) at RT was added TFA (5 mL). The reaction mixture was stirred at RT for 1 hr. Additional TFA (5 mL) added and the reaction was stirred at RT for a further 1 hr. The reaction mixture was quenched by addition of $NaHCO_3$ (aq, 100mL), gas evolved, and was diluted with DCM (50 mL). The organics were isolated and dried ($MgSO_4$), filtered and solvent removed in vacuo to afford ethyl 4-(2-(methylthio)pyrimidin-4-yl)piperidine-4-carboxylate (2.6 g, 9.15 mmol, 97% yield) as a brown oil. Rt 0.97 min (HPLC, acidic); m/z 282 $(M+H)^+$ (ES+); No NMR data collected.

Ethyl 1-(methylsulfonyl)-4-(2-(methylthio)pyrimidin-4-yl)piperidine-4-carboxylate **INTC192**

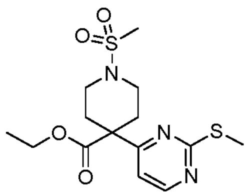

To a stirred solution of ethyl 4-(2-(methylthio)pyrimidin-4-yl)piperidine-4-carboxylate (1.3 g, 4.62 mmol) **INTC191** in DCM (15 mL) at RT was added TEA (1.42 mL, 10.16 mmol) and then MesCl (0.37 mL, 5.08 mmol). After 1 hr, 1 M HCl (*aq,* 50 mL) and DCM (30 mL) were added. The organic layer was isolated by passage through a phase separation cartridge and then concentrated in vacuo to afford ethyl 1-(methylsulfonyl)-4-(2-(methylthio)pyrimidin-4-yl)piperidine-4-carboxylate (1.21 g, 3.37 mmol, 73% yield) as a brown oil. Rt 1.93 min (HPLC, acidic); m/z 360 $(M+H)^+$ (ES+); No NMR data collected.

**Amide formation**

(4-(2-(Methylthio)pyrimidin-4-yl)tetrahydro-2H-pyran-4-yl)methanol INTC197

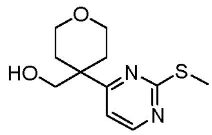

LiCl (0.95 g, 22.4 mmol) followed by $NaBH_4$ (0.85 g, 22.4 mmol) and EtOH (15 mL) was added into a stirring solution of methyl 4-(2-(methylthio)pyrimidin-4-yl)tetrahydro-2H-pyran-4-carboxylate **INTC178** (3 g, 11.2 mmol) in THF (15 mL).The resulting reaction mixture was stirred at RT for 18 hrs. The reaction mixture was acidified with 1M HCl (*aq,* 20 mL) and the volatiles were removed in vacuo. The residue was extracted with DCM (3 x 150 mL). The organic extract was dried ($MgSO_4$), filtered and solvent removed in vacuo. The crude product was purified by chromatography on silica gel (40 g column, 0-100% EA/*iso*-hexanes) to afford (4-(2-(methylthio)pyrimidin-4-yl)tetrahydro-2*H*-pyran-4-yl)methanol (2.3g, 9.09 mmol, 81% yield) as a colourless gum. Rt 0.80 min (UPLC acidic); *m/z* 241 $(M+H)^+$ $(ES^+)$. $^1H$ NMR (500 MHz, DMSO-d6) δ 8.54 (d, J = 5.3 Hz, 1H), 7.23 (d, J = 5.3 Hz, 1H), 4.73 (t, J = 5.6 Hz, 1H), 3.74-3.67 (m, 2H), 3.49 (d, J = 5.7 Hz, 2H), 3.35 - 3.27 (m, 2H), 2.50 (s, 3H), 2.19 - 2.10 (m, 2H), 1.77-1.67 (m, 2H).

4-(2-(Methylthio)pyrimidin-4-yl)tetrahydro-2*H*-pyran-4-carbaldehyde **INTC198**

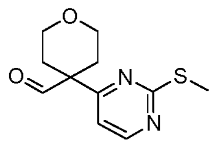

DMP (1.77 g, 4.16 mmol) was added portionwise into a stirring solution of (4-(2-(methylthio)pyrimidin-4-yl) tetrahydro-2*H*-pyran-4-yl)methanol **INTC197** (1 g, 4.16 mmol) in DCM (25 ml). The resulting reaction mixture was stirred at RT for 1 hr. The reaction mixture was poured into sat. $NaHCO_3$ (aq, 100 mL) and extracted with DCM (3 x 100 mL). The organic extract was sequentially washed with saturated sat. $NaHCO_3$ (aq, 100 mL), and brine (100 mL). The organic extract was dried ($MgSO_4$), filtered and solvent in vacuo to afford 4-(2-(methylthio)pyrimidin-4-yl)tetrahydro-2*H*-pyran-4-carbaldehyde (900 mg, 3.40 mmol, 82% yield) as a colorless oil. Rt 1.61 min (HPLC acidic); m/z 239 $(M+H)^+$ $(ES^+)$. $^1H$ NMR (500 MHz, DMSO-d6) δ 9.63 (s, 1H), 8.65 (d, J = 5.2 Hz, 1H), 7.30 (d, J = 5.2 Hz, 1H), 3.68-3.59 (m, 2H), 3.56-3.48 (m, 2H), 2.51 (s, 3H), 2.28 - 2.20 (m, 2H), 2.16-2.09 (m, 2H).

4-(6-Ethoxypyrazin-2-yl)-*N*-((4-(2-(methylthio)pyrimidin-4-yl)tetrahydro-2*H*-pyran-4-yl)methyl)aniline **INTC199**

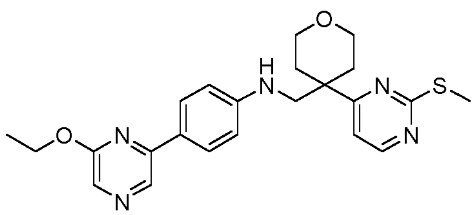

$NaBH(OAc)_3$ (1.07 g, 5.04 mmol) was added into a suspension of 4-(2-(methylthio)pyrimidin-4-yl)tetrahydro-2*H*-pyran-4-carbaldehyde **INTC198** (400 mg, 1.68 mmol) and 4-(6-ethoxypyrazin-2-yl)aniline **INTD18** (542 mg, 2.52 mmol) in DCM (10 ml) and the resulting reaction mixture was stirred at ambient temperature for 18 hrs. The reaction mixture was diluted with DCM (100 mL) and sequentially washed with sat. $NaHCO_3$ (aq, 2 x 100 mL) and brine (100 mL), dried ($MgSO_4$), filtered and solvent removed in vacuo. The crude product was purified by chromatography on silica gel (25 g cartridge, 0-100% EtOAc/iso-hexanes) to afford 4-(6-ethoxypyrazin-2-yl)-*N*-((4-(2-(methylthio)pyrimidin-4-yl)tetrahydro-2H-pyran-4-yl)methyl)aniline (312 mg, 0.706 mmol, 42% yield) as a yellow gum. Rt 2.49 min (HPLC acidic); *m/z* 438 $(M+H)^+$ $(ES^+)$. $^1H$ NMR (500 MHz, DMSO-d6) δ 8.57 (s, 1H), 8.52 (d, J = 5.2 Hz, 1H), 7.99 (s, 1H), 7.81 - 7.74 (m, 2H), 7.29 (d, J = 5.3 Hz, 1H), 6.66 - 6.57 (m, 2H), 6.00 (t, J = 6.6 Hz, 1H), 4.42 (q, J = 7.0 Hz, 2H), 3.80 - 3.70 (m, 2H), 3.38 (d, J = 6.6 Hz, 2H), 3.32 - 3.26 (m, 2H), 2.52 (s, 3H), 2.32 - 2.26 (m, 2H), 1.89-1.80 (m, 2H), 1.38 (t, J = 7.0 Hz, 3H).

**Method Q: Oxidation of thioethers to sulfones or sulfoxides**

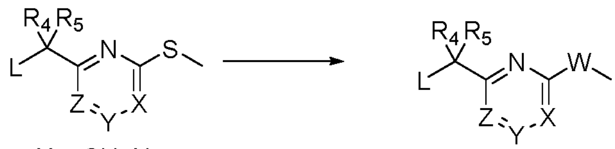

X = CH, N
Y = $CR_2$, N
Z = $CR_3$, N
L = C(O)OAlkyl, C(O)NHAr, $CH_2NHAr$

W = SO, $SO_2$

mCPBA (2.2 eq) was portionwise to a stirred solution of thiother (1 eq) in DCM (20-50 volumes) maintaining the internal temperature at RT. The resulting mixture was stirred at RT for a further 3 hrs. The reaction mixture was poured into sat. aq. $Na_2SO_3$ and extracted with DCM. The organic extract was sequentially washed with sat. aq. $NaHCO_3$ and brine, dried ($MgSO_4$), filtered and solvent removed *in vacuo* to afford the desire compound.

**Table 2:** The following intermediates were made according to Method Q.

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| INTC203 | methyl 8-(2-(methylsulfonyl)pyrimidin-4-yl)-1,4-dioxaspiro[4.5]decane-8-carboxylate 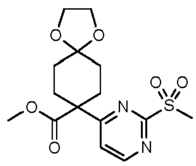 | Method Q using INTC186, [HPLC acidic], 357 (1.49). | 9.06 (d, J = 5.3 Hz, 1H), 7.93 (d, J = 5.3 Hz, 1H), 3.91 - 3.83 (m, 4H), 3.66 (s, 3H), 3.41 (s, 3H), 2.37-2.29 (m, 2H), 2.29 - 2.18 (m, 2H), 1.68-1.56 (m, 4H). |
| INTC207 | 4-(6-ethoxypyrazin-2-yl)-N-((4-(2-(methylsulfinyl)pyrimidin-4-yl)tetrahydro-2H-pyran-4-yl) methyl)aniline 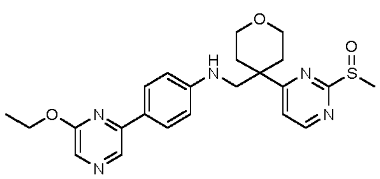 | Method Q using INTC199, [HPLC, acidic], 454 (1.89). | 8.87 (d, J = 5.3 Hz, 1H), 8.56 (s, 1H), 7.99 (s, 1H), 7.77 - 7.71 (m, 3H), 6.57 - 6.47 (m, 2H), 6.07 (t, J = 6.7 Hz, 1H), 4.41 (q, J = 7.0 Hz, 2H), 3.80-3.74 (m, 2H), 3.47-3.42 (m, 2H), 3.31 - 3.24 (m, 2H), 2.83 (s, 3H), 2.36 (d, J = 13.4 Hz, 2H), 1.95 - 1.84 (m, 2H), 1.37 (t, J = 7.0 Hz, 3H). |

(continued)

| INTC | Name/Structure (All examples containing chiral centres are racemates unless stated) | Synthesis Method, [LCMS Method], m/z (M+H)$^+$, (Rt/min) | $^1$H NMR Chemical Shift Data (DMSO-d6 unless stated) |
|---|---|---|---|
| **INTC208** | methyl 2-(2-(methylsulfonyl)pyrimidin-4-yl)-5,8-dioxaspiro[3.4]octane-2-carboxylate 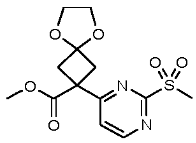 | Method Q using INTC188, [HPLC, acidic], 329 (1.27). | No data collected |
| **INTC210** | methyl 4-methoxy-1-(2-(methylsulfonyl)pyrimidin-4-yl)cyclohexane-1-carboxylate 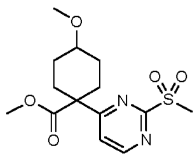 | Method Q using INTC190, [HPLC acidic], 329 (1.46). | No data collected |
| **INTC233** | *tert*-Butyl 4-((5-(6-ethoxypyrazin-2-yl)pyridin-2-yl)carbamoyl)-4-(2-(methylsulfinyl)pyrimidin-4-yl)piperidine-1-carboxylate 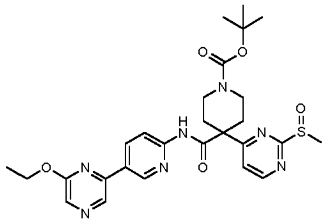 | Method Q using **INTC232,** [HPLC Acidic], 568, (2.23) | No NMR recorded |
| **INTC237** | 1-(*tert*-Butyl) 4-methyl 4-(6-(methylsulfonyl)pyrazin-2-yl)piperidine-1,4-dicarboxylate 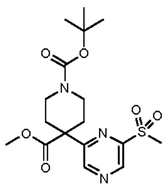 | Method Q using **INTC236,** [UPLC acidic], (M-Boc)+H 300, (1.32) | 9.19 (s, 1H), 9.17 (s, 1H), 3.67 (s, 3H), 3.59 - 3.46 (m, 2H), 3.35 (s, 3H), 3.30-3.20 (m, 2H), 2.36 - 2.28 (m, 2H), 2.28 - 2.19 (m, 2H), 1.40 (d, J = 5.3 Hz, 9H). |
| **INTC242** | 1-(*tert*-Butyl) 4-methyl 4-(4-(methylsulfinyl)pyrimidin-2-yl)piperidine-1,4-dicarboxylate 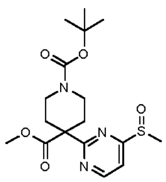 | Method Q using **INTC241,** [UPLC Acidic], (M-Boc)+H 284, (0.57) | No NMR recorded |

**Method R: Formation of sulfonamides from aromatic sulfones**

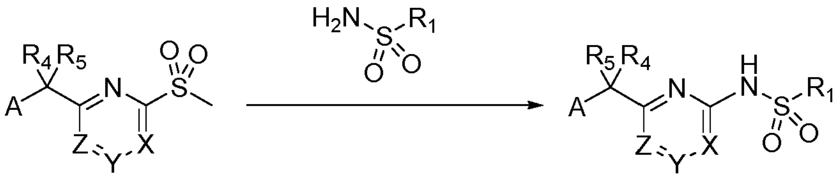

A = $CONHAr_1Ar_2$, $NHCOAr_1Ar_2$ or $CO_2Alkyl$
X = CH, N
Y = $CR_2$, N
Z = $CR_3$, N

To a solution of sulfone (1.0 eq) and primary sulfonamide (1.1 - 2.0 eq) in polar aprotic solvent such as NMP (5-100 volumes) was added an inorganic base (3 eq) such as cesium carbonate and heated to 40-90 °C for 1-3 hrs. The reaction mixture was cooled to RT and diluted with water (50-100 volumes) and the mixture was washed with MTBE (100 volumes) and the aqueous was slowly acidified to pH 5 or lower using an appropriate acid such as HCl. The resulting precipitate was filtered to afford desired sulfonamide product.

**Table 3: The following intermediates were made according to Method R.**

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Base, Solvent |
|---|---|---|---|---|
| **INTC214** | methyl 8-(2-(cyclopropanesulfonamido)pyrimidin-4-yl)-1,4-dioxaspiro [4.5]decane-8-carboxylate 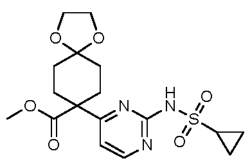 | Method R using INTC203, [HPLC acidic], 398 (1.63). | 11.28 (s, 1H), 8.63-8.54 (m, 1H), 7.21-7.11 (m, 1H), 3.87 (s, 3H), 3.65 (s, 4H), 3.24-3.15 (m, 1H), 2.31-2.23 (m, 2H), 2.18 - 2.02 (m, 2H), 1.70 - 1.52 (m, 4H), 1.19 - 0.99 (m, 4H). | $Cs_2CO_3$, NMP |
| **INTC215** | ethyl 4-(2-(cyclopropanesulfonamido)pyrimidin-4-yl)-1-(methylsulfonyl)piperidine-4-carboxylate 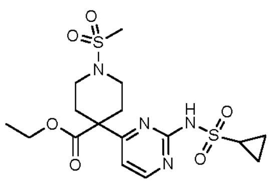 | Method R using INTC205, [HPLC acidic], 433 (1.63). | No data collected | $Cs_2CO_3$, NMP |
| **INTC216** | methyl 2-(2-(cyclopropanesulfonamido)pyrimidin-4-yl)-5,8-dioxaspiro [3.4]octane-2-carboxylate 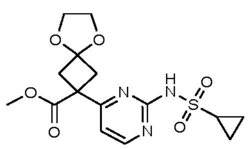 | Method R using INTC208, [UPLC acidic], 370 (0.48). | 11.32 (s, 1H), 8.60 (d, J = 5.2 Hz, 1H), 7.20 (d, J = 5.2 Hz, 1H), 3.87 - 3.82 (m, 2H), 3.82 - 3.78 (m, 2H), 3.66 (s, 3H), 3.27 - 3.18 (m, 1H), 3.05 - 2.97 (m, 2H), 2.98 - 2.84 (m, 2H), 1.17 - 1.08 (m, 2H), 1.07 - 0.99 (m, 2H). | $Cs_2CO_3$, NMP |

(continued)

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Base, Solvent |
|---|---|---|---|---|
| **INTC218** | methyl 1-(2-(cyclopropanesulfonamido)pyrimidin-4-yl)-4-methoxycyclohexane-1-carboxylate 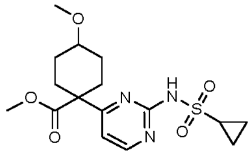 | Method R using INTC210, [HPLC acidic], 370 (1.62). | 11.25 (s, 1H), 8.57 (d, J = 5.3 Hz, 1H), 7.23 - 7.11 (m, 1H), 3.65 (s, 3H), 3.24 (s, 3H), 3.23-3.20 (m, 1H), 2.48-2.54 (m 1H, obscured by DMSO peak) 2.37-2.29 (m, 2H), 1.97 - 1.80 (m, 4H), 1.36 - 1.24 (m, 2H), 1.16 - 1.01 (m, 4H). | $Cs_2CO_3$, NMP |
| **INTC234** | *tert*-Butyl 4-((5-(6-ethoxypyrazin-2-yl)pyridin-2-yl)carbamoyl)-4-(2-(ethylsulfonamido)pyrimidin-4-yl)piperidine-1-carboxylate 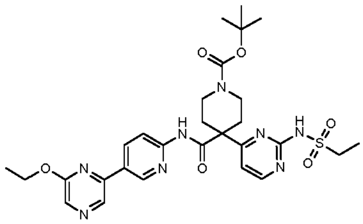 | Method R using **INTC233** and ethane sulfonamide, [HPLC Acidic], 613, (2.48) | 11.23 (s, 1H), 10.18 (s, 1H), 9.04 (dd, J = 2.5, 0.8 Hz, 1H), 8.85 (s, 1H), 8.61 (d, J = 5.3 Hz, 1H), 8.51 (dd, J = 8.8, 2.5 Hz, 1H), 8.26 (s, 1H), 8.23 - 8.17 (m, 1H), 7.23 (d, J = 5.3 Hz, 1H), 4.48 (q, J = 7.0 Hz, 2H), 3.68 - 3.62 (m, 2H), 3.56 - 3.48 (m, 2H), 2.44 (d, J = 14.1 Hz, 2H), 2.13 - 2.04 (m, 2H), 1.41 (d, J = 4.6 Hz, 11H), 1.13 (t, J = 7.3 Hz, 3H), 0.89 - 0.79 (m, 3H). | $Cs_2CO_3$, NMP |
| **INTC238** | 1-(*tert*-Butyl) 4-methyl 4-(6-(cyclopropanesulfonamido)pyrazin-2-yl) piperidine-1,4-dicarboxylate 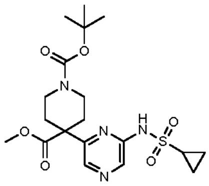 | Method R using **INTC237** and cyclopropyl sulfonamide, [UPLC Basic], (M-Boc)+H 341, (1.12) | No NMR recorded | $Cs_2CO_3$, NMP |
| **INTC243** | *1-(tert-Butyl)* 4-methyl 4-(4-(cyclopropanesulfonamido)pyrimidin-2-yl)piperidine-1,4-dicarboxylate 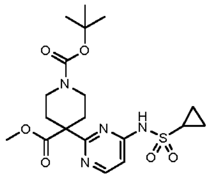 | Method R using **INTC242** and cyclopropyl sulfonamide, [HPLC Acidic], (M-Boc)+H 341, (2.03) | 11.31 (s, 1H), 8.51 (d, J = 5.7 Hz, 1H), 6.82 (d, J = 5.7 Hz, 1H), 3.66 - 3.59 (m, 4H), 3.15 - 3.06 (m, 4H), 2.18 - 2.05 (m, 4H), 1.40 (s, 10H), 1.15 - 1.05 (m, 4H). | $Cs_2CO_3$, NMP |

*N*-(5-(6-ethoxypyrazin-2-yl)pyridin-2-yl)-4-(2-(ethylsulfonamido)pyrimidin-4-yl)piperidine-4-carboxamide hydrochloride **INTC235**

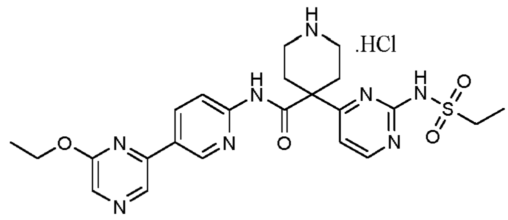

This compound was prepared by Boc-deprotection with HCl of **INTC234,** [HPLC acidic], 513, (2.72).

4-(6-(Cyclopropanesulfonamido)pyrazin-2-yl)-*N*-(5-(6-ethoxypyrazin-2-yl)pyridin-2-yl)piperidine-4-carboxamide hydrochloride **INTC240**

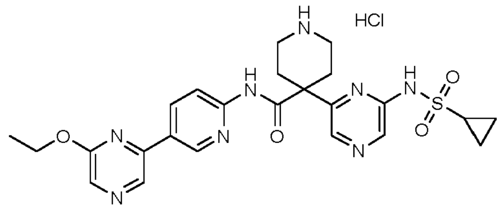

This compound was prepared by Boc-deprotection with HCl of **INTC239,** [UPLC Acidic], 525, (0.83); $^1$H NMR (DMSO-d6) 11.18 (s, 1H), 10.39 (s, 1H), 9.02 (d, J = 2.4 Hz, 1H), 8.94 - 8.73 (m, 3H), 8.52 (dd, J = 8.8, 2.4 Hz, 1H), 8.41 (s, 1H), 8.26 (s, 1H), 8.23 (s, 1H), 8.19 (d, J = 8.8 Hz, 1H), 4.47 (q, J = 7.0 Hz, 2H), 3.37 - 3.22 (m, 2H), 3.15 - 3.11 (m, 3H), 2.74 - 2.68 (m, 2H), 2.43 - 2.40 (m, 2H), 1.39 (t, J = 7.0 Hz, 3H), 1.09 - 1.03 (m, 2H), 0.91 - 0.82 (m, 2H).

4-(4-(Cyclopropanesulfonamido)pyrimidin-2-yl)-*N*-(5-(6-cyclopropylpyrazin-2-yl)pyridin-2-yl)piperidine-4-carboxamide hydrochloride **INTC245**

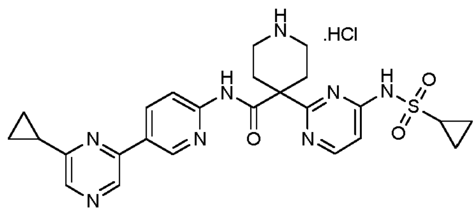

This compound was prepared by Boc-deprotection with HCl of **INTC244,** [HPLC Acidic], 521, (1.30); $^1$H NMR (DMSO-d6) 11.42 (s, 1H), 10.23 (s, 1H), 9.03 - 8.96 (m, 2H), 8.89 - 8.70 (m, 2H), 8.63 - 8.57 (m, 2H), 8.52 - 8.46 (m, 1H), 8.21 (d, J = 8.8 Hz, 1H), 6.89 (d, J = 5.7 Hz, 1H), 3.29 - 3.07 (m, 5H), 2.53 (s, 2H), 2.29 - 2.22 (m, 1H), 1.13 - 1.06 (m, 6H), 0.95 - 0.89 (m, 2H). 2H not observed potentially obscured broad $H_2O$ peak.

4-(4-(Cyclopropanesulfonamido)pyrimidin-2-yl)-*N*-(5-(6-ethoxypyrazin-2-yl)pyridin-2-yl)piperidine-4-carboxamide hydrochloride **INTC247**

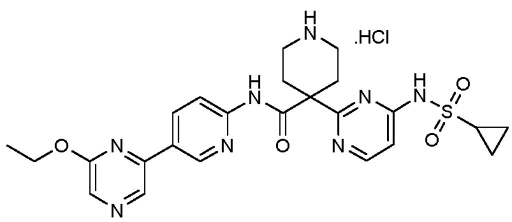

This compound was prepared by Boc-deprotection with HCl of **INTC246,** [HPLC Acidic], 521, (1.30).

**Method 13: *t-BuOK* mediated amide coupling from ester**

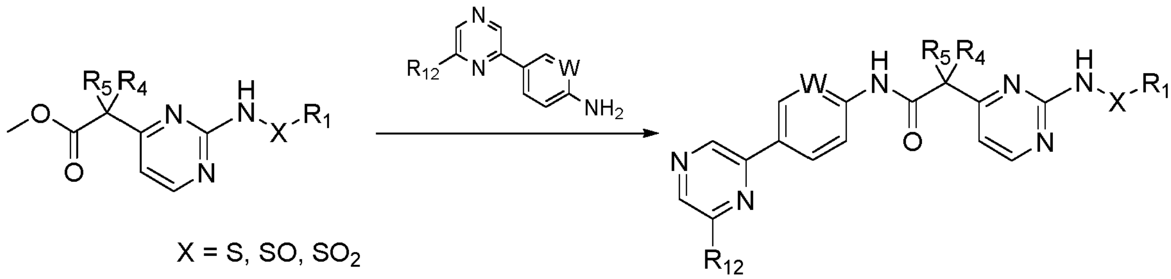

A stirred solution of ester (1.0 eq) and amine (1.1 eq) in THF (10-50 volumes) and DMSO (5 volumes) was cooled to 0 °C under an inert atmosphere. To the reaction mixture was added a solution of t-BuOK (3.3 eq) as a solution THF (10-50 volumes) over 15-60 mins. After addition was complete the reaction was warmed to RT for 1 hr. Reaction was quenched by the addition of aqueous acid either acetic acid or dilute HCl (1 M) until acidic pH was achieved. The reaction was diluted with water and extracted with EtOAc. The organics were combined, dried (phase separator) and concentrated *in vacuo.* The crude product was purified by reverse or normal phase chromatography or a combination of both.

| INTC | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| **INTC232** | *tert*-Butyl 4-((5-(6-ethoxypyrazin-2-yl)pyridin-2-yl)carbamoyl)-4-(2-(methylthio)pyrimidin-4-yl)piperidine-1-carboxylate 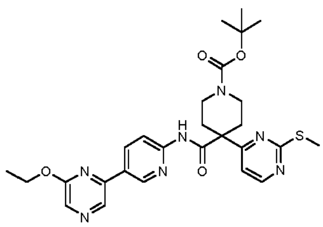 | Method 13 using **INTC187** and **INTD33,** [HPLC Acidic], 552, (2.78) | No NMR recorded |

(4-(6-Ethoxypyrazin-2-yl)-2-fluorophenyl)methanol **INTD87**

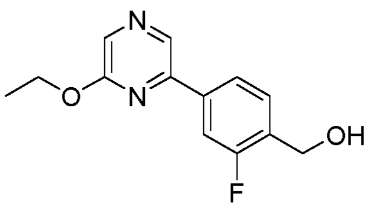

Prepared as for **INTD84** using (4-bromo-2-fluorophenyl)methanol (205 mg, 1.00 mmol) and 2-ethoxy-6-(4,4,5,5-tetramethyl-1,3,2-dioxaborolan-2-yl)pyrazine (250 mg, 1.00 mmol) to afford (4-(6-ethoxypyrazin-2-yl)-2-fluorophenyl) methanol (260 mg, 0.995 mmol, quantitative yield) was isolated as a yellow gum. Rt 1.25 min (UPLC, acidic); m/z 249 $(M+H)^+$ $(ES^+)$; $^1$H NMR (500 MHz, DMSO-d6) δ 8.85 (s, 1H), 8.26 (s, 1H), 7.99 (dd, J = 8.0, 1.7 Hz, 1H), 7.91 (dd, J = 11.6, 1.7 Hz, 1H), 7.66 - 7.57 (m, 1H), 5.37 (t, J = 5.8 Hz, 1H), 4.62 (d, J = 5.8 Hz, 2H), 4.49 (q, J = 7.0 Hz, 2H), 1.41 (t, J = 7.0 Hz, 3H).

4-(6-Ethoxypyrazin-2-yl)-2-fluorobenzaldehyde **INTD88**

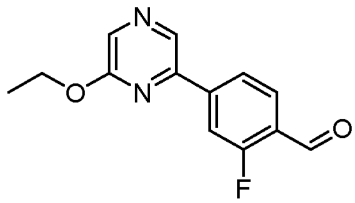

Prepared as for INTD85 using (4-(6-ethoxypyrazin-2-yl)-2-fluorophenyl)methanol **INTD87** (1.00 g, 4.03 mmol) to afford 4-(6-ethoxypyrazin-2-yl)-2-fluorobenzaldehyde (448 mg, 1.78 mmol, 44% yield) as a colourless solid. Rt 0.67 min (UPLC 2, acidic); m/z 247 $(M+H)^+$ $(ES^+)$; $^1$H NMR (500 MHz, DMSO-d6) δ 10.28 (s, 1H), 8.98 (s, 1H), 8.37 (s, 1H), 8.22 - 8.13 (m, 2H), 7.99 (dd, J = 8.3, 7.3 Hz, 1H), 4.52 (q, J = 7.0 Hz, 2H), 1.42 (t, J = 7.0 Hz, 3H).

**Compounds of formula (I-a) - Examples**

The synthesis of a number of known CTPS1 inhibitors is disclosed in WO2019/179652, WO2019/180244 and WO2020/083975 (see compounds **P1** to **P225).** Such compounds are made using general methods disclosed herein and represent further examples of compounds which are CTPS1 inhibitors. The full synthetic methods and characterising data for compounds **P1** to **P225** are provided in WO2019/179652, WO2019/180244 and WO2020/083975.

**Amide formation**

**Method 1: Amide coupling using HATU**

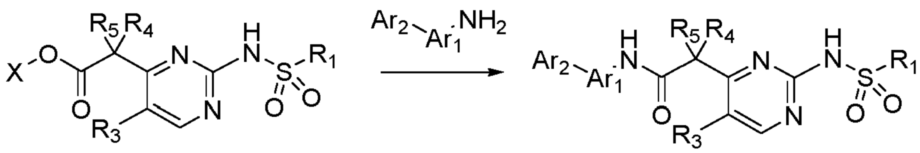

To a stirred suspension of the acid or the potassium salt (1 eq, X= H or K) and DIPEA (6 eq) in DMF (15 vol) the aniline (1 eq) and HATU (1.5 eq) were added. The reaction was stirred at RT for 18 hrs then concentrated *in vacuo.* MeOH and 2M NaOH (aq) were added. The mixture was stirred for 30 min then concentrated *in vacuo.* The aqueous phase acidified to pH 6 with 1M HCl (aq) and the product extracted into DCM. The organics were combined, dried (phase separator) and concentrated *in vacuo.*

The crude product was purified by reverse or normal phase chromatography or a combination of both.

Reference Example: N-(4-(5-Chloropyridin-3-yl)phenyl)-2-(2-(cyclopropanesulfonamido)pyrimidin-4-yl)butanamide **P1**

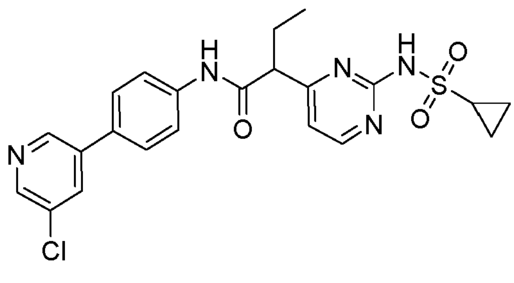

**Method 2: $AlMe_3$ mediated amide coupling from ester**

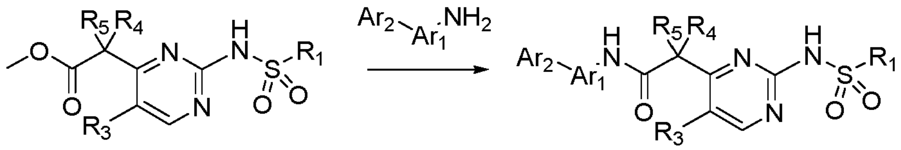

To an ice cooled solution of aniline (2 eq) in toluene (40 volumes) was added $AlMe_3$ (2.0 M in heptane, 2 eq). The mixture was stirred at this temperature for 5 mins then at RT for 10 mins. To this solution was added ester (1 eq) in one portion and the resultant mixture heated and stirred at 80 °C for 2 hrs. The reaction mixture was cooled in an ice bath and carefully quenched with MeOH (10 volumes). After stirring for 20 mins the mixture was diluted in a mixture of DCM/MeOH (10 volumes), filtered through celite and the filtrate concentrated. The crude product was purified by reverse or normal phase chromatography.

Reference Example: 1-(2-(Cyclopropanesulfonamido)pyrimidin-4-yl)-*N*-(4-(6-ethoxypyrazin-2-yl)phenyl)cyclopentane-carboxamide **P2**

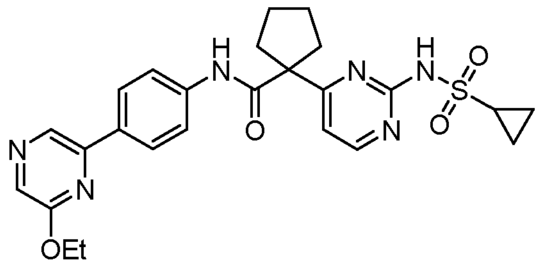

Reference Example: 2-(2-(cyclopropanesulfonamido)pyrimidin-4-yl)-*N*-(4-(6-methoxypyrazin-2-yl)phenyl)-2-methyl-propanamide **P3**

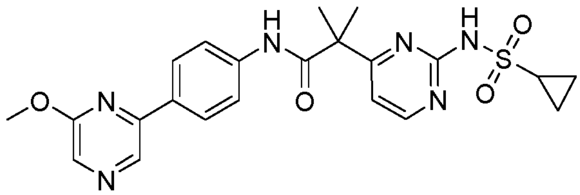

Reference Example: 2-(2-(Cyclopropanesulfonamido)pyrimidin-4-yl)-2-methyl-*N*-(4-(5-(trifluoromethyl)pyridin-3-yl) phenyl)propanamide **P4**

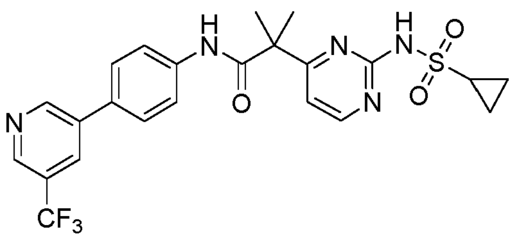

Reference Example: 2-Methyl-N-(2-methyl-4-(6-methylpyrazin-2-yl)phenyl)-2-(2-(methylsulfonamido)pyrimidin-4-yl)propanamide **P5**

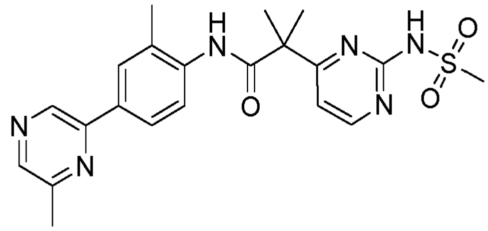

**Method 2b: $DABALMe_3$ mediated amide coupling from ester**

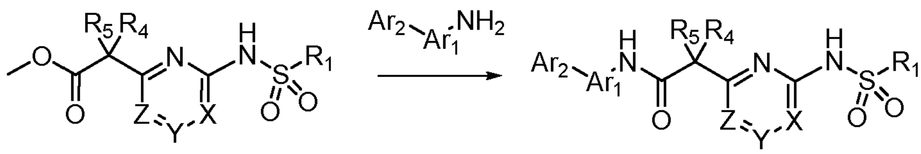

X = CH, N
Y = $CR_2$, N
Z = $CR_2$, N

To a solution of ester (1 eq) and aniline (1.5 eq) in toluene (30 volumes) was added DABAL-$Me_3$ (1.5 eq) and the resulting mixture was heated at 100 °C for 4 h. The reaction mixture was cooled to 0 °C and quenched by careful addition of 1 M HCl (aq, 20 volumes). The aqueous phase was extracted with EtOAc (3 x 20 volumes). The combined organics were washed with 1 M HCl (aq, 2 x 10 volumes), dried over $Na_2SO_4$, filtered and concentrated *in vacuo*. The crude product was purified by reverse or normal phase chromatography.

**Method 3: Amide coupling from potassium salt using T3P**

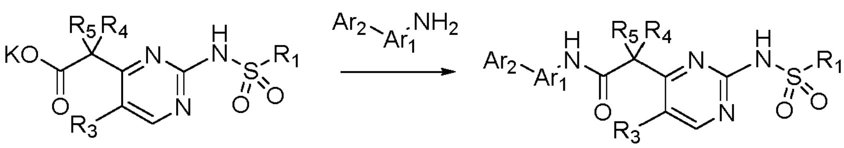

Pyridine (10 eq) followed by T3P (50 wt% in DMF, 2 eq) was added to a stirring solution of amine (1.1 eq) and potassium 2-(2-(cyclopropanesulfonamido)pyrimidin-4-yl)butanoate (1 eq) in DMF (16 volumes). The resulting reaction was stirred at RT for 24 hrs. The crude reaction mixture was concentrated *in vacuo* then diluted with $NH_4Cl$ (sat. aq) and extracted with DCM. The combined organic extracts were dried (phase separator) and the solvent removed. The crude product was purified by reverse or normal phase chromatography.

Reference Example: 2-(2-(Cyclopropanesulfonamido)pyrimidin-4-yl)-*N*-(2-fluoro-4-(pyrazin-2-yl)phenyl)butanamide **P6**

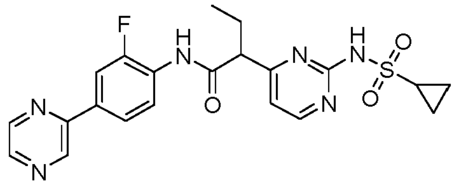

Reference Example: 2-(2-(Cyclopropanesulfonamido)pyrimidin-4-yl)-N-(4-(5-(trifluoromethyl)pyridin-3-yl)phenyl)butanamide **P7**

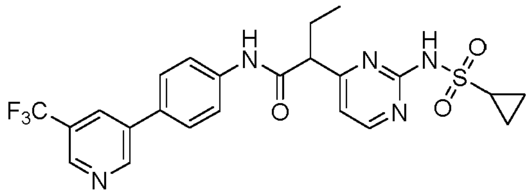

Reference Example: 2-(2-(Cyclopropanesulfonamido)pyrimidin-4-yl)-N-(4-(6-(trifluoromethyl)pyrazin-2-yl)phenyl)acetamide **P8**

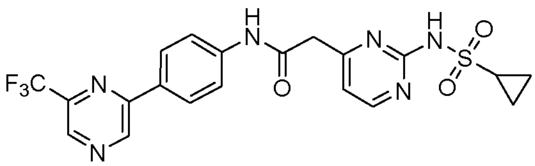

**Method 4: Amide coupling from lithium salt using T3P**

*N*-(5-(6-Ethoxypyrazin-2-yl)pyridin-2-yl)-2-fluoro-2-(2-(*N*-(4-methoxybenzyl)cyclopropanesulfonamido)pyrimidin-4-yl) butanamide INTC51

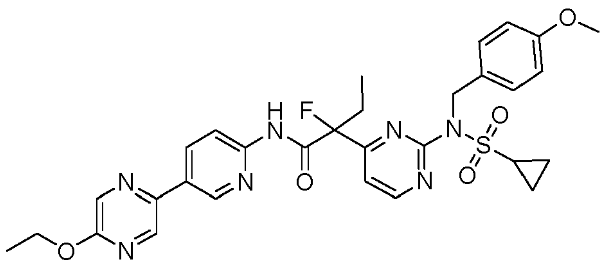

**Method 5: NH-Amide formation *via* amide deprotection and/or decarboxylation**

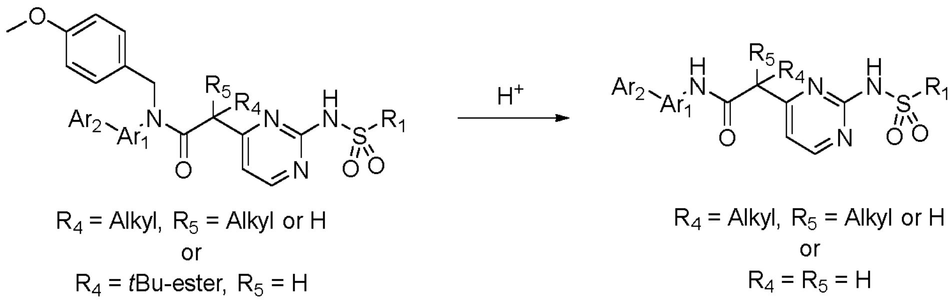


To a solution of the protected amide in DCM a mixture of TFA (88 eq) and triflic acid (1-6 eq) was added and the mixture left stirring at RT for 18-36 hrs and then concentrated *in vacuo.* The crude product was purified by column chromatography on silica gel or by RP chromatography.

**Method 6: Deprotection of Sulfonamide**

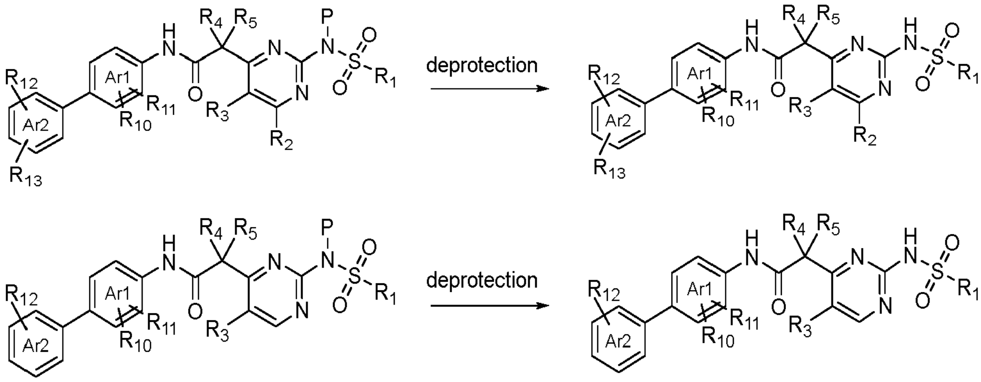


**Method 7: Sulfonylation from aromatic chloride**

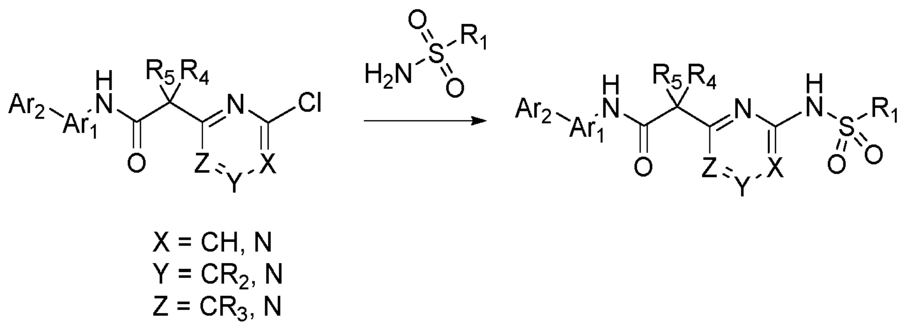


2-Chloro-heteroaromatic intermediate (1 eq), sulfonamide (1.2 eq) and base (2 eq) were dissolved in dioxane (40 volumes). The mixture was degassed (evacuated and backfilled with $N_2$ x 3) then catalyst (10 mol%) was added. The resulting mixture was heated under nitrogen at 90 °C for 2 hrs. The mixture was cooled to RT, diluted with sat. $NH_4Cl$ (aq, 80 volumes) and DCM (80 volumes). The phases were separated and the aqueous was extracted with further DCM (2 x 80 volumes). The combined organics were dried ($MgSO_4$), filtered and concentrated *in vacuo.* The crude product was purified by normal phase chromatography or trituration using a suitable solvent.

**Method 8: Amide coupling using 1-chloro-*N*,*N*,2-trimethylprop-1-en-1-amine**

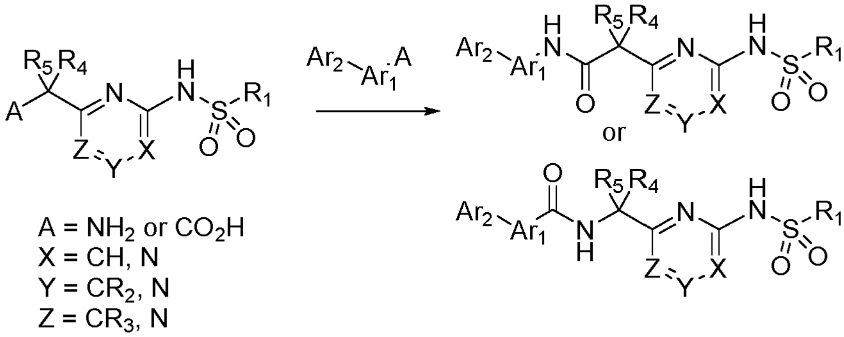


1-Chloro-*N*,*N*,2-trimethylprop-1-en-1-amine (2 eq) was added to a solution of carboxylic acid (1 eq) in DCM (20 volumes). The reaction mixture was stirred at RT for 2 hrs. The reaction mixture was concentrated *in vacuo* and the residue redissolved in DCM (20 volumes) before addition of pyridine (2 mL) followed by addition of the appropriate amine (1.1 eq). The reaction mixture was stirred at RT for 2 hrs. An aqueous work up was performed and the crude product was purified by normal phase chromatography, reverse phase chromatography or trituration from an appropriate solvent.

**Method 9: Suzuki ArBr**

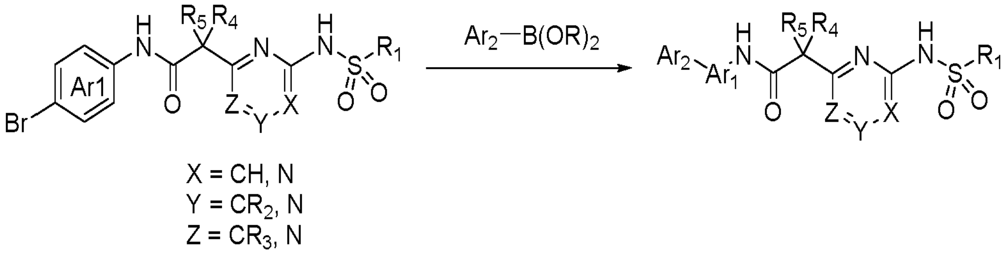


To a suspension of Ar1-Br (1 eq) in dioxane (10 volumes) was added arylboronic acid or ester (1 eq) and a solution of $K_2CO_3$ (2 eq) in water (5 volumes). The resulting suspension was degassed ($N_2$, 5 mins). $PdCl_2$(dppf)-$CH_2Cl_2$ adduct or other appropriate catalyst (10 mol%) was added and the reaction mixture was stirred at 80 °C for 2 hrs. The reaction mixture was then cooled to RT. An aqueous work up was performed and the crude product was purified by normal phase chromatography, reverse phase chromatography or trituration from an appropriate solvent.

**Method 10: T3P with free acid**

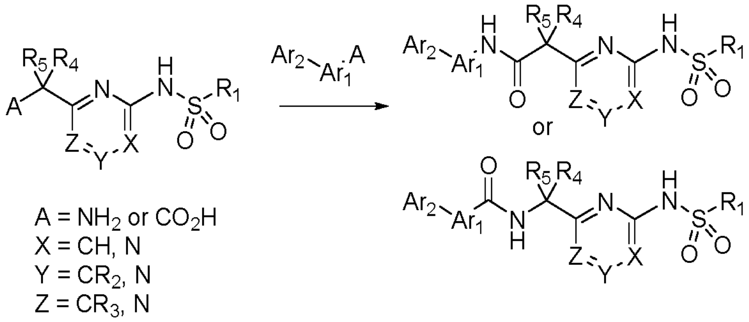


Pyridine (10 eq) followed by T3P (50 wt% in DMF, 2 eq) was added to a stirring solution of amine (1.1 eq) and carboxylic acid (1 eq) in DMF (16 volumes). The resulting reaction was stirred at RT for 24 hrs. The crude reaction mixture was concentrated *in vacuo* then diluted with $NH_4Cl$ (sat. aq) and extracted with DCM. The combined organic extracts were dried (phase separator) and the solvent removed. The crude product was purified by reverse or normal phase chromatography.

**Table 4:** Compounds **P9-P115, P117-P225**

| P | Name/Structure (All examples containing chiral centres are racemates unless stated) | P | Name/Structure (All examples containing chiral centres are racemates unless stated) |
|---|---|---|---|
| **Ref P9** | 2-(2-(cyclopropanesulfonamido)pyrimidin-4-yl)-N-(5-(6-isopropoxypyrazin-2-yl)pyridin-2-yl)-2-methylpropanamide 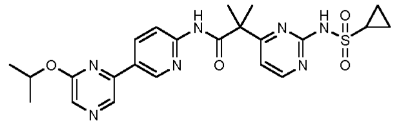 | **Ref P118** | N-((2-(cyclopropanesulfonamido)pyrimidin-4-yl)methyl)-4-(6-ethoxypyrazin-2-yl) benzamide 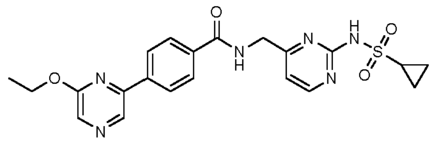 |
| **Ref P10** | 2-(2-(cyclopropanesulfonamido)pyrimidin-4-yl)-N-(4-(6-ethoxypyrazin-2-yl)phenyl)-2-ethylbutanamide 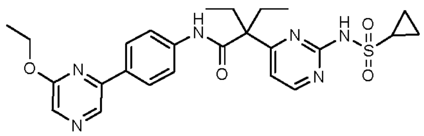 | **Ref P122** | 2-(2-(cyclopropanesulfonamido)pyrimidin-4-yl)-2-methyl-N-(5-(6-(prop-1-en-2-yl)pyrazin-2-yl)pyridin-2-yl)propanamide 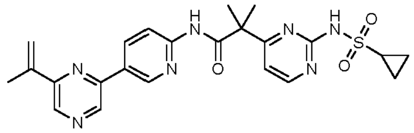 |
| **Ref P11** | 2-(2-(cyclopropanesulfonamido)pyrimidin-4-yl)-N-(2-fluoro-4-(6-(trifluoromethyl)pyrazin-2-yl)phenyl)acetamide 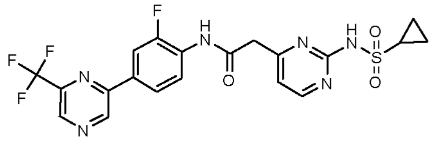 | **Ref P123** | 2-(2-(cyclopropanesulfonamido)-6-methylpyrimidin-4-yl)-N-(5-(6-ethoxypyrazin-2-yl) pyridin-2-yl)-2-methylpropanamide 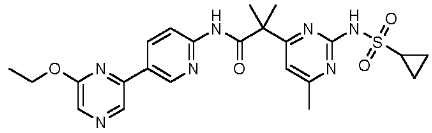 |
| **Ref P12** | 2-(2-(cyclopropanesulfonamido)pyrimidin-4-yl)-N-(2-fluoro-4-(6-isopropoxypyrazin-2-yl) phenyl)acetamide 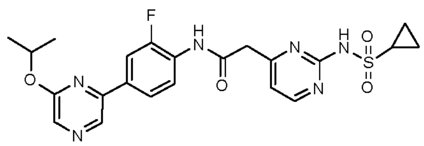 | **Ref P124** | 2-(2-(cyclopropanesulfonamido)-6-(trifluoromethyl)pyrimidin-4-yl)-N-(5-(6-ethoxypyrazin-2-yl)pyridin-2-yl)-2-methylpropanamide 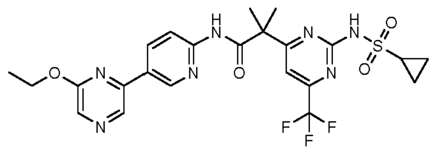 |
| **Ref P13** | 2-(2-(cyclopropanesulfonamido)pyrimidin-4-yl)-N-(4-(5-(trifluoromethyl)pyridin-3-yl)phenyl)acetamide 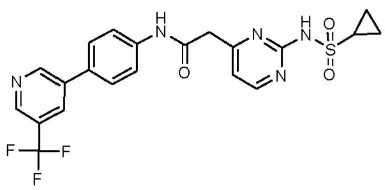 | **Ref P125** | 2-(2-(cyclopropanesulfonamido)pyrimidin-4-yl)-N-(5-(6-cyclopropylpyrazin-2-yl)pyridin-2-yl)-2-methylpropanamide 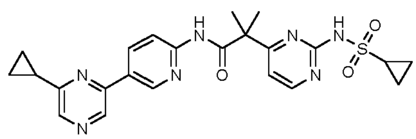 |
| **Ref P14** | 2-(2-(cyclopropanesulfonamido)pyrimidin-4-yl)-N-(4-(5-(2,2,2-trifluoroethoxy)pyridin-3-yl) phenyl)acetamide | **Ref P126** | 2-(2-(cyclopropanesulfonamido)pyrimidin-4-yl)-N-(6-(6-ethoxypyrazin-2-yl)pyridin-3-yl)-2-methylpropanamide 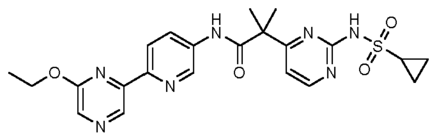 |

(continued)

| P | Name/Structure (All examples containing chiral centres are racemates unless stated) | P | Name/Structure (All examples containing chiral centres are racemates unless stated) |
|---|---|---|---|
| | 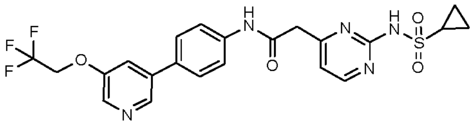 | | |
| **Ref P15** | 2-(2-(cyclopropanesulfonamido)-5-fluoropyrimidin-4-yl)-N-(4-(pyridin-3-yl)phenyl)acetamide 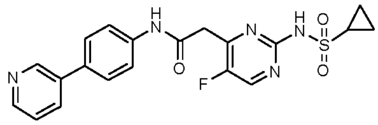 | **Ref P128** | 2-(2-(cyclopropanesulfonamido)pyrimidin-4-yl)-N-(4-(6-cyclopropylpyrazin-2-yl)-2-fluorophenyl)-2-methylpropanamide 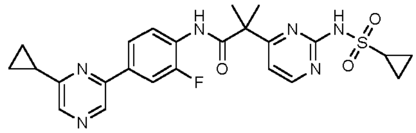 |
| **Ref P16** | 2-(2-(cyclopropanesulfonamido)pyrimidin-4-yl)-N-(4-(pyridin-3-yl)phenyl)acetamide 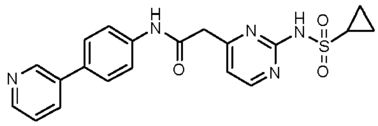 | **Ref P129** | 2-(2-(cyclopropanesulfonamido)-6-methylpyrimidin-4-yl)-N-(4-(6-ethoxypyrazin-2-yl)-2-fluorophenyl)-2-methylpropanamide 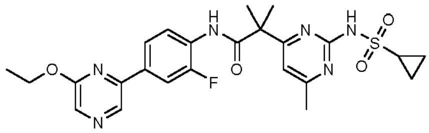 |
| **Ref P17** | N-([1,1'-biphenyl]-4-yl)-2-(2-(cyclopropanesulfonamido)pyrimidin-4-yl)acetamide 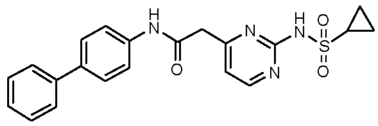 | **Ref P130** | 2-(2-(cyclopropanesulfonamido)-6-(trifluoromethyl)pyrimidin-4-yl)-N-(4-(6-ethoxypyrazin-2-yl)-2-fluorophenyl)-2-methylpropanamide 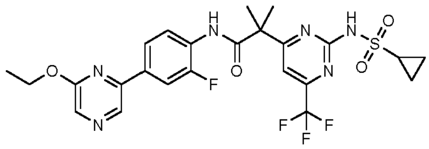 |
| **Ref P18** | 2-(2-(cyclopropanesulfonamido)pyrimidin-4-yl)-N-(4-(6-ethoxypyrazin-2-yl)phenyl)acetamide 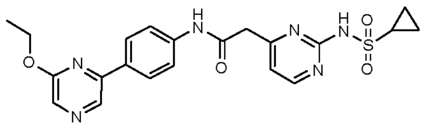 | **Ref P131** | 2-(2-(cyclopropanesulfonamido)pyrimidin-4-yl)-2-methyl-N-(4-(6-(prop-1-en-2-yl)pyrazin-2-yl)phenyl)propanamide 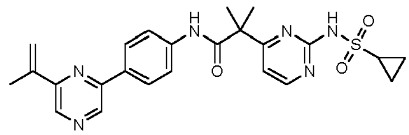 |
| **Ref P19** | 2-(2-(cyclopropanesulfonamido)pyrimidin-4-yl)-N-(4-(6-methoxypyrazin-2-yl)phenyl)acetamide 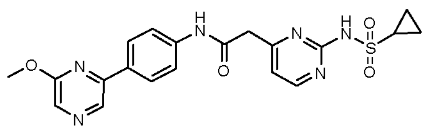 | **Ref P132** | 2-(2-(cyclopropanesulfonamido)pyrimidin-4-yl)-N-(4-(6-isopropylpyrazin-2-yl)phenyl)-2-methylpropanamide 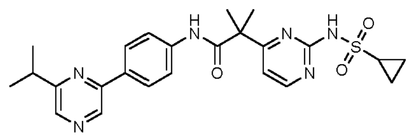 |

(continued)

| P | Name/Structure (All examples containing chiral centres are racemates unless stated) | P | Name/Structure (All examples containing chiral centres are racemates unless stated) |
|---|---|---|---|
| **Ref P20** | 2-(2-(cyclopropanesulfonamido)pyrimidin-4-yl)-N-(4-(6-(2,2,2-trifluoroethoxy)pyrazin-2-yl)phenyl)acetamide 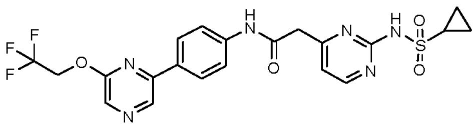 | **Ref P133** | 2-(2-(cyclopropanesulfonamido)pyrimidin-4-yl)-N-(4-(6-(dimethylamino)pyrazin-2-yl)phenyl)-2-methylpropanamide 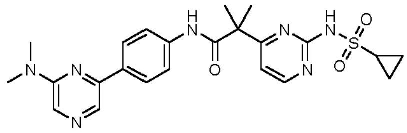 |
| **Ref P21** | 2-(2-(cyclopropanesulfonamido)pyrimidin-4-yl)-N-(4-(6-isopropoxypyrazin-2-yl)phenyl)acetamide 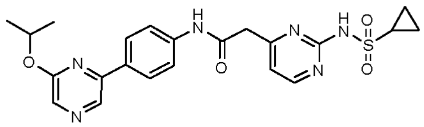 | **Ref P134** | 2-(2-(cyclopropanesulfonamido)-6-methylpyrimidin-4-yl)-N-(4-(6-ethoxypyrazin-2-yl)phenyl)-2-methylpropanamide 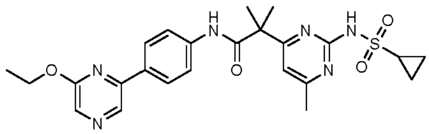 |
| **Ref P22** | 2-(2-(cyclobutanesulfonamido)pyrimidin-4-yl)-N-(5-(6-ethoxypyrazin-2-yl)pyridin-2-yl)-2-methylpropanamide 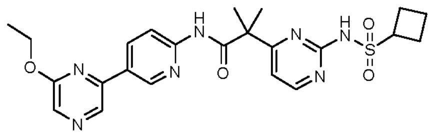 | **Ref P135** | 2-(2-(cyclopropanesulfonamido)-6-(trifluoromethyl)pyrimidin-4-yl)-N-(4-(6-ethoxypyrazin-2-yl)phenyl)-2-methylpropanamide 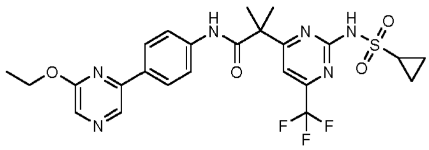 |
| **Ref P23** | 2-(2-(cyclobutanesulfonamido)pyrimidin-4-yl)-N-(2-fluoro-4-(6-isopropoxypyrazin-2-yl)phenyl)-2-methylpropanamide 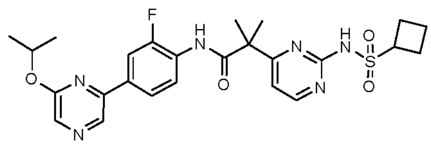 | **Ref P127** | 2-(2-(Cyclopropanesulfonamido)-6-methoxypyrimidin-4-yl)-2-methyl-N-(4-(pyridin-3-yl)phenyl)propanamide 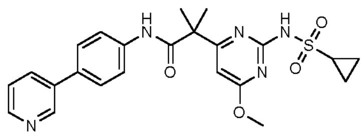 |
| **Ref P24** | 2-(2-(cyclobutanesulfonamido)pyrimidin-4-yl)-N-(4-(6-ethoxypyrazin-2-yl)-2-methylphenyl)-2-methylpropanamide 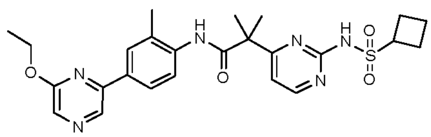 | **Ref P136** | 1-(2-(cyclopropanesulfonamido)pyrimidin-4-yl)-N-(5-(6-ethoxypyrazin-2-yl)pyridin-2-yl)cyclopentane-1-carboxamide 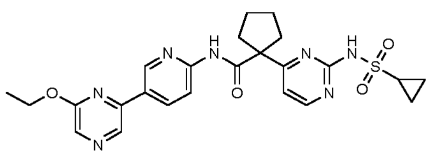 |
| **Ref P25** | 2-(2-(cyclobutanesulfonamido)pyrimidin-4-yl)-N-(4-(6-methoxypyrazin-2-yl)phenyl)-2-methylpropanamide 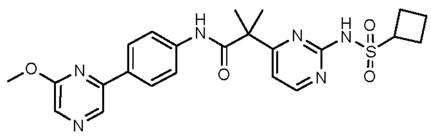 | **Ref P137** | 4-(2-(cyclopropanesulfonamido)pyrimidin-4-yl)-N-(4-(6-ethoxypyrazin-2-yl)phenyl)tetrahydro-2H-pyran-4-carboxamide 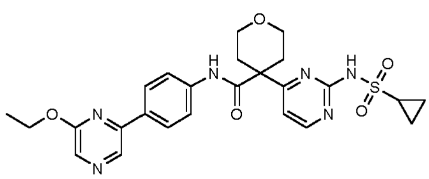 |

(continued)

| P | Name/Structure (All examples containing chiral centres are racemates unless stated) | P | Name/Structure (All examples containing chiral centres are racemates unless stated) |
|---|---|---|---|
| **Ref P26** | 2-(2-(cyclobutanesulfonamido)pyrimidin-4-yl)-N-(4-(6-ethoxypyrazin-2-yl)phenyl)-2-methylpropanamide 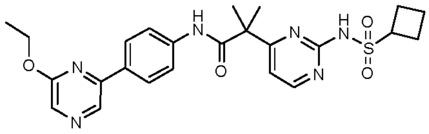 | **Ref P138** | N-(5-(6-ethoxypyrazin-2-yl)pyridin-2-yl)-4-(2-(methylsulfonamido)pyrimidin-4-yl) piperidine-4-carboxamide 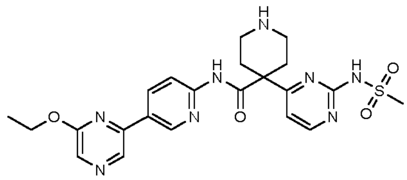 |
| **Ref P27** | 2-(2-(cyclopropanesulfonamido)pyrimidin-4-yl)-N-(5-(6-ethoxypyrazin-2-yl)-3-fluoropyridin-2-yl)-2-methylpropanamide 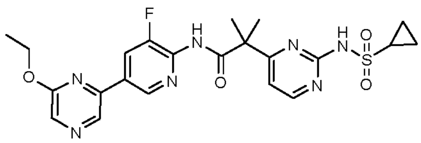 | **Ref P139** | *tert-butyl* 4-(2-(cyclopropanesulfonamido) pyrimidin-4-yl)-4-((5-(6-ethoxypyrazin-2-yl) pyridin-2-yl)carbamoyl)piperidine-1-carboxylate 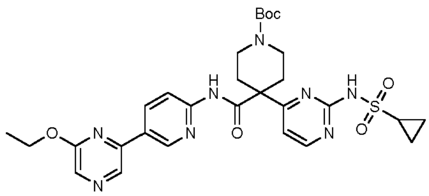 |
| **Ref P28** | 2-(2-(cyclopropanesulfonamido)pyrimidin-4-yl)-N-(5'-ethoxy-[3,3'-bipyridin]-6-yl)-2-methylpropanamide 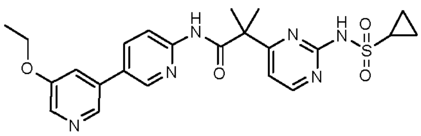 | **Ref P140** | 4-(2-(cyclopropanesulfonamido)pyrimidin-4-yl)-N-(5-(6-ethoxypyrazin-2-yl)pyridin-2-yl)piperidine-4-carboxamide 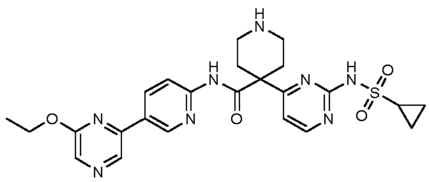 |
| **Ref P29** | N-([3,3'-bipyridin]-6-yl)-2-(2-(cyclopropanesulfonamido)pyrimidin-4-yl)-2-methylpropanamide 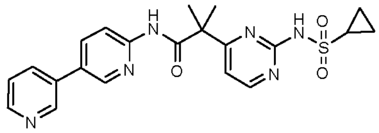 | **Ref P141** | *tert-butyl* 3-(2-(cyclopropanesulfonamido) pyrimidin-4-yl)-3-((5-(6-ethoxypyrazin-2-yl) pyridin-2-yl)carbamoyl)azetidine-1-carboxylate 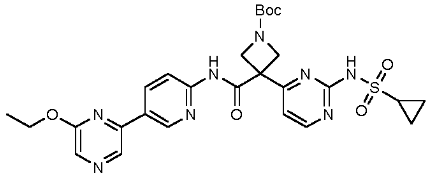 |
| **Ref P30** | 2-(2-(cyclopropanesulfonamido)pyrimidin-4-yl)-2-methyl-N-(5-(6-(trifluoromethyl)pyrazin-2-yl)pyridin-2-yl)propanamide 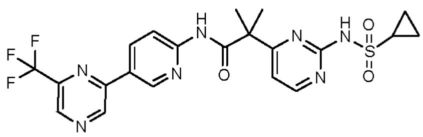 | **Ref P142** | *tert-butyl* 4-((5-(6-ethoxypyrazin-2-yl)pyridin-2-yl)carbamoyl)-4-(2-(methylsulfonamido)pyrimidin-4-yl)piperidine-1-carboxylate 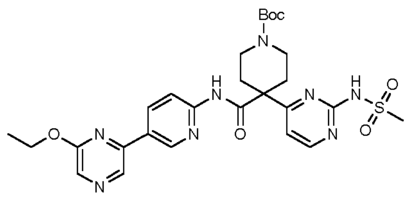 |

(continued)

| P | Name/Structure (All examples containing chiral centres are racemates unless stated) | P | Name/Structure (All examples containing chiral centres are racemates unless stated) |
|---|---|---|---|
| **Ref P31** | 2-(2-(cyclopropanesulfonamido)pyrimidin-4-yl)-N-(5-(6-ethoxypyrazin-2-yl)pyridin-2-yl)-2-methylpropanamide 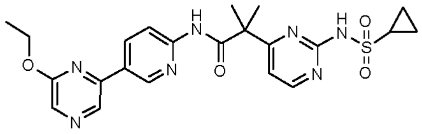 | **Ref P143** | 4-(2-(cyclopropanesulfonamido)pyrimidin-4-yl)-N-(4-(6-ethoxypyrazin-2-yl)-2-fluorophenyl)tetrahydro-2H-pyran-4-carboxamide 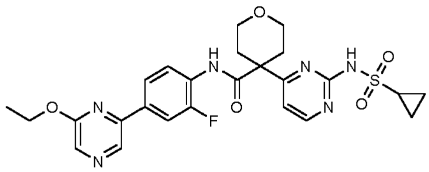 |
| **Ref P32** | 2-(2-(cyclopropanesulfonamido)pyrimidin-4-yl)-N-(5-(6-cyclopropoxypyrazin-2-yl)pyridin-2-yl)-2-methylpropanamide 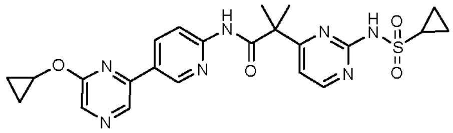 | **Ref P144** | 2-(2-(cyclopropanesulfonamido)pyrimidin-4-yl)-N-(5-(6-ethoxypyrazin-2-yl)-3-fluoropyridin-2-yl)-4-methoxybutanamide 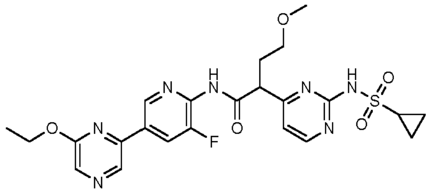 |
| **Ref P33** | N-(2-chloro-4-(6-ethoxypyrazin-2-yl)phenyl)-2-(2-(cyclopropanesulfonamido)pyrimidin-4-yl)-2-methylpropanamide 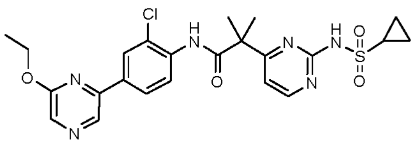 | **Ref P145** | 2-(2-(cyclopropanesulfonamido)pyrimidin-4-yl)-N-(5-(6-ethoxypyrazin-2-yl)pyridin-2-yl)-4-methoxybutanamide 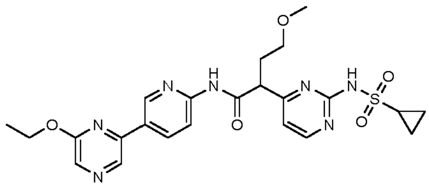 |
| **Ref P34** | N-(2-cyano-4-(6-ethoxypyrazin-2-yl)phenyl)-2-(2-(cyclopropanesulfonamido)pyrimidin-4-yl)-2-methylpropanamide 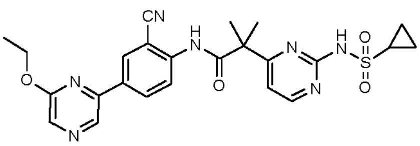 | **Ref P146** | 2-(2-(cyclopropanesulfonamido)pyrimidin-4-yl)-N-(4-(6-ethoxypyrazin-2-yl)-2-fluorophenyl)-4-methoxybutanamide 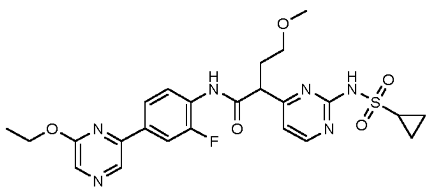 |
| **Ref P35** | 2-(2-(cyclopropanesulfonamido)pyrimidin-4-yl)-N-(2-fluoro-4-(5-isopropoxypyridin-3-yl)phenyl)-2-methylpropanamide 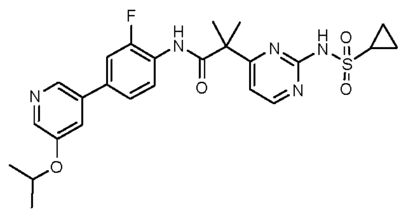 | **Ref P147** | N-(5-(6-ethoxypyrazin-2-yl)pyridin-2-yl)-4-methoxy-2-methyl-2-(2-(methylsulfonamido)pyrimidin-4-yl)butanamide 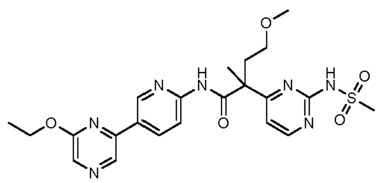 |
| **Ref P36** | 2-(2-(cyclopropanesulfonamido)pyrimidin-4-yl)-N-(2-fluoro-4-(pyridin-3-yl)phenyl)-2-methylpropanamide | **Ref P148** | N-(5'-chloro-[3,3'-bipyridin]-6-yl)-2-(2-(cyclopropanesulfonamido)pyrimidin-4-yl)butanamide |

(continued)

| P | Name/Structure (All examples containing chiral centres are racemates unless stated) | P | Name/Structure (All examples containing chiral centres are racemates unless stated) |
|---|---|---|---|
| | 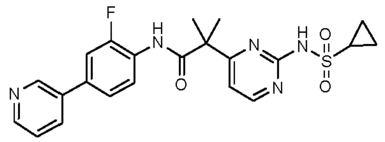 | | 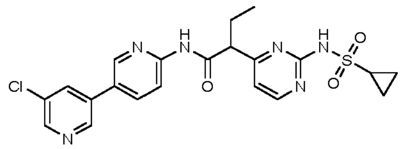 |
| **Ref P37** | 2-(2-(cyclopropanesulfonamido)pyrimidin-4-yl)-N-(2-fluoro-4-(6-(trifluoromethyl)pyrazin-2-yl)phenyl)-2-methylpropanamide 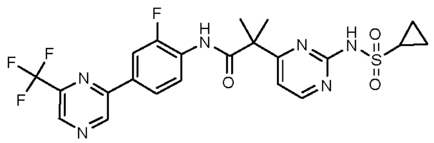 | **Ref P149** | N-(5'-chloro-[3,3'-bipyridin]-6-yl)-2-(2-(cyclopropanesulfonamido)pyrimidin-4-yl)-2-fluorobutanamide 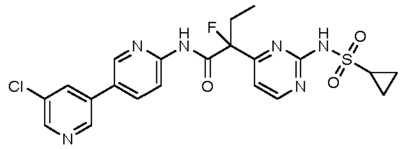 |
| **Ref P38** | 2-(2-(cyclopropanesulfonamido)pyrimidin-4-yl)-N-(4-(6-ethoxypyrazin-2-yl)-2-fluorophenyl)-2-methylpropanamide 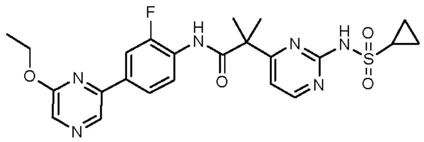 | **Ref P150** | 2-(2-(cyclopropanesulfonamido)pyrimidin-4-yl)-N-(5-(6-cyclopropylpyrazin-2-yl)pyridin-2-yl)-2-fluorobutanamide 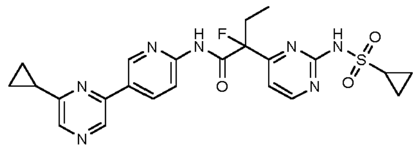 |
| **Ref P39** | 2-(2-(cyclopropanesulfonamido)pyrimidin-4-yl)-N-(2-fluoro-4-(6-isopropoxypyrazin-2-yl)phenyl)-2-methylpropanamide 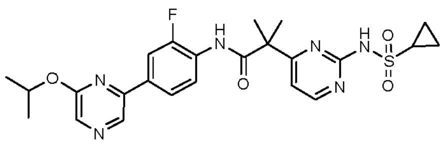 | **Ref P151** | N-(5-(6-ethoxypyrazin-2-yl)pyridin-2-yl)-2-fluoro-2-(2-(methylsulfonamido)pyrimidin-4-yl)butanamide 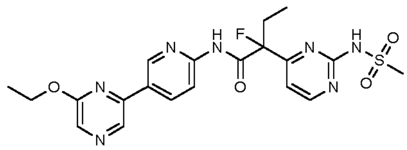 |
| **Ref P40** | 2-(2-(cyclopropanesulfonamido)pyrimidin-4-yl)-N-(4-(6-ethoxypyrazin-2-yl)-2-fluoro-5-methylphenyl)-2-methylpropanamide 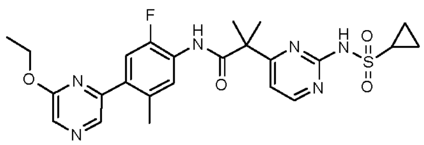 | **Ref P155** | 2-(2-(cyclopropanesulfonamido)pyrimidin-4-yl)-N-(5-(6-ethoxypyrazin-2-yl)-3-methylpyridin-2-yl)butanamide 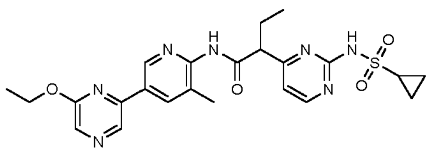 |
| **Ref P41** | 2-(2-(cyclopropanesulfonamido)pyrimidin-4-yl)-N-(4-(6-ethoxypyrazin-2-yl)-2,6-difluorophenyl)-2-methylpropanamide 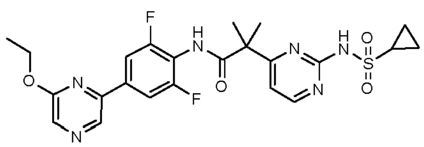 | **Ref P156** | 2-(2-(cyclopropanesulfonamido)pyrimidin-4-yl)-N-(5-(6-cyclopropylpyrazin-2-yl)pyridin-2-yl)butanamide 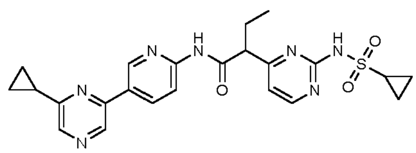 |

(continued)

| P | Name/Structure (All examples containing chiral centres are racemates unless stated) | P | Name/Structure (All examples containing chiral centres are racemates unless stated) |
|---|---|---|---|
| **Ref P42** | 2-(2-(cyclopropanesulfonamido)pyrimidin-4-yl)-N-(2-fluoro-4-(pyrazin-2-yl)phenyl)-2-methylpropanamide 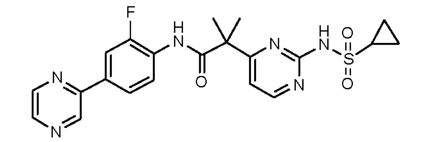 | **Ref P157** | 2-(2-(cyclopropanesulfonamido)pyrimidin-4-yl)-N-(5-(6-(2,2,2-trifluoroethoxy)pyrazin-2-yl)pyridin-2-yl)butanamide 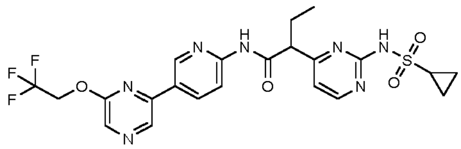 |
| **Ref P43** | 2-(2-(cyclopropanesulfonamido)pyrimidin-4-yl)-2-methyl-N-(2-methyl-4-(6-(trifluoromethyl) pyrazin-2-yl)phenyl)propanamide 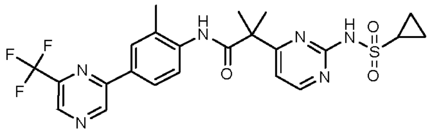 | **Ref P158** | 2-(2-(cyclopropanesulfonamido)pyrimidin-4-yl)-N-(3-fluoro-5-(6-methoxypyrazin-2-yl)pyridin-2-yl)butanamide 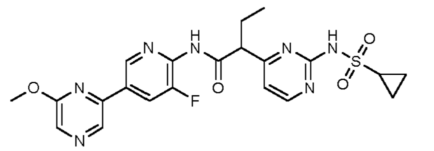 |
| **Ref P44** | 2-(2-(cyclopropanesulfonamido)pyrimidin-4-yl)-N-(4-(6-ethoxypyrazin-2-yl)-2,3-dimethylphenyl)-2-methylpropanamide 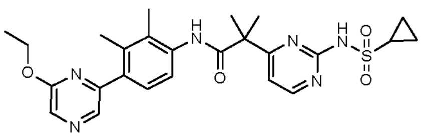 | **Ref P159** | 2-(2-(cyclopropanesulfonamido)pyrimidin-4-yl)-N-(5-(6-methoxypyrazin-2-yl)pyridin-2-yl) butanamide 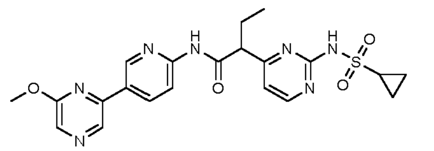 |
| **Ref P45** | 2-(2-(cyclopropanesulfonamido)pyrimidin-4-yl)-N-(4-(6-ethoxypyrazin-2-yl)-5-fluoro-2-methylphenyl)-2-methylpropanamide 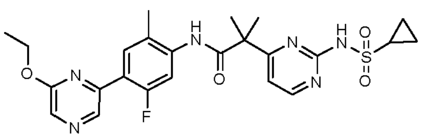 | **Ref P160** | 2-(2-(cyclopropanesulfonamido)pyrimidin-4-yl)-N-(4-(6-cyclopropylpyrazin-2-yl)-2-fluorophenyl)butanamide 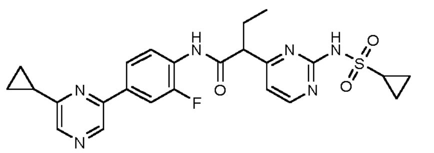 |
| **Ref P46** | 2-(2-(cyclopropanesulfonamido)pyrimidin-4-yl)-N-(4-(6-ethoxypyrazin-2-yl)-2,5-dimethylphenyl)-2-methylpropanamide 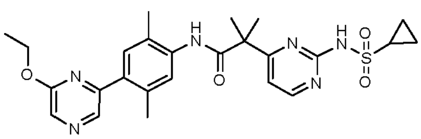 | **Ref P161** | 2-(2-(cyclopropanesulfonamido)pyrimidin-4-yl)-N-(4-(6-ethoxypyrazin-2-yl)-2-methylphenyl)butanamide 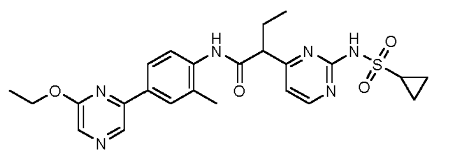 |

(continued)

| P | Name/Structure (All examples containing chiral centres are racemates unless stated) | P | Name/Structure (All examples containing chiral centres are racemates unless stated) |
|---|---|---|---|
| **Ref P47** | 2-(2-(cyclopropanesulfonamido)pyrimidin-4-yl)-N-(4-(6-ethoxypyrazin-2-yl)-2-(trifluoromethoxy)phenyl)-2-methylpropanamide 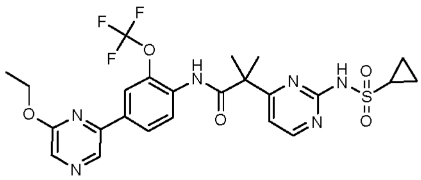 | **Ref P162** | 2-(2-(cyclopropanesulfonamido)pyrimidin-4-yl)-N-(5-(6-ethoxypyrazin-2-yl)-3-fluoropyridin-2-yl)butanamide 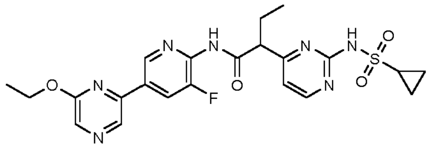 |
| **Ref P48** | 2-(2-(cyclopropanesulfonamido)pyrimidin-4-yl)-N-(4-(6-ethoxypyrazin-2-yl)-5-fluoro-2-methoxyphenyl)-2-methylpropanamide 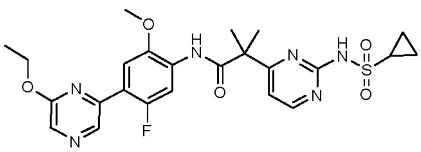 | **Ref P163** | 2-(2-(cyclopropanesulfonamido)pyrimidin-4-yl)-N-(5-(6-ethoxypyrazin-2-yl)pyridin-2-yl)-2-methylbutanamide 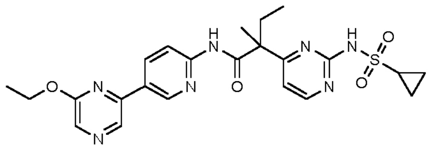 |
| **Ref P49** | 2-(2-(cyclopropanesulfonamido)pyrimidin-4-yl)-N-(4-(6-ethoxypyrazin-2-yl)-2-methoxyphenyl)-2-methylpropanamide 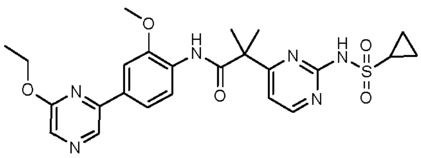 | **Ref P152** | 2-(2-(cyclopropanesulfonamido)pyrimidin-4-yl)-N-(5-(6-ethoxypyrazin-2-yl)pyridin-2-yl)-2-fluoro-3-methylbutanamide 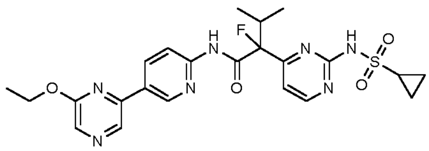 |
| **Ref P50** | 2-(2-(cyclopropanesulfonamido)pyrimidin-4-yl)-2-methyl-N-(4-(pyrimidin-5-yl)phenyl)propanamide 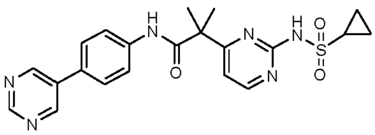 | **Ref P153** | 2-(2-(cyclopropanesulfonamido)pyrimidin-4-yl)-N-(4-(6-ethoxypyrazin-2-yl)-2-fluorophenyl)-3-methylbutanamide 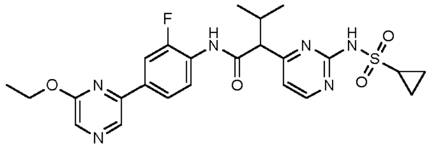 |
| **Ref P51** | N-(4-(5-chloropyridin-3-yl)phenyl)-2-(2-(cyclopropanesulfonamido)pyrimidin-4-yl)-2-methylpropanamide 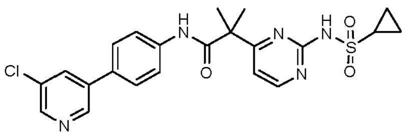 | **Ref P154** | 2-(2-(cyclopropanesulfonamido)pyrimidin-4-yl)-N-(5-(6-ethoxypyrazin-2-yl)pyridin-2-yl)-3-methylbutanamide 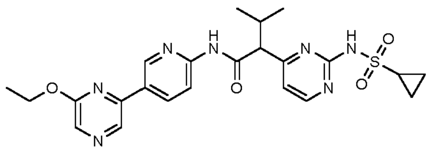 |
| **Ref P52** | N-(4-(5-cyanopyridin-3-yl)phenyl)-2-(2-(cyclopropanesulfonamido)pyrimidin-4-yl)-2-methylpropanamide | **Ref P164** | 2-(2-(cyclopropanesulfonamido)pyrimidin-4-yl)-N-(4-(6-ethoxypyrazin-2-yl)phenyl)-2-methoxyacetamide |

(continued)

| P | Name/Structure (All examples containing chiral centres are racemates unless stated) | P | Name/Structure (All examples containing chiral centres are racemates unless stated) |
|---|---|---|---|
| | 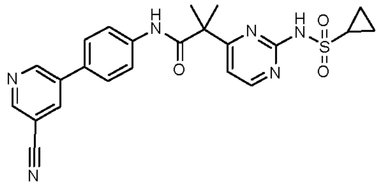 | | 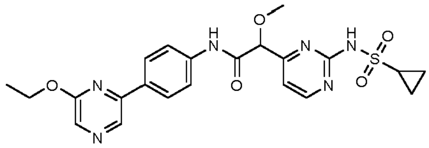 |
| **Ref P53** | 2-(2-(cyclopropanesulfonamido)pyrimidin-4-yl)-N-(4-(5-fluoropyridin-3-yl)phenyl)-2-methylpropanamide 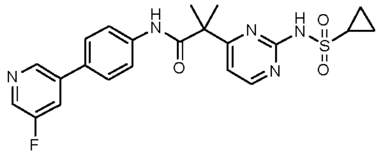 | **Ref P165** | Single enantiomer - stereochemistry unassigned N-(5-(6-ethoxypyrazin-2-yl)pyridin-2-yl)-2-fluoro-2-(2-(methylsulfonamido)pyrimidin-4-yl)butanamide 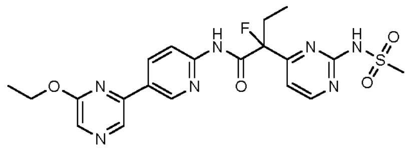 |
| **Ref P54** | 2-(2-(cyclopropanesulfonamido)pyrimidin-4-yl)-2-methyl-N-(4-(5-methylpyridin-3-yl)phenyl)propanamide 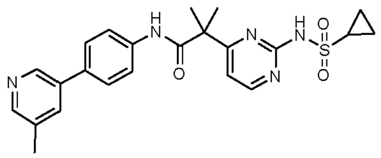 | **Ref P166** | Single enantiomer - stereochemistry unassigned N-(5-(6-ethoxypyrazin-2-yl)pyridin-2-yl)-2-fluoro-2-(2-(methylsulfonamido)pyrimidin-4-yl)butanamide 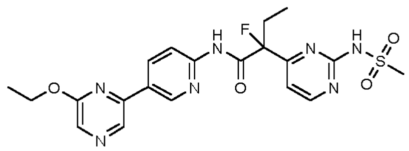 |
| **Ref P55** | 2-(2-(cyclopropanesulfonamido)pyrimidin-4-yl)-N-(4-(5-(difluoromethoxy)pyridin-3-yl)phenyl)-2-methylpropanamide 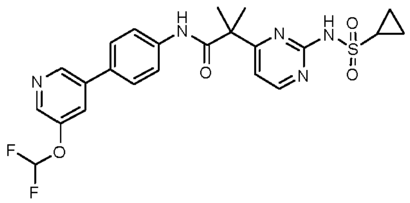 | **Ref P167** | N-(4-(5-chloropyridin-3-yl)phenyl)-2-(6-(cyclopropanesulfonamido)pyridin-2-yl)acetamide 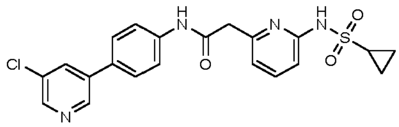 |
| **Ref P56** | 2-(2-(cyclopropanesulfonamido)pyrimidin-4-yl)-N-(4-(5-methoxypyridin-3-yl)phenyl)-2-methylpropanamide 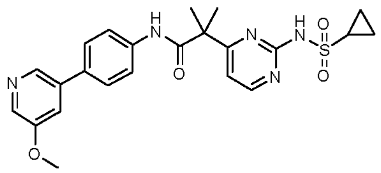 | **Ref P168** | N-(4-(5-cyanopyridin-3-yl)phenyl)-2-(6-(cyclopropanesulfonamido)pyridin-2-yl)acetamide 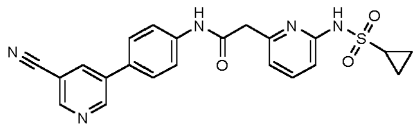 |
| **Ref P57** | 2-(2-(cyclopropanesulfonamido)pyrimidin-4-yl)-N-(4-(5-ethoxypyridin-3-yl)phenyl)-2-methylpropanamide | **Ref P169** | 2-(6-(cyclopropanesulfonamido)pyridin-2-yl)-N-(4-(5-fluoropyridin-3-yl)phenyl)acetamide |

(continued)

| P | Name/Structure (All examples containing chiral centres are racemates unless stated) | P | Name/Structure (All examples containing chiral centres are racemates unless stated) |
|---|---|---|---|
| | 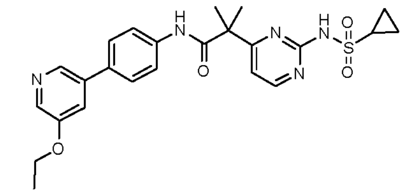 | | 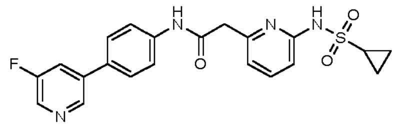 |
| **Ref P58** | 2-(2-(cyclopropanesulfonamido)pyrimidin-4-yl)-N-(4-(5-isopropoxypyridin-3-yl)phenyl)-2-methylpropanamide 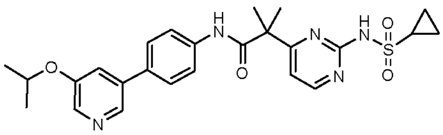 | **Ref P170** | 2-(6-(cyclopropanesulfonamido)pyridin-2-yl)-N-(4-(5-methoxypyridin-3-yl)phenyl)acetamide 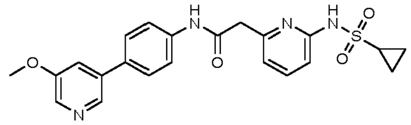 |
| **Ref P59** | 2-(2-(cyclopropanesulfonamido)pyrimidin-4-yl)-2-methyl-N-(4-(pyridin-3-yl)phenyl)propanamide 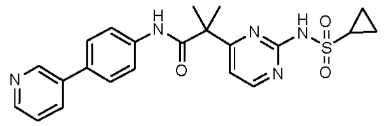 | **Ref P171** | 2-(6-(cyclopropanesulfonamido)pyridin-2-yl)-N-(4-(pyridin-3-yl)phenyl)acetamide 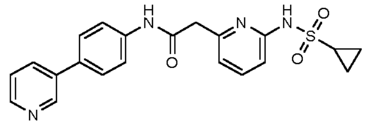 |
| **Ref P60** | 2-(2-(cyclopropanesulfonamido)pyrimidin-4-yl)-2-methyl-N-(3'-(trifluoromethyl)-[1,1'-biphenyl]-4-yl)propanamide 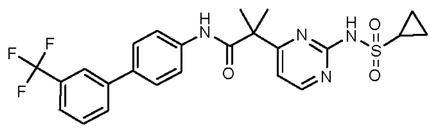 | **Ref P172** | 2-(6-(cyclopropanesulfonamido)pyridin-2-yl)-N-(4-(6-(trifluoromethyl)pyrazin-2-yl)phenyl)acetamide 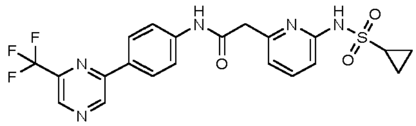 |
| **Ref P61** | N-(3'-chloro-[1,1'-biphenyl]-4-yl)-2-(2-(cyclopropanesulfonamido)pyrimidin-4-yl)-2-methylpropanamide 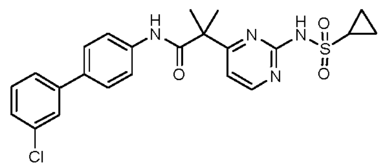 | **Ref P173** | 2-(6-(cyclopropanesulfonamido)pyridin-2-yl)-N-(4-(6-methoxypyrazin-2-yl)phenyl)acetamide 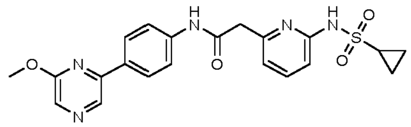 |
| **Ref P62** | N-(3'-cyano-[1,1'-biphenyl]-4-yl)-2-(2-(cyclopropanesulfonamido)pyrimidin-4-yl)-2-methylpropanamide 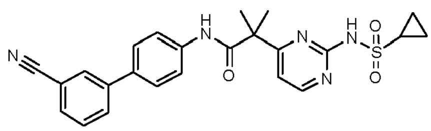 | **Ref P174** | 2-(6-(cyclopropanesulfonamido)pyridin-2-yl)-N-(4-(pyrazin-2-yl)phenyl)acetamide 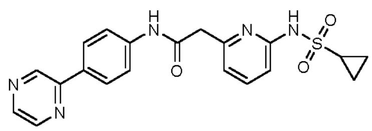 |
| **Ref P63** | 2-(2-(cyclopropanesulfonamido)pyrimidin-4-yl)-N-(3'-ethoxy-[1,1'-biphenyl]-4-yl)-2-methylpropanamide | **Ref P175** | N-([3,3'-bipyridin]-6-yl)-2-(6-(cyclopropanesulfonamido)pyridin-2-yl)-2-methylpropanamide |

(continued)

| P | Name/Structure (All examples containing chiral centres are racemates unless stated) | P | Name/Structure (All examples containing chiral centres are racemates unless stated) |
|---|---|---|---|
| | 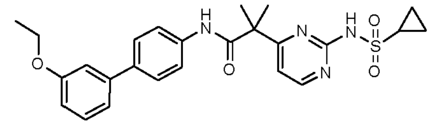 | | 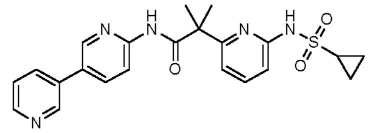 |
| **Ref P64** | 2-(2-(cyclopropanesulfonamido)pyrimidin-4-yl)-2-methyl-N-(4-(6-(trifluoromethyl)pyrazin-2-yl)phenyl)propanamide 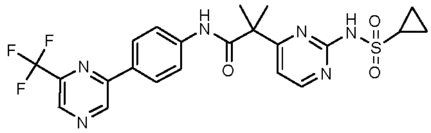 | **Ref P176** | N-(4-(5-chloropyridin-3-yl)phenyl)-2-(6-(cyclopropanesulfonamido)pyridin-2-yl)-2-methylpropanamide 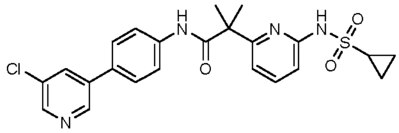 |
| **Ref P65** | 2-(2-(cyclopropanesulfonamido)pyrimidin-4-yl)-N-(4-(6-ethoxypyrazin-2-yl)phenyl)-2-methylpropanamide 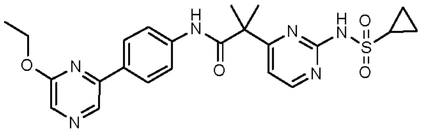 | **Ref P177** | 2-(6-(cyclopropanesulfonamido)pyridin-2-yl)-N-(4-(5-fluoropyridin-3-yl)phenyl)-2-methylpropanamide 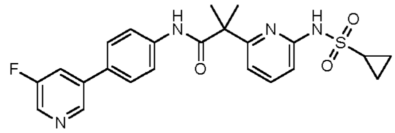 |
| **Ref P66** | 2-(2-(cyclopropanesulfonamido)pyrimidin-4-yl)-N-(4-(6-cyclopropoxypyrazin-2-yl)phenyl)-2-methylpropanamide 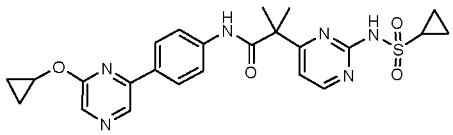 | **Ref P178** | 2-(6-(cyclopropanesulfonamido)pyridin-2-yl)-N-(4-(5-ethoxypyridin-3-yl)phenyl)-2-methylpropanamide 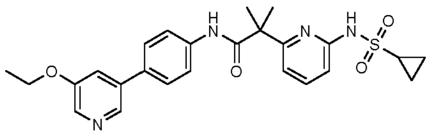 |
| **Ref P67** | 2-(2-(cyclopropanesulfonamido)pyrimidin-4-yl)-N-(4-(6-isopropoxypyrazin-2-yl)phenyl)-2-methylpropanamide 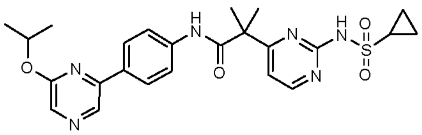 | **Ref P179** | 2-(6-(cyclopropanesulfonamido)pyridin-2-yl)-2-methyl-N-(4-(pyridin-3-yl)phenyl)propanamide 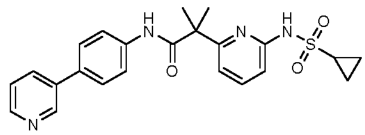 |
| **Ref P68** | 2-(2-(cyclopropanesulfonamido)-5-fluoropyrimidin-4-yl)-N-(4-(6-ethoxypyrazin-2-yl)phenyl)-2-methylpropanamide 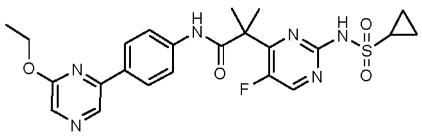 | **Ref P180** | 2-(6-(cyclopropanesulfonamido)pyridin-2-yl)-N-(2-fluoro-4-(pyrazin-2-yl)phenyl)-2-methylpropanamide 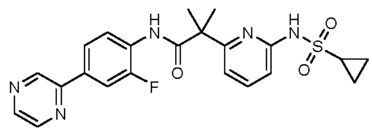 |
| **Ref P69** | N-(4-(6-ethoxypyrazin-2-yl)phenyl)-2-methyl-2-(2-((1-methylcyclopropane)-1-sulfonamido)pyrimidin-4-yl)propanamide | **Ref P181** | 2-(6-(cyclopropanesulfonamido)pyridin-2-yl)-2-methyl-N-(4-(6-(trifluoromethyl)pyrazin-2-yl)phenyl)propanamide |

(continued)

| P | Name/Structure (All examples containing chiral centres are racemates unless stated) | P | Name/Structure (All examples containing chiral centres are racemates unless stated) |
|---|---|---|---|
| | 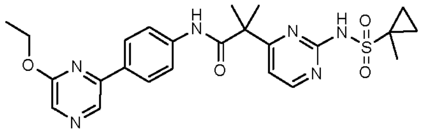 | | 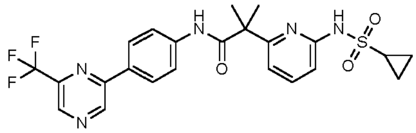 |
| **Ref P70** | 2-(2-(cyclopropanesulfonamido)-5-methylpyrimidin-4-yl)-N-(4-(6-ethoxypyrazin-2-yl)phenyl)-2-methylpropanamide 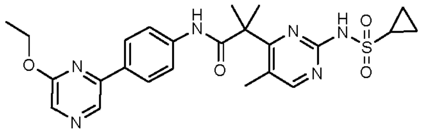 | **Ref P182** | N-(4-(6-chloropyrazin-2-yl)phenyl)-2-(6-(cyclopropanesulfonamido)pyridin-2-yl)-2-methylpropanamide 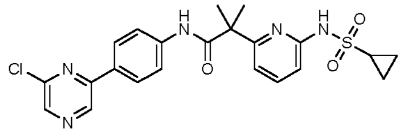 |
| **Ref P71** | 2-(2-(cyclopropanesulfonamido)pyrimidin-4-yl)-2-methyl-N-(4-(pyrazin-2-yl)phenyl)propanamide 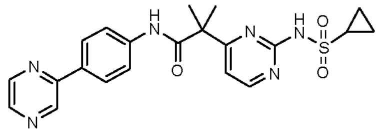 | **Ref P183** | 2-(6-(cyclopropanesulfonamido)pyridin-2-yl)-N-(4-(6-ethoxypyrazin-2-yl)phenyl)-2-methylpropanamide 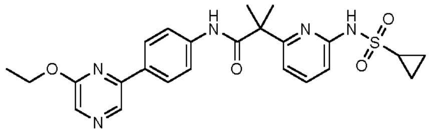 |
| **Ref P72** | N-(4-(6-ethoxypyrazin-2-yl)-2-fluorophenyl)-2-(2-(ethylsulfonamido)pyrimidin-4-yl)-2-methylpropanamide 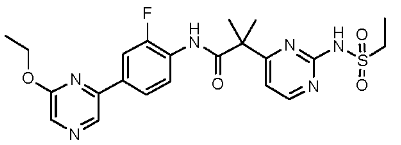 | **Ref P184** | 2-(6-(cyclopropanesulfonamido)pyridin-2-yl)-N-(4-(6-methoxypyrazin-2-yl)phenyl)-2-methylpropanamide 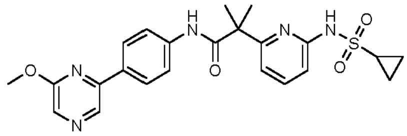 |
| **Ref P73** | 2-(2-(ethylsulfonamido)pyrimidin-4-yl)-2-methyl-N-(4-(6-(trifluoromethyl)pyrazin-2-yl)phenyl)propanamide 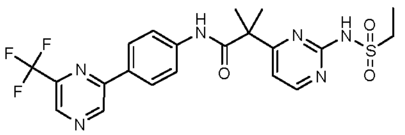 | **Ref P185** | 2-(6-(cyclopropanesulfonamido)pyridin-2-yl)-2-methyl-N-(4-(pyrazin-2-yl)phenyl)propanamide 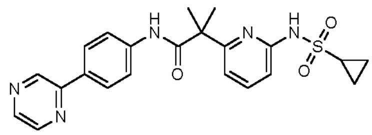 |
| **Ref P74** | N-(4-(6-ethoxypyrazin-2-yl)phenyl)-2-(2-(ethylsulfonamido)pyrimidin-4-yl)-2-methylpropanamide 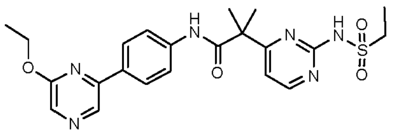 | **Ref P186** | 4-(6-(cyclopropanesulfonamido)pyridin-2-yl)-N-(5-(6-ethoxypyrazin-2-yl)pyridin-2-yl)tetrahydro-2H-pyran-4-carboxamide 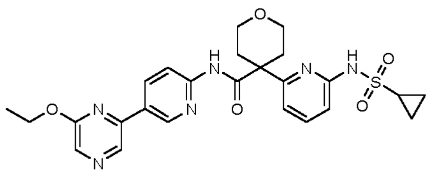 |
| **Ref P75** | N-(5-(6-ethoxypyrazin-2-yl)-3-fluoropyridin-2-yl)-2-methyl-2-(2-(methylsulfonamido)pyrimidin-4-yl)propanamide | **Ref P187** | 2-(6-(cyclopropanesulfonamido)pyridin-2-yl)-N-(5-(6-(trifluoromethyl)pyrazin-2-yl)pyridin-2-yl)butanamide |

(continued)

| P | Name/Structure (All examples containing chiral centres are racemates unless stated) | P | Name/Structure (All examples containing chiral centres are racemates unless stated) |
|---|---|---|---|
| | 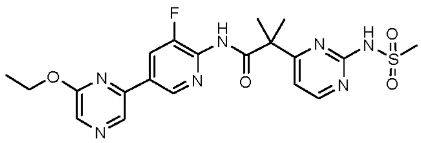 | | 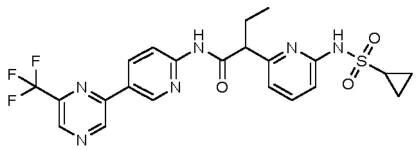 |
| **Ref P76** | N-(5-(6-ethoxypyrazin-2-yl)pyridin-2-yl)-2-methyl-2-(2-(methylsulfonamido)pyrimidin-4-yl)propanamide 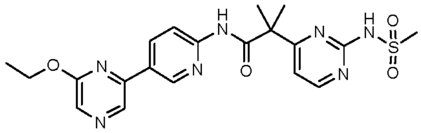 | **Ref P188** | 2-(6-(cyclopropanesulfonamido)pyridin-2-yl)-N-(5-(6-ethoxypyrazin-2-yl)pyridin-2-yl) butanamide 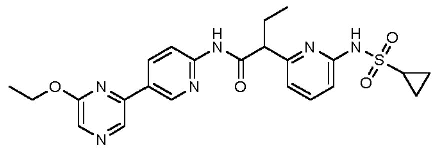 |
| **Ref P77** | N-(2-fluoro-4-(5-isopropoxypyridin-3-yl)phenyl)-2-methyl-2-(2-(methylsulfonamido)pyrimidin-4-yl)propanamide 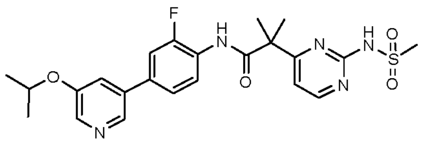 | **Ref P189** | N-(4-(5-chloropyridin-3-yl)phenyl)-2-(6-(cyclopropanesulfonamido)pyridin-2-yl)butanamide 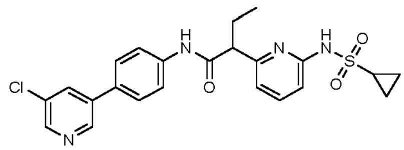 |
| **Ref P78** | N-(2-fluoro-4-(6-isopropoxypyrazin-2-yl)phenyl)-2-methyl-2-(2-(methylsulfonamido)pyrimidin-4-yl)propanamide 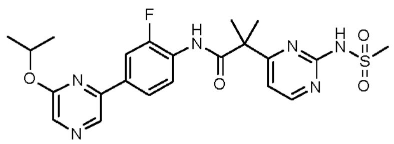 | **Ref P190** | 2-(6-(cyclopropanesulfonamido)pyridin-2-yl)-N-(4-(6-ethoxypyrazin-2-yl)-2-fluorophenyl)butanamide 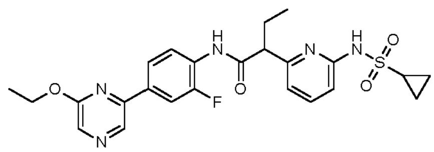 |
| **Ref P79** | 2-methyl-N-(2-methyl-4-(6-(trifluoromethyl) pyrazin-2-yl)phenyl)-2-(2-(methylsulfonamido) pyrimidin-4-yl)propanamide 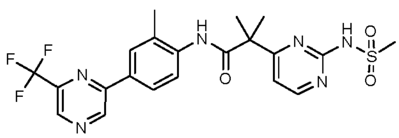 | **Ref P191** | 2-(6-(cyclopropanesulfonamido)pyridin-2-yl)-N-(4-(6-ethoxypyrazin-2-yl)phenyl)butanamide 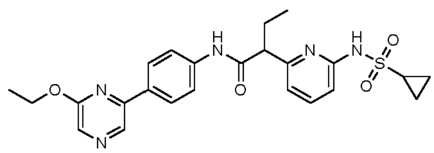 |
| **Ref P80** | 2-methyl-2-(2-(methylsulfonamido)pyrimidin-4-yl)-N-(4-(6-(trifluoromethyl)pyrazin-2-yl) phenyl)propanamide 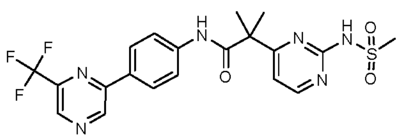 | **Ref P192** | 2-(6-(cyclopropanesulfonamido)pyrazin-2-yl)-N-(4-(pyridin-3-yl)phenyl)acetamide 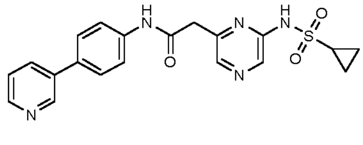 |

(continued)

| P | Name/Structure (All examples containing chiral centres are racemates unless stated) | P | Name/Structure (All examples containing chiral centres are racemates unless stated) |
|---|---|---|---|
| **Ref P81** | N-(4-(6-ethoxypyrazin-2-yl)phenyl)-2-methyl-2-(2-(methylsulfonamido)pyrimidin-4-yl)propanamide 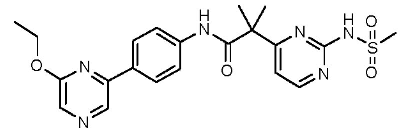 | **Ref P193** | 2-(6-(ethylsulfonamido)pyrazin-2-yl)-N-(4-(pyridin-3-yl)phenyl)acetamide 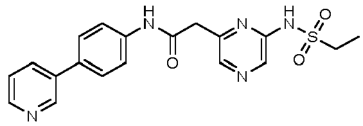 |
| **Ref P82** | 2-(2-((1,1-dimethylethyl)sulfonamido)pyrimidin-4-yl)-N-(4-(6-ethoxypyrazin-2-yl)phenyl)-2-methylpropanamide 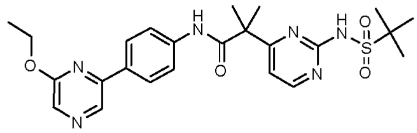 | **Ref P194** | 2-(6-(methylsulfonamido)pyrazin-2-yl)-N-(4-(pyridin-3-yl)phenyl)acetamide 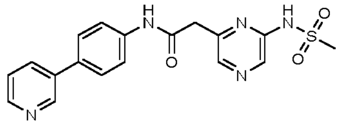 |
| **Ref P83** | 1-(2-(cyclopropanesulfonamido)pyrimidin-4-yl)-N-(4-(6-ethoxypyrazin-2-yl)phenyl)cyclopropanecarboxamide 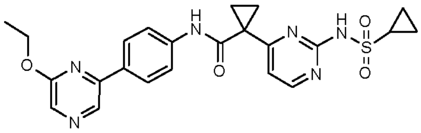 | **Ref P195** | 2-(6-(cyclopropanesulfonamido)pyrazin-2-yl)-N-(5-(6-ethoxypyrazin-2-yl)pyridin-2-yl)-2-methylpropanamide 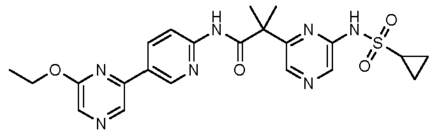 |
| **Ref P84** | 2-(2-(cyclopropanesulfonamido)pyrimidin-4-yl)-N-(5'-(trifluoromethyl)-[3,3'-bipyridin]-6-yl)butanamide 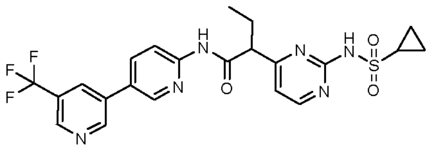 | **Ref P196** | 2-(6-(cyclopropanesulfonamido)pyrazin-2-yl)-N-(4-(6-ethoxypyrazin-2-yl)phenyl)-2-methylpropanamide 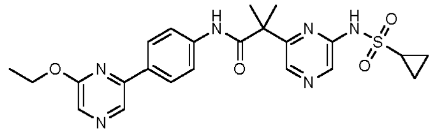 |
| **Ref P85** | 2-(2-(cyclopropanesulfonamido)pyrimidin-4-yl)-N-(5'-(2,2,2-trifluoroethoxy)-[3,3'-bipyridin]-6-yl)butanamide 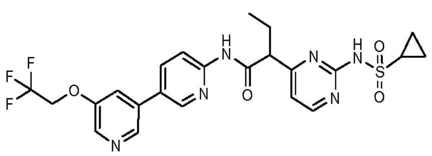 | **Ref P197** | 4-(6-(cyclopropanesulfonamido)pyrazin-2-yl)-N-(5-(6-ethoxypyrazin-2-yl)pyridin-2-yl)tetrahydro-2H-pyran-4-carboxamide 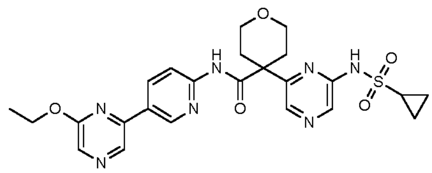 |
| **Ref P86** | N-([3,3'-bipyridin]-6-yl)-2-(2-(cyclopropanesulfonamido)pyrimidin-4-yl)butanamide 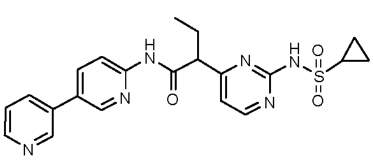 | **Ref P198** | 2-(6-(cyclopropanesulfonamido)pyrazin-2-yl)-N-(5-(6-ethoxypyrazin-2-yl)pyridin-2-yl)-4-methoxy-2-methylbutanamide 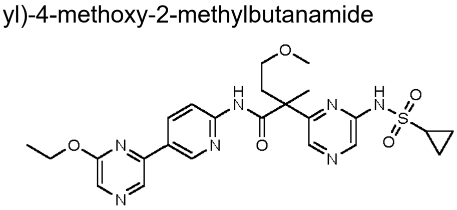 |

(continued)

| P | Name/Structure (All examples containing chiral centres are racemates unless stated) | P | Name/Structure (All examples containing chiral centres are racemates unless stated) |
|---|---|---|---|
| **Ref P87** | 2-(2-(cyclopropanesulfonamido)pyrimidin-4-yl)-N-(5-(6-(trifluoromethyl)pyrazin-2-yl)pyridin-2-yl)butanamide 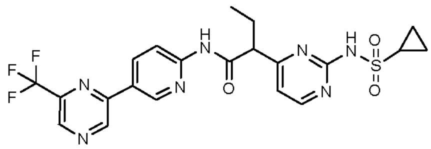 | **Ref P199** | N-(5-(6-ethoxypyrazin-2-yl)pyridin-2-yl)-4-methoxy-2-methyl-2-(6-(methylsulfonamido)pyrazin-2-yl)butanamide 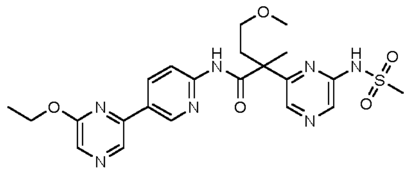 |
| **Ref P88** | 2-(2-(cyclopropanesulfonamido)pyrimidin-4-yl)-N-(5-(6-ethoxypyrazin-2-yl)pyridin-2-yl)butanamide 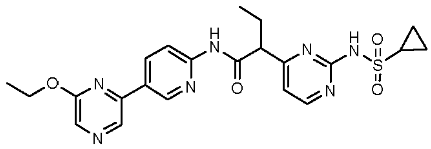 | **Ref P200** | 2-(6-(cyclopropanesulfonamido)pyrazin-2-yl)-N-(5-(6-ethoxypyrazin-2-yl)pyridin-2-yl)-2-fluorobutanamide 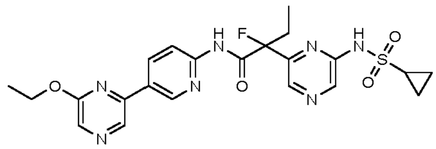 |
| **Ref P89** | 2-(2-(cyclopropanesulfonamido)pyrimidin-4-yl)-N-(5-(6-isopropoxypyrazin-2-yl)pyridin-2-yl)butanamide 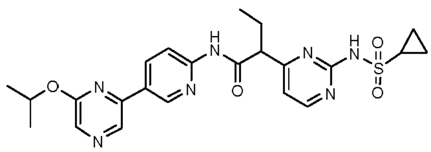 | **Ref P201** | 2-(6-(cyclopropanesulfonamido)pyrazin-2-yl)-N-(5-(6-ethoxypyrazin-2-yl)pyridin-2-yl)butanamide 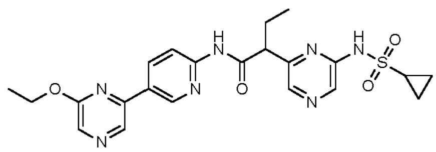 |
| **Ref P90** | N-(4-(5-chloropyridin-3-yl)-2-fluorophenyl)-2-(2-(cyclopropanesulfonamido)pyrimidin-4-yl)butanamide 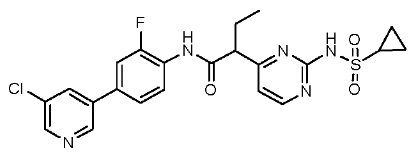 | **Ref P202** | 2-(6-(cyclopropanesulfonamido)pyrazin-2-yl)-N-(4-(6-ethoxypyrazin-2-yl)phenyl)butanamide 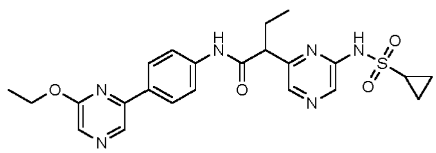 |
| **Ref P91** | 2-(2-(cyclopropanesulfonamido)pyrimidin-4-yl)-N-(2-fluoro-4-(5-(2,2,2-trifluoroethoxy)pyridin-3-yl)phenyl)butanamide 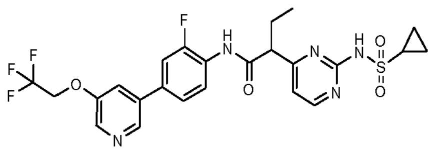 | **Ref P203** | 2-(6-(cyclopropanesulfonamido)pyrazin-2-yl)-N-(5-(6-ethoxypyrazin-2-yl)pyridin-2-yl)-2-methoxyacetamide 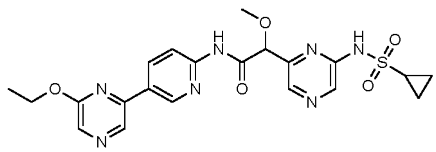 |

(continued)

| P | Name/Structure (All examples containing chiral centres are racemates unless stated) | P | Name/Structure (All examples containing chiral centres are racemates unless stated) |
|---|---|---|---|
| **Ref P92** | 2-(2-(cyclopropanesulfonamido)pyrimidin-4-yl)-N-(2-fluoro-4-(5-isopropoxypyridin-3-yl) phenyl)butanamide 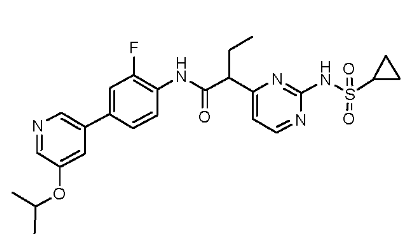 | **Ref P204** | 2-(6-(cyclopropanesulfonamido)pyrazin-2-yl)-N-(4-(6-ethoxypyrazin-2-yl)phenyl)-2-methoxyacetamide 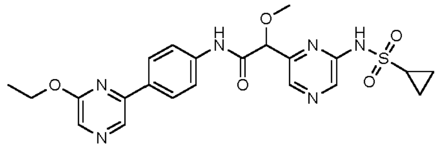 |
| **Ref P93** | 2-(2-(cyclopropanesulfonamido)pyrimidin-4-yl)-N-(2-fluoro-4-(pyridin-3-yl)phenyl)butanamide 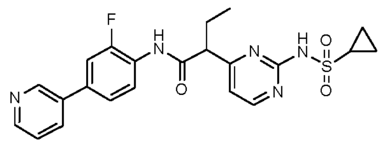 | **Ref P205** | 2-(6-(cyclopropanesulfonamido)pyrazin-2-yl)-N-(5-(6-ethoxypyrazin-2-yl)pyridin-2-yl)-2-methoxypropanamide 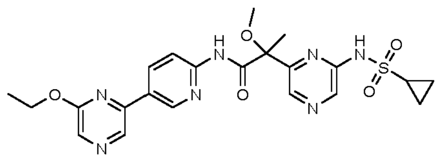 |
| **Ref P94** | 2-(2-(cyclopropanesulfonamido)pyrimidin-4-yl)-N-(2-fluoro-4-(6-(trifluoromethyl)pyrazin-2-yl)phenyl)butanamide 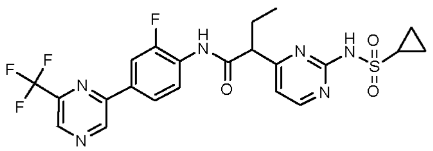 | **Ref P205a** | Single enantiomer - stereochemistry unassigned 2-(6-(cyclopropanesulfonamido)pyrazin-2-yl)-N-(5-(6-ethoxypyrazin-2-yl)pyridin-2-yl)-2-methoxypropanamide 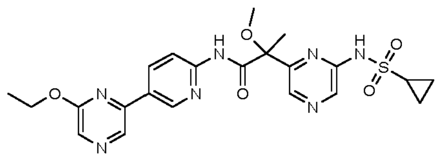 |
| **Ref P95** | 2-(2-(cyclopropanesulfonamido)pyrimidin-4-yl)-N-(2-fluoro-4-(6-methoxypyrazin-2-yl)phenyl)butanamide 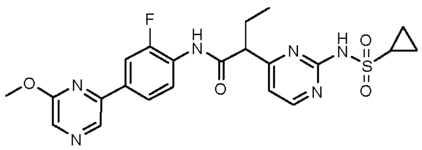 | **Ref P205b** | Single enantiomer - stereochemistry unassigned 2-(6-(cyclopropanesulfonamido)pyrazin-2-yl)-N-(5-(6-ethoxypyrazin-2-yl)pyridin-2-yl)-2-methoxypropanamide 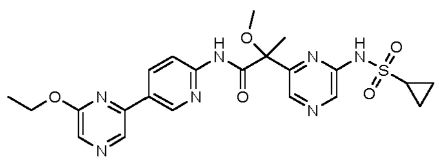 |
| **Ref P96** | 2-(2-(cyclopropanesulfonamido)pyrimidin-4-yl)-N-(4-(6-ethoxypyrazin-2-yl)-2-fluorophenyl)butanamide 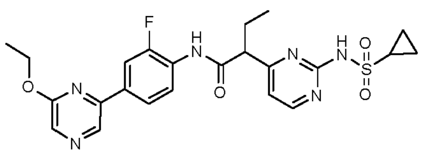 | **Ref P206** | Single enantiomer - stereochemistry unassigned 2-(6-(cyclopropanesulfonamido)pyrazin-2-yl)-N-(5-(6-ethoxypyrazin-2-yl)pyridin-2-yl)-2-fluorobutanamide 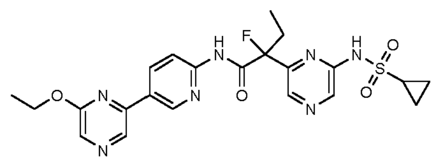 |

(continued)

| P | Name/Structure (All examples containing chiral centres are racemates unless stated) | P | Name/Structure (All examples containing chiral centres are racemates unless stated) |
|---|---|---|---|
| **Ref P97** | 2-(2-(cyclopropanesulfonamido)pyrimidin-4-yl)-N-(2-fluoro-4-(6-isopropoxypyrazin-2-yl)phenyl)butanamide 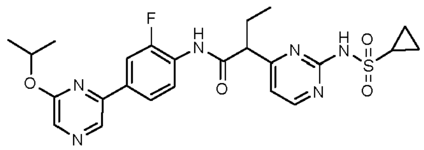 | **Ref P207** | Single enantiomer - stereochemistry unassigned 2-(6-(cyclopropanesulfonamido)pyrazin-2-yl)-N-(5-(6-ethoxypyrazin-2-yl)pyridin-2-yl)-2-fluorobutanamide 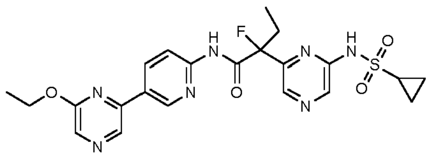 |
| **Ref P98** | 2-(2-(cyclopropanesulfonamido)pyrimidin-4-yl)-N-(2-fluoro-4-(6-(2,2,2-trifluoroethoxy)pyrazin-2-yl)phenyl)butanamide 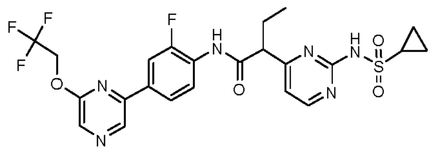 | **Ref P208** | 2-(4-(cyclopropanesulfonamido)pyrimidin-2-yl)-N-(5-(6-ethoxypyrazin-2-yl)pyridin-2-yl)butanamide 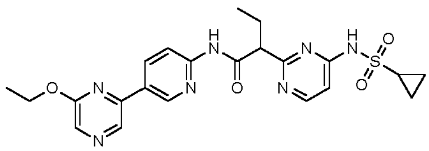 |
| **Ref P99** | N-(4-(5-cyanopyridin-3-yl)phenyl)-2-(2-(cyclopropanesulfonamido)pyrimidin-4-yl)butanamide 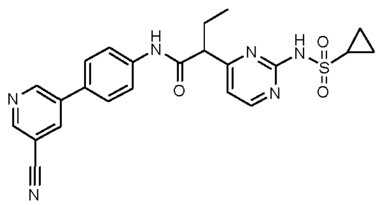 | **Ref P209** | N-(1-(2-(cyclopropanesulfonamido)pyrimidin-4-yl)cyclopropyl)-4-(6-ethoxypyrazin-2-yl)-2-fluorobenzamide 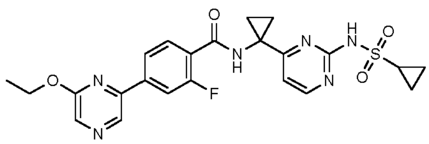 |
| **Ref P100** | 2-(2-(cyclopropanesulfonamido)pyrimidin-4-yl)-N-(4-(5-(2,2,2-trifluoroethoxy)pyridin-3-yl)phenyl)butanamide 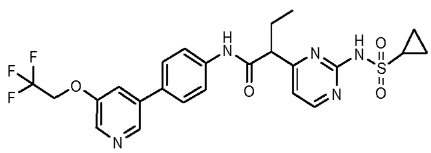 | **Ref P210** | N-(1-(2-(cyclopropanesulfonamido)pyrimidin-4-yl)propyl)-5-(6-ethoxypyrazin-2-yl)picolinamide 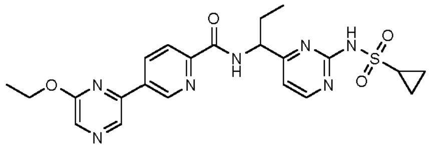 |
| **Ref P101** | 2-(2-(cyclopropanesulfonamido)pyrimidin-4-yl)-N-(4-(5-isopropoxypyridin-3-yl)phenyl)butanamide 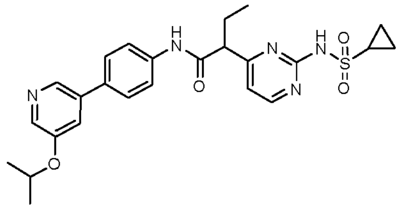 | **Ref P211** | N-(1-(2-(cyclopropanesulfonamido)pyrimidin-4-yl)propyl)-2-fluoro-4-(5-(trifluoromethyl)pyridin-3-yl)benzamide 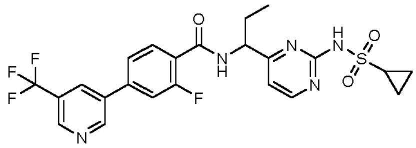 |
| **Ref P102** | 2-(2-(cyclopropanesulfonamido)pyrimidin-4-yl)-N-(4-(pyridin-3-yl)phenyl)butanamide | **Ref P212** | 4-(5-chloropyridin-3-yl)-N-(1-(2-(cyclopropanesulfonamido)pyrimidin-4-yl)propyl)-2-fluorobenzamide |

(continued)

| P | Name/Structure (All examples containing chiral centres are racemates unless stated) | P | Name/Structure (All examples containing chiral centres are racemates unless stated) |
|---|---|---|---|
| | 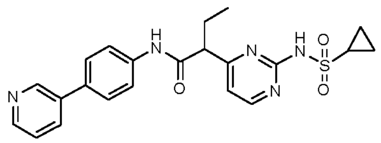 | | 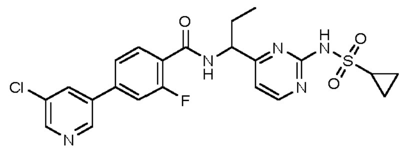 |
| **Ref P103** | 2-(2-(cyclopropanesulfonamido)pyrimidin-4-yl)-N-(4-(6-(trifluoromethyl)pyrazin-2-yl)phenyl)butanamide 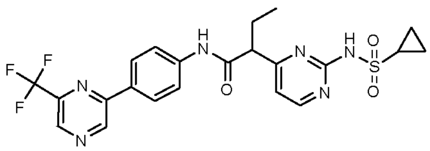 | **Ref P213** | N-(1-(2-(cyclopropanesulfonamido)pyrimidin-4-yl)propyl)-4-(5-(trifluoromethyl)pyridin-3-yl)benzamide 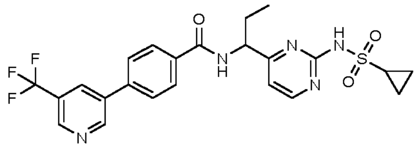 |
| **Ref P104** | N-(4-(6-chloropyrazin-2-yl)phenyl)-2-(2-(cyclopropanesulfonamido)pyrimidin-4-yl)butanamide 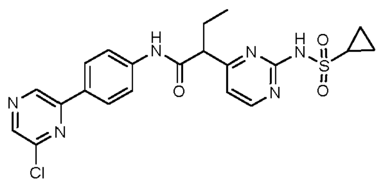 | **Ref P214** | 4-(5-chloropyridin-3-yl)-N-(1-(2-(cyclopropanesulfonamido)pyrimidin-4-yl)propyl)benzamide 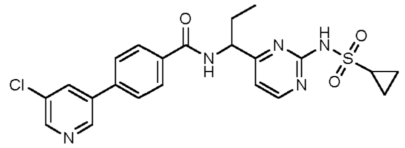 |
| **Ref P105** | 2-(2-(cyclopropanesulfonamido)pyrimidin-4-yl)-N-(4-(6-ethoxypyrazin-2-yl)phenyl)butanamide 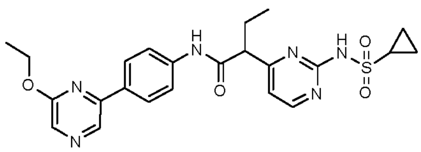 | **Ref P215** | N-(1-(2-(cyclopropanesulfonamido)pyrimidin-4-yl)propyl)-4-(6-ethoxypyrazin-2-yl)-2-(trifluoromethyl)benzamide 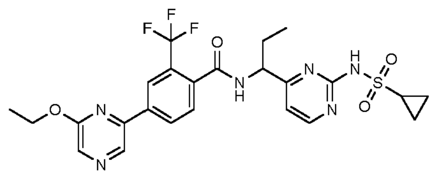 |
| **Ref P106** | 2-(2-(cyclopropanesulfonamido)pyrimidin-4-yl)-N-(4-(6-methoxypyrazin-2-yl)phenyl)butanamide 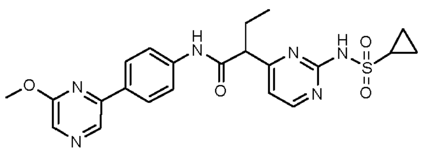 | **Ref P216** | N-(1-(2-(cyclopropanesulfonamido)pyrimidin-4-yl)propyl)-4-(6-ethoxypyrazin-2-yl)-2-fluorobenzamide 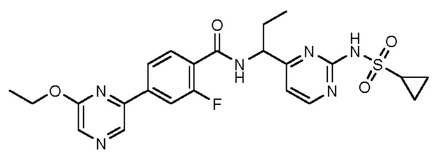 |
| **Ref P107** | 2-(2-(cyclopropanesulfonamido)pyrimidin-4-yl)-N-(4-(6-isopropoxypyrazin-2-yl)phenyl)butanamide 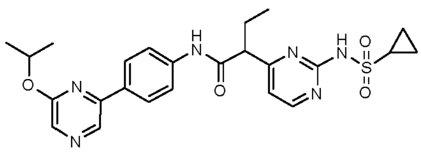 | **Ref P217** | N-(1-(2-(cyclopropanesulfonamido)pyrimidin-4-yl)propyl)-4-(6-(trifluoromethyl)pyrazin-2-yl)benzamide 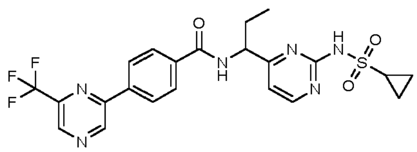 |
| **Ref P108** | 2-(2-(cyclopropanesulfonamido)pyrimidin-4-yl)-N-(4-(6-(2,2,2-trifluoroethoxy)pyrazin-2-yl)phenyl)butanamide | **Ref P218** | N-(1-(2-(cyclopropanesulfonamido)pyrimidin-4-yl)propyl)-4-(6-isopropoxypyrazin-2-yl)benzamide |

(continued)

| P | Name/Structure (All examples containing chiral centres are racemates unless stated) | P | Name/Structure (All examples containing chiral centres are racemates unless stated) |
|---|---|---|---|
| | 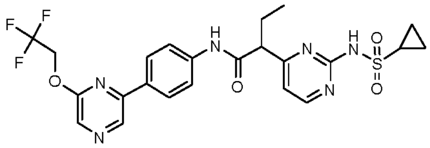 | | 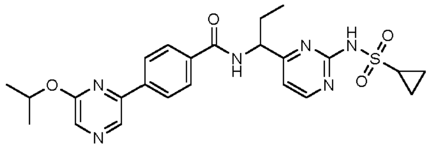 |
| **Ref P109** | 2-(2-(cyclopropanesulfonamido)pyrimidin-4-yl)-N-(4-(pyrazin-2-yl)phenyl)butanamide 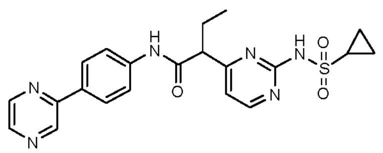 | **Ref P219** | N-(1-(2-(cyclopropanesulfonamido)pyrimidin-4-yl)propyl)-4-(6-ethoxypyrazin-2-yl)benzamide 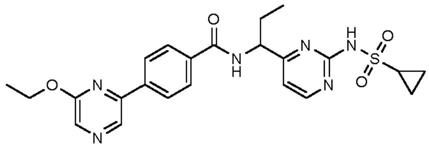 |
| **Ref P110** | 2-(2-(cyclopropanesulfonamido)pyrimidin-4-yl)-N-(4-(6-ethoxypyrazin-2-yl)phenyl)-4-methoxybutanamide 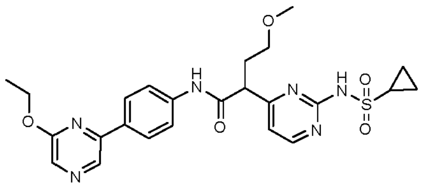 | **Ref P220** | N-(2-(2-(cyclopropanesulfonamido)pyrimidin-4-yl)butan-2-yl)-4-(6-ethoxypyrazin-2-yl)-2-fluorobenzamide 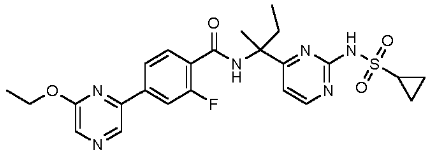 |
| **Ref P111** | 2-(2-(cyclopropanesulfonamido)pyrimidin-4-yl)-N-(4-(pyridin-3-yl)phenyl)propanamide 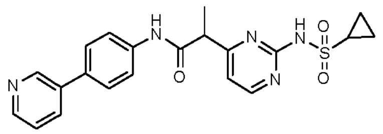 | **Ref P221** | N-(2-(6-(cyclopropanesulfonamido)pyrazin-2-yl)propan-2-yl)-2-fluoro-4-(6-isopropoxypyrazin-2-yl)benzamide 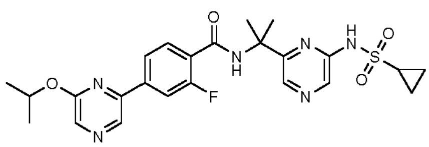 |
| **Ref P112** | 2-(2-(Cyclopropanesulfonamido)pyrimidin-4-yl)-N-(5-(6-ethoxypyrazin-2-yl)pyridin-2-yl)-2-fluorobutanamide 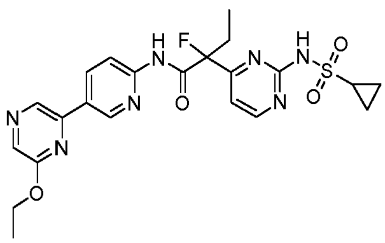 | **Ref P222** | N-(2-(6-(cyclopropanesulfonamido)pyrazin-2-yl)propan-2-yl)-4-(6-(trifluoromethyl)pyrazin-2-yl)benzamide 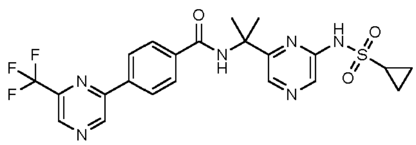 |
| **Ref P113** | Single enantiomer - stereochemistry not assigned 2-(2-(Cyclopropanesulfonamido)pyrimidin-4-yl)-N-(5-(6-ethoxypyrazin-2-yl)pyridin-2-yl)-2-fluorobutanamide | **Ref P223** | N-(1-(6-(cyclopropanesulfonamido)pyrazin-2-yl)propyl)-4-(6-ethoxypyrazin-2-yl)-2-fluorobenzamide |

(continued)

| P | Name/Structure (All examples containing chiral centres are racemates unless stated) | P | Name/Structure (All examples containing chiral centres are racemates unless stated) |
|---|---|---|---|
| | 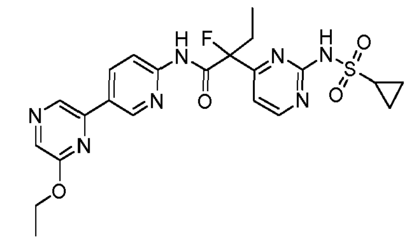 | | 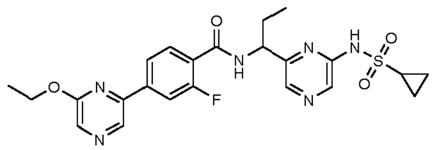 |
| **Ref P114** | Single enantiomer - stereochemistry not assigned 2-(2-(Cyclopropanesulfonamido)pyrimidin-4-yl)-N-(5-(6-ethoxypyrazin-2-yl)pyridin-2-yl)-2-fluorobutanamide 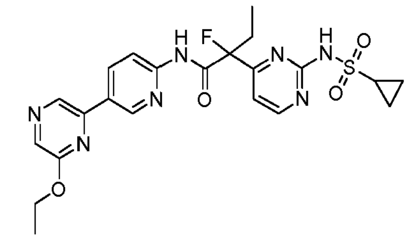 | **Ref P224** | Single enantiomer - stereochemistry unassigned N-(1-(6-(cyclopropanesulfonamido) pyrazin-2-yl)propyl)-4-(6-ethoxypyrazin-2-yl)-2-fluorobenzamide 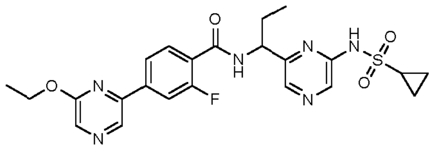 |
| **Ref P115** | 4-(2-(Cyclopropanesulfonamido)pyrimidin-4-yl)-*N*-(5-(6-ethoxypyrazin-2-yl)pyridin-2-yl)tetrahydro-2H-pyran-4-carboxamide 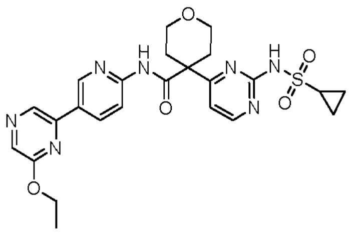 | **Ref P225** | Single enantiomer - stereochemistry unassigned N-(1-(6-(cyclopropanesulfonamido) pyrazin-2-yl)propyl)-4-(6-ethoxypyrazin-2-yl)-2-fluorobenzamide 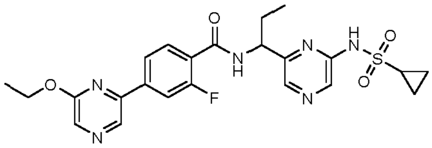 |
| **Ref P117** | 2-(2-(cyclopropanesulfonamido)pyrimidin-4-yl)-N-(4-(6-ethoxypyrazin-2-yl)-2-fluorophenyl)-2,2-difluoroacetamide 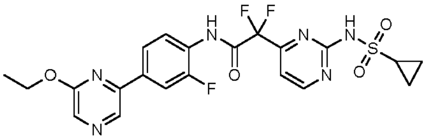 | | |

Reference Example: 2-(2-(Cyclopropanesulfonamido)pyrimidin-4-yl)-N-(5-(6-isopropylpyrazin-2-yl)pyridin-2-yl)-2-methylpropanamide P116

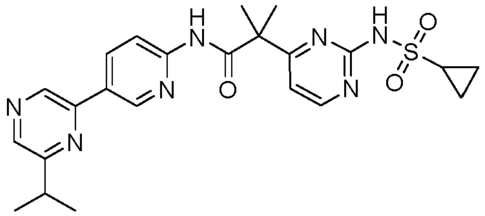

A solution of 2-(2-(cyclopropanesulfonamido)pyrimidin-4-yl)-2-methyl-*N*-(5-(6-(prop-1-en-2-yl)pyrazin-2-yl)pyridin-2-yl)propanamide **P122** (77 mg, 0.161 mmol) in MeOH/DCM (4:1, 10 mL) was hydrogenated using the H-Cube flow hydrogenation apparatus (10% Pd/C, 30x4 mm, Full hydrogen, 25 °C, 1 mL/min). The crude product was purified by chromatography on silica gel (12 g column, 50-100% EtOAc/iso-hexane) to afford 2-(2-(cyclopropanesulfonamido) pyrimidin-4-yl)-*N*-(5-(6-isopropylpyrazin-2-yl)pyridin-2-yl)-2-methylpropanamide (21 mg, 0.043 mmol, 27% yield) as a white solid. Rt 2.22 mins (HPLC acidic); m/z 482 $(M+H)^+$ $(ES^+)$; $^1$H NMR (500 MHz, DMSO-d6) δ 11.23 (s, 1H), 10.15 (s, 1H), 9.10 (s, 1H), 9.03 (dd, J = 2.4, 0.8 Hz, 1H), 8.59 (d, J = 5.3 Hz, 1H), 8.56 (s, 1H), 8.52 (dd, J = 8.8, 2.5 Hz, 1H), 8.21 (dd, J = 8.8, 0.8 Hz, 1H), 7.19 (d, J = 5.3 Hz, 1H), 3.23 - 3.10 (m, 2H), 1.61 (s, 6H), 1.32 (d, J = 6.9 Hz, 6H), 1.04 - 0.97 (m, 2H), 0.80 - 0.72 (m, 2H).

The following compounds were prepared using the methods described herein and below. Numbering of certain intermediates refers either to intermediates disclosed herein, or intermediates disclosed in WO2019/179652, WO2019/180244 and/or WO2020/083975.

1-(2-(Cyclopropanesulfonamido)pyrimidin-4-yl)-*N*-(5-(6-ethoxypyrazin-2-yl)pyridin-2-yl)-4-oxocyclohexanecarboxamide **P226**

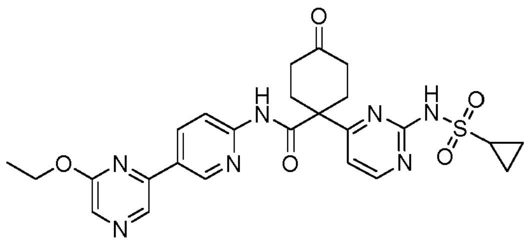

A solution of HCl (1N in water) (17.19 mL, 17.19 mmol) was added into a stirring solution of 8-(2-(cyclopropanesulfonamido)pyrimidin-4-yl)-*N*-(5-(6-ethoxypyrazin-2-yl)pyridin-2-yl)-1,4-dioxaspiro[4.5]decane-8-carboxamide **P244** (1.0 g, 1.72 mmol) in THF (30 mL). The resulting reaction mixture was stirred at 30 °C for 14 days. The reaction mixture was diluted with EtOAc (200 mL) and washed with water (100 mL) and brine (100 mL). The organic extract was dried ($MgSO_4$), filtered and concentrated in vacuo. The crude product was purified by chromatography on silica gel (40 g cartridge, 0-100% EtOAc/iso-hexanes) to afford 1-(2-(cyclopropanesulfonamido)pyrimidin-4-yl)-*N*-(5-(6-ethoxypyrazin-2-yl)pyridin-2-yl)-4-oxocyclohexanecarboxamide (414 mg, 0.762 mmol, 44% yield) as a white solid. Rt 2.03 min (HPLC acidic); *m/z 538* $(M+H)^+$ $(ES^+)$. $^1$H NMR (500 MHz, DMSO-d6) δ 11.32 (s, 1H), 10.33 (s, 1H), 9.03 (d, J = 2.5 Hz, 1H), 8.85 (s, 1H), 8.64 (d, J = 5.3 Hz, 1H), 8.51 (dd, J = 8.8, 2.5 Hz, 1H), 8.31 - 8.20 (m, 2H), 7.30 (d, J = 5.3 Hz, 1H), 4.48 (q, J = 7.0 Hz, 2H), 3.28-3.21 (m, 1H), 2.78-2.68 (m, 2H), 2.60-2.41 (m, 4H (obscured by DMSO)), 2.39 - 2.32 (m, 2H), 1.40 (t, J = 7.0 Hz, 3H), 110-1.02 (m, 2H), 0.92-0.82 (m, 2H).

1-(2-(Cyclopropanesulfonamido)pyrimidin-4-yl)-*N*-(5-(6-ethoxypyrazin-2-yl)pyridin-2-yl)-4-hydroxycyclohexane-1-carboxamide

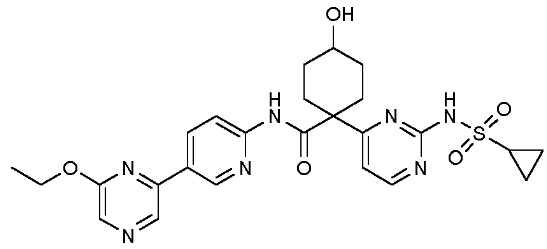

$NaBH_4$ (10.6 mg, 0.28 mmol) was added into a stirring suspension of 1-(2-(cyclopropanesulfonamido)pyrimidin-4-yl)-*N*-(5-(6-ethoxypyrazin-2-yl)pyridin-2-yl)-4-oxocyclohexanecarboxamide **P226** (100 mg, 0.186 mmol) in EtOH (20 mL) and stirred at RT for 3 hrs. The reaction mixture was concentrated in vacuo and the crude product was purified by chromatography on RP Flash C18 (24 g column, 0-100% MeCN/Water 0.1% formic acid) to afford two diastereoisomers of the title compound.

**P227 - First eluting peak from column**

1-(2-(cyclopropanesulfonamido)pyrimidin-4-yl)-*N*-(5-(6-ethoxypyrazin-2-yl)pyridin-2-yl)-4-hydroxycyclohexanecarboxamide (26 mg, 0.048 mmol, 26% yield) as a white solid. Rt 1.85 min (HPLC, acidic); m/z 540 $(M+H)^+$ $(ES^+)$; $^1H$ NMR (500 MHz, DMSO-d6) δ 11.24 (s, 1H), 10.05 (s, 1H), 9.06 - 8.98 (m, 1H), 8.85 (s, 1H), 8.57 (d, J = 5.3 Hz, 1H), 8.51 (dd, J = 8.8, 2.5 Hz, 1H), 8.26 (s, 1H), 8.23 - 8.20 (m, 1H), 7.19 (d, J = 5.3 Hz, 1H), 4.58 (d, J = 4.7 Hz, 1H), 4.48 (q, J = 7.0 Hz, 2H), 3.56-3.45 (m, 1H), 3.29-3.22 (m, 1H), 2.60 (d, J = 13.6 Hz, 2H), 1.91 - 1.77 (m, 4H), 1.52 - 1.37 (m, 5H), 1.07-0.99 (m, 2H), 0.88-0.78 (m, 2H).

**P228 - Second eluting peak from Column**

1-(2-(cyclopropanesulfonamido)pyrimidin-4-yl)-*N*-(5-(6-ethoxypyrazin-2-yl)pyridin-2-yl)-4-hydroxycyclohexanecarboxamide (23 mg, 0.042 mmol, 22% yield) as a white solid. Rt 1.95 min (HPLC, acidic); m/z 540 $(M+H)^+$ $(ES^+)$; $^1H$ NMR (500 MHz, DMSO-d6) δ 11.31 (s, 1H), 9.75 (s, 1H), 9.02 (d, J = 2.4 Hz, 1H), 8.83 (s, 1H), 8.62 (s, 1H), 8.48 (dd, J = 8.8, 2.5 Hz, 1H), 8.25 (s, 1H), 8.13 (d, J = 8.8 Hz, 1H), 7.29 (s, 1H), 4.51 - 4.44 (m, 3H), 3.70-3.62 (m, 1H), 3.29-3.20 (m, 1H), 2.22-2.11 (m, 2H), 1.80-1.68 (m, 2H), 1.41-1.29 (m, 7H), 1.11-1.05 (m, 2H), 0.96-0.88 (m, 2H).

1-(2-(Cyclopropanesulfonamido)pyrimidin-4-yl)-4-(dimethylamino)-*N*-(5-(6-ethoxypyrazin-2-yl)pyridin-2-yl)cyclohexane-1-carboxamide

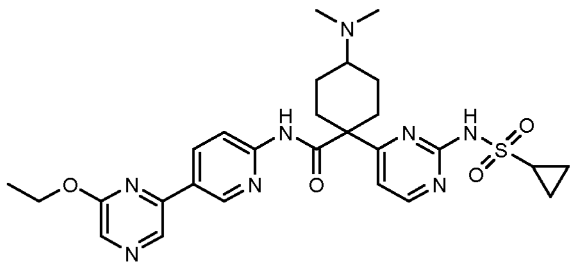

$NaH(BOAc)_3$ (118 mg, 0.558 mmol) was added into a suspension of 1-(2-(cyclopropanesulfonamido)pyrimidin-4-yl)-*N*-(5-(6-ethoxypyrazin-2-yl)pyridin-2-yl)-4-oxocyclohexanecarboxamide (100 mg, 0.186 mmol) **P226** and dimethylamine (2M in THF) (0.93 mL, 1.86 mmol) in DCM (10 mL) and the resulting reaction mixture was stirred at RT for 18 hrs. The reaction mixture was concentrated in vacuo and the crude product was purified by preparative HPLC (Acidic prep method (5-95% MeCN in water) to afford two diastereoisomers of the title compound.

**P229 - First eluting peak from Prep HPLC**

1-(2-(cyclopropanesulfonamido)pyrimidin-4-yl)-4-(dimethylamino)-*N*-(5-(6-ethoxypyrazin-2-yl)pyridin-2-yl)cyclohexane-1-carboxamide (22 mg, 0.037 mmol, 20% yield) as a white solid. Rt 1.40 min (HPLC, acidic); m/z 567 $(M+H)^+$ $(ES^+)$; $^1H$ NMR (500 MHz, DMSO-d6) δ 10.17 (s, 1H), 9.02 (d, J = 2.4 Hz, 1H), 8.84 (s, 1H), 8.60 - 8.44 (m, 2H), 8.25 (s, 1H), 8.22 - 8.20 (m, 2H), 7.09 (d, J = 5.3 Hz, 1H), 4.48 (q, J = 7.0 Hz, 2H), 3.28-3.20 (m, 1H), 2.72 (d, J = 13.4 Hz, 2H), 2.32 (s, 6H),

2.25 (s, 1H), 1.91 (d, J = 12.3 Hz, 2H), 1.79 (t, J = 12.6 Hz, 2H), 1.51 (q, J = 12.2 Hz, 2H), 1.40 (t, J = 7.0 Hz, 3H), 1.03-0.98 (m, 2H), 0.87 - 0.73 (m, 2H).

**P230 - Second eluting peak from Prep HPLC**

1-(2-(cyclopropanesulfonamido)pyrimidin-4-yl)-4-(dimethylamino)-*N*-(5-(6-ethoxypyrazin-2-yl)pyridin-2-yl)cyclohexane-1-carboxamide (26 mg, 0.045 mmol, 24% yield) as a white solid. Rt 1.48 min (HPLC, acidic); m/z 567 $(M+H)^+$ $(ES^+)$; $^1H$ NMR (500 MHz, DMSO-d6) δ 9.65 (s, 1H), 9.02 (d, J = 2.4 Hz, 1H), 8.83 (s, 1H), 8.55 (d, J = 5.2 Hz, 1H), 8.48 (dd, J = 8.8, 2.4 Hz, 1H), 8.25 (s, 1H), 8.20 (s, 1H), 8.10 (d, J = 8.8 Hz, 1H), 7.19 (d, J = 5.3 Hz, 1H), 4.47 (q, J = 7.0 Hz, 2H), 3.25-3.18 (m, 1H), 2.78-2.67 (m, 2H), 2.48-2.39 (m, 1H), 2.26 (s, 6H), 2.02 (t, J = 12.4 Hz, 2H), 1.87-1.77 (m, 2H), 1.42 - 1.29 (m, 5H), 1.07-1.00 (m, 2H), 0.97 - 0.85 (m, 2H).

**Method 11: *i*-PrMgCl mediated amide coupling from ester**

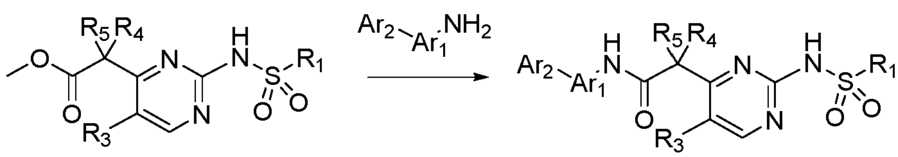

To an ice cooled solution of aniline (1.1 eq) in THF (10-50 volumes) was added *i*-PrMgCl (2.0 M in THF, 2.0 eq) dropwise over 5-15 mins to maintain an internal temperature of less than 10 °C. The reaction mixture was warmed to RT over 45 mins, then a solution of ester (1.0 eq) in THF (5-20 volumes) was added dropwise over 5 - 15 min. The reaction mixture was stirred at ambient temperature for 5-15 mins then further *i*-PrMgCl (2.0 M in THF, 2.0 eq) was added dropwise over 5-20 min. The reaction mixture was stirred at RT for 30 mins and then the solution was slowly poured into 1M HCl (aq) and extracted with EtOAc. The organics were combined, dried (phase separator) and concentrated *in vacuo*. The crude product was purified by reverse or normal phase chromatography or a combination of both.

**Reductive amination General method:**

**Method 12: Reductive amination**

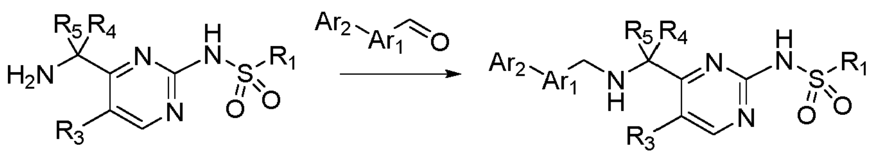

To a suspension of aldehyde (1.5 eq) and amine (HCl salt can be used, 1.0 eq) in an organic solvent such as DCM (2-10 volumes) was added AcOH (1.0 eq) at RT and stirred for up to 1 hr. $NaBH(OAc)_3$ (1-2 eq) was then added and stirring continued for up to 24 hrs and monitored by LCMS. On completion 1% $NH_3$ in MeOH (10 volumes) was added and the volatiles removed in vacuo. The crude product was purified by reverse or normal phase chromatography or a combination of both.

*N*-(4-(1-((4-(6-Ethoxypyrazin-2-yl)-2-fluorobenzyl)amino)propyl)pyrimidin-2-yl)cyclopropanesulfonamide **P235**

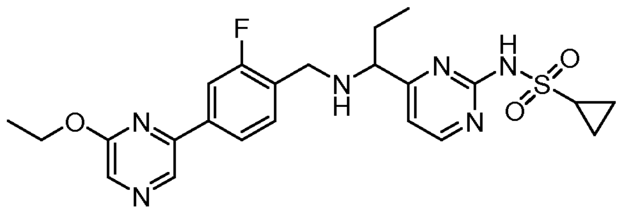

A suspension of 4-(6-ethoxypyrazin-2-yl)-2-fluorobenzaldehyde **INTD88** (259 mg, 1.05 mmol) and *N*-(4-(1-aminopropyl)pyrimidin-2-yl)cyclopropanesulfonamide **INTC162** (300 mg, 1.05 mmol) in DCM (2 mL) was treated with AcOH (0.065 mL, 1.14 mmol) and stirred for 15 mins then $NaBH(OAc)_3$ (223 mg, 1.06 mmol) was added and the reaction mixture was stirred at RT for 3 hrs. To the reaction mixture was added 1% $NH_3$ in MeOH (2 mL) and the volatiles were removed in vacuo. The crude product was purified by chromatography on RP Flash C18 (12 g cartridge, 15-70% MeCN/10 mM ammonium bicarbonate). The crude material was purified by capture and release on SCX (1 g) eluting with MeOH (20 mL) then removing product with 1% $NH_3$ in MeOH (30 mL). The crude material was finally purified a second time by chromatography on RP Flash C18 (12 g cartridge, 10-50% MeCN/10 mM Ammonium Bicarbonate) to afford *N*-(4-(1-((4-(6-ethoxypyrazin-2-yl)-2-fluorobenzyl)amino)propyl)pyrimidin-2-yl)cyclopropanesulfonamide (20 mg, 0.031 mmol, 3% yield) as a yellow gum. Rt 2.02 min (HPLC, basic); m/z 487 $(M+H)^+$ $(ES^+)$; $^1H$ NMR (500 MHz, DMSO-d6) δ 8.85 (s, 1H), 8.27 - 8.26 (m, 2H), 7.96 (d, J = 7.8 Hz, 1H), 7.87 (d, J = 11.4 Hz, 1H), 7.67 - 7.58 (m, 1H), 7.23 (d, J = 5.1 Hz, 1H), 5.38 (t, J = 5.7 Hz, 1H), 4.62 (d, J = 5.8 Hz, 2H), 4.49 (q, J = 7.1 Hz, 2H), 3.52 - 3.50 (m, 1H), 1.71 - 1.67 (m, 2H), 1.41 (t, J = 7.0 Hz, 3H), 1.13 - 1.09 (m, 2H), 1.04 - 1.01 (m, 2H), 0.84 (t, J = 7.4 Hz, 3H). Two exchangeable protons not observed.

**Table 5:** Preparation methods and characterisation data of certain intermediates and examples **P242 onwards**

| INTC # or P# | Name/Structure (All examples containing chiral centres are racemates unless stated) | Synthesis Method, [LCMS Method], m/z $(M+H)^+$, (Rt/Min) | $^1H$ NMR Chemical Shift Data (DMSO-d6 unless stated) |
|---|---|---|---|
| **INTC 246** | *tert*-Butyl 4-(4-(cyclopropanesulfonamido)pyrim idin-2-yl)-4-((5-(6-ethoxypyrazin-2-yl)pyridin-2-yl)carbamoyl)piperidine-1-carboxylate 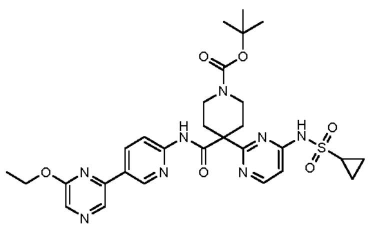 | Method 11 using **INTC243** and **INTD33,** [UPLC Acidic], 625, (1.66) | 11.32 (s, 1H), 9.99 (s, 1H), 9.01 (d, J = 2.4 Hz, 1H), 8.84 (s, 1H), 8.58 - 8.55 (m, 1H), 8.53 - 8.47 (m, 1H), 8.25 (s, 1H), 8.21 (d, J = 8.8 Hz, 1H), 6.85 (d, J = 5.8 Hz, 1H), 4.48 (q, J = 7.0 Hz, 2H), 3.51 - 3.48 (m, 2H), 3.38 - 3.32 (m, 2H), 3.16 - 3.12 (m, 1H), 2.32 - 2.29 (m, 4H), 1.41 (s, 9H), 1.40 (t, J = 7.10 Hz, 3H), 1.08 (s, 2H), 0.96 - 0.91 (m, 2H). |
| **INTC 239** | *tert*-Butyl 4-(6-(cyclopropanesulfonamido)pyraz in-2-yl)-4-((5-(6-ethoxypyrazin-2-yl)pyridin-2-yl)carbamoyl)piperidine-1-carboxylate 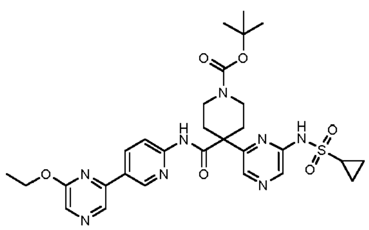 | Method 11 using **INTC238** and **INTD33,** [UPLC Acidic], 625, (1.63) | 11.06 (s, 1H), 10.17 (s, 1H), 9.01 (s, 1H), 8.83 (s, 1H), 8.52 - 8.46 (m, 1H), 8.42 (s, 1H), 8.25 (s, 1H), 8.21 - 8.15 (m, 2H), 4.47 (q, J = 7.0 Hz, 2H), 3.71 - 3.65 (m, 2H), 3.28 - 3.16 (m, 2H), 3.13 - 3.03 (m, 1H), 2.18 - 2.05 (m, 2H), 1.43 - 1.36 (m, 12H), 1.05 - 1.02 (m, 2H), 0.86 - 0.81 (m, 2H). 2H not observed, obscured by DMSO peak. |

(continued)

| INTC # or P# | Name/Structure (All examples containing chiral centres are racemates unless stated) | Synthesis Method, [LCMS Method], m/z $(M+H)^+$, (Rt/Min) | $^1H$ NMR Chemical Shift Data (DMSO-d6 unless stated) |
|---|---|---|---|
| **INTC 244** | *tert*-Butyl 4-(4-(cyclopropanesulfonamido)pyrim idin-2-yl)-4-((5-(6-cyclopropylpyrazin-2-yl)pyridin-2-yl)carbamoyl)piperidine-1-carboxylate 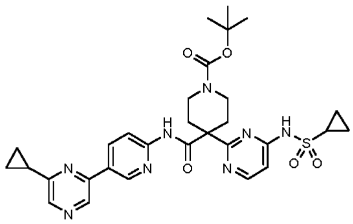 | Method 11 using **INTC243** and **INTD54,** [HPLC Acidic], 621, (2.57) | 11.32 (s, 1H), 9.97 (s, 1H), 9.02 - 8.95 (m, 2H), 8.58 (s, 1H), 8.56 (s, 1H), 8.46 (dd, J = 8.8, 2.4 Hz, 1H), 8.19 (d, J = 8.8 Hz, 1H), 6.85 (d, J = 5.8 Hz, 1H), 3.50 - 3.47 (m, 2H), 3.42 - 3.26 (m, 2H), 3.16 - 3.12 (m, 1H), 2.32 - 2.22 (m, 5H), 1.41 (s, 9H), 1.17 - 1.06 (m, 6H), 0.96 - 0.91 (m, 2H). |
| **P242** | 1-(2-(cyclopropanesulfonamido)pyrim idin-4-yl)-*N*-(5-(6-ethoxypyrazin-2-yl)pyridin-2-yl)-4,4-difluorocyclohexane-1-carboxamide 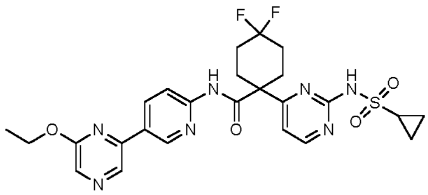 | Method 11: using INTC212 and INTD33, [HPLC Acidic], 560, (2.42) | 11.33 (s, 1H), 10.28 (s, 1H), 9.03 (dd, J = 2.5, 0.8 Hz, 1H), 8.85 (s, 1H), 8.63 (d, J = 5.3 Hz, 1H), 8.51 (dd, J = 8.8, 2.5 Hz, 1H), 8.26 (s, 1H), 8.19 (dd, J = 8.8, 0.8 Hz, 1H), 7.26 (d, J = 5.4 Hz, 1H), 4.48 (q, J = 7.1 Hz, 2H), 3.27-3.18 (m, 1H), 2.65-2.53 (m, 2H), 2.29-2.18 (m, 2H), 2.13-2.01 (m, 4H), 1.40 (t, J = 7.0 Hz, 3H), 1.11-1.02 (m, 2H), 0.92-0.83 (m, 2H). |
| **P244** | 8-(2-(cyclopropanesulfonamido)pyrim idin-4-yl)-*N*-(5-(6-ethoxypyrazin-2-yl)pyridin-2-yl)-1,4-dioxaspiro[4.5]decane-8-carboxamide 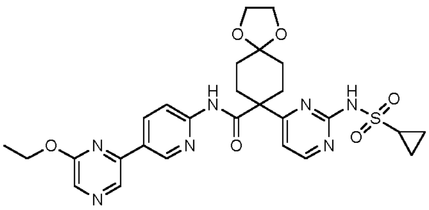 | Method 11: using INTC214 and INTD33, [HPLC Acidic], 582, (2.20) | 11.29 (s, 1H), 10.05 (s, 1H), 9.02 (dd, J = 2.5, 0.8 Hz, 1H), 8.84 (s, 1H), 8.60 (d, J = 5.3 Hz, 1H), 8.50 (dd, J = 8.8, 2.5 Hz, 1H), 8.26 (s, 1H), 8.22 - 8.11 (m, 1H), 7.23 (s, 1H), 4.48 (q, J = 7.0 Hz, 2H), 3.88 (s, 4H), 3.28-3.18 (m, 1H), 2.55-2.47 (m, 2H obscured by water peak), 2.27 - 2.13 (m, 2H), 1.79-1.62 (m, 4H), 1.40 (t, J = 7.0 Hz, 3H), 1.09-1.00 (m, 2H), 0.94-0.80 (m, 2H). |
| **P248** | *4-(2-((N,N*-dimethylsulfamoyl)amino)pyrimi din-4-yl)-*N*-(5-(6-ethoxypyrazin-2-yl) pyridin-2-yl)tetrahydro-2*H*-pyran-4-carboxamide 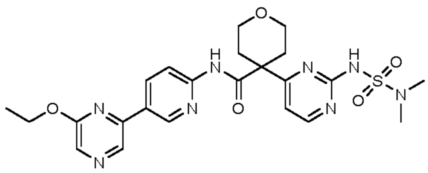 | Method R: using INTC204, [HPLC Acidic], 529, (2.10) | 11.04 (s, 1H), 10.02 (s, 1H), 9.04 (d, J = 2.5 Hz, 1H), 8.85 (s, 1H), 8.60 (d, J = 5.3 Hz, 1H), 8.51 (dd, J = 8.8, 2.5 Hz, 1H), 8.26 (s, 1H), 8.19 (d, J = 8.8 Hz, 1H), 7.22 (d, J = 5.3 Hz, 1H), 4.48 (q, J = 7.0 Hz, 2H), 3.73 - 3.57 (m, 4H), 2.84 (s, 6H), 2.47-2.37 (m, 2H), 2.28 - 2.17 (m, 2H), 1.40 (t, J = 7.0 Hz, 3H). |

(continued)

| INTC # or P# | Name/Structure (All examples containing chiral centres are racemates unless stated) | Synthesis Method, [LCMS Method], m/z $(M+H)^+$, (Rt/Min) | $^1$H NMR Chemical Shift Data (DMSO-d6 unless stated) |
|---|---|---|---|
| **P251** | 4-(2-(cyclopropanesulfonamido)pyrimidin-4-yl)-*N*-(5-(6-ethoxypyrazin-2-yl)pyridin-2-yl)-1-(methylsulfonyl)piperidine-4-carboxamide 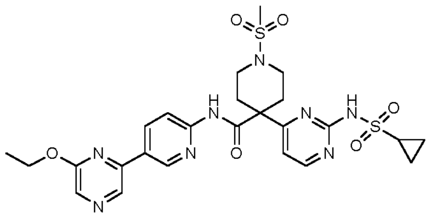 | Method 11: using INTC215 and INTD33, [HPLC Acidic], 603, (2.05) | 11.33 (s, 1H), 10.27 (s, 1H), 9.03 (d, J = 2.4 Hz, 1H), 8.85 (s, 1H), 8.63 (d, J = 5.3 Hz, 1H), 8.54 - 8.48 (m, 1H), 8.26 (s, 1H), 8.21 (d, J = 8.8 Hz, 1H), 7.26 (d, J = 5.3 Hz, 1H), 4.48 (q, J = 7.0 Hz, 2H), 3.47 - 3.40 (m, 2H), 3.29 - 3.21 (m, 1H), 3.10 (t, J = 10.7 Hz, 2H), 2.87 (s, 2H), 2.64 - 2.57 (m, 2H), 2.33 - 2.21 (m, 2H), 1.43 - 1.37 (m, 3H), 1.32 - 1.09 (m, 1H), 1.09 - 1.04 (m, 2H), 0.92 - 0.80 (m, 2H). |
| **P254** | N-(4-(1-(((5-(6-ethoxypyrazin-2-yl)pyridin-2-yl)methyl)amino)cyclopropyl)pyrimidin-2-yl)cyclopropanesulfonamide 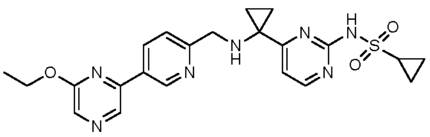 | Method 12 using INTC156 and INTD85, [UPLC Basic], 468, (1.07) | 11.08 (s, 1H), 9.22 (d, J = 2.3 Hz, 1H), 8.88 (s, 1H), 8.50 - 8.42 (m, 2H), 8.29 (s, 1H), 7.64 (d, J = 8.2 Hz, 1H), 7.43 (d, J = 5.2 Hz, 1H), 4.50 (q, J = 7.0 Hz, 2H), 3.94 (s, 2H), 3.16 - 3.13 (m, 1H), 1.41 (t, J = 7.0 Hz, 3H), 1.38 - 1.33 (m, 2H), 1.26 - 1.21 (m, 2H), 1.09 - 0.99 (m, 4H). One exchangeable proton not observed |
| **P255** | N-(4-(1-((4-(6-ethoxypyrazin-2-yl)-2-fluorobenzyl)amino)cyclopropyl) pyrimidin-2-yl)cyclopropanesulfonamide 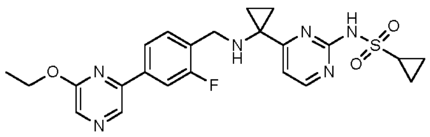 | Method 12 using INTC156 and INTD88, [UPLC Basic], 485, (1.36) | 11.06 (s, 1H), 8.86 (d, J = 2.4 Hz, 1H), 8.48 (s, 1H), 8.27 (d, J = 2.5 Hz, 1H), 7.97 (d, J = 8.3 Hz, 1H), 7.90 (d, J = 11.5 Hz, 1H), 7.70 - 7.63 (m, 1H), 7.43 (s, 1H), 4.53 - 4.45 (m, 2H), 3.84 (s, 2H), 3.16 - 3.12 (m, 1H), 1.44 - 1.39 (m, 3H), 1.38 - 1.32 (m, 2H), 1.27 - 1.21 (m, 2H), 1.09 - 1.01 (m, 4H). One exchangeable proton not observed |
| **P256** | N-(4-(4-(((4-(6-ethoxypyrazin-2-yl)phenyl)amino)methyl)tetrahyd ro-2H-pyran-4-yl)pyrimidin-2-yl)cyclopropanesulfonamide 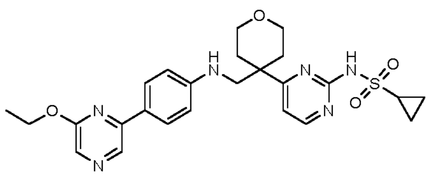 | Method R using INTC207, [HPLC Acidic], 511, (2.19) | 11.23 (s, 1H), 8.57 (s, 1H), 8.51 (d, J = 5.3 Hz, 1H), 8.00 (s, 1H), 7.83 - 7.74 (m, 2H), 7.22 (d, J = 5.1 Hz, 1H), 6.67 - 6.59 (m, 2H), 6.02 (d, J = 6.5 Hz, 1H), 4.42 (q, J = 7.0 Hz, 2H), 3.80-3.69 (m, 2H), 3.42 - 3.27 (m, 4H), 3.25-3.17 (m, 1H), 2.34-2.25 (m, 2H), 1.89-1.80 (m, 2H), 1.38 (t, J = 7.0 Hz, 3H), 1.14-1.06 (m, 2H), 1.07 - 0.98 (m, 2H |
| **P258** | 2-(2-(cyclopropanesulfonamido)pyrimidin-4-yl)-*N*-(5-(6-ethoxypyrazin-2-yl)pyridin-2-yl)-5,8-dioxaspiro[3.4]octane-2-carboxamide 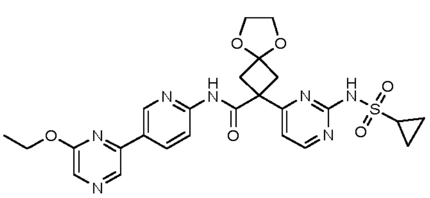 | Method 11: using INTC216 and INTD33, [UPLC acidic], 554, (1.32) | 11.31 (s, 1H), 10.56 (s, 1H), 9.03 (d, J = 2.4 Hz, 1H), 8.84 (s, 1H), 8.60 (d, J = 5.3 Hz, 1H), 8.51 (dd, J = 8.8, 2.5 Hz, 1H), 8.26 - 8.20 (m, 2H), 7.30 (d, J = 5.3 Hz, 1H), 4.48 (q, J = 7.0 Hz, 2H), 3.89 - 3.78 (m, 4H), 3.32 - 3.25 (m, 1H), 3.18 - 3.11 (m, 2H), 2.99 - 2.92 (m, 2H), 1.39 (t, J = 7.0 Hz, 3H), 1.10 - 1.01 (m, 2H), 0.94 - 0.87 (m, 2H). |

(continued)

| INTC # or P# | Name/Structure (All examples containing chiral centres are racemates unless stated) | Synthesis Method, [LCMS Method], m/z $(M+H)^+$, (Rt/Min) | $^1$H NMR Chemical Shift Data (DMSO-d6 unless stated) |
|---|---|---|---|
| **P260** | Mix of diastereoisomers: 1-(2-(cyclopropanesulfonamido)pyrim idin-4-yl)-*N*-(5-(6-ethoxypyrazin-2-yl)pyridin-2-yl)-4-methoxycyclohexane-1-carboxamide 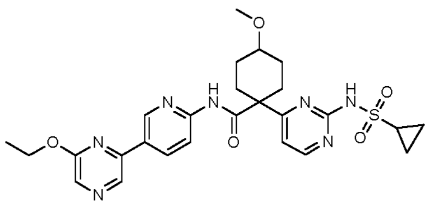 | Method 11: using INTC218 and INTD33, [HPLC Acidic], 554, (2.17 and 2.27) | 11.26 (s, 1H), 10.04 (s, 1H), 9.02 (d, J = 2.4 Hz, 1H), 8.84 (d, J = 1.9 Hz, 1H), 8.60 (d, J = 5.6 Hz, 1H), 8.55 - 8.46 (m, 1H), 8.25 (s, 1H), 8.22 - 8.14 (m, 1H), 7.22 (d, J = 5.4 Hz, 1H), 4.48 (qd, J = 7.0, 2.3 Hz, 2H), 3.30 - 3.18 (m, 4H), 2.59 (d, J = 13.3 Hz, 2H), 2.40-2.19 (m, 1H), 1.97-1.84 (m, 2H), 1.83 - 1.50 (m, 2H), 1.48 - 1.36 (m, 4H), 1.11 - 0.98 (m, 2H), 0.94 - 0.71 (m, 3H). |
| **P261** | *N*-(4-(1-((4-(6-ethoxypyrazin-2-yl)phenyl) amino)propyl)pyrimidin -2-yl)cyclopropanesulfonamidearbo xamide 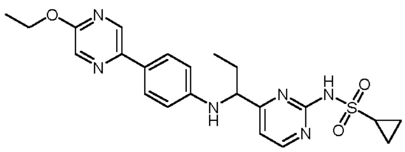 | Method 12 using INTC155 and INTD18, [UPLC Acidic], 455, (1.48) | Methanol-d4, 8.45 - 8.39 (m, 2H), 7.90 (s, 1H), 7.86 - 7.81 (m, 2H), 7.70 - 7.62 (m, 1H), 7.61 - 7.54 (m, 1H), 7.12 (d, J = 5.2 Hz, 1H), 6.69 - 6.64 (m, 2H), 4.49 (q, J = 7.1 Hz, 2H), 4.45 - 4.39 (m, 1H), 3.24 - 3.15 (m, 1H), 2.06 - 1.85 (m, 2H), 1.44 (t, J = 7.1 Hz, 3H), 1.34 - 1.25 (m, 1H), 1.24 - 1.15 (m, 1H), 1.09 (t, J = 7.4 Hz, 3H), 1.07 - 1.00 (m, 1H), 0.96 - 0.86 (m, 1H). |
| **P288** | 4-(2-(Cyclopropanesulfonamido)pyri midin-4-yl)-*N*-(5-(6-ethoxypyrazin-2-yl)pyridin-2-yl)-1-(2-methoxyacetyl)piperidine-4-carboxamide 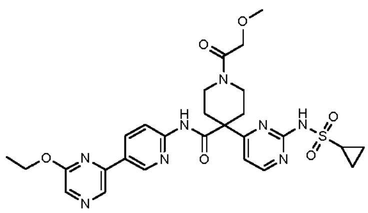 | Prepared by reacting Ref **P140** (in WO2019/179 652) with 2-methoxyacet yl chloride, TEA, in DCM at RT for 24h, [UPLC Acidic], 597, (1.21) | 11.38 (s, 1H), 10.21 (s, 1H), 9.02 (dd, J = 2.5, 0.8 Hz, 1H), 8.83 (s, 1H), 8.61 (d, J = 5.4 Hz, 1H), 8.49 (dd, J = 8.8, 2.5 Hz, 1H), 8.25 (s, 1H), 8.21 - 8.13 (m, 1H), 7.25-7.20 (m, 1H), 4.47 (q, J = 7.0 Hz, 2H), 4.10 (m, 2H), 3.90 (d, J = 13.5 Hz, 1H), 3.60 (d, J = 13.6 Hz, 1H), 3.28 (s, 3H), 3.26-3.21 (dt, m, 1H), 2.47 (s, 3H), 2.20 - 2.05 (m, 3H), 1.39 (t, J = 7.0 Hz, 3H), 1.05-1.03 (m, 2H), 0.91-0.85 (m, 2H). |
| **P289** | 4-(2-(Cyclopropanesulfonamido)pyri midin-4-yl)-*N*-(5-(6-ethoxypyrazin-2-yl)pyridin-2-yl)-1-(ethylsulfonyl)piperidine-4-carboxamide 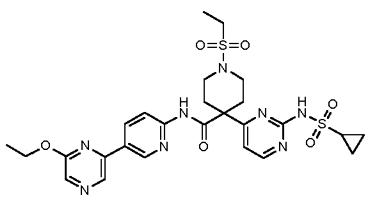 | Prepared by reacting Ref **P140** (in WO2019/179 652) with ethanesulfon yl chloride, TEA, in DCM at RT for 24h, [UPLC Acidic], 617, (1.38) | 11.32 (s, 1H), 10.25 (s, 1H), 9.02 (d, J = 2.5 Hz, 1H), 8.84 (s, 1H), 8.62 (d, J = 5.4 Hz, 1H), 8.50 (dd, J = 8.8, 2.5 Hz, 1H), 8.25 (s, 1H), 8.20 (d, J = 8.8 Hz, 1H), 7.26 - 7.22 (m, 1H), 4.47 (q, J = 7.0 Hz, 2H), 3.51 - 3.45 (m, 2H), 3.21 - 3.13 (m, 2H), 3.05 (q, J = 7.4 Hz, 2H), 2.61 - 2.54 (m, 2H), 2.26 - 2.18 (m, 1H), 1.39 (t, J = 7.0 Hz, 3H), 1.18 (t, J = 7.3 Hz, 3H), 1.08 - 1.02 (m, 2H), 0.91 - 0.80 (m, 4H). |

(continued)

| INTC # or P# | Name/Structure (All examples containing chiral centres are racemates unless stated) | Synthesis Method, [LCMS Method], m/z $(M+H)^+$, (Rt/Min) | $^1$H NMR Chemical Shift Data (DMSO-d6 unless stated) |
|---|---|---|---|
| **P290** | 4-(2-(Cyclopropanesulfonamido)pyri midin-4-yl)-1-(cyclopropylsulfonyl)-*N*-(5-(6-ethoxypyrazin-2-yl)pyridin-2-yl)piperidine-4-carboxamide 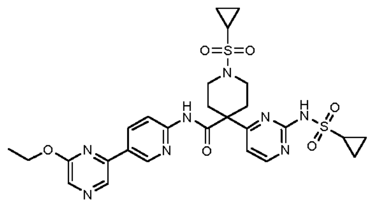 | Prepared by reacting Ref **P140** (in WO2019/179 652) with cyclopropane sulfonyl chloride, TEA, in DCM at RT for 24h, [UPLC Acidic], 629, (1.38) | 11.32 (s, 1H), 10.29 (s, 1H), 9.03 (dd, J = 2.4, 0.8 Hz, 1H), 8.85 (s, 1H), 8.63 (d, J = 5.3 Hz, 1H), 8.51 (dd, J = 8.8, 2.5 Hz, 1H), 8.26 (s, 1H), 8.21 (dd, J = 8.8, 0.8 Hz, 1H), 7.26 (d, J = 5.4 Hz, 1H), 4.48 (q, J = 7.0 Hz, 2H), 3.55 - 3.49 (m, 2H), 3.29 - 3.11 (m, 3H), 2.69 - 2.52 (m, 4H), 2.29 - 2.17 (m, 2H), 1.40 (t, J = 7.0 Hz, 3H), 1.10 - 1.01 (m, 2H), 1.01 - 0.79 (m, 5H). |
| **P291** | 4-(2-(Cyclopropanesulfonamido)pyri midin-4-yl)-1-(*N*,*N*-dimethylsulfamoyl)-N-(5-(6-ethoxypyrazin-2-yl)pyridin-2-yl)piperidine-4-carboxamide 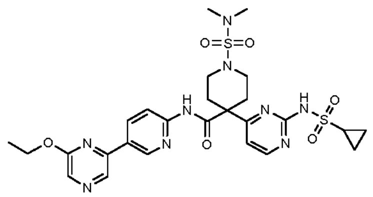 | Prepared by reacting Ref **P140** (in WO2019/179 652) with dimethylsulfa moyl chloride, TEA, in DCM at RT for 24h, [UPLC Acidic], 632, (1.45) | 11.32 (s, 1H), 10.24 (s, 1H), 9.02 (d, J = 2.4 Hz, 1H), 8.84 (s, 1H), 8.62 (d, J = 5.3 Hz, 1H), 8.50 (dd, J = 8.8, 2.4 Hz, 1H), 8.25 (s, 1H), 8.19 (d, J = 8.7 Hz, 1H), 7.24 (d, J = 5.4 Hz, 1H), 4.47 (q, J = 7.0 Hz, 2H), 3.45 - 3.39 (m, 2H), 3.26 - 3.21 (m, 1H), 3.20 - 3.12 (m, 2H), 2.74 (s, 6H), 2.59 - 2.53 (m, 2H), 2.26 - 2.18 (m, 2H), 1.39 (t, J = 7.0 Hz, 3H), 1.07 - 1.04 (m, 2H), 0.86 - 0.82 (m, 2H). |
| **P292** | 4-(2-(Cyclopropanesulfonamido)pyri midin-4-yl)-*N*-(5-(6-ethoxypyrazin-2-yl)pyridin-2-yl)-1-((trifluoromethyl)sulfonyl)piperidi ne-4-carboxamide 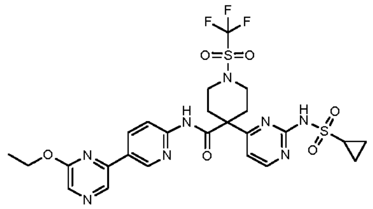 | Prepared by reacting Ref **P140** (in WO2019/179 652) with trifluorometh anesulfonic anhydride, TEA, in DCM at RT for 24h, [UPLC Acidic], 657, (1.62) | 11.35 (s, 1H), 10.36 (s, 1H), 9.03 (d, J = 2.4 Hz, 1H), 8.84 (s, 1H), 8.62 (d, J = 5.3 Hz, 1H), 8.51 (dd, J = 8.8, 2.5 Hz, 1H), 8.25 (s, 1H), 8.19 (d, J = 8.8 Hz, 1H), 7.22 (d, J = 5.3 Hz, 1H), 4.47 (q, J = 7.0 Hz, 2H), 3.78 - 3.74 (m, 2H), 3.53 - 3.49 (m, 2H), 3.26 - 3.18 (m, 1H), 2.69 - 2.55 (m, 2H), 2.29 (s, 2H), 1.39 (t, J = 7.0 Hz, 3H), 1.08 - 1.03 (m, 2H), 0.92 - 0.77 (m, 2H). |
| **P293** | 4-(2-(Cyclopropanesulfonamido)pyri midin-4-yl)-*N*-(5-(6-ethoxypyrazin-2-yl)pyridin-2-yl)-1-((1-methyl-1*H*-pyrazol-3-yl) sulfonyl)piperidine-4-carboxamide 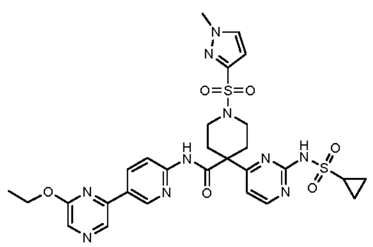 | Prepared by reacting Ref **P140** (in WO2019/179 652) with 1-methyl-1*H*-pyrazole-3-sulfonyl chloride, TEA, in DCM at RT for 24h, [UPLC Acidic], 669, (1.34) | 11.30 (s, 1H), 10.23 (s, 1H), 9.01 (d, J = 2.5 Hz, 1H), 8.83 (s, 1H), 8.61 (d, J = 5.3 Hz, 1H), 8.47 (dd, J = 8.8, 2.5 Hz, 1H), 8.25 (s, 1H), 8.06 (d, J = 8.8 Hz, 1H), 7.90 (d, J = 2.3 Hz, 1H), 7.20 (d, J = 5.3 Hz, 1H), 6.65 (d, J = 2.3 Hz, 1H), 4.47 (q, J = 7.0 Hz, 2H), 3.88 (s, 3H), 3.58 - 3.38 (m, 2H), 3.25 - 3.16 (m, 1H), 2.92 - 2.85 (m, 2H), 2.62 - 2.56 (m, 2H), 2.25 - 2.16 (m, 2H), 1.39 (t, J = 7.0 Hz, 3H), 1.07 - 1.00 (m, 2H), 0.91 - 0.79 (m, 2H). |

(continued)

| INTC # or P# | Name/Structure (All examples containing chiral centres are racemates unless stated) | Synthesis Method, [LCMS Method], m/z $(M+H)^+$, (Rt/Min) | $^1H$ NMR Chemical Shift Data (DMSO-d6 unless stated) |
|---|---|---|---|
| **P294** | 1-(cyanomethyl)-4-(2-(cyclopropanesulfonamido)pyrim idin-4-yl)-N-(5-(6-ethoxypyrazin-2-yl)pyridin-2-yl)piperidine-4-carboxamide 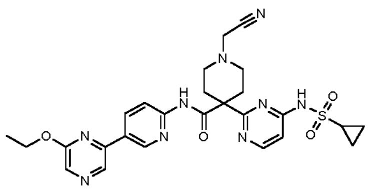 | Alkylation capping using **INTC247** and 2-bromoacetoni trile, [UPLC Acidic], 564, (1.23) | 11.34 (s, 1H), 9.92 (s, 1H), 9.04 - 9.00 (m, 1H), 8.84 (s, 1H), 8.57 (s, 1H), 8.51 (dd, J = 8.8, 2.5 Hz, 1H), 8.25 (s, 1H), 8.21 (d, J = 8.7 Hz, 1H), 6.84 (s, 1H), 4.48 (q, J = 7.0 Hz, 2H), 3.70 (s, 2H), 3.18 - 3.14 (m, 1H), 2.42 (s, 6H), 1.40 (t, J = 7.0 Hz, 3H), 1.09 - 1.05 (m, 2H), 0.96 - 0.91 (m, 2H). 2H obscured in DMSO peak |
| **P295** | ethyl 2-(4-(2-(cyclopropanesulfonamido) pyrim idin-4-yl)-4-((5-(6-ethoxypyrazin-2-yl)pyridin-2-yl)carbamoyl)piperidin-1-yl) acetate 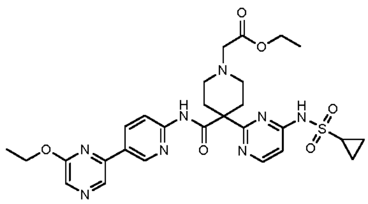 | Prepared by reacting **INTC247** and ethyl 2-oxoacetate, [UPLC Acidic], 611, (0.95) | 11.32 (s, 1H), 9.83 (s, 1H), 9.01 (d, J = 2.5 Hz, 1H), 8.84 (s, 1H), 8.50 (dd, J = 8.8, 2.5 Hz, 1H), 8.25 (s, 1H), 8.20 (d, J = 8.8 Hz, 1H), 6.81 (s, 1H), 4.48 (q, J = 7.0 Hz, 2H), 4.06 (q, J = 7.2 Hz, 2H), 3.20 - 3.17 (m, 2H), 3.15 - 3.12 (m, 1H), 2.60 - 2.57 (m, 3H), 2.40 - 2.36 (m, 4H), 1.93 - 1.90 (m, 1H), 1.40 (t, J = 7.0 Hz, 3H), 1.16 (t, J = 7.1 Hz, 3H), 1.07 - 1.04 (m, 2H), 0.92 (d, J = 6.4 Hz, 2H). 1H obscured in DMSO. |
| **P296** | *N*-(5-(6-ethoxypyrazin-2-yl)pyridin-2-yl)-4-(2-(ethylsulfonamido)pyrimidin-4-yl)-1-(2-methoxyacetyl)piperidine-4-carboxamide 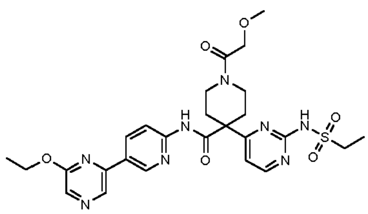 | Prepared as for **P288** using **INTC235** and 2-methoxyacet yl chloride, [UPLC Acidic], 585, (1.18) | 11.24 (s, 1H), 10.23 (s, 1H), 9.04 (d, J = 2.5 Hz, 1H), 8.85 (s, 1H), 8.62 (s, 1H), 8.51 (dd, J = 8.7, 2.5 Hz, 1H), 8.26 (s, 1H), 8.21 (d, J = 8.8 Hz, 1H), 7.24 (s, 1H), 4.48 (q, J = 7.0 Hz, 2H), 4.20 - 4.02 (m, 2H), 3.89 (s, 1H), 3.59 (s, 1H), 3.51 (s, 2H), 3.41 - 3.33 (m, 1H), 3.29 (s, 3H), 3.22 (s, 1H), 2.24 - 1.95 (m, 2H), 1.40 (t, J = 7.0 Hz, 3H), 1.12 (t, J = 7.4 Hz, 3H), 0.89 - 0.80 (m, 2H). |
| **P297** | *N*-(5-(6-ethoxypyrazin-2-yl)pyridin-2-yl)-4-(2-(ethylsulfonamido)pyrimidin-4-yl)-1-(methylsulfonyl)piperidine-4-carboxamide 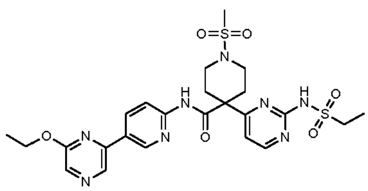 | Prepared as for **P288** using **INTC235** and methanesulfo nyl chloride, [UPLC Acidic], 591, (1.26) | 11.25 (s, 1H), 10.27 (s, 1H), 9.03 (d, J = 2.5 Hz, 1H), 8.84 (s, 1H), 8.62 (d, J = 5.3 Hz, 1H), 8.51 (dd, J = 8.8, 2.5 Hz, 1H), 8.25 (s, 1H), 8.22 (d, J = 8.8 Hz, 1H), 7.24 (d, J = 5.3 Hz, 1H), 4.47 (q, J = 7.0 Hz, 2H), 3.53 (q, J = 7.3 Hz, 2H), 3.42 (d, J = 12.9 Hz, 2H), 3.09 (t, J = 10.7 Hz, 2H), 2.86 (s, 3H), 2.59 - 2.54 (m, 2H), 2.28 - 2.19 (m, 2H), 1.39 (t, J = 7.0 Hz, 3H), 1.13 (t, J = 7.3 Hz, 3H). |

(continued)

| INTC # or P# | Name/Structure (All examples containing chiral centres are racemates unless stated) | Synthesis Method, [LCMS Method], m/z $(M+H)^+$, (Rt/Min) | $^1$H NMR Chemical Shift Data (DMSO-d6 unless stated) |
|---|---|---|---|
| **P298** | *N*-(5-(6-ethoxypyrazin-2-yl)pyridin-2-yl)-4-(2-(ethylsulfonamido)pyrimidin-4-yl)-1-(ethylsulfonyl)piperidine-4-carboxamide 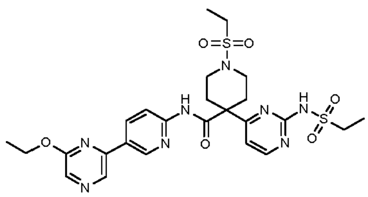 | Prepared as for **P288** using **INTC235** and ethanesulfon yl chloride, [UPLC Acidic], 605, (1.34) | 11.24 (s, 1H), 10.25 (s, 1H), 9.03 (d, J = 2.5 Hz, 1H), 8.84 (s, 1H), 8.61 (d, J = 5.3 Hz, 1H), 8.50 (dd, J = 8.8, 2.5 Hz, 1H), 8.25 (s, 1H), 8.21 (d, J = 8.8 Hz, 1H), 7.23 (d, J = 5.3 Hz, 1H), 4.47 (q, J = 7.0 Hz, 2H), 3.56 - 3.43 (m, 4H), 3.20 - 3.12 (m, 2H), 3.04 (q, J = 7.4 Hz, 2H), 2.58-2.50 (m, 2H), 2.25 - 2.14 (m, 2H), 1.39 (t, J = 7.0 Hz, 3H), 1.18 (t, J = 7.4 Hz, 3H), 1.12 (t, J = 7.3 Hz, 3H). |
| **P299** | 1-(Cyclopropylsulfonyl)-N-(5-(6-ethoxypyrazin-2-yl)pyridin-2-yl)-4-(2-(ethylsulfonamido)pyrimidin-4-yl)piperidine-4-carboxamide 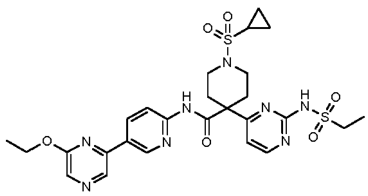 | Prepared as for **P288** using **INTC235** and cyclopropane sulfonyl chloride, [UPLC Acidic], 617, (1.35) | 11.27 (s, 1H), 10.28 (s, 1H), 9.04 (d, J = 2.4 Hz, 1H), 8.84 (s, 1H), 8.61 (d, J = 5.3 Hz, 1H), 8.51 (dd, J = 8.8, 2.5 Hz, 1H), 8.25 (s, 1H), 8.22 (d, J = 8.8 Hz, 1H), 7.23 - 7.20 (m, 1H), 4.48 (q, J = 7.0 Hz, 2H), 3.53 - 3.47 (m, 4H), 3.19 (t, J = 11.0 Hz, 2H), 2.67 - 2.51 (m, 4H), 2.27 - 2.18 (m, 1H), 1.40 (t, J = 7.0 Hz, 3H), 1.12 (t, J = 7.4 Hz, 3H), 0.99 - 0.88 (m, 4H) |
| **P300** | *N*-(5-(6-ethoxypyrazin-2-yl)pyridin-2-yl)-4-(2-(ethylsulfonamido)pyrimidin-4-yl)-1-((1-methyl-1*H*-pyrazol-3-yl)sulfonyl) piperidine-4-carboxamide 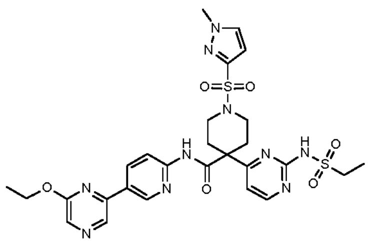 | Prepared as for **P288** using **INTC235** and 1-methyl-1*H*-pyrazole-3-sulfonyl chloride, [UPLC Acidic], 657, (1.30) | 11.22 (s, 1H), 10.24 (s, 1H), 9.01 (dd, J = 2.4, 0.8 Hz, 1H), 8.83 (s, 1H), 8.60 (d, J = 5.3 Hz, 1H), 8.48 (dd, J = 8.7, 2.5 Hz, 1H), 8.25 (s, 1H), 8.10 - 8.05 (m, 1H), 7.90 (d, J = 2.3 Hz, 1H), 7.20 (d, J = 5.3 Hz, 1H), 6.65 (d, J = 2.3 Hz, 1H), 4.47 (q, J = 7.0 Hz, 2H), 3.88 (s, 3H), 3.53 - 3.39 (m, 4H), 2.93 - 2.84 (m, 2H), 2.60 - 2.53 (m, 2H), 2.24 - 2.15 (m, 2H), 1.39 (t, J = 7.0 Hz, 3H), 1.09 (t, J = 7.3 Hz, 3H). |
| **P301** | Single diastereoisomerunassigned 1-(2-(Cyclopropanesulfonamido)pyri midin-4-yl)-N-(5-(6-cyclopropylpyrazin-2-yl) pyridin-2-yl)-4-methoxycyclohexane-1-carboxamide 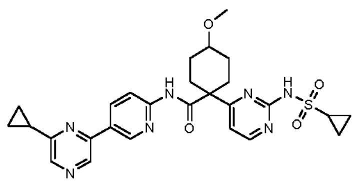 | Method 11, using **INTC218** and **INTD54,** [UPLC Acidic], 549, (1.37) | 11.28 (s, 1H), 10.01 (s, 1H), 9.01 (s, 1H), 8.98 (d, J = 2.4 Hz, 1H), 8.58 (s, 1H), 8.51 - 8.31 (m, 1H), 8.18 (d, J = 8.8 Hz, 1H), 7.17 (s, 1H), 3.24 (s, 3H), 2.78 - 2.53 (m, 2H), 2.33 - 2.15 (m, 1H), 2.03 - 1.77 (m, 4H), 1.55 - 1.33 (m, 2H), 1.22 - 0.98 (m, 6H), 0.91 - 0.75 (m, 2H). 1H not observed, 2H obscured under DMSO peak |

(continued)

| INTC # or P# | Name/Structure (All examples containing chiral centres are racemates unless stated) | Synthesis Method, [LCMS Method], m/z $(M+H)^+$, (Rt/Min) | $^1$H NMR Chemical Shift Data (DMSO-d6 unless stated) |
|---|---|---|---|
| **P302** | Single diastereoisomerunassigned 1-(2-(Cyclopropanesulfonamido)pyri midin-4-yl)-*N*-(5-(6-ethoxypyrazin-2-yl)pyridin-2-yl)-4-(pyrrolidin-1-yl)cyclohexane-1-carboxamide 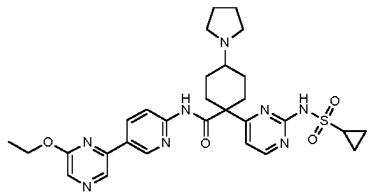 | Prepared as for **P229** using **P226** and pyrrolidine, [UPLC Acidic], 593, (0.91) | 9.56 (s, 1H), 9.01 (d, J = 2.4 Hz, 1H), 8.82 (s, 1H), 8.47 (dd, J = 8.8, 2.6 Hz, 2H), 8.24 (s, 1H), 8.18 (s, 2H), 8.09 (d, J = 8.8 Hz, 1H), 7.10 (s, 1H), 4.46 (q, J = 7.0 Hz, 2H), 3.15 - 3.11 (m, 1H), 2.87 - 2.83 (m, 3H), 2.71 - 2.68 (m, 2H), 2.02 - 1.99 (m, 3H), 1.96 - 1.92 (m, 2H), 1.78 - 1.75 (m, 5H), 1.45 - 1.41 (m, 2H), 1.38 (t, J = 7.0 Hz, 3H), 1.01 - 0.97 (m, 2H), 0.90 - 0.85 (m, 2H). Isolated as formate salt |
| **P303** | Single diastereoisomerunassigned 1-(2-(cyclopropanesulfonamido)pyrim idin-4-yl)-N-(5-(6-ethoxypyrazin-2-yl)pyridin-2-yl)-4-(pyrrolidin-1-yl)cyclohexane-1-carboxamide 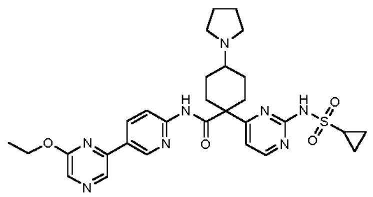 | Prepared as for **P229** using **P226** and pyrrolidine, [UPLC Acidic], 593, (0.85) | 10.19 (s, 1H), 9.04 - 9.00 (m, 1H), 8.84 (s, 1H), 8.52 (d, J = 5.3 Hz, 1H), 8.49 (dd, J = 8.8, 2.5 Hz, 1H), 8.26 - 8.23 (m, 3H), 8.23 - 8.17 (m, 1H), 7.06 (d, J = 5.3 Hz, 1H), 4.47 (q, J = 7.0 Hz, 2H), 3.28 - 3.19 (m, 1H), 2.73 - 2.57 (m, 5H), 2.05 - 1.99 (m, 3H), 1.85 - 1.73 (m, 7H), 1.65 - 1.48 (m, 2H), 1.39 (t, J = 7.0 Hz, 3H), 1.03 - 0.96 (m, 2H), 0.85 - 0.76 (m, 2H). Isolated as formate salt |
| **P304** | Single diastereoisomerunassigned 4-amino-1-(2-(cyclopropanesulfonamido) pyrim idin-4-yl)-N-(5-(6-ethoxypyrazin-2-yl)pyridin-2-yl)cyclohexane-1-carboxamide 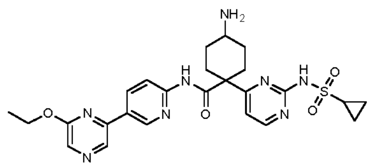 | Prepared as for **P229** using **P226** and ammonium acetate, [UPLC Acidic], 539, (0.78) | 10.22 (s, 1H), 9.05 (d, J = 2.5 Hz, 1H), 8.85 (s, 1H), 8.52 (dd, J = 8.8, 2.5 Hz, 1H), 8.33 (d, J = 5.2 Hz, 1H), 8.31 (s, 1H), 8.26 (s, 1H), 8.22 (d, J = 8.8 Hz, 1H), 6.70 (s, 1H), 4.49 (q, J = 7.1 Hz, 2H), 3.19 - 3.08 (m, 2H), 2.71 - 2.65 (m, 2H), 2.02 - 1.96 (m, 2H), 1.92 - 1.82 (m, 2H), 1.68 - 1.54 (m, 2H), 1.41 (t, J = 7.1 Hz, 3H), 0.91 - 0.86 (m, 2H), 0.71 - 0.65 (m, 2H), 2H not observed Isolated as partial formate salt |
| **P305** | Single diastereoisomerunassigned 1-(2-(cyclopropanesulfonamido)pyrim idin-4-yl)-N-(5-(6-ethoxypyrazin-2-yl)pyridin-2-yl)-4-morpholinocyclohexane-1-carboxamide 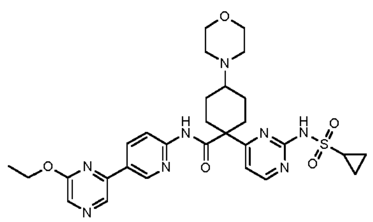 | Prepared as for **P229** using **P226** and morpholine, [UPLC Basic], 609, (1.12) | 9.64 (s, 1H), 9.02 (d, J = 2.4 Hz, 1H), 8.83 (s, 1H), 8.62 (d, J = 5.3 Hz, 1H), 8.47 (dd, J = 8.8, 2.5 Hz, 1H), 8.25 (s, 1H), 8.16 (s, 1H formate), 8.10 (d, J = 8.8 Hz, 1H), 7.32 (d, J = 5.3 Hz, 1H), 4.47 (q, J = 7.0 Hz, 2H), 3.53 (t, J = 4.6 Hz, 4H), 3.30 - 3.21 (m, 1H), 2.71 (d, J = 13.3 Hz, 2H), 2.42 - 2.36 (m, 4H), 2.28 - 2.24 (m, 1H), 2.08 - 1.99 (m, 2H), 1.81 - 1.75 (m, 2H), 1.39 (t, J = 7.0 Hz, 3H), 1.34 - 1.24 (m, 2H), 1.10 (dt, J = 6.5, 3.3 Hz, 2H), 1.04 - 0.94 (m, 2H). 1H not observed. Isolated as formate salt. |

(continued)

| INTC # or P# | Name/Structure (All examples containing chiral centres are racemates unless stated) | Synthesis Method, [LCMS Method], m/z $(M+H)^+$, (Rt/Min) | $^1$H NMR Chemical Shift Data (DMSO-d6 unless stated) |
|---|---|---|---|
| **P306** | Single diastereoisomerunassigned 1-(2-(cyclopropanesulfonamido)pyrim idin-4-yl)-N-(5-(6-ethoxypyrazin-2-yl)pyridin-2-yl)-4-morpholinocyclohexane-1-carboxamide 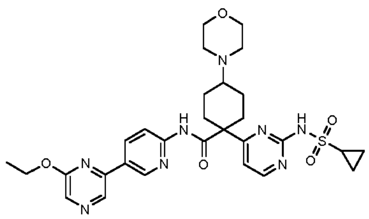 | Prepared as for **P229** using **P226** and morpholine, [UPLC Basic], 609, (1.07) | 10.13, (s, 1H), 9.03 - 8.99 (m, 1H), 8.83 (s, 1H), 8.57 (d, J = 5.3 Hz, 1H), 8.49 (dd, J = 8.8, 2.5 Hz, 1H), 8.25 (s, 1H), 8.23 - 8.19 (m, 1H), 8.15 (s, 1H) formate, 7.16 (d, J = 5.3 Hz, 1H), 4.47 (q, J = 7.0 Hz, 2H), 3.57 - 3.51 (m, 4H), 3.30 - 3.21 (m, 1H), 2.72 - 2.66 (m, 2H), 2.45 (d, J = 5.0 Hz, 4H), 2.30 - 2.22 (m, 1H), 1.80-1.84 (m, 2H), 1.82 - 1.73 (m, 2H), 1.51 - 1.42 (m, 2H), 1.39 (t, J = 7.0 Hz, 3H), 1.04-0.97 (m, 2H), 0.86 - 0.78 (m, 2H). 1H not observed. Isolated as formate salt |
| **P307** | Single diastereoisomerunassigned 1-(2-(cyclopropanesulfonamido)pyrim idin-4-yl)-N-(5-(6-ethoxypyrazin-2-yl)pyridin-2-yl)-4-(methyl(oxetan-3-yl)amino)cyclohexane-1-carboxamide 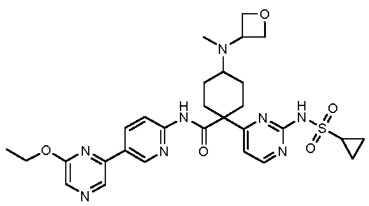 | Prepared as for **P229** using **P226** and N-methyloxetan -3-amine, [UPLC Basic], 609, (1.01) | 10.16 (s, 1H), 9.01 (d, J = 2.5 Hz, 1H), 8.83 (s, 1H), 8.55 (d, J = 5.3 Hz, 1H), 8.50 (dd, J = 8.8, 2.5 Hz, 1H), 8.25 (s, 1H), 8.21 (d, J = 8.8 Hz, 1H), 8.15 (s, 0.4H, partial formate salt), 7.13 (d, J = 5.4 Hz, 1H), 4.51 - 4.42 (m, 6H), 3.91 - 3.82 (m, 1H), 3.29 - 3.19 (m, 1H), 2.71 - 2.65 (m, 2H), 2.43 - 2.30 (m, 1H), 2.06 (s, 3H), 1.77 - 1.68 (m, 2H), 1.68 - 1.62 (m, 2H), 1.52 - 1.47 (m, 1H), 1.47 - 1.38 (m, 3H), 1.40 - 1.36 (m, 1H), 1.04 - 0.97 (m, 2H), 0.83 - 0.76 (m, 2H). 1H not observed. Isolated as partial formate salt |
| **P308** | Single diastereoisomerunassigned 1-(2-(cyclopropanesulfonamido)pyrim idin-4-yl)-N-(5-(6-ethoxypyrazin-2-yl)pyridin-2-yl)-4-((2-methoxyethyl)(methyl)amino)cyc lohexane-1-carboxamide 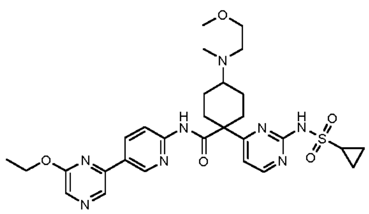 | Prepared as for **P229** using **P226** and 2-methoxy-N-methylethan-1-amine, [UPLC Basic], 611, (0.98) | 10.14 (s, 1H), 9.06 - 9.00 (m, 1H), 8.84 (s, 1H), 8.55 (d, J = 5.4 Hz, 1H), 8.50 (dd, J = 8.7, 2.5 Hz, 1H), 8.30-8.24 (m, 1H), 8.22 (d, J = 8.8 Hz, 1H), 8.19 (s, 1H, formate), 7.13 (d, J = 5.4 Hz, 1H), 4.48 (q, J = 7.0 Hz, 2H), 3.37 (t, J = 6.1 Hz, 2H), 3.29 - 3.22 (m, 1H), 3.22 (s, 3H), 2.77 - 2.64 (m, 2H), 2.61 - 2.53 (m, 1H), 2.21 (s, 3H), 1.88-1.66 (m, 4H), 1.55 - 1.43 (m, 2H), 1.40 (t, J = 7.0 Hz, 3H), 1.05 - 0.95 (m, 2H), 0.85 - 0.77 (m, 2H). 1H not observed. Isolated as formate salt. 2H obscured by DMSO peak. |

(continued)

| INTC # or P# | Name/Structure (All examples containing chiral centres are racemates unless stated) | Synthesis Method, [LCMS Method], m/z $(M+H)^+$, (Rt/Min) | $^1$H NMR Chemical Shift Data (DMSO-d6 unless stated) |
|---|---|---|---|
| **P309** | Single diastereoisomerunassigned 1-(2-(cyclopropanesulfonamido)pyrim idin-4-yl)-N-(5-(6-ethoxypyrazin-2-yl)pyridin-2-yl)-4-((2-methoxyethyl)(methyl)amino)cyc lohexane-1-carboxamide 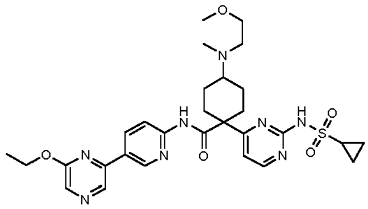 | Prepared as for **P229** using **P226** and 2-methoxy-N-methylethan-1-amine, [UPLC Basic], 611, (1.02) | 9.60 (s, 1H), 9.02 (d, J = 2.5 Hz, 1H), 8.83 (s, 1H), 8.55 (s, 1H), 8.47 (dd, J = 9.0, 2.5 Hz, 1H), 8.25 (s, 1H), 8.18 (s, 1H formate), 8.08 (d, J = 8.8 Hz, 1H), 7.20-7.10 (m, 1H), 4.47 (q, J = 7.0 Hz, 2H), 3.26 (s, 1H), 3.21 (s, 3H), 2.78 - 2.72 (m, 2H), 2.13 (s, 3H), 2.02 - 1.92 (m, 2H), 1.73 - 1.67 (m, 2H), 1.39 (t, J = 7.0 Hz, 3H), 1.28 - 1.22 (m, 2H), 1.06 - 0.97 (m, 2H), 0.96 - 0.78 (m, 2H). 1H not observed. Isolated as formate salt. 5H obscured by DMSO and $H_2O$ peaks |
| **P310** | Single diastereoisomerunassigned 1-(2-(cyclopropanesulfonamido)pyrim idin-4-yl)-4-((2,2-difluoroethyl)(methyl) amino)-N-(5-(6-ethoxypyrazin-2-yl)pyridin-2-yl)cyclohexane-1-carboxamide 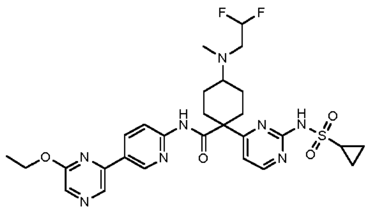 | Prepared as for **P229** using **P226** and 2,2-difluoro-N-methylethan-1-amine, HCl, [UPLC Basic], 617, (1.33) | 10.16 (s, 1H), 9.02 (d, J = 2.5 Hz, 1H), 8.84 (s, 1H), 8.58 - 8.53 (m, 1H), 8.53 - 8.45 (m, 1H), 8.27 - 8.20 (m, 2H), 7.13 (d, J = 5.4 Hz, 1H), 6.14 - 5.86 (m, 1H), 4.48 (q, J = 7.0 Hz, 2H), 3.69 - 3.60 (m, 1H), 3.30 - 3.21 (m, 1H), 2.79 - 2.66 (m, 4H), 2.27 (s, 3H), 1.81 - 1.72 (m, 4H), 1.53 - 1.45 (m, 2H), 1.40 (t, J = 7.0 Hz, 3H), 1.05 - 0.99 (m, 2H), 0.85 - 0.77 (m, 2H). 1H not observed. Isolated as partial formate salt. |
| **P311** | Single diastereoisomerunassigned 1-(2-(cyclopropanesulfonamido)pyrim idin-4-yl)-N-(5-(6-ethoxypyrazin-2-yl)pyridin-2-yl)-4-(4-methylpiperazin-1-yl)cyclohexane-1-carboxamide 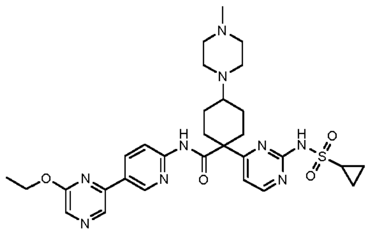 | Prepared as for **P229** using **P226** and 1-methylpipera zine, [UPLC Basic], 622, (0.97) | 9.60 (s, 1H), 9.02 (d, J = 2.5 Hz, 1H), 8.83 (s, 1H), 8.55 (s, 1H), 8.47 (dd, J = 9.0, 2.5 Hz, 1H), 8.25 (s, 1H), 8.18 (s, 1H formate), 8.08 (d, J = 8.8 Hz, 1H), 7.20-7.10 (m, 1H), 4.47 (q, J = 7.0 Hz, 2H), 3.26 (s, 1H), 3.21 (s, 3H), 2.78 - 2.72 (m, 2H), 2.13 (s, 3H), 2.02 - 1.92 (m, 2H), 1.73 - 1.67 (m, 2H), 1.39 (t, J = 7.0 Hz, 3H), 1.28 - 1.22 (m, 2H), 1.06 - 0.97 (m, 2H), 0.96 - 0.78 (m, 2H). 1H not observed. Isolated as formate salt. 5H obscured by DMSO and $H_2O$ peaks |
| **P312** | Single diastereoisomerunassigned 1-(2-(cyclopropanesulfonamido)pyrim idin-4-yl)-N-(5-(6-ethoxypyrazin-2-yl)pyridin-2-yl)-4-(4-methylpiperazin-1-yl)cyclohexane-1-carboxamide 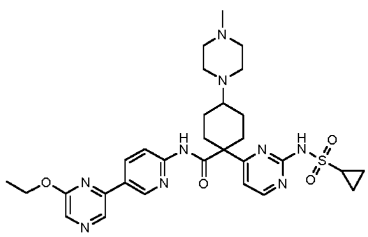 | Prepared as for **P229** using **P226** and 1-methylpipera zine, [UPLC Basic], 622, (0.91) | 10.12 (s, 1H), 9.04 - 9.00 (m, 1H), 8.84 (s, 1H), 8.57 - 8.53 (m, 1H), 8.53 - 8.47 (m, 1H), 8.28 - 8.23 (m, 1H), 8.23 - 8.16 (m, 1.5H) (formate), 7.13 (d, J = 5.4 Hz, 1H), 4.48 (q, J = 7.0 Hz, 2H), 3.28 - 3.21 (m, 1H), 2.72 - 2.66 (m, 2H), 2.42 - 2.25 (m, 8H), 2.16 (s, 3H), 1.84 (d, J = 12.5 Hz, 2H), 1.81 - 1.72 (m, 2H), 1.51 - 1.43 (m, 2H), 1.43 - 1.37 (m, 3H), 1.05 - 0.98 (m, 2H), 0.85 - 0.78 (m, 2H), 1H not observed. Isolated as partial formate salt. 1H obscured by DMSO |

(continued)

| INTC # or P# | Name/Structure (All examples containing chiral centres are racemates unless stated) | Synthesis Method, [LCMS Method], m/z $(M+H)^+$, (Rt/Min) | $^1H$ NMR Chemical Shift Data (DMSO-d6 unless stated) |
|---|---|---|---|
| **P313** | Single diastereoisomerunassigned 1-(2-(cyclopropanesulfonamido)pyrimidin-4-yl)-N-(5-(6-cyclopropylpyrazin-2-yl)pyridin-2-yl)-4-methoxycyclohexane-1-carboxamide 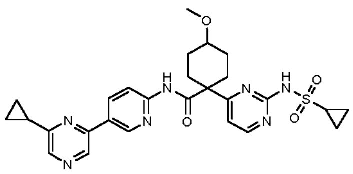 | Method 11, using **INTC218** and **INTD54,** [UPLC Acidic], 550, (1.42) | 11.29 (s, 1H), 9.88 (s, 1H), 9.00 - 8.98 (m, 2H), 8.60 - 8.58 (m, 2H), 8.45 (dd, J = 8.8, 2.5 Hz, 1H), 8.14 (d, J = 8.7 Hz, 1H), 7.20 (s, 1H) 3.22 (s, 3H), 2.38 - 2.34 (m, 2H), 2.32 - 2.18 (m, 3H), 1.82 - 1.75 (m, 2H), 1.58 -1.50 (m, 2H), 1.29 -1.24 (m, 2H), 1.10 -1.05 (m, 5H), 0.92 -0.85 (m, 3H) |
| **P314** | 4-(6-(cyclopropanesulfonamido)pyrazin-2-yl)-N-(5-(6-ethoxypyrazin-2-yl)pyridin-2-yl)-1-(methylsulfonyl)piperidine-4-carboxamide 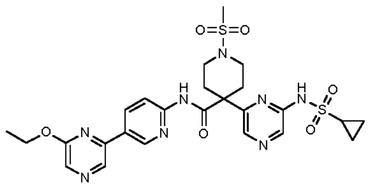 | Prepared as for **P288** using **INTC240** and methanesulfo nyl chloride, [HPLC Acidic], 603, (2.02) | 11.08 (s, 1H), 10.26 (s, 1H), 9.01 (d, J = 2.4 Hz, 1H), 8.83 (s, 1H), 8.49 (dd, J = 8.8, 2.4 Hz, 1H), 8.42 (s, 1H), 8.25 (s, 1H), 8.23 - 8.17 (m, 2H), 4.47 (q, J = 7.0 Hz, 2H), 3.47 - 3.41 (m, 2H), 3.15 - 3.04 (m, 3H), 2.86 (s, 3H), 2.69 - 2.63 (m, 2H), 2.32 - 2.23 (m, 2H), 1.42 - 1.36 (m, 3H), 1.07 - 1.00 (m, 2H), 0.88 - 0.77 (m, 2H). |
| **P315** | 4-(4-(cyclopropanesulfonamido)pyrimidin-2-yl)-N-(5-(6-cyclopropylpyrazin-2-yl)pyridin-2-yl)-1-(methylsulfonyl)piperidine-4-carboxamide 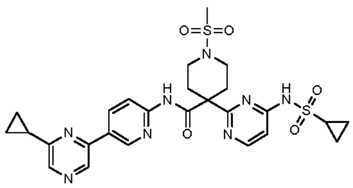 | Prepared as for **P288** using **INTC245** and methanesulfo nyl chloride, [HPLC Acidic], 599, (2.06) | 11.35 (s, 1H), 10.06 (s, 1H), 9.03 - 8.96 (m, 2H), 8.59 (d, J = 5.7 Hz, 2H), 8.47 (dd, J = 8.8, 2.4 Hz, 1H), 8.22 (d, J = 8.7 Hz, 1H), 6.87 (d, J = 5.7 Hz, 1H), 3.37 - 3.32 (m, 1H), 3.22 - 3.13 (m, 4H), 2.85 (s, 3H), 2.49 - 2.38 (m, 4H), 2.30 - 2.22 (m, 1H), 1.21 - 1.02 (m, 6H), 0.99 - 0.91 (m, 2H). |
| **P316** | 4-(4-(cyclopropanesulfonamido)pyrimidin-2-yl)-N-(5-(6-ethoxypyrazin-2-yl)pyridin-2-yl)-1-(methylsulfonyl)piperidine-4-carboxamide 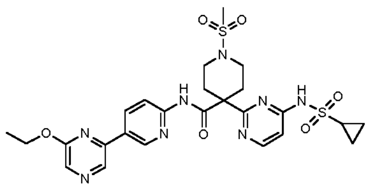 | Prepared as for **P288** using **INTC247** and methanesulfo nyl chloride, [UPLC Acidic], 603, (1.33) | 11.35 (s, 1H), 10.08 (s, 1H), 9.02 (d, J = 2.4 Hz, 1H), 8.84 (s, 1H), 8.59 (d, J = 5.7 Hz, 1H), 8.53 - 8.47 (m, 1H), 8.27 - 8.20 (m, 2H), 6.88 (d, J = 5.7 Hz, 1H), 4.48 (q, J = 7.0 Hz, 2H), 3.34 - 3.30 (m, 2H), 3.22 - 3.14 (m, 3H), 2.85 (s, 3H), 2.50 - 2.38 (m, 4H), 1.40 (t, J = 7.0 Hz, 3H), 1.17 = 1.05 (m, 2H), 0.99 - 0.91 (m, 2H). |

(continued)

| INTC # or P# | Name/Structure (All examples containing chiral centres are racemates unless stated) | Synthesis Method, [LCMS Method], m/z (M+H)+, (Rt/Min) | $^1$H NMR Chemical Shift Data (DMSO-d6 unless stated) |
|---|---|---|---|
| **P317** | 4-(4-(cyclopropanesulfonamido)pyrimidin-2-yl)-N-(5-(6-cyclopropylpyrazin-2-yl)pyridin-2-yl)-1-(ethylsulfonyl)piperidine-4-carboxamide 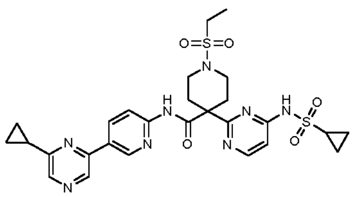 | Prepared as for **P288** using **INTC245** and ethanesulfon yl chloride, [HPLC Acidic], 613, (2.15) | 11.35 (s, 1H), 10.06 (s, 1H), 9.00 (s, 1H), 8.98 (d, J = 2.4 Hz, 1H), 8.61 - 8.56 (m, 2H), 8.47 (dd, J = 8.8, 2.4 Hz, 1H), 8.21 (d, J = 8.8 Hz, 1H), 6.87 (d, J = 5.7 Hz, 1H), 3.41 - 3.34 (m, 2H), 3.29 - 3.21 (m, 2H), 3.21 - 3.12 (m, 1H), 3.04 (q, J = 7.3 Hz, 2H), 2.48 - 2.34 (m, 4H), 2.30 - 2.22 (m, 1H), 1.19 (t, J = 7.3 Hz, 3H), 1.16 - 1.06 (m, 6H), 0.98 - 0.90 (m, 2H). |
| **P318** | 4-(4-(cyclopropanesulfonamido)pyrimidin-2-yl)-N-(5-(6-ethoxypyrazin-2-yl)pyridin-2-yl)-1-(ethylsulfonyl)piperidine-4-carboxamide 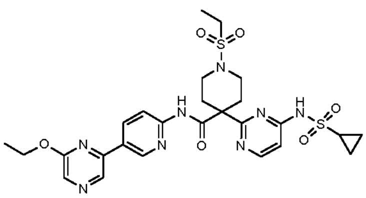 | Prepared as for **P288** using **INTC247** and ethanesulfon yl chloride, [HPLC Acidic], 617, (2.16) | 11.35 (s, 1H), 10.07 (s, 1H), 9.02 (d, J = 2.4 Hz, 1H), 8.84 (s, 1H), 8.59 - 8.55 (m, 1H), 8.50 (dd, J = 8.7, 2.4 Hz, 1H), 8.25 (s, 1H), 8.22 (d, J = 8.8 Hz, 1H), 6.86 (d, J = 5.9 Hz, 1H), 4.48 (q, J = 7.0 Hz, 2H), 3.43 - 3.21 (m, 4H), 3.20 - 3.12 (m, 1H), 3.04 (q, J = 7.3 Hz, 2H), 2.47 - 2.37 (m, 4H), 1.40 (t, J = 7.0 Hz, 3H), 1.19 (t, J = 7.3 Hz, 3H), 1.09 - 1.05 (m, 2H), 0.96 - 0.91 (m, 2H). |

## Compounds of formula (I-b) - Intermediates

The synthesis of intermediates **INTA1** to **INTA117** and **INTB1** to **INTB120** is disclosed in WO2019/106156, and uses the general methods shown below.

## Method A: Formation of thiazole amines from ketoesters

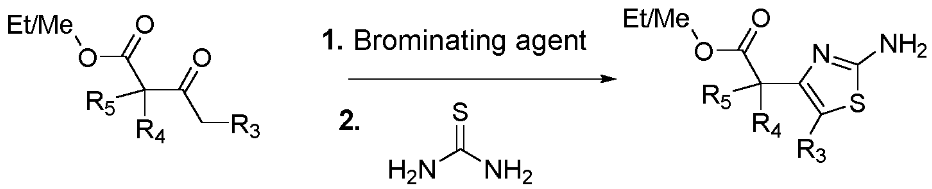


To a solution of ketoester (1 eq) in an alcoholic solvent such as MeOH or EtOH (1 volume) at 0 °C was added bromine (1.5 eq) dropwise over 10 mins. The reaction was stirred at 0 °C for 10 mins. The reaction mixture was then heated at 30 °C for 2 hrs. After cooling to RT the reaction mixture was diluted with water. The product was extracted using an appropriate solvent such as EtOAc. The combined organics were dried ($Na_2SO_4$) and concentrated in *vacuo.* The resulting compound was dissolved in alcoholic solvent such as MeOH or EtOH (1 volume) and thiourea (1 eq) was added. The reaction mixture was heated at 40 °C for 1 hr, then stirred at RT for 18 hrs. The reaction mixture was concentrated in *vacuo* and purified by normal phase chromatography or *via* trituration with an appropriate solvent.

## Method B: Formation of sulfonamides from sulfonyl chlorides

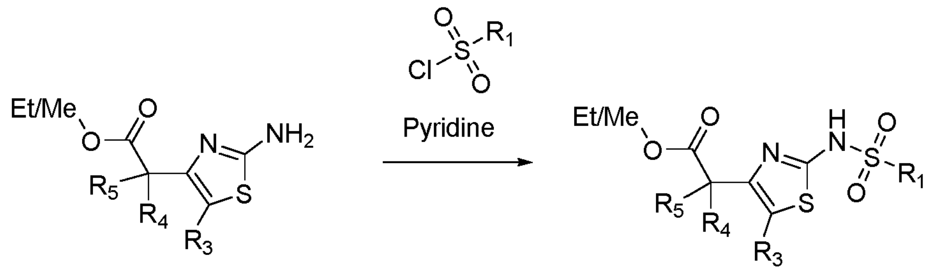


A solution of amine (1.0 eq) and appropriate sulfonyl chloride (1.1 eq) in pyridine (3M volumes) was warmed to 40 °C and stirred for 18 hrs. The reaction mixture was purified by normal or reverse phase chromatography or *via* trituration with an appropriate solvent.

**Method C: Formation of sulfonamides from heterocyclic halides**

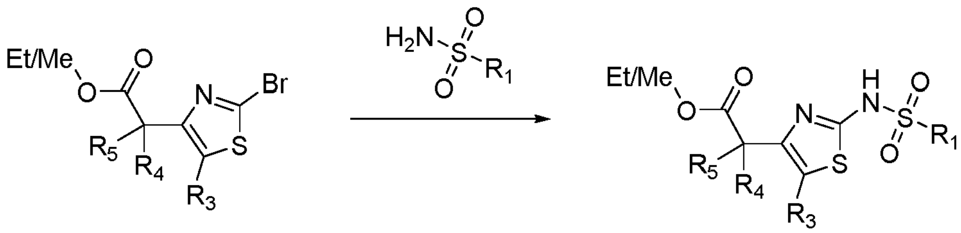

A suspension of (1*R*,2*R*)-*N*1,*N*2-dimethylcyclohexane-1,2-diamine (0.2 eq), bromothiazole intermediate (1 eq), alkylsulfonamide (1 eq), and $K_2CO_3$ (1.1 eq) in dioxane (10 volumes) at 40 °C was degassed ($N_2$, 5 mins) then copper(I) iodide (0.1 equiv.) was added. The solution was again degassed ($N_2$, 5 mins) before being warmed to 80 °C. The reaction was progressed for 2 hrs before being allowed to cool to RT. 1M HCl (aq) was added and the aqueous phase was extracted with EtOAc. The organic phases were combined, dried ($Na_2SO_4$), filtered and concentrated. The crude material was purified by normal phase chromatography.

**Method D: Formation of acids from esters**

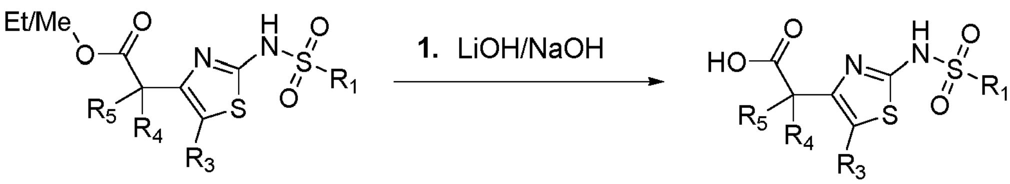


To a solution of ester (1.0 eq.) in THF/MeOH (2:1) was added a solution of appropriate base (2.0 eq. of LiOH or NaOH aq solutions at 1-2M). The reaction was stirred at RT or with heating up to 50 °C. The reaction was concentrated in *vacuo* to half volume and was acidified with 1M HCl. The product was extracted using an appropriate organic solvent (EtOAc). The combined organics were dried ($Na_2SO_4$) and concentrated in *vacuo* to give the desired compound.

**Aniline intermediate preparation**

**Method E: Suzuki coupling of halo anilines with heteroaromatic boronates**

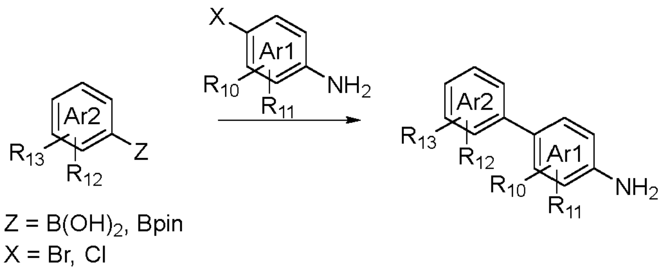


A solution of Ar1-X (1 eq) and Ar2-Z (1 eq) in solvent (3 volumes) and base (2.5 eq) was degassed ($N_2$, 5 min) and heated to 40 °C whereupon Pd catalyst (3 mol %) was added and the reaction mixture further degassed ($N_2$, 5 min) before being heated to 90 °C for 90 mins. The reaction mixture was allowed to cool to RT. In general, the desired compound is purified by column chromatography.

**Method F: Suzuki coupling of heteroaromatic halides with aniline boronates**

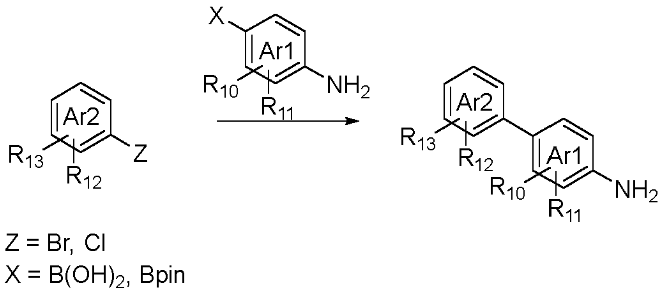


Pd catalyst (5 mol %) was added to a degassed ($N_2$, 5 mins) solution of Ar1-X (1 eq), Ar2-Z (1 eq) and base (3 eq, 6.85 mmol) in solvent (3 volumes). The solution was then degassed further ($N_2$, 5 mins) and then heated to 90 °C for 2 hrs then allowed to cool to RT. In general, the desired compound is purified by column chromatography.

**Method G: Telescoped boronate formation and Suzuki coupling**

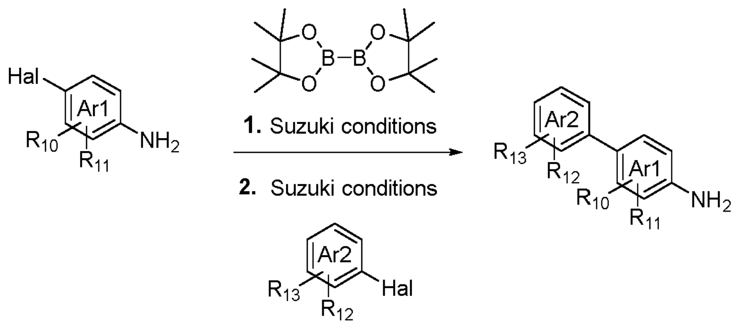


Bispin (1.1 eq) and KOAc (4 eq) were added to Ar1-Hal (1 eq) in dioxane (5 volumes). The reaction was heated to 60 °C and degassed ($N_2$, 5 mins). $PdCl_2$(dppf)-DCM adduct (5 mol %) was added to the reaction mixture and the temperature was increased to 90 °C for 1 hr. The reaction mixture was then cooled to RT and a solution of Ar2-Hal (1 eq) in dioxane (3 volumes) was added, followed by a solution of $K_2CO_3$ (4 eq) in water (2 volumes). The temperature was then increased to 90 °C for 18 hrs. The reaction was cooled to RT, an aqueous work up was performed and the crude compound was purified by normal phase chromatography.

**Method H: Suzuki coupling of halo pyrimidines with heteroaromatic boronates**

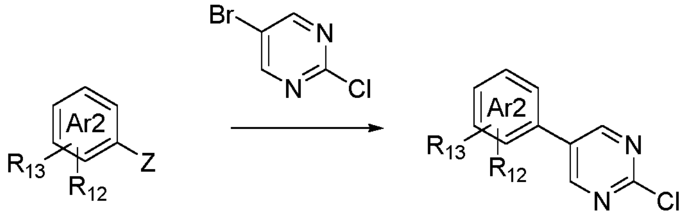

Z = B(OH)$_2$, Bpin

A solution of 5-bromo-2-chloropyrimidine (1.2 eq) and Ar2-Z (1 eq) in solvent (3 volumes) and base (4 eq) was degassed ($N_2$, 5 min) and heated to 40 °C whereupon Pd catalyst (5 mol%) was added and the reaction mixture further degassed ($N_2$, 5 min) before being heated to 90 °C for up to 24 hrs. The reaction mixture was allowed to cool to RT and an aqueous work-up was performed. In general, the desired compound is purified by column chromatography.

**Compounds of formula (I-b) - Examples**

The synthesis of a number of known CTPS1 inhibitors is disclosed in WO2019/106156 (see compounds **T1** to **T465).** Such compounds are made using general methods disclosed herein and represent further examples of compounds which are CTPS1 inhibitors. The full synthetic methods and characterising data for compounds **T1** to **T465** are provided in WO2019/106156.

**Amide couplings**

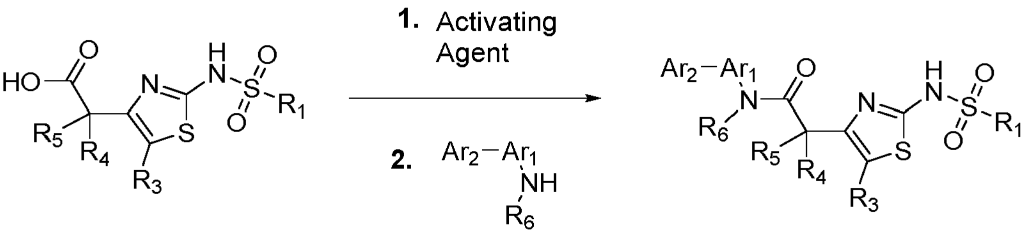


**Method 1:** HATU (1.2 eq) was added to a solution of appropriate acid (1 eq), amine (1 eq) and DIPEA (3 eq) in DCM (10 volumes) at RT. The reaction was stirred at RT for 18 hrs. The solvent was removed and the crude product was purified by normal phase chromatography, reverse phase chromatography or trituration from an appropriate solvent.

**Method 1b:** 1-chloro-N,N,2-trimethylprop-1-en-1-amine (2 eq) was added to a solution of 2-(2-(cyclopropanesulfonamido)thiazol-4-yl)-2-methylpropanoic acid (1 eq) in DCM (20 volumes). The reaction mixture was stirred at RT for 2 hrs. The reaction mixture was concentrated in *vacuo* and the residue redissolved in pyridine (2 mL) before addition of the appropriate amine (1.1 eq). The reaction mixture was stirred at RT for 2 hrs. An aqueous work up was performed and the crude product was purified by normal phase chromatography, reverse phase chromatography or trituration from an appropriate solvent.

**Method 1c:** T3P (50% in EtOAc, 2.5 eq) was added to a solution of appropriate acid (1 eq), amine (1 eq) and pyridine (3 eq) in a mixture of EtOAc (20 volumes) and DMF (10 volumes). The reaction was stirred for 1 hr at RT. An aqueous work up was performed and the crude product was purified by normal phase chromatography, reverse phase chromatography or trituration from an appropriate solvent.

**Method 1e:** Thionyl chloride (2 eq) was added to a solution of an appropriate acid (1 eq) in toluene (20 volumes) at 70 °C. The reaction mixture was stirred at 70 °C for 1 hr. The reaction mixture was cooled to RT and concentrated to dryness. The resulting intermediate was redissolved in EtOAc (10 volumes) and a solution of amine (1.1 eq) in EtOAc (20 volumes) was added followed by triethylamine (2 eq). The reaction mixture was stirred at 40 °C for 16 hrs. An aqueous work up was performed and the crude product was purified by normal phase chromatography, reverse phase chromatography or trituration from an appropriate solvent.

**Suzuki couplings**

**Method 2: Suzuki coupling of Ar1-Bromide with heteroaromatic boronates**

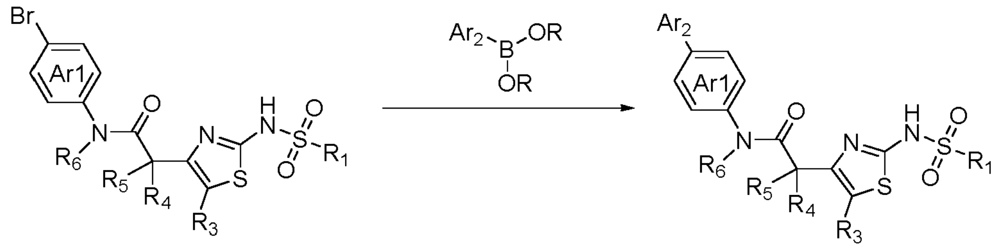

To a suspension of Ar1-Br (1 eq) in dioxane (10 volumes) was added arylboronic acid or ester (1 eq) and 2M $K_2PO_4$ (2 eq). The resulting suspension was heated to 60 °C and degassed ($N_2$, 5 mins). Pd 170 or other appropriate catalyst (5 mol %) was added and the reaction mixtures were stirred at 60 °C for 16 hrs. The reaction mixture was then cooled to RT. An aqueous work up was performed and the crude product was purified by normal phase chromatography, reverse phase chromatography or trituration from an appropriate solvent.

**Method 2b: Suzuki coupling of Ar1-B(OR)$_2$ with heteroaromatic halides**

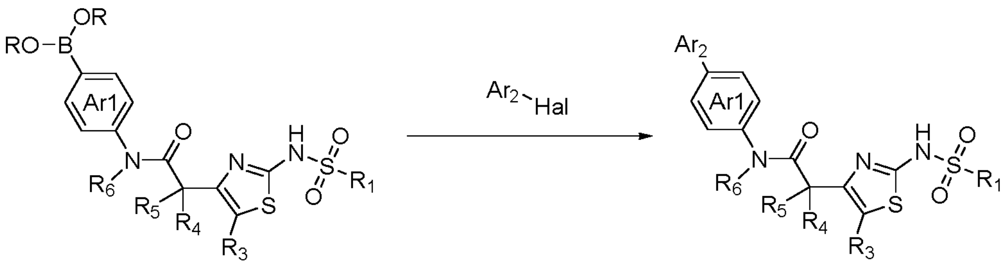

$PdCl_2$(dppf)-$CH_2Cl_2$ (10 mol %) or other appropriate catalyst was added to a degassed ($N_2$, 5 mins) solution of Ar1-B(OR)$_2$ (1 eq), Ar2-halide (1.2 eq) and $K_2CO_3$ (5 eq) in dioxane (10 volumes) and water (15 volumes). The solution was then degassed further ($N_2$, 5 mins) and then heated to 90 °C for 1-2 hrs. The reaction mixture was allowed to cool to RT. An aqueous work up was performed and the crude product was purified by normal phase chromatography, reverse phase chromatography or trituration from an appropriate solvent.

**Method 3: Coupling of primary amides with 2-chloropyrimidines**

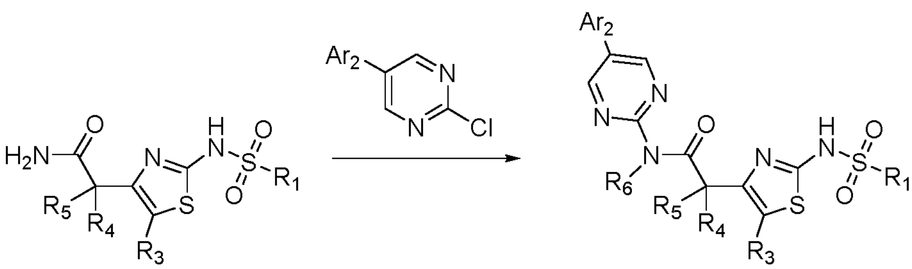

To a solution of amide (1 eq) and 2-chloropyrimidine (1 eq) in dioxane (30 volumes) was added $Cs_2CO_3$ (1.5 eq). The reaction mixture was heated to 60 °C and degassed ($N_2$, 5 mins). Pd 177 (10 mol %) was added to the reaction mixture and the temperature was increased to 90 °C. After 2 hrs, the reaction was stirred for 16 hrs at 60 °C. The reaction mixture was cooled to RT and an aqueous work up was performed. The crude product was purified by normal phase chromatography, reverse phase chromatography or trituration from an appropriate solvent.

Reference Example: N-([1,1'-biphenyl]-4-yl)-2-(2-(methylsulfonamido)thiazol-4-yl)acetamide **T1**

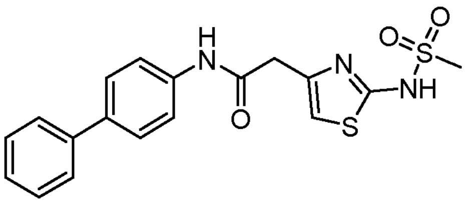

Reference Example: N-([1,1'-biphenyl]-4-yl)-2-(2-(cyclopropanesulfonamido)thiazol-4-yl)acetamide **T2**

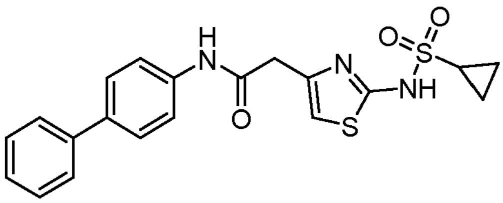

Reference Example: 2-(2-(cyclopropanesulfonamido)thiazol-4-yl)-2-ethyl-N-(5-(pyrazin-2-yl)pyridin-2-yl)butanamide **T3**

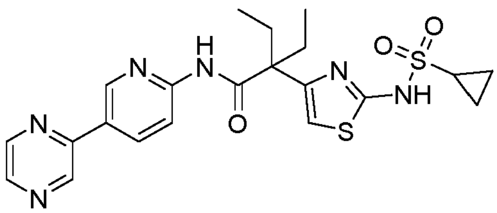

Prepared as **Method 1b** from **INTB41** and 5-(pyrazin-2-yl)pyridin-2-amine (Cheng et al., 2016).

Reference Example: 2-(2-(cyclopropanesulfonamido)thiazol-4-yl)-2-methyl-N-(4-(pyrimidin-2-yl)phenyl)propanamide **T4**

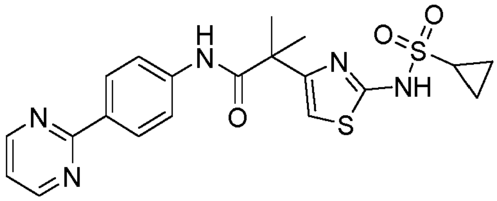

Reference Example: 2-(2-(cyclopropanesulfonamido)thiazol-4-yl)-N-(4-(pyridin-3-yl)phenyl)butanamide **T5**

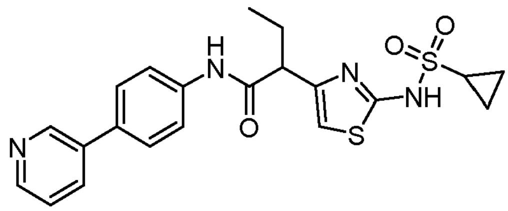

The racemate T5 (180 mg) was separated by chiral preparative HPLC (Gilson, iso-hexane + 0.2% TFA: DCM, 4:1 with EtOH 30%). A salt exchange (TFA to HCl) was undertaken by adding 1.25M HCl (EtOH, 2 mL x 5) and removing solvent to afford:

**Peak A: Stereochemistry of product was not assigned**

2-(2-(cyclopropanesulfonamido)thiazol-4-yl)-N-(4-(pyridin-3-yl)phenyl)butanamide.HCl **T6.**

**Peak B: Stereochemistry of product was not assigned**

Reference Example: 2-(2-(cyclopropanesulfonamido)thiazol-4-yl)-N-(4-(pyridin-3-yl)phenyl)butanamide.HCl **T7.**

Reference Example: N-(4-(5-chloropyridin-3-yl)phenyl)-2-(2-(cyclopropanesulfonamido)thiazol-4-yl)butanamide **T8**

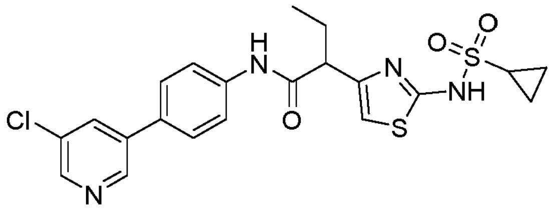

Prepared as **Method 1** from **INTB38.**

The racemate **T8** was separated by chiral preparative HPLC (30% EtOH vs 4:1 isoehexanes +0.2%TFA:DCM IA column). A salt exchange (TFA to HCl) was undertaken by adding 1.25M HCl (EtOH, 2 mL x 5) and removing solvent to afford:

**Peak A: Stereochemistry of product was not assigned**

N-(4-(5-chloropyridin-3-yl)phenyl)-2-(2-(cyclopropanesulfonamido)thiazol-4-yl)butanamide.HCl.

**Peak B: Stereochemistry of product was not assigned**

Reference Example: N-(4-(5-chloropyridin-3-yl)phenyl)-2-(2-(cyclopropanesulfonamido)thiazol-4-yl)butanamide.HCl **T10.**

Reference Example: 2-(2-(cyclopropanesulfonamido)thiazol-4-yl)-3-methyl-N-(4-(pyrimidin-5-yl)phenyl)butanamide **T11**

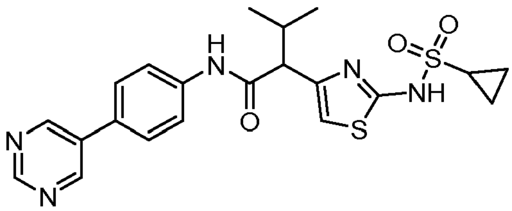

Prepared as **Method 1** from **INTB40.**

Reference Example: 2-(2-(cyclopropanesulfonamido)thiazol-4-yl)-3-methyl-N-(4-(pyridin-3-yl)phenyl)butanamide **T12**

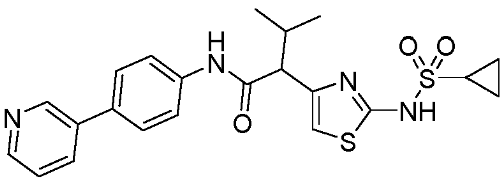

Prepared as **Method 1** from **INTB40** and 4-(pyridin-3-yl)aniline (Xing-Li et al, 2009)
The racemate **T12** was separated by chiral preparative HPLC (Gilson, iso-hexane + 0.2% TFA: DCM, 4:1 with EtOH 30%). A salt exchange (TFA to HCl) was undertaken by adding 1.25M HCl (EtOH, 2 mL x 5) and removing solvent to afford:

**Peak A: Stereochemistry of product was not assigned**

Reference Example: 2-(2-(cyclopropanesulfonamido)thiazol-4-yl)-3-methyl-N-(4-(pyridin-3-yl)phenyl)butanamide.HCl **T13.**

**Peak B: Stereochemistry of product was not assigned**

Reference Example: 2-(2-(cyclopropanesulfonamido)thiazol-4-yl)-3-methyl-N-(4-(pyridin-3-yl)phenyl)butanamide.HCl **T14.**

Reference Example: 2-(2-(cyclopropanesulfonamido)thiazol-4-yl)-N-(4-(5-methoxypyridin-3-yl)phenyl)-2-methylpropanamide **T15**

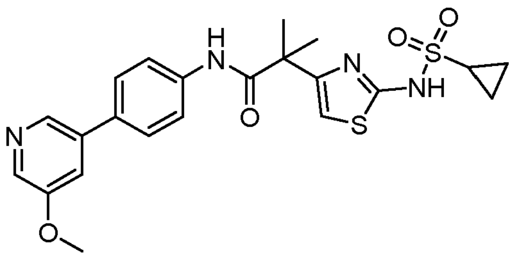

Reference Example: N-(2-chloro-4-(pyridin-3-yl)phenyl)-2-(2-(cyclopropanesulfonamido)thiazol-4-yl)acetamide **T16**

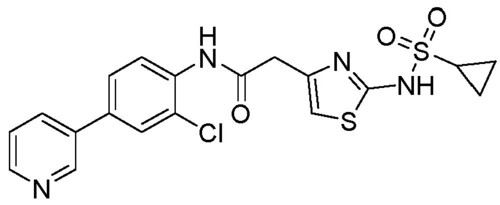

Reference Example: 2-(2-(cyclopropanesulfonamido)-5-methylthiazol-4-yl)-2-methyl-N-(4-(6-(trifluoromethyl)pyrazin-2-yl)phenyl)propanamide **T17**

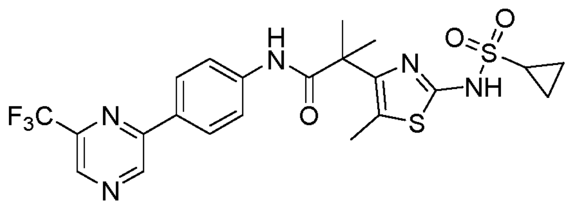

Reference Example: 2-(2-(cyclopropanesulfonamido)thiazol-4-yl)-2-methyl-N-(4-(pyrimidin-5-yl)phenyl)propanamide **T18**

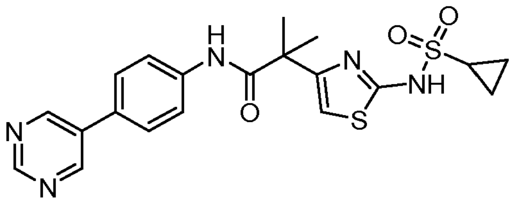

Reference Example: 6-(4-(2-(2-(cyclopropanesulfonamido)thiazol-4-yl)-2-methylpropanamido)phenyl)-N,N-dimethylpyrazine-2-carboxamide **T19**

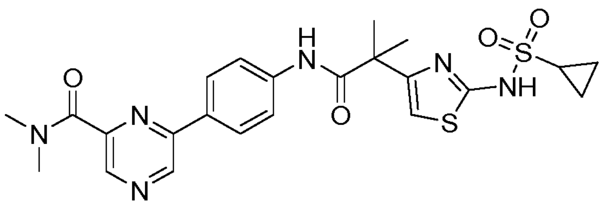

Reference Example: N-(5-(5-cyanopyridin-3-yl)pyrimidin-2-yl)-2-(2-(cyclopropanesulfonamido)thiazol-4-yl)-2-methylpropanamide **T20**

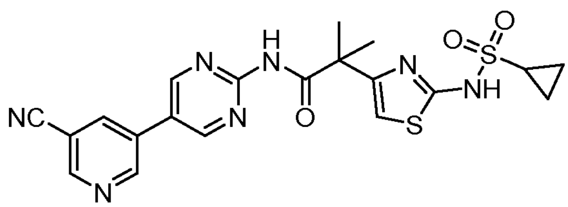

Reference Example: N-([1,1'-biphenyl]-4-yl)-2-(5-chloro-2-(cyclopropanesulfonamido)thiazol-4-yl)acetamide **T21**

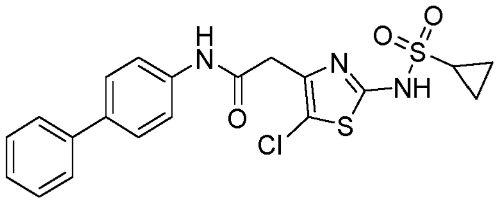

Reference Example: 2-(2-(cyclopropanesulfonamido)thiazol-4-yl)-N-(4-(6-ethynylpyrazin-2-yl)phenyl)butanamide **T22**

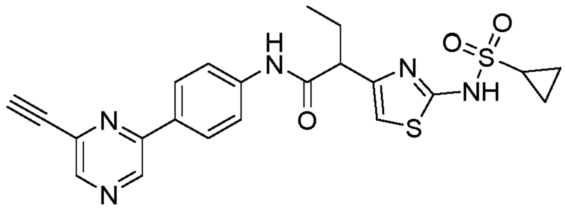

**Table 6:** Reference Examples (Ref): Compounds **T23-T322, T422-T443** and **T445-T465.**

| T# | Name/Structure (All examples containing chiral centres are racemates unless stated) | T# | Name/Structure (All examples containing chiral centres are racemates unless stated) |
|---|---|---|---|
| **Ref T23** | 2-(2-(cyclopropanesulfonamido)thiazol-4-yl)-N-(6-(pyrimidin-5-yl)pyridin-3-yl)acetamide 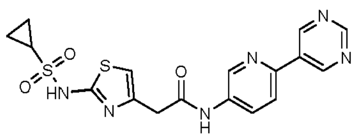 | **Ref T197** | 2-(2-(cyclopropanesulfonam ido)thiazol-4-yl)-N-(2-fluoro-4-(6-(trifluoromethyl)pyrazin-2-yl)phenyl)butanamide 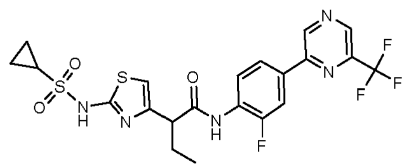 |
| **Ref T24** | 2-(2-(cyclopropanesulfonamido)thiazol-4-yl)-N-(5-phenylpyridin-2-yl)acetamide 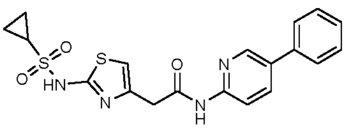 | **Ref T198** | 2-(2-(cyclopropanesulfonam ido)thiazol-4-yl)-N-(4-(6-ethoxypyrazin-2-yl)-2-fluorophenyl)butanamide 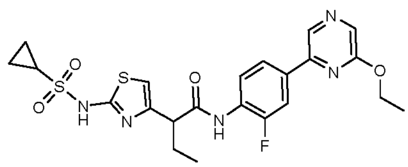 |
| **Ref T25** | 2-(2-(cyclopropanesulfonamido)thiazol-4-yl)-N-(4'-fluoro-[1,1'-biphenyl]-4-yl)acetamide | **Ref T199** | 2-(2-(cyclopropanesulfonam ido)thiazol-4-yl)-N-(4-(5-ethoxypyridin-3-yl)-2-fluorophenyl)butanamide |

(continued)

| T# | Name/Structure (All examples containing chiral centres are racemates unless stated) | T# | Name/Structure (All examples containing chiral centres are racemates unless stated) |
|---|---|---|---|
| | 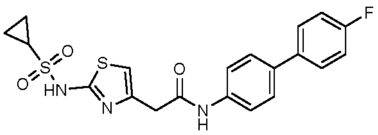 | | 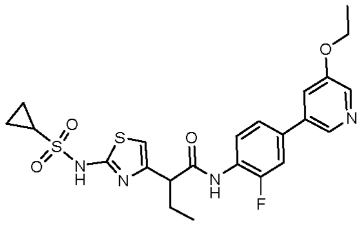 |
| **Ref T26** | 2-(2-(cyclopropanesulfonamido)thiazol-4-yl)-N-methyl-N-(4-(pyridin-3-yl)phenyl)acetamide 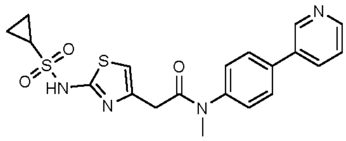 | **Ref T200** | 2-(2-(cyclopropanesulfonam ido)thiazol-4-yl)-N-(2-fluoro-4-(5-fluoropyridin-3-yl)phenyl)butanamide 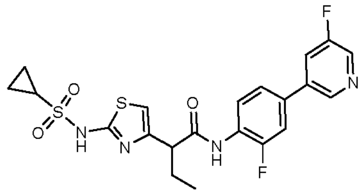 |
| **Ref T27** | 2-([2,3'-bipyridin]-5-yl)-2-(2-(cyclopropanesulfonamido)thiazol-4-yl)propanamide 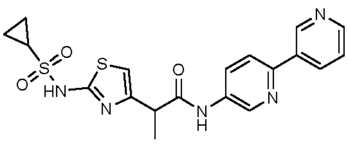 | **Ref T201** | 2-(2-(cyclopropanesulfonamido)thiazol-4-yl)-N-(2-fluoro-4-(pyridin-3-yl)phenyl)butanamide 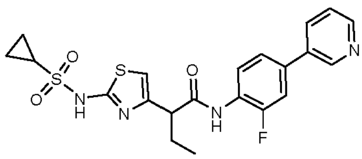 |
| **Ref T28** | 2-(2-(cyclopropanesulfonamido)thiazol-4-yl)-N-(3'-methoxy-[1,1'-biphenyl]-4-yl)-2-methylpropanamide 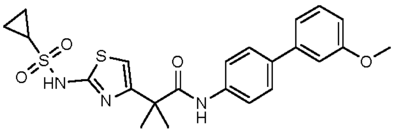 | **Ref T202** | N-(4-(5-cyanopyridin-3-yl)-2-fluorophenyl)-2-(2-(cyclopropanesulfonam ido)thiazol-4- 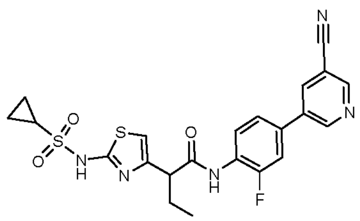 yl)butanamide |
| **Ref T29** | 2-(2-(cyclopropanesulfonamido)thiazol-4-yl)-2-methyl-N-(4-(pyridin-3- 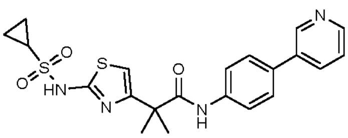 yl)phenyl)propanamide | **Ref T203** | N-(4-(5-chloropyridin-3-yl)-2-fluorophenyl)-2-(2-(cyclopropanesulfonam ido)thiazol-4-yl)butanamide 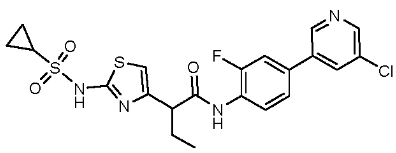 |
| **Ref T30** | 2-(2-(cyclopropanesulfonamido)thiazol-4-yl)-2-methyl-N-(4-(5-methylpyridin-3-yl)phenyl)propanamide | **Ref T204** | **Single enantiomer** - **stereochemistry unassigned** 2-(2-(cyclopropanesulfonam ido) thiazol-4-yl)-N-(4-(6-ethoxypyrazin-2-yl) phenyl)butanamide |

(continued)

| T# | Name/Structure (All examples containing chiral centres are racemates unless stated) | T# | Name/Structure (All examples containing chiral centres are racemates unless stated) |
|---|---|---|---|
| | 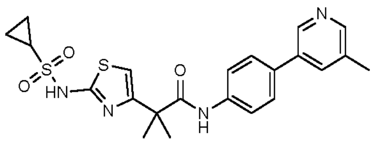 | | 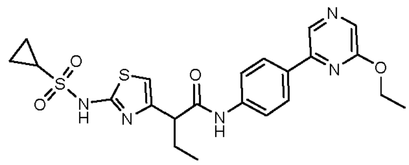 |
| **Ref T31** | 2-(2-(cyclopropanesulfonamido)thiazol-4-yl)-2-methyl-N-(4-(pyridazin-4-yl)phenyl)propanamide<br>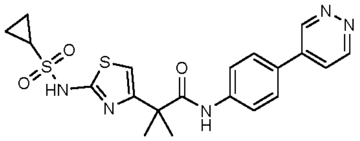 | **Ref T205** | **Single enantiomer - stereochemistry unassigned** 2-(2-(cyclopropanesulfonamido) thiazol-4-yl)-N-(4-(6-ethoxypyrazin-2-yl) phenyl)butanamide<br>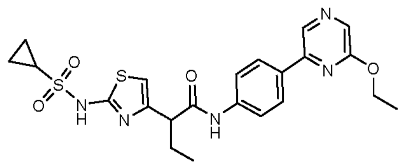 |
| **Ref T32** | 2-(2-(cyclopropanesulfonamido)thiazol-4-yl)-2-methyl-N-(4-(pyrazin-2-yl)phenyl)propanamide<br>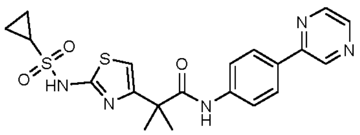 | **Ref T206** | N-(4-(1-(5-(6-ethoxypyrazin-2-yl)indolin-1-yl)-1-oxobutan-2-yl)thiazol-2-yl)cyclopropanesulfonamide<br>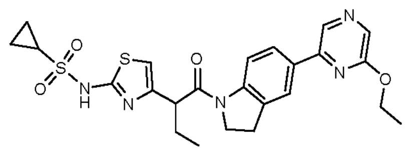 |
| **Ref T33** | 2-(2-(cyclopropanesulfonamido)thiazol-4-yl)-N-(4-(6-methoxypyrazin-2-yl)phenyl)butanamide<br>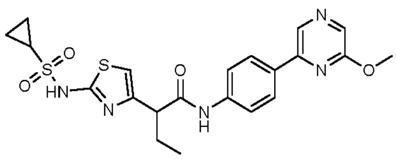 | **Ref T207** | 2-(2-(cyclopropanesulfonam ido)thiazol-4-yl)-N-(2-fluoro-4-(6-(2,2,2-trifluoroethoxy) pyrazin-2-yl)phenyl)butanamide<br>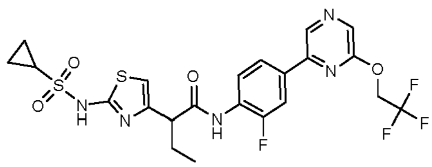 |
| **Ref T34** | N-(3-cyano-4-(pyrazin-2-yl)phenyl)-2-(2-(cyclopropanesulfonamido)thiazol-4-yl)-2-methylpropanamide<br>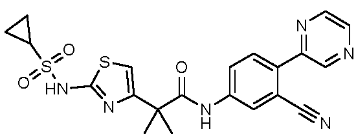 | **Ref T208** | 2-(2-(cyclopropanesulfonam ido)thiazol-4-yl)-N-(2-fluoro-4-(6-methoxypyrazin-2-yl) phenyl)butanamide<br>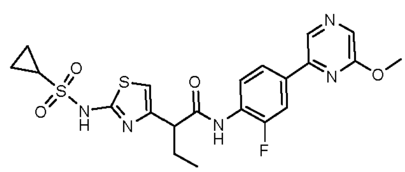 |
| **Ref T35** | 2-(2-(cyclopropanesulfonamido)thiazol-4-yl)-2-methyl-N-(5-(6-(trifluoromethyl)pyrazin-2-yl)pyridin-2-yl)propanamide<br>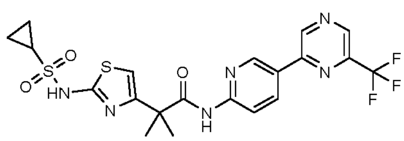 | **Ref T209** | 2-(2-(cyclopropanesulfonam ido)thiazol-4-yl)-N-(4-(5-(difluoromethoxy)pyridin-3-yl)-2-fluorophenyl)butanamide<br>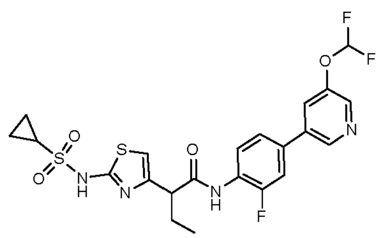 |

(continued)

| T# | Name/Structure (All examples containing chiral centres are racemates unless stated) | T# | Name/Structure (All examples containing chiral centres are racemates unless stated) |
|---|---|---|---|
| **Ref T36** | 2-(2-(cyclopropanesulfonamido)thiazol-4-yl)-N-(2,3-difluoro-4-(pyridin-3-yl)phenyl)-2-methylpropanamide 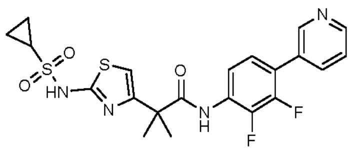 | **Ref T210** | 2-(2-(cyclopropanesulfonam ido)thiazol-4-yl)-N-(4-(5-(difluoromethoxy)pyridin-3-yl)phenyl)butanamide 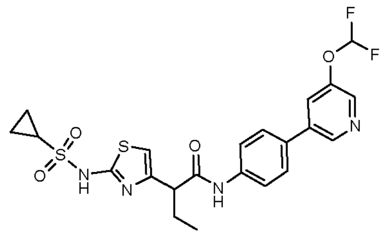 |
| **Ref T37** | 2-(2-(cyclopropanesulfonamido)thiazol-4-yl)-2-methyl-N-(5-(pyridin-3-yl)pyrimidin-2-yl)propanamide 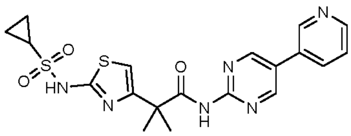 | **Ref T211** | 2-(2-(cyclopropanesulfonam ido)thiazol-4-yl)-N-(4-(5-(2,2,2-trifluoroethoxy)pyridin-3-yl)phenyl)butanamide 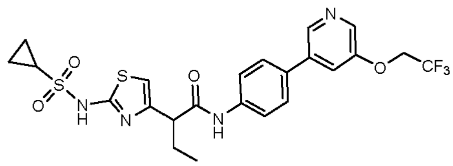 |
| **Ref T38** | 2-(2-(cyclopropanesulfonamido)thiazol-4-yl)-2-methyl-N-(5-(6-propoxypyrazin-2-yl)pyridin-2-yl)propanamide 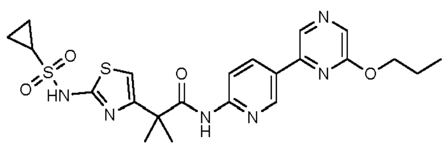 | **Ref T212** | 2-(2-(cyclopropanesulfonamido)thiazol-4-yl)-N-(2-fluoro-4-(5-(2,2,2-trifluoroethoxy)pyridin-3-yl)phenyl)butanamide 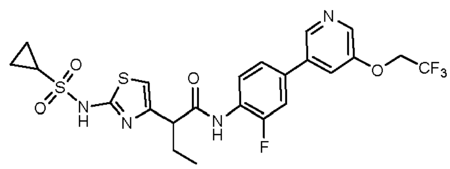 |
| **Ref T39** | 2-(2-(cyclopropanesulfonamido)thiazol-4-yl)-N-(3-fluoro-4-(pyrazin-2-yl)phenyl)-2-methylpropanamide 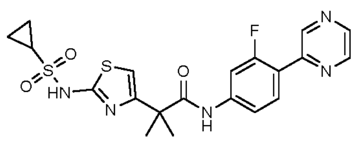 | **Ref T213** | 2-(cyclopropanesulfonamido)-N-(4-(pyridin-3-yl)phenyl)-5,6-dihydro-4H-cyclopenta[d]thiazole-4-carboxamide 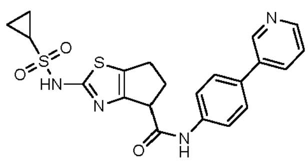 |
| **Ref T40** | 2-(2-(cyclopropanesulfonamido)thiazol-4-yl)-2-methyl-N-(4-(pyridin-3-yl)-2-(trifluoromethoxy)phenyl)propanamid 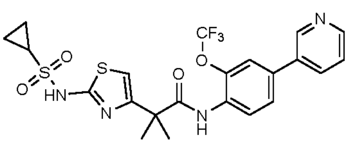 e | **Ref T214** | 2-(2-(cyclopropanesulfonam ido)thiazol-4-yl)-2-methoxy-N-(5-(6-(trifluoromethyl)pyrazin-2-yl)pyridin-2-yl)acetamide 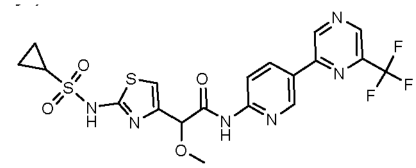 |
| **Ref T41** | N-(2-chloro-4-(pyridin-3-yl)phenyl)-2-(2-(cyclopropanesulfonamido)thiazol-4-yl)-2-methylpropanamide | **Ref T215** | 2-(2-(cyclopropanesulfonamido)thiazol-4-yl)-N-(2-methoxy-4-(pyridin-3-yl)phenyl)acetamide |

(continued)

| T# | Name/Structure (All examples containing chiral centres are racemates unless stated) | T# | Name/Structure (All examples containing chiral centres are racemates unless stated) |
|---|---|---|---|
| | 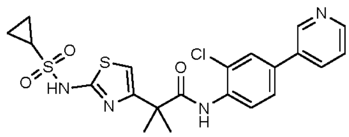 | | 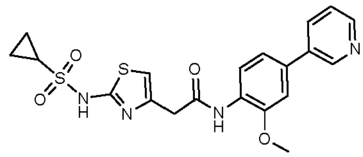 |
| Ref T42 | 2-(2-(cyclopropanesulfonamido)thiazol-4-yl)-N-(2-fluoro-4-(pyridin-3-yl)phenyl)-2-methylpropanamide 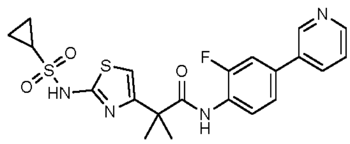 | Ref T216 | 2-(2-(cyclopropanesulfonamido)thiazol-4-yl)-N-(2-fluoro-4-(pyridin-3-yl)phenyl)acetamide 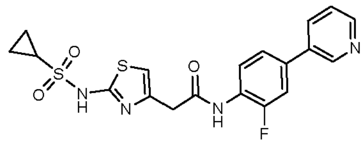 |
| Ref T43 | 2-(2-(cyclopropanesulfonamido)thiazol-4-yl)-N-(3-methoxy-4-(pyrazin-2-yl)phenyl)-2-methylpropanamide 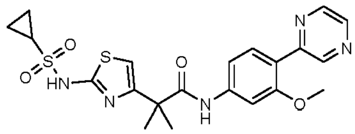 | Ref T217 | N-(4-(5-cyanopyridin-3-yl)phenyl)-2-(cyclopropanesulfonamido)-5,6-dihydro-4H-cyclopenta[d]thiazole-4-carboxamide 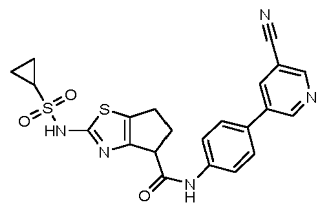 |
| Ref T44 | 2-(2-(cyclopropanesulfonamido)thiazol-4-yl)-N-(4-(2-methoxypyridin-3-yl)phenyl)-2-methylpropanamide 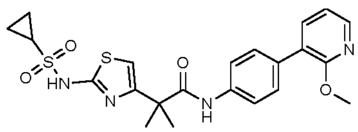 | Ref T218 | 2-(cyclopropanesulfonamido)-N-(4-(5-fluoropyridin-3-yl)phenyl)-5,6-dihydro-4H-cyclopenta[d]thiazole-4-carboxamide 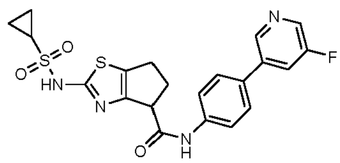 |
| Ref T45 | 2-(2-(cyclopropanesulfonamido)thiazol-4-yl)-N-(4-(5-(hydroxymethyl)pyridin-3-yl)phenyl) acetamide 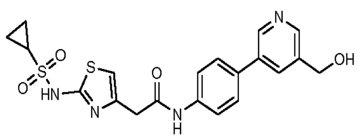 | Ref T219 | 2-(2-(cyclopropanesulfonamido)thiazol-4-yl)-2-methoxy-N-(4-(pyridin-3-yl)phenyl) acetamide 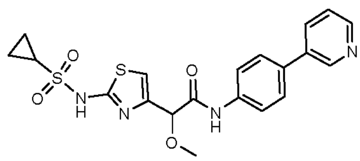 |
| Ref T46 | 2-(2-(cyclopropanesulfonamido)thiazol-4-yl)-N-(4-(6-methoxypyridin-3-yl)phenyl)-2-methylpropanamide 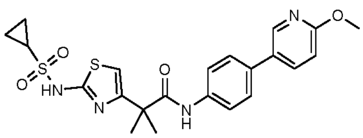 | Ref T220 | 2-(2-(cyclopropanesulfonam ido)thiazol-4-yl)-N-(2-fluoro-4-(pyridin-3-yl)phenyl)-2-methoxyacetamide 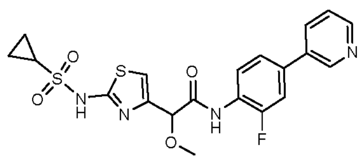 |

(continued)

| T# | Name/Structure (All examples containing chiral centres are racemates unless stated) | T# | Name/Structure (All examples containing chiral centres are racemates unless stated) |
|---|---|---|---|
| **Ref T47** | N-(4-(5-cyanopyridin-3-yl)phenyl)-2-(2-(cyclopropanesulfonamido)thiazol-4-yl)-2-methylpropanamide 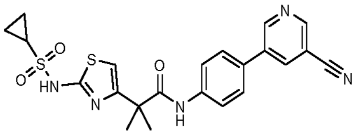 | **Ref T221** | N-(2-chloro-4-(pyridin-3-yl)phenyl)-2-(2-(cyclopropanesulfonamido)thiazol-4-yl)butanamide 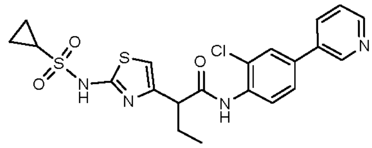 |
| **Ref T48** | 2-(2-(cyclopropanesulfonamido)thiazol-4-yl)-2-methyl-N-(4-(5-(trifluoromethyl)pyridin-3-yl)phenyl)propanamide 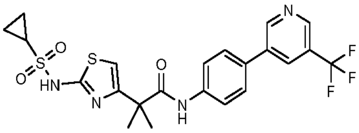 | **Ref T222** | **Single enantiomer - stereochemistry unassigned** 2-(2-(cyclopropanesulfonam ido)thiazol-4-yl)-N-(4-(6-(2,2,2-trifluoroethoxy)pyrazin-2-yl)phenyl)butanamide 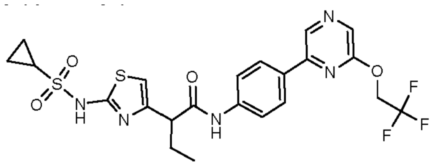 |
| **Ref T49** | 2-(2-(cyclopropanesulfonamido)thiazol-4-yl)-2-methyl-N-(4-(5-(methylsulfonyl)pyridin-3-yl)phenyl)propanamide 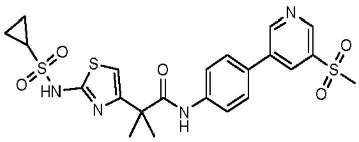 | **Ref T223** | **Single enantiomer - stereochemistry unassigned** 2-(2-(cyclopropanesulfonam ido)thiazol-4-yl)-N-(4-(6-(2,2,2-trifluoroethoxy)pyrazin-2-yl)phenyl)butanamide 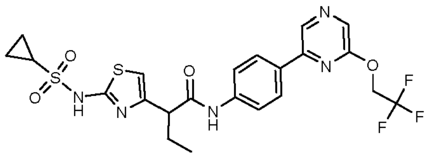 |
| **Ref T50** | 2-(2-(cyclopropanesulfonamido)thiazol-4-yl)-N-(2-methoxy-4-(5-methoxypyridin-3-yl)phenyl)-2-methylpropanamide 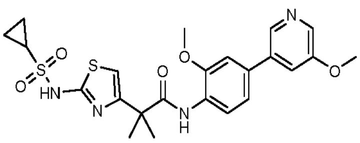 | **Ref T224** | 2-(2-(cyclopropanesulfonam ido)thiazol-4-yl)-N-(5'-methoxy-[3,3'-bipyridin]-6-yl)-2-methylpropanamide 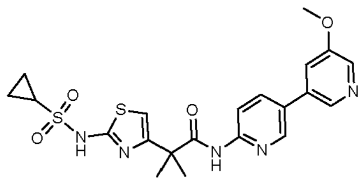 |
| **Ref T51** | 2-(2-(cyclopropanesulfonamido)thiazol-4-yl)-2-methyl-N-(6-(trifluoromethyl)-[2.3'-bipyridin]-6'-yl) propanamide 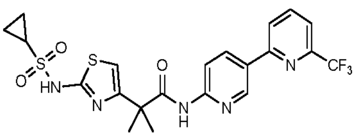 | **Ref T225** | N-(5'-chloro-[3,3'-bipyridin]-6-yl)-2-(2-(cyclopropanesulfonamido)thiazol-4-yl)-2-methylpropanamide 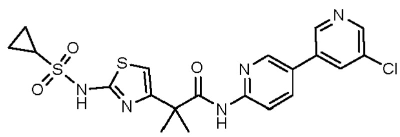 |
| **Ref T52** | 2-(2-(cyclopropanesulfonamido)thiazol-4-yl)-N-(4-(6-ethoxypyrazin-2-yl)phenyl)-2-methylpropanamide | **Ref T226** | N-(5'-cyano-[3,3'-bipyridin]-6-yl)-2-(2-(cyclopropanesulfonamido)thiazol-4-yl)-2-methylpropanamide |

(continued)

| T# | Name/Structure (All examples containing chiral centres are racemates unless stated) | T# | Name/Structure (All examples containing chiral centres are racemates unless stated) |
|---|---|---|---|
| | 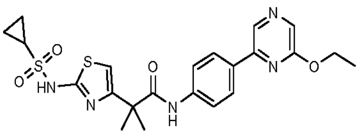 | | 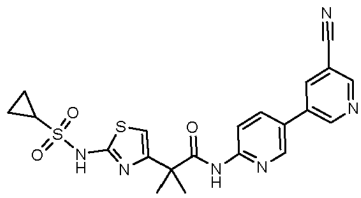 |
| **Ref T53** | 2-(2-(cyclopropanesulfonamido)thiazol-4-yl)-2-methyl-N-(4-(6-morpholinopyrazin-2-yl)phenyl) propanamide 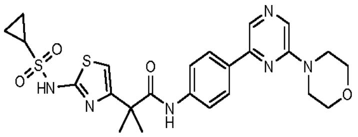 | **Ref T227** | 2-(2-(cyclopropanesulfonam ido)thiazol-4-yl)-N-(5-fluoro-[3,3'-bipyridin]-6-yl)-2-methylpropanamide 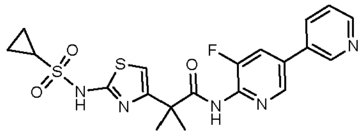 |
| **Ref T54** | N-(4-(6-cyclobutoxypyrazin-2-yl)phenyl)-2-(2-(cyclopropanesulfonamido)thiazol-4-yl)-2-methylpropanamide 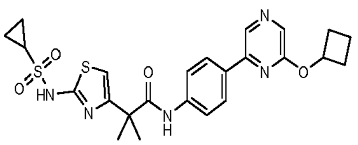 | **Ref T228** | N-(5'-cyano-5-fluoro-[3,3'-bipyridin]-6-yl)-2-(2-(cyclopropanesulfonamido)thiazol-4-yl)-2-methylpropanamide 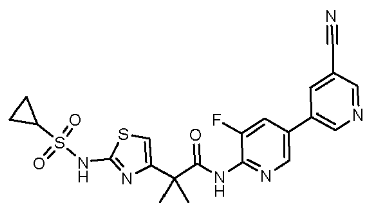 |
| **Ref T55** | 2-(2-(cyclopropanesulfonamido)thiazol-4-yl)-2-methyl-N-(4-(6-propoxypyrazin-2-yl)phenyl)propanamide 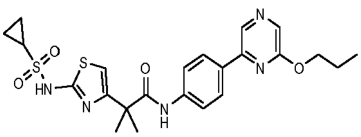 | **Ref T229** | N-(5'-chloro-5-fluoro-[3,3'-bipyridin]-6-yl)-2-(2-(cyclopropanesulfonamido)thiazol-4-yl)-2-methylpropanamide 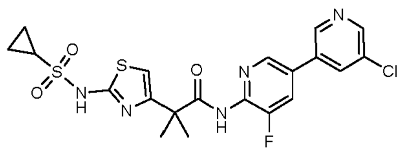 |
| **Ref T56** | 2-(2-(cyclopropanesulfonamido)thiazol-4-yl)-N-(2-fluoro-4-(5-methoxypyridin-3-yl)phenyl)-2-methylpropanamide 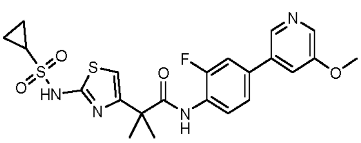 | **Ref T230** | 2-(2-(cyclopropanesulfonam ido)thiazol-4-yl)-N-(5,5'-difluoro-[3,3'-bipyridin]-6-yl)-2-methylpropanamide 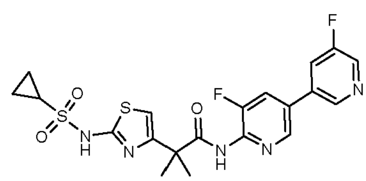 |
| **Ref T57** | 2-(2-(cyclopropanesulfonamido)thiazol-4-yl)-2-methoxy-N-(4-(6-methoxypyrazin-2-yl)phenyl) acetamide 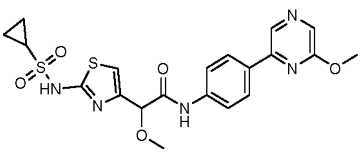 | **Ref T231** | N-(5-(3-chloro-5-methylphenyl)pyridin-2-yl)-2-(2-(cyclopropanesulfonamido)thiazol-4-yl)-2-methylpropanamide 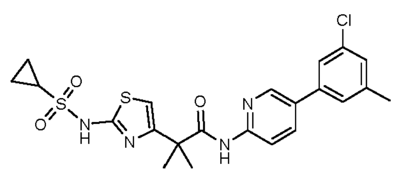 |

(continued)

| T# | Name/Structure (All examples containing chiral centres are racemates unless stated) | T# | Name/Structure (All examples containing chiral centres are racemates unless stated) |
|---|---|---|---|
| Ref T58 | N-(4-(5-chloropyridin-3-yl)phenyl)-2-(2-(cyclopropanesulfonamido)thiazol-4-yl)-2-isopropoxyacetamide 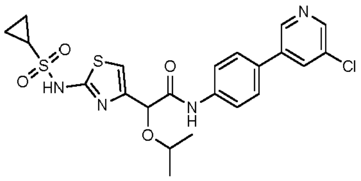 | Ref T232 | 2-(2-(cyclopropanesulfonam ido)thiazol-4-yl)-N-(5-(3-methoxyphenyl)pyridin-2-yl)-2-methylpropanamide 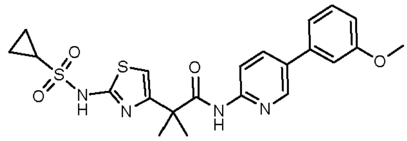 |
| Ref T59 | 2-(2-(cyclopropanesulfonamido)thiazol-4-yl)-4-methoxy-N-(5-(6-(trifluoromethyl)pyrazin-2-yl)pyridin-2-yl)butanamide 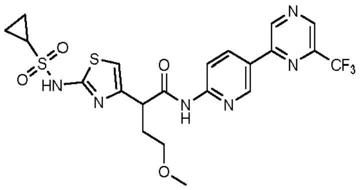 | Ref T233 | 2-(2-(cyclopropanesulfonam ido)thiazol-4-yl)-N-(5-(3-fluoro-5-methoxyphenyl)pyridin-2-yl)-2-methylpropanamide 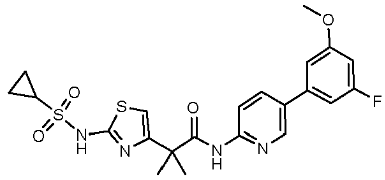 |
| Ref T60 | N-([1,1'-biphenyl]-4-yl)-2-(2-(cyclopropanesulfonamido)thiazol-4-yl)-2,2-difluoroacetamide 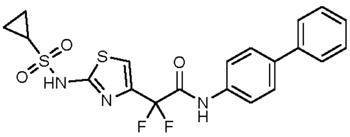 | Ref T234 | 2-(2-(cyclopropanesulfonam ido)thiazol-4-yl)-N-(5-(3,5-dimethoxyphenyl)pyridin-2-yl)-2-methylpropanamide 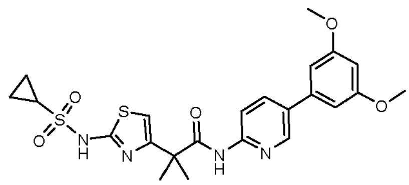 |
| Ref T61 | 2-(2-(cyclobutanesulfonamido)thiazol-4-yl)-N-(4-(6-methoxypyrazin-2-yl)phenyl)acetamide 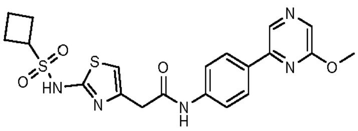 | Ref T235 | 2-(2-(cyclopropanesulfonam ido)thiazol-4-yl)-2-methyl-N-(5-(3-(trifluoromethyl)phenyl)pyridin-2-yl)propanamide 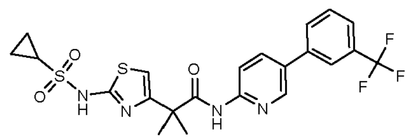 |
| Ref T62 | N-([3,3'-bipyridin]-6-yl)-2-(2-(cyclopropanesulfonamido)thiazol-4-yl)butanamide 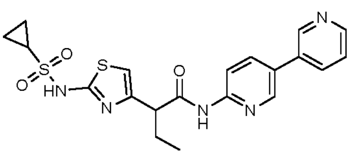 | Ref T236 | 2-(2-(cyclopropanesulfonam ido)thiazol-4-yl)-2-methyl-N-(5-(3-(trifluoromethoxy)phenyl)pyridin-2-yl)propanamide 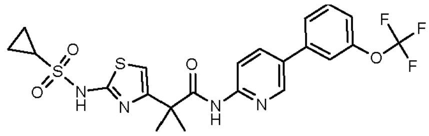 |
| Ref T63 | 2-(2-(cyclopropanesulfonamido)thiazol-4-yl)-2-methyl-N-(5-phenylpyridin-2-yl)propanamide | Ref T237 | 2-(2-(cyclopropanesulfonamido)thiazol-4-yl)-N-(5-(3-(2-hydroxypropan-2-yl)phenyl) pyridin-2-yl)-2-methylpropanamide |

(continued)

| T# | Name/Structure (All examples containing chiral centres are racemates unless stated) | T# | Name/Structure (All examples containing chiral centres are racemates unless stated) |
|---|---|---|---|
| | 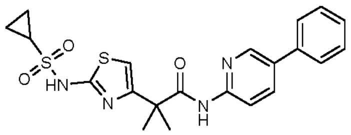 | | 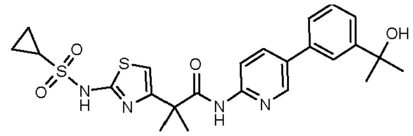 |
| **Ref T64** | 2-(2-(cyclopropanesulfonamido)thiazol-4-yl)-N-(5-(pyrimidin-5-yl)pyridin-2-yl)acetamide 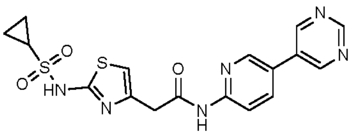 | **Ref T238** | 2-(2-(cyclopropanesulfonam ido)thiazol-4-yl)-2-methyl-N-(5-(3-morpholinophenyl)pyridin-2-yl)propanamide 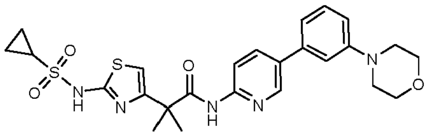 |
| **Ref T65** | N-([3,3'-bipyridin]-6-yl)-2-(2-(cyclopropanesulfonamido)thiazol-4-yl)acetamide 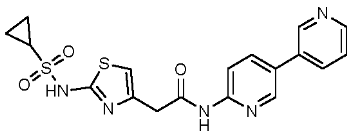 | **Ref T239** | 2-(2-(cyclopropanesulfonam ido)thiazol-4-yl)-2-methyl-N-(6-phenylpyridin-3- 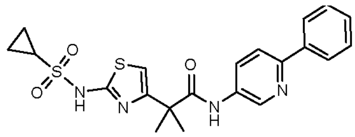 yl)propanamide |
| **Ref T66** | 2-(2-(cyclopropanesulfonamido)thiazol-4-yl)-N-(6-phenylpyridin-3-yl)acetamide 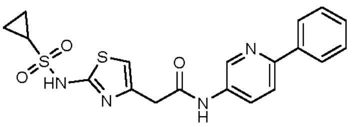 | **Ref T240** | 2-(2-(cyclopropanesulfonam ido)thiazol-4-yl)-N-(4-(2-fluoropyridin-3-yl)phenyl)-2-methylpropanamide 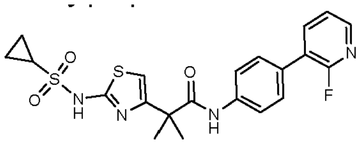 |
| **Ref T67** | N-([2,3'-bipyridin]-5-yl)-2-(2-(cyclopropanesulfonamido)thiazol-4-yl)acetamide 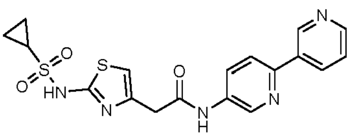 | **Ref T241** | 2-(2-(cyclopropanesulfonam ido)thiazol-4-yl)-N-(4-(5-(hydroxymethyl)pyridin-3-yl)phenyl)-2-methylpropanamide 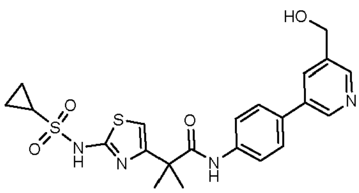 |
| **Ref T68** | 2-(2-(cyclopropanesulfonamido)thiazol-4-yl)-2-methyl-N-(4-(pyridazin-3-yl)phenyl)propanamide 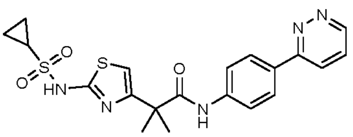 | **Ref T242** | 2-(2-(cyclopropanesulfonam ido)thiazol-4-yl)-N-(4-(2-methoxypyrimidin-5-yl)phenyl) acetamide 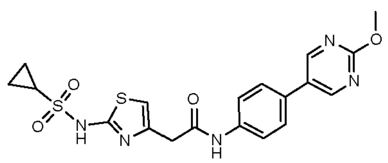 |
| **Ref T69** | 2-(2-(cyclopropanesulfonamido)thiazol-4-yl)-N-(4-(pyridazin-4-yl)phenyl)butanamide | **Ref T243** | N-(4'-(tert-butyl)-[1,1'-biphenyl]-4-yl)-2-(2-(cyclopropanesulfonamido)thiazol-4-yl)acetamide |

(continued)

| T# | Name/Structure (All examples containing chiral centres are racemates unless stated) | T# | Name/Structure (All examples containing chiral centres are racemates unless stated) |
|---|---|---|---|
| | 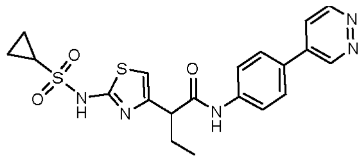 | | 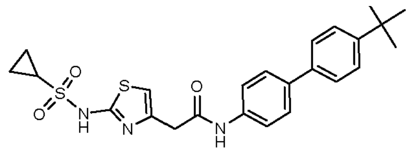 |
| **Ref T70** | 2-(2-(cyclopropanesulfonamido)thiazol-4-yl)-N-(4-(pyrazin-2-<br>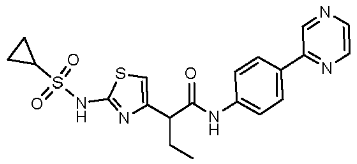<br>yl)phenyl)butanamide | **Ref T244** | 2-(2-(cyclopropanesulfonam ido)thiazol-4-yl)-N-(4-(pyridin-3-yl)phenyl)acetamide<br>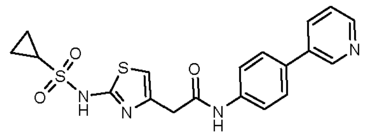 |
| **Ref T71** | N-(4-(5-chloropyridin-3-yl)phenyl)-2-(2-(cyclopropanesulfonamido)thiazol-4-yl)-4-methoxybutanamide<br>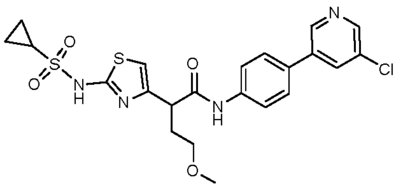 | **Ref T245** | 2-(2-(cyclopropanesulfonam ido)thiazol-4-yl)-N-(4-(pyridin-4-yl)phenyl)acetamide<br>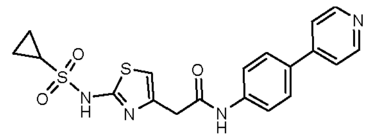 |
| Ref **T72** | **Single enantiomer - stereochemistry unassigned** 2-(2-(cyclopropanesulfonamido)thiazol-4-yl)-N-(4-(5-fluoropyridin-3-yl)phenyl)butanamide<br>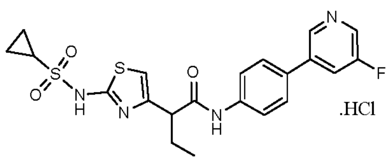<br>.HCl | **Ref T246** | 2-(2-(cyclopropanesulfonam ido)thiazol-4-yl)-N-(2'-methoxy-[1,1'-biphenyl]-4-yl)acetamide<br>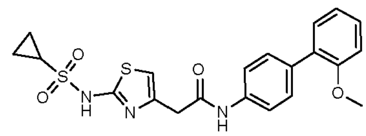 |
| **Ref T73** | 2-(2-(cyclopropanesulfonamido)thiazol-4-yl)-N-(4-(6-(trifluoromethyl)pyrazin-2-yl)phenyl) butanamide<br>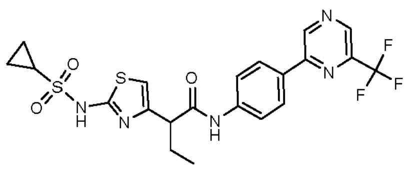 | **Ref T247** | 2-(2-(cyclopropanesulfonam ido)thiazol-4-yl)-N-(4-(pyrimidin-5-yl)phenyl)acetamide<br>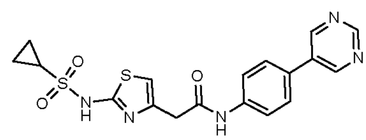 |
| **Ref T74** | 2-(2-(cyclopropanesulfonamido)thiazol-4-yl)-3-methyl-N-(4-(pyrazin-2-yl)phenyl)butanamide<br>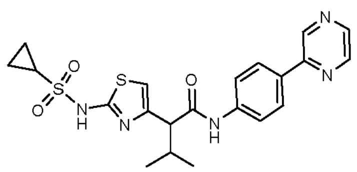 | **Ref T248** | 2-(2-(cyclopropanesulfonamido)thiazol-4-yl)-2-methyl-N-(4-(2-(trifluoromethyl)pyridin-3-yl)phenyl)propanamide<br>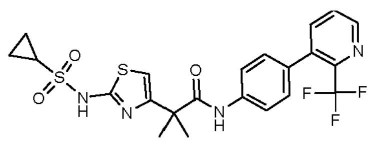 |

(continued)

| T# | Name/Structure (All examples containing chiral centres are racemates unless stated) | T# | Name/Structure (All examples containing chiral centres are racemates unless stated) |
|---|---|---|---|
| **Ref T75** | 2-(2-(cyclopropanesulfonamido)thiazol-4-yl)-N-(4-(6-propoxypyrazin-2-yl)phenyl)butanamide 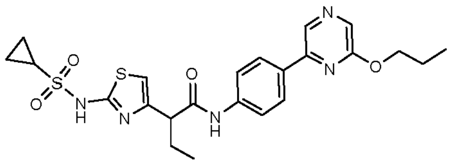 | **Ref T249** | 2-(2-(cyclopropanesulfonamido)thiazol-4-yl)-2-methyl-N-(5'-methyl-[3,3'-bipyridin]-6-yl)propanamide 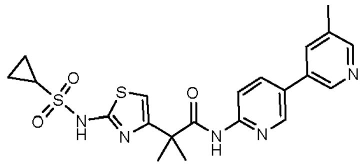 |
| **Ref T76** | 2-(2-(cyclopropanesulfonamido)thiazol-4-yl)-N-(4-(6-isopropoxypyrazin-2-yl)phenyl)butanamide 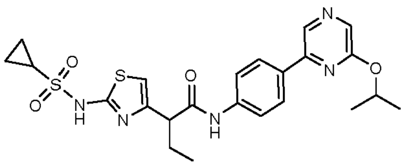 | **Ref T250** | 2-(2-(cyclopropanesulfonamido)thiazol-4-yl)-N-(4-(2-methoxy-4-methylpyridin-3-yl)phenyl)-2-methylpropanamide 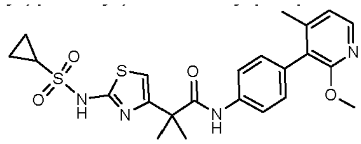 |
| **Ref T77** | 2-(2-(cyclopropanesulfonamido)thiazol-4-yl)-N-(4-(6-cyclopropoxypyrazin-2-yl)phenyl)butanamide 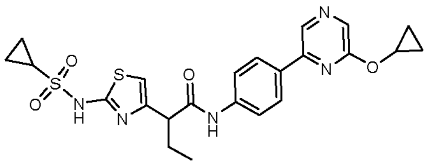 | **Ref T251** | 2-(2-(cyclopropanesulfonamido)thiazol-4-yl)-N-(4-(6-methoxy-5-methylpyridin-3-yl)phenyl)-2-methylpropanamide 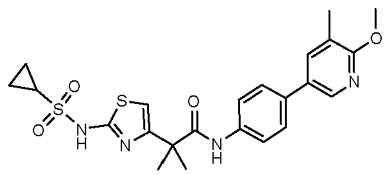 |
| **Ref T78** | N-(4-(6-chloropyrazin-2-yl)phenyl)-2-(2-(cyclopropanesulfonamido)thiazol-4-yl)butanamide 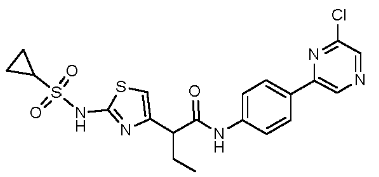 | **Ref T252** | N-(4-(5-chloropyridin-3-yl)phenyl)-2-(2-(cyclopropanesulfonamido)thiazol-4-yl)-2-methylpropanamide 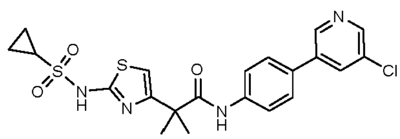 |
| **Ref T79** | N-(4-(6-cyanopyrazin-2-yl)phenyl)-2-(2-(cyclopropanesulfonamido)thiazol-4-yl)butanamide 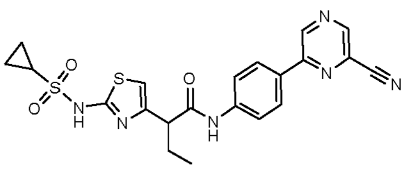 | **Ref T253** | 2-(2-(cyclopropanesulfonamido)thiazol-4-yl)-N-(4-(5-fluoropyridin-3-yl)phenyl)-2-methylpropanamide 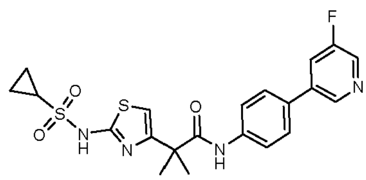 |

(continued)

| T# | Name/Structure (All examples containing chiral centres are racemates unless stated) | T# | Name/Structure (All examples containing chiral centres are racemates unless stated) |
|---|---|---|---|
| Ref T80 | **Single enantiomer - stereochemistry unassigned** 2-(2-(cyclopropanesulfonamido)thiazol-4-yl)-N-(4-(6-(trifluoromethyl)pyrazin-2-yl)phenyl) butanamide 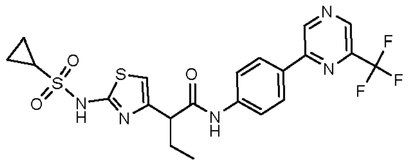 | Ref T254 | 2-(2-(cyclopropanesulfonamido)thiazol-4-yl)-2-methyl-N-(4-(4-methylpyridin-3-yl)phenyl)propanamide 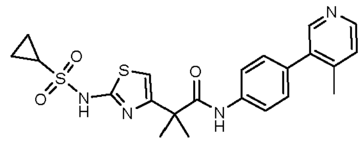 |
| Ref T81 | 2-(2-(cyclopropanesulfonamido)thiazol-4-yl)-N-(4-(6-methoxypyrazin-2-yl)phenyl)acetamide 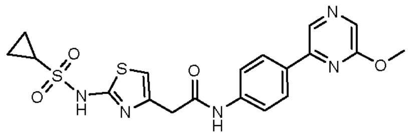 | Ref T255 | 2-(2-(cyclopropanesulfonamido)thiazol-4-yl)-2-methyl-N-(4-(4-(trifluoromethyl)pyridin-3-yl)phenyl)propanamide 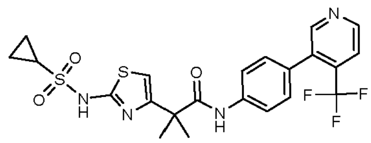 |
| Ref T82 | 2-(2-(cyclopropanesulfonamido)thiazol-4-yl)-N-(4-(pyrazin-2-yl)phenyl)acetamide 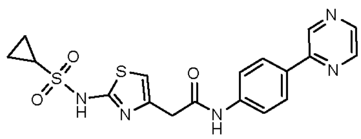 | Ref T256 | N-(4-(5-chloropyridin-3-yl)-2-methoxyphenyl)-2-(2-(cyclopropanesulfonamido)thiazol-4-yl)-2-methylpropanamide 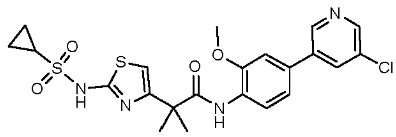 |
| Ref T83 | N-([1,1'-biphenyl]-4-yl)-2-(cyclopropanesulfonamido)-4,5,6,7-tetrahydrobenzo[d]thiazole-4-carboxamide 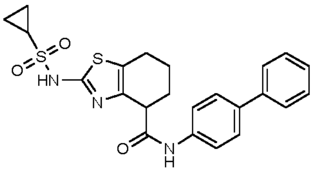 | Ref T257 | 2-(2-(cyclopropanesulfonamido)thiazol-4-yl)-N-(4-(5-(dimethylamino)pyridin-3-yl)phenyl)-2-methylpropanamide 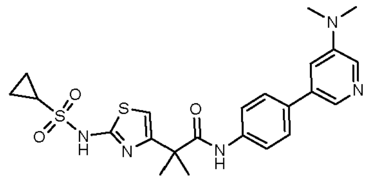 |
| Ref T84 | 2-(cyclopropanesulfonamido)-N-(4-(pyridin-3-yl) phenyl)-4,5,6,7-tetrahydrobenzo[d]thiazole-4-carboxamide 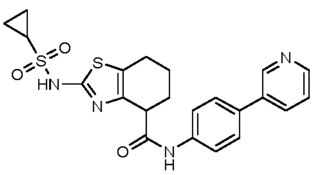 | Ref T258 | 2-(2-(cyclopropanesulfonamido)thiazol-4-yl)-N-(2-methoxy-4-(5-methylpyridin-3-yl) phenyl)-2-methylpropanamide 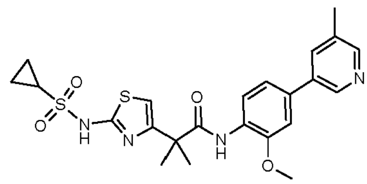 |
| Ref T85 | N-([1,1'-biphenyl]-4-yl)-2-(2-(cyclopropanesulfonamido)thiazol-4-yl)butanamide | Ref T259 | 2-(2-(cyclopropanesulfonamido)thiazol-4-yl)-N-(2-methoxy-4-(5-(trifluoromethyl)pyridin-3-yl)phenyl)-2-methylpropanamide |

(continued)

| T# | Name/Structure (All examples containing chiral centres are racemates unless stated) | T# | Name/Structure (All examples containing chiral centres are racemates unless stated) |
|---|---|---|---|
| | 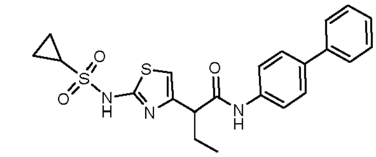 | | 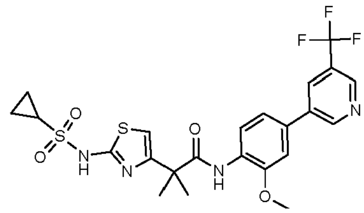 |
| **Ref T86** | N-([1,1'-biphenyl]-4-yl)-2-(2-(cyclopropanesulfonamido)thiazol-4-yl)-3-methylbutanamide<br>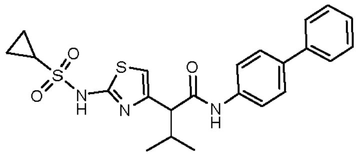 | **Ref T260** | 2-(2-(cyclopropanesulfonamido)thiazol-4-yl)-N-(4-(6-methoxypyridin-3-yl)phenyl) acetamide<br>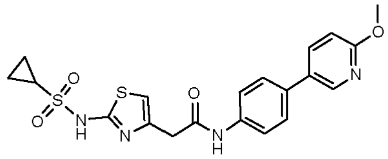 |
| **Ref T87** | N-(3'-chloro-[1,1'-biphenyl]-4-yl)-2-(2-(cyclopropanesulfonamido)thiazol-4-yl)-2-methylpropanamide<br>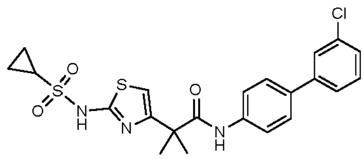 | **Ref T261** | 2-(2-(cyclopropanesulfonamido)thiazol-4-yl)-N-(5'-fluoro-[3,3'-bipyridin]-6-yl)-2-methylpropanamide<br>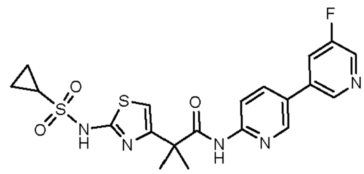 |
| **Ref T88** | N-(3'-cyano-[1,1'-biphenyl]-4-yl)-2-(2-(cyclopropanesulfonamido)thiazol-4-yl)-2-methylpropanamide<br>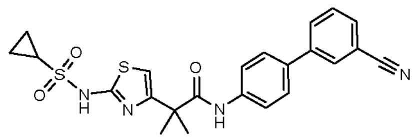 | **Ref T262** | N-(5-(6-chloropyrazin-2-yl)pyrid in-2-yl)-2-(2-(cyclopropanesulfonamido)thiazol-4-yl)-2-methylpropanamide<br>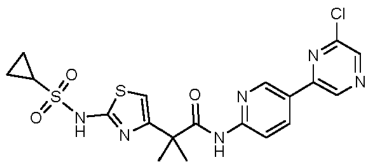 |
| **Ref T89** | 2-(2-(cyclopropanesulfonamido)thiazol-4-yl)-2,2-difluoro-N-(4-(pyridin-3-yl)phenyl)acetamide<br>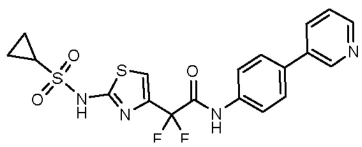 | **Ref T263** | N-(5-(6-cyanopyrazin-2-yl)pyridin-2-yl)-2-(2-(cyclopropanesulfonamido)thiazol-4-yl)-2-methylpropanamide<br>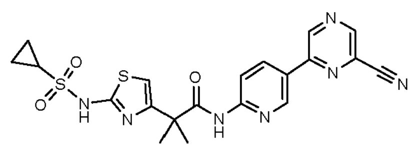 |
| **Ref T90** | N-(4-(5-fluoropyridin -3-yl)phenyl)-2-(2-(cyclopropanesulfonamido)thiazol-4-yl)butanamide<br>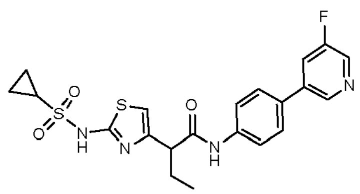 | **Ref T264** | 2-(2-(cyclopropanesulfonamido)thiazol-4-yl)-2-methyl-N-(5-(pyrimidin-5-yl)pyridin-2-yl)propanamide<br>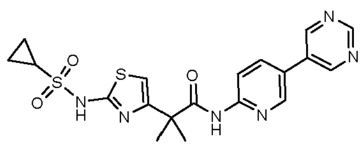 |

(continued)

| T# | Name/Structure (All examples containing chiral centres are racemates unless stated) | T# | Name/Structure (All examples containing chiral centres are racemates unless stated) |
|---|---|---|---|
| **Ref T91** | **Single enantiomer - stereochemistry unassigned** N-(4-(5-fluoropyridin-3-yl)phenyl)-2-(2-(cyclopropanesulfonamido)thiazol-4-yl)butanamide.HCl 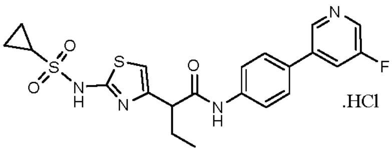 | **Ref T265** | 2-(2-(cyclopropanesulfonamido)thiazol-4-yl)-N-(4-(6-methoxypyrazin-2-yl)phenyl)-2-methylpropanamide 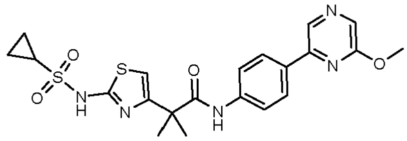 |
| **Ref T92** | 2-(2-(cyclopropanesulfonamido)thiazol-4-yl)-2-ethyl-N-(4-(pyridin-3-yl)phenyl)butanamide 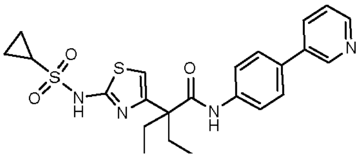 | **Ref T266** | 2-(2-(cyclopropanesulfonamido)thiazol-4-yl)-2-methyl-N-(4-(6-methylpyrazin-2-yl) phenyl)propanamide 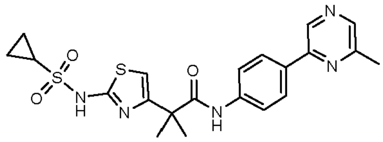 |
| **Ref T93** | N-(4-(5-cyanopyridin-3-yl)phenyl)-2-(2-(cyclopropanesulfonamido)thiazol-4-yl)-2-ethylbutanamide 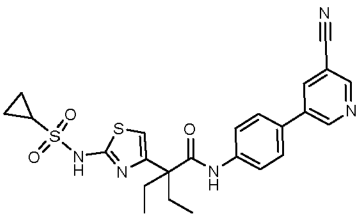 | **Ref T267** | 2-(2-(cyclopropanesulfonamido)thiazol-4-yl)-2-methyl-N-(4-(6-(trifluoromethyl)pyrazin-2-yl)phenyl)propanamide 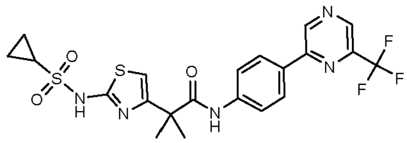 |
| **Ref T94** | 2-(2-(cyclopropanesulfonamido)thiazol-4-yl)-N-(4-(5-ethoxypyridin-3-yl)phenyl)propanamide 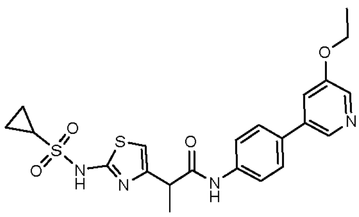 | **Ref T268** | N-(4-(6-chloropyridin-2-yl)phenyl)-2-(2-(cyclopropanesulfonamido)thiazol-4-yl)-2-methylpropanamide 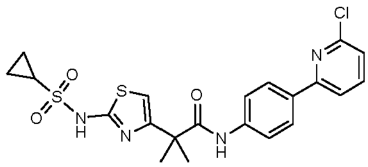 |
| **Ref T95** | N-(4-(5-chloropyridin-3-yl)phenyl)-2-(2-(cyclopropanesulfonamido)thiazol-4-yl)propanamide 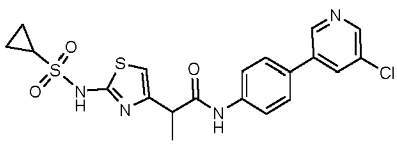 | **Ref T269** | 2-(2-(cyclopropanesulfonamido)thiazol-4-yl)-N-(4-(6-methoxypyridin-2-yl)phenyl)-2-methylpropanamide 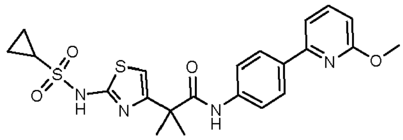 |
| **Ref T96** | 2-(2-(cyclopropanesulfonamido)thiazol-4-yl)-N-(4-(5-ethoxypyridin-3-yl)phenyl)butanamide | **Ref T270** | 2-(2-(cyclopropanesulfonamido)thiazol-4-yl)-2-methyl-N-(4-(6-(trifluoromethyl)pyridin-2-yl)phenyl)propanamide |

(continued)

| T# | Name/Structure (All examples containing chiral centres are racemates unless stated) | T# | Name/Structure (All examples containing chiral centres are racemates unless stated) |
|---|---|---|---|
| | 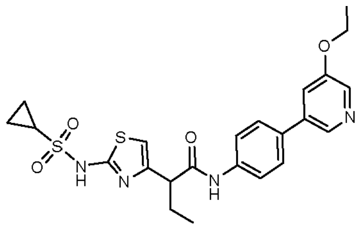 | | 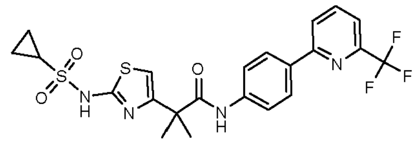 |
| **Ref T97** | N-([1,1'-biphenyl]-4-yl)-2-(2-(cyclopropanesulfonamido)thiazol-4-yl)-2-methylpropanamide 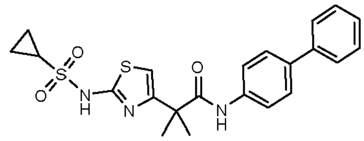 | **Ref T271** | 2-(2-(cyclopropanesulfonamido)thiazol-4-yl)-N-(4-(4-methoxypyridin-2-yl)phenyl)-2-methylpropanamide 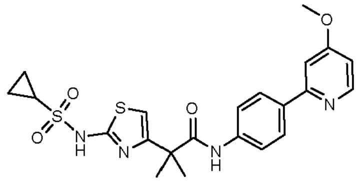 |
| **Ref T98** | 2-(2-(cyclopropanesulfonamido)thiazol-4-yl)-N-(4-(4-methylpyridin-3-yl)phenyl)acetamide 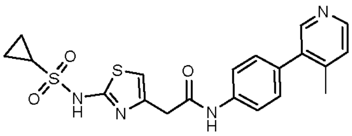 | **Ref T272** | 2-(2-(cyclopropanesulfonamido)thiazol-4-yl)-N-(4-(6-isopropoxypyrazin-2-yl)phenyl)-2-methylpropanamide 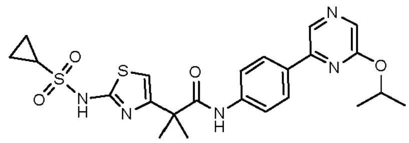 |
| **Ref T99** | 2-(2-(cyclopropanesulfonamido)thiazol-4-yl)-N-(4-(5-methylpyridin-3-yl)phenyl)acetamide 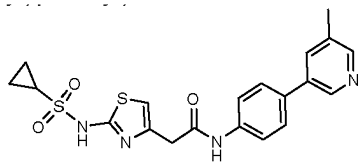 | **Ref T273** | 2-(2-(cyclopropanesulfonamido)thiazol-4-yl)-N-(4-(6-cyclopropoxypyrazin-2-yl)phenyl)-2-methylpropanamide 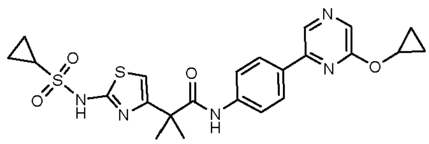 |
| **Ref T100** | 2-(2-(cyclopropanesulfonamido)thiazol-4-yl)-N-(4-(2-methylpyridin-3-yl)phenyl)acetamide 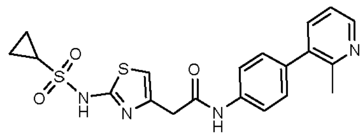 | **Ref T274** | 2-(2-(cyclopropanesulfonamido)thiazol-4-yl)-N-(2-fluoro-4-(pyrazin-2-yl)phenyl)-2-methylpropanamide 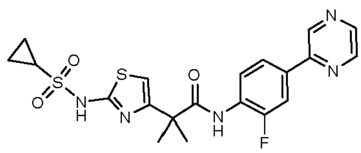 |
| **Ref T101** | 2-(2-(cyclopropanesulfonamido)thiazol-4-yl)-N-(4-(6-methylpyridin-3-yl)phenyl)acetamide 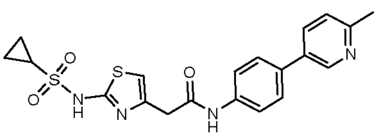 | **Ref T275** | 2-(2-(cyclopropanesulfonamido)thiazol-4-yl)-N-(2-fluoro-4-(6-methoxypyrazin-2-yl)phenyl)-2-methylpropanamide 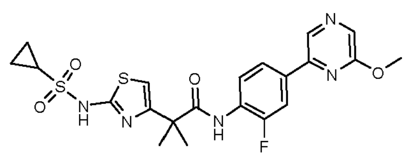 |

(continued)

| T# | Name/Structure (All examples containing chiral centres are racemates unless stated) | T# | Name/Structure (All examples containing chiral centres are racemates unless stated) |
|---|---|---|---|
| **Ref T102** | 2-(2-(cyclopropanesulfonamido)thiazol-4-yl)-N-(4-(pyridin-3-yl)phenyl)propanamide 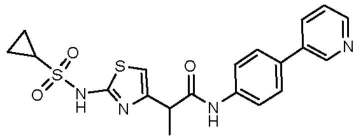 | **Ref T276** | N-(4-(6-chloro-3-methylpyrazin-2-yl)phenyl)-2-(2-(cyclopropanesulfonamido)thiazol-4-yl)-2-methylpropanamide 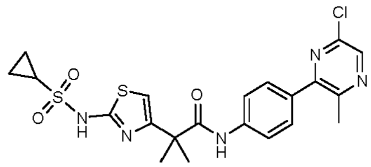 |
| **Ref T103** | 2-(2-(cyclopropanesulfonamido)thiazol-4-yl)-2-methyl-N-(4-(2-methylpyridin-3-yl)phenyl)propanamide 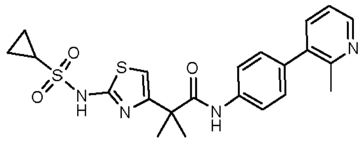 | **Ref T277** | N-(4-(6-chloro-5-methylpyrazin-2-yl)phenyl)-2-(2-(cyclopropanesulfonamido)thiazol-4-yl)-2-methylpropanamide 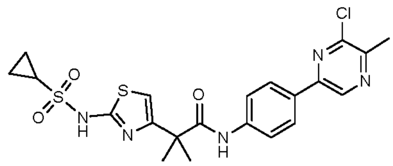 |
| **Ref T104** | 2-(2-(cyclopropanesulfonamido)thiazol-4-yl)-2-oxo-N-(4-(pyridin-3-yl)phenyl)acetamide 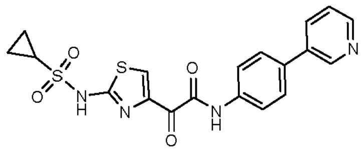 | **Ref T278** | 2-(2-(cyclopropanesulfonamido)thiazol-4-yl)-2-methyl-N-(4-(6-(pyrrolidin-1- 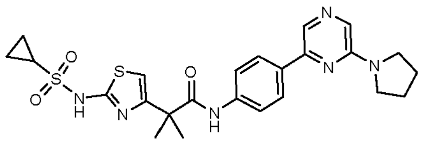 yl)pyrazin-2-yl)phenyl)propanamide |
| **Ref T105** | 2-(2-(cyclopropanesulfonamido)thiazol-4-yl)-2-methyl-N-(4-(6-methylpyridin-3-yl)phenyl)propanamide 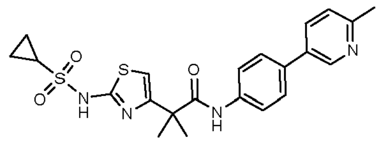 | **Ref T279** | 2-(2-(cyclopropanesulfonamido)thiazol-4-yl)-N-(4-(6-(2-(dimethylamino)ethoxy)pyrazin-2-yl)phenyl)-2-methylpropanamide 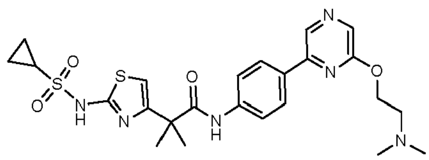 |
| **Ref T106** | **Single enantiomer - stereochemistry unassigned** 2-(2-(cyclopropanesulfonamido)thiazol-4-yl)-N-(4-(6-(trifluoromethyl)pyrazin-2-yl)phenyl)butanamide 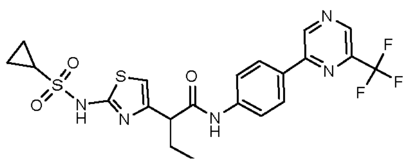 | **Ref T280** | 2-(2-(cyclopropanesulfonamido)thiazol-4-yl)-2-methyl-N-(4-(3-methylpyrazin-2-yl)phenyl)propanamide 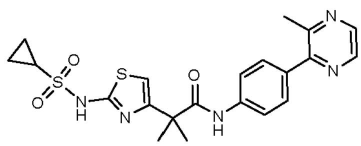 |

(continued)

| T# | Name/Structure (All examples containing chiral centres are racemates unless stated) | T# | Name/Structure (All examples containing chiral centres are racemates unless stated) |
|---|---|---|---|
| **Ref T107** | **Single enantiomer - stereochemistry unassigned** 2-(2-(cyclopropanesulfonamido)thiazol-4-yl)-2-methoxy-N-(4-(6-methoxypyrazin-2-yl)phenyl) acetamide 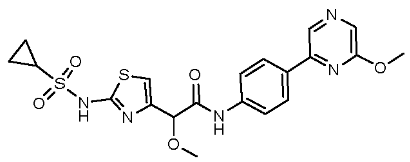 | **Ref T281** | N-(4-(6-acetamidopyrazin-2-yl)phenyl)-2-(2-(cyclopropanesulfonamido)thiazol-4-yl)-2-methylpropanamide 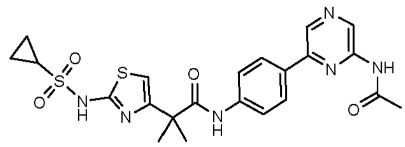 |
| **Ref T108** | **Single enantiomer - stereochemistry unassigned** 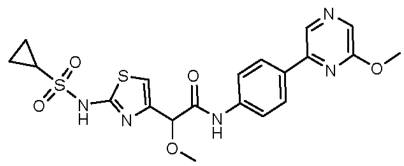 2-(2-(cyclopropanesulfonamido)thiazol-4-yl)-2-methoxy-N-(4-(6-methoxypyrazin-2-yl)phenyl) acetamide | **Ref T282** | 2-(2-(cyclopropanesulfonamido)thiazol-4-yl)-N-(4-(5,6-dimethylpyrazin-2-yl)phenyl)-2-methylpropanamide 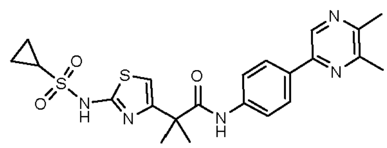 |
| **Ref T109** | 2-(2-(cyclopropanesulfonamido)thiazol-4-yl)-2-methyl-N-(5-(6-(2,2,2-trifluoroethoxy)pyrazin-2-yl)pyridin-2-yl)propanamide 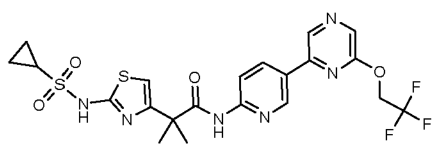 | **Ref T283** | 2-(2-(cyclopropanesulfonamido)thiazol-4-yl)-N-(4-(6-(hydroxymethyl)pyrazin-2-yl) phenyl)-2-methylpropanamide 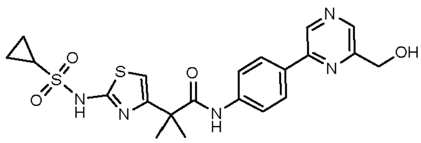 |
| **Ref T110** | 2-(2-(cyclopropanesulfonamido)thiazol-4-yl)-N-(3-fluoro-5-(pyrazin-2-yl)pyridin-2-yl)-2-methylpropanamide 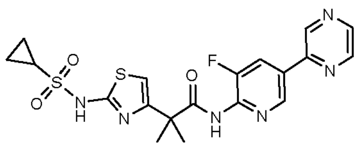 | **Ref T284** | 2-(2-(cyclopropanesulfonamido)thiazol-4-yl)-N-(4-(3,6-dimethylpyrazin-2-yl)phenyl)-2-methylpropanamide 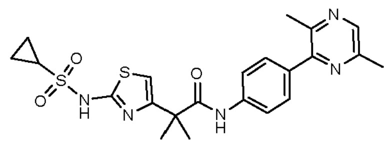 |
| **Ref T111** | 2-(2-(cyclopropanesulfonamido)thiazol-4-yl)-N-(5-(6-ethoxypyrazin-2-yl)-3-fluoropyridin-2-yl)-2-methylpropanamide 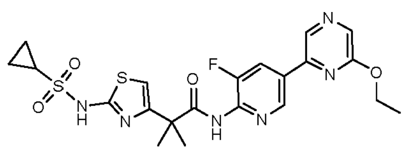 | **Ref T285** | 2-(2-(cyclopropanesulfonamido)thiazol-4-yl)-N-(4-(5-methoxypyridin-3-yl)-2-methylphenyl)-2-methylpropanamide 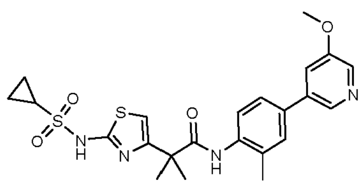 |
| **Ref T112** | 2-(2-(cyclopropanesulfonamido)thiazol-4-yl)-N-(3-fluoro-5-(6-(trifluoromethyl)pyrazin-2-yl) pyridin-2-yl)-2-methylpropanamide | **Ref T286** | N-(4-(5-cyanopyridin-3-yl)-2-methylphenyl)-2-(2-(cyclopropanesulfonamido)thiazol-4-yl)-2-methylpropanamide |

(continued)

| T# | Name/Structure (All examples containing chiral centres are racemates unless stated) | T# | Name/Structure (All examples containing chiral centres are racemates unless stated) |
|---|---|---|---|
| | 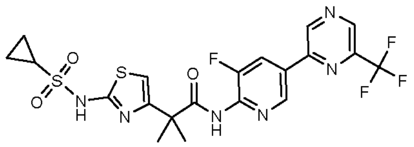 | | 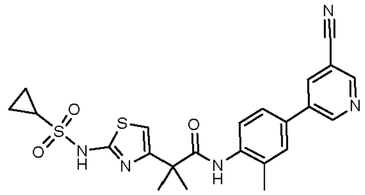 |
| **Ref T113** | N-(5-(6-cyanopyrazin-2-yl)-3-fluoropyridin-2-yl)-2-(2-(cyclopropanesulfonamido)thiazol-4-yl)-2-methylpropanamide<br>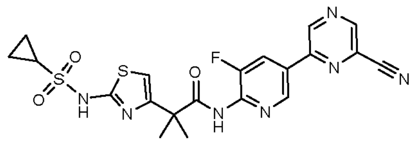 | **Ref T287** | 2-(2-(cyclopropanesulfonamido)thiazol-4-yl)-N-(4-(5-fluoropyridin-3-yl)-2-methylphenyl)-2-methylpropanamide<br>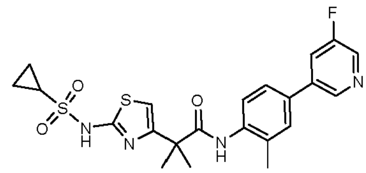 |
| **Ref T114** | 2-(2-(cyclopropanesulfonamido)thiazol-4-yl)-2-methyl-N-(5'-(2,2,2-trifluoroethoxy)-[3,3'-bipyridin]-6-yl)propanamide<br>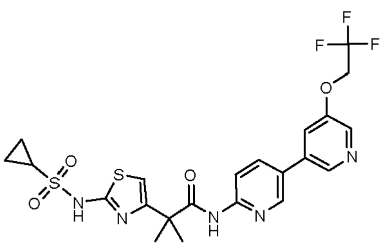 | **Ref T288** | N-(4-(5-chloropyridin-3-yl)-3-methylphenyl)-2-(2-(cyclopropanesulfonamido)thiazol-4-yl)-2-methylpropanamide<br>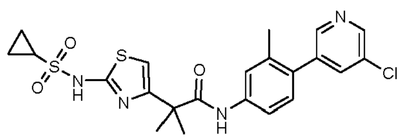 |
| **Ref T115** | 2-(2-(cyclopropanesulfonamido)thiazol-4-yl)-N-(5'-(difluoromethoxy)-[3,3'-bipyridin]-6-yl)-2-methylpropanamide<br>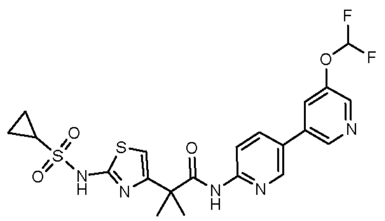 | **Ref T289** | N-(4-(5-cyanopyridin-3-yl)-3-ethoxyphenyl)-2-(2-(cyclopropanesulfonamido)thiazol-4-yl)-2-methylpropanamide<br>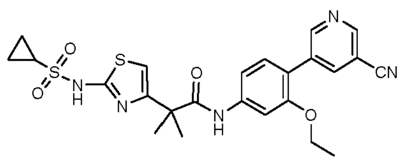 |
| **Ref T116** | N-([2,3'-bipyridin]-5-yl)-2-(2-(cyclopropanesulfonamido)thiazol-4-yl)-2-methylpropanamide<br>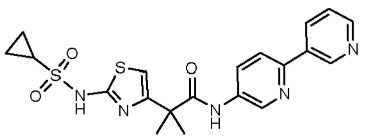 | **Ref T290** | 2-(2-(cyclopropanesulfonamido)thiazol-4-yl)-N-(4-(5-ethoxypyridin-3-yl)phenyl)-2-methylpropanamide<br>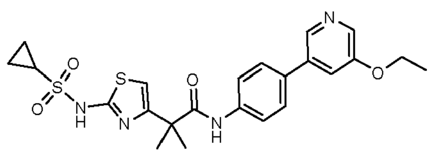 |
| **Ref T117** | 2-(2-(cyclopropanesulfonamido)thiazol-4-yl)-2-methyl-N-(6-(pyrimidin-5-yl)pyridin-3-yl)propanamide | **Ref T291** | 2-(2-(cyclopropanesulfonamido)thiazol-4-yl)-N-(4-(6-cyclopropylpyrazin-2-yl)phenyl) butanamide |

(continued)

| T# | Name/Structure (All examples containing chiral centres are racemates unless stated) | T# | Name/Structure (All examples containing chiral centres are racemates unless stated) |
|---|---|---|---|
| | 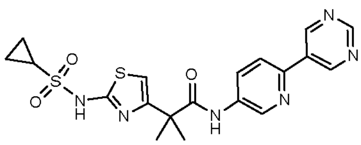 | | 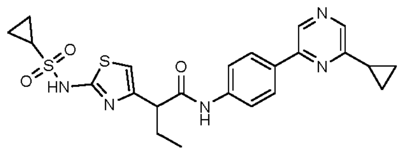 |
| Ref T118 | 2-(2-(cyclopropanesulfonamido)thiazol-4-yl)-N-(4-(5-(difluoromethoxy)pyridin-3-yl)phenyl)-2-methylpropanamide 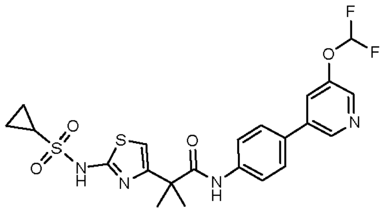 | Ref T292 | 2-(2-(cyclopropanesulfonamido)thiazol-4-yl)-N-(5-(5-methoxypyridin-3-yl)pyrimidin-2-yl)-2-methylpropanamide 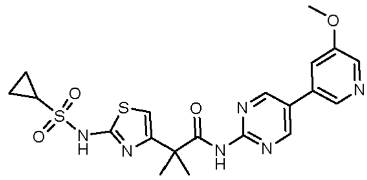 |
| Ref T119 | 2-(2-(cyclopropanesulfonamido)thiazol-4-yl)-2-ethyl-N-(4-(6-methoxypyrazin-2-yl)phenyl)butanamide 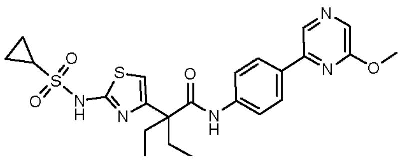 | Ref T293 | 2-(2-(cyclopropanesulfonamido)thiazol-4-yl)-N-(5-(5-fluoropyridin-3-yl)pyrimidin-2-yl)-2-methylpropanamide 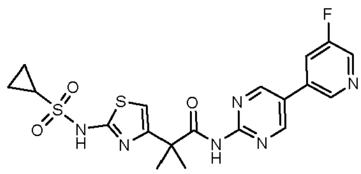 |
| Ref T120 | N-(4-(5-chloropyridin-3-yl)phenyl)-2-(2-(cyclopropanesulfonamido)thiazol-4-yl)-2-ethylbutanamide 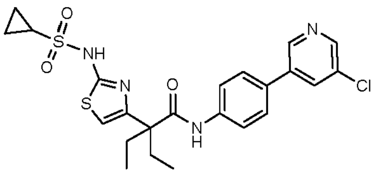 | Ref T294 | 2-(2-(cyclopropanesulfonamido)thiazol-4-yl)-2-methyl-N-(5-(5-(trifluoromethyl)pyridin-3-yl)pyrimidin-2-yl)propanamide 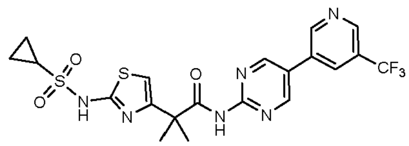 |
| Ref T121 | 2-(2-(cyclopropanesulfonamido)thiazol-4-yl)-2-ethyl-N-(2-fluoro-4-(pyridin-3-yl)phenyl)butanamide 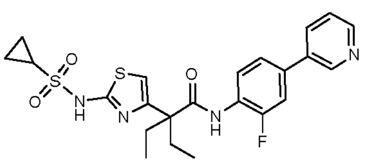 | Ref T295 | N-(5-(6-ethoxypyrazin-2-yl)pyridin-2-yl)-2-methyl-2-(2-((2-methylpropyl)sulfonamido)thiazol-4-yl)propanamide 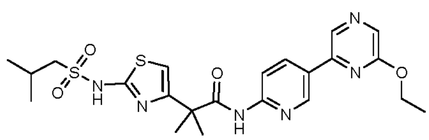 |
| Ref T122 | 2-(2-(cyclopropanesulfonamido)thiazol-4-yl)-2-ethyl-N-(4-(pyrazin-2-yl)phenyl)butanamide 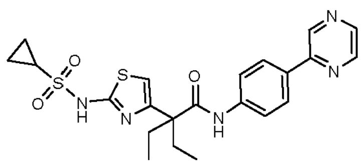 | Ref T296 | N-(4-(6-ethoxypyrazin-2-yl)phenyl)-2-methyl-2-(2-((trifluoromethyl)sulfonamido)thiazol-4-yl)propanamide 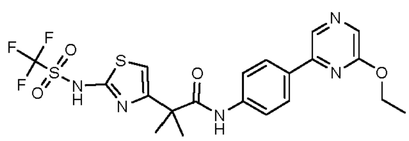 |

(continued)

| T# | Name/Structure (All examples containing chiral centres are racemates unless stated) | T# | Name/Structure (All examples containing chiral centres are racemates unless stated) |
|---|---|---|---|
| **Ref T123** | 2-(2-(cyclopropanesulfonamido)thiazol-4-yl)-2-ethyl-N-(4-(6-propoxypyrazin-2-yl)phenyl)butanamide 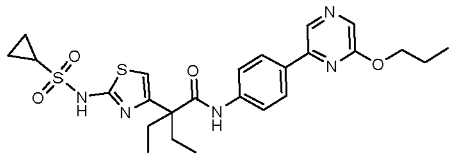 | **Ref T297** | 2-methyl-2-(2-((1-methylethyl)sulfonamido)thiazol-4-yl)-N-(4-(pyridin-3-yl)phenylpropanamide 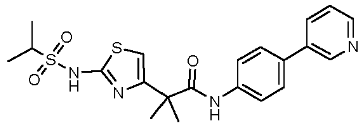 |
| **Ref T124** | 2-(2-(cyclopropanesulfonamido)thiazol-4-yl)-N-(2-fluoro-4-(6-(trifluoromethyl)pyrazin-2-yl)phenyl)-2-methylpropanamide 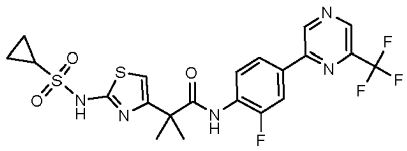 | **Ref T298** | N-(4-(6-ethoxypyrazin-2-yl)phenyl)-2-methyl-2-(2-((1-methylethyl)sulfonamido)thiazol-4-yl)propanamide 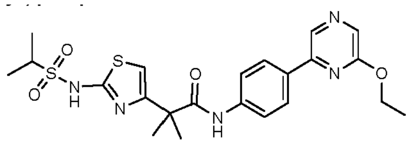 |
| **Ref T125** | 2-(2-(cyclopropanesulfonamido)thiazol-4-yl)-N-(4-(5-ethoxypyridin-3-yl)-2-fluorophenyl)-2-methylpropanamide 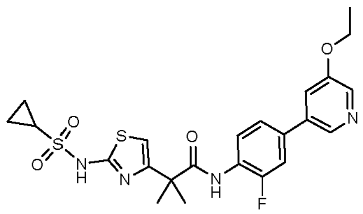 | **Ref T299** | 2-methyl-2-(2-((1-methylcyclopropane)-1-sulfonamido)thiazol-4-yl)-N-(4-(pyridin-3-yl)phenyl)propanamide 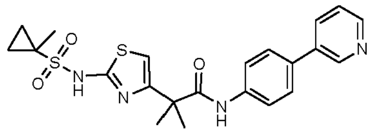 |
| **Ref T126** | 2-(2-(cyclopropanesulfonamido)thiazol-4-yl)-N-(2-fluoro-4-(5-fluoropyridin-3-yl)phenyl)-2-methylpropanamide 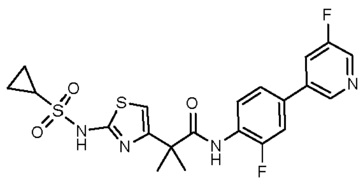 | **Ref T300** | N-(4-(5-chloropyridin-3-yl)phenyl)-2-methyl-2-(2-((1-methylcyclopropane)-1-sulfonamido)thiazol-4-yl)propanamide 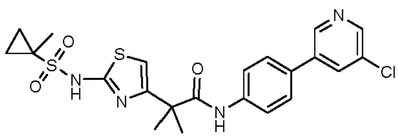 |
| **Ref T127** | 2-(2-(cyclopropanesulfonamido)thiazol-4-yl)-2-methyl-N-(4-(6-(2,2,2-trifluoroethoxy)pyrazin-2-yl)phenyl)propanamide 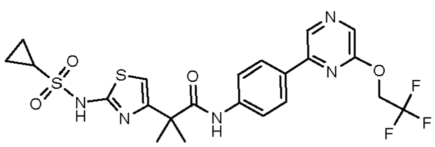 | **Ref T301** | N-(4-(6-ethoxypyrazin-2-yl)phenyl)-2-methyl-2-(2-((1-methylcyclopropane)-1-sulfonamido)thiazol-4-yl)propanamide 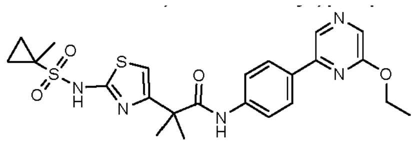 |
| **Ref T128** | N-(4-(5-chloropyridin-3-yl)-2-fluorophenyl)-2-(2-(cyclopropanesulfonamido)thiazol-4-yl)-2-methylpropanamide | **Ref T302** | 2-methyl-2-(2-((1-methylcyclopropane)-1-sulfonamido)thiazol-4-yl)-N-(4-(6-(trifluoromethyl)pyrazin-2-yl)phenyl)propanamide |

(continued)

| T# | Name/Structure (All examples containing chiral centres are racemates unless stated) | T# | Name/Structure (All examples containing chiral centres are racemates unless stated) |
|---|---|---|---|
| | 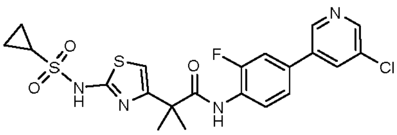 | | 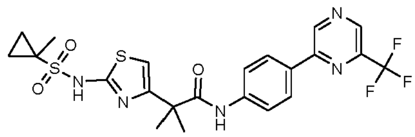 |
| **Ref T129** | N-(4-(5-cyanopyridin-3-yl)-2-fluorophenyl)-2-(2-(cyclopropanesulfonamido)thiazol-4-yl)-2-methylpropanamide 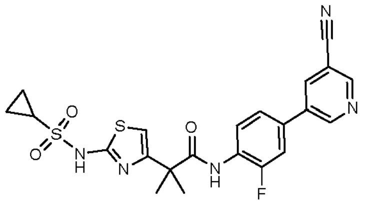 | **Ref T303** | 2-(2-((1,1-dimethylethyl)sulfonamido)thiazol-4-yl)-2-methyl-N-(4-(pyridin-3-yl)phenyl) propanamide 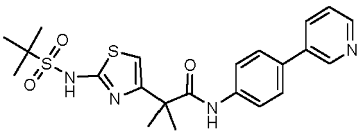 |
| **Ref T130** | 1-(2-(cyclopropanesulfonamido)thiazol-4-yl)-N-(4-(6-methoxypyrazin-2-yl)phenyl)cyclopentane-1-carboxamide 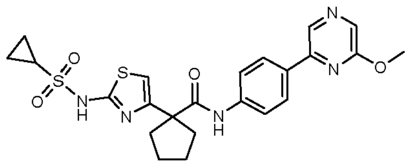 | **Ref T304** | 2-(2-((1,1-dimethylethyl)sulfonamido)thiazol-4-yl)-N-(4-(6-ethoxypyrazin-2-yl)phenyl)-2-methylpropanamide 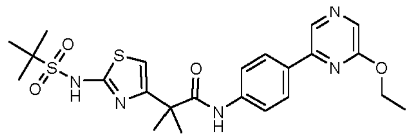 |
| **Ref T131** | 2-(2-(cyclopropanesulfonamido)thiazol-4-yl)-N-(2-fluoro-4-(6-(2,2,2-trifluoroethoxy)pyrazin-2-yl)phenyl)-2-methylpropanamide 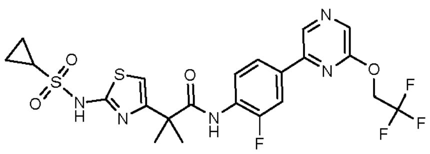 | **Ref T305** | 2-(2-((1,1-dimethylethyl)sulfonamido)thiazol-4-yl)-2-methyl-N-(4-(6-(trifluoromethyl) pyrazin-2-yl)phenyl)propanamide 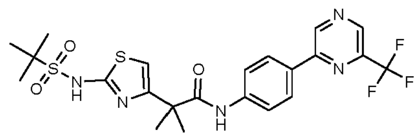 |
| **Ref T132** | 2-(2-(cyclopropanesulfonamido)thiazol-4-yl)-N-(2-fluoro-4-(5-(2,2,2-trifluoroethoxy)pyridin-3-yl)phenyl)-2-methylpropanamide 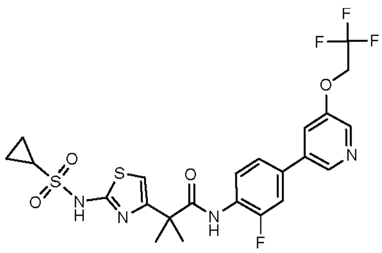 | **Ref T306** | 2-(2-(cyclobutanesulfonamido)thiazol-4-yl)-2-methyl-N-(4-(pyridin-3-yl)phenyl)propanamide 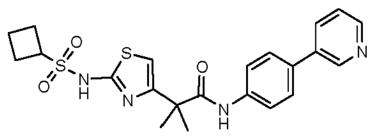 |

(continued)

| T# | Name/Structure (All examples containing chiral centres are racemates unless stated) | T# | Name/Structure (All examples containing chiral centres are racemates unless stated) |
|---|---|---|---|
| **Ref T133** | 2-(2-(cyclopropanesulfonamido)thiazol-4-yl)-N-(2-fluoro-4-(5-(trifluoromethyl)pyridin-3-yl)phenyl)-2-methylpropanamide 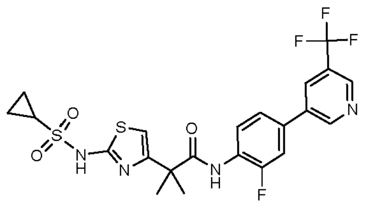 | **Ref T307** | 2-(2-(cyclobutanesulfonamido)thiazol-4-yl)-N-(4-(6-ethoxypyrazin-2-yl)phenyl)-2-methylpropanamide 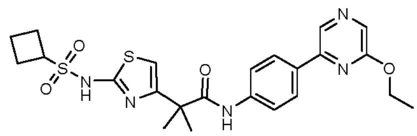 |
| **Ref T134** | 2-(2-(cyclopropanesulfonamido)thiazol-4-yl)-N-(4-(6-ethoxypyrazin-2-yl)-2-fluorophenyl)-2-methylpropanamide 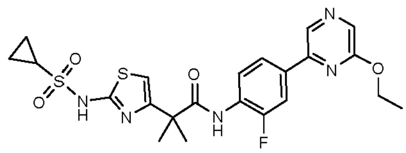 | **Ref T308** | 2-(2-(cyclobutanesulfonamido)thiazol-4-yl)-2-methyl-N-(4-(6-(trifluoromethyl)pyrazin-2-yl)phenyl)propanamide 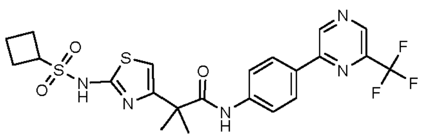 |
| **Ref T135** | 2-(2-(cyclopropanesulfonamido)thiazol-4-yl)-2-methyl-N-(4-(5-(2,2,2-trifluoroethoxy)pyridin-3-yl)phenyl)propanamide 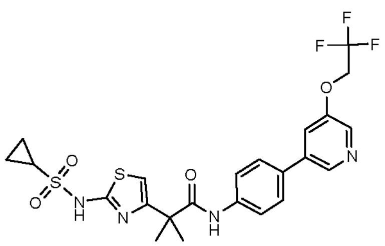 | **Ref T309** | N-(4-(5-cyanopyridin-3-yl)phenyl)-2-(2-(cyclopropanesulfonamido)thiazol-4-yl)-N,2-dimethylpropanamide 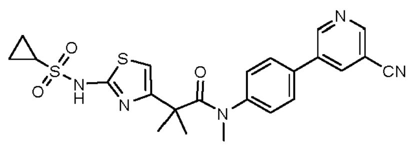 |
| **Ref T136** | 2-(2-(cyclopropanesulfonamido)thiazol-4-yl)-N-(4-(6-ethynylpyrazin-2-yl)phenyl)-2-methylpropanamide 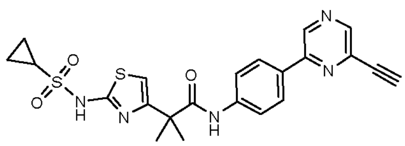 | **Ref T310** | 2-(2-(cyclopropanesulfonamido)thiazol-4-yl)-N,2-dimethyl-N-(4-(6-(trifluoromethyl)pyrazin-2-yl)phenyl)propanamide 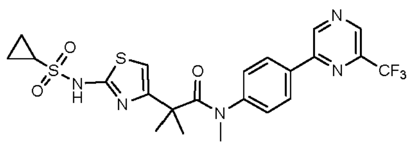 |
| **Ref T137** | 2-(2-(cyclopropanesulfonamido)thiazol-4-yl)-N-(4-(6-ethoxypyrazin-2-yl)-2-methylphenyl)-2-methylpropanamide 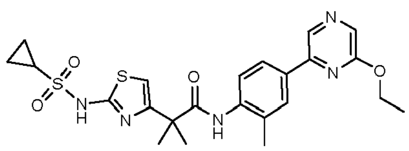 | **Ref T311** | 2-methyl-2-(2-((2-methylpropyl)sulfonamido)thiazol-4-yl)-N-(4-(pyridin-3-yl)phenyl)propanamide 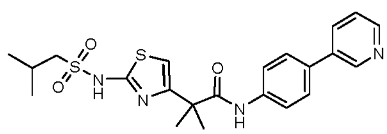 |
| **Ref T138** | N-(4-(6-chloropyrazin-2-yl)-2-methylphenyl)-2-(2-(cyclopropanesulfonamido)thiazol-4-yl)-2-methylpropanamide | **Ref T312** | N-(4-(6-ethoxypyrazin-2-yl)phenyl)-2-methyl-2-(2-((2-methylpropyl)sulfonamido)thiazol-4-yl)propanamide |

(continued)

| T# | Name/Structure (All examples containing chiral centres are racemates unless stated) | T# | Name/Structure (All examples containing chiral centres are racemates unless stated) |
|---|---|---|---|
| | 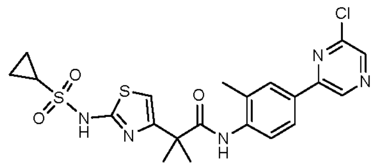 | | 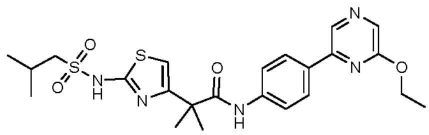 |
| **Ref T139** | 2-(2-(cyclopropanesulfonamido)thiazol-4-yl)-N-(4-(5-(difluoromethoxy)pyridin-3-yl)-2-fluorophenyl)-2-methylpropanamide 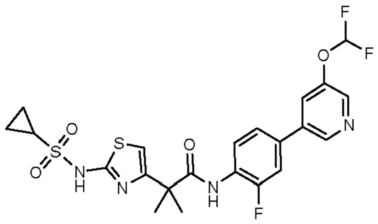 | **Ref T313** | 2-methyl-2-(2-((2-methylpropyl)sulfonamido)thiazol-4-yl)-N-(4-(6-(trifluoromethyl)pyrazin-2-yl)phenyl)propanamide 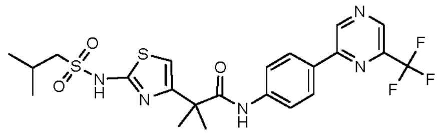 |
| **Ref T140** | 2-(2-(cyclopropanesulfonamido)thiazol-4-yl)-2-methyl-N-(5-(pyrazin-2-yl)pyri din-2-yl)propanamide 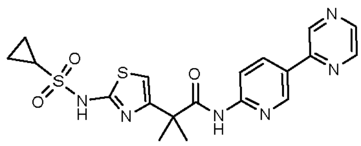 | **Ref T314** | N-(4-(5-chloropyridin-3-yl)phenyl)-2-(2-(cyclopropanesulfonamido)-5-methylthiazol-4-yl)-2-methylpropanamide 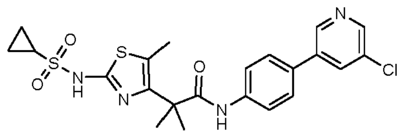 |
| **Ref T141** | N-(5-(6-cyclobutoxypyrazin-2-yl)pyridin-2-yl)-2-(2-(cyclopropanesulfonamido)thiazol-4-yl)-2-methylpropanamide 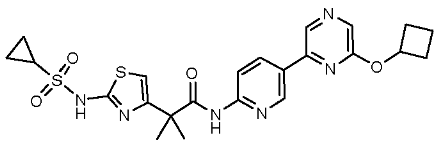 | **Ref T315** | 2-(2-(cyclopropanesulfonamido)thiazol-4-yl)-N-methyl-N-(4-(pyridin-3-yl)phenyl)butanamide 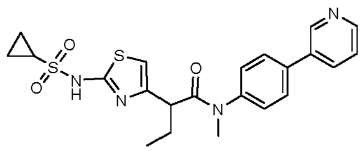 |
| **Ref T142** | 2-(2-(cyclopropanesulfonamido)thiazol-4-yl)-N-(5-(6-cyclopropoxypyrazin-2-yl)pyridin-2-yl)-2-methylpropanamide 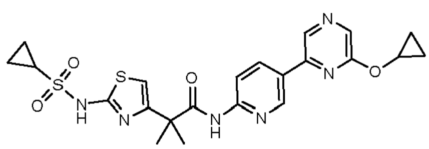 | **Ref T316** | N-(4-(5-cyanopyridin-3-yl)phenyl)-2-(2-(cyclopropanesulfonamido)thiazol-4-yl)-N-methylbutanamide 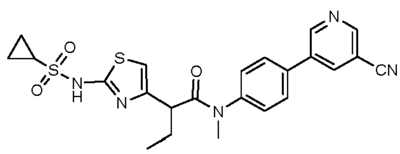 |
| **Ref T143** | 2-(2-(cyclopropanesulfonamido)thiazol-4-yl)-N-(5-(6-isopropoxypyrazin-2-yl)pyridin-2-yl)-2-methylpropanamide 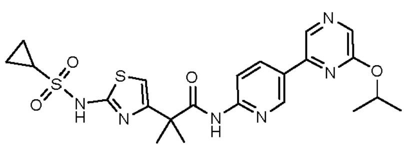 | **Ref T317** | 2-(2-(cyclopropanesulfonamido)-5-methylthiazol-4-yl)-N-(4-(6-ethoxypyrazin-2-yl)phenyl)-2-methylpropanamide 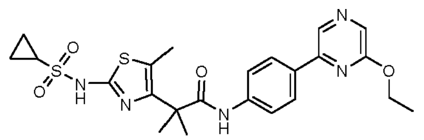 |

(continued)

| T# | Name/Structure (All examples containing chiral centres are racemates unless stated) | T# | Name/Structure (All examples containing chiral centres are racemates unless stated) |
|---|---|---|---|
| **Ref T144** | N-([3,3'-bipyridin]-6-yl)-2-(2-(cyclopropanesulfonamido)thiazol-4-yl)-2-ethylbutanamide 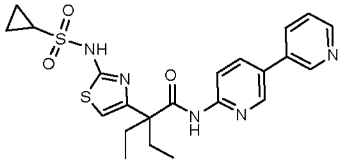 | **Ref T318** | N-(4-(5-chloropyridin-3-yl)phenyl)-2-(2-(cyclopropanesulfonamido)thiazol-4-yl)-N,2-dimethylpropanamide 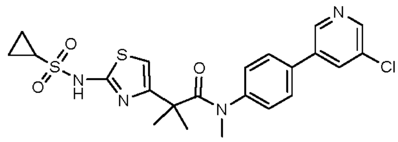 |
| **Ref T145** | 2-(2-(cyclopropanesulfonamido)thiazol-4-yl)-N-(5'-ethoxy-[3,3'-bipyridin]-6-yl)-2-ethylbutanamide 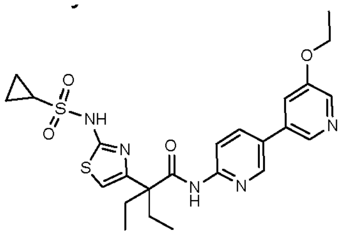 | **Ref T319** | 2-(2-(cyclopropanesulfonamido)-5-methylthiazol-4-yl)-2-methyl-N-(4-(pyridin-3-yl)phenyl)propanamide 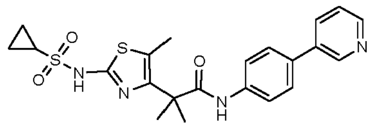 |
| **Ref T146** | 2-(2-(cyclopropanesulfonamido)thiazol-4-yl)-2-methyl-N-(5'-propoxy-[3,3'-bipyridin]-6-yl)propanamide 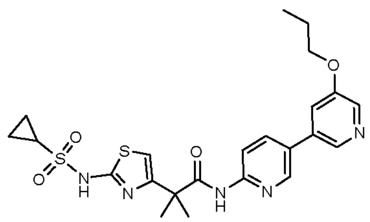 | **Ref T320** | N-(4-(5-cyanopyridin-3-yl)-2,6-dimethylphenyl)-2-(2-(cyclopropanesulfonamido)thiazol-4-yl)-2-methylpropanamide 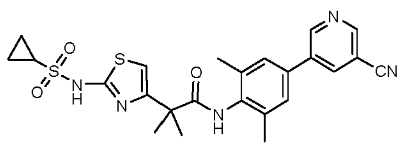 |
| **Ref T147** | 2-(2-(cyclopropanesulfonamido)thiazol-4-yl)-2-ethyl-N-(5-(6-(trifluoromethyl)pyrazin-2-yl)pyridin-2-yl)butanamide 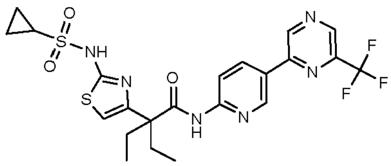 | **Ref T321** | N-(4-(5-chloropyridin-3-yl)-2,6-dimethylphenyl)-2-(2-(cyclopropanesulfonamido)thiazol-4-yl)-2-methylpropanamide 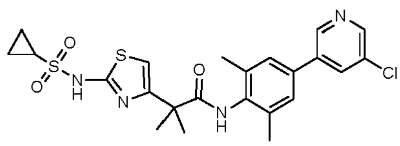 |
| **Ref T148** | N-([3,3'-bipyridin]-6-yl)-2-(2-(cyclopropanesulfonamido)thiazol-4-yl)-2-methylpropanamide 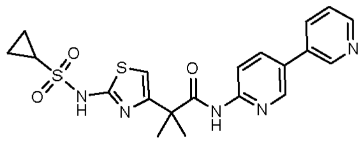 | **Ref T322** | N-(4-(5-cyanopyridin-3-yl)-3-methylphenyl)-2-(2-(cyclopropanesulfonamido)thiazol-4-yl)-2-methylpropanamide 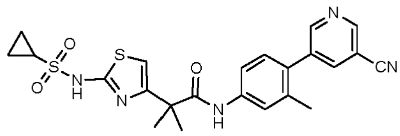 |
| **Ref T149** | 2-(2-(cyclopropanesulfonamido)thiazol-4-yl)-N-(2-methoxy-4-(pyridin-3-yl)phenyl)-2-methylpropanamide | **Ref T422** | 2-(2-(cyclopropanesulfonamido)thiazol-4-yl)-*N*-(5-(6-ethoxypyrazin-2-yl)pyridin-2-yl)-2,2-difluoroacetamide |

(continued)

| T# | Name/Structure (All examples containing chiral centres are racemates unless stated) | T# | Name/Structure (All examples containing chiral centres are racemates unless stated) |
|---|---|---|---|
| | 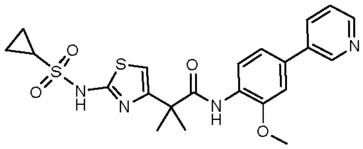 | | 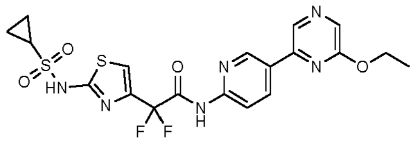 |
| **Ref T150** | 2-(2-(cyclopropanesulfonamido)thiazol-4-yl)-N-(3-methoxy-4-(pyridin-3-yl)phenyl)-2-methylpropanamide 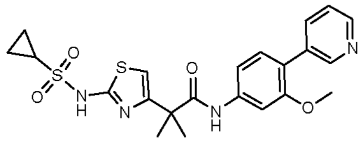 | **Ref T423** | 2-(2-(cyclopropanesulfonamido)thiazol-4-yl)-*N*-(4-(6-ethoxypyrazin-2-yl)-2-fluorophenyl)acetamide 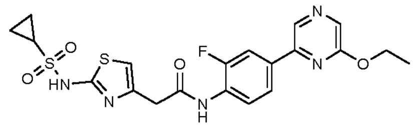 |
| **Ref T151** | 2-(2-(cyclopropanesulfonamido)thiazol-4-yl)-N-(3-fluoro-4-(pyridin-3-yl)phenyl)-2-methylpropanamide 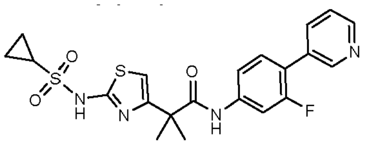 | **Ref T424** | 2-(2-(cyclopropanesulfonamido)thiazol-4-yl)-*N*-(4-(6-ethoxypyrazin-2-yl)phenyl)acetamide 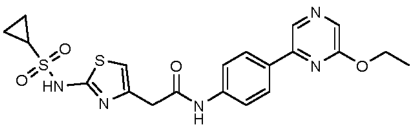 |
| **Ref T152** | N-(3-cyano-4-(pyridin-3-yl)phenyl)-2-(2-(cyclopropanesulfonamido)thiazol-4-yl)-2-methylpropanamide 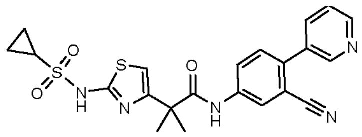 | **Ref T425** | 2-methyl-2-(2-(methylsulfonamido)thiazol-4-yl)-*N*-(5-(6-(trifluoromethyl)pyrazin-2-yl)pyridin-2-yl)propanamide 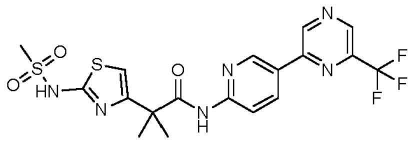 |
| **Ref T153** | N-(3-chloro-4-(pyridin-3-yl)phenyl)-2-(2-(cyclopropanesulfonamido)thiazol-4-yl)-2-methylpropanamide 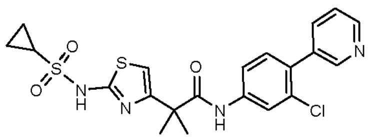 | **Ref T426** | *N*-(2-fluoro-4-(5-(trifluoromethyl)pyridin-3-yl)phenyl)-2-methyl-2-(2-(methylsulfonamido)thiazol-4-yl)propanamide 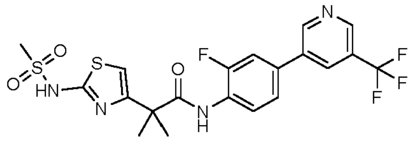 |
| **Ref T154** | N-(4-(6-cyanopyrazin-2-yl)phenyl)-2-(2-(cyclopropanesulfonamido)thiazol-4-yl)-2-methylpropanamide 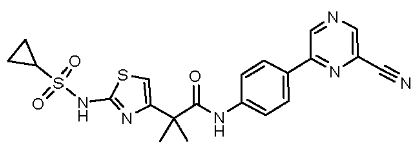 | **Ref T427** | 2-(2-((cyclopropylmethyl)sulfonamido)thiazol-4-yl)-*N*-(2-fluoro-4-(5-(trifluoromethyl)pyridin-3-yl)phenyl)-2-methylpropanamide 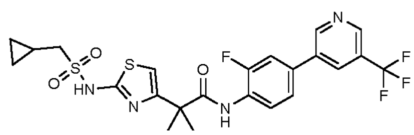 |
| **Ref** T**155** | N-(4-(6-chloropyrazin-2-yl)phenyl)-2-(2-(cyclopropanesulfonamido)thiazol-4-yl)-2-methylpropanamide | **Ref T428** | N-(4-(5-chloro-4-methylpyridin-3-yl)phenyl)-2-(2-(cyclopropanesulfonamido)thiazol-4-yl)-2-methylpropanamide |

(continued)

| T# | Name/Structure (All examples containing chiral centres are racemates unless stated) | T# | Name/Structure (All examples containing chiral centres are racemates unless stated) |
|---|---|---|---|
| | 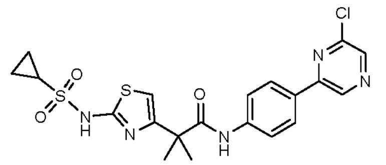 | | 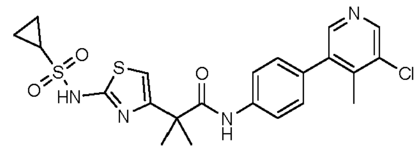 |
| **Ref T156** | 2-(2-(cyclopropanesulfonamido)thiazol-4-yl)-2-ethyl-N-(4-(5-fluoropyridin-3-yl)phenyl)butanamide 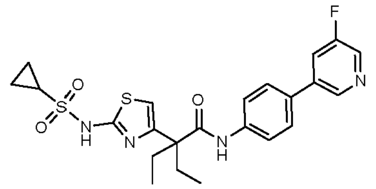 | **Ref T429** | *N*-(4-(6-ethoxypyrazin-2-yl)-2-(trifluoromethyl)phenyl)-2-methyl-2-(2-(methylsulfonamido)thiazol-4-yl)propanamide 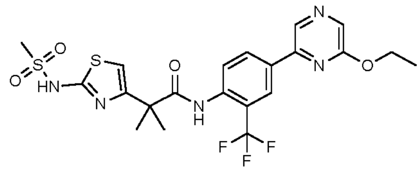 |
| **Ref T157** | 2-(2-(cyclopropanesulfonamido)thiazol-4-yl)-2-methyl-N-(4-(5-propoxypyridin-3-yl)phenyl)propanamide 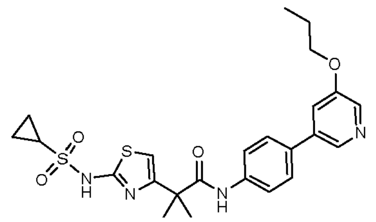 | **Ref T430** | 2-(2-((cyclopropylmethyl)sulfonamido)thiazol-4-yl)-*N*-(2-fluoro-4-(6-(trifluoromethyl)pyrazin-2-yl)phenyl)-2-methylpropanamide 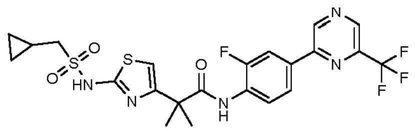 |
| **Ref T158** | 2-(2-(cyclopropanesulfonamido)thiazol-4-yl)-N-(4-(5-isopropoxypyridin-3-yl)phenyl)-2-methylpropanamide 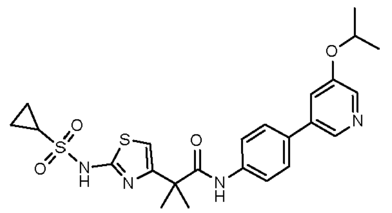 | **Ref T431** | *N*-(4-(6-ethoxypyrazin-2-yl)-2-fluorophenyl)-2-methyl-2-(2-(methylsulfonamido)thiazol-4-yl)propanamide 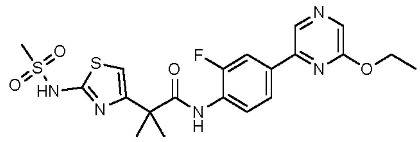 |
| **Ref T159** | 2-(2-(cyclopropanesulfonamido)thiazol-4-yl)-N-(2-fluoro-4-(5-isopropoxypyridin-3-yl)phenyl)-2-methylpropanamide 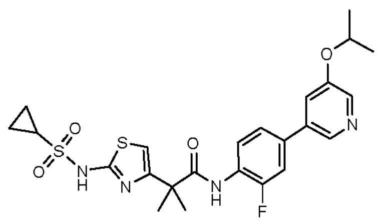 | **Ref T432** | *N*-(4-(6-ethoxypyrazin-2-yl)-2-fluorophenyl)-2-(2-((2-methoxyethyl)sulfonamido)thiazol-4-yl)-2-methylpropanamide 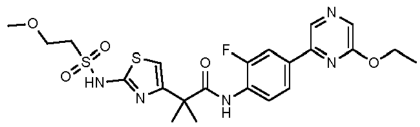 |
| **Ref T160** | N-(4-(6-chloropyrazin-2-yl)phenyl)-2-(2-(cyclopropanesulfonamido)thiazol-4-yl)-2-ethylbutanamide | **Ref T433** | 2-(2-((cyclopropylmethyl)sulfonamido)thiazol-4-yl)-*N*-(4-(6-ethoxypyrazin-2-yl)-2-fluorophenyl)-2-methylpropanamide |

(continued)

| T# | Name/Structure (All examples containing chiral centres are racemates unless stated) | T# | Name/Structure (All examples containing chiral centres are racemates unless stated) |
|---|---|---|---|
| | 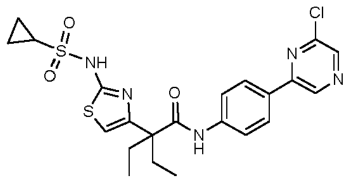 | | 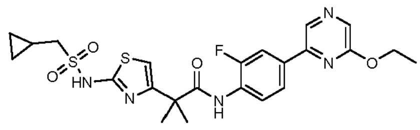 |
| **Ref T161** | N-(4-(6-cyanopyrazin-2-yl)phenyl)-2-(2-(cyclopropanesulfonamido)thiazol-4-yl)-2-ethylbutanamide 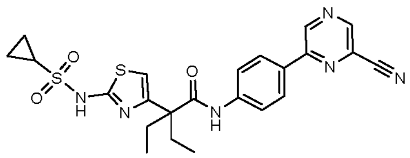 | **Ref T434** | *N*-(4-(6-ethoxypyrazin-2-yl)phenyl)-2-methyl-2-(2-(methylsulfonamido)thiazol-4-yl)propanamide 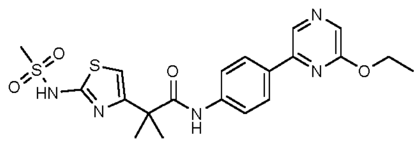 |
| **Ref T163** | 2-methyl-2-(2-(methylsulfonamido)thiazol-4-yl)-N-(4-(pyridin-3-yl)phenyl)propanamide 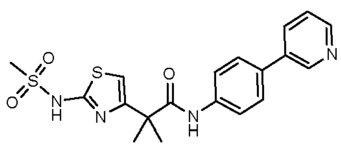 | **Ref T435** | 2-(2-(cyclopropanesulfonamido)thiazol-4-yl)-*N*-(5-(6-ethoxypyrazin-2-yl)-3-fluoropyridin-2-yl)-4-methoxybutanamide 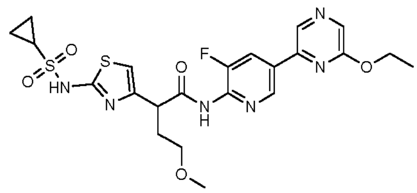 |
| **Ref T164** | 2-(2-(cyclopropanesulfonamido)thiazol-4-yl)-N,2-dimethyl-N-(4-(pyridin-3-yl)phenyl)propanamide 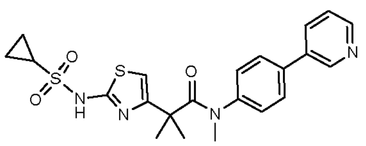 | **Ref T436** | 2-(2-(cyclopropanesulfonamido)thiazol-4-yl)-*N*-(5-(6-ethoxypyrazin-2-yl)pyridin-2-yl)-4-methoxybutanamide 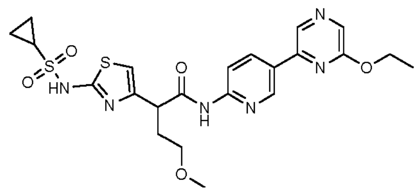 |
| **Ref T169** | 2-(cyclopropanesulfonamido)-N-(5-(6-(trifluoromethyl)pyrazin-2-yl)pyridin-2-yl)-5,6-dihydro-4H-cyclopenta[d]thiazole-4-carboxamide 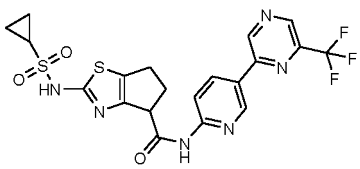 | **Ref T437** | 2-(2-(cyclopropanesulfonamido)thiazol-4-yl)-4-methoxy-N-(4-(6-(trifluoromethyl)pyrazin-2-yl)phenyl)butanamide 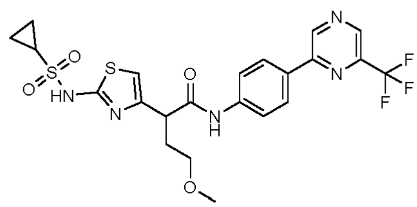 |
| **Ref T170** | 2-(2-(cyclopropanesulfonamido)thiazol-4-yl)-4-methoxy-N-(5-(pyrazin-2-yl)pyri din-2-yl)butanamide | **Ref T438** | 2-(2-(cyclopropanesulfonamido)thiazol-4-yl)-*N*-(5-(6-ethoxypyrazin-2-yl)-3-methylpyridin-2-yl)butanamide |

(continued)

| T# | Name/Structure (All examples containing chiral centres are racemates unless stated) | T# | Name/Structure (All examples containing chiral centres are racemates unless stated) |
|---|---|---|---|
| | 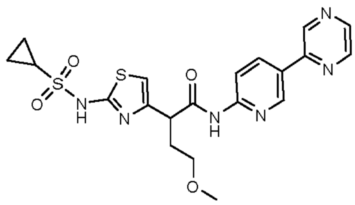 | | 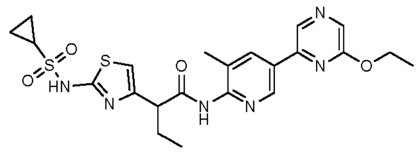 |
| **Ref T171** | 2-(2-(cyclopropanesulfonamido)thiazol-4-yl)-4-methoxy-N-(5'-methoxy-[3,3'-bipyridin]-6-yl)butanamide 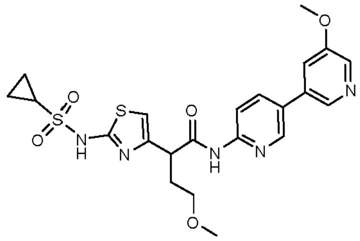 | **Ref T439** | *N*-(2-chloro-4-(6-ethoxypyrazin-2-yl)phenyl)-2-(2-(cyclopropanesulfonamido)thiazol-4-yl)butanamide 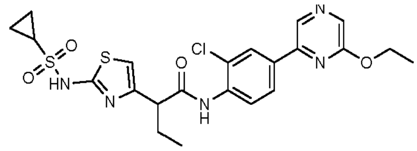 |
| **Ref T172** | 2-(2-(cyclopropanesulfonamido)thiazol-4-yl)-2-isopropoxy-N-(5-(6-(trifluoromethyl)pyrazin-2-yl) pyridin-2-yl)acetamide 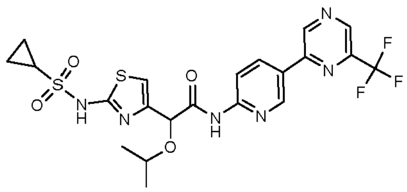 | **Ref T440** | *N*-(2-cyano-4-(6-ethoxypyrazin-2-yl)phenyl)-2-(2-(cyclopropanesulfonamido)thiazol-4-yl)butanamide 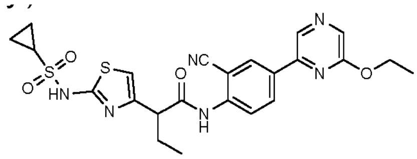 |
| **Ref T173** | 2-(2-(cyclopropanesulfonamido)thiazol-4-yl)-N-(5-(6-propoxypyrazin-2-yl)pyridin-2-yl)butanamide 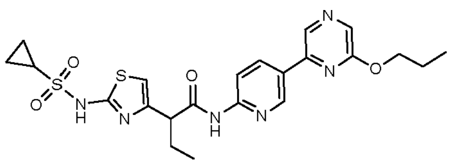 | **Ref T441** | 2-(2-(cyclopropanesulfonamido)thiazol-4-yl)-*N*-(4-(6-ethoxypyrazin-2-yl)-2-methylphenyl)butanamide 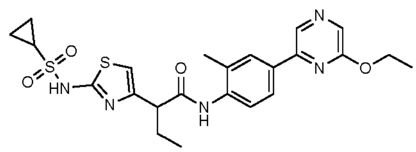 |
| **Ref T174** | 2-(2-(cyclopropanesulfonamido)thiazol-4-yl)-N-(5-(6-isopropoxypyrazin-2-yl)pyridin-2-yl) butanamide 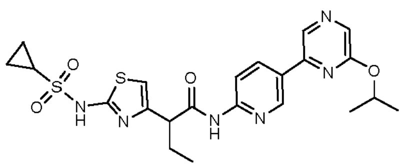 | **Ref T442** | 2-(2-(cyclopropanesulfonamido)thiazol-4-yl)-*N*-(4-(6-ethoxypyrazin-2-yl)-2-(trifluoromethoxy)phenyl)butanamide 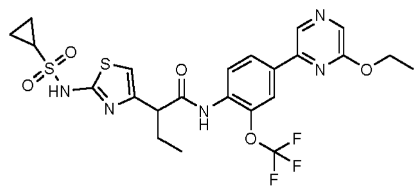 |
| **Ref T175** | 2-(2-(cyclopropanesulfonamido)thiazol-4-yl)-N-(5-(6-(trifluoromethyl)pyrazin-2-yl)pyridin-2-yl)butanamide | **Ref T443** | 2-(2-(cyclopropanesulfonamido)thiazol-4-yl)-*N*-(4-(6-ethoxypyrazin-2-yl)-2-methoxyphenyl)butanamide |

(continued)

| T# | Name/Structure (All examples containing chiral centres are racemates unless stated) | T# | Name/Structure (All examples containing chiral centres are racemates unless stated) |
|---|---|---|---|
| | 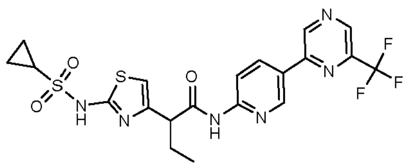 | | 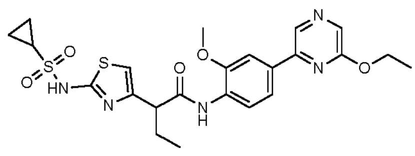 |
| **Ref T176** | 2-(2-(cyclopropanesulfonamido)thiazol-4-yl)-N-(5-(6-methoxypyrazin-2-yl)pyridin-2-yl)butanamide 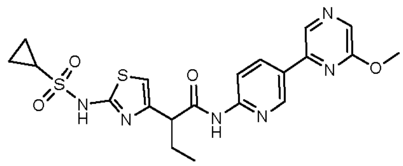 | **Ref T445** | 2-(2-(cyclopropanesulfonamido)thiazol-4-yl)-*N*-(5-(6-ethoxypyrazin-2-yl)-3-fluoropyridin-2-yl)-2-methoxyacetamide 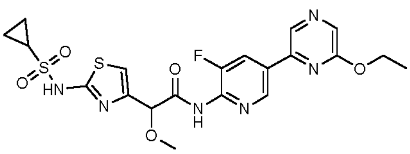 |
| **Ref T177** | 2-(2-(cyclopropanesulfonamido)thiazol-4-yl)-N-(5-(6-ethoxypyrazin-2-yl)pyridin-2-yl)butanamide 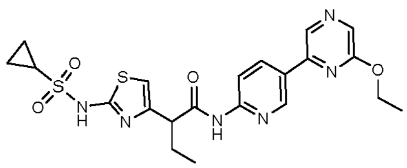 | **Ref T446** | 2-(2-(cyclopropanesulfonamido)thiazol-4-yl)-*N*-(5-(6-ethoxypyrazin-2-yl)pyridin-2-yl)-2-methoxyacetamide 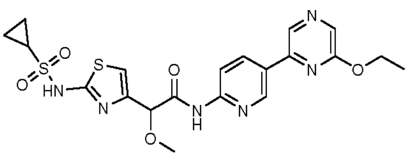 |
| **Ref T178** | N-(5-(6-cyanopyrazin-2-yl)pyridin-2-yl)-2-(2-(cyclopropanesulfonamido)thiazol-4-vl)butanamide 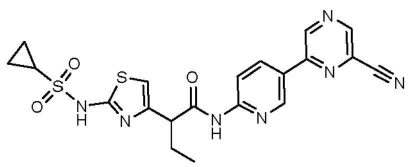 | **Ref T447** | 2-(2-(cyclopropanesulfonamido)thiazol-4-yl)-*N*-(4-(5-fluoropyridin-3-yl)-2-(trifluoromethyl)phenyl)-2-methoxyacetamide 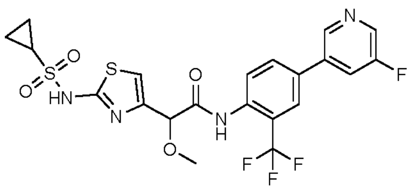 |
| **Ref T179** | 2-(2-(cyclopropanesulfonamido)thiazol-4-yl)-N-(5'-fluoro-[3,3'-bipyridin]-6-yl)butanamide 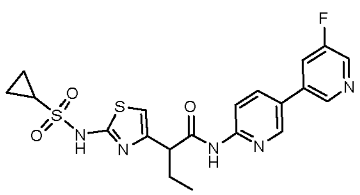 | **Ref T448** | 2-(2-(cyclopropanesulfonamido)thiazol-4-yl)-*N*-(2-fluoro-4-(5-(trifluoromethyl)pyridin-3-yl)phenyl)-2-methoxyacetamide 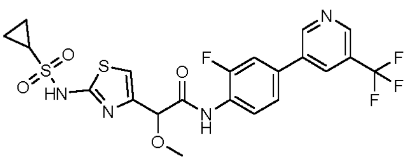 |
| **Ref T180** | N-(5'-cyano-[3,3'-bipyridin]-6-yl)-2-(2-(cyclopropanesulfonamido)thiazol-4-yl)butanamide | **Ref T449** | 2-(2-(cyclopropanesulfonamido)thiazol-4-yl)-*N*-(4-(6-ethoxypyrazin-2-yl)-2-(trifluoromethyl)phenyl)-2-methoxyacetamide |

(continued)

| T# | Name/Structure (All examples containing chiral centres are racemates unless stated) | T# | Name/Structure (All examples containing chiral centres are racemates unless stated) |
|---|---|---|---|
| | 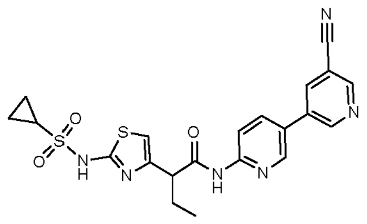 | | 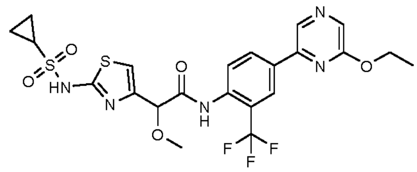 |
| **Ref T181** | 2-(2-(cyclopropanesulfonamido)thiazol-4-yl)-N-(5-phenylpyridin-2-yl)butanamide 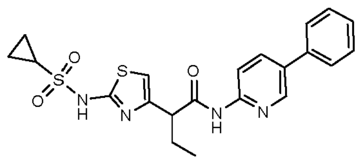 | **Ref T450** | *N*-(2-chloro-4-(6-ethoxypyrazin-2-yl)phenyl)-2-(2-(cyclopropanesulfonamido)thiazol-4-yl)-2-methoxyacetamide 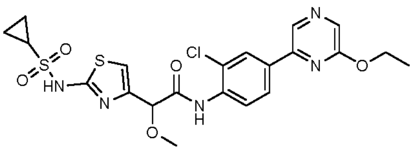 |
| **Ref T182** | 2-(2-(cyclopropanesulfonamido)thiazol-4-yl)-N-(5-(6-(2,2,2-trifluoroethoxy)pyrazin-2-yl)pyridin-2-yl)butanamide 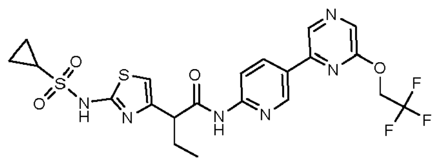 | **Ref T451** | *N*-(2-cyano-4-(6-ethoxypyrazin-2-yl)phenyl)-2-(2-(cyclopropanesulfonamido)thiazol-4-yl)-2-methoxyacetamide 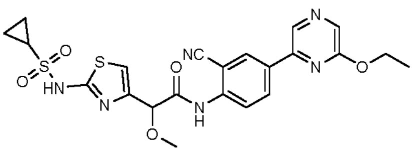 |
| **Ref T183** | 2-(2-(cyclopropanesulfonamido)thiazol-4-yl)-N-(5-(6-ethoxypyrazin-2-yl)-3-fluoropyridin-2-yl)butanamide 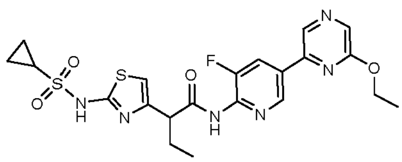 | **Ref T452** | *N*-(2-fluoro-4-(6-(trifluoromethyl)pyrazin-2-yl)phenyl)-2-methoxy-2-(2-(methylsulfonamido)thiazol-4-yl)acetamide 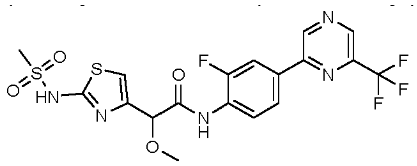 |
| **Ref T184** | N-(5-(6-cyanopyrazin-2-yl)-3-fluoropyridin-2-yl)-2-(2-(cyclopropanesulfonamido)thiazol-4-yl) butanamide 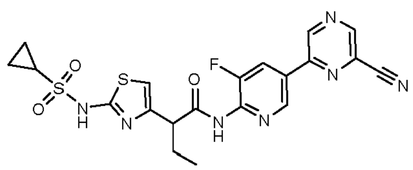 | **Ref T453** | 2-(2-(cyclopropanesulfonamido)thiazol-4-yl)-*N*-(4-(6-ethoxypyrazin-2-yl)-2,6-difluorophenyl)-2-methoxyacetamide 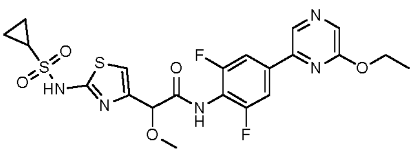 |
| **Ref T185** | 2-(2-(cyclopropanesulfonamido)thiazol-4-yl)-N-(5'-(2,2,2-trifluoroethoxy)-[3,3'-bipyridin]-6-yl)butanamide | **Ref T454** | 2-(2-(cyclopropanesulfonamido)thiazol-4-yl)-*N*-(4-(6-ethoxypyrazin-2-yl)-2-(trifluoromethoxy)phenyl)-2-methoxyacetamide |

(continued)

| T# | Name/Structure (All examples containing chiral centres are racemates unless stated) | T# | Name/Structure (All examples containing chiral centres are racemates unless stated) |
|---|---|---|---|
| | 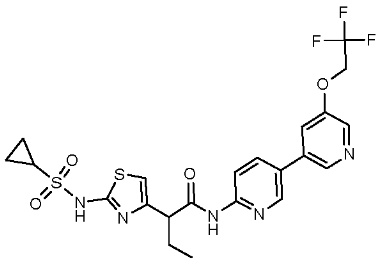 | | 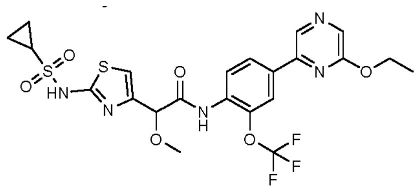 |
| **Ref T186** | 2-(2-(cyclopropanesulfonamido)thiazol-4-yl)-N-(5'-(difluoromethoxy)-[3,3'-bipyridin]-6-yl) butanamide 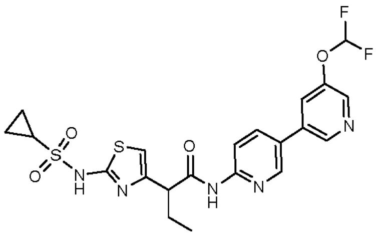 | **Ref T455** | *N*-(4-(6-ethoxypyrazin-2-yl)phenyl)-2-methoxy-2-(2-(methylsulfonamido)thiazol-4-yl) acetamide 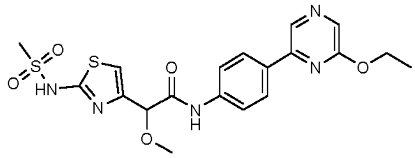 |
| **Ref T187** | N-(5-(6-chloropyrazin-2-yl)pyridin-2-yl)-2-(2-(cyclopropanesulfonamido)thiazol-4-yl)butanamide 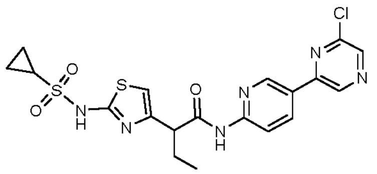 | **Ref T456** | **Single enantiomer - stereochemistry unassigned** 2-(2-(cyclopropanesulfonamido)thiazol-4-yl)-*N*-(5-(6-ethoxypyrazin-2-yl)-3-fluoropyridin-2-yl)butanamide 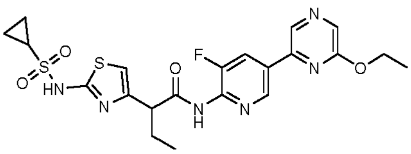 |
| **Ref T188** | 2-(2-(cyclopropanesulfonamido)thiazol-4-yl)-N-(2,3-difluoro-4-(pyridin-3-yl)phenyl)acetamide 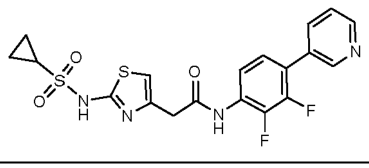 | **Ref T457** | **Single enantiomer - stereochemistry unassigned** 2-(2-(cyclopropanesulfonamido)thiazol-4-yl)-*N*-(5-(6-ethoxypyrazin-2-yl)-3-fluoropyridin-2-yl)butanamide 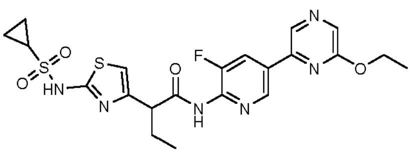 |
| **Ref T189** | 2-(2-(cyclopropanesulfonamido)thiazol-4-yl)-N-(4-(6-ethoxypyrazin-2-yl)phenyl)butanamide 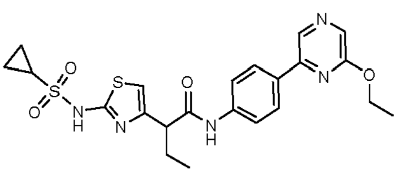 | **Ref T458** | **Single enantiomer - stereochemistry unassigned** 2-(2-(cyclopropanesulfonamido)thiazol-4-yl)-N-(2-fluoro-4-(6-(trifluoromethyl)pyrazin-2-yl)phenyl)-2-methoxyacetamide 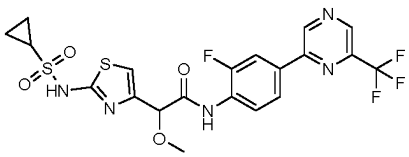 |

(continued)

| T# | Name/Structure (All examples containing chiral centres are racemates unless stated) | T# | Name/Structure (All examples containing chiral centres are racemates unless stated) |
|---|---|---|---|
| Ref T190 | 2-(2-(cyclopropanesulfonamido)thiazol-4-yl)-4-methoxy-N-(4-(6-methoxypyrazin-2-yl)phenyl)butanamide 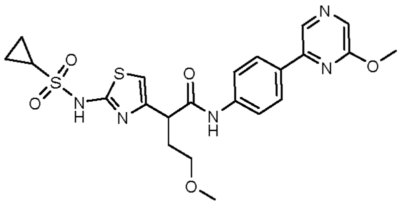 | Ref T459 | **Single enantiomer - stereochemistry unassigned** 2-(2-(cyclopropanesulfonamido)thiazol-4-yl)-N-(2-fluoro-4-(6-(trifluoromethyl)pyrazin-2-yl)phenyl)-2-methoxyacetamide 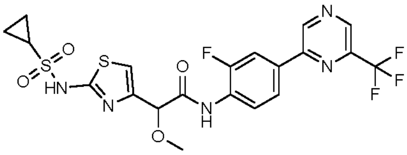 |
| Ref T191 | 2-(2-(cyclopropanesulfonamido)thiazol-4-yl)-N-(4-(5-fluoropyridin-3-yl)phenyl)-4-methoxybutanamide 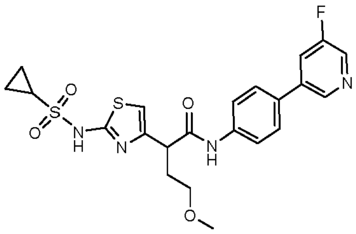 | Ref T460 | 4-(2-(cyclopropanesulfonamido)thiazol-4-yl)-N-(4-(6-ethoxypyrazin-2-yl)-2-fluorophenyl)tetrahydro-2H-pyran-4-carboxamide 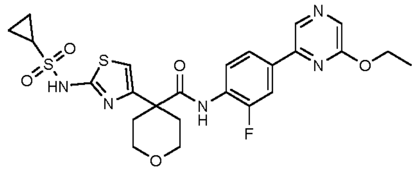 |
| Ref T192 | 2-(2-(cyclopropanesulfonamido)thiazol-4-yl)-N-(4-(6-ethoxypyrazin-2-yl)phenyl)propanamide 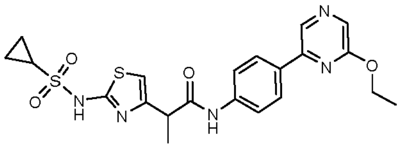 | Ref T461 | 4-(2-(cyclopropanesulfonamido)thiazol-4-yl)-N-(5-(6-(trifluoromethyl)pyrazin-2-yl)pyridin-2-yl)tetrahydro-2H-pyran-4-carboxamide 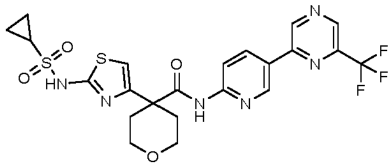 |
| Ref T193 | 2-(2-(cyclopropanesulfonamido)thiazol-4-yl)-N-(4-(5-(trifluoromethyl)pyridin-3-yl)phenyl)butanamide 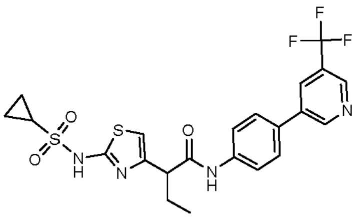 | Ref T462 | 4-(2-(cyclopropanesulfonamido)thiazol-4-yl)-N-(5-(6-ethoxypyrazin-2-yl)pyridin-2-yl) tetrahydro-2H-pyran-4-carboxamide 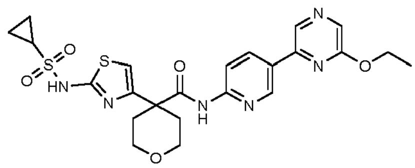 |
| Ref T194 | N-(4-(5-cyanopyridin-3-yl)phenyl)-2-(2-(cyclopropanesulfonamido)thiazol-4-yl)butanamide | Ref T463 | N-(4-(1-(4-(5-methoxypyridin-3-yl)phenyl)-2-oxopyrrolidin-3-yl)thiazol-2-yl)cyclopropanesulfonamide |

(continued)

| T# | Name/Structure (All examples containing chiral centres are racemates unless stated) | T# | Name/Structure (All examples containing chiral centres are racemates unless stated) |
|---|---|---|---|
| | 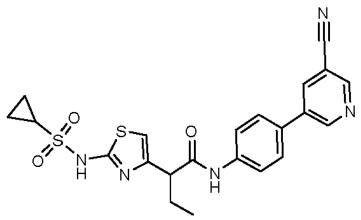 | | 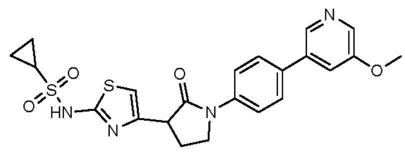 |
| **Ref T195** | 2-(2-(cyclopropanesulfonamido)thiazol-4-yl)-N-(4-(6-(2,2,2-trifluoroethoxy)pyrazin-2-yl)phenyl)butanamide 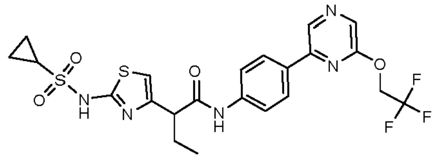 | **Ref T464** | 2-(2-(cyclopropanesulfonamido)thiazol-4-yl)-2-methyl-N-(5-(6-methylpyrazin-2-yl)pyridin-2-yl)propanamide 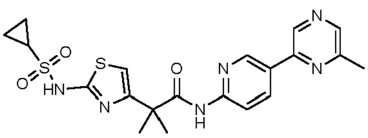 |
| **Ref T196** | 2-(2-(cyclopropanesulfonamido)thiazol-4-yl)-N-(2-fluoro-4-(pyrazin-2-yl)phenyl)butanamide 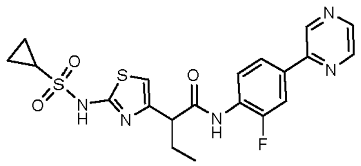 | **Ref T465** | N-(4-(6-cyanopyrazin-2-yl)-2-methylphenyl)-2-(2-(cyclopropanesulfonamido)thiazol-4-yl)-2-methylpropanamide 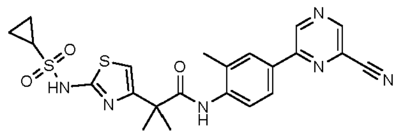 |

**Method 2c: Telescoped boronate formation and Suzuki coupling on sulfonamide scaffold**

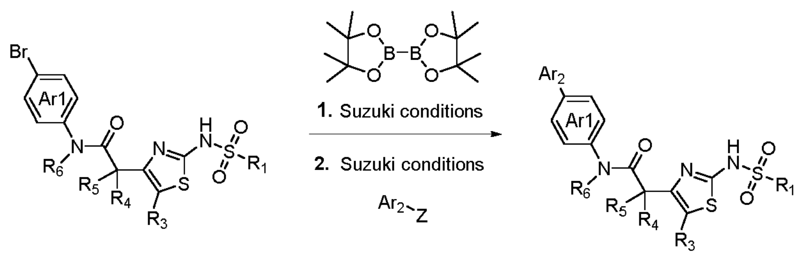


Z = Br, Cl

A suspension of Ar1-Br (1 eq), bispin (1.1 eq) and KOAc (2 eq) in dioxane (50 volumes) was degassed ($N_2$, 5 mins, x 3) then charged with $PdCl_2$(dppf)-DCM adduct (5 mol %) and again degassed ($N_2$, 5 mins, x 3). The reaction mixture was heated to 90 °C for 1 hr and then the reaction was allowed to cool to RT. Ar2-Z (1 eq) and 2M $K_2CO_3$ (aq, 2 eq) were added and the reaction was then heated to 90 °C for 18 hrs. The reaction was allowed to cool to RT, an aqueous work up was performed and the crude compound was purified by normal phase chromatography.

Reference Example: 2-Amino-2-(2-(cyclopropanesulfonamido)thiazol-4-yl)-N-(4-(6-ethoxypyrazin-2-yl)phenyl)acetamide **T325**

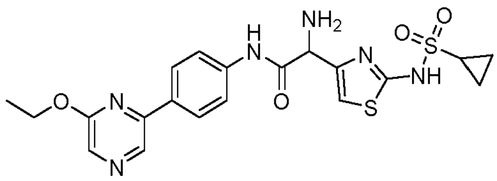

Reference Example: 2-acetamido-2-(2-(cyclopropanesulfonamido)thiazol-4-yl)-N-(4-(6-ethoxypyrazin-2-yl)phenyl) acetamide **T326**

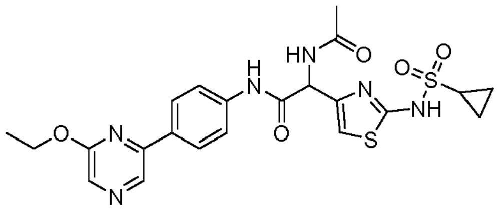

Reference Example: methyl (1-(2-(cyclopropanesulfonamido)thiazol-4-yl)-2-((4-(6-ethoxypyrazin-2-yl)phenyl)amino)-2-oxoethyl)carbamate **T327**

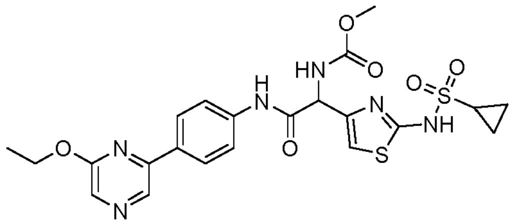

Reference Example: 2-(2-(cyclopropanesulfonamido)thiazol-4-yl)-2-(dimethylamino)-N-(4-(6-ethoxypyrazin-2-yl)phenyl)acetamide **T328**

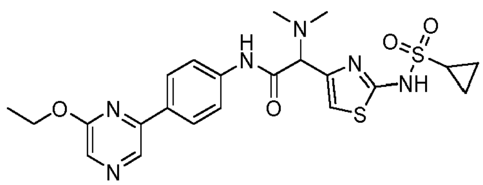

Reference Example: 2-(2-(cyclopropanesulfonamido)thiazol-4-yl)-N-(4-(6-ethoxypyrazin-2-yl)phenyl)-4-hydroxybutanamide **T329**

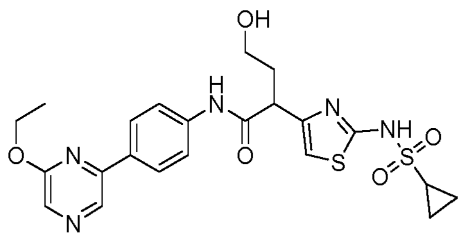

The racemate **T410** was separated by chiral preparative HPLC [Chiralpak® IB (Daicel Ltd.) column (4.6 mm x 25 mm), flow rate 0.5 mL $min^{-1}$ eluting with a mixture of (30% of ethanol) ethanol in heptane + 0.2% $Et_2NH$, UV detection at 254 nm followed by SCX (300 mg) purification (elution with MeOH) to afford:

Reference Example: 2-(2-(cyclopropanesulfonamido)thiazol-4-yl)-N-(4-(6-ethoxypyrazin-2-yl)phenyl)-2-methoxyacetamide **T410**

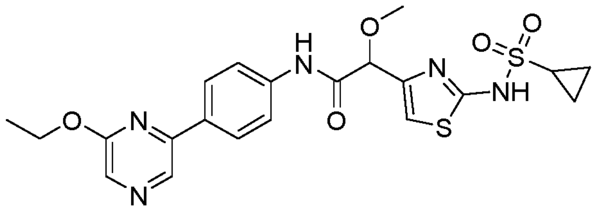

The racemate T410 was prepared using Method 1b was separated by chiral preparative HPLC using a Diacel Chiralpak IB column (30% EtOH (0.1 % DEA) in iso-hexane (0.2% DEA) to afford:

**Peak 1: Stereochemistry of product was unassigned**

2-(2-(cyclopropanesulfonamido)thiazol-4-yl)-N-(4-(6-ethoxypyrazin-2-yl)phenyl)-2-methoxyacetamide **T330.**

**Peak 2: Stereochemistry of product was unassigned**

2-(2-(cyclopropanesulfonamido)thiazol-4-yl)-N-(4-(6-ethoxypyrazin-2-yl)phenyl)-2-methoxyacetamide **T331.**

**Table 7:** Compounds **T332-T416**

| T# | Name/Structure (All examples containing chiral centres are racemates unless stated) | T# | Name/Structure (All examples containing chiral centres are racemates unless stated) |
|---|---|---|---|
| **Ref T332** | 2-(2-((2-methoxyethyl)sulfonamido)thiazol-4-yl)-2-methyl-N-(5-(6-(trifluoromethyl)pyrazin-2-yl)pyridin-2-yl)propanamide 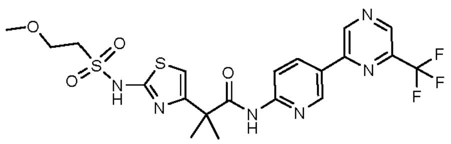 | **Ref T375** | 2-(2-(cyclopropanesulfonamido)thiazol-4-yl)-N-(4-(6-(2-methoxypropan-2-yl)pyrazin-2-yl)phenyl)-2-methylpropanamide 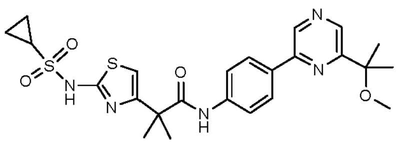 |
| **Ref T333** | 2-(2-(cyclopentanesulfonamido)thiazol-4-yl)-2-methyl-N-(4-(pyridin-3-yl)phenyl)propanamide 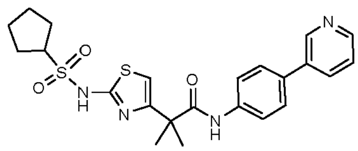 | **Ref T376** | 2-(5-chloro-2-(cyclopropanesulfonamido)thiazol-4-yl)-N-(4-(6-ethoxypyrazin-2-yl)phenyl)-2-methylpropanamide 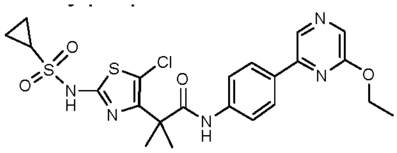 |
| **Ref T334** | 2-(2-(cyclopentanesulfonamido)thiazol-4-yl)-2-methyl-N-(4-(6-(trifluoromethyl)pyrazin-2-yl)phenyl)propanamide 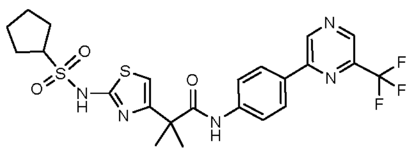 | **Ref T377** | 2-(2-(cyclopropanesulfonamido)-5-methoxythiazol-4-yl)-N-(4-(6-ethoxypyrazin-2-yl)phenyl)-2-methylpropanamide 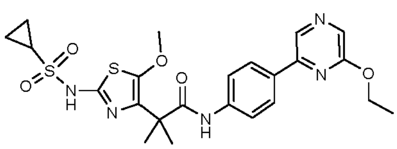 |

(continued)

| T# | Name/Structure (All examples containing chiral centres are racemates unless stated) | T# | Name/Structure (All examples containing chiral centres are racemates unless stated) |
|---|---|---|---|
| **Ref T335** | 2-(2-(cyclopentanesulfonamido)thiazol-4-yl)-N-(4-(6-ethoxypyrazin-2-yl)phenyl)-2-methylpropanamide 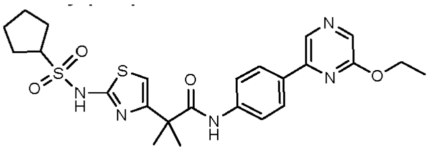 | **Ref T378** | N-(4-(6-(cyclopentylmethoxy)pyrazin-2-yl)phenyl)-2-(2-(cyclopropanesulfonamido) thiazol-4-yl)-2-methylpropanamide 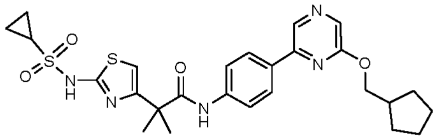 |
| **Ref T336** | 2-(2-(cyclopropanesulfonamido)thiazol-4-yl)-N-(5-(6-isopropylpyrazin-2-yl)pyridin-2-yl)-2-methylpropanamide 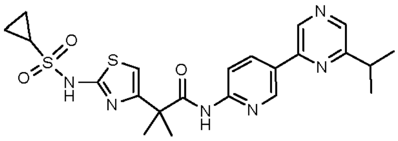 | **Ref T379** | 2-(2-(cyclopropanesulfonamido)thiazol-4-yl)-N-(4-(6-hydroxypyrazin-2-yl)phenyl)-2-methylpropanamide 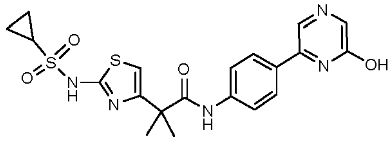 |
| **Ref T337** | 2-(2-(cyclopropanesulfonamido)thiazol-4-yl)-N-(5'-ethoxy-[3,3'-bipyridin]-6-yl)-2-methylpropanamide 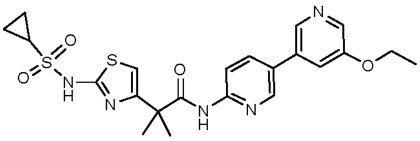 | **Ref T380** | 2-(2-(ethylsulfonamido)thiazol-4-yl)-2-methyl-N-(5'-(trifluoromethyl)-[3,3'-bipyridin]-6-yl)propanamide 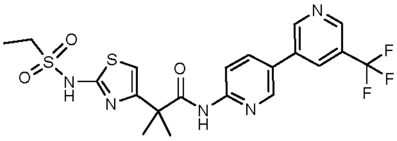 |
| **Ref T338** | 2-(2-(cyclopropanesulfonamido)thiazol-4-yl)-N-(5-(6-(2-hydroxypropan-2-yl)pyrazin-2-yl)pyri din-2-yl)-2-methylpropanamide 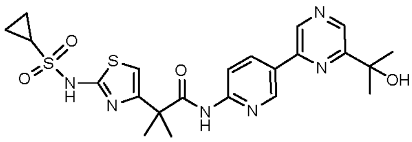 | **Ref T381** | 2-(2-(ethylsulfonamido)thiazol-4-yl)-2-methyl-N-(5-(6-(trifluoromethyl)pyrazin-2-yl)pyridin-2-yl) propanamide 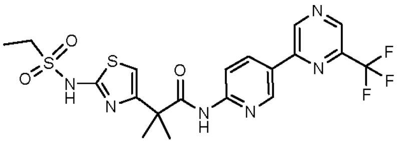 |
| **Ref T339** | 2-(2-(cyclopropanesulfonamido)thiazol-4-yl)-N-(5-(6-(2-methoxypropan-2-yl)pyrazin-2-yl)pyri din-2-yl)-2-methylpropanamide 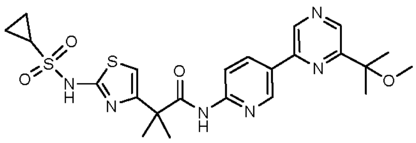 | **Ref T382** | N-(5-(6-ethoxypyrazin-2-yl)pyridin-2-yl)-2-(2-(ethylsulfonamido)thiazol-4-yl)-2-methylpropanamide 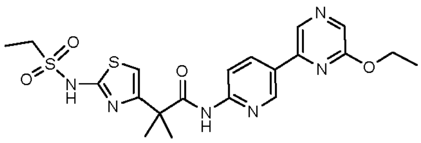 |
| **Ref T340** | 2-(2-(cyclopropanesulfonamido)-5-methylthiazol-4-yl)-2-methyl-N-(5-(6-(trifluoromethyl)pyrazin-2-yl)pyridin-2-yl)propanamide 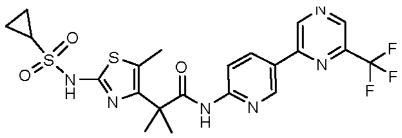 | **Ref T383** | 2-(2-(ethylsulfonamido)thiazol-4-yl)-N-(2-fluoro-4-(6-isopropoxypyrazin-2-yl)phenyl)-2-methylpropanamide 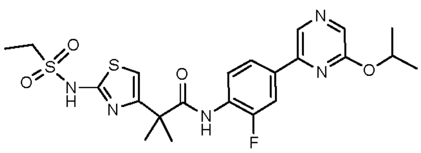 |

(continued)

| T# | Name/Structure (All examples containing chiral centres are racemates unless stated) | T# | Name/Structure (All examples containing chiral centres are racemates unless stated) |
|---|---|---|---|
| **Ref T341** | 2-(2-(cyclopropanesulfonamido)thiazol-4-yl)-N-(5-(6-ethoxypyrazin-2-yl)pyridin-2-yl)-2-methylpropanamide 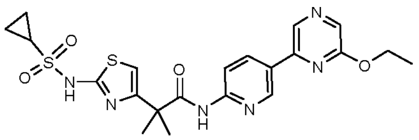 | **Ref T384** | N-(4-(5-cyanopyridin-3-yl)phenyl)-2-(2-(ethylsulfonamido)thiazol-4-yl)-2-methylpropanamide 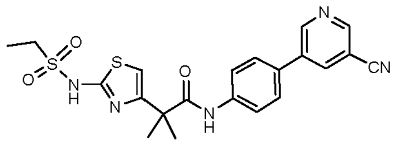 |
| **Ref T342** | 2-(2-(cyclopropanesulfonamido)-5-methylthiazol-4-yl)-N-(5-(6-ethoxypyrazin-2-yl)pyridin-2-yl)-2-methylpropanamide 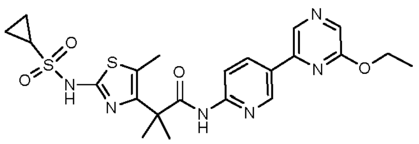 | **Ref T385** | 2-(2-(ethylsulfonamido)thiazol-4-yl)-N-(4-(5-fluoropyridin-3-yl)phenyl)-2-methylpropanamide 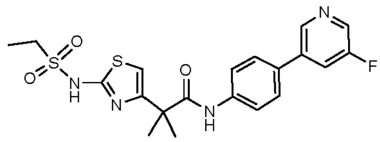 |
| **Ref T343** | N-(4-(5-chloropyridin-3-yl)-2-(trifluoromethyl) phenyl)-2-(2-(cyclopropanesulfonamido)thiazol-4-yl)-2-methylpropanamide 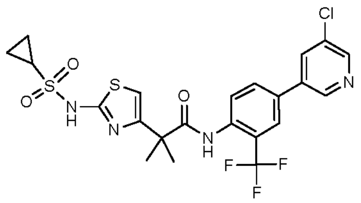 | **Ref T386** | 2-(2-(ethylsulfonamido)thiazol-4-yl)-2-methyl-N-(4-(pyridin-3-yl)phenyl)propanamide 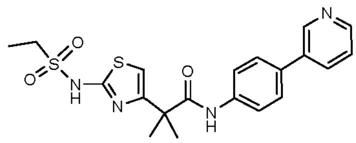 |
| **Ref T344** | N-(4-(5-cyanopyridin-3-yl)-2-(trifluoromethyl) phenyl)-2-(2-(cyclopropanesulfonamido)thiazol-4-yl)-2-methylpropanamide 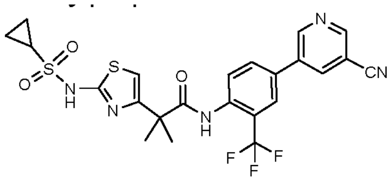 | **Ref T387** | 2-(2-(ethylsulfonamido)thiazol-4-yl)-2-methyl-N-(4-(6-(trifluoromethyl)pyrazin-2-yl)phenyl)propanamide 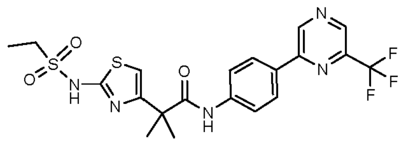 |
| **Ref T345** | 2-(2-(cyclopropanesulfonamido)thiazol-4-yl)-N-(4-(5-fluoropyridin-3-yl)-2-(trifluoromethyl) phenyl)-2-methylpropanamide 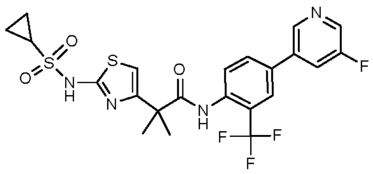 | **Ref T388** | 2-(2-(ethylsulfonamido)thiazol-4-yl)-N-(4-(6-isopropoxypyrazin-2-yl)phenyl)-2-methylpropanamide 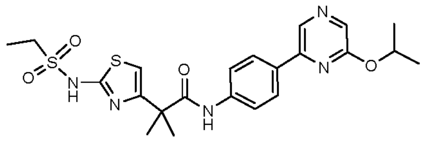 |
| **Ref T346** | 2-(2-(cyclopropanesulfonamido)thiazol-4-yl)-2-methyl-N-(2-(trifluoromethyl)-4-(6-(trifluoromethyl)pyrazin-2-yl)phenyl)propanamide | **Ref T389** | 2-methyl-2-(2-(methylsulfonamido)thiazol-4-yl)-N-(5'-(trifluoromethyl)-[3,3'-bipyridin]-6-yl)propanamide |

(continued)

| T# | Name/Structure (All examples containing chiral centres are racemates unless stated) | T# | Name/Structure (All examples containing chiral centres are racemates unless stated) |
|---|---|---|---|
| | 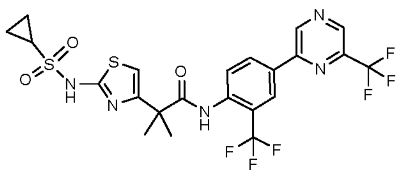 | | 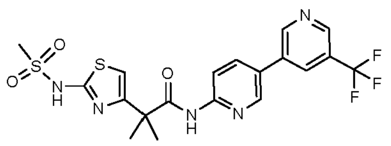 |
| **Ref T347** | 2-(2-(cyclopropanesulfonamido)thiazol-4-yl)-N-(4-(6-ethoxypyrazin-2-yl)-2-(trifluoromethyl)phenyl)-2-methylpropanamide 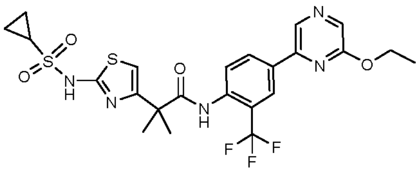 | **Ref T390** | N-(5-(6-ethoxypyrazin-2-yl)pyridin-2-yl)-2-methyl-2-(2-(methylsulfonamido)thiazol-4-yl)propanamide 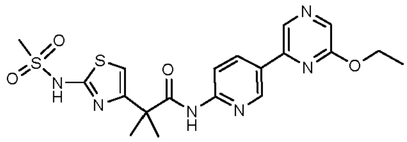 |
| **Ref T348** | N-(4-(5-chloropyridin-3-yl)-2,6-diethylphenyl)-2-(2-(cyclopropanesulfonamido)thiazol-4-yl)-2-methylpropanamide 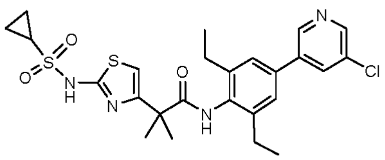 | **Ref T391** | N-(2-fluoro-4-(6-(trifluoromethyl)pyrazin-2-yl)phenyl)-2-methyl-2-(2-(methylsulfonamido) thiazol-4-yl)propanamide 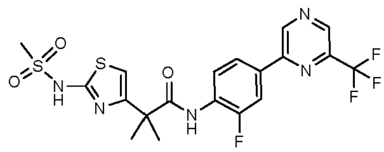 |
| **Ref T349** | N-(4-(5-cyanopyridin-3-yl)-2,6-diethylphenyl)-2-(2-(cyclopropanesulfonamido)thiazol-4-yl)-2-methylpropanamide 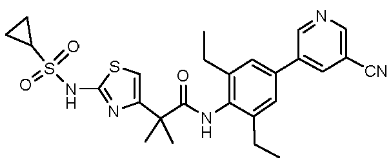 | **Ref T392** | N-(2-fluoro-4-(6-isopropoxypyrazin-2-yl)phenyl)-2-methyl-2-(2-(methylsulfonamido)thiazol-4-yl)propanamide 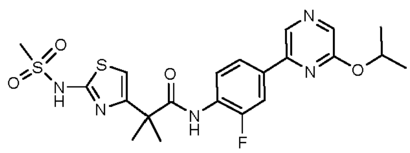 |
| **Ref T350** | 2-(2-(cyclopropanesulfonamido)-5-methylthiazol-4-yl)-N-(2-fluoro-4-(5-(trifluoromethyl)pyridin-3-yl)phenyl)-2-methylpropanamide 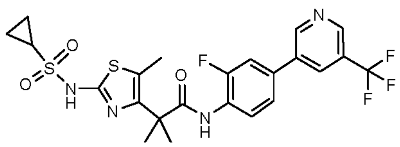 | **Ref T393** | N-(4-(5-chloropyridin-3-yl)phenyl)-2-methyl-2-(2-(methylsulfonamido)thiazol-4-yl)propanamide 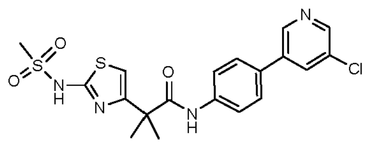 |
| **Ref T351** | N-(4-(5-chloropyridin-3-yl)-2,6-difluorophenyl)-2-(2-(cyclopropanesulfonamido)thiazol-4-yl)-2-methylpropanamide 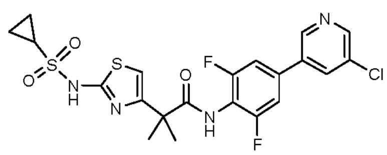 | **Ref T394** | 2-methyl-2-(2-(methylsulfonamido)thiazol-4-yl)-N-(4-(6-(trifluoromethyl)pyrazin-2-yl)phenyl) propanamide 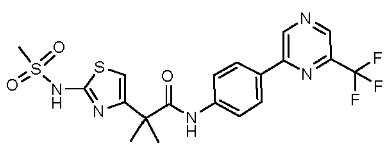 |

(continued)

| T# | Name/Structure (All examples containing chiral centres are racemates unless stated) | T# | Name/Structure (All examples containing chiral centres are racemates unless stated) |
|---|---|---|---|
| **Ref T352** | N-(4-(5-chloropyridin-3-yl)-2-fluoro-5-methylphenyl)-2-(2-(cyclopropanesulfonamido) thiazol-4-yl)-2-methylpropanamide<br>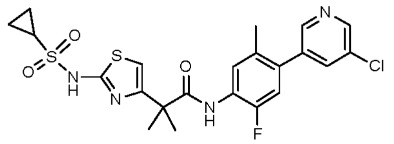 | **Ref T395** | N-(4-(6-isopropoxypyrazin-2-yl)phenyl)-2-methyl-2-(2-(methylsulfonamido)thiazol-4-yl)propanamide<br>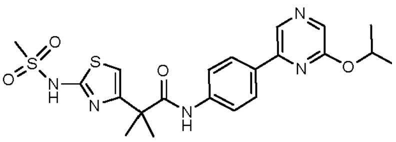 |
| **Ref T353** | 2-(2-(cyclopropanesulfonamido)thiazol-4-yl)-N-(2-fluoro-4-(6-(2-methoxypropan-2-yl)pyrazin-2-yl)phenyl)-2-methylpropanamide<br>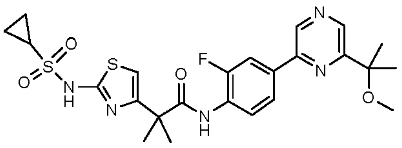 | **Ref T396** | 2-(2-((cyclopropylmethyl)sulfonamido)thiazol-4-yl)-2-methyl-N-(5-(6-(trifluoromethyl)pyrazin-2-yl) pyridin-2-yl)propanamide<br>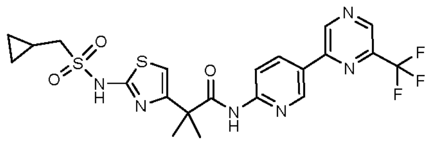 |
| **Ref T354** | 2-(2-(cyclopropanesulfonamido)-5-methylthiazol-4-yl)-N-(2-fluoro-4-(6-(trifluoromethyl)pyrazin-2-yl)phenyl)-2-methylpropanamide<br>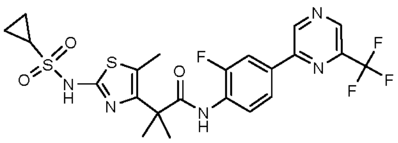 | **Ref T397** | 1-(2-(cyclopropanesulfonamido)thiazol-4-yl)-N-(4-(6-(trifluoromethyl)pyrazin-2-yl)phenyl)cyclopropane-1-carboxamide<br>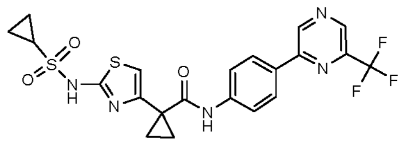 |
| **Ref T355** | N-(4-(6-cyanopyrazin-2-yl)-2-fluorophenyl)-2-(2-(cyclopropanesulfonamido)thiazol-4-yl)-2-methylpropanamide<br>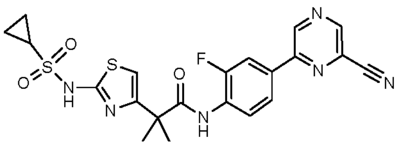 | **Ref T398** | 1-(2-(cyclopropanesulfonamido)thiazol-4-yl)-N-(4-(6-ethoxypyrazin-2-yl)phenyl)cyclopropane-1-carboxamide<br>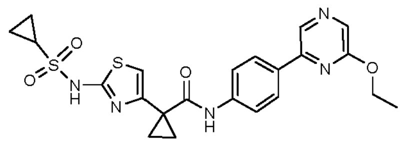 |
| **Ref T356** | 2-(2-(cyclopropanesulfonamido)thiazol-4-yl)-N-(4-(6-ethylpyrazin-2-yl)-2-fluorophenyl)-2-methylpropanamide<br>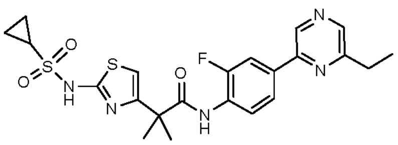 | **Ref T399** | 2-(2-(cyclopropanesulfonamido)thiazol-4-yl)-N-(2-fluoro-4-(6-(trifluoromethyl)pyrazin-2-yl)phenyl)-4-methoxybutanamide<br>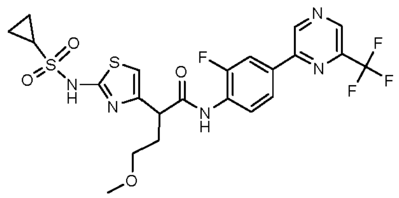 |
| **Ref T357** | 2-(2-(cyclopropanesulfonamido)thiazol-4-yl)-N-(2-fluoro-4-(6-isopropoxypyrazin-2-yl)phenyl)-2-methylpropanamide | **Ref T400** | 2-(2-(cyclopropanesulfonamido)thiazol-4-yl)-N-(2-fluoro-4-(6-isopropoxypyrazin-2-yl)phenyl)-4-methoxybutanamide |

(continued)

| T# | Name/Structure (All examples containing chiral centres are racemates unless stated) | T# | Name/Structure (All examples containing chiral centres are racemates unless stated) |
|---|---|---|---|
| | 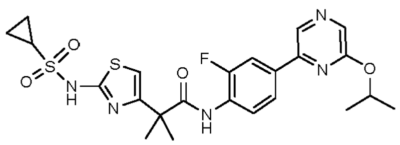 | | 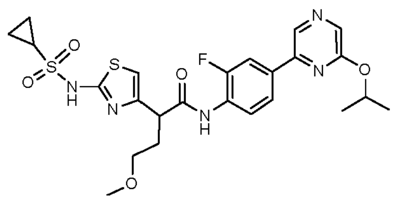 |
| **Ref T358** | 2-(2-(cyclopropanesulfonamido)-5-methylthiazol-4-yl)-N-(4-(6-ethoxypyrazin-2-yl)-2-fluorophenyl)-2-methylpropanamide<br>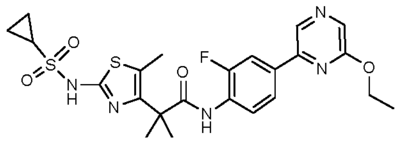 | **Ref T401** | 2-(2-(cyclopropanesulfonamido)thiazol-4-yl)-N-(5-(6-isopropylpyrazin-2-yl)pyridin-2-yl)butanamide<br>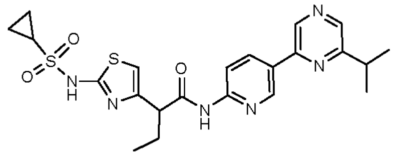 |
| **Ref T359** | N-(4-(5-chloropyridin-3-yl)-2-isopropylphenyl)-2-(2-(cyclopropanesulfonamido)thiazol-4-yl)-2-methylpropanamide<br>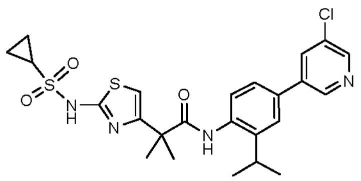 | **Ref T402** | 2-(2-(cyclopropanesulfonamido)thiazol-4-yl)-N-(5-(6-(2-methoxypropan-2-yl)pyrazin-2-yl)pyridin-2-yl)butanamide<br>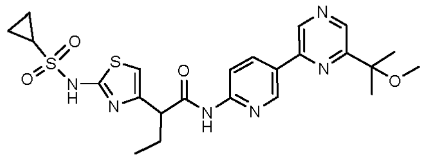 |
| **Ref T360** | N-(4-(5-cyanopyridin-3-yl)-2-isopropylphenyl)-2-(2-(cyclopropanesulfonamido)thiazol-4-yl)-2-methylpropanamide<br>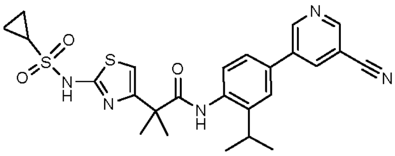 | **Ref T403** | 2-(2-(cyclopropanesulfonamido)thiazol-4-yl)-N-(2-fluoro-4-(6-(2-methoxypropan-2-yl)pyrazin-2-yl)phenyl)butanamide<br>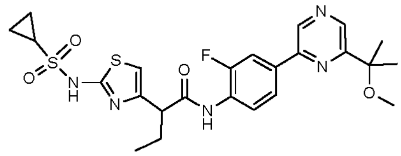 |
| **Ref T361** | 2-(2-(cyclopropanesulfonamido)thiazol-4-yl)-N-(2-isopropyl-4-(6-(trifluoromethyl)pyrazin-2-yl)phenyl)-2-methylpropanamide<br>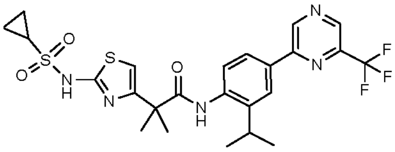 | **Ref T404** | N-(4-(6-cyanopyrazin-2-yl)-2-fluorophenyl)-2-(2-(cyclopropanesulfonamido)thiazol-4-yl)butanamide<br>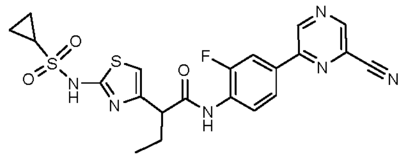 |
| **Ref T362** | 2-(2-(cyclopropanesulfonamido)thiazol-4-yl)-N-(4-(6-ethoxypyrazin-2-yl)-2-isopropylphenyl)-2-methylpropanamide<br>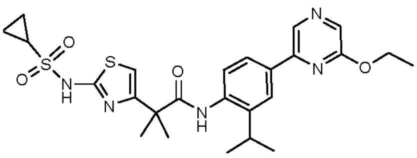 | **Ref T405** | 2-(2-(cyclopropanesulfonamido)thiazol-4-yl)-N-(4-(6-ethylpyrazin-2-yl)-2-fluorophenyl)butanamide<br>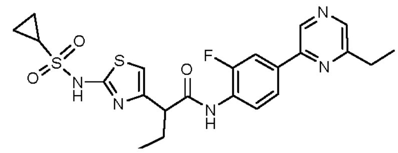 |

(continued)

| T# | Name/Structure (All examples containing chiral centres are racemates unless stated) | T# | Name/Structure (All examples containing chiral centres are racemates unless stated) |
|---|---|---|---|
| **Ref T363** | N-(4-(5-chloropyridin-3-yl)-3-fluoro-2-methylphenyl)-2-(2-(cyclopropanesulfonamido) thiazol-4-yl)-2-methylpropanamide 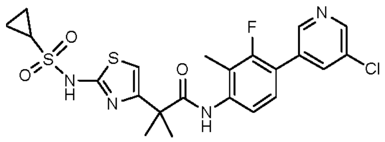 | **Ref T406** | 2-(2-(cyclopropanesulfonamido)thiazol-4-yl)-N-(4-(6-(2-methoxypropan-2-yl)pyrazin-2-yl) phenyl)butanamide 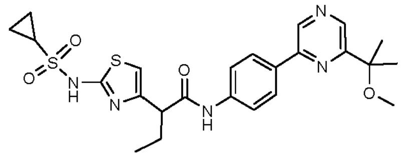 |
| **Ref T364** | N-(4-(5-chloropyridin-3-yl)-5-fluoro-2-methylphenyl)-2-(2-(cyclopropanesulfonamido) thiazol-4-yl)-2-methylpropanamide 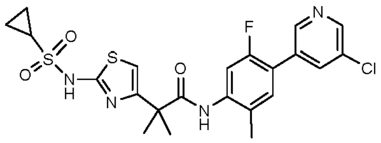 | **Ref T407** | tert-butyl (1-(2-(cyclopropanesulfonamido)thiazol-4-yl)-2-((4-(6-ethoxypyrazin-2-yl)phenyl)amino)-2-oxoethyl)carbamate 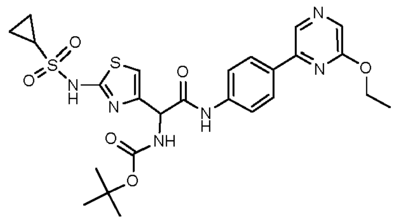 |
| **Ref T365** | N-(4-(5-chloropyridin-3-yl)-2,3-dimethylphenyl)-2-(2-(cyclopropanesulfonamido) thiazol-4-yl)-2-methylpropanamide 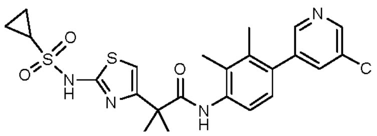 | **Ref T408** | 2-(2-(cyclopropanesulfonamido)thiazol-4-yl)-N-(2-fluoro-4-(6-(trifluoromethyl)pyrazin-2-yl)phenyl)-2-methoxyacetamide 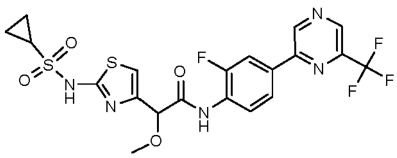 |
| **Ref T366** | N-(4-(5-chloropyridin-3-yl)-2,5-dimethylphenyl)-2-(2-(cyclopropanesulfonamido) thiazol-4-yl)-2-methylpropanamide 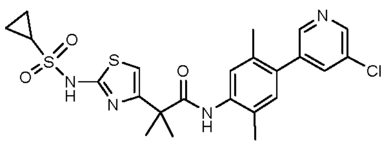 | **Ref T409** | 2-(2-(cyclopropanesulfonamido)thiazol-4-yl)-N-(4-(6-ethoxypyrazin-2-yl)-2-fluorophenyl)-2-methoxvacetamide 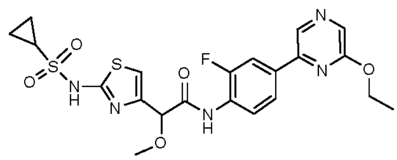 |
| **Ref T367** | N-(4-(5-cyanopyridin-3-yl)-3-fluoro-2-methylphenyl)-2-(2-(cyclopropanesulfonamido) thiazol-4-yl)-2-methylpropanamide 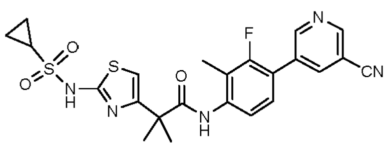 | **Ref T410** | 2-(2-(cyclopropanesulfonamido)thiazol-4-yl)-N-(4-(6-ethoxypyrazin-2-yl)phenyl)-2-methoxyacetamide 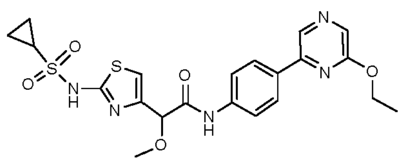 |
| **Ref T368** | 2-(2-(cyclopropanesulfonamido)thiazol-4-yl)-2-methyl-N-(2-methyl-4-(6-(trifluoromethyl) pyrazin-2-yl)phenyl)propanamide | **Ref T411** | 2-(2-(cyclopropanesulfonamido)thiazol-4-yl)-N-(4-(6-isopropoxypyrazin-2-yl)phenyl)-2-methoxyacetamide |

(continued)

| T# | Name/Structure (All examples containing chiral centres are racemates unless stated) | T# | Name/Structure (All examples containing chiral centres are racemates unless stated) |
|---|---|---|---|
| | 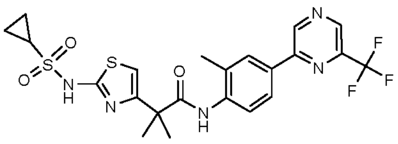 | | 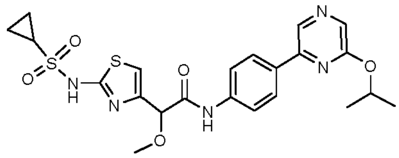 |
| **Ref T369** | N-(4-(5-chloropyridin-3-yl)-5-fluoro-2-methoxyphenyl)-2-(2-(cyclopropanesulfonamido) thiazol-4-yl)-2-methylpropanamide 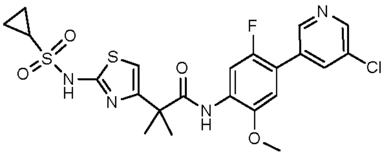 | **Ref T412** | **Single enantiomer - stereochemistry unassigned** 2-(2-(cyclopropanesulfonamido)thiazol-4-yl)-N-(4-(6-ethoxypyrazin-2-yl)-2-fluorophenyl)butanamide 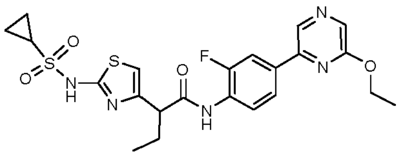 |
| **Ref T370** | N-(4-(5-chloropyridin-3-yl)-3-(trifluoromethyl) phenyl)-2-(2-(cyclopropanesulfonamido)thiazol-4-yl)-2-methylpropanamide 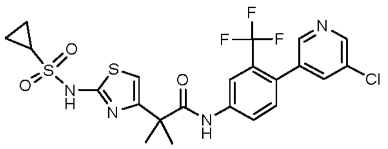 | **Ref T413** | **Single enantiomer - stereochemistry unassigned** 2-(2-(cyclopropanesulfonamido)thiazol-4-yl)-N-(4-(6-ethoxypyrazin-2-yl)-2-fluorophenyl)butanamide 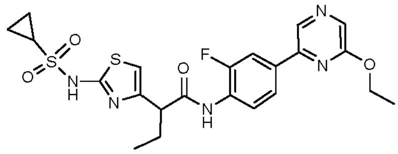 |
| **Ref T371** | 2-(2-(cyclopropanesulfonamido)thiazol-4-yl)-N-(4-(6-ethoxypyrazin-2-yl)-3-methylphenyl)-2-methylpropanamide 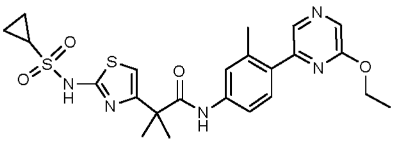 | **Ref T414** | **Single enantiomer - stereochemistry unassigned** 2-(2-(cyclopropanesulfonamido)thiazol-4-yl)-N-(5-(6-(trifluoromethyl)pyrazin-2-yl)pyridin-2-yl)butanamide 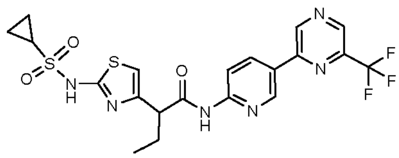 |
| **Ref T372** | N-(4-(5-chloropyridin-3-yl)-3-ethoxyphenyl)-2-(2-(cyclopropanesulfonamido)thiazol-4-yl)-2-methylpropanamide 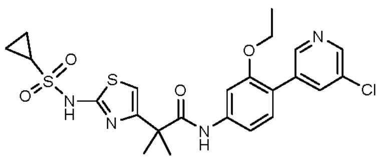 | **Ref T415** | **Single enantiomer - stereochemistry unassigned** 2-(2-(cyclopropanesulfonamido)thiazol-4-yl)-N-(2-fluoro-4-(5-(trifluoromethyl)pyridin-3-yl) phenyl)butanamide 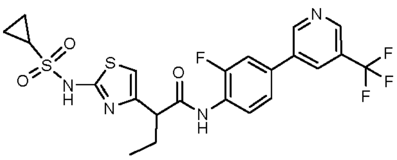 |

(continued)

| T# | Name/Structure (All examples containing chiral centres are racemates unless stated) | T# | Name/Structure (All examples containing chiral centres are racemates unless stated) |
|---|---|---|---|
| **Ref T373** | N-(4-(5-chloropyridin-3-yl)phenyl)-1-(2-(cyclopropanesulfonamido)thiazol-4-yl)cyclopropane-1-carboxamide 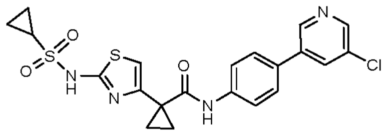 | **Ref T416** | **Single enantiomer - stereochemistry unassigned** 2-(2-(cyclopropanesulfonamido)thiazol-4-yl)-N-(2-fluoro-4-(5-(trifluoromethyl)pyridin-3-yl)phenyl)butanamide 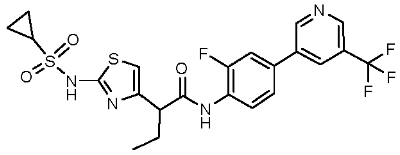 |
| **Ref T374** | N-(4-(5-cyanopyridin-3-yl)phenyl)-2-(2-(cyclopropanesulfonamido)-5-methylthiazol-4-yl)-2-methylpropanamide 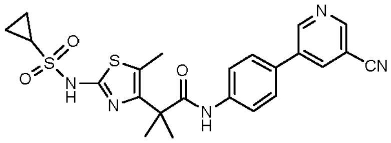 | | |

Reference Example: 2-(2-(Cyclopropanesulfonamido)thiazol-4-yl)-N-(4-(6-(ethylamino)pyrazin-2-yl)phenyl)butanamide **T444**

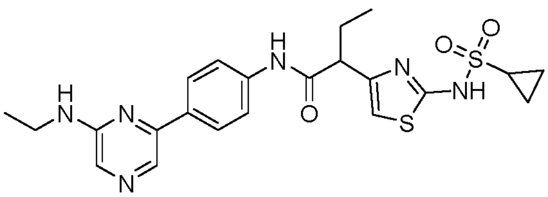

Reference Example: 2-Amino-2-(2-(cyclopropanesulfonamido)thiazol-4-yl)-*N*-(5-(6-(trifluoromethyl)pyrazin-2-yl)pyridin-2-yl)acetamide hydrochloride **T417**

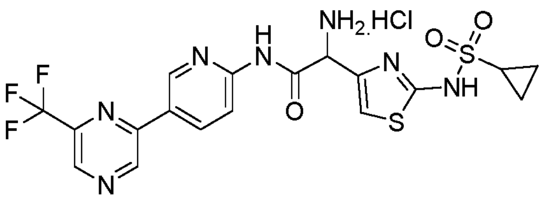

Reference Example: 2-Amino-2-(2-(cyclopropanesulfonamido)thiazol-4-yl)-*N*-(4-(6-ethoxypyrazin-2-yl)-2-fluorophenyl)acetamide hydrochloride **T419**

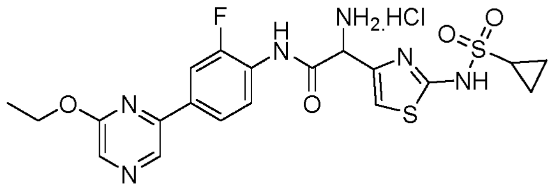

Reference Example: 2-Amino-2-(2-(cyclopropanesulfonamido)thiazol-4-yl)-*N*-(2-fluoro-4-(6-(trifluoromethyl)pyrazin-2-yl)phenyl)acetamide **T418**

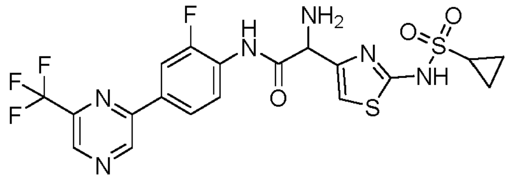

Reference Example: 2-(2-(Cyclopropanesulfonamido)thiazol-4-yl)-2-(dimethylamino)-*N*-(2-fluoro-4-(6-(trifluoromethyl) pyrazin-2-yl)phenyl)acetamide **T420**

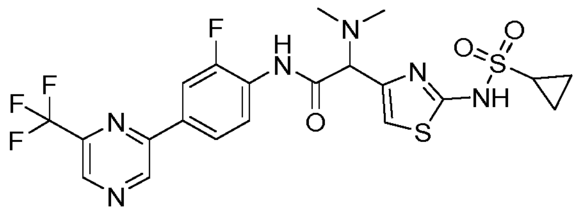

Reference Example: 2-(2-(Cyclopropanesulfonamido)thiazol-4-yl)-2-(dimethylamino)-*N*-(4-(6-ethoxypyrazin-2-yl)-2-fluorophenyl)acetamide **T421**

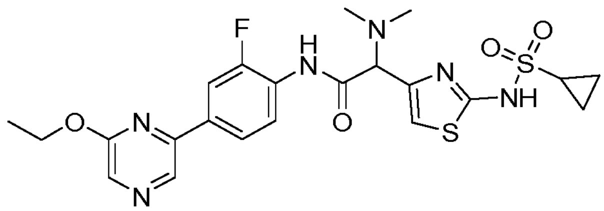

N-(4-(1-((2-fluoro-4-(pyridin-3-yl)phenyl)amino)-2-methylpropan-2-yl)thiazol-2-yl)cyclopropanesulfonamide **T466**

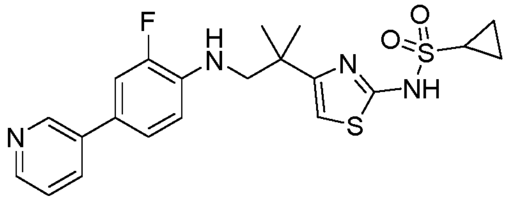

$LiAlH_4$ (0.870 mL, 1.74 mmol) was added into a solution of 2-(2-(cyclopropanesulfonamido)thiazol-4-yl)-N-(2-fluoro-4-(pyridin-3-yl)phenyl)-2-methylpropanamide Reference Example **T42** (200 mg, 0.434 mmol) in THF (20 mL, 0.434 mmol) and the resulting solution was stirred at RT for 16 hrs. The reaction mixture was quenched with sat. $NH_4Cl$ (aq, 50 mL) and extracted with EtOAc (3 x 50 mL). The organic extract was dried ($MgSO_4$), filtered and solvent removed in vacuo. The crude product was purified by chromatography on RP Flash C18 (12 g cartridge, 5-100% MeCN/10 mM ammonium bicarbonate) to afford N-(4-(1-((2-fluoro-4-(pyridin-3-yl)phenyl)amino)-2-methylpropan-2-yl)thiazol-2-yl)cyclopropanesulfonamide (23 mg, 0.050 mmol, 12 % yield) as a white solid. Rt 1.83 min (HPLC basic); *m/z* 447 $(M+H)^+$ $(ES^+)$. $^1H$ NMR (500 MHz, DMSO-d6) δ 12.57 (s, 1H), 8.82 (d, J = 2.4 Hz, 1H), 8.47-8.43 (m,1H), 8.00-7.95 (m, 1H), 7.46 (dd, J = 13.4, 2.2 Hz, 1H), 7.39 (dd, J = 8.0, 4.7 Hz, 1H), 7.35-7.31 (m, 1H), 6.92 (t, J = 8.9 Hz, 1H), 6.40 (s, 1H), 5.50 (t, J = 4.4 Hz, 1H), 3.36-3.28 (m, 2H (obscured by water peak)), 2.56 - 2.51 (m, 1H, (obscured by DMSO peak)), 1.27 (s, 6H), 0.90-0.79 (m, 4H).

**Compounds of formula (I-c)** - **Intermediates**

Known synthetic intermediates were procured from commercial sources or were obtained using published literature procedures. Additional intermediates were prepared by the representative synthetic processes described herein.

The synthesis of **INTE1** to **INTE39** and **INTF1** to **INTF53** is disclosed in WO2019/106146 and uses the general methods disclosed below.

**Method A: Reductive amination**

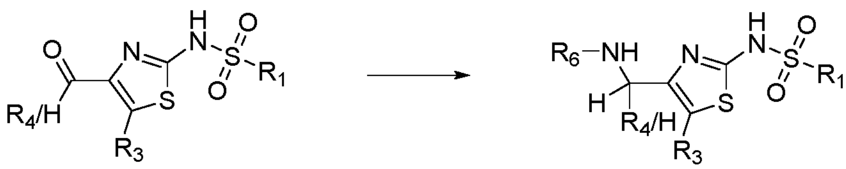

A solution of aldehyde/ketone (1 eq.) in THF was treated with AcOH (1 eq.), amine (1 eq.) and a reducing agent such as STAB (1.2 eq.) and stirred at RT for 1 hr. The reaction mixture was quenched by addition of MeOH then loaded directly on to SCX (1 g/mmol of substrate), washed with MeOH and the product was eluted with 1 M $NH_3$ in MeOH. The crude product was then concentrated onto silica and purified by normal phase chromatography.

**Method B: Benzylamine deprotection (TFA)**

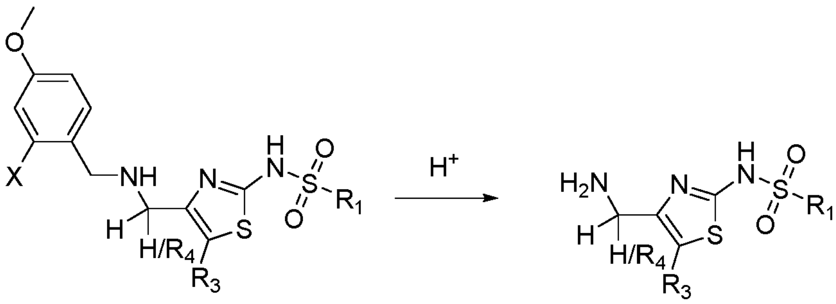


X = H or OMe

Benzylamine derivative (1 eq.) was dissolved in TFA (50 eq.) and heated to 70 °C for 1 - 24 hrs. The reaction was allowed to cool to RT, then was loaded on to SCX (1 g/mmol of substrate) and washed with MeOH. The required compound was eluted with 1% $NH_3$ in MeOH.

**Method D: Ester deprotection with TFA**

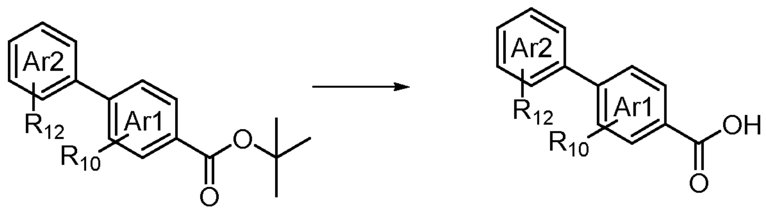

A solution of the ester (1 eq) in DCM (20 volumes) was treated with TFA (10 eq.) and stirred at RT for 3 hrs. The reaction mixture was then concentrated and azeotroped with MeOH and MeCN. No further purification was undertaken.

**Method E: Ester deprotection with base**

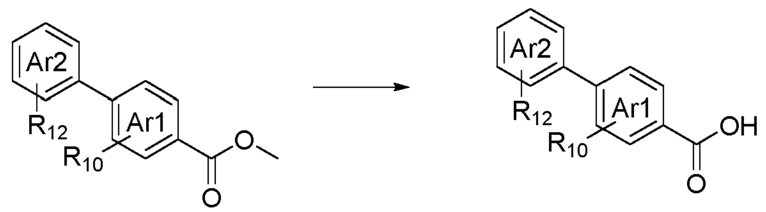

A solution of the ester (1 eq) in a mixture of THF/MeOH (4/1 volumes) was treated with LiOH (2.2-6 eq.) and stirred between RT and 50 °C for between 3 hrs and 18hrs. The organic solvents were removed *in vacuo* then acidified with 1 M HCl and extracted with EtOAc. The organic phases were combined, dried ($Na_2SO_4$), filtered and concentrated. The products were used directly in the next step with no further purification undertaken.

**Method F: Potassium salt formation**

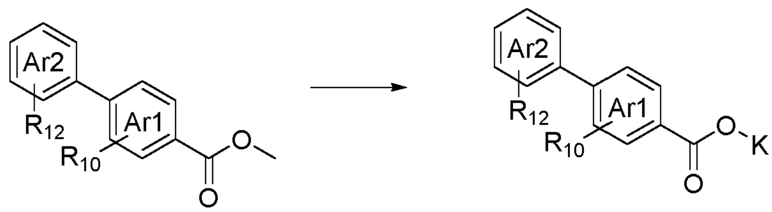

A solution of the ester (1 eq.) in THF (4 volumes) was treated with TMSOK (1 eq.) and stirred at RT for 2 hrs before the reaction mixtures were filtered and washed with iso-hexanes. The products were used directly in the next step with no further purification undertaken.

**Compounds of formula (I-c) - Examples**

The synthesis of a number of known CTPS1 inhibitors is disclosed in WO2019/106146 (see compounds R1 to R93). Such compounds are made using general methods disclosed herein and represent further examples of compounds which are CTPS1 inhibitors. The full synthetic methods and characterising data for compounds R1 to R93 are provided in WO2019/106146.

**Method 1: Amide coupling**

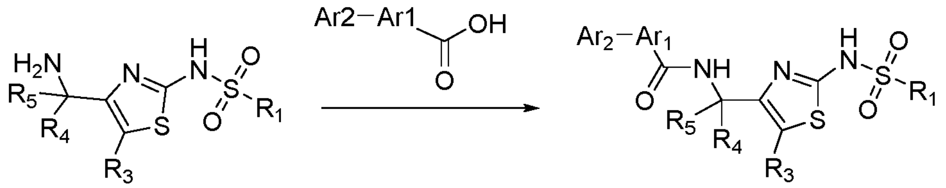


**Method 1a:** HATU (1.2 eq.) was added to a solution of appropriate acid (1 eq.), amine (1 eq.) and DIPEA (3 eq.) in DMF (10 volumes) at RT. The reaction was stirred at RT for 18 hrs. The solvent was removed and the crude product was purified by normal phase chromatography, reverse phase chromatography or trituration from an appropriate solvent.

**Method 1b:** 1-chloro-*N,N*,2-trimethylprop-1-en-1-amine (2 eq.) was added to a solution of appropriate acid (1 eq.) in DCM (20 volumes). The reaction mixture was stirred at RT for 2 hrs. The reaction mixture was concentrated *in vacuo* and the residue dissolved in DCM (20 volumes) before addition of DIPEA (3 eq.) and the appropriate amine (1 eq). The reaction mixture was stirred at RT for 2 hrs. An aqueous work up was performed and the crude product was purified by normal phase chromatography, reverse phase chromatography or trituration from an appropriate solvent.

**Method 1c:** T3P (50wt% in EtOAc, 2.5 eq.) was added to a solution of appropriate acid (1 eq.), amine (1 eq.) and pyridine (3 eq.) in a mixture of EtOAc (20 volumes) and DMF (10 volumes). The reaction was stirred for 1 hr at RT. An aqueous work up was performed and the crude product was purified by normal phase chromatography, reverse phase chromatography or trituration from an appropriate solvent.

**Method 2a: Suzuki [ArB(OR)$_2$ core]**

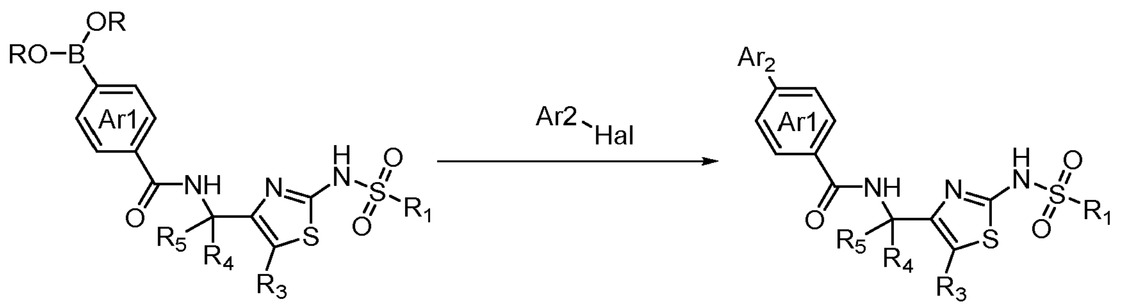


PdCl$_2$(dppf)-CH$_2$Cl$_2$ (10 mol %) or other appropriate catalyst was added to a degassed (N$_2$, 5 mins) solution of Ar1-B(OR)$_2$ (1 eq.), Ar2-halide (1 eq.) and K$_2$CO$_3$ (3 eq.) in dioxane (10 volumes) and water (1 volumes). The solution was then degassed further (N$_2$, 5 mins) and heated to 90 °C for 1-2 hrs. The reaction mixture was allowed to cool to RT. An aqueous workup was performed and the crude product was purified by normal phase chromatography, reverse phase chromatography or trituration from an appropriate solvent.

**Method 2b: Telescoped Miyaura Borylation/Suzuki Protocol**

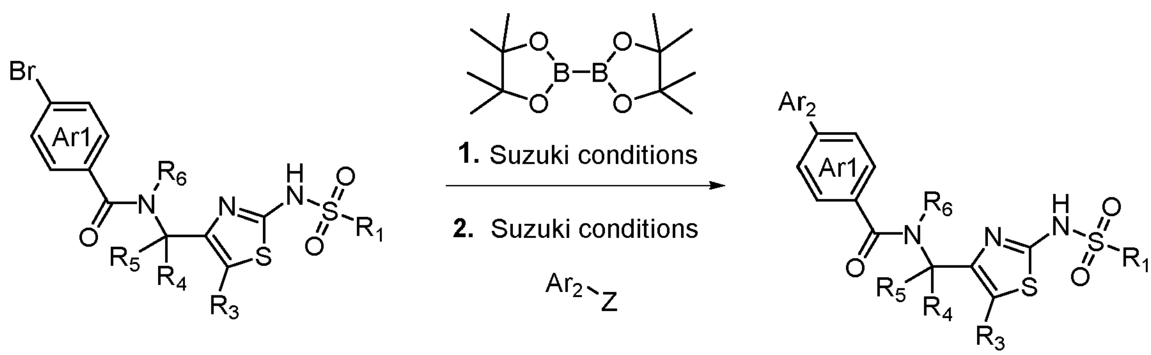


Z = Br, Cl

A suspension of Ar1-Br (1 eq.), Bispin (1.1 eq.) and KOAc (2 eq.) in dioxane (50 volumes) was degassed ($N_2$) then charged with $PdCl_2$(dppf).$CH_2Cl_2$ (5 mol %) and again degassed ($N_2$). The reaction mixture was heated to 90 °C for 1-24 hrs, recharging the Pd-catalyst if required. On formation of the boronate ester the reaction was allowed to cool to RT. Ar2-Z (1 eq.) and 2 M $K_2CO_3$ (aq, 2 eq.) were added, degassed ($N_2$) and the reaction was then heated to 90 °C for 18 hrs. The reaction was allowed to cool to RT, an aqueous work up was performed and the crude compound was purified by normal phase chromatography.

**Representative for Method 1a**

Reference Example: *N*-((2-(cyclopropanesulfonamido)thiazol-4-yl)methyl)-4-(pyridin-3-yl)benzamide **R1**

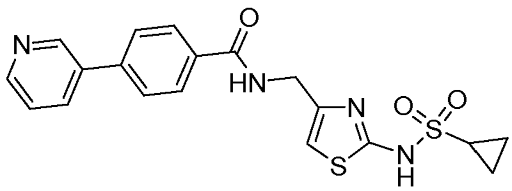

**Representative for Method 1b**

Reference Example: N-(1-(2-(cyclopropanesulfonamido)thiazol-4-yl)propyl)-4-(5-(trifluoromethyl)pyridin-3-yl)benzamide **R2**

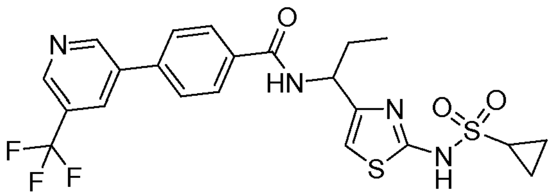

The racemic mixture R2 was separated by chiral preparative HPLC using chiral method A. A salt exchange (TFA to HCl) was undertaken by adding 1.25 M HCl (EtOH, 2 mL x 5) and removing solvent to afford:

**Peak 1: Stereochemistry of product was not defined R3**

Reference Example: *N*-(1-(2-(cyclopropanesulfonamido)thiazol-4-yl)propyl)-4-(5-(trifluoromethyl)pyridin-3-yl) benzamide. HCl **R3.**

**Peak 2: Stereochemistry of product was not defined R4**

Reference Example: *N*-(1-(2-(cyclopropanesulfonamido)thiazol-4-yl)propyl)-4-(5-(trifluoromethyl)pyridin-3-yl) benzamide. HCl **R4.**

**Representative for Method 1c**

Reference Example: *N*-(1-(2-(cyclopropanesulfonamido)thiazol-4-yl)propyl)-4-(6-(trifluoromethyl)pyrazin-2-yl)benzamide **R5**

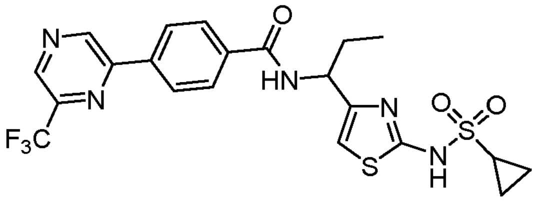

**Representative for Method 2a**

Reference Example: *N*-(2-(2-(cyclopropanesulfonamido)thiazol-4-yl)propan-2-yl)-4-(6-ethoxypyrazin-2-yl)-2-fluorobenzamide **R6**

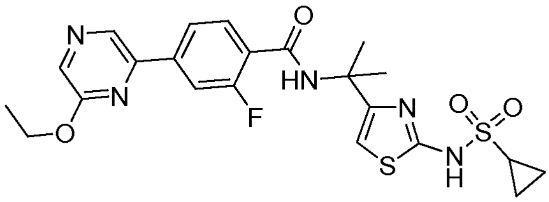

**Representative for Method 2b**

Reference Example: *N*-(2-(2-(cyclopropanesulfonamido)thiazol-4-yl)propan-2-yl)-4-(6-ethoxypyrazin-2-yl)-2-methoxybenzamide **R7**

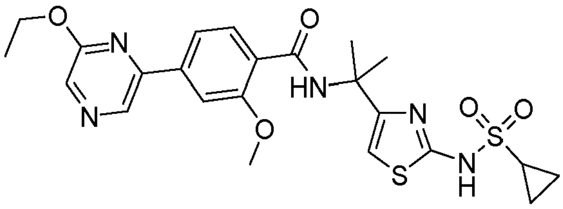

**Table 8:** Compounds **R8** to **R93**

| R | Name/Structure (All examples containing chiral centres are racemic unless stated) | R | Name/Structure (All examples containing chiral centres are racemic unless stated) |
|---|---|---|---|
| **Ref R8** | N-((2-(cyclopropanesulfonamido)thiazol-4-yl) methyl)-5-phenylpicolinamide 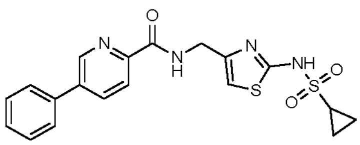 | **Ref R51** | N-(2-(2-(cyclopropanesulfonamido)thiazol-4-yl)propan-2-yl)-4-(6-(trifluoromethyl) pyrazin-2-yl)benzamide 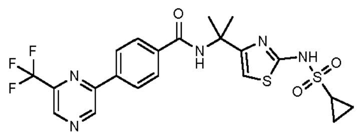 |

(continued)

| R | Name/Structure (All examples containing chiral centres are racemic unless stated) | R | Name/Structure (All examples containing chiral centres are racemic unless stated) |
|---|---|---|---|
| **Ref R9** | N-((2-(cyclopropanesulfonamido)thiazol-4-yl) methyl)-[1,1'-biphenyl]-4-carboxamide 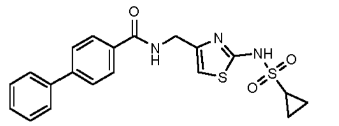 | **Ref R52** | 4-(6-chloropyrazin-2-yl)-N-(2-(2-(cyclopropanesulfon-amido)thiazol-4-yl)propan-2-yl) benzamide 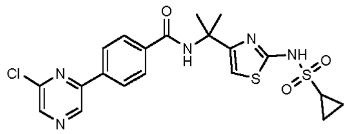 |
| **Ref R10** | N-((2-(cyclopropanesulfonamido)thiazol-4-yl) methyl)-2-fluoro-4-(6-(trifluoromethyl)-pyrazin-2-yl) benzamide 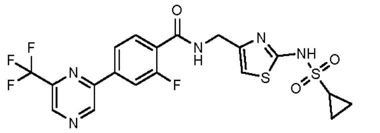 | **Ref R53** | N-(2-(2-(cyclopropanesulfonamido)thiazol-4-yl)propan-2-yl)-4-(6-methylpyrazin-2-yl)benzamide 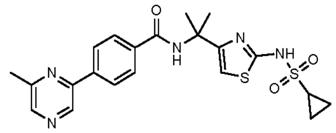 |
| **Ref R11** | N-((2-(cyclopropanesulfonamido)thiazol-4-yl) methyl)-4-(6-ethoxypyrazin-2-yl)-2- 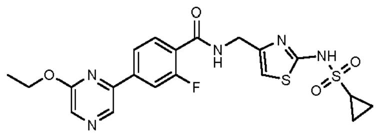 fluorobenzamide | **Ref R54** | N-(2-(2-(cyclopropanesulfonamido)thiazol-4-yl)propan-2-yl)-4-(6-methoxypyrazin-2-yl)benzamide 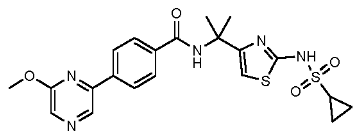 |
| **Ref R12** | N-((2-(cyclopropanesulfonamido)thiazol-4-yl) methyl)-4-(6-(trifluoromethyl)pyrazin-2-yl)benzamide 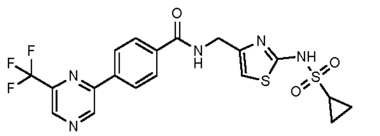 | **Ref R55** | N-(2-(2-(cyclopropanesulfonamido)thiazol-4-yl)propan-2-yl)-4-(6-ethoxypyrazin-2-yl)benzamide 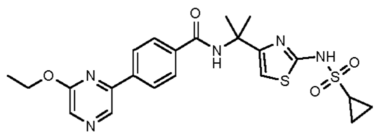 |
| **Ref R13** | N-((2-(cyclopropanesulfonamido)thiazol-4-yl) methyl)-4-(6-isopropoxypyrazin-2-yl)benzamide 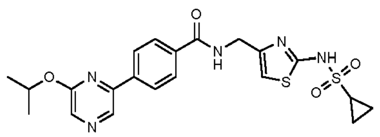 | **Ref R56** | N-(2-(2-(cyclopropanesulfonamido)thiazol-4-yl)propan-2-yl)-4-(6-isopropoxypyrazin-2-yl)benzamide 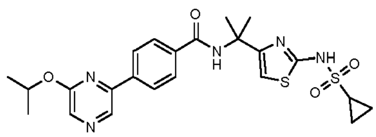 |
| **Ref R14** | N-((2-(cyclopropanesulfonamido)thiazol-4-yl) methyl)-4-(6-ethoxypyrazin-2-yl)benzamide 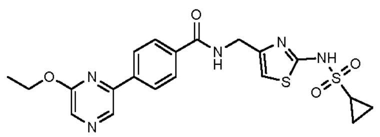 | **Ref R57** | N-(2-(2-(cyclopropanesulfonamido)thiazol-4-yl)propan-2-yl)-4-(6-(2,2,2-trifluoroethoxy)pyrazin-2-yl)benzamide 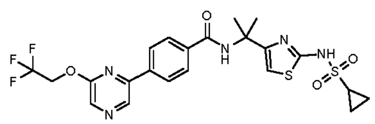 |

(continued)

| R | Name/Structure (All examples containing chiral centres are racemic unless stated) | R | Name/Structure (All examples containing chiral centres are racemic unless stated) |
|---|---|---|---|
| **Ref R15** | N-(3-(2-(cyclopropanesulfonamido)thiazol-4-yl) pentan-3-yl)-4-(5-(trifluoromethyl) pyridin-3-yl)benzamide 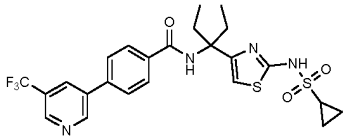 | **Ref R58** | N-(2-(2-(cyclopropanesulfonamido)thiazol-4-yl)propan-2-yl)-4-(pyrazin-2-yl)benzamide 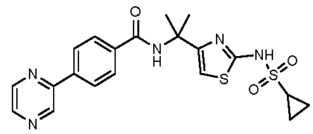 |
| **Ref R16** | N-(3-(2-(cyclopropanesulfonamido)thiazol-4-yl) pentan-3-yl)-4-(5-fluoropyridin-3-yl)benzamide 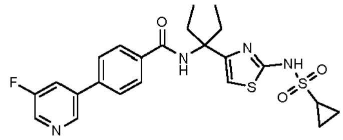 | **Ref R59** | RACEMIC, N-(1-(2-(cyclopropanesulfonamido)thiazol-4-yl)propyl)-4-(5-fluoropyridin-3-yl)benzamide 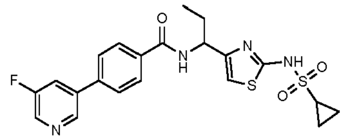 |
| **Ref R17** | N-(3-(2-(cyclopropanesulfonamido)thiazol-4-yl) pentan-3-yl)-4-(5-methylpyridin-3-yl)benzamide 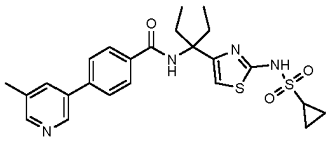 | **Ref R60** | RACEMIC, N-(1-(2-(cyclopropanesulfonamido)thiazol-4-yl)propyl)-4-(5-methylpyridin-3-yl)benzamide 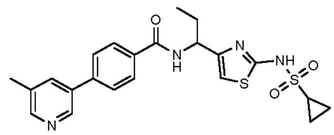 |
| **Ref R18** | N-(3-(2-(cyclopropanesulfonamido)thiazol-4-yl) pentan-3-yl)-4-(pyridin-3-yl)benzamide 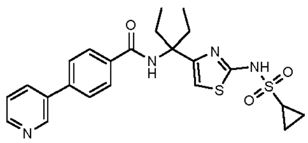 | **Ref R61** | RACEMIC, N-(1-(2-(cyclopropanesulfonamido)thiazol-4-yl)propyl)-4-(pyridin-3-yl) benzamide 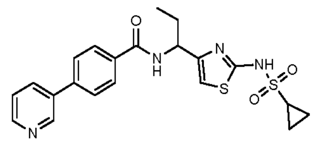 |
| **Ref R19** | N-(3-(2-(cyclopropanesulfonamido)thiazol-4-yl) pentan-3-yl)-4-(6-(trifluoromethyl) pyrazin-2-yl)benzamide 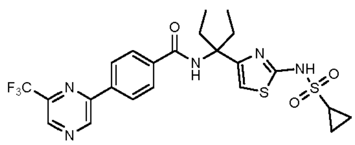 | **Ref R62** | RACEMIC, N-(1-(2-(cyclopropanesulfonamido)thiazol-4-yl)propyl)-4-(6-ethoxypyrazin-2-yl)-2-fluorobenzamide 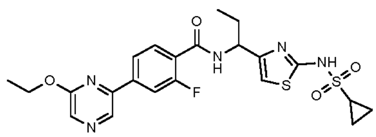 |
| **Ref R20** | 4-(6-chloropyrazin-2-yl)-N-(3-(2-(cyclopropanesulfonamido) thiazol-4-yl)pentan-3-yl)benzamide 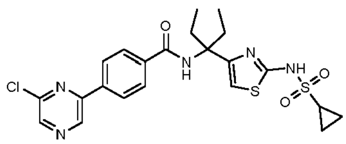 | **Re R63** | RACEMIC, N-(1-(2-(cyclopropanesulfonamido)thiazol-4-yl)propyl)-4-(6-ethoxypyrazin-2-yl)-2-fluoro-N-methylbenzamide 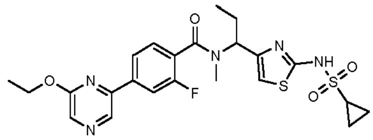 |

(continued)

| R | Name/Structure (All examples containing chiral centres are racemic unless stated) | R | Name/Structure (All examples containing chiral centres are racemic unless stated) |
|---|---|---|---|
| **Ref R21** | N-(3-(2-(cyclopropanesulfonamido)thiazol-4-yl) pentan-3-yl)-4-(6-methylpyrazin-2-yl)benzamide 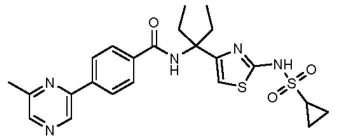 | **Ref R64** | RACEMIC, N-(1-(2-(cyclopropanesulfonamido)thiazol-4-yl)propyl)-2-fluoro-4-(6-isopropoxypyrazin-2-yl)benzamide 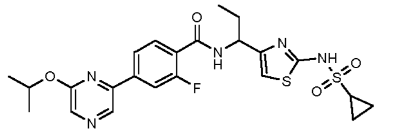 |
| **Ref R22** | N-(3-(2-(cyclopropanesulfonamido)thiazol-4-yl) pentan-3-yl)-4-(pyrazin-2-yl)benzamide 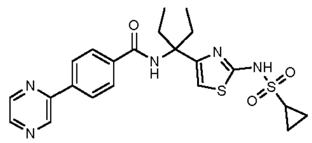 | **Ref R65** | RACEMIC, 4-(6-chloropyrazin-2-yl)-N-(1-(2-(cyclopropanesulfonamido)thiazol-4-yl)propyl)benzamide 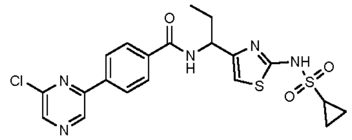 |
| **Ref R23** | N-(2-(2-(cyclopropanesulfonamido)thiazol-4-yl)propan-2-yl)-5-(6-ethoxypyrazin-2-yl)-3-fluoropicolinamide 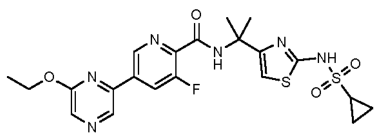 | **Ref R66** | RACEMIC, N-(1-(2-(cyclopropanesulfonamido)thiazol-4-yl)propyl)-4-(6-methylpyrazin-2-yl)benzamide 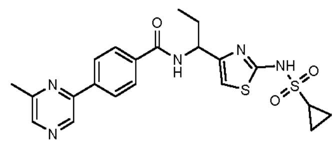 |
| **Ref R24** | N-(2-(2-(cyclopropanesulfonamido)thiazol-4-yl)propan-2-yl)-5-(6-(trifluoromethyl)pyrazin-2-yl)picolinamide 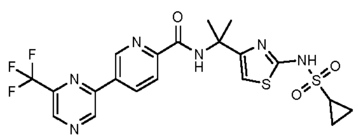 | **Ref R67** | RACEMIC, N-(1-(2-(cyclopropanesulfonamido)thiazol-4-yl)propyl)-4-(pyrazin-2-yl) benzamide 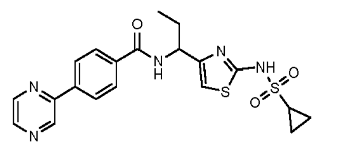 |
| **Ref R25** | 5-(6-chloropyrazin-2-yl)-N-(2-(2-(cyclopropanesulfonamido) thiazol-4-yl)propan-2-yl)picolinamide 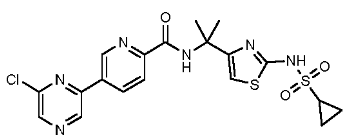 | **Ref R68** | SINGLE ENANTIOMER - stereochemistry unassigned, N-(1-(2-(cyclopropanesulfonamido)thiazol-4-yl)propyl)-4-(5-fluoropyridin-3-yl)benzamide 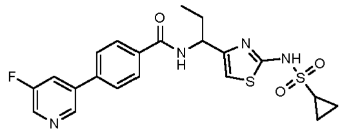 |
| **Ref R26** | N-(2-(2-(cyclopropanesulfonamido)thiazol-4-yl)propan-2-yl)-5-(6-ethoxypyrazin-2-yl)picolinamide 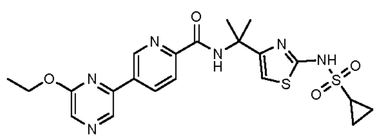 | **Ref R69** | SINGLE ENANTIOMER - stereochemistry unassigned, N-(1-(2-(cyclopropanesulfonamido)thiazol-4-yl)propyl)-4-(5-fluoropyridin-3-yl)benzamide 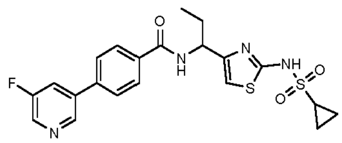 |

(continued)

| R | Name/Structure (All examples containing chiral centres are racemic unless stated) | R | Name/Structure (All examples containing chiral centres are racemic unless stated) |
|---|---|---|---|
| **Ref R27** | N-(2-(2-(cyclopropanesulfonamido)thiazol-4-yl)propan-2-yl)-[2,2'-bipyridine]-5-carboxamide 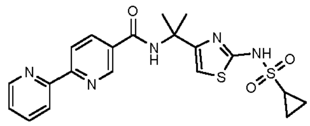 | **Ref R70** | SINGLE ENANTIOMER - stereochemistry unassigned, N-(1-(2-(cyclopropanesulfonamido)thiazol-4-yl)propyl)-4-(6-ethoxypyrazin-2-yl)-2-fluorobenzamide 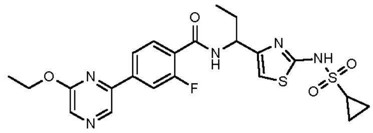 |
| **Ref R28** | 4-(5-chloropyridin-3-yl)-N-(2-(2-(cyclopropanesulfon-amido)thiazol-4-yl)propan-2-yl)benzamide 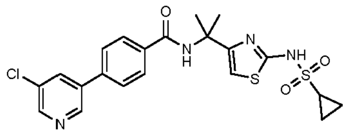 | **Ref R71** | SINGLE ENANTIOMER-stereochemistry unassigned, N-(1-(2-(cyclopropanesulfonamido)thiazol-4-yl)propyl)-4-(6-ethoxypyrazin-2-yl)-2-fluorobenzamide 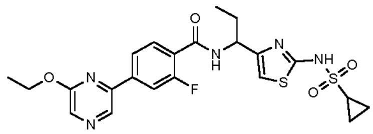 |
| **Ref R29** | N-(2-(2-(cyclopropanesulfonamido)thiazol-4-yl)propan-2-yl)-2-fluoro-4-(5-(trifluoromethyl)pyridin-3-yl) benzamide 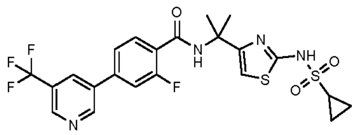 | **Ref R72** | N-(2-(2-(cyclopropanesulfon-amido)-5-methylthiazol-4-yl)propan-2-yl)-5-(6-ethoxypyrazin-2-yl)picolinamide 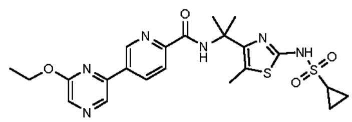 |
| **Ref R30** | 4-(5-chloropyridin-3-yl)-N-(2-(2-(cyclopropanesulfon-amido)thiazol-4-yl)propan-2-yl)-2-fluorobenzamide 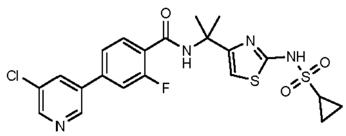 | **Ref R73** | N-(2-(5-chloro-2-(cyclopropanesulfonamido)thiazol-4-yl)propan-2-yl)-5-(6-ethoxypyrazin-2-yl)picolinamide 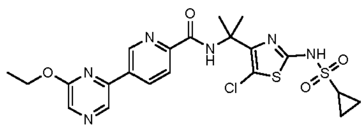 |
| **Ref R31** | N-(2-(2-(cyclopropanesulfonamido)thiazol-4-yl)propan-2-yl)-2-fluoro-4-(5-fluoropyridin-3-yl)benzamide 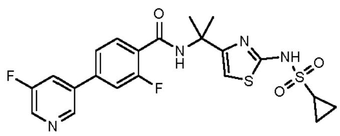 | **Ref R74** | N-(2-(2-(cyclopropanesulfon-amido)-5-methylthiazol-4-yl)propan-2-yl)-4-(6-ethoxypyrazin-2-yl)-2-fluorobenzamide 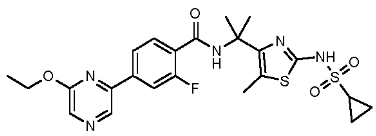 |
| **Ref R32** | N-(2-(2-(cyclopropanesulfonamido)thiazol-4-yl)propan-2-yl)-2-methoxy-4-(5-(trifluoromethyl)pyridin-3-yl)benzamide 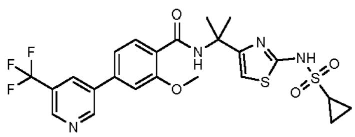 | **Ref R75** | N-(2-(5-chloro-2-(cyclopropanesulfonamido)thiazol-4-yl)propan-2-yl)-4-(6-ethoxypyrazin-2-yl)-2-fluorobenzamide 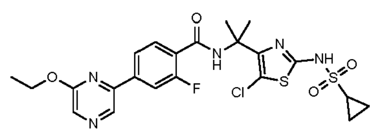 |

(continued)

| R | Name/Structure (All examples containing chiral centres are racemic unless stated) | R | Name/Structure (All examples containing chiral centres are racemic unless stated) |
|---|---|---|---|
| **Ref R33** | N-(2-(2-(cyclopropanesulfonamido)thiazol-4-yl)propan-2-yl)-4-(2-methylpyridin-3-yl)benzamide 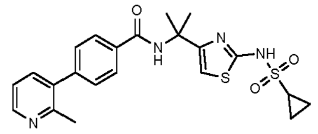 | **Ref R76** | N-(2-(2-(cyclopropanesulfon-amido)-5-methylthiazol-4-yl)propan-2-yl)-2-methyl-4-(6-(trifluoromethyl)pyrazin-2-yl) benzamide 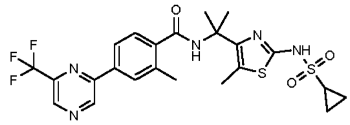 |
| **Ref R34** | 4-(5-acetylpyridin-3-yl)-N-(2-(2-(cyclopropanesulfon-amido)thiazol-4-yl)propan-2-yl)benzamide 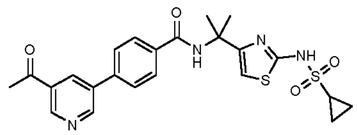 | **Ref R77** | N-(2-(5-chloro-2-(cyclopropanesulfonamido)thiazol-4-yl)propan-2-yl)-2-methyl-4-(6-(trifluoromethyl)pyrazin-2-yl) benzamide 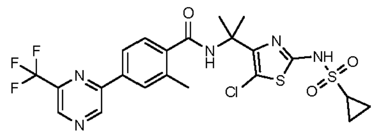 |
| **Ref R35** | N-(2-(2-(cyclopropanesulfonamido)thiazol-4-yl)propan-2-yl)-4-(5-(trifluoromethyl)pyridin-3-yl)benzamide 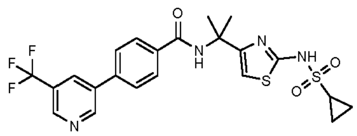 | **Ref R78** | N-(2-(2-(cyclopropanesulfon-amido)-5-methylthiazol-4-yl)propan-2-yl)-4-(6-(trifluoromethyl)pyrazin-2-yl)benzamide 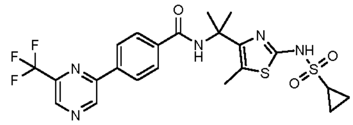 |
| **Ref R36** | N-(2-(2-(cyclopropanesulfonamido)thiazol-4-yl)propan-2-yl)-4-(5-fluoropyridin-3-yl)benzamide 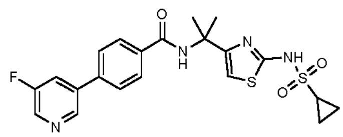 | **Ref R79** | N-(2-(5-chloro-2-(cyclopropanesulfonamido)thiazol-4-yl)propan-2-yl)-4-(6-(trifluoromethyl)pyrazin-2-yl)benzamide 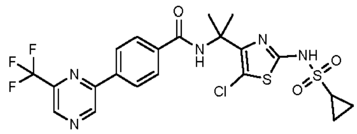 |
| **Ref R37** | N-(2-(2-(cyclopropanesulfonamido)thiazol-4-yl)propan-2-yl)-4-(5-methylpyridin-3-yl)benzamide 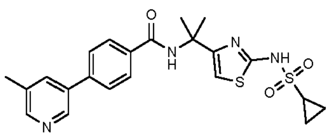 | **Ref R80** | N-(1-(2-(cyclopropanesulfonamido)thiazol-4-yl)cyclo-propyl)-5-(6-ethoxypyrazin-2-yl)picolinamide 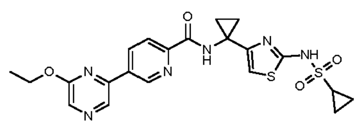 |
| **Ref R38** | N-(2-(2-(cyclopropanesulfonamido)thiazol-4-yl)propan-2-yl)-4-(5-methoxypyridin-3-yl)benzamide 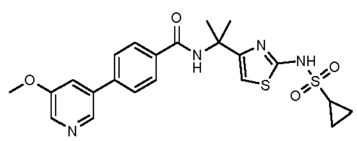 | **Ref R81** | N-(1-(2-(cyclopropanesulfonamido)thiazol-4-yl)cyclo-propyl)-4-(pyridin-3-yl)benzamide 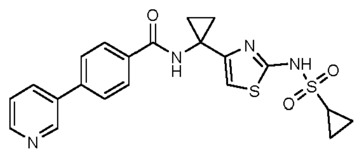 |

(continued)

| R | Name/Structure (All examples containing chiral centres are racemic unless stated) | R | Name/Structure (All examples containing chiral centres are racemic unless stated) |
|---|---|---|---|
| **Ref R39** | N-(2-(2-(cyclopropanesulfonamido)thiazol-4-yl)propan-2-yl)-4-(pyridin-3-yl)benzamide 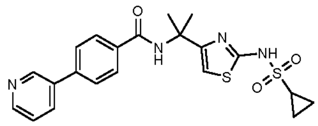 | **Ref R82** | N-(1-(2-(cyclopropanesulfonamido)thiazol-4-yl)cyclo-propyl)-4-(6-ethoxypyrazin-2-yl)-2-fluorobenzamide 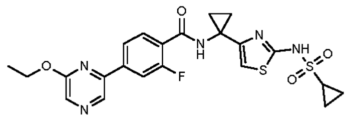 |
| **Ref R40** | N-(2-(2-(cyclopropanesulfonamido)thiazol-4-yl)propan-2-yl)-3'-(trifluoromethyl)-[1,1'-biphenyl]-4-carboxamide 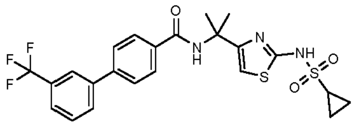 | **Ref R83** | N-(1-(2-(cyclopropanesulfonamido)thiazol-4-yl)cyclopropyl)-2-methyl-4-(6-(trifluoromethyl)pyrazin-2-yl)benzamide 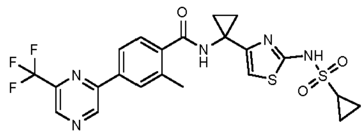 |
| **Ref R41** | N-(2-(2-(cyclopropanesulfonamido)thiazol-4-yl)propan-2-yl)-4-(6-ethylpyrazin-2-yl)-2-fluorobenzamide 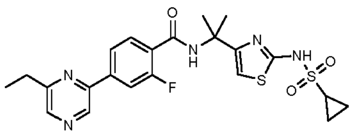 | **Ref R84** | N-(1-(2-(cyclopropanesulfonamido)thiazol-4-yl)cyclopropyl)-4-(6-(trifluoromethyl) pyrazin-2-yl)benzamide 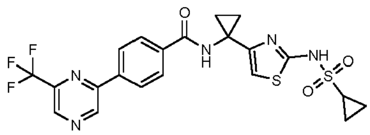 |
| **Ref R42** | N-(2-(2-(cyclopropane sulfonamido)thiazol-4-yl) propan-2-yl)-2-fluoro-4-(6-(trifluoromethyl)pyrazin-2-yl)benzamide 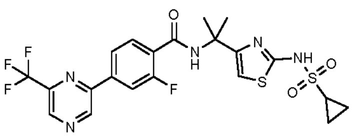 | **Ref R85** | N-(1-(2-(cyclopropanesulfonamido)thiazol-4-yl)-3-methoxypropyl)-4-(5-fluoropyridin-3-yl)benzamide 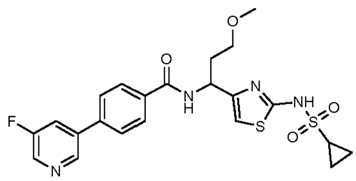 |
| **Ref R43** | N-(2-(2-(cyclopropane sulfonamido)thiazol-4-yl) propan-2-yl)-2-fluoro-4-(6-isopropoxypyrazin-2-yl) benzamide 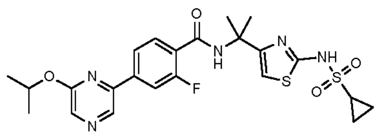 | **Ref R86** | N-(1-(2-(cyclopropanesulfonamido)thiazol-4-yl)-3-methoxypropyl)-4-(6-ethyl-pyrazin-2-yl)-2-fluorobenzamide 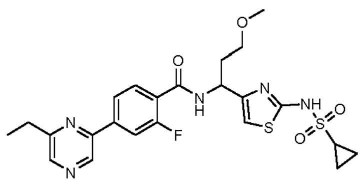 |
| **Ref R44** | N-(2-(2-(cyclopropanesulfon amido)thiazol-4-yl) propan-2-yl)-2-fluoro-4-(6-(2,2,2-trifluoroethoxy) pyrazin-2-yl)benzamide 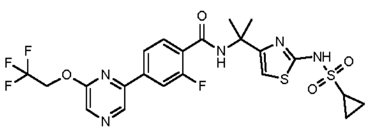 | **Ref R87** | N-(1-(2-(cyclopropanesulfon-amido)thiazol-4-yl)-3-methoxypropyl)-2-fluoro-4-(6-(trifluoromethyl)pyrazin-2-yl)benzamide 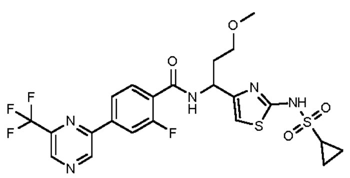 |

(continued)

| R | Name/Structure (All examples containing chiral centres are racemic unless stated) | R | Name/Structure (All examples containing chiral centres are racemic unless stated) |
|---|---|---|---|
| **Ref R45** | N-(2-(2-(cyclopropane sulfonamido)thiazol-4-yl) propan-2-yl)-2-methyl-4-(6-(trifluoromethyl)pyrazin-2-yl)benzamide 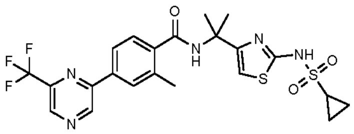 | **Ref R88** | N-(1-(2-(cyclopropanesulfon-amido)thiazol-4-yl)-3-methoxypropyl)-4-(6-ethoxypyrazin-2-yl)-2-fluorobenzamide 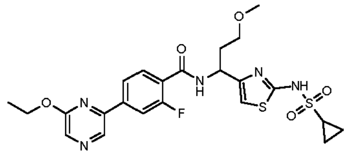 |
| **Ref R46** | N-(2-(2-(cyclopropanesulfonamido)thiazol-4-yl)propan-2-yl)-4-(6-ethoxypyrazin-2-yl)-2-methylbenzamide 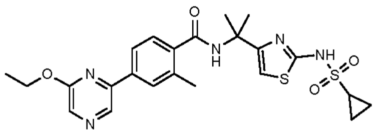 | **Ref R89** | N-(1-(2-(cyclopropanesulfon-amido)thiazol-4-yl)-3-methoxypropyl)-2-fluoro-4-(6-isopropoxypyrazin-2-yl)benzamide 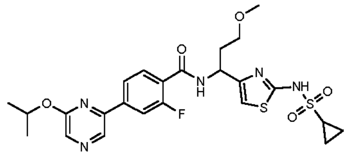 |
| **Ref R47** | N-(2-(2-(cyclopropanesulfonamido)thiazol-4-yl)propan-2-yl)-4-(6-ethoxypyrazin-2-yl)-2-(trifluoromethyl)benzamide 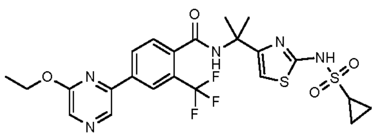 | **Ref R90** | N-(1-(2-(cyclopropanesulfon-amido)thiazol-4-yl)-3-methoxypropyl)-4-(6-ethoxy-pyrazin-2-yl)benzamide 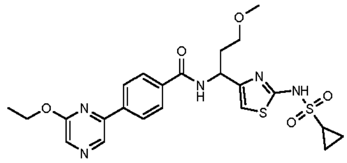 |
| **Ref R48** | N-(2-(2-(cyclopropanesulfonamido)thiazol-4-yl)propan-2-yl)-2-methoxy-4-(6-(trifluoromethyl)pyrazin-2-yl)benzamide 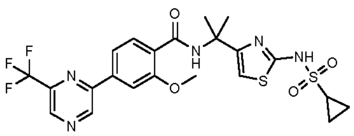 | **Ref R91** | N-(1-(2-(cyclopropanesulfon-amido)thiazol-4-yl)ethyl)-4-(6-ethoxypyrazin-2-yl)-2-fluorobenzamide 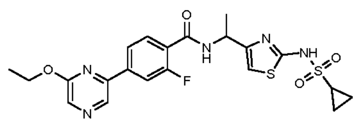 |
| **Ref R49** | 4-(6-chloropyrazin-2-yl)-N-(2-(2-(cyclopropanesulfonamido)thiazol-4-yl)propan-2-yl)-2-methoxybenzamide 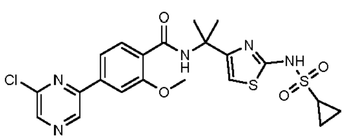 | **Ref R92** | SINGLE ENANTIOMER - stereochemistry unassigned N-(1-(2-(cyclopropanesulfon-amido)thiazol-4-yl)-3-methoxypropyl)-4-(6-ethoxypyrazin-2-yl)-2-fluorobenzamide 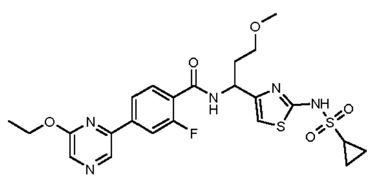 |

(continued)

| R | Name/Structure (All examples containing chiral centres are racemic unless stated) | R | Name/Structure (All examples containing chiral centres are racemic unless stated) |
|---|---|---|---|
| **Ref R50** | 4-(6-cyanopyrazin-2-yl)-N-(2-(2-(cyclopropanesulfonamido)thiazol-4-yl)propan-2-yl)-2-methoxybenzamide 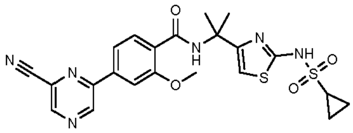 | **Ref R93** | SINGLE ENANTIOMER - stereochemistry unassigned N-(1-(2-(cyclopropanesulfonamido)thiazol-4-yl)-3-methoxypropyl)-4-(6-ethoxypyrazin-2-yl)-2-fluorobenzamide 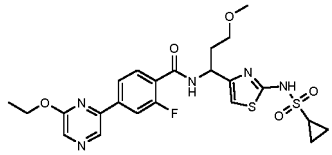 |

*N*-(4-(2-((4-(6-Ethoxypyrazin-2-yl)-2-fluorobenzyl)amino)propan-2-yl)thiazol-2-yl)cyclopropanesulfonamide **R94**

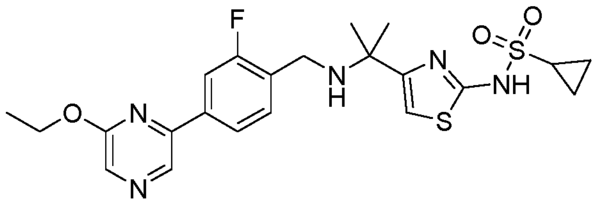

A solution of 4-(6-ethoxypyrazin-2-yl)-2-fluorobenzaldehyde (75 mg, 0.305 mmol) **INTF57** and *N*-(4-(2-aminopropan-2-yl)thiazol-2-yl)cyclopropanesulfonamide **INTE14** (80 mg, 0.305 mmol) in DCM (2 mL) was treated with AcOH (0.02 mL, 0.35 mmol) and stirred for 1 hr whereupon sodium triacetoxyborohydride (70 mg, 0.33 mmol) was added and the reaction mixture was stirred at RT for 4 hrs. The reaction mixture was treated with 1% $NH_3$ in MeOH (2 mL) and concentrated in vacuo. The crude product was purified by chromatography on RP Flash C18 (12 g cartridge, 15-70% MeCN/10 mM Ammonium Bicarbonate) to *N*-(4-(2-((4-(6-ethoxypyrazin-2-yl)-2-fluorobenzyl)amino)propan-2-yl)thiazol-2-yl)cyclopropanesulfonamide (30 mg, 0.056 mmol, 18% yield) was isolated as a colourless solid. Rt 0.95 min (UPLC acidic); *m/z* 492 $(M+H)^+$ $(ES^+)$. $^1H$ NMR (500 MHz, DMSO-d6) δ 8.86 - 8.81 (m, 1H), 8.28 - 8.18 (m, 1H), 7.96 - 7.93 (m, 1H), 7.91 - 7.82 (m, 1H), 7.67 - 7.50 (m, 1H), 6.49-6.14 (v. br. m., 3H), 4.49 (q, J = 7.1 Hz, 2H), 3.59 - 3.48 (m, 2H), 2.50 - 2.39 (m, 1H), 1.48 - 1.14 (m, 9H), 0.91 - 0.59 (m, 4H).

*N*-(4-(2-(((5-(6-Ethoxypyrazin-2-yl)pyridin-2-yl)methyl)amino)propan-2-yl)thiazol-2-yl)cyclopropanesulfonamide **R95**

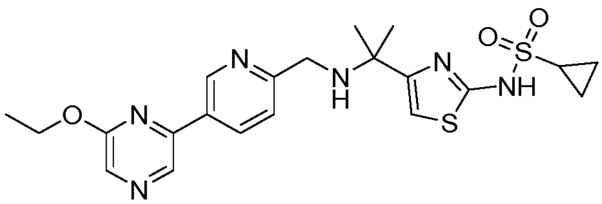

Prepared as for **R94** using 5-(6-ethoxypyrazin-2-yl)picolinaldehyde (70 mg, 0.305 mmol) **INTF55** and *N*-(4-(2-aminopropan-2-yl)thiazol-2-yl)cyclopropanesulfonamide **INTE14** (80 mg, 0.305 mmol) to afford *N*-(4-(2-(((5-(6-ethoxypyrazin-2-yl)pyridin-2-yl)methyl)amino)propan-2-yl)thiazol-2-yl)cyclopropanesulfonamide (36 mg, 0.073 mmol, 24% yield) as a red solid. Rt 0.81 min (UPLC acidic); *m/z* 475 $(M+H)^+$ $(ES^+)$. $^1H$ NMR (500 MHz, DMSO-d6) δ 9.21 (d, J = 2.3 Hz, 1H), 8.88 (s, 1H), 8.44 (dd, J = 8.2, 2.4 Hz, 1H), 8.30 (s, 1H), 7.62 (d, J = 8.1 Hz, 1H), 6.50 (s, 1H), 4.49 (q, J = 7.0 Hz, 2H), 3.68 (s, 2H), 2.59 - 2.53 (m, 1H), 1.45 - 1.37 (m, 9H), 0.92 - 0.82 (m, 4H), 2 x N-H not observed.

## Biological Examples

### Biological Example 1 - Human CTPS1 Enzyme Inhibition

The enzyme inhibitory activities of compounds invented against the target of interest were determined using the ADP-Glo™ Max assay (Promega, UK). Assays for human CTPS1 were performed in 1x assay buffer containing 50mM Tris, 10mM $MgCl_2$, 0.01% Tween-20, pH to 8.0 accordingly. Finally, immediately before use, L-cysteine was added to the 1x assay buffer to a final concentration of 2mM. All reagents are from Sigma-Aldrich unless specified otherwise. Human full length active C-terminal FLAG-$His_8$-tag CTPS1 (UniProtKB - P17812, CTPS1[1-591]-GGDYKDDDDKGGHHHHHHHH (CTPS1[1-591]-SEQ ID NO: 1)) was obtained from Proteros biostructures GmbH.

*Assay Procedure*

3x human CTPS1 protein was prepared in 1x assay buffer to the final working protein concentration required for the reaction. A 2uL volume per well of 3x human CTPS1 protein was mixed with 2uL per well of 3x test compound (compound prepared in 1x assay buffer to an appropriate final 3x compound concentration respective to the concentration response curve designed for the compounds under test) for 10 minutes at 25°C. The enzymatic reaction was then initiated by addition of a 2uL per well volume of a pre-mixed substrate mix (UltraPure ATP from ADP-Glo™ Max kit (0.31mM), GTP (0.034mM), UTP (0.48mM) and L-glutamine (0.186mM)) and the mixture was incubated for an appropriate amount of time within the determined linear phase of the reaction at 25°C under sealed plate conditions with constant agitation at 500 revolutions per minute (rpm). ADP-Glo™ Max reagent was added for 60 minutes (6μL per well) and subsequently ADP-Glo™ Max development reagent was added for 60 minutes (12uL per well) prior to signal detection in a microplate reader (EnVision® Multilabel Reader, Perkin Elmer). Following each reagent addition over the course of the assay, assay plates were pulse centrifuged for 30 seconds at 500rpm.

In all cases, the enzyme converts ATP to ADP and the ADP-Glo™ Max reagent subsequently depletes any remaining endogenous ATP in the reaction system. The ADP-Glo™ Max detection reagent converts the ADP that has been enzymatically produced back into ATP and using ATP as a substrate together with luciferin for the enzyme luciferase, light is generated which produces a detectable luminescence. The luminescent signal measured is directly proportional to the amount of ADP produced by the enzyme reaction and a reduction in this signal upon compound treatment demonstrates enzyme inhibition. The percentage inhibition produced by each concentration of compound was calculated using the equation shown below:

$$\%\,Inhibition = 1 - \frac{(Mean_{Min} - Mean_{Inh})}{(Mean_{Min} - Mean_{Max})}\; x\;100$$

Percentage inhibition was then plotted against compound concentration, and the 50% inhibitory concentration ($IC_{50}$) was determined from the resultant concentration-response curve.

The data for compounds of formula (I) tested are presented below.

**Table 9:** *Human CTPS1 Enzyme Inhibition data grouped by potency range* (± indicates $IC_{50}$ in the range of >10 to 20 micromolar, + indicates $IC_{50}$ in the range >1 to 10 micromolar, ++ indicates $IC_{50}$ in the range >0.1 to 1 micromolar, +++ indicates $IC_{50}$ of ≤0.1 micromolar)

| P | CTPS1 |
|---|---|
| Ref P1 | ++ |
| Ref P2 | +++ |
| Ref P3 | +++ |
| Ref P4 | ++ |
| Ref P5 | + |
| Ref P6 | ++ |
| Ref P7 | ++ |
| Ref P8 | +++ |
| Ref P9 | +++ |

(continued)

| P | CTPS1 |
|---|---|
| Ref P10 | +++ |
| Ref P11 | +++ |
| Ref P12 | +++ |
| Ref P13 | ++ |
| Ref P14 | ++ |
| Ref P15 | + |
| Ref P16 | ++ |
| Ref P17 | + |
| Ref P18 | +++ |
| Ref P19 | +++ |
| Ref P20 | +++ |
| Ref P21 | +++ |
| Ref P22 | ++ |
| Ref P23 | ++ |
| Ref P24 | ++ |
| Ref P25 | ++ |
| Ref P26 | ++ |
| Ref P27 | +++ |
| Ref P28 | ++ |
| Ref P29 | + |
| Ref P30 | +++ |
| Ref P31 | +++ |
| Ref P32 | +++ |
| Ref P33 | +++ |
| Ref P34 | +++ |
| Ref P35 | ++ |
| Ref P36 | + |
| Ref P37 | +++ |
| Ref P38 | +++ |
| Ref P39 | +++ |
| Ref P40 | ++ |
| Ref P41 | +++ |
| Ref P42 | + |
| Ref P43 | ++ |
| Ref P44 | ++ |
| Ref P45 | ++ |
| Ref P46 | ++ |
| Ref P47 | +++ |
| Ref P48 | ++ |

(continued)

| P | CTPS1 |
|---|---|
| **Ref P49** | +++ |
| **Ref P50** | + |
| **Ref P51** | ++ |
| **Ref P52** | ++ |
| **Ref P53** | ++ |
| **Ref P54** | ++ |
| **Ref P55** | +++ |
| **Ref P56** | ++ |
| **Ref P57** | ++ |
| **Ref P58** | ++ |
| **Ref P59** | + |
| **Ref P60** | + |
| **Ref P61** | + |
| **Ref P62** | + |
| **Ref P63** | + |
| **Ref P64** | +++ |
| **Ref P65** | +++ |
| **Ref P66** | ++ |
| **Ref P67** | +++ |
| **Ref P68** | ++ |
| **Ref P69** | ++ |
| **Ref P70** | + |
| **Ref P71** | + |
| **Ref P72** | ++ |
| **Ref P73** | ++ |
| **Ref P74** | ++ |
| **Ref P75** | ++ |
| **Ref P76** | ++ |
| **Ref P77** | + |
| **Ref P78** | ++ |
| **Ref P79** | ++ |
| **Ref P80** | ++ |
| **Ref P81** | +++ |
| **Ref P82** | + |
| **Ref P83** | +++ |
| **Ref P84** | ++ |
| **Ref P85** | ++ |
| **Ref P86** | + |
| **Ref P87** | +++ |

(continued)

| P | CTPS1 |
|---|---|
| Ref P88 | +++ |
| Ref P89 | +++ |
| Ref P90 | ++ |
| Ref P91 | ++ |
| Ref P92 | ++ |
| Ref P93 | + |
| Ref P94 | +++ |
| Ref P95 | +++ |
| Ref P96 | +++ |
| Ref P97 | +++ |
| Ref P98 | +++ |
| Ref P99 | ++ |
| Ref P100 | ++ |
| Ref P101 | ++ |
| Ref P102 | ++ |
| Ref P103 | ++ |
| Ref P104 | ++ |
| Ref P105 | +++ |
| Ref P106 | +++ |
| Ref P107 | +++ |
| Ref P108 | +++ |
| Ref P109 | ++ |
| Ref P110 | +++ |
| Ref P111 | ++ |
| Ref P112 | +++ |
| Ref P113 | +++ |
| Ref P114 | +++ |
| Ref P115 | +++ |
| Ref P116 | ++ |
| Ref P117 | +++ |
| Ref P118 | +++ |
| Ref P122 | ++ |
| Ref P123 | ++ |
| Ref P124 | ++ |
| Ref P125 | ++ |
| Ref P126 | +++ |
| Ref P128 | ++ |
| Ref P129 | ++ |
| Ref P130 | ++ |

(continued)

| P | CTPS1 |
|---|---|
| **Ref P131** | ++ |
| **Ref P132** | ++ |
| **Ref P133** | + |
| **Ref P134** | ++ |
| **Ref P135** | ++ |
| **Ref P136** | +++ |
| **Ref P137** | +++ |
| **Ref P138** | ++ |
| **Ref P139** | ++<br>n = 4 |
| | +++<br>n=6 |
| **Ref P140** | ++ |
| **Ref P141** | ++ |
| **Ref P142** | ++ |
| **Ref P143** | +++ |
| **Ref P144** | +++ |
| **Ref P145** | +++ |
| **Ref P146** | +++ |
| **Ref P147** | ++ |
| **Ref P148** | +++ |
| **Ref P149** | +++ |
| **Ref P150** | +++ |
| **Ref P151** | +++ |
| **Ref P152** | +++ |
| **Ref P153** | +++ |
| **Ref P154** | +++ |
| **Ref P155** | +++ |
| **Ref P156** | +++ |
| **Ref P157** | +++ |
| **Ref P158** | ++ |
| **Ref P159** | +++ |
| **Ref P160** | +++ |
| **Ref P161** | +++ |
| **Ref P162** | +++ |
| **Ref P163** | +++ |
| **Ref P164** | +++ |
| **Ref P165** | +++ |
| **Ref P166** | +++ |
| **Ref P167** | ++ |

(continued)

| P | CTPS1 |
|---|---|
| **Ref P168** | ++ |
| **Ref P169** | ++ |
| **Ref P170** | ++ |
| **Ref P171** | + |
| **Ref P172** | ++ |
| **Ref P173** | +++ |
| **Ref P174** | + |
| **Ref P175** | + |
| **Ref P176** | + |
| **Ref P177** | + |
| **Ref P178** | + |
| **Ref P179** | + |
| **Ref P180** | + |
| **Ref P181** | + |
| **Ref P182** | ++ |
| **Ref P183** | ++ |
| **Ref P184** | ++ |
| **Ref P185** | + |
| **Ref P186** | +++ |
| **Ref P187** | ++ |
| **Ref P188** | +++ |
| **Ref P189** | + |
| **Ref P190** | ++ |
| **Ref P191** | ++ |
| **Ref P192** | + |
| **Ref P193** | + |
| **Ref P194** | 58.6 uM |
| **Ref P195** | +++ |
| **Ref P196** | +++ |
| **Ref P197** | +++ |
| **Ref P198** | +++ |
| **Ref P199** | ++ |
| **Ref P200** | +++ |
| **Ref P201** | +++ |
| **Ref P202** | +++ |
| **Ref P203** | +++ |
| **Ref P204** | +++ |
| **Ref P205** | +++ |
| **Ref P206** | +++ |

(continued)

| P | CTPS1 |
|---|---|
| Ref P207 | +++ |
| Ref P208 | +++ |
| Ref P209 | ++ |
| Ref P210 | ++ |
| Ref P211 | ++ |
| Ref P212 | ++ |
| Ref P213 | ++ |
| Ref P214 | ++ |
| Ref P215 | ++ |
| Ref P216 | +++ |
| Ref P217 | ++ |
| Ref P218 | +++ |
| Ref P219 | ++ |
| Ref P220 | ++ |
| Ref P221 | ++ |
| Ref P222 | ++ |
| Ref P223 | ++ |
| Ref P224 | ++ |
| Ref P225 | +++ |

**Table 10:** *Human CTPS1 Enzyme Inhibition data grouped by potency range* (± indicates $IC_{50}$ in the range of >10 to 20 micromolar, + indicates $IC_{50}$ in the range >1 to 10 micromolar, ++ indicates $IC_{50}$ in the range >0.1 to 1 micromolar, +++ indicates $IC_{50}$ of ≤0.1 micromolar)

| T# | CTPS1 |
|---|---|
| Ref T1 | + |
| Ref T2 | ++ |
| Ref T3 | ++ |
| Ref T4 | + |
| Ref T5 | +++ |
| Ref T6 | ++ |
| Ref T7 | +++ |
| Ref T8 | +++ |
| Ref T9 | +++ |
| Ref T10 | +++ |
| Ref T11 | ++ |
| Ref T12 | ++ |
| Ref T13 | ++ |
| Ref T14 | ++ |
| Ref T15 | ++ |
| Ref T16 | ++ |

(continued)

| T# | CTPS1 |
|---|---|
| Ref T17 | ++ |
| Ref T18 | +++ |
| Ref T19 | ++ |
| Ref T20 | ++ |
| Ref T21 | + |
| Ref T22 | +++ |
| Ref T23 | ++ |
| Ref T24 | ++ |
| Ref T25 | + |
| Ref T26 | ++ |
| Ref T27 | ++ |
| Ref T28 | ++ |
| Ref T29 | +++ |
| Ref T30 | +++ |
| Ref T31 | + |
| Ref T32 | +++ |
| Ref T33 | +++ |
| Ref T34 | ++ |
| Ref T35 | +++ |
| Ref T36 | ++ |
| Ref T37 | ++ |
| Ref T38 | +++ |
| Ref T39 | ++ |
| Ref T40 | +++ |
| Ref T41 | ++ |
| Ref T42 | +++ |
| Ref T43 | ++ |
| Ref T44 | ++ |
| Ref T45 | ++ |
| Ref T46 | + |
| Ref T47 | +++ |
| Ref T48 | +++ |
| Ref T49 | ++ |
| Ref T50 | +++ |
| Ref T51 | +++ |
| Ref T52 | +++ |
| Ref T53 | + |
| Ref T54 | ++ |
| Ref T55 | +++ |

(continued)

| T# | CTPS1 |
|---|---|
| Ref T56 | +++ |
| Ref T57 | +++ |
| Ref T58 | +++ |
| Ref T59 | +++ |
| Ref T60 | ++ |
| Ref T61 | +++ |
| Ref T62 | ++ |
| Ref T63 | ++ |
| Ref T64 | ++ |
| Ref T65 | ++ |
| Ref T66 | ++ |
| Ref T67 | ++ |
| Ref T68 | ++ |
| Ref T69 | + |
| Ref T70 | ++ |
| Ref T71 | +++ |
| Ref T72 | +++ |
| Ref T73 | +++ |
| Ref T74 | ++ |
| Ref T75 | +++ |
| Ref T76 | +++ |
| Ref T77 | +++ |
| Ref T78 | +++ |
| Ref T79 | ++ |
| Ref T80 | +++ |
| Ref T81 | +++ |
| Ref T82 | +++ |
| Ref T83 | + |
| Ref T84 | ++ |
| Ref T85 | ++ |
| Ref T86 | + |
| Ref T87 | ++ |
| Ref T88 | ++ |
| Ref T89 | +++ |
| Ref T90 | +++ |
| Ref T91 | +++ |
| Ref T92 | +++ |
| Ref T93 | +++ |
| Ref T94 | +++ |

(continued)

| T# | CTPS1 |
|---|---|
| Ref T95 | +++ |
| Ref T96 | +++ |
| Ref T97 | ++ |
| Ref T98 | ++ |
| Ref T99 | +++ |
| Ref T100 | ++ |
| Ref T101 | + |
| Ref T102 | +++ |
| Ref T103 | ++ |
| Ref T104 | ++ |
| Ref T105 | + |
| Ref T106 | +++ |
| Ref T107 | +++ |
| Ref T108 | +++ |
| Ref T109 | +++ |
| Ref T110 | ++ |
| Ref T111 | +++ |
| Ref T112 | +++ |
| Ref T113 | + |
| Ref T114 | ++ |
| Ref T115 | +++ |
| Ref T116 | ++ |
| Ref T117 | ++ |
| Ref T118 | +++ |
| Ref T119 | +++ |
| Ref T120 | +++ |
| Ref T121 | +++ |
| Ref T122 | +++ |
| Ref T123 | +++ |
| Ref T124 | +++ |
| Ref T125 | +++ |
| Ref T126 | +++ |
| Ref T127 | +++ |
| Ref T128 | +++ |
| Ref T129 | +++ |
| Ref T130 | +++ |
| Ref T131 | +++ |
| Ref T132 | ++ |
| Ref T133 | +++ |

(continued)

| T# | CTPS1 |
|---|---|
| **Ref T134** | +++ |
| **Ref T135** | ++ |
| **Ref T136** | +++ |
| **Ref T137** | +++ |
| **Ref T138** | +++ |
| **Ref T139** | +++ |
| **Ref T140** | ++ |
| **Ref T141** | ++ |
| **Ref T142** | +++ |
| **Ref T143** | +++ |
| **Ref T144** | ++ |
| **Ref T145** | +++ |
| **Ref T146** | ++ |
| **Ref T147** | +++ |
| **Ref T148** | ++ |
| **Ref T149** | +++ |
| **Ref T150** | ++ |
| **Ref T151** | ++ |
| **Ref T152** | ++ |
| **Ref T153** | ++ |
| **Ref T154** | ++ |
| **Ref T155** | +++ |
| **Ref T156** | +++ |
| **Ref T157** | +++ |
| **Ref T158** | +++ |
| **Ref T159** | +++ |
| **Ref T160** | +++ |
| **Ref T161** | ++ (n = 2) |
| | + (n = 6) |
| **Ref T163** | ++ |
| **Ref T164** | + |
| **Ref T169** | ++ |
| **Ref T170** | ++ |
| **Ref T171** | +++ |
| **Ref T172** | +++ |
| **Ref T173** | +++ |
| **Ref T174** | +++ |
| **Ref T175** | +++ |
| **Ref T176** | +++ |

(continued)

| T# | CTPS1 |
|---|---|
| Ref T177 | +++ |
| Ref T178 | + |
| Ref T179 | +++ |
| Ref T180 | +++ |
| Ref T181 | ++ |
| Ref T182 | +++ |
| Ref T183 | +++ |
| Ref T184 | + |
| Ref T185 | +++ (n = 1) |
| | ++ (n = 2) |
| Ref T186 | +++ |
| Ref T187 | +++ |
| Ref T188 | ++ |
| Ref T189 | +++ |
| Ref T190 | +++ |
| Ref T191 | +++ |
| Ref T192 | +++ |
| Ref T193 | +++ |
| Ref T194 | +++ |
| Ref T195 | +++ |
| Ref T196 | +++ |
| Ref T197 | +++ |
| Ref T198 | +++ |
| Ref T199 | +++ |
| Ref T200 | +++ |
| Ref T201 | +++ |
| Ref T202 | +++ |
| Ref T203 | +++ |
| Ref T204 | +++ |
| Ref T205 | +++ |
| Ref T206 | +++ |
| Ref T207 | +++ |
| Ref T208 | +++ |
| Ref T209 | +++ |
| Ref T210 | +++ |
| Ref T211 | +++ (n = 1) |
| | ++ (n = 2) |
| Ref T212 | +++ |
| Ref T213 | ++ |

(continued)

| T# | CTPS1 |
|---|---|
| Ref T214 | +++ |
| Ref T215 | +++ |
| Ref T216 | ++ |
| Ref T217 | +++ |
| Ref T218 | ++ |
| Ref T219 | +++ |
| Ref T220 | +++ |
| Ref T221 | +++ |
| Ref T222 | ++ |
| Ref T223 | +++ |
| Ref T224 | +++ |
| Ref T225 | +++ |
| Ref T226 | +++ |
| Ref T227 | ++ |
| Ref T228 | +++ |
| Ref T229 | +++ |
| Ref T230 | +++ |
| Ref T231 | + |
| Ref T232 | ++ |
| Ref T233 | + |
| Ref T234 | + |
| Ref T235 | ++ |
| Ref T236 | ++ |
| Ref T237 | + |
| Ref T238 | + |
| Ref T239 | ++ |
| Ref T240 | ++ |
| Ref T241 | ++ |
| Ref T242 | + |
| Ref T243 | + |
| Ref T244 | ++ |
| Ref T245 | + |
| Ref T246 | ++ |
| Ref T247 | ++ |
| Ref T248 | + |
| Ref T249 | +++ |
| Ref T250 | + |
| Ref T251 | + |
| Ref T252 | +++ |

(continued)

| T# | CTPS1 |
|---|---|
| **Ref T253** | +++ |
| **Ref T254** | ++ |
| **Ref T255** | + |
| **Ref T256** | +++ |
| **Ref T257** | ++ |
| **Ref T258** | ++ |
| **Ref T259** | +++ |
| **Ref T260** | + |
| **Ref T261** | +++ |
| **Ref T262** | +++ |
| **Ref T263** | + |
| **Ref T264** | ++ |
| **Ref T265** | +++ |
| **Ref T266** | +++ |
| **Ref T267** | +++ |
| **Ref T268** | ++ |
| **Ref T269** | ++ |
| **Ref T270** | ++ |
| **Ref T271** | + |
| **Ref T272** | +++ |
| **Ref T273** | +++ |
| **Ref T274** | +++ |
| **Ref T275** | +++ |
| **Ref T276** | ++ |
| **Ref T277** | ++ |
| **Ref T278** | +++ |
| **Ref T279** | + |
| **Ref T280** | + |
| **Ref T281** | ++ |
| **Ref T282** | ++ |
| **Ref T283** | ++ |
| **Ref T284** | + |
| **Ref T285** | +++ |
| **Ref T286** | +++ |
| **Ref T287** | +++ |
| **Ref T288** | ++ |
| **Ref T289** | ++ |
| **Ref T290** | +++ |
| **Ref T291** | +++ |

(continued)

| T# | CTPS1 |
|---|---|
| Ref T292 | ++ |
| Ref T293 | ++ |
| Ref T294 | ++ |
| Ref T295 | ++ |
| Ref T296 | ++ |
| Ref T297 | + |
| Ref T298 | +++ |
| Ref T299 | ++ |
| Ref T300 | ++ |
| Ref T301 | +++ |
| Ref T302 | ++ |
| Ref T303 | + |
| Ref T304 | ++ |
| Ref T305 | ++ |
| Ref T306 | ++ |
| Ref T307 | +++ |
| Ref T308 | ++ |
| Ref T309 | + |
| Ref T310 | + |
| Ref T311 | + |
| Ref T312 | ++ |
| Ref T313 | ++ |
| Ref T314 | ++ |
| Ref T315 | ++ |
| Ref T316 | ++ |
| Ref T317 | ++ |
| Ref T318 | + |
| Ref T319 | + |
| Ref T320 | ++ |
| Ref T321 | +++ |
| Ref T322 | ++ |
| Ref T325 | +++ |
| Ref T326 | +++ |
| Ref T327 | +++ |
| Ref T328 | +++ |
| Ref T329 | +++ |
| Ref T330 | +++ |
| Ref T331 | +++ |
| Ref T332 | ++ |

(continued)

| T# | CTPS1 |
|---|---|
| Ref T333 | + |
| Ref T334 | ++ |
| Ref T335 | ++ |
| Ref T336 | ++ |
| Ref T337 | +++ |
| Ref T338 | + |
| Ref T339 | + |
| Ref T340 | ++ |
| Ref T341 | +++ |
| Ref T342 | +++ |
| Ref T343 | +++ |
| Ref T344 | +++ |
| Ref T345 | +++ |
| Ref T346 | ++ |
| Ref T347 | +++ |
| Ref T348 | ++ |
| Ref T349 | ++ |
| Ref T350 | ++ |
| Ref T351 | ++ (n = 1) |
| | +++ (n = 2) |
| Ref T352 | ++ |
| Ref T353 | ++ |
| Ref T354 | ++ |
| Ref T355 | + |
| Ref T356 | +++ |
| Ref T357 | +++ |
| Ref T358 | +++ |
| Ref T359 | +++ |
| Ref T360 | ++ |
| Ref T361 | ++ |
| Ref T362 | +++ |
| Ref T363 | +++ |
| Ref T364 | ++ |
| Ref T365 | +++ |
| Ref T366 | ++ |
| Ref T367 | +++ |
| Ref T368 | +++ |
| Ref T369 | ++ |
| Ref T370 | + |

(continued)

| T# | CTPS1 |
|---|---|
| **Ref T371** | +++ |
| **Ref T372** | ++ |
| **Ref T373** | +++ |
| **Ref T374** | + |
| **Ref T375** | ++ |
| **Ref T376** | ++ |
| **Ref T377** | ++ |
| **Ref T378** | + |
| **Ref T379** | + |
| **Ref T380** | +++ |
| **Ref T381** | +++ |
| **Ref T382** | +++ |
| **Ref T383** | +++ |
| **Ref T384** | ++ |
| **Ref T385** | +++ |
| **Ref T386** | ++ |
| **Ref T387** | +++ |
| **Ref T388** | +++ |
| **Ref T389** | ++ |
| **Ref T390** | +++ |
| **Ref T391** | +++ |
| **Ref T392** | +++ |
| **Ref T393** | ++ |
| **Ref T394** | +++ |
| **Ref T395** | +++ |
| **Ref T396** | ++ |
| **Ref T397** | ++ |
| **Ref T398** | +++ |
| **Ref T399** | +++ |
| **Ref T400** | +++ |
| **Ref T401** | ++ |
| **Ref T402** | + |
| **Ref T403** | ++ |
| **Ref T404** | ++ |
| **Ref T405** | +++ |
| **Ref T406** | ++ |
| **Ref T407** | +++ |
| **Ref T408** | +++ |
| **Ref T409** | +++ |

(continued)

| T# | CTPS1 |
|---|---|
| Ref T410 | +++ |
| Ref T411 | +++ |
| Ref T412 | +++ |
| Ref T413 | +++ |
| Ref T414 | +++ |
| Ref T415 | ++ |
| Ref T416 | +++ |
| Ref T417 | ++ |
| Ref T418 | ++ |
| Ref T419 | +++ |
| Ref T420 | +++ |
| Ref T421 | +++ |
| Ref T422 | ++ |
| Ref T423 | +++ |
| Ref T424 | +++ |
| Ref T425 | +++ |
| Ref T426 | ++ |
| Ref T427 | ++ |
| Ref T428 | ++ |
| Ref T429 | +++ |
| Ref T430 | ++ |
| Ref T431 | +++ |
| Ref T432 | ++ |
| Ref T433 | +++ |
| Ref T434 | +++ |
| Ref T435 | +++ |
| Ref T436 | +++ |
| Ref T437 | +++ |
| Ref T438 | +++ |
| Ref T439 | +++ |
| Ref T440 | +++ |
| Ref T441 | +++ |
| Ref T442 | +++ |
| Ref T443 | +++ |
| Ref T444 | +++ |
| Ref T445 | +++ |
| Ref T446 | +++ |
| Ref T447 | +++ |
| Ref T448 | +++ |

(continued)

| T# | CTPS1 |
|---|---|
| Ref T449 | +++ |
| Ref T450 | +++ |
| Ref T451 | +++ |
| Ref T452 | +++ |
| Ref T453 | +++ |
| Ref T454 | +++ |
| Ref T455 | +++ |
| Ref T456 | +++ |
| Ref T457 | +++ |
| Ref T458 | +++ |
| Ref T459 | +++ |
| Ref T463 | ++ |
| Ref T464 | +++ |
| Ref T465 | + |

**Table 11:** *Human CTPS1 Enzyme Inhibition data grouped by potency range* (± indicates $IC_{50}$ in the range of >10 to 21 micromolar, + indicates $IC_{50}$ in the range >1 to 10 micromolar, ++ indicates $IC_{50}$ in the range >0.1 to 1 micromolar, +++ indicates $IC_{50}$ of ≤0.1 micromolar)

| R | CTPS1 | R | CTPS1 | R | CTPS1 |
|---|---|---|---|---|---|
| Ref R1 | ++ | Ref R35 | +++ | Ref R69 | +++ |
| Ref R2 | ++ | Ref R36 | ++ | Ref R70 | ++ |
| Ref R3 | ++ | Ref R37 | +++ | Ref R71 | +++ |
| Ref R4 | +++ | Ref R38 | ++ | Ref R72 | + |
| Ref R5 | +++ | Ref R39 | ++ | Ref R73 | + |
| Ref R6 | +++ | Ref R40 | ++ | Ref R74 | ++ |
| Ref R7 | ++ | Ref R41 | ++ | Ref R75 | ++ |
| Ref R8 | + | Ref R42 | +++ | Ref R76 | ++ |
| Ref R9 | + | Ref R43 | +++ | Ref R77 | + |
| Ref R10 | +++ | Ref R44 | ++ | Ref R78 | ++ |
| Ref R11 | +++ | Ref R45 | ++ | Ref R79 | ++ |
| Ref R12 | +++ | Ref R46 | ++ | Ref R80 | +++ |
| Ref R13 | +++ | Ref R47 | ++ | Ref R81 | ++ |
| Ref R14 | +++ | Ref R48 | ++ | Ref R82 | +++ |
| Ref R15 | +++ | Ref R49 | ++ | Ref R83 | +++ |
| Ref R16 | ++ | Ref R50 | + | Ref R84 | +++ |
| Ref R17 | ++ | Ref R51 | +++ | Ref R85 | ++ |
| Ref R18 | ++ | Ref R52 | +++ | Ref R86 | ++ |
| Ref R19 | +++ | Ref R53 | ++ | Ref R87 | ++ |
| Ref R20 | +++ | Ref R54 | +++ | Ref R88 | ++ |
| Ref R21 | +++ | Ref R55 | +++ | Ref R89 | +++ |

(continued)

| R | CTPS1 | R | CTPS1 | R | CTPS1 |
|---|---|---|---|---|---|
| Ref R22 | ++ | Ref R56 | +++ | Ref R90 | +++ |
| Ref R23 | ++ | Ref R57 | ++ | Ref R91 | +++ |
| Ref R24 | ++ | Ref R58 | ++ | Ref R92 | +++ |
| Ref R25 | ++ | Ref R59 | ++ | Ref R93 | +++ |
| Ref R26 | ++ | Ref R60 | ++ | | |
| Ref R27 | + | Ref R61 | ++ | | |
| Ref R28 | +++ | Ref R62 | ++ | | |
| Ref R29 | +++ | Ref R63 | ++ | | |
| Ref R30 | ++ | Ref R64 | ++ | | |
| Ref R31 | ++ | Ref R65 | +++ | | |
| Ref R32 | ++ | Ref R66 | ++ | | |
| Ref R33 | + | Ref R67 | ++ | | |
| Ref R34 | + | Ref R68 | + | | |

All reference examples disclosed in Tables 9 to 11 were found to demonstrate inhibition of CTPS1 enzyme in this assay. Consequently, these compounds may be expected to have utility in the inhibition of CTPS1. The compounds of the invention are also expected to have utility as research tools, for example, for use in CTPS assays.

The data for all compounds of formula (I) (including (I-a), (I-b) and (I-c) tested wherein $R_1$ is $R_{1a}$; and/or $R_4$ and $R_5$ are $R_{4a}$ and $R_{5a}$; and/or A is $A_a$ are presented below.

**Table 12:** *Human CTPS1 Enzyme Inhibition data grouped by potency range* (± indicates $IC_{50}$ in the range of >10 to 20 micromolar, + indicates $IC_{50}$ in the range >1 to 10 micromolar, ++ indicates $IC_{50}$ in the range >0.1 to 1 micromolar, +++ indicates $IC_{50}$ of ≤0.1 micromolar)

| P | CTPS1 | P | CTPS1 |
|---|---|---|---|
| P226 | +++ | P296 | +++ |
| P227 | +++ | P297 | +++ |
| P228 | +++ | P298 | +++ |
| P229 | +++ | P299 | +++ |
| P230 | + | P300 | +++ |
| P235 | ++ | P301 | +++ |
| P242 | +++ | P302 | +++ |
| P244 | +++ | P303 | ++ |
| P248 | ++ | P304 | +++ |
| P251 | +++ | P305 | ++ |
| P254 | ++ | P306 | +++ |
| P255 | ++ | P307 | +++ |
| P256 | ++ | P308 | +++ |
| P258 | +++ | P309 | ++ |
| P260 | +++ | P310 | +++ |
| P261 | ++ | P311 | ++ |
| P288 | +++ | P312 | +++ |
| P289 | +++ | P313 | +++ |

(continued)

| P | CTPS1 | P | CTPS1 |
|---|---|---|---|
| P290 | +++ | P314 | +++ |
| P291 | +++ | P315 | +++ |
| P292 | +++ | P316 | +++ |
| P293 | +++ | P317 | +++ |
| P294 | +++ | P318 | +++ |
| P295 | +++ | | |

**Table 13:** *Human CTPS1 Enzyme Inhibition data grouped by potency range (*+++ indicates $IC_{50}$ of ≤0.1 micromolar)

| R | CTPS1 |
|---|---|
| T466 | ± |

**Table 14:** *Human CTPS1 Enzyme Inhibition data grouped by potency range (*+++ indicates $IC_{50}$ of ≤0.1 micromolar)

| R | CTPS1 |
|---|---|
| R94 | +++ |

All compounds of the invention which have been tested were found to demonstrate inhibition of CTPS1 enzyme in this assay (see Tables 12 to 14). Consequently, these compounds may be expected to have utility in the inhibition of CTPS1.

In particular, activity is retained following reduction of the amide (group A) to the amine derivative (see Examples **R94** and **T466).**

**Biological Example 2 - RapidFire/MS-based Enzyme Selectivity Assays.**

*Human CTPS1 versus CTPS2 Selectivity Assessment by RapidFire/MS Analysis.*

The enzyme inhibitory activities against each target isoform of interest may be determined for the compounds of the invention using an optimised RapidFire high-throughput mass spectrometry (RF/MS) assay format. RF/MS assays for both human CTPS1 and CTPS2 may be performed in assay buffer consisting of 50mM HEPES (Merck), 20mM $MgCl_2$, 5mM KCl, 1mM DTT, 0.01% Tween-20, pH to 8.0 accordingly. Human full-length active C-terminal FLAG-His-tag CTPS1 (UniProtKB - P17812, CTPS1[1-591]-GGDYKDDDDKGGHHHHHHHH (CTPS1[1-591]-SEQ ID NO: 1)) may be obtained from Proteros biostructures GmbH. Human full length active C-terminal FLAG-His-Avi tagged CTPS2 (UniProtKB - Q9NRF8, CTPS2 [1-586]- DYKDDDDKHHHHHHGLNDIFEAQKIEWHE (CTPS2 [1-586]-SEQ ID NO: 2)) may be obtained from Harker Bio.

*Assay Procedure*

Human CTPS (1 or 2) protein may be prepared in 1x assay buffer to the final working protein concentration required for the reaction. A 2uL volume per well of 2x CTPS (1 or 2) protein may be mixed with 40nL of compound using acoustic (ECHO) delivery and incubated for 10 minutes at 25°C. Each isoform enzymatic reaction may be subsequently initiated by addition of 2uL per well of a 2x substrate mix in assay buffer. For hCTPS1: ATP (0.3mM), UTP (0.2mM), GTP (0.07mM) and L-glutamine (0.1mM). For hCTPS2: ATP (0.1mM), UTP (0.04mM), GTP (0.03mM) and L-glutamine (0.1mM). Each mixture may be incubated for an appropriate amount of time per isoform within the determined linear phase of the reaction at 25°C. A 60uL volume of stop solution (1% formic acid with 0.5uM $^{13}C_9$-$^{15}N_3$-CTP in $H_20$) may be added and the plate immediately heat-sealed and centrifuged for 10 minutes at 4,000rpm. Following centrifugation, plates may be loaded onto the Agilent RapidFire microfluidic solid phase extraction system coupled to an API4000 triple quadrupole mass spectrometer (RF/MS) for analysis.

In all cases, the enzyme converts UTP to CTP. Highly specific and sensitive multiple reaction monitoring (MRM) MS methods may be optimised for the detection of the enzymatic reaction product, CTP, and the stable isotope labelled product standard $^{13}C_9$-$^{15}N_3$-CTP. Readout for data analysis may be calculated as the ratio between the peak area of the product CTP and the internal standard $^{13}C_9$-$^{15}N_3$-CTP. For data reporting, the following equation may be used:

$$R = \frac{P}{IS}$$

(R = ratio/readout, P = product signal area, IS = internal standard signal area)

For each screening plate, the means of the negative (DMSO) and positive control values were used for the calculation of the respective assay window (S/B) and Z' values. The median of the respective control values was used for calculation of percent inhibition according to the following equation:

$$I = \frac{R_{neg} - R_{sample}}{[R_{neg} - R_{pos}]} \%$$

(I = Inhibition, $R_{neg}$ = median of negative control readout values, $R_{pos}$ = median of positive control readout values, $R_{sample}$ = sample readout value)

Percentage inhibition was then plotted against compound concentration, and the 50% inhibitory concentration ($IC_{50}$) was determined from the resultant concentration-response curve.

Fold selectivity between CTPS1 and CTPS2 was subsequently calculated according to the following equation:

$$\text{Fold selectivity} = \frac{\text{CTPS2 } IC_{50}}{\text{CTPS1 } IC_{50}}$$

The data for all compounds disclosed herein that were tested in Biological Example 2 are presented below.

**Table 15:** Selectivity data split into grouping of 2-30 fold (+), >30-60 fold (++) or >60 fold (+++)

| P | Selectivity |
|---|---|
| **Ref P1** | + |
| **Ref P2** | +++ |
| **Ref P9** | +++ |
| **Ref P12** | ++ |
| **Ref P16** | ++ |
| **Ref P18** | ++ |
| **Ref P21** | ++ |
| **Ref P31** | +++ |
| **Ref P34** | + |
| **Ref P38** | + |
| **Ref P39** | + |
| **Ref P59** | + |
| **Ref P65** | ++ |
| **Ref P68** | ++ |
| **Ref P70** | + |
| **Ref P74** | ++ |
| **Ref P76** | ++ |
| **Ref P83** | +++ |
| **Ref P87** | ++ |

(continued)

| P | Selectivity |
|---|---|
| **Ref P88** | +++ |
| **Ref P89** | +++ |
| **Ref P95** | + |
| **Ref P96** | + |
| **Ref P98** | +++ |
| **Ref P103** | + |
| **Ref P105** | ++ |
| **Ref P108** | +++ |
| **Ref P110** | ++ |
| **Ref P112** | ++ |
| **Ref P113** | + |
| **Ref P114** | +++ |
| **Ref P115** | +++ |
| **Ref P118** | +++ |
| **Ref P125** | ++ |
| **Ref P128** | + |
| **Ref P132** | ++ |
| **Ref P136** | +++ |
| **Ref P143** | +++ |
| **Ref P145** | +++ |
| **Ref P146** | +++ |
| **Ref P151** | +++ |
| **Ref P155** | + |
| **Ref P158** | + |
| **Ref P159** | +++ |
| **Ref P161** | + |
| **Ref P162** | ++ |
| **Ref P163** | +++ |
| **Ref P164** | + |
| **Ref P188** | ++ |
| **Ref P191** | ++ |
| **Ref P195** | +++ |
| **Ref P196** | +++ |
| **Ref P197** | +++ |
| **Ref P198** | +++ |
| **Ref P200** | ++ |
| **Ref P201** | +++ |
| **Ref P202** | +++ |
| **Ref P205a** | +++ |

(continued)

| P | Selectivity |
|---|---|
| **Ref P205b** | ++ |
| **Ref P206** | +++ |
| **Ref P207** | + |
| **Ref P216** | +++ |
| **Ref P221** | + |
| **Ref P222** | + |

**Table 16:** Selectivity data split into grouping of 2-30 fold (+), >30-60 fold (++) or >60 fold (+++)

| R | Selectivity | R | Selectivity |
|---|---|---|---|
| **Ref R5** | + | **Ref R69** | +++ |
| **Ref R6** | ++ | **Ref R70** | +++ |
| **Ref R7** | ++ | **Ref R71** | +++ |
| **Ref R11** | ++ | **Ref R73** | + |
| **Ref R19** | ++ | **Ref R74** | ++ |
| **Ref R23** | ++ | **Ref R75** | +++ |
| **Ref R25** | ++ | **Ref R76** | + |
| **Ref R26** | +++ | **Ref R78** | ++ |
| **Ref R41** | ++ | **Ref R79** | + |
| **Ref R42** | ++ | **Ref R80** | +++ |
| **Ref R43** | ++ | **Ref R82** | +++ |
| **Ref R45** | ++ | **Ref R83** | +++ |
| **Ref R46** | + | **Ref R84** | +++ |
| **Ref R47** | + | **Ref R86** | +++ |
| **Ref R48** | ++ | **Ref R87** | ++ |
| **Ref R51** | ++ | **Ref R88** | +++ |
| **Ref R52** | +++ | **Ref R89** | +++ |
| **Ref R54** | + | **Ref R90** | ++ |
| **Ref R55** | +++ | **Ref R91** | +++ |
| **Ref R56** | +++ | **Ref R92** | +++ |
| **Ref R62** | +++ | **Ref R93** | +++ |
| **Ref R63** | + | | |
| **Ref R64** | ++ | | |
| **Ref R68** | ++ | | |

The data for all compounds of formula (I) tested wherein $R_1$ is $R_{1a}$; and/or $R_4$ and $R_5$ are $R_{4a}$ and $R_{5a}$; and/or A is $A_a$ are presented in Table 17.

**Table 17:** Selectivity data split into grouping of 2-30 fold (+), >30-60 fold (++) or >60 fold (+++)

| P | Selectivity | P | Selectivity |
|---|---|---|---|
| **P226** | +++ | **P299** | +++ |

(continued)

| P | Selectivity | P | Selectivity |
|---|---|---|---|
| **P227** | +++ | **P300** | +++ |
| **P228** | +++ | **P301** | +++ |
| **P229** | +++ | **P302** | +++ |
| **P230** | + | **P303** | +++ |
| **P242** | +++ | **P305** | +++ |
| **P244** | +++ | **P306** | +++ |
| **P248** | +++ | **P307** | +++ |
| **P251** | +++ | **P308** | +++ |
| **P289** | +++ | **P309** | +++ |
| **P290** | +++ | **P312** | +++ |
| **P291** | +++ | **P313** | +++ |
| **P292** | +++ | **P314** | +++ |
| **P293** | +++ | **P315** | +++ |
| **P294** | +++ | **P316** | +++ |
| **P297** | +++ | **P317** | +++ |
| **P298** | +++ | **P318** | +++ |

All compounds tested in the assay described in Biological Assay 2 were found to have at least 2 fold selectivity for CTPS1 over CTPS2, with many compounds having a selectivity for CTPS1 of over 60 fold. In particular, these compounds may be expected to have utility in the treatment of diseases whereby a selective CTPS1 compound is beneficial.

The compounds of the invention are also expected to have utility as research tools, for example, for use in CTPS assays.

Throughout the specification and the claims and clauses which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

REFERENCES

Evans, D. R. & Guy, H. I. Mammalian pyrimidine biosynthesis: fresh insights into an ancient pathway. J. Biol. Chem. 279, 33035-33038 (2004).

Fairbanks, L. D. et al. Importance of ribonucleotide availability to proliferating T-lymphocytes from healthy humans. Disproportionate expansion of pyrimidine pools and contrasting effects of de novo synthesis inhibitors. J. Biol. Chem. 270, 29682-29689 (1995).

Higgins, M. J. et al. Regulation of human cytidine triphosphate synthetase 1 by glycogen synthase kinase 3. J. Biol. Chem. 282, 29493-29503 (2007).

Kursula, P. et al. Structure of the synthetase domain of human CTP synthetase, a target for anticancer therapy. Acta Crystallogr Sect F Struct Biol Cryst Commun. 62 (Pt7): 613-617 (2006).

Lieberman I. Enzymatic amination of uridine triphosphate to cytidine triphosphate. The J. Biol. Chem. 222 (2): 765-75 (1956).

Martin E. et al. CTP synthase 1 deficiency in humans reveals its central role in lymphocytes proliferation. Nature. Jun

12; 510(7504):288-92 (2014). Erratum in: Nature. Jul 17; 511(7509):370 (2014).

McCluskey GD et al., Exploring the Potent Inhibition of CTP Synthase by Gemcitabine-5'-Triphosphate. Chembiochem. 17, 2240-2249 (2016).

Ostrander, D. B. et al. Effect of CTP synthetase regulation by CTP on phospholipid synthesis in Saccharomyces cerevisiae. J. Biol. Chem. 273, 18992-19001 (1998).

Sakamoto K. et al. Identification of cytidine-5-triphosphate synthase1-selective inhibitory peptide from random peptide library displayed on T7 phage. Peptides. 2017; 94:56-63 (2017).

Salu et al. Drug-eluting stents: a new treatment in the prevention of restenosis Part I: experimental studies. Acta Cardiol, 59, 51-61 (2004).

Sousa J. E. et al. Drug-Eluting Stents. Circulation, 107 (2003) 2274 (Part I), 2283 (Part II).

Tang R. et al. CTP synthase 1, a smooth muscle-sensitive therapeutic target for effective vascular repair. Arterioscler Thromb Vasc Biol. 33(10), 1-19, (2013).

van den Berg, A. A. et al. Cytidine triphosphate (CTP) synthetase activity during cell cycle progression in normal and malignant T-lymphocytic cells. Eur. J. Cancer 31, 108-112 (1995).

van Kuilenburg, A.B.P. et al. Identification of a cDNA encoding an isoform of human CTP synthetase. Biochimica et Biophysica Acta 1492548-552 (2000).

## Claims

1. A compound of formula (I):

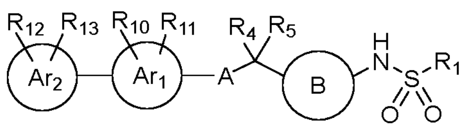

(I)

wherein ring B is selected from the group consisting of:

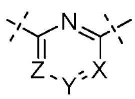

(B-a)

wherein X, Y and Z are as defined below; and

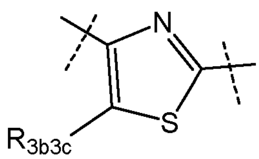

(B-bc);

wherein $R_{3b3c}$ is $R_{3b}$ or $R_{3c}$ as defined below;
wherein when B is (B-a) the compound of formula (I) is a compound of formula (I-a):

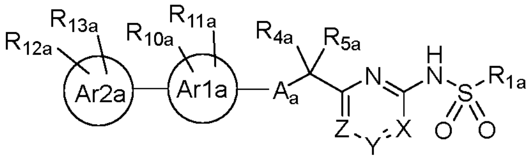

(I-a)

wherein:

$A_a$ is $A_{aa}$ or $A_{ba}$;
wherein:

$A_{aa}$ is an amine linker having the following structure: -NH-, $-CH_2NH-$ or $-NHCH_2-$;
$A_{ba}$ is an amide linker having the following structure: -C(=O)NH- or -NHC(=O)-;

X is N or CH;
Y is N or $CR_{2a}$;
Z is N or $CR_{3a}$;
with the proviso that when at least one of X or Z is N, Y cannot be N;
$R_{2a}$ is H, halo, $C_{1-2}$alkyl, $OC_{1-2}$alkyl, $C_{1-2}$haloalkyl or $OC_{1-2}$haloalkyl; and
$R_{3a}$ is H, halo, $CH_3$, $OCH_3$, $CF_3$ or $OCF_3$;
wherein at least one of $R_{2a}$ and $R_{3a}$ is H;
$R_{1a}$ is $R_{1aa}$ or $R_{1ba}$;
wherein:

$R_{1aa}$ is $NR_{32a}R_{33a}$;
$R_{1ba}$ is $C_{1-5}$alkyl, $C_{0-2}$alkylene$C_{3-5}$cycloalkyl which cycloalkyl is optionally substituted by $CH_3$, or $CF_3$;

$R_{4a}$ and $R_{5a}$ are $R_{4aa}$ and $R_{5aa}$, or $R_{4ba}$ and $R_{5ba}$;
wherein:

$R_{4aa}$ and $R_{5aa}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21a}R_{22a}$; or
one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or

$R_{4aa}$ and $R_{5aa}$ together with the carbon atom to which they are attached form a $C_{3-6}$heterocycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heterocycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or
$R_{4aa}$ and $R_{5aa}$ together with the carbon atom to which they are attached form a $C_{3-6}$heterocycloalkyl comprising one nitrogen atom, wherein said nitrogen atom is substituted by $-S(O)_2R_{29a}$; or
$R_{4ba}$ and $R_{5ba}$ are each independently H, $C_{1-6}$alkyl, $C_{1-6}$alkylOH, $C_{1-6}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, or $R_{4ba}$ and $R_{5ba}$ together with the carbon atom to which they are attached form a $C_{3-6}$cycloalkyl or $C_{3-6}$heterocycloalkyl; and
when $A_a$ is -NHC(=O)- or $-NHCH_2-$:
$R_{4ba}$ and $R_{5ba}$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl and $NR_{21a}R_{22a}$;

Ar1a is a 6-membered aryl or heteroaryl;
Ar2a is a 6-membered aryl or heteroaryl and is attached to Ar1a in the para position relative to group $A_a$;
$R_{10a}$ is H, halo, $C_{1-3}$alkyl, $C_{1-2}$haloalkyl, $OC_{1-2}$alkyl, $OC_{1-2}$haloalkyl or CN;
$R_{11a}$ is H, F, Cl, $C_{1-2}$alkyl, $CF_3$, $OCH_3$ or CN;
$R_{12a}$ is attached to Ar2 in the ortho or meta position relative to Ar1a and $R_{12a}$ is H, halo, $C_{1-4}$alkyl, $C_{2-4}$alkenyl, $C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, $NHC(O)C_{1-3}$alkyl or $NR_{23a}R_{24a}$; and

when $A_a$ is -NHC(=O)-, -NH- or -$NHCH_2$-:
$R_{12a}$ may additionally be selected from CN, $OCH_2CH_2N(CH_3)_2$ and a $C_{3-6}$heterocycloalkyl comprising one nitrogen located at the point of attachment to Ar2a, or $R_{12a}$ together with a nitrogen atom to which it is attached forms an N-oxide ($N^+$-$O^-$);

$R_{13a}$ is H or halo;
$R_{21a}$ is H, $C_{1-5}$alkyl, $C(O)C_{1-5}$alkyl, $C(O)OC_{1-5}$alkyl, $C_{1-3}$alkyl$OC_{1-2}$alkyl, $C_{1-4}$haloalkyl, or $C_{4-6}$heterocycloalkyl;
$R_{22a}$ is H or $CH_3$;
$R_{23a}$ is H or $C_{1-2}$alkyl; and
$R_{24a}$ is H or $C_{1-2}$alkyl
$R_{29a}$ is $C_{1-3}$alkyl, $C_{0-2}$alkylene$C_{3-5}$cycloalkyl which cycloalkyl is optionally substituted by $CH_3$, $CF_3$, $N(C_{1-3}alkyl)_2$, or a 5 or 6 membered heteroaryl wherein the 5 or 6 membered heteroaryl is optionally substituted by methyl;
$R_{32a}$ is $C_{1-3}$alkyl and $R_{33a}$ is $C_{1-3}$alkyl; or
$R_{32a}$ and $R_{33a}$ together with the nitrogen atom to which they are attached form a $C_{3-5}$heterocycloalkyl;

wherein

$R_{1a}$ is $R_{1aa}$; and/or
$R_{4a}$ and $R_{5a}$ are $R_{4aa}$ and $R_{6aa}$; and/or
$A_a$ is $A_{aa}$; and

wherein when B is (B-bc) and $R_{3b3c}$ is $R_{3b}$, the compound of formula (I) is a compound of formula (I-b):

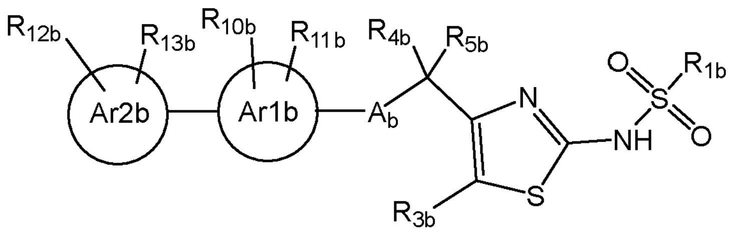

(I-b)

wherein:

$A_b$ is $A_{ab}$ or $A_{bb}$;
wherein:

$A_{ab}$ is -$NR_{6b}CH_2$- or -$NR_{6b}$-;
$A_{bb}$ is -$NR_{6b}C(=O)$-;

$R_{1b}$ is $R_{1ab}$ or $R_{1bb}$;
wherein:

$R_{1ab}$ is $NR_{32b}R_{33b}$;
$R_{1bb}$ is $C_{1-5}$alkyl, $C_{0-2}$alkylene$C_{3-5}$cycloalkyl which cycloalkyl is optionally substituted by $CH_3$, $C_{1-3}$alkylene$OC_{1-2}$alkyl, or $CF_3$;

$R_{3b}$ is H, halo, $CH_3$, $OC_{1-2}$alkyl or $CF_3$;

or $R_{3b}$ together with $R_{5bb}$ forms a 5- or 6-membered cycloalkyl or 5 or 6 membered oxygen-containing heterocycloalkyl;
$R_{4b}$ and $R_{5b}$ are either $R_{4ab}$ and $R_{5ab}$ or $R_{4bb}$ and $R_{5bb}$;
wherein:

$R_{4ab}$ and $R_{5ab}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and $NR_{21b}R_{22b}$; or
one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ab}$ and $R_{5ab}$ together with the carbon atom to which they are attached may be substituted by one or two substituents, each substituent being independently selected from the group consisting of $C_{1-3}$alkyl or O$C_{1-3}$alkyl; or

$R_{4ab}$ and $R_{5ab}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ab}$ and $R_{5ab}$ together with the carbon atom to which they are attached may be substituted by one or two substituents, each substituent being independently selected from the group consisting of $C_{1-3}$alkyl or O$C_{1-3}$alkyl; or
$R_{4ab}$ and $R_{5ab}$ together with the carbon atom to which they are attached form a $C_{3-6}$heterocycloalkyl comprising one nitrogen atom, wherein said nitrogen atom is substituted by $-S(O)_2R_{29b}$; or
$R_{4bb}$ and $R_{5bb}$ are each independently H, halo, $C_{1-6}$alkyl, $C_{0-2}$alkylene$C_{3-6}$cycloalkyl, $C_{0-2}$alkylene-$C_{3-6}$heterocycloalkyl, O$C_{1-6}$alkyl, O$C_{0-2}$alkylene$C_{3-6}$cycloalkyl, $C_{1-3}$alkyleneO$C_{1-3}$alkyl, $C_{1-6}$alkylOH, $C_{1-6}$haloalkyl, O$C_{1-6}$haloalkyl or $NR_{21b}R_{22b}$,

or $R_{4bb}$ is H and $R_{5bb}$ together with $R_{3b}$ form a 5- or 6-membered cycloalkyl or 5 or 6 membered oxygen-containing heterocycloalkyl,
or $R_{4bb}$ and $R_{5bb}$ together with the carbon atom to which they are attached form a $C_{3-6}$cycloalkyl or $C_{3-6}$heterocycloalkyl,
or $R_{4bb}$ is H and $R_{5bb}$ and $R_{6b}$ are a $C_{2-3}$alkylene chain forming a 5- or 6-membered ring;
or $R_{4bb}$ is O and $R_{5bb}$ is absent;

$R_{6b}$ is H or $C_{1-3}$alkyl,

or $R_{6b}$ together with $R_{11b}$ when in the ortho-position to group $A_b$ are a $C_2$alkylene chain forming a 5-membered ring,
or $R_{5bb}$ and $R_{6b}$ are a $C_{2-3}$alkylene chain forming a 5- or 6-membered ring and $R_{4bb}$ is H;

Ar1b is 6-membered aryl or heteroaryl;
Ar2b is a 6-membered aryl or heteroaryl and is attached to Ar1b in the para position relative to group $A_b$;
$R_{10b}$ is H, halo, $C_{1-3}$alkyl, O$C_{1-2}$alkyl, $C_{1-2}$haloalkyl, O$C_{1-2}$haloalkyl or CN;
$R_{11b}$ is H, F, Cl, $CH_3$, ethyl, $OCH_3$, $CF_3$, $OCF_3$ or CN,
or $R_{11b}$, when in the ortho-position to group $A_b$, together with $R_{6b}$ are a $C_2$alkylene chain forming a 5-membered ring;
$R_{12b}$ is attached to Ar2b in the ortho or meta position relative to Ar1b and $R_{12b}$ is H, halo, $C_{1-4}$alkyl, $C_{2-4}$alkynyl, $C_{0-2}$alkylene$C_{3-5}$cycloalkyl, O$C_{1-4}$alkyl, O$C_{0-2}$alkylene$C_{3-5}$cycloalkyl, $OCH_2CH_2N(CH_3)_2$, OH, $C_{1-4}$alkylOH, CN, $C_{1-3}$alkyleneO$C_{1-3}$alkyl, $C_{1-4}$haloalkyl, O$C_{1-4}$haloalkyl, C(=O)$C_{1-2}$alkyl, $NR_{23b}R_{24b}$, $SO_2C_{1-4}$alkyl, $SOC_{1-4}$alkyl, $SC_{1-4}$alkyl, SH, $C(O)N(CH_3)_2$, NHC(O)$C_{1-3}$alkyl, $C_{3-6}$heterocycloalkyl comprising one nitrogen located at the point of attachment to Ar2b, or $R_{12b}$ together with a nitrogen atom to which it is attached forms an N-oxide ($N^+-O^-$);
$R_{13b}$ is H, halo, $CH_3$ or $OCH_3$;
$R_{21b}$ is H, $C_{1-5}$alkyl, C(O)$C_{1-5}$alkyl, C(O)O$C_{1-5}$alkyl, $C_{1-3}$alkylO$C_{1-2}$alkyl, $C_{1-4}$haloalkyl, or $C_{4-6}$heterocycloalkyl;
$R_{22b}$ is H or $CH_3$;

$R_{23b}$ is H or $C_{1-2}$alkyl;
$R_{24b}$ is H or $C_{1-2}$alkyl;
$R_{29b}$ is $C_{1-3}$alkyl, $C_{0-2}$alkylene$C_{3-5}$cycloalkyl which cycloalkyl is optionally substituted by $CH_3$, $CF_3$, $N(C_{1-3}alkyl)_2$, or a 5 or 6 membered heteroaryl wherein the 5 or 6 membered heteroaryl is optionally substituted by methyl; and
$R_{32b}$ is $C_{1-3}$alkyl and $R_{33b}$ is $C_{1-3}$alkyl; or
$R_{32b}$ and $R_{33b}$ together with the nitrogen atom to which they are attached form a $C_{3-5}$heterocycloalkyl;

wherein:

$R_{1b}$ is $R_{1ab}$; and/or
$R_{4b}$ and $R_{5b}$ are $R_{4ab}$ and $R_{5ab}$; and/or
$A_b$ is $A_{ab}$; or

wherein when B is (B-bc) and $R_{3b3c}$ is $R_{3c}$, the compound of formula (I) is a compound of formula (I-c):

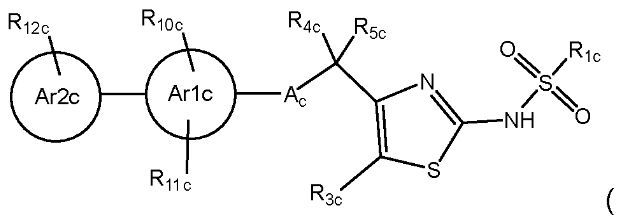
(I-c)

wherein:

$A_c$ is $A_{ac}$ or $A_{bc}$;
wherein:

$A_{ac}$ is $-CH_2NR_{6c}-$;
$A_{bc}$ is $-C(=O)NR_{6c}-$;

$R_{1c}$ is $R_{1ac}$ or $R_{1bc}$;
wherein:

$R_{1ac}$ is $NR_{32c}R_{33c}$;

$R_{1bc}$ is $C_{1-5}$alkyl, $C_{0-2}$alkylene$C_{3-5}$cycloalkyl which cycloalkyl is optionally substituted by $CH_3$, $C_{1-3}$alkyleneO$C_{1-2}$alkyl, or $CF_3$;
$R_{3c}$ is H, $CH_3$, halo, O$C_{1-2}$alkyl or $CF_3$;
$R_{4c}$ and $R_{5c}$ are either $R_{4ac}$ and $R_{5ac}$ or $R_{4bc}$ and $R_{5bc}$;
wherein:

$R_{4ac}$ and $R_{5ac}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and $NR_{21c}R_{22c}$; or
one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ac}$ and $R_{5ac}$ together with the carbon atom to which they are attached may be substituted by one or two substituents, each substituent being independently selected from the group consisting of $C_{1-3}$alkyl or O$C_{1-3}$alkyl; or

$R_{4ac}$ and $R_{5ac}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$hetero-

cycloalkyl ring, and wherein the $C_{3-6}$heteroycloalkyl formed by $R_{4ac}$ and $R_{5ac}$ together with the carbon atom to which they are attached may be substituted by one or two substituents, each substituent being independently selected from the group consisting of $C_{1-3}$alkyl or $OC_{1-3}$alkyl; or

$R_{4ac}$ and $R_{5ac}$ together with the carbon atom to which they are attached form a $C_{3-6}$heterocycloalkyl comprising one nitrogen atom, wherein said nitrogen atom is substituted by $-S(O)_2R_{29c}$; or

$R_{4bc}$ and $R_{5bc}$ are each independently H, $C_{1-6}$alkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, $C_{1-6}$alkylOH or $C_{1-6}$haloalkyl,

or $R_{4bc}$ and $R_{5bc}$ together with the carbon atom to which they are attached form a $C_{3-6}$cycloalkyl or $C_{3-6}$heterocycloalkyl ring;

$R_{6c}$ is H or $C_{1-3}$alkyl;

Ar1c is a 6-membered aryl or heteroaryl;

Ar2c is a 6-membered aryl or heteroaryl and is attached to Ar1c in the para position relative to group $A_c$;

$R_{10c}$ is H, halo, $C_{1-3}$alkyl, $OC_{1-2}$alkyl, $C_{1-2}$haloalkyl, $OC_{1-2}$haloalkyl or CN;

$R_{11c}$ is H, F, Cl, $CH_3$, ethyl, $OCH_3$, $CF_3$, $OCF_3$ or CN;

$R_{12c}$ is attached to Ar2c in the meta or ortho position relative to Ar1c and $R_{12c}$ is H, halo, $C_{1-4}$alkyl, $C_{2-4}$alkynyl, $C(=O)C_{1-2}$alkyl, $C_{0-2}$alkylene$C_{3-5}$cycloalkyl, $OC_{1-4}$alkyl, $C_{1-3}$alkylene$OC_{1-3}$alkyl, $C_{1-4}$haloalkyl, $OC_{1-4}$haloalkyl, CN, $OC_{0-2}$alkylene$C_{3-5}$cycloalkyl, $OCH_2CH_2N(CH_3)_2$, OH, $C_{1-4}$alkylOH, $NR_{23c}R_{24c}$, $SO_2CH_3$, $C(O)N(CH_3)_2$, $NHC(O)C_{1-3}$alkyl, or a $C_{3-6}$heterocycloalkyl comprising one nitrogen located at the point of attachment to Ar2c, or $R_{12c}$ together with a nitrogen atom to which it is attached forms an N-oxide ($N^+$-$O^-$);

$R_{21c}$ is H, $C_{1-5}$alkyl, $C(O)C_{1-5}$alkyl, $C(O)OC_{1-5}$alkyl, $C_{1-3}$alkyl$OC_{1-2}$alkyl, $C_{1-4}$haloalkyl, or $C_{4-6}$heterocycloalkyl;

$R_{22c}$ is H or $CH_3$;

$R_{23c}$ is H or $C_{1-2}$alkyl;

$R_{24c}$ is H or $C_{1-2}$alkyl;

$R_{29c}$ is $C_{1-3}$alkyl, $C_{0-2}$alkylene$C_{3-5}$cycloalkyl which cycloalkyl is optionally substituted by $CH_3$, $CF_3$, $N(C_{1-3}alkyl)_2$, or a 5 or 6 membered heteroaryl wherein the 5 or 6 membered heteroaryl is optionally substituted by methyl; and

$R_{32c}$ is $C_{1-3}$alkyl and $R_{33c}$ is $C_{1-3}$alkyl; or

$R_{32c}$ and $R_{33c}$ together with the nitrogen atom to which they are attached form a $C_{3-5}$heterocycloalkyl;

wherein:

$R_{1c}$ is $R_{1ac}$; and/or

$R_{4c}$ and $R_{5c}$ are $R_{4ac}$ and $R_{5ac}$; and/or

$A_c$ is $A_{ac}$;

or a salt and/or solvate thereof.

**2.** The compound, salt and/or solvate thereof according to claim 1 which is a compound of formula (I):

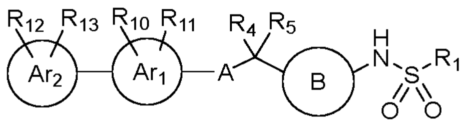

(I)

wherein ring B is selected from the group consisting of:

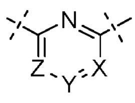

(B-a)

wherein X, Y and Z are as defined below; and:

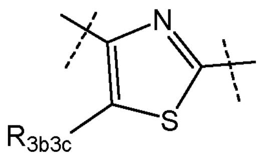

(B-bc);

wherein $R_{3b3c}$ is $R_{3b}$ or $R_{3c}$ as defined below;
wherein when B is (B-a) the compound of formula (I) is a compound of formula (I-a):

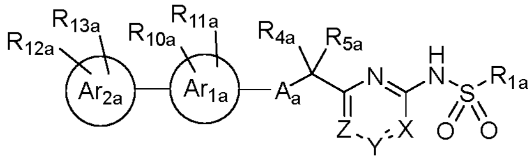

(I-a)

wherein:

$A_a$ is $A_{aa}$ or $A_{ba}$;
wherein:

$A_{aa}$ is an amine linker having the following structure: -NH-, $-CH_2NH-$ or $-NHCH_2-$;
$A_{ba}$ is an amide linker having the following structure: -C(=O)NH- or -NHC(=O)-;

X is N or CH;
Y is N or $CR_{2a}$;
Z is N or $CR_{3a}$;
with the proviso that when at least one of X or Z is N, Y cannot be N;
$R_{2a}$ is H, halo, $C_{1-2}$alkyl, $OC_{1-2}$alkyl, $C_{1-2}$haloalkyl or $OC_{1-2}$haloalkyl; and
$R_{3a}$ is H, halo, $CH_3$, $OCH_3$, $CF_3$ or $OCF_3$;
wherein at least one of $R_{2a}$ and $R_{3a}$ is H;
$R_{1a}$ is $R_{1aa}$ or $R_{1ba}$;
wherein:

$R_{1aa}$ is $NR_{32a}R_{33a}$;
$R_{1ba}$ is $C_{1-5}$alkyl, $C_{0-2}$alkylene$C_{3-5}$cycloalkyl which cycloalkyl is optionally substituted by $CH_3$, or $CF_3$;

$R_{4a}$ and $R_{5a}$ are $R_{4aa}$ and $R_{5aa}$, or $R_{4ba}$ and $R_{5ba}$;
wherein:

$R_{4aa}$ and $R_{5aa}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21a}R_{22a}$; or
one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or

$R_{4aa}$ and $R_{5aa}$ together with the carbon atom to which they are attached form a $C_{3-6}$heterocycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heterocycloalkyl formed by $R_{4aa}$ and $R_{5aa}$ together with the carbon atom to which they are attached may be substituted by one or two substituents, each substituent being independently selected from the group consisting of $C_{1-3}$alkyl or $OC_{1-3}$alkyl; or

$R_{4aa}$ and $R_{5aa}$ together with the carbon atom to which they are attached form a $C_{3-6}$heterocycloalkyl comprising one nitrogen atom, wherein said nitrogen atom is substituted by $-S(O)_2R_{29a}$; or
$R_{4ba}$ and $R_{5ba}$ are each independently H, $C_{1-6}$alkyl, $C_{1-6}$alkylOH, $C_{1-6}$haloalkyl, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, or $R_{4ba}$ and $R_{5ba}$ together with the carbon atom to which they are attached form a $C_{3-6}$cycloalkyl or $C_{3-6}$heterocycloalkyl; and
when $A_a$ is -NHC(=O)- or $-NHCH_2-$:
$R_{4ba}$ and $R_{5ba}$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl and $NR_{21a}R_{22a}$;

Ar1a is a 6-membered aryl or heteroaryl;
Ar2a is a 6-membered aryl or heteroaryl and is attached to Ar1a in the para position relative to group $A_a$;
$R_{10a}$ is H, halo, $C_{1-3}$alkyl, $C_{1-2}$haloalkyl, $OC_{1-2}$alkyl, $OC_{1-2}$haloalkyl or CN;
$R_{11a}$ is H, F, Cl, $C_{1-2}$alkyl, $CF_3$, $OCH_3$ or CN;
$R_{12a}$ is attached to Ar2 in the ortho or meta position relative to Ar1a and $R_{12a}$ is H, halo, $C_{1-4}$alkyl, $C_{2-4}$alkenyl, $C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, NHC(O)$C_{1-3}$alkyl or $NR_{23a}R_{24a}$; and
when $A_a$ is -NHC(=O)-, -NH- or $-NHCH_2-$:
$R_{12a}$ may additionally be selected from CN, $OCH_2CH_2N(CH_3)_2$ and a $C_{3-6}$heterocycloalkyl comprising one nitrogen located at the point of attachment to Ar2a, or $R_{12a}$ together with a nitrogen atom to which it is attached forms an N-oxide ($N^+$-$O^-$);
$R_{13a}$ is H or halo;
$R_{21a}$ is H, $C_{1-5}$alkyl, C(O)$C_{1-5}$alkyl, C(O)O$C_{1-5}$alkyl;
$R_{22a}$ is H or $CH_3$;
$R_{23a}$ is H or $C_{1-2}$alkyl; and
$R_{24a}$ is H or $C_{1-2}$alkyl
$R_{29a}$ is $C_{1-3}$alkyl, $C_{0-2}$alkylene$C_{3-5}$cycloalkyl which cycloalkyl is optionally substituted by $CH_3$, or $CF_3$;
$R_{32a}$ is $C_{1-3}$alkyl and $R_{33}$ is $C_{1-3}$alkyl; or
$R_{32a}$ and $R_{33a}$ together with the nitrogen atom to which they are attached form a $C_{3-5}$heterocycloalkyl;

wherein

$R_{1a}$ is $R_{1aa}$; and/or
$R_{4a}$ and $R_{5a}$ are $R_{4aa}$ and $R_{5aa}$; and/or
$A_a$ is $A_{aa}$; and

wherein when B is (B-bc) and $R_{3b3c}$ is $R_{3b}$, the compound of formula (I) is a compound of formula (I-b):

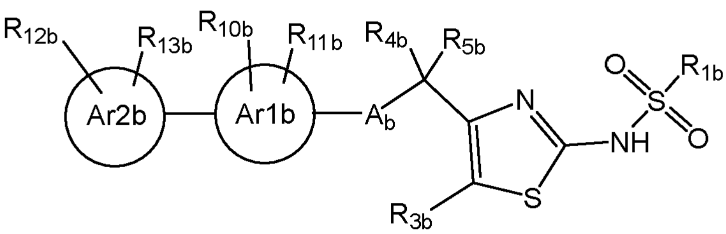

(I-b)

wherein:

$A_b$ is $A_{ab}$ or $A_{bb}$;
wherein:

$A_{ab}$ is $-NR_{6b}CH_2-$ or $-NR_{6b}-$;
$A_{bb}$ is $-NR_{6b}C(=O)-$;

$R_{1b}$ is $R_{1ab}$ or $R_{1bb}$;
wherein:

$R_{1ab}$ is $NR_{32b}R_{33b}$;

$R_{1bb}$ is $C_{1-5}$alkyl, $C_{0-2}$alkylene$C_{3-5}$cycloalkyl which cycloalkyl is optionally substituted by $CH_3$, $C_{1-3}$alkylene$OC_{1-2}$alkyl, or $CF_3$;
$R_{3b}$ is H, halo, $CH_3$, $OC_{1-2}$alkyl or $CF_3$;
or $R_{3b}$ together with $R_{5bb}$ forms a 5- or 6-membered cycloalkyl or 5 or 6 membered oxygen-containing heterocycloalkyl;
$R_{4b}$ and $R_{5b}$ are either $R_{4ab}$ and $R_{5ab}$ or $R_{4bb}$ and $R_{5bb}$;

wherein:

$R_{4ab}$ and $R_{5ab}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, halo, $OC_{1-3}$haloalkyl, $OC_{0-2}$alkylene$C_{3-6}$cycloalkyl, $OC_{0-2}$alkylene$C_{3-6}$heterocycloalkyl, $OC_{1-3}$alkyl and $NR_{21b}R_{22b}$; or
one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ab}$ and $R_{5ab}$ together with the carbon atom to which they are attached may be substituted by one or two substituents, each substituent being independently selected from the group consisting of $C_{1-3}$alkyl or $OC_{1-3}$alkyl; or

$R_{4ab}$ and $R_{5ab}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ab}$ and $R_{5ab}$ together with the carbon atom to which they are attached may be substituted by one or two substituents, each substituent being independently selected from the group consisting of $C_{1-3}$alkyl or $OC_{1-3}$alkyl; or
$R_{4ab}$ and $R_{5ab}$ together with the carbon atom to which they are attached form a $C_{3-6}$heterocycloalkyl comprising one nitrogen atom, wherein said nitrogen atom is substituted by $-S(O)_2R_{29b}$; or
$R_{4bb}$ and $R_{5bb}$ are each independently H, halo, $C_{1-6}$alkyl, $C_{0-2}$alkylene$C_{3-6}$cycloalkyl, $C_{0-2}$alkylene$C_{3-6}$heterocycloalkyl, $OC_{1-6}$alkyl, $OC_{0-2}$alkylene$C_{3-6}$cycloalkyl, $C_{1-3}$alkylene$OC_{1-3}$alkyl, $C_{1-6}$alkylOH, $C_{1-6}$haloalkyl, $OC_{1-6}$haloalkyl or $NR_{21b}R_{22b}$,

or $R_{4bb}$ is H and $R_{5bb}$ together with $R_{3b}$ form a 5- or 6-membered cycloalkyl or 5 or 6 membered oxygen-containing heterocycloalkyl,
or $R_{4bb}$ and $R_{5bb}$ together with the carbon atom to which they are attached form a $C_{3-6}$cycloalkyl or $C_{3-6}$heterocycloalkyl,
or $R_{4bb}$ is H and $R_{5bb}$ and $R_{6b}$ are a $C_{2-3}$alkylene chain forming a 5- or 6-membered ring;
or $R_{4bb}$ is O and $R_{5bb}$ is absent;

$R_{6b}$ is H or $C_{1-3}$alkyl,

or $R_{6b}$ together with $R_{11b}$ when in the ortho-position to group $A_b$ are a $C_2$alkylene chain forming a 5-membered ring,
or $R_{5bb}$ and $R_{6b}$ are a $C_{2-3}$alkylene chain forming a 5- or 6-membered ring and $R_{4bb}$ is H;

Ar1b is 6-membered aryl or heteroaryl;
Ar2b is a 6-membered aryl or heteroaryl and is attached to Ar1b in the para position relative to group $A_b$;
$R_{10b}$ is H, halo, $C_{1-3}$alkyl, $OC_{1-2}$alkyl, $C_{1-2}$haloalkyl, $OC_{1-2}$haloalkyl or CN;
$R_{11b}$ is H, F, Cl, $CH_3$, ethyl, $OCH_3$, $CF_3$, $OCF_3$ or CN,
or $R_{11b}$, when in the ortho-position to group $A_b$, together with $R_{6b}$ are a $C_2$alkylene chain forming a 5-membered ring;
$R_{12b}$ is attached to Ar2b in the ortho or meta position relative to Ar1b and $R_{12b}$ is H, halo, $C_{1-4}$alkyl, $C_{2-4}$alkynyl, $C_{0-2}$alkylene$C_{3-5}$cycloalkyl, $OC_{1-4}$alkyl, $OC_{0-2}$alkylene$C_{3-5}$cycloalkyl, $OCH_2CH_2N(CH_3)_2$, OH, $C_{1-4}$alkylOH, CN, $C_{1-3}$alkylene$OC_{1-3}$alkyl, $C_{1-4}$haloalkyl, $OC_{1-4}$haloalkyl, $C(=O)C_{1-2}$alkyl, $NR_{23b}R_{24b}$, $SO_2C_{1-4}$alkyl, $SOC_{1-4}$alkyl, $SC_{1-4}$alkyl, SH, $C(O)N(CH_3)_2$, $NHC(O)C_{1-3}$alkyl, $C_{3-6}$heterocycloalkyl comprising one nitrogen located at the point of attachment to Ar2b, or $R_{12b}$ together with a nitrogen atom to which it is attached forms an N-oxide ($N^+$-$O^-$);

$R_{13b}$ is H, halo, $CH_3$ or $OCH_3$;
$R_{21b}$ is H, $C_{1-5}$alkyl, C(O)$C_{1-5}$alkyl, C(O)O$C_{1-5}$alkyl;
$R_{22b}$ is H or $CH_3$;
$R_{23b}$ is H or $C_{1-2}$alkyl;
$R_{24b}$ is H or $C_{1-2}$alkyl;
$R_{29b}$ is $C_{1-3}$alkyl, $C_{0-2}$alkylene$C_{3-5}$cycloalkyl which cycloalkyl is optionally substituted by $CH_3$, or $CF_3$; and
$R_{32b}$ is $C_{1-3}$alkyl and $R_{33b}$ is $C_{1-3}$alkyl; or
$R_{32b}$ and $R_{33b}$ together with the nitrogen atom to which they are attached form a $C_{3-6}$heterocycloalkyl;

wherein:

$R_{1b}$ is $R_{1ab}$; and/or
$R_{4b}$ and $R_{5b}$ are $R_{4ab}$ and $R_{5ab}$; and/or
$A_b$ is $A_{ab}$; or

wherein when B is (B-bc) and $R_{3b3c}$ is $R_{3c}$, the compound of formula (I) is a compound of formula (I-c):

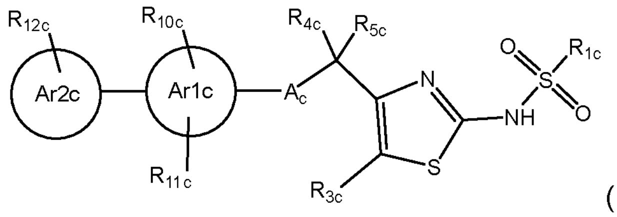

(I-c)

wherein:

$A_c$ is $A_{ac}$ or $A_{bc}$;

$A_{ac}$ is -$CH_2NR_{6c}$-;
$A_{bc}$ is -C(=O)$NR_{6c}$-;

$R_{1c}$ is $R_{1ac}$ or $R_{1bc}$;
wherein:
$R_{1ac}$ is $NR_{32c}R_{33c}$;
$R_{1bc}$ is $C_{1-5}$alkyl, $C_{0-2}$alkylene$C_{3-5}$cycloalkyl which cycloalkyl is optionally substituted by $CH_3$, $C_{1-3}$alkyleneO$C_{1-2}$alkyl, or $CF_3$;
$R_{3c}$ is H, $CH_3$, halo, O$C_{1-2}$alkyl or $CF_3$;
$R_{4c}$ and $R_{5c}$ are either $R_{4ac}$ and $R_{5ac}$ or $R_{4bc}$ and $R_{5bc}$;
wherein:

$R_{4ac}$ and $R_{5ac}$ together with the carbon atom to which they are attached form a $C_{3-6}$cycloalkyl which is:

substituted by one or two substituents, each substituent being independently selected from the group consisting of $C_{1-3}$alkyl, oxo, OH, $C_{1-3}$alkylOH, $C_{1-3}$haloalkyl, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, halo, O$C_{1-3}$haloalkyl, O$C_{0-2}$alkylene$C_{3-6}$cycloalkyl, O$C_{0-2}$alkylene$C_{3-6}$heterocycloalkyl, O$C_{1-3}$alkyl and $NR_{21c}R_{22c}$; or
one of the carbons of the $C_{3-6}$cycloalkyl is a spiro centre such that a spirocyclic ring system is formed by the $C_{3-6}$cycloalkyl ring and a further $C_{3-6}$cycloalkyl ring or a $C_{3-6}$heterocycloalkyl ring, and wherein the $C_{3-6}$cycloalkyl formed by $R_{4ac}$ and $R_{5ac}$ together with the carbon atom to which they are attached may be substituted by one or two substituents, each substituent being independently selected from the group consisting of $C_{1-3}$alkyl or O$C_{1-3}$alkyl; or

$R_{4ac}$ and $R_{5ac}$ together with the carbon atom to which they are attached form a $C_{3-6}$heteroycloalkyl wherein one of the carbons of the $C_{3-6}$heterocycloalkyl is a spiro centre such that a spirocyclic ring system is formed by the $C_{3-6}$heterocycloalkyl ring and a further $C_{3-6}$cycloalkyl ring or a $C_{3-6}$heterocycloalkyl ring, and wherein the $C_{3-6}$heteroycloalkyl formed by $R_{4ac}$ and $R_{5ac}$ together with the carbon atom to which they are attached may be substituted by one or two substituents, each substituent being

independently selected from the group consisting of $C_{1-3}$alkyl or $OC_{1-3}$alkyl; or
$R_{4ac}$ and $R_{5ac}$ together with the carbon atom to which they are attached form a $C_{3-6}$heterocycloalkyl comprising one nitrogen atom, wherein said nitrogen atom is substituted by $-S(O)_2R_{29c}$; or
$R_{4bc}$ and $R_{5bc}$ are each independently H, $C_{1-6}$alkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, $C_{1-6}$alkylOH or $C_{1-6}$haloalkyl,
or $R_{4bc}$ and $R_{5bc}$ together with the carbon atom to which they are attached form a $C_{3-6}$cycloalkyl or $C_{3-6}$heterocycloalkyl ring;

$R_{6c}$ is H or $C_{1-3}$alkyl;
Ar1c is a 6-membered aryl or heteroaryl;
Ar2c is a 6-membered aryl or heteroaryl and is attached to Ar1c in the para position relative to group $A_c$;
$R_{10c}$ is H, halo, $C_{1-3}$alkyl, $OC_{1-2}$alkyl, $C_{1-2}$haloalkyl, $OC_{1-2}$haloalkyl or CN;
$R_{11c}$ is H, F, Cl, $CH_3$, ethyl, $OCH_3$, $CF_3$, $OCF_3$ or CN;
$R_{12c}$ is attached to Ar2c in the meta or ortho position relative to Ar1c and $R_{12c}$ is H, halo, $C_{1-4}$alkyl, $C_{2-4}$alkynyl, $C(=O)C_{1-2}$alkyl, $C_{0-2}$alkylene$C_{3-5}$cycloalkyl, $OC_{1-4}$alkyl, $C_{1-3}$alkylene$OC_{1-3}$alkyl, $C_{1-4}$haloalkyl, $OC_{1-4}$haloalkyl, CN, $OC_{0-2}$alkylene$C_{3-5}$cycloalkyl, $OCH_2CH_2N(CH_3)_2$, OH, $C_{1-4}$alkylOH, $NR_{23c}R_{24c}$, $SO_2CH_3$, $C(O)N(CH_3)_2$, $NHC(O)C_{1-3}$alkyl, or a $C_{3-6}$heterocycloalkyl comprising one nitrogen located at the point of attachment to Ar2c, or $R_{12c}$ together with a nitrogen atom to which it is attached forms an N-oxide ($N^+$-$O^-$);
$R_{21c}$ is H, $C_{1-5}$alkyl, $C(O)C_{1-5}$alkyl, $C(O)OC_{1-5}$alkyl;
$R_{22c}$ is H or $CH_3$;
$R_{23c}$ is H or $C_{1-2}$alkyl;
$R_{24c}$ is H or $C_{1-2}$alkyl;
$R_{29c}$ is $C_{1-3}$alkyl, $C_{0-2}$alkylene$C_{3-5}$cycloalkyl which cycloalkyl is optionally substituted by $CH_3$, or $CF_3$; and
$R_{32c}$ is $C_{1-3}$alkyl and $R_{33c}$ is $C_{1-3}$alkyl; or
$R_{32c}$ and $R_{33c}$ together with the nitrogen atom to which they are attached form a $C_{3-5}$heterocycloalkyl;

wherein:

$R_{1c}$ is $R_{1ac}$; and/or
$R_{4c}$ and $R_{5c}$ are $R_{4ac}$ and $R_{5ac}$; and/or
$A_c$ is $A_{ac}$;

or a salt and/or solvate thereof.

**3.** The compound, salt and/or solvate thereof according to claim 1 or 2, which is the compound or a pharmaceutically acceptable salt and/or solvate thereof.

**4.** The pharmaceutically acceptable salt and solvate of a compound of formula (I) according to claim 3.

**5.** The pharmaceutically acceptable salt of a compound of formula (I) according to claim 3.

**6.** The pharmaceutically acceptable solvate of a compound of formula (I) according to claim 3.

**7.** The compound of formula (I) according to claim 1 or 2.

**8.** The compound, salt and/or solvate thereof according to any one of claims 1 to 7 wherein the compound of formula (I) is a compound of formula (I-a).

**9.** The compound, salt and/or solvate thereof according to any one of claims 1 to 7 wherein the compound of formula (I) is a compound of formula (I-b).

**10.** The compound, salt and/or solvate thereof according to any one of claims 1 to 7 wherein the compound of formula (I) is a compound of formula (I-c).

**11.** The compound, salt and/or solvate thereof according to any one of claims 8 to 10 wherein $R_{1a}$ is $R_{1aa}$, $R_{1b}$ is $R_{1ab}$ or $R_{1c}$ is $R_{1ac}$.

**12.** The compound, salt and/or solvate thereof according to any one of claims 8 to 11 wherein $R_{4a}$ and $R_{5a}$ are $R_{4aa}$ and

$R_{5aa}$, $R_{4b}$ and $R_{5b}$ are $R_{4ab}$ and $R_{5ab}$ or $R_{4c}$ and $R_{5c}$ are $R_{4ac}$ and $R_{5ac}$.

**13.** The compound, salt and/or solvate thereof according to any one of claims 8 to 12 wherein $A_a$ is $A_{aa}$, $A_b$ is $A_{ab}$ or $A_c$ is $A_{ac}$.

**14.** The compound, salt and/or solvate thereof according to claim 1 which is selected from the group consisting of:

1-(2-(cyclopropanesulfonamido)pyrimidin-4-yl)-*N*-(5-(6-ethoxypyrazin-2-yl)pyridin-2-yl)-4-oxocyclohexanecarboxamide;
1-(2-(cyclopropanesulfonamido)pyrimidin-4-yl)-*N*-(5-(6-ethoxypyrazin-2-yl)pyridin-2-yl)-4-hydroxycyclohexanecarboxamide;
1-(2-(cyclopropanesulfonamido)pyrimidin-4-yl)-*N*-(5-(6-ethoxypyrazin-2-yl)pyridin-2-yl)-4-hydroxycyclohexanecarboxamide (diastereomer 1);
1-(2-(cyclopropanesulfonamido)pyrimidin-4-yl)-*N*-(5-(6-ethoxypyrazin-2-yl)pyridin-2-yl)-4-hydroxycyclohexanecarboxamide (diastereomer 2);
1-(2-(cyclopropanesulfonamido)pyrimidin-4-yl)-4-(dimethylamino)-*N*-(5-(6-ethoxypyrazin-2-yl)pyridin-2-yl)cyclohexane-1-carboxamide;
1-(2-(cyclopropanesulfonamido)pyrimidin-4-yl)-4-(dimethylamino)-*N*-(5-(6-ethoxypyrazin-2-yl)pyridin-2-yl)cyclohexane-1-carboxamide (diastereomer 1);
1-(2-(cyclopropanesulfonamido)pyrimidin-4-yl)-4-(dimethylamino)-*N*-(5-(6-ethoxypyrazin-2-yl)pyridin-2-yl)cyclohexane-1-carboxamide (diastereomer 2);
*N*-(4-(1-((4-(6-Ethoxypyrazin-2-yl)-2-fluorobenzyl)amino)propyl)pyrimidin-2-yl)cyclopropanesulfonamide;
1-(2-(cyclopropanesulfonamido)pyrimidin-4-yl)-*N*-(5-(6-ethoxypyrazin-2-yl)pyridin-2-yl)-4,4-difluorocyclohexane-1-carboxamide;
8-(2-(cyclopropanesulfonamido)pyrimidin-4-yl)-*N*-(5-(6-ethoxypyrazin-2-yl)pyridin-2-yl)-1,4-dioxaspiro[4.5]decane-8-carboxamide;
4-(2-((*N,N*-dimethylsulfamoyl)amino)pyrimidin-4-yl)-*N*-(5-(6-ethoxypyrazin-2-yl)pyridin-2-yl)tetrahydro-2*H*-pyran-4-carboxamide;
4-(2-(cyclopropanesulfonamido)pyrimidin-4-yl)-*N*-(5-(6-ethoxypyrazin-2-yl)pyridin-2-yl)-1-(methylsulfonyl)piperidine-4-carboxamide;
N-(4-(1-(((5-(6-ethoxypyrazin-2-yl)pyridin-2-yl)methyl)amino)cyclopropyl)pyrimidin-2-yl)cyclopropanesulfonamide;
N-(4-(1-((4-(6-ethoxypyrazin-2-yl)-2-fluorobenzyl)amino)cyclopropyl)pyrimidin-2-yl)cyclopropanesulfonamide;
N-(4-(4-(((4-(6-ethoxypyrazin-2-yl)phenyl)amino)methyl)tetrahydro-2H-pyran-4-yl)pyrimidin-2-yl)cyclopropanesulfonamide;
2-(2-(cyclopropanesulfonamido)pyrimidin-4-yl)-*N*-(5-(6-ethoxypyrazin-2-yl)pyridin-2-yl)-5,8-dioxaspiro[3.4]octane-2-carboxamide;
1-(2-(cyclopropanesulfonamido)pyrimidin-4-yl)-N-(5-(6-ethoxypyrazin-2-yl)pyridin-2-yl)-4-methoxycyclohexane-1-carboxamide;
*N*-(4-(1-((4-(6-ethoxypyrazin-2-yl)phenyl)amino)propyl)pyrimidin-2-yl)cyclopropanesulfonamidearboxamide;
4-(2-(Cyclopropanesulfonamido)pyrimidin-4-yl)-*N*-(5-(6-ethoxypyrazin-2-yl)pyridin-2-yl)-1-(2-methoxyacetyl)piperidine-4-carboxamide;
4-(2-(Cyclopropanesulfonamido)pyrimidin-4-yl)-*N*-(5-(6-ethoxypyrazin-2-yl)pyridin-2-yl)-1-(ethylsulfonyl)piperidine-4-carboxamide;
4-(2-(Cyclopropanesulfonamido)pyrimidin-4-yl)-1-(cyclopropylsulfonyl)-*N*-(5-(6-ethoxypyrazin-2-yl)pyridin-2-yl)piperidine-4-carboxamide;
4-(2-(Cyclopropanesulfonamido)pyrimidin-4-yl)-1-(*N,N*-dimethylsulfamoyl)-*N*-(5-(6-ethoxypyrazin-2-yl)pyridin-2-yl)piperidine-4-carboxamide;
4-(2-(Cyclopropanesulfonamido)pyrimidin-4-yl)-*N*-(5-(6-ethoxypyrazin-2-yl)pyridin-2-yl)-1-((trifluoromethyl)sulfonyl)piperidine-4-carboxamide;
4-(2-(Cyclopropanesulfonamido)pyrimidin-4-yl)-*N*-(5-(6-ethoxypyrazin-2-yl)pyridin-2-yl)-1-((1-methyl-1*H*-pyrazol-3-yl)sulfonyl)piperidine-4-carboxamide;
1-(cyanomethyl)-4-(2-(cyclopropanesulfonamido)pyrimidin-4-yl)-N-(5-(6-ethoxypyrazin-2-yl)pyridin-2-yl)piperidine-4-carboxamide;
ethyl 2-(4-(2-(cyclopropanesulfonamido)pyrimidin-4-yl)-4-((5-(6-ethoxypyrazin-2-yl)pyridin-2-yl)carbamoyl)piperidin-1-yl)acetate;
*N*-(5-(6-ethoxypyrazin-2-yl)pyridin-2-yl)-4-(2-(ethylsulfonamido)pyrimidin-4-yl)-1-(2-methoxyacetyl)piperidine-4-carboxamide;

*N*-(5-(6-ethoxypyrazin-2-yl)pyridin-2-yl)-4-(2-(ethylsulfonamido)pyrimidin-4-yl)-1-(methylsulfonyl)piperidine-4-carboxamide;
*N*-(5-(6-ethoxypyrazin-2-yl)pyridin-2-yl)-4-(2-(ethylsulfonamido)pyrimidin-4-yl)-1-(ethylsulfonyl)piperidine-4-carboxamide;
1-(Cyclopropylsulfonyl)-*N*-(5-(6-ethoxypyrazin-2-yl)pyridin-2-yl)-4-(2-(ethylsulfonamido)pyrimidin-4-yl)piperidine-4-carboxamide;
*N*-(5-(6-ethoxypyrazin-2-yl)pyridin-2-yl)-4-(2-(ethylsulfonamido)pyrimidin-4-yl)-1-((1-methyl-1*H*-pyrazol-3-yl)sulfonyl)piperidine-4-carboxamide;
1-(2-(Cyclopropanesulfonamido)pyrimidin-4-yl)-N-(5-(6-cyclopropylpyrazin-2-yl)pyridin-2-yl)-4-methoxycyclohexane-1-carboxamide (diastereomer 1);
1-(2-(Cyclopropanesulfonamido)pyrimidin-4-yl)-N-(5-(6-cyclopropylpyrazin-2-yl)pyridin-2-yl)-4-methoxycyclohexane-1-carboxamide (diastereomer 2);
1-(2-(Cyclopropanesulfonamido)pyrimidin-4-yl)-*N*-(5-(6-ethoxypyrazin-2-yl)pyridin-2-yl)-4-(pyrrolidin-1-yl)cyclohexane-1-carboxamide (diastereomer 1);
1-(2-(Cyclopropanesulfonamido)pyrimidin-4-yl)-*N*-(5-(6-ethoxypyrazin-2-yl)pyridin-2-yl)-4-(pyrrolidin-1-yl)cyclohexane-1-carboxamide (diastereomer 2);
4-amino-1-(2-(cyclopropanesulfonamido)pyrimidin-4-yl)-N-(5-(6-ethoxypyrazin-2-yl)pyridin-2-yl)cyclohexane-1-carboxamide (diastereomer 1);
1-(2-(cyclopropanesulfonamido)pyrimidin-4-yl)-N-(5-(6-ethoxypyrazin-2-yl)pyridin-2-yl)-4-morpholinocyclohexane-1-carboxamide (diastereomer 1);
1-(2-(cyclopropanesulfonamido)pyrimidin-4-yl)-N-(5-(6-ethoxypyrazin-2-yl)pyridin-2-yl)-4-morpholinocyclohexane-1-carboxamide (diastereomer 2);
1-(2-(cyclopropanesulfonamido)pyrimidin-4-yl)-N-(5-(6-ethoxypyrazin-2-yl)pyridin-2-yl)-4-(methyl(oxetan-3-yl)amino)cyclohexane-1-carboxamide (diastereomer 1);
1-(2-(cyclopropanesulfonamido)pyrimidin-4-yl)-N-(5-(6-ethoxypyrazin-2-yl)pyridin-2-yl)-4-((2-methoxyethyl)(methyl)amino)cyclohexane-1-carboxamide (diastereomer 1);
1-(2-(cyclopropanesulfonamido)pyrimidin-4-yl)-N-(5-(6-ethoxypyrazin-2-yl)pyridin-2-yl)-4-((2-methoxyethyl)(methyl)amino)cyclohexane-1-carboxamide (diastereomer 2);
1-(2-(cyclopropanesulfonamido)pyrimidin-4-yl)-4-((2,2-difluoroethyl)(methyl)amino)-N-(5-(6-ethoxypyrazin-2-yl)pyridin-2-yl)cyclohexane-1-carboxamide (diastereomer 1);
1-(2-(cyclopropanesulfonamido)pyrimidin-4-yl)-N-(5-(6-ethoxypyrazin-2-yl)pyridin-2-yl)-4-(4-methylpiperazin-1-yl)cyclohexane-1-carboxamide (diastereomer 1);
1-(2-(cyclopropanesulfonamido)pyrimidin-4-yl)-N-(5-(6-ethoxypyrazin-2-yl)pyridin-2-yl)-4-(4-methylpiperazin-1-yl)cyclohexane-1-carboxamide (diastereomer 2);
4-(6-(cyclopropanesulfonamido)pyrazin-2-yl)-N-(5-(6-ethoxypyrazin-2-yl)pyridin-2-yl)-1-(methylsulfonyl)piperidine-4-carboxamide;
4-(4-(cyclopropanesulfonamido)pyrimidin-2-yl)-N-(5-(6-cyclopropylpyrazin-2-yl)pyridin-2-yl)-1-(methylsulfonyl)piperidine-4-carboxamide;
4-(4-(cyclopropanesulfonamido)pyrimidin-2-yl)-N-(5-(6-ethoxypyrazin-2-yl)pyridin-2-yl)-1-(methylsulfonyl)piperidine-4-carboxamide;
4-(4-(cyclopropanesulfonamido)pyrimidin-2-yl)-N-(5-(6-cyclopropylpyrazin-2-yl)pyridin-2-yl)-1-(ethylsulfonyl)piperidine-4-carboxamide;
4-(4-(cyclopropanesulfonamido)pyrimidin-2-yl)-N-(5-(6-ethoxypyrazin-2-yl)pyridin-2-yl)-1-(ethylsulfonyl)piperidine-4-carboxamide;
N-(4-(1-((2-fluoro-4-(pyridin-3-yl)phenyl)amino)-2-methylpropan-2-yl)thiazol-2-yl)cyclopropanesulfonamide;
*N*-(4-(2-((4-(6-Ethoxypyrazin-2-yl)-2-fluorobenzyl)amino)propan-2-yl)thiazol-2-yl)cyclopropanesulfonamide;
*N*-(4-(2-(((5-(6-ethoxypyrazin-2-yl)pyridin-2-yl)methyl)amino)propan-2-yl)thiazol-2-yl)cyclopropanesulfonamide;
and pharmaceutically acceptable salts and/or solvates of any one thereof.

**15.** A pharmaceutical composition comprising a compound, pharmaceutically acceptable salt and/or solvate thereof according to any one of claims 3 to 14.

**16.** The compound, pharmaceutically acceptable salt and/or solvate thereof according to any one of claims 3 to 14 or a pharmaceutical composition according to claim 15 for use as a medicament.

**17.** The compound pharmaceutically acceptable salt and/or solvate thereof according to any one of claims 3 to 14 or a

pharmaceutical composition according to claim 15, for use in the treatment of a haematological cancer.

**18.** The compound pharmaceutically acceptable salt and/or solvate thereof according to any one of claims 3 to 14 or a pharmaceutical composition according to claim 15, for use in the treatment of a non-haematological cancer.

**19.** A compound which is selected from the group consisting of:

- a compound of formula **(II-a):**

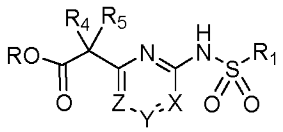

**(II-a)**

wherein X, Y and Z are as defined in claim 1, R is H, $C_{1-6}$alkyl (e.g. methyl and ethyl) or benzyl, $R_1$ is $R_{1aa}$ and/or $R_4$ and $R_5$ are $R_{4aa}$ and $R_{5aa}$; and $R_{1aa}$, $R_{4aa}$ and $R_{5aa}$ are as defined in claim 1;
and salts thereof;

- a compound of formula **(XX-a)**:

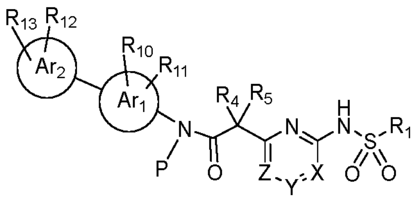

**(XX-a)**

wherein X, Y, Z, $R_{10}$, $R_{11}$, $R_{12}$, $R_{13}$, $Ar_1$ and $Ar_2$ are as defined in claim 1, P is a nitrogen protecting group such as para-methoxybenzyl, $R_1$ is $R_{1aa}$ and/or $R_4$ and $R_5$ are $R_{4aa}$ and $R_{5aa}$; and $R_{1aa}$, $R_{4aa}$ and $R_{5aa}$ are as defined in claim 1;
and salts thereof;

- a compound of formula **(XXIV-a):**

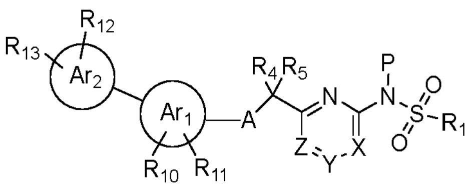

**(XXIV-a)**

wherein X, Y, Z, $R_{10}$, $R_{11}$, $R_{12}$, $R_{13}$, A, $Ar_1$ and $Ar_2$ are as defined in claim 1, P is a nitrogen protecting group such as para-methoxybenzyl, $R_1$ is $R_{1aa}$ and/or $R_4$ and $R_5$ are $R_{4aa}$ and $R_{5aa}$; and $R_{1aa}$, $R_{4aa}$ and $R_{5aa}$ are as defined in claim 1;
and salts thereof;

- a compound of formula **(XXXI-a):**

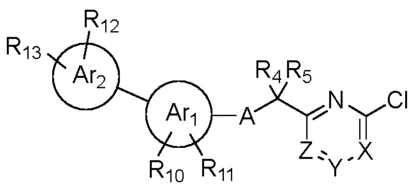

**(XXXI-a)**;

wherein X, Y, Z, $R_{10}$, $R_{11}$, $R_{12}$, $R_{13}$, $Ar_1$ and $A_2$ are as defined in claim 1, A is $A_a$ and/or $R_4$ and $R_5$ are $R_{4aa}$ and $R_{5aa}$; and $R_{4aa}$ and $R_{5aa}$ are as defined in claim 1; and $R_{4aa}$ and $R_{5aa}$ are as defined in claim 1;
and salts thereof;

- a compound of formula **(XXXXII-a):**

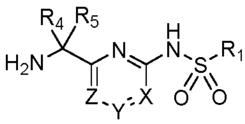

**(XXXXII-a)**;

wherein X, Y and Z are as defined in claim 1, $R_1$ is $R_{1aa}$ and/or $R_4$ and $R_5$ are $R_{4aa}$ and $R_{5aa}$; and $R_{1aa}$, $R_{4aa}$ and $R_{5aa}$ are as defined in claim 1;
and salts thereof;

- a compound of formula **(LI-a):**

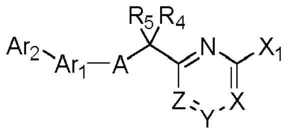

**(LI-a)**;

wherein X, Y, Z, $Ar_1$ and $Ar_2$ are as defined in claim 1, $X_1$ is Cl or Br, A is $A_{aa}$ and/or $R_4$ and $R_5$ are $R_{4aa}$ and $R_{5aa}$. and $A_{aa}$, $R_{4aa}$ and $R_{5aa}$ are as defined in claim 1;
and salts thereof;

- a compound of formula **(LXXIII-a):**

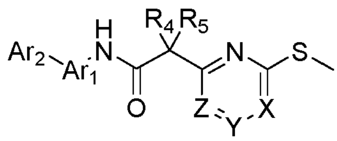

**(LXXIII-a)** ;

wherein X, Y, Z, $A_1$, $Ar_2$ are as defined in claim 1, and $R_4$ and $R_5$ are $R_{4aa}$ and $R_{5aa}$; and $R_{4aa}$ and $R_{5aa}$ are as defined in claim 1;
and salts thereof;

- a compound of formula **(LXXIV-a):**

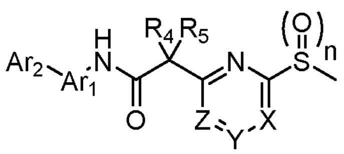

**(LXXIV-a)** n = 1 sulfoxide
n = 2 sulfone ;

wherein X, Y, Z, $Ar_1$ and $Ar_2$ are as defined in claim 1, and $R_4$ and $R_5$ are $R_{4aa}$ and $R_{5aa}$; and $R_{4aa}$ and $R_{5aa}$ are as defined in claim 1;
and salts thereof;

- a compound of formula **(LXXXIII-a):**

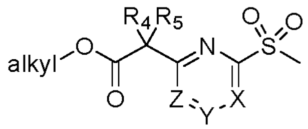

**(LXXXIII-a)** ;

wherein X, Y and Z are as defined in claim 1, alkyl is $C_{1-4}$alkyl such as methyl or ethyl, e.g. methyl and $R_4$ and $R_5$ are $R_{4aa}$ and $R_{5aa}$; and $R_{4aa}$ and $R_{5aa}$ are as defined in claim 1;
and salts thereof;

- a compound of formula **(XXXIV-a):**

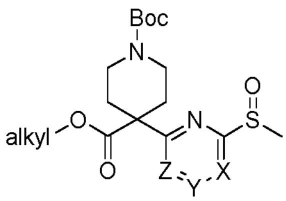

**(XXXIV-a)**;

wherein X, Y and Z are as defined in claim 1, and alkyl is $C_{1-4}$alkyl such as methyl or ethyl, e.g. methyl;
and salts thereof;

- a compound of formula **(LVIII-a):**

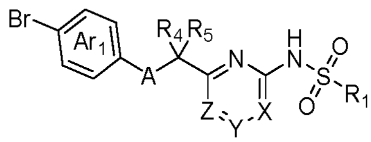

**(LVIII-a)**;

wherein X, Y, Z, and $Ar_1$ are as defined in claim 1, A is $A_a$ and/or $R_1$ is $R_{1aa}$ and/or $R_4$ and $R_5$ are $R_{4aa}$ and $R_{5aa}$; and $R_{1aa}$, $R_{4aa}$ and $R_{5aa}$ are as defined in claim 1;
and salts thereof;

- a compound of formula **(XXXIII-a):**

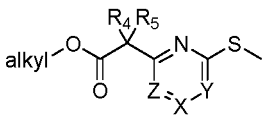

**(XXXIII-a)**;

wherein X, Y and Z are as defined in claim 1, alkyl is $C_{1-4}$alkyl such as methyl or ethyl, e.g. methyl and $R_4$ and $R_5$ are $R_{4aa}$ and $R_{5aa}$; and $R_{4aa}$ and $R_{5aa}$ are as defined in claim 1;
and salts thereof;

- a compound of formula **(II-b)**

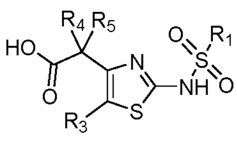

,

wherein $R_3$ is as defined in claim 1, $R_1$ is $R_{1ab}$ and/or $R_4$ and $R_5$ are $R_{4ab}$ and $R_{5ab}$; and $R_{1ab}$, $R_{4ab}$ and $R_{5ab}$ are as defined in claim 1;

and salts thereof;

- a compound of formula **(IV-b)**

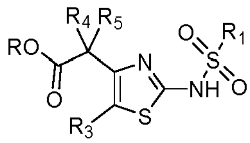

wherein $R_3$ is as defined in claim 1, R is $C_{1-6}$alkyl (e.g. methyl, ethyl) or benzyl, $R_1$ is $R_{1ab}$ and/or $R_4$ and $R_5$ are $R_{4ab}$ and $R_{6ab}$; and $R_{1ab}$, $R_{4ab}$ and $R_{5ab}$ are as defined in claim 1;
and salts thereof;

- a compound of formula **(II-c):**

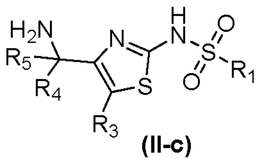

**(II-c)** ;

wherein $R_3$ is as defined in claim 1, $R_1$ is $R_{1ac}$ and/or $R_4$ and $R_5$ are $R_{4ac}$ and $R_{5ac}$; and $R_{1ac}$, $R_{4ac}$ and $R_{5ac}$ are as defined in claim 1;
and salts thereof;

- a compound of formula (VIII-c):

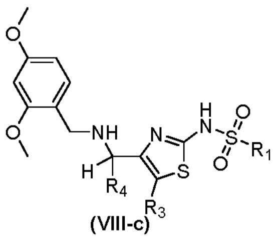

**(VIII-c)** ;

wherein $R_3$ and $R_4$ are as defined in claim 1 and $R_1$ is $R_{1ac}$; and $R_{1ac}$ are as defined in claim 1; and salts thereof.

**Patentansprüche**

1. Verbindung der Formel (I):

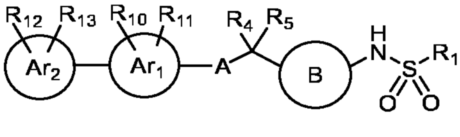

(I)

wobei Ring B ausgewählt ist aus der Gruppe bestehend aus:

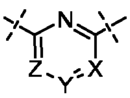

(B-a)

wobei X, Y und Z wie unten definiert sind; und

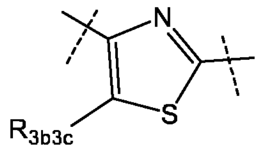

(B-bc);

wobei $R_{3b3c}$ $R_{3b}$ oder $R_{3c}$ ist, wie unten definiert;

wobei, wenn B (B-a) ist, die Verbindung der Formel (I) eine Verbindung der Formel (I-a) ist:

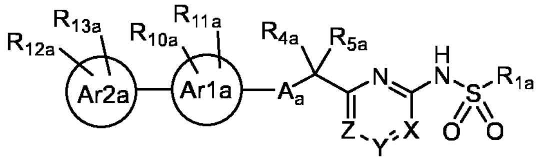

(I-a)

wobei:

$A_a$ $A_{aa}$ oder $A_{ba}$ ist;
wobei:

$A_{aa}$ ein Amin-Linker mit folgender Struktur ist: -NH-, $-CH_2NH-$ oder $-NHCH_2-$;
$A_{ba}$ ein Amid-Linker mit folgender Struktur ist: - C(=O)NH- oder -NHC(=O)-;

X N oder CH ist;
Y N oder $CR_{2a}$ ist;
Z N oder $CR_{3a}$ ist;
mit der Maßgabe, dass, wenn mindestens eines von X oder Z N ist, Y nicht N sein kann;
$R_{2a}$ H, Halogen, $C_{1-2}$-Alkyl, $OC_{1-2}$-Alkyl, $C_{1-2}$-Halogenalkyl oder $OC_{1-2}$-Halogenalkyl ist; und
$R_{3a}$ H, Halogen, $CH_3$, $OCH_3$, $CF_3$ oder $OCF_3$ ist;
wobei mindestens eines von $R_{2a}$ und $R_{3a}$ H ist;
$R_{1a}$ $R_{1aa}$ oder $R_{1ba}$ ist;
wobei:
$R_{1aa}$ $NR_{32a}R_{33a}$ ist;
$R_{1ba}$ $C_{1-5}$-Alkyl, $C_{0-2}$-Alkylen$C_{3-5}$-Cycloalkyl ist, wobei das Cycloalkyl gegebenenfalls mit $CH_3$ oder $CF_3$ substituiert ist;
$R_{4a}$ und $R_{5a}$ $R_{4aa}$ und $R_{5aa}$, oder $R_{4ba}$ und $R_{5ba}$ sind;
wobei:
$R_{4aa}$ und $R_{5aa}$ zusammen mit dem Kohlenstoffatom, an das sie gebunden sind, ein $C_{3-6}$-Cycloalkyl bilden, das:

durch einen oder zwei Substituenten substituiert ist, wobei jeder Substituent unabhängig ausgewählt ist aus der Gruppe bestehend aus $C_{1-3}$-Alkyl, Oxo, OH, $C_{1-3}$-AlkylOH, $C_{1-3}$-Halogenalkyl, $C_{0-2}$-Alkylen-$C_{3-6}$-Cycloalkyl, $C_{0-2}$-Alkylen-$C_{3-6}$-Heterocycloalkyl, $C_{1-3}$-Alkylen-$OC_{1-3}$-Alkyl, Halogen, $OC_{1-3}$-Halogenalkyl, $OC_{0-2}$Alkylen-$C_{3-6}$-Cycloalkyl, $OC_{0-2}$-Alkylen-$C_{3-6}$-Heterocycloalkyl, $OC_{1-3}$-Alkyl und $NR_{21a}$-$R_{22a}$; oder
eines der Kohlenstoffatome des $C_{3-6}$-Cycloalkyls ein Spirozentrum ist, sodass ein spirocyclisches Ringsystem durch den $C_{3-6}$-Cycloalkylring und einen weiteren $C_{3-6}$-Cycloalkylring oder einen $C_{3-6}$-Heterocycloalkylring gebildet wird, und wobei das durch $R_{4aa}$ und $R_{5aa}$ gebildete $C_{3-6}$-Cycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder
$R_{4aa}$ und $R_{5aa}$ zusammen mit dem Kohlenstoffatom, an das sie gebunden sind, ein $C_{3-6}$-Heterocycloalkyl bilden, wobei eines der Kohlenstoffatome des $C_{3-6}$-Heterocycloalkyls ein Spirozentrum ist, sodass ein spirocyclisches Ringsystem durch den $C_{3-6}$-Heterocycloalkylring und einen weiteren $C_{3-6}$-Cycloalkylring oder einen $C_{3-6}$-Heterocycloalkylring gebildet wird, und wobei das durch $R_{4aa}$ und $R_{5aa}$ gebildete $C_{3-6}$-Heterocycloalkyl zusammen mit dem Kohlenstoffatom, an das sie gebunden

sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder

$R_{4aa}$ und $R_{5aa}$ bilden zusammen mit dem Kohlenstoffatom, an das sie gebunden sind, ein $C_{3-6}$-Heterocycloalkyl, das ein Stickstoffatom umfasst, wobei dieses Stickstoffatom durch $-S(O)_2R_{29a}$ substituiert ist; oder

$R_{4ba}$ und $R_{5ba}$ jeweils unabhängig H, $C_{1-6}$-Alkyl, $C_{1-6}$-Alkyl-OH, $C_{1-6}$-Halogenalkyl, $C_{0-2}$-AlkylenC$_{3-6}$-Cycloalkyl, $C_{0-2}$-AlkylenC$_{3-6}$-Heterocycloalkyl, $C_{1-3}$-AlkylenOC$_{1-3}$-Alkyl sind oder $R_{4ba}$ und $R_{5ba}$ zusammen mit dem Kohlenstoffatom, an das sie gebunden sind, ein $C_{3-6}$-Cycloalkyl oder $C_{3-6}$-Heterocycloalkyl bilden; und

wenn $A_a$ -NHC(=O)- oder $-NHCH_2-$ ist:

$R_{4ba}$ und $R_{5ba}$ zusätzlich aus Halogen, $OC_{1-6}$-Halogenalkyl, $OC_{0-2}$-AlkylenC$_{3-6}$-Cycloalkyl, $OC_{0-2}$-AlkylenC$_{3-6}$-Heterocycloalkyl, $OC_{1-6}$-Alkyl and $NR_{21a}R_{22a}$ ausgewählt sein können;

Ar1a ein 6-gliedriges Aryl oder Heteroaryl ist;

Ar2a ein 6-gliedriges Aryl oder Heteroaryl ist und an Ar1a in para-Stellung zu der Gruppe $A_a$ gebunden ist;

$R_{10a}$ H, Halogen, $C_{1-3}$-Alkyl, $C_{1-2}$-Halogenalkyl, $OC_{1-2}$-Alkyl, $OC_{1-2}$-Halogenalkyl oder CN ist;

$R_{11a}$ H, F, Cl, $C_{1-2}$-Alkyl, $CF_3$, $OCH_3$ oder CN ist;

$R_{12a}$ an Ar2 in ortho- oder meta-Stellung relativ zu Ar1a gebunden ist und $R_{12a}$ H, Halogen, $C_{1-4}$-Alkyl, $C_{2-4}$-Alkenyl, $C_{0-2}$-Alkylen-$C_{3-5}$-Cycloalkyl, $OC_{1-4}$-Alkyl, $OC_{0-2}$-Alkylen-$C_{3-5}$-Cycloalkyl, $C_{1-4}$-Halogenalkyl, $OC_{1-4}$-Halogenalkyl, Hydroxy, $C_{1-4}$-AlkylOH, $SO_2C_{1-2}$-Alkyl, C(O)N($C_{1-2}$-Alkyl)$_2$, NHC(O)$C_{1-3}$-Alkyl oder $NR_{23a}R_{24a}$ ist; und

wenn $A_a$ -NHC(=O)- oder $-NHCH_2-$ ist:

$R_{12a}$ zusätzlich aus CN, $OCH_2CH_2N(CH_3)_2$ und einem $C_{3-6}$-Heterocycloalkyl, das ein Stickstoffatom umfasst, das sich an der Bindungsstelle an Ar2a befindet, ausgewählt sein kann, oder $R_{12a}$ zusammen mit dem Stickstoffatom, an das es gebunden ist, ein N-Oxid ($N^+$-$O^-$) bildet;

$R_{13a}$ H oder Halogen ist;

$R_{21a}$ H, $C_{1-5}$-Alkyl, C(O)$C_{1-5}$-Alkyl, C(O)O$C_{1-5}$-Alkyl, $C_{1-3}$-AlkylO$C_{1-2}$-Alkyl, $C_{1-4}$-Halogenalkyl, oder $C_{4-6}$-Heterocycloalkyl ist;

$R_{22a}$ H oder $CH_3$ ist;

$R_{23a}$ H oder $C_{1-2}$-Alkyl ist; und

$R_{24a}$ H oder $C_{1-2}$-Alkyl ist

$R_{29a}$ $C_{1-3}$-Alkyl, $C_{0-2}$-Alkylen-$C_{3-5}$-Cycloalkyl, wobei das Cycloalkyl gegebenenfalls mit $CH_3$, $CF_3$, N($C_{1-3}$Alkyl)$_2$ substituiert ist, oder ein 5- oder 6-gliedriges Heteroaryl ist, wobei das 5- oder 6-gliedrige Heteroaryl gegebenenfalls mit Methyl substituiert ist;

$R_{32a}$ ist $C_{1-3}$-Alkyl und $R_{33a}$ $C_{1-3}$-Alkyl; oder

$R_{32a}$ und $R_{33a}$ bilden zusammen mit dem Stickstoffatom, an das sie gebunden sind, ein $C_{3-5}$-Heterocycloalkyl;

wobei

$R_{1a}$ $R_{1aa}$ ist und/oder
$R_{4a}$ und $R_{5a}$ $R_{4aa}$ und $R_{5aa}$ sind; und/oder
$A_a$ $A_{aa}$ ist; und

wobei, wenn B (B-bc) ist und $R_{3b3c}$ $R_{3b}$ ist, die Verbindung der Formel (I) eine Verbindung der Formel (I-b) ist:

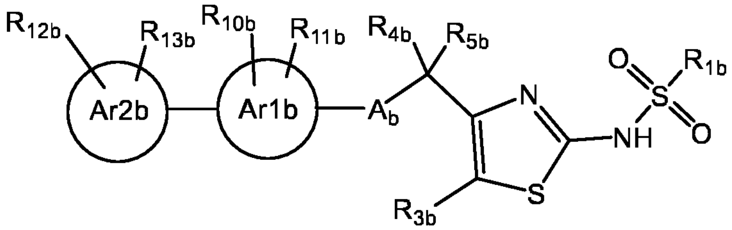

(I-b)

wobei:

$A_b$ $A_{ab}$ oder $A_{bb}$ ist;
wobei:

$A_{ab}$ $-NR_{6b}CH_2-$ oder $-NR_{6b}-$ ist;
$A_{bb}$ $-NR_{6b}C(=O)-$ ist;

$R_{1b}$ $R_{1ab}$ oder $R_{1bb}$ ist;
wobei:
$R_{1ab}$ $NR_{32b}R_{33b}$ ist;
$R_{1bb}$ $C_{1-5}$-Alkyl, $C_{0-2}$-Alkylen-$C_{3-5}$-Cycloalkyl ist, wobei das Cycloalkyl gegebenenfalls mit $CH_3$, $C_{1-3}$-Alkylen$OC_{1-2}$-Alkyl, oder $CF_3$ substituiert ist;
$R_{3b}$ H, Halogen, $CH_3$, $OC_{1-2}$-Alkyl oder $CF_3$ ist;
oder $R_{3b}$ zusammen mit $R_{5bb}$ ein 5- oder 6-gliedriges Cycloalkyl oder 5- oder 6-gliedriges sauerstoffhaltiges Heterocycloalkyl bildet;
$R_{4b}$ und $R_{5b}$ entweder $R_{4ab}$ und $R_{5ab}$ oder $R_{4bb}$ und $R_{5bb}$ sind;
wobei:
$R_{4ab}$ und $R_{5ab}$ zusammen mit dem Kohlenstoffatom, an das sie gebunden sind, ein $C_{3-6}$-Cycloalkyl bilden, das:

durch einen oder zwei Substituenten substituiert ist, wobei jeder Substituent unabhängig ausgewählt ist aus der Gruppe bestehend aus $C_{1-3}$-Alkyl, Oxo, OH, $C_{1-3}$-AlkylOH, $C_{1-3}$-Halogenalkyl, $C_{0-2}$-Alkylen-$C_{3-6-}$-Cycloalkyl, $C_{0-2}$-Alkylen-$C_{3-6}$-Heterocycloalkyl, $C_{1-3}$-Alkylen-$OC_{1-3}$-Alkyl, Halogen, $OC_{1-3}$-Halogenalkyl, $OC_{0-2}$-Alkylen-$C_{3-6}$-Cycloalkyl, $OC_{0-2}$-Alkylen-$C_{3-6}$-Heterocycloalkyl, $OC_{1-3}$-Alkyl und $NR_{21b}R_{22b}$;
oder
eines der Kohlenstoffatome des $C_{3-6}$-Cycloalkyls ein Spirozentrum ist, sodass ein spirocyclisches Ringsystem durch den $C_{3-6}$-Cycloalkylring und einen weiteren $C_{3-6}$-Cycloalkylring oder einen $C_{3-6}$-Heterocycloalkylring gebildet wird, und wobei das durch $R_{4ab}$ und $R_{5ab}$ gebildete $C_{3-6}$-Cycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder
$R_{4ab}$ und $R_{5ab}$ zusammen mit dem Kohlenstoffatom, an das sie gebunden sind, ein $C_{3-6}$-Heterocycloalkyl bilden, wobei eines der Kohlenstoffatome des $C_{3-6}$-Heterocycloalkyls ein Spirozentrum ist, sodass ein spirocyclisches Ringsystem durch den $C_{3-6}$-Heteroalkylring und einen weiteren $C_{3-6}$-Cycloalkylring oder einen $C_{3-6}$-Heteroalkylring gebildet wird, und wobei das durch $R_{4ab}$ und $R_{5ab}$ gebildete $C_{3-6}$-Heterocycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder
$R_{4ab}$ und $R_{5ab}$ bilden zusammen mit dem Kohlenstoffatom, an das sie gebunden sind, ein $C_{3-6}$-Heterocycloalkyl, das ein Stickstoffatom umfasst, wobei dieses Stickstoffatom durch $-S(O)_2R_{29b}$ substituiert ist; oder
$R_{4bb}$ und $R_{5bb}$ sind unabhängig voneinander H, Halogen, $C_{1-6}$Alkyl, $C_{0-2}$Alkylen-$C_{3-6}$-Cycloalkyl, $C_{0-2}$-Alkylen-$C_{3-6}$-Heterocycloalkyl, $OC_{1-6}$-Alkyl, $OC_{0-2}$-Alkylen-$C_{3-6}$-Cycloalkyl, $C_{1-3}$-Alkylen-$OC_{1-3}$-Alkyl, $C_{1-6}$-AlkylOH, $C_{1-6}$-Halogenalkyl, $OC_{1-6}$-Halogenalkyl oder $NR_{21b}R_{22b}$,
oder $R_{4bb}$ H ist und $R_{5bb}$ zusammen mit $R_{3b}$ ein 5- oder 6-gliedriges Cycloalkyl oder 5- oder 6-gliedriges sauerstoffhaltiges Heterocycloalkyl bildet,
oder $R_{4bb}$ und $R_{5bb}$ zusammen mit dem Kohlenstoffatom, an das sie gebunden sind, ein $C_{3-6}$-Cycloalkyl oder $C_{3-6}$-Heterocycloalkyl bilden,
oder $R_{4bb}$ H ist und $R_{5bb}$ und $R_{6b}$ eine $C_{2-3}$-Alkylenkette sind, die einen 5- oder 6-gliedrigen Ring bildet;
oder $R_{4bb}$ O ist und $R_{5bb}$ fehlt;
$R_{6b}$ H oder $C_{1-3}$-Alkyl ist,
oder $R_{6b}$ zusammen mit $R_{11b}$, wenn sie sich in ortho-Position zur Gruppe $A_b$ befinden, eine $C_2$-Alkylenkette sind, die einen 5-gliedrigen Ring bildet,

oder $R_{5bb}$ und $R_{6b}$ eine $C_{2-3}$-Alkylenkette sind, die einen 5- oder 6-gliedrigen Ring bildet, und $R_{4bb}$ H ist;

Ar1b ein 6-gliedriges Aryl oder Heteroaryl ist;

Ar2b ein 6-gliedriges Aryl oder Heteroaryl ist und an Ar1b in der para-Stellung zur Gruppe $A_b$ gebunden ist;

$R_{10b}$ H, Halogen, $C_{1-3}$-Alkyl, $OC_{1-2}$-Alkyl, $C_{1-2}$-Halogenalkyl, $OC_{1-2}$-Halogenalkyl oder CN ist;

$R_{11b}$ H, F, Cl, $CH_3$, Ethyl, $OCH_3$, $CF_3$, $OCF_3$ oder CN ist,

oder $R_{11b}$, wenn sie sich in ortho-Stellung zur Gruppe $A_b$ befinden,

zusammen mit $R_{6b}$ eine $C_2$-Alkylenkette sind, die einen 5-gliedrigen Ring bildet;

$R_{12b}$ in ortho- oder meta-Position relativ zu Ar1b an Ar2b gebunden ist und $R_{12b}$ H, Halogen, $C_{1-4}$-Alkyl, $C_{2-4}$-Alkynyl, $C_{0-2}$-Alkylen-$C_{3-5}$-Cycloalkyl, $OC_{1-4}$-Alkyl, $OC_{0-2}$-Alkylen-$C_{3-5}$-Cycloalkyl, $OCH_2CH_2N(CH_3)_2$, OH, $C_{1-4}$-AlkylOH, CN, $C_{1-3}$-Alkylen-$OC_{1-3}$-Alkyl, $C_{1-4}$-Halogenalkyl, $OC_{1-4}$-Halogenalkyl, $C(=O)C_{1-2}$-Alkyl, $NR_{23b}R_{24b}$, $SO_2C_{1-4}$-Alkyl, $SOC_{1-4}$-Alkyl, $SC_{1-4}$-Alkyl, SH, $C(O)N(CH_3)_2$, NHC(O) $C_{1-3}$-Alkyl, $C_{3-6}$-Heterocycloalkyl ist, umfassend ein Stickstoffatom am Bindungspunkt zu Ar2b, oder $R_{12b}$ bildet zusammen mit einem Stickstoffatom, an das es gebunden ist, ein N-Oxid ($N^+$-$O^-$).

$R_{13b}$ H, Halogen, $CH_3$ oder $OCH_3$ ist;

$R_{21b}$ H, $C_{1-5}$-Alkyl, $C(O)C_{1-5}$-Alkyl, $C(O)OC_{1-5}$-Alkyl, $C_{1-3}$-Alkyl-$OC_{1-2}$-Alkyl, $C_{1-4}$-Halogenalkyl, oder $C_{4-6}$-Heterocycloalkyl ist;

$R_{22b}$ H oder $CH_3$ ist;

$R_{23b}$ H oder $C_{1-2}$-Alkyl ist;

$R_{24b}$ H oder $C_{1-2}$-Alkyl ist;

$R_{29b}$ $C_{1-3}$-Alkyl, $C_{0-2}$-Alkylen-$C_{3-5}$-Cycloalkyl, wobei das Cycloalkyl gegebenenfalls durch $CH_3$, $CF_3$, $N(C_{1-3}\text{-Alkyl})_2$substituiert ist, oder ein 5- oder 6-gliedriges Heteroaryl ist, wobei das 5- oder 6-gliedrige Heteroaryl gegebenenfalls durch Methyl substituiert ist; und

$R_{32b}$ $C_{1-3}$-Alkyl und $R_{33b}$ $C_{1-3}$-Alkyl ist; oder

$R_{32b}$ und $R_{33b}$ zusammen mit dem Stickstoffatom, an das sie gebunden sind, ein $C_{3-5}$-Heterocycloalkyl bilden;

wobei:

$R_{1b}$ $R_{1ab}$ ist; und/oder

$R_{4b}$ und $R_{5b}$ $R_{4ab}$ und $R_{5ab}$ sind; und/oder

$A_b$ $A_{ab}$ ist; oder

wobei, wenn B (B-bc) ist und $R_{3b3c}$ $R_{3c}$ ist, die Verbindung der Formel (I) eine Verbindung der Formel (I-c) ist:

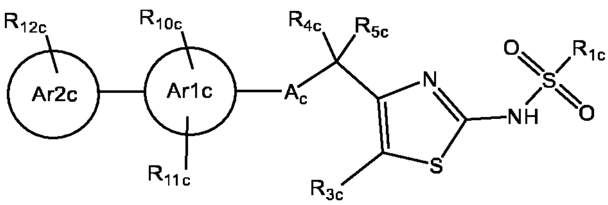

(I-c)

wobei:

$A_c$ $A_{ac}$ oder $A_{bc}$ ist;

wobei:

$A_{ac}$ -$CH_2NR_{6c}$- ist;

$A_{bc}$ -$C(=O)NR_{6c}$- ist;

$R_{1c}$ $R_{1ac}$ oder $R_{1bc}$ ist;

wobei:

$R_{1ac}$ $NR_{32c}R_{33c}$ ist;

$R_{1bc}$ $C_{1-5}$-Alkyl, $C_{0-2}$-Alkylen-$C_{3-5}$-Cycloalkyl ist, wobei das Cycloalkyl gegebenenfalls durch $CH_3$, $C_{1-3-}$-Alkylen-$OC_{1-2}$-Alkyl oder $CF_3$ substituiert ist;

$R_{3c}$ H, $CH_3$, Halogen, $OC_{1-2}$-Alkyl oder $CF_3$ ist;

$R_{4c}$ und $R_{5c}$ entweder $R_{4ac}$ und $R_{5ac}$ oder $R_{4bc}$ und $R_{5bc}$ sind;

wobei:

$R_{4ac}$ und $R_{5ac}$ zusammen mit dem Kohlenstoffatom, an das sie gebunden sind, ein $C_{3-6}$-Cycloalkyl bilden, das:

durch einen oder zwei Substituenten substituiert ist, wobei jeder Substituent unabhängig ausgewählt ist aus der Gruppe bestehend aus $C_{1-3}$-Alkyl, Oxo, OH, $C_{1-3}$-AlkylOH, $C_{1-3}$-Halogenalkyl, $C_{0-2}$-Alkylen-$C_{3-6}$-Cycloalkyl, $C_{0-2}$-Alkylen-$C_{3-6}$-Heterocycloalkyl, $C_{1-3}$-Alkylen-$OC_{1-3}$-Alkyl, Halogen, $OC_{1-3}$-Halogenalkyl, $OC_{0-2}$-Alkylen-$C_{3-6}$-Cycloalkyl, $OC_{0-2}$-Alkylen-$C_{3-6}$-Heterocycloalkyl, $OC_{1-3}$-Alkyl und $NR_{21c}R_{22c}$; oder

eines der Kohlenstoffatome des $C_{3-6}$-Cycloalkyls ein Spirozentrum ist, sodass ein spirocyclisches Ringsystem durch den $C_{3-6}$-Cycloalkylring und einen weiteren $C_{3-6}$-Cycloalkylring oder einen $C_{3-6}$-Heterocycloalkylring gebildet wird, und wobei das durch $R_{4ac}$ und $R_{5ac}$ gebildete $C_{3-6}$-Cycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder

$R_{4ac}$ und $R_{5ac}$ zusammen mit dem Kohlenstoffatom, an das sie gebunden sind, ein $C_{3-6}$-Heterocycloalkyl bilden, wobei eines der Kohlenstoffatome des $C_{3-6}$-Heterocycloalkyls ein Spirozentrum ist, sodass ein spirocyclisches Ringsystem durch den $C_{3-6}$-Heterocycloalkylring und einen weiteren $C_{3-6}$-Cycloalkylring oder einen $C_{3-6}$-Heterocycloalkylring gebildet wird, und wobei das durch $R_{4ac}$ und $R_{5ac}$ gebildete $C_{3-6}$-Heteroycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder

$R_{4ac}$ und $R_{5ac}$ zusammen mit dem Kohlenstoffatom, an das sie gebunden sind, ein $C_{3-6}$-Heterocycloalkyl bilden, das ein Stickstoffatom umfasst, wobei dieses Stickstoffatom durch -$S(O)_2R_{29c}$ substituiert ist; oder

$R_{4bc}$ and $R_{5bc}$ jeweils unabhängig H, $C_{1-6}$-Alkyl, $C_{0-2}$-Alkylen-$C_{3-6}$-Cycloalkyl, $C_{0-2}$-Alkylen-$C_{3-6}$-Heterocycloalkyl, $C_{1-3}$-Alkylen-$OC_{1-3}$-Alkyl, $C_{1-6}$-AlkylOH oder $C_{1-6}$-Halogenalkyl sind,

oder $R_{4bc}$ und $R_{5bc}$ zusammen mit dem Kohlenstoffatom, an das sie gebunden sind, ein $C_{3-6}$-Cycloalkyl oder einen $C_{3-6}$-Heterocycloalkylring bilden,

$R_{6c}$ H oder $C_{1-3}$-Alkyl ist;

Ar1c ein 6-gliedriges Aryl oder Heteroaryl ist;

Ar2c ein 6-gliedriges Aryl oder Heteroaryl ist und an Ar1c in der para-Stellung relativ zur Gruppe $A_c$ gebunden ist;

$R_{10c}$ H, Halogen, $C_{1-3}$-Alkyl, $OC_{1-2}$-Alkyl, $C_{1-2}$-Halogenalkyl, $OC_{1-2}$-Halogenalkyl oder CN ist;

$R_{11c}$ H, F, Cl, $CH_3$, Ethyl, $OCH_3$, $CF_3$, $OCF_3$ oder CN ist,

$R_{12c}$ in meta- oder ortho-Position relativ zu Ar1c an Ar2c gebunden ist und $R_{12c}$ ist H, Halogen, $C_{1-4}$-Alkyl, $C_{2-4}$-Alkynyl, $C(=O)C_{1-2}$-Alkyl, $C_{0-2}$-Alkylen-$C_{3-5}$-Cycloalkyl, $OC_{1-4}$-Alkyl, $C_{1-3}$-Alkylen-$OC_{1-3}$-Alkyl, $C_{1-4}$-Halogenalkyl, $OC_{1-4}$-Halogenalkyl, CN, $OC_{0-2}$-Alkylen-$C_{3-5}$-Cycloalkyl, $OCH_2CH_2N(CH_3)_2$, OH, $C_{1-4}$-AlkylOH, $NR_{23c}R_{24c}$, $SO_2CH_3$, $C(O)N(CH_3)_2$, $NHC(O)C_{1-3}$-Alkyl, oder ein $C_{3-6}$-Heterocycloalkyl ist, umfassend ein Stickstoffatom am Bindungspunkt zu Ar2c, oder $R_{12c}$ zusammen mit einem Stickstoffatom, an das es gebunden ist, ein N-Oxid ($N^+$-$O^-$) bildet;

$R_{21c}$ H, $C_{1-5-}$Alkyl, $C(O)C_{1-5-}$Alkyl, $C(O)OC_{1-5-}$Alkyl, $C_{1-3}$Alkyl$OC_{1-2}$ Alkyl, $C_{1-4}$-Halogenalkyl oder $C_{4-6}$Heterocycloalkyl ist;

$R_{22c}$ H oder $CH_3$ ist;

$R_{23c}$ H oder $C_{1-2}$-Alkyl ist;
$R_{24c}$ H oder $C_{1-2}$-Alkyl ist;
$R_{29c}$ $C_{1-3}$-Alkyl, $C_{0-2}$-Alkylen-$C_{3-5}$-Cycloalkyl, wobei das Cycloalkyl gegebenenfalls durch $CH_3$, $CF_3$, $N(C_{1-3}\text{-Alkyl})_2$ substituiert ist, oder ein 5- oder 6-gliedriges Heteroaryl ist, wobei das 5- oder 6-gliedrige Heteroaryl gegebenenfalls durch Methyl substituiert ist; und
$R_{32c}$ $C_{1-3}$-Alkyl ist und $R_{33c}$ $C_{1-3}$-Alkyl ist; oder
$R_{32c}$ und $R_{33c}$ zusammen mit dem Stickstoffatom, an das sie gebunden sind, ein $C_{3-5}$-Heterocycloalkyl bilden;
wobei:

$R_{1c}$ $R_{1ac}$ ist; und/oder

$R_{4c}$ und $R_{5c}$ $R_{4ac}$ und $R_{5ac}$ sind; und/oder
$A_c$ $A_{ac}$ ist;

oder ein Salz und/oder Solvat davon.

**2.** Verbindung, das Salz und/oder das Solvat davon nach Anspruch 1, die eine Verbindung der Formel (I) ist:

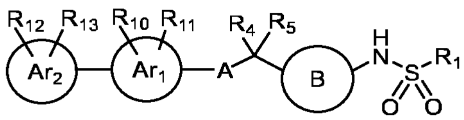
(I)

wobei Ring B ausgewählt ist aus der Gruppe bestehend aus:

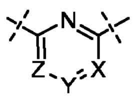
(B-a)

wobei X, Y und Z wie unten definiert sind; und:

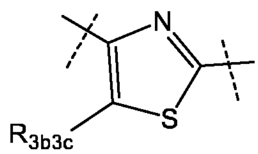
(B-bc);

wobei $R_{3b3c}$ $R_{3b}$ oder $R_{3c}$ wie unten definiert ist;

wobei, wenn B (B-a) ist, die Verbindung der Formel (I) eine Verbindung der Formel (I-a) ist:

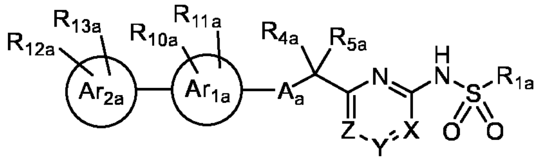
(I-a)

wobei:
$A_a$ $A_{aa}$ oder $A_{ba}$ ist;
wobei:

$A_{aa}$ ein Amin-Linker mit folgender Struktur ist: -NH-, $-CH_2NH-$ oder $-NHCH_2-$;
$A_{ba}$ ein Amid-Linker mit der folgenden Struktur ist: - C(=O)NH- oder -NHC(=O)-;

X N oder CH ist;
Y N oder $CR_{2a}$ ist;
Z N oder $CR_{3a}$ ist;
mit der Maßgabe, dass, wenn mindestens eines von X oder Z N ist, Y nicht N sein kann;
$R_{2a}$ H, Halogen, $C_{1-2}$-Alkyl, $OC_{1-2}$-Alkyl, $C_{1-2}$-Halogenalkyl oder $OC_{1-2}$-Halogenalkyl ist; und
$R_{3a}$ H, Halogen, $CH_3$, $OCH_3$, $CF_3$ oder $OCF_3$ ist;
wobei mindestens eines von $R_{2a}$ und $R_{3a}$ H ist;
$R_{1a}$ $R_{1aa}$ oder $R_{1ba}$ ist;
wobei:
$R_{1aa}$ $NR_{32a}R_{33a}$ ist;
$R_{1ba}$ $C_{1-5-}$-Alkyl, $C_{0-2}$-Alkylen$C_{3-5}$-Cycloalkyl ist, wobei das Cycloalkyl gegebenenfalls mit $CH_3$ oder $CF_3$ substituiert ist;
$R_{4a}$ und $R_{5a}$ $R_{4aa}$ und $R_{5aa}$, oder $R_{4ba}$ und $R_{5ba}$ sind;
wobei:
$R_{4aa}$ und $R_{5aa}$ zusammen mit dem Kohlenstoffatom, an das sie gebunden sind, ein $C_{3-6}$-Cycloalkyl bilden, das:

durch einen oder zwei Substituenten substituiert ist, wobei jeder Substituent unabhängig ausgewählt ist aus der Gruppe bestehend aus $C_{1-3}$-Alkyl, Oxo, OH, $C_{1-3}$-AlkylOH, $C_{1-3}$-Halogenalkyl, $C_{0-2}$-Alkylen$C_{3-6}$-Cycloalkyl, $C_{0-2}$-Alkylen-$C_{3-6}$-Heterocycloalkyl, $C_{1-3}$-Alkylen$OC_{1-3}$-Alkyl, Halogen, $OC_{1-3}$-Halogenalkyl, $OC_{0-2}$Alkylen$C_{3-6}$-Cycloalkyl $OC_{0-2}$-Alkylen-$C_{3-6}$-Heterocycloalkyl, $OC_{1-3}$-Alkyl und $NR_{21a}R_{22a}$; oder
eines der Kohlenstoffatome des $C_{3-6}$-Cycloalkyls ein Spirozentrum ist, sodass ein spirocyclisches Ringsystem durch den $C_{3-6}$-Cycloalkylring und einen weiteren $C_{3-6}$-Cycloalkylring oder einen $C_{3-6}$-Heterocycloalkylring gebildet wird, und wobei das durch $R_{4aa}$ und $R_{5aa}$ gebildete $C_{3-6}$-Cycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder
$R_{4aa}$ und $R_{5aa}$ zusammen mit dem Kohlenstoffatom, an das sie gebunden sind, ein $C_{3-6}$-Heterocycloalkyl bilden, wobei eines der Kohlenstoffatome des $C_{3-6}$-Heterocycloalkyls ein Spirozentrum ist, sodass ein spirocyclisches Ringsystem durch den $C_{3-6}$-Heterocycloalkylring und einen weiteren $C_{3-6}$-Cycloalkylring oder einen $C_{3-6}$-Heterocycloalkylring gebildet wird, und wobei das durch $R_{4aa}$ und $R_{5aa}$ gebildete $C_{3-6}$-Heterocycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder
$R_{4aa}$ und $R_{5aa}$ zusammen mit dem Kohlenstoffatom, an das sie gebunden sind, ein $C_{3-6}$-Heterocycloalkyl bilden, das ein Stickstoffatom umfasst, wobei dieses Stickstoffatom durch -$S(O)_2R_{29a}$ substituiert ist; oder
$R_{4ba}$ und $R_{5ba}$ jeweils unabhängig H, $C_{1-6}$-Alkyl, $C_{1-6}$-Alkyl-OH, $C_{1-6}$-Halogenalkyl, $C_{0-2}$-Alkylen-$C_{3-6}$-Cycloalkyl, $C_{0-2}$-Alkylen-$C_{3-6}$-Heterocycloalkyl, $C_{1-3}$-Alkylen-$OC_{1-3}$-Alkyl sind oder $R_{4ba}$ und $R_{5ba}$ zusammen mit dem Kohlenstoffatom, an das sie gebunden sind, ein $C_{3-6}$-Cycloalkyl oder $C_{3-6}$-Heterocycloalkyl bilden; und
wenn $A_a$ -NHC(=O)- oder -$NHCH_2$- ist:

können $R_{4ba}$ und $R_{5ba}$ zusätzlich aus Halogen, $OC_{1-6}$-Halogenalkyl, $OC_{0-2}$-Alkylen-$C_{3-6}$-Cycloalkyl, $OC_{0-2}$-Alkylen-$C_{3-6}$-Heterocycloalkyl, $OC_{1-6}$-Alkyl and $NR_{21a}R_{22a}$ ausgewählt sein;
Ar1a ein 6-gliedriges Aryl oder Heteroaryl ist;
Ar2a ein 6-gliedriges Aryl oder Heteroaryl ist und an Ar1a in der para-Stellung relativ zur Gruppe $A_a$ gebunden ist;
$R_{10a}$ H, Halogen, $C_{1-3}$-Alkyl, $C_{1-2}$-Halogenalkyl, $OC_{1-2}$-Alkyl, $OC_{1-2}$-Halogenalkyl oder CN ist;
$R_{11a}$ H, F, Cl, $C_{1-2}$-Alkyl, $CF_3$, $OCH_3$ oder CN ist;
$R_{12a}$ an Ar2 in ortho- oder meta-Stellung relativ zu Ar1a gebunden ist und $R_{12a}$ ist H, Halogen, $C_{1-4}$-Alkyl, $C_{2-4}$-Alkenyl, $C_{0-2}$-Alkylen$C_{3-5-}$-Cycloalkyl $OC_{1-4}$-Alkyl, $OC_{0-2}$-Alkylen-$C_{3-5}$-Cycloalkyl, $C_{1-4}$-Halogenalkyl, $OC_{1-4}$-Halogenalkyl, Hydroxy, $C_{1-4}$-AlkylOH, $SO_2C_{1-2}$-Alkyl, $C(O)N(C_{1-2}Alkyl)_2$, $NHC(O)C_{1-3}$-Alkyl oder $NR_{23a}R_{24a}$; und
wenn $A_a$ -NHC(=O)-, -NH- oder -$NHCH_2$- ist:

kann $R_{12a}$ zusätzlich aus CN, $OCH_2CH_2N(CH_3)_2$ und einem $C_{3-6}$-Heterocycloalkyl, das ein Stickstoffatom umfasst, das sich an der Bindungsstelle an Ar2a befindet, ausgewählt sein, oder bildet $R_{12a}$ zusammen mit dem Stickstoffatom, an das es gebunden ist, ein N-Oxid ($N^+$-$O^-$) ;
$R_{13a}$ H oder Halogen ist;

$R_{21a}$ H, $C_{1-5}$-Alkyl, $C(O)C_{1-5}$-Alkyl, $C(O)OC_{1-5}$-Alkyl ist;
$R_{22a}$ H oder $CH_3$ ist;
$R_{23a}$ H oder $C_{1-2}$-Alkyl ist; und
$R_{24a}$ H oder $C_{1-2}$-Alkyl ist
$R_{29a}$ $C_{1-3}$-Alkyl, $C_{0-2}$-Alkylen-$C_{3-5}$-Cycloalkyl ist, wobei das Cycloalkyl gegebenenfalls mit $CH_3$, oder $CF_3$ substituiert ist;
$R_{32a}$ $C_{1-3}$-Alkyl ist und $R_{33}$ $C_{1-3}$-Alkyl ist; oder
$R_{32a}$ und $R_{33a}$ zusammen mit dem Stickstoffatom, an das sie gebunden sind, ein $C_{3-5}$-Heterocycloalkyl bilden;
wobei

$R_{1a}$ $R_{1aa}$ ist und/oder
$R_{4a}$ und $R_{5a}$ $R_{4aa}$ und $R_{5aa}$ sind; und/oder
$A_a$ $A_{aa}$ ist; und

wobei, wenn B (B-bc) ist und $R_{3b3c}$ $R_{3b}$ ist, die Verbindung der Formel (I) eine Verbindung der Formel (I-b) ist:

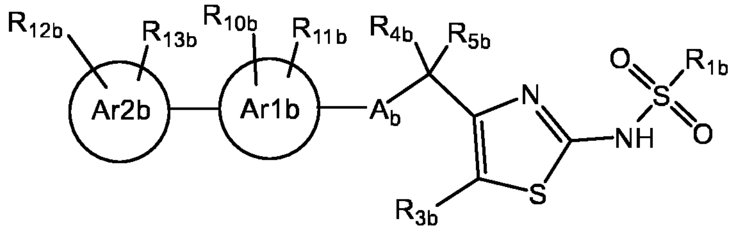
(I-b)

wobei:

$A_b$ $A_{ab}$ oder $A_{bb}$ ist;
wobei:

$A_{ab}$ $-NR_{6b}CH_2-$ oder $-NR_{6b}-$ ist;
$A_{bb}$ $-NR_{6b}C(=O)-$ ist;

$R_{1b}$ $R_{1ab}$ oder $R_{1bb}$ ist;
wobei:
$R_{1ab}$ $NR_{32b}R_{33b}$ ist;

$R_{1bb}$ $C_{1-5}$-Alkyl, $C_{0-2}$-Alkylen-$C_{3-5}$-Cycloalkyl ist, wobei das Cycloalkyl gegebenenfalls mit $CH_3$, $C_{1-3}$-Alkylen$OC_{1-2}$-Alkyl, oder $CF_3$ substituiert ist;
$R_{3b}$ H, Halogen, $CH_3$, $OC_{1-2}$-Alkyl oder $CF_3$ ist;
oder $R_{3b}$ zusammen mit $R_{5bb}$ ein 5- oder 6-gliedriges Cycloalkyl oder 5- oder 6-gliedriges sauerstoffhaltiges Heterocycloalkyl bildet;
$R_{4b}$ und $R_{5b}$ entweder $R_{4ab}$ und $R_{5ab}$ oder $R_{4bb}$ und $R_{5bb}$ sind;
wobei:
$R_{4ab}$ und $R_{5ab}$ zusammen mit dem Kohlenstoffatom, an das sie gebunden sind, ein $C_{3-6}$-Cycloalkyl bilden, das:

durch einen oder zwei Substituenten substituiert ist, wobei jeder Substituent unabhängig ausgewählt ist aus der Gruppe bestehend aus $C_{1-3}$-Alkyl, Oxo, OH, $C_{1-3}$-AlkylOH, $C_{1-3}$-Halogenalkyl, $C_{0-2}$-Alkylen-$C_{3-6}$-Cycloalkyl, $C_{0-2}$-Alkylen-$C_{3-6}$-Heterocycloalkyl, $C_{1-3}$-Alkylen-$OC_{1-3}$-Alkyl, Halogen, $OC_{1-3}$-Halogenalkyl, $OC_{0-2}$-Alkylen-$C_{3-6}$-Cycloalkyl, $OC_{0-2}$-Alkylen-$C_{3-6}$-Heterocycloalkyl, $OC_{1-3}$-Alkyl und $NR_{21b}R_{22b}$; oder
eines der Kohlenstoffatome des $C_{3-6}$-Cycloalkyls ein Spirozentrum ist, sodass ein spirocyclisches Ringsystem durch den $C_{3-6}$-Cycloalkylring und einen weiteren $C_{3-6}$-Cycloalkylring oder einen $C_{3-6}$-Heterocycloalkylring gebildet wird, und wobei das durch $R_{4ab}$ und $R_{5ab}$ gebildete $C_{3-6}$-Cycloalkyl zusammen mit dem Kohlenstoffatom, an das sie gebunden sind, durch einen

oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder

$R_{4ab}$ und $R_{5ab}$ zusammen mit dem Kohlenstoffatom, an das sie gebunden sind, ein $C_{3-6}$-Heterocycloalkyl bilden, wobei eines der Kohlenstoffatome des $C_{3-6}$-Heterocycloalkyls ein Spirozentrum ist, sodass ein spirocyclisches Ringsystem durch den $C_{3-6}$-Heterocycloalkylring und einen weiteren $C_{3-6}$-Cycloalkylring oder einen $C_{3-6}$-Heterocycloalkylring gebildet wird, und wobei der durch $R_{4ab}$ und $R_{5ab}$ gebildete $C_{3-6}$-Heteroycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder

$R_{4ab}$ und $R_{5ab}$ zusammen mit dem Kohlenstoffatom, an das sie gebunden sind, ein $C_{3-6}$-Heterocycloalkyl bilden, das ein Stickstoffatom umfasst, wobei dieses Stickstoffatom durch $-S(O)_2R_{29b}$ substituiert ist; oder

$R_{4bb}$ und $R_{5bb}$ unabhängig voneinander H, Halogen, $C_{1-6}$Alkyl, $C_{0-2}$Alkylen-$C_{3-6}$-Cycloalkyl, $C_{0-2}$-Alkylen-$C_{3-6}$-Heterocycloalkyl, $OC_{1-6}$-Alkyl, $OC_{0-2}$-Alkylen-$C_{3-6}$-Cycloalkyl, $C_{1-3}$-Alkylen-$OC_{1-3}$-Alkyl, $C_{1-6}$-AlkylOH, $C_{1-6}$-Halogenalkyl, $OC_{1-6}$-Halogenalkyl oder $NR_{21b}R_{22b}$ sind,

oder $R_{4bb}$ H ist und $R_{5bb}$ zusammen mit $R_{3b}$ ein 5- oder 6-gliedriges Cycloalkyl oder 5- oder 6-gliedriges sauerstoffhaltiges Heterocycloalkyl bildet,

oder $R_{4bb}$ und $R_{5bb}$ zusammen mit dem Kohlenstoffatom bilden, an das sie gebunden sind, ein $C_{3-6}$-Cycloalkyl oder $C_{3-6}$-Heterocycloalkyl, oder $R_{4bb}$ H ist und $R_{5bb}$ und $R_{6b}$ eine $C_{2-3}$-Alkylenkette sind, die einen 5- oder 6-gliedrigen Ring bildet;

oder $R_{4bb}$ O ist und $R_{5bb}$ fehlt;

$R_{6b}$ H oder $C_{1-3}$-Alkyl ist,

oder $R_{6b}$ zusammen mit $R_{11b}$, wenn sie sich in ortho-Position zur Gruppe $A_b$ befinden, eine $C_2$-Alkylenkette sind, die einen 5-gliedrigen Ring bildet,

oder $R_{5bb}$ und $R_{6b}$ eine $C_{2-3}$-Alkylenkette sind, die einen 5- oder 6-gliedrigen Ring bildet, und $R_{4bb}$ H ist;

Ar1b ein 6-gliedriges Aryl oder Heteroaryl ist;

Ar2b ein 6-gliedriges Aryl oder Heteroaryl ist und an Ar1b in der para-Stellung relativ zur Gruppe $A_b$ gebunden ist;

$R_{10b}$ H, Halogen, $C_{1-3}$-Alkyl, $OC_{1-2}$-Alkyl, $C_{1-2}$-Halogenalkyl, $OC_{1-2}$-Halogenalkyl oder CN ist;

$R_{11b}$ H, F, Cl, $CH_3$, Ethyl, $OCH_3$, $CF_3$, $OCF_3$ oder CN ist,

oder $R_{11b}$, wenn sie sich in ortho-Stellung zur Gruppe $A_b$ befinden, zusammen mit $R_{6b}$ eine $C_2$-Alkylenkette sind, die einen 5-gliedrigen Ring bildet;

$R_{12b}$ in ortho- oder meta-Position relativ zu Ar1b an Ar2b gebunden ist und $R_{12b}$ H, Halogen, $C_{1-4}$-Alkyl, $C_{2-4}$-Alkynyl, $C_{0-2}$-Alkylen-$C_{3-5}$-Cycloalkyl, $OC_{1-4}$-Alkyl, $OC_{0-2}$-Alkylen-$C_{3-3}$-Cycloalkyl, $OCH_2CH_2N(CH_3)_2$, OH, $C_{1-4}$-AlkylOH, CN, $C_{1-3}$-Alkylen-$OC_{1-3}$-Alkyl, $C_{1-4}$-Halogenalkyl, $OC_{1-4}$-Halogenalkyl, C (=O) $C_{1-2}$-Alkyl, $NR_{23b}R_{24b}$, $SO_2C_{1-4}$-Alkyl, $SOC_{1-4}$-Alkyl, $SC_{1-4}$-Alkyl, SH, $C(O)N(CH_3)_2$, $NHC(O)C_{1-3}$-Alkyl, $C_{3-6}$-Heterocycloalkyl ist, umfassend ein Stickstoffatom am Bindungspunkt zu Ar2b, oder $R_{12b}$ zusammen mit einem Stickstoffatom, an das es gebunden ist, ein N-Oxid ($N^+$-$O^-$) bildet.

$R_{13b}$ H, Halogen, $CH_3$ oder $OCH_3$ ist;

$R_{21b}$ H, $C_{1-5}$-Alkyl, $C(O)C_{1-5}$-Alkyl, $C(O)OC_{1-5}$-Alkyl ist;

$R_{22b}$ H oder $CH_3$ ist;

$R_{23b}$ H oder $C_{1-2}$-Alkyl ist;

$R_{24b}$ H oder $C_{1-2}$-Alkyl ist;

$R_{29b}$ $C_{1-3}$-Alkyl, $C_{0-2}$-Alkylen-$C_{3-5}$-Cycloalkyl ist, wobei das Cycloalkyl gegebenenfalls mit $CH_3$, oder $CF_3$ substituiert ist; und

$R_{32b}$ $C_{1-3}$-Alkyl und $R_{33b}$ $C_{1-3}$-Alkyl ist; oder

$R_{32b}$ und $R_{33b}$ zusammen mit dem Stickstoffatom, an das sie gebunden sind, ein $C_{3-5}$-Heterocycloalkyl bilden;

wobei:

$R_{1b}$ $R_{1ab}$ ist; und/oder

$R_{4b}$ und $R_{5b}$ $R_{4ab}$ und $R_{5ab}$ sind; und/oder

$A_b$ $A_{ab}$ ist; oder

wobei, wenn B (B-bc) ist und $R_{3b3c}$ $R_{3c}$ ist, die Verbindung der Formel (I) eine Verbindung der Formel (I-c) ist:

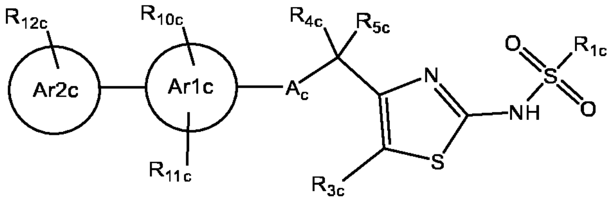

(I-c)

wobei:

$A_c$ $A_{ac}$ oder $A_{bc}$ ist;
$A_{ac}$ $-CH_2NR_{6c}-$ ist;
$A_{bc}$ $-C(=O)NR_{6c}-$ ist;
$R_{1c}$ $R_{1ac}$ oder $R_{1bc}$ ist;
wobei:
$R_{1ac}$ $NR_{32c}R_{33c}$ ist;
$R_{1bc}$ $C_{1-5}$-Alkyl, $C_{0-2}$-Alkylen-$C_{3-5}$-Cycloalkyl ist, wobei das Cycloalkyl gegebenenfalls durch $CH_3$, $C_{1-3-}$-Alkylen-$OC_{1-2}$-Alkyl oder $CF_3$ substituiert ist;
$R_{3c}$ H, $CH_3$, Halogen, $OC_{1-2}$-Alkyl oder $CF_3$ ist;
$R_{4c}$ und $R_{5c}$ entweder $R_{4ac}$ und $R_{5ac}$ oder $R_{4bc}$ und $R_{5bc}$ sind;
wobei:
$R_{4ac}$ und $R_{5ac}$ zusammen mit dem Kohlenstoffatom, an das sie gebunden sind, ein $C_{3-6}$-Cycloalkyl bilden, das:

durch einen oder zwei Substituenten substituiert ist, wobei jeder Substituent unabhängig ausgewählt ist aus der Gruppe bestehend aus $C_{1-3}$-Alkyl, Oxo, OH, $C_{1-3}$-AlkylOH, $C_{1-3}$-Halogenalkyl, $C_{0-2}$-Alkylen-$C_{3-6}$-Cycloalkyl, $C_{0-2}$-Alkylen-$C_{3-6}$-Heterocycloalkyl, $C_{1-3}$-Alkylen-$OC_{1-3}$-Alkyl, Halogen, $OC_{1-3}$-Halogenalkyl, $OC_{0-2}$-Alkylen-$C_{3-6}$-Cycloalkyl, $OC_{0-2}$-Alkylen-$C_{3-6}$-Heterocycloalkyl, $OC_{1-3}$-Alkyl und $NR_{21c}R_{22c}$; oder
eines der Kohlenstoffatome des $C_{3-6}$-Cycloalkyls ein Spirozentrum ist, sodass ein spirocyclisches Ringsystem durch den $C_{3-6}$-Cycloalkylring und einen weiteren $C_{3-6}$-Cycloalkylring oder einen $C_{3-6}$-Heterocycloalkylring gebildet wird, und wobei das durch $R_{4ac}$ und $R_{5ac}$ gebildete $C_{3-6}$-Cycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder
$R_{4ac}$ und $R_{5ac}$ zusammen mit dem Kohlenstoffatom, an das sie gebunden sind, ein $C_{3-6}$-Heterocycloalkyl bilden, wobei eines der Kohlenstoffatome des $C_{3-6}$-Heterocycloalkyls ein Spirozentrum ist, sodass ein spirocyclisches Ringsystem durch den $C_{3-6}$-Heterocycloalkylring und einen weiteren $C_{3-6}$-Cycloalkylring oder einen $C_{3-6}$-Heterocycloalkylring gebildet wird, und wobei der durch $R_{4ac}$ und $R_{5ac}$ gebildete $C_{3-6}$-Heteroycloalkyl zusammen mit dem Kohlenstoffatom, an das sie gebunden sind, durch einen oder zwei Substituenten substituiert sein kann, wobei jeder Substituent unabhängig voneinander aus der Gruppe ausgewählt ist, die aus $C_{1-3}$-Alkyl oder $OC_{1-3}$-Alkyl besteht; oder
$R_{4ac}$ und $R_{5ac}$ zusammen mit dem Kohlenstoffatom, an das sie gebunden sind, ein $C_{3-6}$-Heterocycloalkyl bilden, das ein Stickstoffatom umfasst, wobei dieses Stickstoffatom durch $-S(O)_2R_{29c}$ substituiert ist; oder
$R_{4bc}$ and $R_{5bc}$ jeweils unabhängig H, $C_{1-6}$-Alkyl, $C_{0-2}$-Alkylen-$C_{3-6}$-Cycloalkyl, $C_{0-2}$-Alkylen-$C_{3-6}$-Heterocycloalkyl, $C_{1-3}$-Alkylen-$OC_{1-3}$-Alkyl, $C_{1-6}$-AlkylOH oder $C_{1-6}$-Halogenalkyl sind,
oder $R_{4bc}$ und $R_{5bc}$ zusammen mit dem Kohlenstoffatom, an das sie gebunden sind, ein $C_{3-6}$-Cycloalkyl oder einen $C_{3-6}$-Heterocycloalkylring bilden,
$R_{6c}$ H oder $C_{1-3}$-Alkyl ist;

Ar1c ein 6-gliedriges Aryl oder Heteroaryl ist;
Ar2c ein 6-gliedriges Aryl oder Heteroaryl ist und an Ar1c in der para-Stellung zur Gruppe $A_c$ gebunden ist;
$R_{10c}$ H, Halogen, $C_{1-3}$-Alkyl, $OC_{1-2}$-Alkyl, $C_{1-2}$-Halogenalkyl, $OC_{1-2}$-Halogenalkyl oder CN ist;
$R_{11c}$ H, F, Cl, $CH_3$, Ethyl, $OCH_3$, $CF_3$, $OCF_3$ oder CN ist,
$R_{12c}$ in meta- oder ortho-Position relativ zu Ar1c an Ar2c gebunden ist und $R_{12c}$ H, Halogen, $C_{1-4}$-Alkyl, $C_{2-4}$-Alkynyl, $C(=O)C_{1-2}$-Alkyl, $C_{0-2}$-Alkylen-$C_{3-5}$-Cycloalkyl, $OC_{1-4}$-Alkyl, $C_{1-3}$-Alkylen-$OC_{1-3}$-Alkyl, $C_{1-4}$-Halogenalkyl, $OC_{1-4}$-Halogenalkyl, CN, $OC_{0-2}$-Alkylen-$C_{3-5}$-Cycloalkyl, $OCH_2CH_2N(CH_3)_2$, OH, $C_{1-4}$-AlkylOH, $NR_{23c}R_{24c}$, $SO_2CH_3$, $C(O)N(CH_3)_2$, $NHC(O)C_{1-3}$-Alkyl, oder ein $C_{3-6}$-Heterocycloalkyl ist, umfassend ein Stickstoffatom am Bindungspunkt zu Ar2c, oder $R_{12c}$ zusammen mit einem Stickstoffatom, an das es gebunden ist, ein N-Oxid ($N^+$-$O^-$) bildet;
$R_{21c}$ H, $C_{1-3}$-Alkyl, $C(O)C_{1-5}$-Alkyl, $C(O)OC_{1-5}$-Alkyl ist;
$R_{22c}$ H oder $CH_3$ ist;
$R_{23c}$ H oder $C_{1-2}$-Alkyl ist;
$R_{24c}$ H oder $C_{1-2}$-Alkyl ist;
$R_{29c}$ $C_{1-3}$-Alkyl, $C_{0-2}$-Alkylen-$C_{3-5}$-Cycloalkyl ist, wobei das Cycloalkyl gegebenenfalls mit $CH_3$, oder $CF_3$ substituiert ist; und
$R_{32c}$ $C_{1-3}$-Alkyl ist und $R_{33c}$ $C_{1-3}$-Alkyl ist; oder
$R_{32c}$ und $R_{33c}$ zusammen mit dem Stickstoffatom, an das sie gebunden sind, ein $C_{3-5}$-Heterocycloalkyl bilden;
wobei:

$R_{1c}$ $R_{1ac}$ ist; und/oder

$R_{4c}$ und $R_{5c}$ $R_{4ac}$ und $R_{5ac}$ sind; und/oder
$A_c$ $A_{ac}$ ist;

oder ein Salz und/oder Solvat davon.

**3.** Verbindung, Salz und/oder Solvat davon nach Anspruch 1 oder 2, die die Verbindung oder ein pharmazeutisch verträgliches Salz und/oder Solvat davon ist.

**4.** Pharmazeutisch verträgliches Salz und Solvat einer Verbindung der Formel (I) nach Anspruch 3.

**5.** Pharmazeutisch verträgliches Salz einer Verbindung der Formel (I) nach Anspruch 3.

**6.** Pharmazeutisch akzeptables Solvat einer Verbindung der Formel (I) nach Anspruch 3.

**7.** Verbindungen der Formel (I) nach Anspruch 1 oder 2.

**8.** Verbindung, Salz und/oder Solvat davon nach einem der Ansprüche 1 bis 7, wobei die Verbindung der Formel (I) eine Verbindung der Formel (I-a) ist.

**9.** Verbindung, Salz und/oder Solvat davon nach einem der Ansprüche 1 bis 7, wobei die Verbindung der Formel (I) eine Verbindung der Formel (I-b) ist.

**10.** Verbindung, Salz und/oder Solvat davon nach einem der Ansprüche 1 bis 7, wobei die Verbindung der Formel (I) eine Verbindung der Formel (I-c) ist.

**11.** Verbindung, Salz und/oder Solvat davon nach einem der Ansprüche 8 bis 10 , wobei $R_{1a}$ $R_{1aa}$ ist, $R_{1b}$ $R_{1ab}$ ist oder $R_{1c}$ $R_{1ac}$ ist.

**12.** Verbindung, Salz und/oder Solvat davon nach einem der Ansprüche 8 bis 11, wobei $R_{4a}$ und $R_{5a}$ $R_{4aa}$ und $R_{5aa}$ sind, $R_{4b}$ und $R_{5b}$ $R_{4ab}$ und $R_{5ab}$ sind oder $R_{4c}$ und $R_{5c}$ $R_{4ac}$ und $R_{5ac}$ sind.

**13.** Verbindung, das Salz und/oder das Solvat davon nach einem der Ansprüche 8 bis 12, wobei $A_a$ $A_{aa}$ ist, $A_b$ $A_{ab}$ ist oder $A_c$ $A_{ac}$ ist.

**14.** Verbindung, das Salz und/oder das Solvat davon nach Anspruch 1, ausgewählt aus der Gruppe bestehend aus:

1-(2-(Cyclopropansulfonamido)pyrimidin-4-yl)-*N*-(5-(6-ethoxypyrazin-2-yl)pyridin-2-yl)-4-oxocyclohexancarboxamid; 1-(2-(Cyclopropansulfonamido)pyrimidin-4-yl)-*N*-(5-(6-ethoxypyrazin-2-yl)pyridin-2-yl)-4-hydroxycyclohexancarboxamid;
1-(2-(Cyclopropansulfonamido)pyrimidin-4-yl)-*N*-(5-(6-ethoxypyrazin-2-yl)pyridin-2-yl)-4-hydroxycyclohexancarboxamid (Diastereomer 1);
1-(2-(Cyclopropansulfonamido)pyrimidin-4-yl)-*N*-(5-(6-ethoxypyrazin-2-yl)pyridin-2-yl)-4-hydroxycyclohexancarboxamid (Diastereomer 2);
1-(2-(Cyclopropansulfonamido)pyrimidin-4-yl)-4-(dimethylamino)-*N*-(5-(6-ethoxypyrazin-2-yl)pyridin-2-yl)cyclohexan-1-carboxamid;
1-(2-(Cyclopropansulfonamido)pyrimidin-4-yl)-4-(dimethylamino)-*N*-(5-(6-ethoxypyrazin-2-yl)pyridin-2-yl)cyclohexan-1-carboxamid (Diastereomer 1);
1-(2-(Cyclopropansulfonamido)pyrimidin-4-yl)-4-(dimethylamino)-*N*-(5-(6-ethoxypyrazin-2-yl)pyridin-2-yl)cyclohexan-1-carboxamid (Diastereomer 2);
*N*-(4-(1-((4-(6-Ethoxypyrazin-2-yl)-2-fluorbenzyl)amino)propyl)pyrimidin-2-yl)cyclopropansulfonamid;
1-(2-(Cyclopropansulfonamido)pyrimidin-4-yl)-*N*-(5-(6-ethoxypyrazin-2-yl)pyridin-2-yl)-4,4-difluorcyclohexan-1-carboxamid;
8-(2-(Cyclopropansulfonamido)pyrimidin-4-yl)-*N*-(5-(6-ethoxypyrazin-2-yl)pyridin-2-yl)-1,4-dioxaspiro[4.5]decan-8-carboxamid;
4-(2-((*N,N*-Dimethylsulfamoyl)amino)pyrimidin-4-yl)-*N*-(5-(6-ethoxypyrazin-2-yl)pyridin-2-yl)tetrahydro-2*H*-pyran-4-carboxamid;
4-(2-(Cyclopropansulfonamido)pyrimidin-4-yl)-*N*-(5-(6-ethoxypyrazin-2-yl)pyridin-2-yl)-1-(methylsulfonyl)piperidin-4-carboxamid;
N-(4-(1-((5-(6-Ethoxypyrazin-2-yl)pyridin-2-yl)methyl)amino)cyclopropyl)pyrimidin-2-yl)cyclopropansulfonamid;
N-(4-(1-((4-(6-Ethoxypyrazin-2-yl)-2-fluorbenzyl)amino)cyclopropyl)pyrimidin-2-yl)cyclopropansulfonamid;
N-(4-(4-((4-(6-Ethoxypyrazin-2-yl)phenyl)amino)methyl)tetrahydro-2H-pyran-4-yl)pyrimidin-2-yl)cyclopropansulfonamid;
2-(2-(Cyclopropansulfonamido)pyrimidin-4-yl)-*N*-(5-(6-ethoxypyrazin-2-yl)pyridin-2-yl)-5,8-dioxaspiro[3.4]octan-2-carboxamid;
1-(2-(Cyclopropansulfonamido)pyrimidin-4-yl)-*N*-(5-(6-ethoxypyrazin-2-yl)pyridin-2-yl)-4-methoxycyclohexan-1-carboxamid;
*N*-(4-(1-((4-(6-Ethoxypyrazin-2-yl)phenyl)amino)propyl)pyrimidin-2-yl)cyclopropansulfonamidcarboxamid;
4-(2-(Cyclopropansulfonamido)pyrimidin-4-yl)-*N*-(5-(6-ethoxypyrazin-2-yl)pyridin-2-yl)-1-(2-methoxyacetyl)piperidin-4-carboxamid;
4-(2-(Cyclopropansulfonamido)pyrimidin-4-yl)-*N*-(5-(6-ethoxypyrazin-2-yl)pyridin-2-yl)-1-(ethylsulfonyl)piperidin-4-carboxamid;
4-(2-(Cyclopropansulfonamido)pyrimidin-4-yl)-1-(cyclopropylsulfonyl)-*N*-(5-(6-ethoxypyrazin-2-yl)pyridin-2-yl) piperidin-4-carboxamid;
4-(2-(Cyclopropansulfonamido)pyrimidin-4-yl)-1-(*N,N*-dimethylsulfamoyl)-*N*-(5-(6-ethoxypyrazin-2-yl)pyridin-2-yl)piperidin-4-carboxamid;
4-(2-(Cyclopropansulfonamido)pyrimidin-4-yl)-*N*-(5-(6-ethoxypyrazin-2-yl)pyridin-2-yl)-1-((trifluormethyl)sulfonyl)piperidin-4-carboxamid;
4-(2-(Cyclopropansulfonamido)pyrimidin-4-yl)-*N*-(5-(6-ethoxypyrazin-2-yl)pyridin-2-yl)-1-((1-methyl-1*H*-pyrazol-3-yl)sulfonyl)piperidin-4-carboxamid;
1-(Cyanomethyl)-4-(2-(cyclopropansulfonamido)pyrimidin-4-yl)-N-(5-(6-ethoxypyrazin-2-yl)pyridin-2-yl)piperidin-4-carboxamid;
Ethyl-2-(4-(2-(cyclopropansulfonamido)pyrimidin-4-yl)-4-((5-(6-ethoxypyrazin-2-yl)pyridin-2-yl)carbamoyl)piperidin-1-yl)acetat;
*N*-(5-(6-Ethoxypyrazin-2-yl)pyridin-2-yl)-4-(2-(ethylsulfonamido)pyrimidin-4-yl)-1-(2-methoxyacetyl)piperidin-4-carboxamid;
*N*-(5-(6-Ethoxypyrazin-2-yl)pyridin-2-yl)-4-(2-(ethylsulfonamido)pyrimidin-4-yl)-1-(methylsulfonyl)piperidin-4-carboxamid;
*N*-(5-(6-Ethoxypyrazin-2-yl)pyridin-2-yl)-4-(2-(ethylsulfonamido)pyrimidin-4-yl)-1-(ethylsulfonyl)piperidin-4-carboxamid;
1-(Cyclopropylsulfonyl)-*N*-(5-(6-ethoxypyrazin-2-yl)pyridin-2-yl)-4-(2-(ethylsulfonamido)pyrimidin-4-yl)piperidin-4-carboxamid;
*N*-(5-(6-Ethoxypyrazin-2-yl)pyridin-2-yl)-4-(2-(ethylsulfonamido)pyrimidin-4-yl)-1-((1-methyl-1*H*-pyrazol-3-yl) sulfonyl)piperidin-4-carboxamid;
1-(2-(Cyclopropansulfonamido)pyrimidin-4-yl)-N-(5-(6-cyclopropylpyrazin-2-yl)pyridin-2-yl)-4-methoxycyclohexan-1-carboxamid (Diastereomer 1);
1-(2-(Cyclopropansulfonamido)pyrimidin-4-yl)-N-(5-(6-cyclopropylpyrazin-2-yl)pyridin-2-yl)-4-methoxycyclohexan-1-carboxamid (Diastereomer 2);
1-(2-(Cyclopropansulfonamido)pyrimidin-4-yl)-*N*-(5-(6-ethoxypyrazin-2-yl)pyridin-2-yl)-4-(pyrrolidin-1-yl)cyclohexan-1-carboxamid (Diastereomer 1);
1-(2-(Cyclopropansulfonamido)pyrimidin-4-yl)-*N*-(5-(6-ethoxypyrazin-2-yl)pyridin-2-yl)-4-(pyrrolidin-1-yl)cyclohexan-1-carboxamid (Diastereomer 2);
4-Amino-1-(2-(cyclopropansulfonamido)pyrimidin-4-yl)-N-(5-(6-ethoxypyrazin-2-yl)pyridin-2-yl)cyclohexan-1-carboxamid (Diastereomer 1);
1-(2-(Cyclopropansulfonamido)pyrimidin-4-yl)-N-(5-(6-ethoxypyrazin-2-yl)pyridin-2-yl)-4-morpholinocyclohexan-1-carboxamid (Diastereomer 1);
1-(2-(Cyclopropansulfonamido)pyrimidin-4-yl)-N-(5-(6-ethoxypyrazin-2-yl)pyridin-2-yl)-4-morpholinocyclohexan-1-carboxamid (Diastereomer 2);
1-(2-(Cyclopropansulfonamido)pyrimidin-4-yl)-N-(5-(6-ethoxypyrazin-2-yl)pyridin-2-yl)-4-(methyl(oxetan-3-yl) amino)cyclohexan-1-carboxamid (Diastereomer 1);
1-(2-(Cyclopropansulfonamido)pyrimidin-4-yl)-N-(5-(6-ethoxypyrazin-2-yl)pyridin-2-yl)-4-((2-methoxyethyl) (methyl)amino)cyclohexan-1-carboxamid (Diastereomer 1);
1-(2-(Cyclopropansulfonamido)pyrimidin-4-yl)-N-(5-(6-ethoxypyrazin-2-yl)pyridin-2-yl)-4-((2-methoxyethyl) (methyl)amino)cyclohexan-1-carboxamid (Diastereomer 2);
1-(2-(Cyclopropansulfonamido)pyrimidin-4-yl)-4-((2,2-difluorethyl) (methyl)amino)-N-(5-(6-ethoxypyrazin-2-yl) pyridin-2-yl)cyclohexan-1-carboxamid (Diastereomer 1); 1-(2-(Cyclopropansulfonamido)pyrimidin-4-yl)-N-(5-(6-ethoxypyrazin-2-yl)pyridin-2-yl)-4-(4-methylpiperazin-1-yl)cyclohexan-1-carboxamid (Diastereomer 1);
1-(2-(Cyclopropansulfonamido)pyrimidin-4-yl)-N-(5-(6-ethoxypyrazin-2-yl)pyridin-2-yl)-4-(4-methylpiperazin-1-yl)cyclohexan-1-carboxamid (Diastereomer 2);
4-(6-(Cyclopropansulfonamido)pyrazin-2-yl)-N-(5-(6-ethoxypyrazin-2-yl)pyridin-2-yl)-1-(methylsulfonyl)piperidin-4-carboxamid;
4-(4-(Cyclopropansulfonamido)pyrimidin-2-yl)-N-(5-(6-cyclopropylpyrazin-2-yl)pyridin-2-yl)-1-(methylsulfonyl) piperidin-4-carboxamid;
4-(4-(Cyclopropansulfonamido)pyrimidin-2-yl)-N-(5-(6-ethoxypyrazin-2-yl)pyridin-2-yl)-1-(methylsulfonyl)piperidin-4-carboxamid;
4-(4-(Cyclopropansulfonamido)pyrimidin-2-yl)-N-(5-(6-cyclopropylpyrazin-2-yl)pyridin-2-yl)-1-(ethylsulfonyl)piperidin-4-carboxamid;
4-(4-(Cyclopropansulfonamido)pyrimidin-2-yl)-N-(5-(6-ethoxypyrazin-2-yl)pyridin-2-yl)-1-(ethylsulfonyl)piperidin-4-carboxamid;
N-(4-(1-((2-Fluor-4-(pyridin-3-yl)phenyl)amino)-2-methylpropan-2-yl)thiazol-2-yl)cyclopropansulfonamid;
*N*-(4-(2-((4-(6-Ethoxypyrazin-2-yl)-2-fluorbenzyl)amino)propan-2-yl)thiazol-2-yl)cyclopropansulfonamid;
*N*-(4-(2-(((5-(6-Ethoxypyrazin-2-yl)pyridin-2-yl)methyl)amino)propan-2-yl)thiazol-2-yl)cyclopropansulfonamid;
und pharmazeutisch verträgliche Salze und/oder Solvate oder beliebige davon.

**15.** Pharmazeutische Zusammensetzung, umfassend eine Verbindung, ein pharmazeutisch verträgliches Salz und/oder ein Solvat davon nach einem der Ansprüche 3 bis 14.

**16.** Verbindung, das pharmazeutisch verträgliche Salz und/oder das Solvat davon nach einem der Ansprüche 3 bis 14 oder eine pharmazeutische Zusammensetzung nach Anspruch 15 zur Verwendung als Medikament.

**17.** Verbindung, das pharmazeutisch verträgliches Salz und/oder Solvat nach einem der Ansprüche 3 bis 14 oder eine pharmazeutische Zusammensetzung nach Anspruch 15 zur Verwendung bei der Behandlung eines hämatologischen Krebses.

**18.** Verbindung, das pharmazeutisch verträgliches Salz und/oder Solvat nach einem der Ansprüche 3 bis 14 oder eine pharmazeutische Zusammensetzung nach Anspruch 15 zur Verwendung bei der Behandlung eines nicht-hämatologischen Krebses.

**19.** Verbindung, die ausgewählt ist aus der Gruppe bestehend aus:

- Verbindung der Formel **(II-a):**

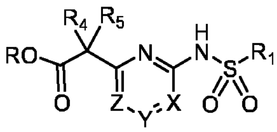

**(II-a)**

wobei X, Y und Z wie in Anspruch 1 definiert sind, R H, $C_{1-6}$-Alkyl (z. B. Methyl und Ethyl) oder Benzyl ist, $R_1$ $R_{1aa}$ ist und/oder $R_4$ und $R_5$ $R_{4aa}$ und $R_{5aa}$ sind; und R1aa, R4aa und R5aa wie in Anspruch 1 definiert sind; und Salze davon;

- eine Verbindung der Formel **(XX-a):**

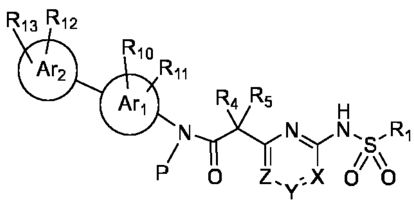

**(XX-a)**

wobei X, Y, Z, $R_{10}$, $R_{11}$, $R_{12}$, $R_{13}$, $Ar_1$ und $Ar_2$ wie in Anspruch 1 definiert sind, P eine Stickstoffschutzgruppe wie para-Methoxybenzyl ist, $R_1$ $R_{1aa}$ und/oder $R_4$ ist und $R_5$ $R_{4aa}$ und $R_{5aa}$ sind; und R1aa, R4aa und R5aa wie in Anspruch 1 definiert sind; und Salze davon;

- eine Verbindung der Formel **(XXIV-a):**

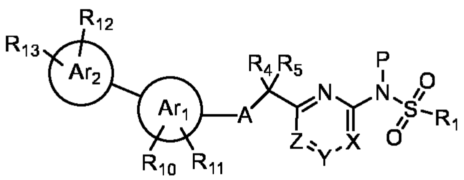

**(XXIV-a)**

wobei X, Y, Z, $R_{10}$, $R_{11}$, $R_{12}$, $R_{13}$, A, $Ar_1$ und $Ar_2$ wie in Anspruch 1 definiert sind, P eine Stickstoffschutzgruppe wie para-Methoxybenzyl ist, $R_1$ $R_{1aa}$ ist und/oder $R_4$ und $R_5$ $R_{4aa}$ und $R_{5aa}$ sind; und R1aa, R4aa und R5aa wie in Anspruch 1 definiert sind; und Salze davon;

- eine Verbindung der Formel **(XXXI-a):**

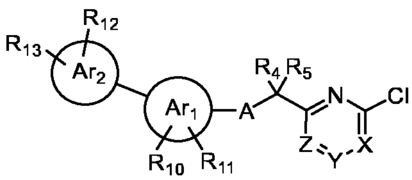

**(XXXI-a);**

wobei X, Y, Z, $R_{10}$, $R_{11}$, $R_{12}$, $R_{13}$, $Ar_1$ und $A_2$ wie in Anspruch 1 definiert sind, A $A_a$ ist und/oder $R_4$ und $R_5$ $R_{4aa}$ und $R_{5aa}$ sind; und R4aa und R5aa wie in Anspruch 1 definiert sind;
und Salze davon;

- eine Verbindung der Formel **(XXXXII-a):**

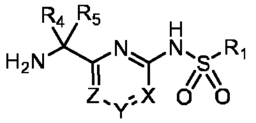

**(XXXXII-a);**

wobei X, Y und Z wie in Anspruch 1 definiert sind, $R_1$ $R_{1aa}$ ist und/oder $R_4$ und $R_5$ $R_{4aa}$ und $R_{5aa}$ sind; und R1aa, R4aa und R5aa wie in Anspruch 1 definiert sind;
und Salze davon;

- eine Verbindung der Formel **(LI-a):**

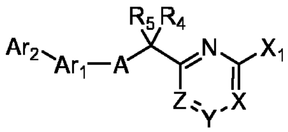

**(LI-a);**

wobei X, Y, Z, $Ar_1$ und $Ar_2$ in Anspruch 1 definiert sind, $X_1$ Cl oder Br ist, A $A_{aa}$ und/oder $R_4$ ist und $R_5$ $R_{4aa}$ und $R_{5aa}$ sind; und Aaa, R4aa und R5aa wie in Anspruch 1 definiert sind;
und Salze davon;

- Verbindung der Formel **(LXXIII-a):**

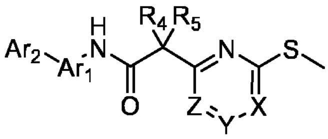

**(LXXIII-a)** ;

wobei X, Y, Z, $A_1$, $Ar_2$ in Anspruch 1 definiert sind, und $R_4$ und $R_5$ $R_{4aa}$ und $R_{5aa}$ sind; und R4aa und R5aa wie in Anspruch 1 definiert sind;
und Salze davon;

- eine Verbindung der Formel **(LXXIV-a):**

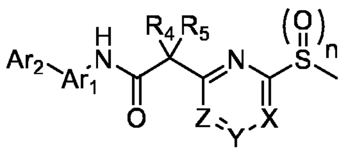

**(LXXIV-a)**

;

n = 1 Sulfoxid
n = 2 Sulfon
wobei X, Y, Z, $Ar_1$ und $Ar_2$ in Anspruch 1 definiert sind, und $R_4$ und $R_5$ $R_{4aa}$ und $R_{5aa}$ sind; und R4aa und R5aa wie in Anspruch 1 definiert sind;
und Salze davon;

- Verbindung der Formel **(LXXXIII-a):**

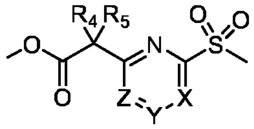

(LXXXIII-a) ;

wobei X, Y und Z wie in Anspruch 1 definiert sind, Alkyl $C_{1-4}$-Alkyl wie Methyl oder Ethyl, z. B. Methyl ist und $R_4$ und $R_5$ $R_{4aa}$ und $R_{5aa}$ sind; und R4aa und R5aa wie in Anspruch 1 definiert sind; und Salze davon;

- eine Verbindung der Formel **(XXXIV-a):**

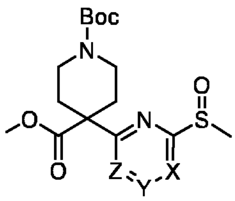

**(XXXIV-a);**

wobei X, Y und Z wie in Anspruch 1 definiert sind und Alkyl $C_{1-4}$-Alkyl wie Methyl oder Ethyl, z. B. Methyl, ist; und Salze davon;

- eine Verbindung der Formel **(LVIII-a):**

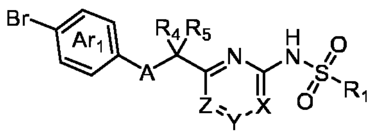

**(LVIII-a);**

wobei X, Y, Z und $Ar_1$ wie in Anspruch 1 definiert sind, A $A_a$ ist und/oder $R_1$ $R_{1aa}$ ist und/oder $R_4$ und $R_5$ $R_{4aa}$ und $R_{5aa}$ sind; und R1aa, R4aa und R5aa wie in Anspruch 1 definiert sind; und Salze davon;

- eine Verbindung der Formel **(XXXIII-a):**

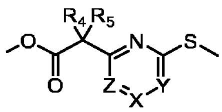

**(XXXIII-a);**

wobei X, Y und Z wie in Anspruch 1 definiert sind, Alkyl $C_{1-4-}$-Alkyl wie Methyl oder Ethyl, z. B. Methyl ist und $R_4$ und $R_5$ $R_{4aa}$ und $R_{5aa}$ sind; und R4aa und R5aa wie in Anspruch 1 definiert sind; und Salze davon;

- Verbindung der Formel (II-b),

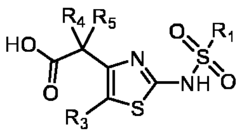

,

wobei $R_3$ wie in Anspruch 1 definiert ist, $R_1$ $R_{1ab}$ ist und/oder $R_4$ und $R_5$ $R_{4ab}$ und $R_{5ab}$ sind; und R1ab, R4ab und R5ab wie in Anspruch 1 definiert sind; und Salze davon;

- Verbindung der Formel (IV-b),

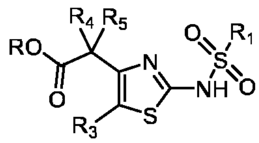

wobei $R_3$ wie in Anspruch 1 definiert ist, R $C_{1-6}$-Alkyl (z. B. Methyl, Ethyl) oder Benzyl ist, $R_1$ $R_{1ab}$ ist und/oder $R_4$ und $R_5$ $R_{4ab}$ und $R_{5ab}$ sind; und R1ab, R4ab und R5ab wie in Anspruch 1 definiert sind;
und Salze davon;

- Verbindung der Formel **(II-c):**

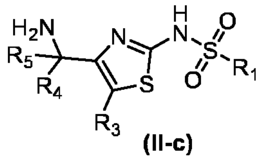

**(II-c)** ;

wobei $R_3$ wie in Anspruch 1 definiert ist, $R_1$ $R_{1ac}$ ist und/oder $R_4$ und $R_5$ $R_{4ac}$ und $R_{5ac}$ sind; und R1ac, R4ac und R5ac wie in Anspruch 1 definiert sind;
und Salze davon;

- eine Verbindung der Formel **(VIII-c):**

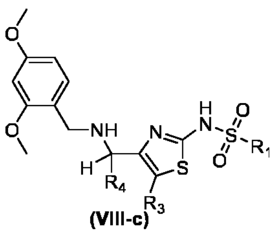

**(VIII-c)** ;

wobei $R_3$ und $R_4$ wie in Anspruch 1 definiert sind und $R_1$ $R_{1ac}$ ist; und R1ac wie in Anspruch 1 definiert sind;
und Salze davon.

## Revendications

**1.** Composé de formule (I) :

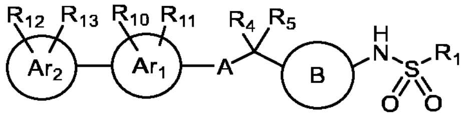

(I)

dans lequel le cycle B est choisi dans le groupe composé de :

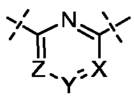

(B-a)

dans lequel X, Y et Z sont tels que définis ci-dessous ; et

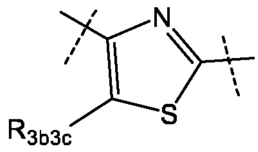

(B-bc) ;

dans lequel $R_{3b3c}$ est $R_{3b}$ ou $R_{3c}$ tel que défini ci-dessous ;

dans lequel lorsque B est (B-a), le composé de formule (I) est un composé de formule (I-a) :

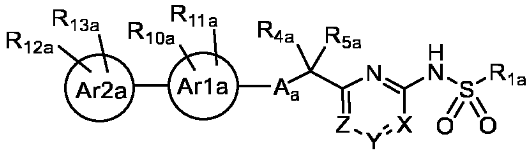

(I-a)

dans lequel :

$A_a$ est $A_{aa}$ ou $A_{ba}$ ;

dans lequel :

$A_{aa}$ est un agent de liaison amine ayant la structure suivante : -NH-, $-CH_2NH-$ ou $-NHCH_2-$ ;

$A_{ba}$ est un agent de liaison amide ayant la structure suivante : -C(=O)NH- ou -NHC(=O)- ;

X est N ou CH ;

Y est N ou $CR_{2a}$ ;

Z est N ou $CR_{3a}$ ;

à condition que lorsqu'au moins l'un de X ou Z est N, Y ne peut pas être N ;

$R_{2a}$ est H, halogéno, alkyle en $C_{1-2}$, Oalkyle en $C_{1-2}$, halogénoalkyle en $C_{1-2}$ ou Ohalogénoalkyle en $C_{1-2}$ ; et

$R_{3a}$ est H, halogéno, $CH_3$, $OCH_3$, $CF_3$ ou $OCF_3$ ;

dans lequel au moins l'un de $R_{2a}$ et $R_{3a}$ est H ;

$R_{1a}$ est $R_{1aa}$ ou $R_{1ba}$ ;

dans lequel :

$R_{1aa}$ est $NR_{32a}R_{33a}$ ;

$R_{1ba}$ est alkyle en $C_{1-5}$, alkylène en $C_{0-2}$cycloalkyle en $C_{3-5}$, lequel cycloalkyle est éventuellement substitué par $CH_3$, ou $CF_3$ ;

$R_{4a}$ et $R_{5a}$ sont $R_{4aa}$ et $R_{5aa}$, ou $R_{4ba}$ et $R_{5ba}$ ;

dans lequel :

$R_{4aa}$ et $R_{5aa}$ ensemble avec l'atome de carbone auquel ils sont attachés forment un cycloalkyle en $C_{3-6}$ qui est :

substitué par un ou deux substituants, chaque substituant étant choisi indépendamment dans le groupe composé d'alkyle en $C_{1-3}$, d'oxo, d'OH, d'alkyle en $C_{1-3}$OH, d'halogénoalkyle en $C_{1-3}$, d'alkylène en $C_{0-2}$cycloalkyle en $C_{3-6}$, d'alkylène en $C_{0-2}$hétérocycloalkyle en $C_{3-6}$, d'alkylène en $C_{1-3}$Oalkyle en $C_{1-3}$, d'halogéno, d'Ohalogénoalkyle en $C_{1-3}$, d'Oalkylène en $C_{0-2}$cycloalkyle en $C_{3-6}$, d'Oalkylène en $C_{0-2}$hétérocycloalkyle en $C_{3-6}$, d'Oalkyle en $C_{1-3}$ et de $NR_{21a}R_{22a}$ ; ou

un des carbones du cycloalkyle en $C_{3-6}$ est un centre spiro de sorte qu'un système de cycle spirocyclique est formé par le cycle cycloalkyle en $C_{3-6}$ et un autre cycle cycloalkyle en $C_{3-6}$ ou un cycle hétérocycloalkyle en $C_{3-6}$, et dans lequel le cycloalkyle en $C_{3-6}$ formé par $R_{4aa}$ et $R_{5aa}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou

$R_{4aa}$ et $R_{5aa}$ ensemble avec l'atome de carbone auquel ils sont attachés forment un hétérocycloalkyle en $C_{3-6}$ dans lequel un des carbones de l'hétérocycloalkyle en $C_{3-6}$ est un centre spiro de sorte qu'un système de cycle spirocyclique est formé par le cycle hétérocycloalkyle en $C_{3-6}$ et un autre cycle cycloalkyle en $C_{3-6}$ ou un cycle hétérocycloalkyle en $C_{3-6}$, et dans lequel l'hétérocycloalkyle en $C_{3-6}$ formé par $R_{4aa}$ et $R_{5aa}$ ensemble avec l'atome de carbone auquel ils sont attachés peuvent être substitués par un ou deux substituants, chaque substituant étant indépendamment

choisi dans le groupe composé d'alkyle en $C_{1-3}$ ou d'Oalkyle en $C_{1-3}$ ; ou

$R_{4aa}$ et $R_{5aa}$ ensemble avec l'atome de carbone auquel ils sont attachés forment un hétérocycloalkyle en $C_{3-6}$ comprenant un atome d'azote, dans lequel ledit atome d'azote est substitué par -$S(O)_2R_{29a}$ ; ou

$R_{4ba}$ et $R_{5ba}$ sont chacun indépendamment H, alkyle en $C_{1-6}$, alkyle en $C_{1-6}$OH, halogénoalkyle en $C_{1-6}$, alkylène en $C_{0-2}$cycloalkyle en $C_{3-6}$, alkylène en $C_{0-2}$hétérocycloalkyle en $C_{3-6}$, alkylène en $C_{1-3}$Oalkyle en $C_{1-3}$, ou $R_{4ba}$ et $R_{5ba}$ ensemble avec l'atome de carbone auquel ils sont attachés forment un cycloalkyle en $C_{3-6}$ un hétérocycloalkyle en $C_{3-6}$ ; et

lorsque $A_a$ est -NHC(=O)- ou -$NHCH_2$- :

$R_{4ba}$ et $R_{5ba}$ peuvent par ailleurs être choisis parmi halogéno, Ohalogénoalkyle en $C_{1-6}$, Oalkylène en $C_{0-2}$cycloalkyle en $C_{3-6}$, Oalkylène en $C_{0-2}$hétérocycloalkyle en $C_{3-6}$, Oalkyle en $C_{1-6}$ et $NR_{21a}R_{22a}$ ;

Ar1a est un aryle ou hétéroaryle à 6 chaînons ;

Ar2a est un aryle ou hétéroaryle à 6 chaînons et est attaché à Ar1a en position para par rapport au groupe $A_a$ ;

$R_{10a}$ est H, halogéno, alkyle en $C_{1-3}$, halogénoalkyle en $C_{1-2}$, Oalkyle en $C_{1-2}$, Ohalogénoalkyle en $C_{1-2}$ ou CN ;

$R_{11a}$ est H, F, Cl, alkyle en $C_{1-2}$, $CF_3$, $OCH_3$ ou CN ;

$R_{12a}$ est attaché à Ar2 en position ortho ou méta par rapport à Ar1a et $R_{12a}$ est H, halogéno, alkyle en $C_{1-4}$, alcényle en $C_{2-4}$, alkylène en $C_{0-2}$cycloalkyle en $C_{3-5}$, Oalkyle en $C_{1-4}$, Oalkylène en $C_{0-2}$cycloalkyle en $C_{3-5}$, halogénoalkyle en $C_{1-4}$, Ohalogénoalkyle en $C_{1-4}$, hydroxy, alkyle en $C_{1-4}$OH, $SO_2$alkyle en $C_{1-2}$, C(O)N(alkyle en $C_{1-2}$)$_2$, NHC(O)alkyle en $C_{1-3}$ ou $NR_{23a}R_{24a}$ ; et

lorsque $A_a$ est -NHC(=O)-, -NH- ou -$NHCH_2$- :

$R_{12a}$ peut par ailleurs être choisi parmi CN, $OCH_2CH_2N(CH_3)_2$ et un hétérocycloalkyle en $C_{3-6}$ comprenant un azote situé au niveau du point d'attachement à Ar2a, ou $R_{12a}$ ensemble avec un atome d'azote auquel il est attaché forme un N-oxyde ($N^+$-$O^-$) ;

$R_{13a}$ est H ou halogéno ;

$R_{21a}$ est H, alkyle en $C_{1-5}$, C(O)alkyle en $C_{1-5}$, C(O)Oalkyle en $C_{1-5}$, alkyle en $C_{1-3}$Oalkyle en $C_{1-2}$, halogénoalkyle en $C_{1-4}$ ou hétérocycloalkyle en $C_{4-6}$ ;

$R_{22a}$ est H ou $CH_3$ ;

$R_{23a}$ est H ou alkyle en $C_{1-2}$ ; et

$R_{24a}$ est H ou alkyle en $C_{1-2}$

$R_{29a}$ est alkyle en $C_{1-3}$, alkylène en $C_{0-2}$cycloalkyle en $C_{3-5}$, lequel cycloalkyle est éventuellement substitué par $CH_3$, $CF_3$, N(alkyle en $C_{1-3}$)$_2$, ou un hétéroaryle à 5 ou 6 chaînons dans lequel l'hétéroaryle à 5 ou 6 chaînons est éventuellement substitué par méthyle ;

$R_{32a}$ est alkyle en $C_{1-3}$ et $R_{33a}$ est alkyle en $C_{1-3}$ ; ou

$R_{32a}$ et $R_{33a}$ ensemble avec l'atome d'azote auquel ils sont attachés forment un hétérocycloalkyle en $C_{3-5}$ ;

dans lequel

$R_{1a}$ est $R_{1aa}$ ; et/ou

$R_{4a}$ et $R_{5a}$ sont $R_{4aa}$ et $R_{5aa}$ ; et/ou

$A_a$ est $A_{aa}$ ; et

dans lequel lorsque B est (B-bc) et $R_{3b3c}$ est $R_{3b}$, le composé de formule (I) est un composé de formule (I-b) :

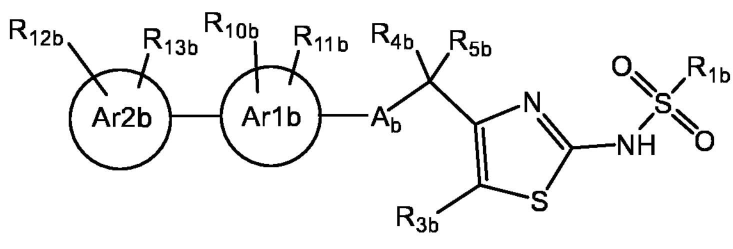

(I-b)

dans lequel :

$A_b$ est $A_{ab}$ ou $A_{bb}$ ;
dans lequel :

$A_{ab}$ est $-NR_{6b}CH_2-$ ou $-NR_{6b}-$ ;
$A_{bb}$ est $-NR_{6b}C(=O)-$ ;
$R_{1b}$ est $R_{1ab}$ ou $R_{1bb}$ ;
dans lequel :

$R_{1ab}$ est $NR_{32b}R_{33b}$ ;
$R_{1bb}$ est alkyle en $C_{1-5}$, alkylène en $C_{0-2}$cycloalkyle en $C_{3-5}$, lequel cycloalkyle est éventuellement substitué par $CH_3$, alkylène en $C_{1-3}$Oalklye en $C_{1-2}$, ou $CF_3$ ;
$R_{3b}$ est H, halogéno, $CH_3$, Oalkyle en $C_{1-2}$ $CF_3$ ;
ou $R_{3b}$ ensemble avec $R_{5bb}$ forme un cycloalkyle à 5 ou 6 chaînons ou hétérocycloalkyle contenant de l'oxygène à 5 ou 6 chaînons ;
$R_{4b}$ et $R_{5b}$ sont soit $R_{4ab}$ et $R_{5ab}$ soit $R_{4bb}$ et $R_{5bb}$ ;
dans lequel :
$R_{4ab}$ et $R_{5ab}$ ensemble avec l'atome de carbone auquel ils sont attachés forment un cycloalkyle en $C_{3-6}$ qui est :

substitué par un ou deux substituants, chaque substituant étant choisi indépendamment dans le groupe composé d'alkyle en $C_{1-3}$, d'oxo, d'OH, d'alkyle en $C_{1-3}$OH, d'halogénoalkyle en $C_{1-3}$, d'alkylène en $C_{0-2}$cycloalkyle en $C_{3-6}$, d'alkylène en $C_{0-2}$hétérocycloalkyle en $C_{3-6}$, d'alkylène en $C_{1-3}$Oalkyle en $C_{1-3}$, d'halogéno, d'Ohalogénoalkyle en $C_{1-3}$, d'Oalkylène en $C_{0-2}$cycloalkyle en $C_{3-6}$, d'Oalkylène en $C_{0-2}$hétérocycloalkyle en $C_{3-6}$, d'Oalkyle en $C_{1-3}$ et de $NR_{21b}R_{22b}$ ; ou
un des carbones du cycloalkyle en $C_{3-6}$ est un centre spiro de sorte qu'un système de cycle spirocyclique est formé par le cycle cycloalkyle en $C_{3-6}$ et un autre cycle cycloalkyle en $C_{3-6}$ ou un cycle hétérocycloalkyle en $C_{3-6}$, et dans lequel le cycloalkyle en $C_{3-6}$ formé par $R_{4ab}$ et $R_{5ab}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou
$R_{4ab}$ et $R_{5ab}$ ensemble avec l'atome de carbone auquel ils sont attachés forment un hétérocycloalkyle en $C_{3-6}$ dans lequel un des carbones de l'hétérocycloalkyle en $C_{3-6}$ est un centre spiro de sorte qu'un système de cycle spirocyclique est formé par le cycle hétérocycloalkyle en $C_{3-6}$ et un autre cycle cycloalkyle en $C_{3-6}$ ou un cycle hétérocycloalkyle en $C_{3-6}$, et dans lequel l'hétérocycloalkyle en $C_{3-6}$ formé par $R_{4ab}$ et $R_{5ab}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou
$R_{4ab}$ et $R_{5ab}$ ensemble avec l'atome de carbone auquel ils sont attachés forment un hétérocycloalkyle en $C_{3-6}$ comprenant un atome d'azote, dans lequel ledit atome d'azote est substitué par $-S(O)_2R_{29b}$ ; ou
$R_{4bb}$ et $R_{5bb}$ sont chacun indépendamment H, halogéno, alkyle en $C_{1-6}$, alkylène en $C_{0-2}$cycloalkyle en $C_{3-6}$, alkylène en $C_{0-2}$hétérocycloalkyle en $C_{3-6}$, Oalkyle en $C_{1-6}$, Oalkylène en $C_{0-2}$cycloakylle en $C_{3-6}$, alkylène en $C_{1-3}$Oalkyle en $C_{1-3}$, alkyle en $C_{1-6}$OH, halogénoalkyle en $C_{1-6}$, Ohalogénoalkyle en $C_{1-6}$ ou $NR_{21b}R_{22b}$,
ou $R_{4bb}$ est H et $R_{5bb}$ ensemble avec $R_{3b}$ forment un cycloalkyle à 5 ou 6 chaînons ou hétérocycloalkyle contenant de l'oxygène à 5 ou 6 chaînons,
ou $R_{4bb}$ et $R_{5bb}$ ensemble avec l'atome de carbone auquel ils sont attachés forment un cycloalkyle en $C_{3-6}$ ou hétérocycloalkyle en $C_{3-6}$,
ou $R_{4bb}$ est H et $R_{5bb}$ et $R_{6b}$ sont une chaîne alkylène en $C_{2-3}$ formant un cycle à 5 ou 6 chaînons ;
ou $R_{4bb}$ est O et $R_{5bb}$ est absent ;
$R_{6b}$ est H ou alkyle en $C_{1-3}$,

ou $R_{6b}$ ensemble avec $R_{11b}$ lorsqu'ils sont en position ortho par rapport au groupe $A_b$ sont une chaîne alkylène en $C_2$ formant un cycle à 5 chaînons, ou $R_{5bb}$ et $R_{6b}$ sont une chaîne alkylène en $C_{2-3}$ formant un cycle à 5 ou 6 chaînons et $R_{4bb}$ est H ;
Ar1b est un aryle ou hétéroaryle à 6 chaînons ;
Ar2b est un aryle ou hétéroaryle à 6 chaînons et est attaché à Ar1b en position para par rapport au groupe $A_b$ ;
$R_{10b}$ est H, halogéno, alkyle en $C_{1-3}$, Oalkyle en $C_{1-2}$, halogénoalkyle en $C_{1-2}$, Ohalogénoalkyle en $C_{1-2}$ ou CN ;
$R_{11b}$ est H, F, Cl, $CH_3$, éthyle, $OCH_3$, $CF_3$, $OCF_3$ ou CN,
ou $R_{11b}$, lorsqu'il est en position ortho par rapport au groupe $A_b$, ensemble avec $R_{6b}$ sont une chaîne alkylène en $C_2$ formant un cycle à 5 chaînons ;
$R_{12b}$ est attaché à Ar2b en position ortho ou méta par rapport à Ar1b et $R_{12b}$ est H, halogéno, alkyle en $C_{1-4}$, alcynyle en $C_{2-4}$, alkylène en $C_{0-2}$cycloakyle en $C_{3-5}$, Oalkyle en $C_{1-4}$, Oalkylène en $C_{0-2}$cycloalkyle en $C_{3-5}$, $OCH_2CH_2N(CH_3)_2$, OH, alkyle en $C_{1-4}$OH, CN, alkylène en $C_{1-3}$Oalkyle en $C_{1-3}$, halogénoalklye en $C_{1-4}$, Ohalogénoalkyle en $C_{1-4}$, C(=O)alkyle en $C_{1-2}$, $NR_{23b}R_{24b}$, $SO_2$alkyle en $C_{1-4}$, SOalkyle en $C_{1-4}$, Salkyle en $C_{1-4}$, SH, C(O) $N(CH_3)_2$, NHC(O)alkyle en $C_{1-3}$, hétérocycloalkyle en $C_{3-6}$ comprenant un azote situé au niveau du point d'attachement à Ar2b, ou $R_{12b}$ ensemble avec un atome d'azote auquel il est attaché forme un N-oxyde ($N^+$-$O^-$) ;
$R_{13b}$ est H, halogéno, $CH_3$ ou $OCH_3$ ;
$R_{21b}$ est H, alkyle en $C_{1-5}$, C(O)alkyle en $C_{1-5}$, C(O)Oalkyle en $C_{1-5}$, alkyle en $C_{1-3}$Oalkyle en $C_{1-2}$, halogénoalkyle en $C_{1-4}$ou hétérocycloalkyle en $C_{4-6}$ ;
$R_{22b}$ est H ou $CH_3$ ;
$R_{23b}$ est H ou alkyle en $C_{1-2}$ ;
$R_{24b}$ est H ou alkyle en $C_{1-2}$ ;
$R_{29b}$ est alkyle en $C_{1-3}$, alkylène en $C_{0-2}$cycloalkyle en $C_{3-5}$, lequel cycloalkyle est éventuellement substitué par $CH_3$, $CF_3$, N(alkyle en $C_{1-3})_2$, ou un hétéroaryle à 5 ou 6 chaînons, dans lequel l'hétéroaryle à 5 ou 6 chaînons est éventuellement substitué par méthyle ; et
$R_{32b}$ est alkyle en $C_{1-3}$ et $R_{33b}$ est alkyle en $C_{1-3}$ ; ou
$R_{32b}$ et $R_{33b}$ ensemble avec l'atome d'azote auquel ils sont attachés forment un hétérocycloalkyle en $C_{3-5}$ ;
dans lequel :

$R_{1b}$ est $R_{1ab}$ ; et/ou
$R_{4b}$ et $R_{5b}$ et $R_{4ab}$ et $R_{5ab}$ ; et/ou
$A_b$ est $A_{ab}$ ; ou
dans lequel lorsque B est (B-bc) et $R_{3b3c}$ est $R_{3c}$, le composé de formule (I) est un composé de formule (I-c) :

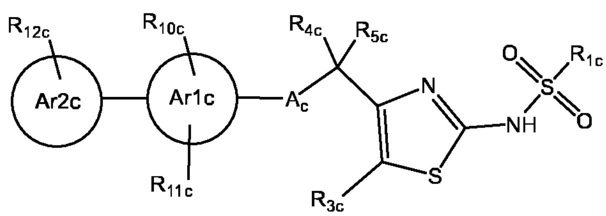

(I-c)

dans lequel :
$A_c$ est $A_{ac}$ ou $A_{bc}$ ;
dans lequel :

$A_{ac}$ est -$CH_2NR_{6c}$- ;
$A_{bc}$ est -C(=O)$NR_{6c}$- ;
$R_{1c}$ est $R_{1ac}$ ou $R_{1bc}$ ;
dans lequel :

$R_{1ac}$ est $NR_{32c}R_{33c}$ ;
$R_{1bc}$ est alkyle en $C_{1-5}$, alkylène en $C_{0-2}$cycloalkyle en $C_{3-5}$, lequel cycloalkyle est éventuellement substitué par $CH_3$, alkylène en $C_{1-3}$Oalkyle en $C_{1-2}$, ou $CF_3$ ;
$R_{3c}$ est H, $CH_3$, halogéno, Oalkyle en $C_{1-2}$ ou $CF_3$ ;
$R_{4c}$ et $R_{5c}$ sont soit $R_{4ac}$ et $R_{5ac}$ soit $R_{4bc}$ et $R_{5bc}$ ;
dans lequel :
$R_{4ac}$ et $R_{5ac}$ ensemble avec l'atome de carbone auquel ils sont attachés forment un cycloalkyle en $C_{3-6}$ qui est :

substitué par un ou deux substituants, chaque substituant étant choisi indépendamment dans le groupe composé d'alkyle en $C_{1-3}$, d'oxo, d'OH, d'alkyle en $C_{1-3}$OH, d'halogénoalkyle en $C_{1-3}$ , d'alkylène en $C_{0-2}$cycloalkyle en $C_{3-6}$, d'alkylène en $C_{0-2}$hétérocycloalkyle en $C_{3-6}$, d'alkylène en $C_{1-3}$Oalkyle en $C_{1-3}$, d'halogéno, d'Ohalogénoalkyle en $C_{1-3}$, d'Oalkylène en $C_{0-2}$cycloalkyle en $C_{3-6}$, d'Oalkylène en $C_{0-2}$hétérocycloalkyle en $C_{3-6}$, d'Oalkyle en $C_{1-3}$ et de $NR_{21c}R_{22c}$ ; ou
un des carbones du cycloalkyle en $C_{3-6}$ est un centre spiro de sorte qu'un système de cycle spirocyclique est formé par le cycle cycloalkyle en $C_{3-6}$ et un autre cycle cycloalkyle en $C_{3-6}$ ou un cycle hétérocycloalkyle en $C_{3-6}$, et dans lequel le cycloalkyle en $C_{3-6}$ formé par $R_{4ac}$ et $R_{5ac}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou
$R_{4ac}$ et $R_{5ac}$ ensemble avec l'atome de carbone auquel ils sont attachés forment un hétérocycloalkyle en $C_{3-6}$ dans lequel un des carbones de l'hétérocycloalkyle en $C_{3-6}$ est un centre spiro de sorte qu'un système de cycle spirocyclique est formé par le cycle hétérocycloalkyle en $C_{3-6}$ et un autre cycle cycloalkyle en $C_{3-6}$ ou un cycle hétérocycloalkyle en $C_{3-6}$, et dans lequel l'hétérocycloalkyle en $C_{3-6}$ formé par $R_{4ac}$ et $R_{5ac}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou
$R_{4ac}$ et $R_{5ac}$ ensemble avec l'atome de carbone auquel ils sont attachés forment un hétérocycloalkyle en $C_{3-6}$ comprenant un atome d'azote, dans lequel ledit atome d'azote est substitué par - $S(O)_2R_{29c}$ ; ou
$R_{4bc}$ et $R_{5bc}$ sont chacun indépendamment H, alkyle en $C_{1-6}$, alkylène en $C_{0-2}$cycloalkyle en $C_{3-6}$, alkylène en $C_{0-2}$hétérocycloalkyle en $C_{3-6}$, alkylène en $C_{1-3}$Oalkyle en $C_{1-3}$, alkyle en $C_{1-6}$OH ou halogénoalkyle en $C_{1-6}$,
ou $R_{4bc}$ et $R_{5bc}$ ensemble avec l'atome de carbone auquel ils sont attachés forment un cycle cycloalkyle en $C_{3-6}$ ou hétérocycloalkyle en $C_{3-6}$ ;
$R_{6c}$ est H ou alkyle en $C_{1-3}$ ;
Ar1c est un aryle ou hétéroaryle à 6 chaînons ;
Ar2c est un aryle ou hétéroaryle à 6 chaînons et est attaché à Ar1c en position para par rapport au groupe $A_c$ ;
$R_{10c}$ est H, halogéno, alkyle en $C_{1-3}$, Oalkyle en $C_{1-2}$, halogénoalkyle en $C_{1-2}$, Ohalogénoalkyle en $C_{1-2}$ ou CN ;
$R_{11c}$ est H, F, Cl, $CH_3$, éthyle, $OCH_3$, $CF_3$, $OCF_3$ ou CN ;

$R_{12c}$ est attaché à Ar2c en position méta ou ortho par rapport à Ar1c et $R_{12c}$ est H, halogéno, alkyle en $C_{1-4}$, alcynyle en $C_{2-4}$, C(=O)alkyle en $C_{1-2}$, alkylène en $C_{0-2}$cycloalkyle en $C_{3-5}$, Oalkyle en $C_{1-4}$, alkylène en $C_{1-3}$Oalkyle en $C_{1-3}$, halogénoalkyle en $C_{1-4}$, Ohalogénoalkyle en $C_{1-4}$, CN, Oalkylène en $C_{0-2}$cycloalkyle en $C_{3-5}$, $OCH_2CH_2N(CH_3)_2$, OH, alkyle en $C_{1-4}$OH, $NR_{23c}R_{24c}$, $SO_2CH_3$, $C(O)N(CH_3)_2$, NHC(O)alkyle en $C_{1-3}$, ou un hétérocycloalkyle en $C_{3-6}$ comprenant un azote situé au niveau du point d'attachement à Ar2c, ou $R_{12c}$ ensemble avec un atome d'azote auquel il est attaché forme un N-oxyde ($N^+$-$O^-$) ;
$R_{21c}$ est H, alkyle en $C_{1-5}$, C(O)alkyle en $C_{1-5}$, C(O)Oalkyle en $C_{1-5}$, alkyle en $C_{1-3}$Oalkyle en $C_{1-2}$, halogénoalkyle en $C_{1-4}$ou hétérocycloalkyle en $C_{4-6}$ ;
$R_{22c}$ est H ou $CH_3$ ;
$R_{23c}$ est H ou alkyle en $C_{1-2}$ ;
$R_{24c}$ est H ou alkyle en $C_{1-2}$ ;
$R_{29c}$ est alkyle en $C_{1-3}$, alkylène en $C_{0-2}$cycloalkyle en $C_{3-5}$, lequel cycloalkyle est éventuellement substitué par $CH_3$, $CF_3$, N(alkyle en $C_{1-3}$)$_2$, ou un hétéroaryle à 5 ou 6 chaînons, dans lequel l'hétéroaryle à 5 ou 6 chaînons est éventuellement substitué par méthyle ; et
$R_{32c}$ est alkyle en $C_{1-3}$ et $R_{33c}$ est alkyle en $C_{1-3}$ ; ou $R_{32c}$ et $R_{33c}$ ensemble avec l'atome d'azote auquel ils sont attachés forment un hétérocycloalkyle en $C_{3-5}$ ;
dans lequel :

$R_{1c}$ est $R_{1ac}$ ; et/ou
$R_{4c}$ et $R_{5c}$ sont $R_{4ac}$ et $R_{5ac}$ ; et/ou
$A_c$ est $A_{ac}$ ;
ou un sel et/ou solvate de celui-ci.

2. Composé, sel et/ou solvate de celui-ci selon la revendication 1, qui est un composé de formule (I) :

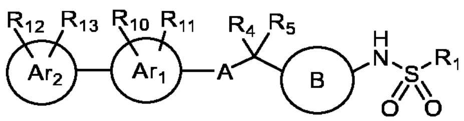
(I)

dans lequel le cycle B est choisi dans le groupe composé de :

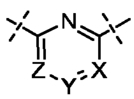
(B-a)

dans lequel X, Y et Z sont tels que définis ci-dessous ; et :

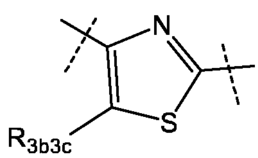
(B-bc) ;

dans lequel $R_{3b3c}$ est $R_{3b}$ ou $R_{3c}$ tel que défini ci-dessous ;

dans lequel lorsque B est (B-a), le composé de formule (I) est un composé de formule (I-a) :

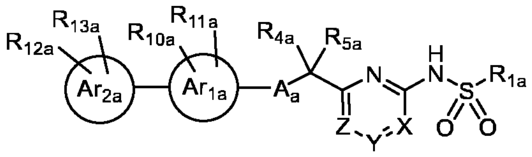

(I-a)

dans lequel :
$A_a$ est $A_{aa}$ ou $A_{ba}$ ;
dans lequel :

$A_{aa}$ est un agent de liaison amine ayant la structure suivante : -NH-, -$CH_2$NH- ou -NH$CH_2$- ;
$A_{ba}$ est un agent de liaison amide ayant la structure suivante : -C(=O)NH- ou -NHC(=O)- ;
X est N ou CH ;
Y est N ou $CR_{2a}$ ;
Z est N ou $CR_{3a}$ ;
à condition que lorsqu'au moins l'un de X ou Z est N, Y ne peut pas être N ;
$R_{2a}$ est H, halogéno, alkyle en $C_{1-2}$, Oalkyle en $C_{1-2}$, halogénoalkyle en $C_{1-2}$ ou Ohalogénoalkyle en $C_{1-2}$ ; et
$R_{3a}$ est H, halogéno, $CH_3$, $OCH_3$, $CF_3$ ou $OCF_3$ ;
dans lequel au moins l'un de $R_{2a}$ et $R_{3a}$ est H ;
$R_{1a}$ est $R_{1aa}$ ou $R_{1ba}$ ;
dans lequel :

$R_{1aa}$ est $NR_{32a}R_{33a}$ ;
$R_{1ba}$ est alkyle en $C_{1-5}$, alkylène en $C_{0-2}$cycloalkyle en $C_{3-5}$, lequel cycloalkyle est éventuellement substitué par $CH_3$, ou $CF_3$ ;
$R_{4a}$ et $R_{5a}$ sont $R_{4aa}$ et $R_{5aa}$, ou $R_{4ba}$ et $R_{5ba}$ ;
dans lequel :

$R_{4aa}$ et $R_{5aa}$ ensemble avec l'atome de carbone auquel ils sont attachés forment un cycloalkyle en $C_{3-6}$ qui est :

substitué par un ou deux substituants, chaque substituant étant choisi indépendamment dans le groupe composé d'alkyle en $C_{1-3}$, d'oxo, d'OH, d'alkyle en $C_{1-3}$OH, d'halogénoalkyle en $C_{1-3}$, d'alkylène en $C_{0-2}$cycloalkyle en $C_{3-6}$, d'alkylène en $C_{0-2}$hétérocycloalkyle en $C_{3-6}$, d'alkylène en $C_{1-3}$Oalkyle en $C_{1-3}$, d'halogéno, d'Ohalogénoalkyle en $C_{1-3}$, d'Oalkylène en $C_{0-2}$cycloalkyle en $C_{3-6}$, d'Oalylkène en $C_{0-2}$hétérocycloalkyle en $C_{3-6}$, d'Oalkyle en $C_{1-3}$ et de $NR_{21a}R_{22a}$ ; ou
un des carbones du cycloalkyle en $C_{3-6}$ est un centre spiro de sorte qu'un système de cycle spirocyclique est formé par le cycle cycloalkyle en $C_{3-6}$ et un autre cycle cycloalkyle en $C_{3-6}$ ou un cycle hétérocycloalkyle en $C_{3-6}$, et dans lequel le cycloalkyle en $C_{3-6}$ formé par $R_{4aa}$ et $R_{5aa}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou
$R_{4aa}$ et $R_{5aa}$ ensemble avec l'atome de carbone auquel ils sont attachés forment un hétérocycloalkyle en $C_{3-6}$ dans lequel un des carbones de l'hétérocycloalkyle en $C_{3-6}$ est un centre spiro de sorte qu'un système de cycle spirocyclique est formé par le cycle hétérocycloalkyle en $C_{3-6}$ et un autre cycle cycloalkyle en $C_{3-6}$ ou un cycle hétérocycloalkyle en $C_{3-6}$, et dans lequel l'hétérocycloalkyle en $C_{3-6}$ formé par $R_{4aa}$ et $R_{5aa}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou
$R_{4aa}$ et $R_{5aa}$ ensemble avec l'atome de carbone auquel ils sont attachés forment un hétérocycloalkyle en $C_{3-6}$ comprenant un atome d'azote, dans lequel ledit atome d'azote est substitué par - $S(O)_2R_{29a}$ ; ou
$R_{4ba}$ et $R_{5ba}$ sont chacun indépendamment H, alkyle en $C_{1-6}$, alkyle en $C_{1-6}$OH, halogénoalkyle en $C_{1-6}$, alkylène en $C_{0-2}$cycloalkyle en $C_{3-6}$, alkylène en $C_{0-2}$hétérocycloalkyle en $C_{3-6}$, alkylène en $C_{1-3}$Oalkyle en $C_{1-3}$, ou $R_{4ba}$ et $R_{5ba}$ ensemble avec l'atome de carbone auquel ils sont attachés forment un cycloalkyle en $C_{3-6}$ ou hétérocycloalkyle en $C_{3-6}$ ; et
lorsque $A_a$ est -NHC(=O)- ou -NH$CH_2$- :

$R_{4ba}$ et $R_{5ba}$ peuvent par ailleurs être choisis parmi halogéno, Ohalogénoalkyle en $C_{1-6}$, Oalkylène en $C_{0-2}$cycloalkyle en $C_{3-6}$, Oalkylène en $C_{0-2}$hétérocycloalkyle en $C_{3-6}$, Oalkyle en $C_{1-6}$ et

$NR_{21a}R_{22a}$ ;
Ar1a est un aryle ou hétéroaryle à 6 chaînons ;
Ar2a est un aryle ou hétéroaryle à 6 chaînons et est attaché à Ar1a en position para par rapport au groupe $A_a$ ;
$R_{10a}$ est H, halogéno, alkyle en $C_{1-3}$, halogénoalkyle en $C_{1-2}$, Oalkyle en $C_{1-2}$, Ohalogénoalkyle en $C_{1-2}$ ou CN ;
$R_{11a}$ est H, F, Cl, alkyle en $C_{1-2}$, $CF_3$, $OCH_3$ ou CN ;
$R_{12a}$ est attaché à Ar2 en position ortho ou méta par rapport à Ar1a et $R_{12a}$ est H, halogéno, alkyle en $C_{1-4}$, alcényle en $C_{2-4}$, alkylène en $C_{0-2}$cycloalkyle en $C_{3-5}$, Oalkyle en $C_{1-4}$, Oalkylène en $C_{0-2}$cycloalkyle en $C_{3-5}$, halogénoalkyle en $C_{1-4}$, Ohalogénoalkyle en $C_{1-4}$, hydroxy, alkyle en $C_{1-4}$OH, $SO_2$alkyle en $C_{1-2}$, C(O)N(alkyle en $C_{1-2}$)$_2$, NHC(O)alkyle en $C_{1-3}$ ou $NR_{23a}R_{24a}$ ; et
lorsque $A_a$ est -NHC(=O)-, -NH- ou -$NHCH_2$- :

$R_{12a}$ peut par ailleurs être choisi parmi CN, $OCH_2CH_2N(CH_3)_2$ et un hétérocycloalkyle en $C_{3-6}$ comprenant un azote situé au niveau du point d'attachement à Ar2a, ou $R_{12a}$ ensemble avec un atome d'azote auquel il est attaché forme un N-oxyde ($N^+$-$O^-$) ;
$R_{13a}$ est H ou halogéno ;
$R_{21a}$ est H, alkyle en $C_{1-5}$, C(O)alkyle en $C_{1-5}$, C(O)Oalkyle en $C_{1-5}$ ;
$R_{22a}$ est H ou $CH_3$ ;
$R_{23a}$ est H ou alkyle en $C_{1-2}$ ; et
$R_{24a}$ est H ou alkyle en $C_{1-2}$
$R_{29a}$ est alkyle en $C_{1-3}$, alkylène en $C_{0-2}$cycloalkyle en $C_{3-5}$, lequel cycloalkyle est éventuellement substitué par $CH_3$, ou $CF_3$ ;
$R_{32a}$ est alkyle en $C_{1-3}$ et $R_{33}$ est alkyle en $C_{1-3}$ ; ou
$R_{32a}$ et $R_{33a}$ ensemble avec l'atome d'azote auquel ils sont attachés forment un hétérocycloalkyle en $C_{3-5}$ ;
dans lequel
$R_{1a}$ est $R_{1aa}$ ; et/ou
$R_{4a}$ et $R_{5a}$ sont $R_{4aa}$ et $R_{5aa}$ ; et/ou
$A_a$ est $A_{aa}$ ; et
dans lequel lorsque B est (B-bc) et $R_{3b3c}$ est $R_{3b}$, le composé de formule (I) est un composé de formule (I-b) :

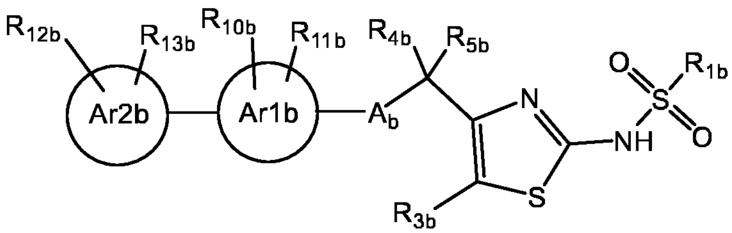
(I-b)

dans lequel :
$A_b$ est $A_{ab}$ ou $A_{bb}$ ;
dans lequel :

$A_{ab}$ est -$NR_{6b}CH_2$- ou -$NR_{6b}$- ;
$A_{bb}$ est -$NR_{6b}C(=O)$ - ;
$R_{1b}$ est $R_{1ab}$ ou $R_{1bb}$ ;
dans lequel :

$R_{1ab}$ est $NR_{32b}R_{33b}$ ;
$R_{1bb}$ est alkyle en $C_{1-5}$, alkylène en $C_{0-2}$cycloaklyle en $C_{3-5}$, lequel cycloalkyle est éventuellement substitué par $CH_3$, alkylène en $C_{1-3}$Oalkyle en $C_{1-2}$, ou $CF_3$ ;
$R_{3b}$ est H, halogéno, $CH_3$, Oalkyle en $C_{1-2}$ ou $CF_3$ ;
ou $R_{3b}$ ensemble avec $R_{5bb}$ forme un cycloalkyle à 5 ou 6 chaînons ou hétérocycloalkyle contenant de l'oxygène à 5 ou 6 chaînons ;
$R_{4b}$ et $R_{5b}$ sont soit $R_{4ab}$ et $R_{5ab}$ soit $R_{4bb}$ et $R_{5bb}$ ;

dans lequel :

$R_{4ab}$ et $R_{5ab}$ ensemble avec l'atome de carbone auquel ils sont attachés forment un cycloalkyle en $C_{3-6}$ qui est :

substitué par un ou deux substituants, chaque substituant étant choisi indépendamment dans le groupe composé d'alkyle en $C_{1-3}$, d'oxo, d'OH, d'alkyle en $C_{1-3}OH$, d'halogénoalkyle en $C_{1-3}$, d'alkylène en $C_{0-2}$cycloalkyle en $C_{3-6}$, d'alkylène en $C_{0-2}$hétérocycloalkyle en $C_{3-6}$, d'alkylène en $C_{1-3}$Oalkyle en $C_{1-3}$, d'halogéno, d'Ohalogénoalkyle en $C_{1-3}$, d'Oalkylène en $C_{0-2}$cycloalkyle en $C_{3-6}$, d'Oalkylène en $C_{0-2}$hétérocycloalkyle en $C_{3-6}$, d'Oalkyle en $C_{1-3}$ et de $NR_{21b}R_{22b}$ ; ou

un des carbones du cycloalkyle en $C_{3-6}$ est un centre spiro de sorte qu'un système de cycle spirocyclique est formé par le cycle cycloalkyle en $C_{3-6}$ et un autre cycle cycloalkyle en $C_{3-6}$ ou un cycle hétérocycloalkyle en $C_{3-6}$, et dans lequel le cycloalkyle en $C_{3-6}$ formé par $R_{4ab}$ et $R_{5ab}$ ensemble avec l'atome de carbone auquel ils sont attachés peuvent être substitués par un ou deux substituants, chaque substituant étant indépendamment choisi dans le groupe composé d'alkyle en $C_{1-3}$ ou d' Oalkyle en $C_{1-3}$ ; ou

$R_{4ab}$ et $R_{5ab}$ ensemble avec l'atome de carbone auquel ils sont attachés forment un hétérocycloalkyle en $C_{3-6}$ dans lequel un des carbones de l'hétérocycloalkyle en $C_{3-6}$ est un centre spiro de sorte qu'un système de cycle spirocyclique est formé par le cycle hétérocycloalkyle en $C_{3-6}$ et un autre cycle cycloalkyle en $C_{3-6}$ ou un cycle hétérocycloalkyle en $C_{3-6}$, et dans lequel l'hétérocycloalkyle en $C_{3-6}$ formé par $R_{4ab}$ et $R_{5ab}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou

$R_{4ab}$ et $R_{5ab}$ ensemble avec l'atome de carbone auquel ils sont attachés forment un hétérocycloalkyle en $C_{3-6}$ comprenant un atome d'azote, dans lequel ledit atome d'azote est substitué par - S $(O)_2R_{29b}$ ; ou

$R_{4bb}$ et $R_{5bb}$ sont chacun indépendamment H, halogéno, alkyle en $C_{1-6}$, alkylène en $C_{0-2}$cycloalkyle en $C_{3-6}$, alkylène en $C_{0-2}$hétérocycloalkyle en $C_{3-6}$, Oalkyle en $C_{1-6}$, Oalkylène en $C_{0-2}$cycloalkyle en $C_{3-6}$, alkylène en $C_{1-3}$Oalkyle en $C_{1-3}$, alkyle en $C_{1-6}OH$, halogénoalkyle en $C_{1-6}$, Ohalogénoalkyle en $C_{1-6}$ ou $NR_{21b}R_{22b}$,

ou $R_{4bb}$ est H et $R_{5bb}$ ensemble avec $R_{3b}$ forment un cycloalkyle à 5 ou 6 chaînons ou hétérocycloalkyle contenant de l'oxygène à 5 ou 6 chaînons,

ou $R_{4bb}$ et $R_{5bb}$ ensemble avec l'atome de carbone auquel ils sont attachés forment un cycloalkyle en $C_{3-6}$ ou hétérocycloalkyle en $C_{3-6}$,

ou $R_{4bb}$ est H et $R_{5bb}$ et $R_{6b}$ sont une chaîne alkylène en $C_{2-3}$ formant un cycle à 5 ou 6 chaînons ;

ou $R_{4bb}$ est O et $R_{5bb}$ est absent ;

$R_{6b}$ est H ou alkyle en $C_{1-3}$,

ou $R_{6b}$ ensemble avec $R_{11b}$ lorsqu'ils sont en position ortho par rapport au groupe $A_b$ sont une chaîne alkylène en $C_2$ formant un cycle à 5 chaînons,

ou $R_{5bb}$ et $R_{6b}$ sont une chaîne alkylène en $C_{2-3}$ formant un cycle à 5 ou 6 chaînons et $R_{4bb}$ est H ;

Ar1b est un aryle ou hétéroaryle à 6 chaînons ;

Ar2b est un aryle ou hétéroaryle à 6 chaînons et est attaché à Ar1b en position para par rapport au groupe $A_b$ ;

$R_{10b}$ est H, halogéno, alkyle en $C_{1-3}$, Oalkyle en $C_{1-2}$, halogénoalkyle en $C_{1-2}$, Ohalogénoalkyle en $C_{1-2}$ ou CN ;

$R_{11b}$ est H, F, Cl, $CH_3$, éthyle, $OCH_3$, $CF_3$, $OCF_3$ ou CN,

ou $R_{11b}$, lorsqu'il est en position ortho par rapport au groupe $A_b$, ensemble avec $R_{6b}$ sont une chaîne alkylène en $C_2$ formant un cycle à 5 chaînons,

$R_{12b}$ attaché à Ar2b en position ortho ou méta par rapport à Ar1b et $R_{12b}$ est H, halogéno, alkyle en $C_{1-4}$, alcynyle en $C_{2-4}$, alkylène en $C_{0-2}$cycloalkyle en $C_{3-5}$, Oalkyle en $C_{1-4}$, Oalkylène en $C_{0-2}$cycloalkyle en $C_{3-5}$, $OCH_2CH_2N(CH_3)_2$, OH,

alkyle en $C_{1-4}$OH, CN, alkylène en $C_{1-3}$Oalkyle en $C_{1-3}$, halogénoalkyle en $C_{1-4}$, Ohalogénoalkyle en $C_{1-4}$, C(=O)alkyle en $C_{1-2}$, $NR_{23b}R_{24b}$, $SO_2$alkyle en $C_{1-4}$, SOalkyle en $C_{1-4}$, Salkyle en $C_{1-4}$, SH, $C(O)N(CH_3)_2$, NHC(O)alkyle en $C_{1-3}$, hétérocycloalkyle en $C_{3-6}$ comprenant un azote situé au niveau du point d'attachement à Ar2b, ou $R_{12b}$ ensemble avec un atome d'azote auquel il est attaché forme un N-oxyde ($N^+$-$O^-$) ;
$R_{13b}$ est H, halogéno, $CH_3$ ou $OCH_3$ ;
$R_{21b}$ est H, alkyle en $C_{1-5}$, C(O)alkyle en $C_{1-5}$, C(O)Oalkyle en $C_{1-5}$ ;
$R_{22b}$ est H ou $CH_3$ ;
$R_{23b}$ est H ou alkyle en $C_{1-2}$ ;
$R_{24b}$ est H ou alkyle en $C_{1-2}$ ;
$R_{29b}$ est alkyle en $C_{1-3}$, alkylène en $C_{0-2}$cycloalkyle en $C_{3-5}$, lequel cycloalkyle est éventuellement substitué par $CH_3$, ou $CF_3$ ; et
$R_{32b}$ est alkyle en $C_{1-3}$ et $R_{33b}$ est alkyle en $C_{1-3}$ ; ou
$R_{32b}$ et $R_{33b}$ ensemble avec l'atome d'azote auquel ils sont attachés forment un hétérocycloalkyle en $C_{3-5}$ ;
dans lequel :

$R_{1b}$ est $R_{1ab}$ ; et/ou
$R_{4b}$ et $R_{5b}$ sont $R_{4ab}$ et $R_{5ab}$ ; et/ou
$A_b$ est $A_{ab}$ ; ou
dans lequel lorsque B est (B-bc) et $R_{3b3c}$ est $R_{3c}$, le composé de formule (I) est un composé de formule (I-c) :

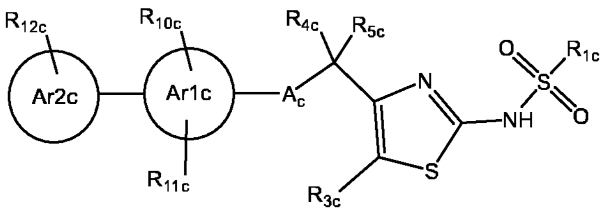

(I-c)

dans lequel :

$A_c$ est $A_{ac}$ ou $A_{bc}$ ;
$A_{ac}$ est $-CH_2NR_{6c}-$ ;
$A_{bc}$ est $-C(=O)NR_{6c}-$ ;
$R_{1c}$ est $R_{1ac}$ ou $R_{1bc}$ ;
dans lequel :

$R_{1ac}$ est $NR_{32c}R_{33c}$ ;
$R_{1bc}$ est alkyle en $C_{1-5}$, alkylène en $C_{0-2}$cycloalkyle en $C_{3-5}$, lequel cycloalkyle est éventuellement substitué par $CH_3$, alkylène en $C_{1-3}$Oalkyle en $C_{1-2}$, ou $CF_3$ ;
$R_{3c}$ est H, $CH_3$, halogéno, Oalkyle en $C_{1-2}$ ou $CF_3$ ;
$R_{4c}$ et $R_{5c}$ sont soit $R_{4ac}$ et $R_{5ac}$ soit $R_{4bc}$ et $R_{5bc}$ ;
dans lequel :
$R_{4ac}$ et $R_{5ac}$ ensemble avec l'atome de carbone auquel ils sont attachés forment un cycloalkyle en $C_{3-6}$ qui est :

substitué par un ou deux substituants, chaque substituant étant choisi indépendamment dans le groupe composé d'alkyle en $C_{1-3}$, d'oxo, d'OH, d'alkyle en $C_{1-3}$OH, d'halogénoalkyle en $C_{1-3}$, d'alkylène en $C_{0-2}$cycloalkyle en $C_{3-6}$, d'alkylène en $C_{0-2}$hétérocycloalkyle en $C_{3-6}$, d'alkylène en $C_{1-3}$Oalkyle en $C_{1-3}$, d'halogéno, d'Ohalogénoalkyle en $C_{1-3}$, d'Oalkylène en $C_{0-2}$cycloalkyle en $C_{3-6}$, d'Oalkylène en $C_{0-2}$hétérocycloalkyle en $C_{3-6}$, d'Oalkyle en $C_{1-3}$ et de $NR_{21c}R_{22c}$ ; ou

un des carbones du cycloalkyle en $C_{3-6}$ est un centre spiro de sorte qu'un système de cycle spirocyclique est formé par le cycle cycloalkyle en $C_{3-6}$ et un autre cycle cycloalkyle en $C_{3-6}$ou un cycle hétérocycloalkyle en $C_{3-6}$, et dans lequel le cycloalkyle en $C_{3-6}$ formé par $R_{4ac}$ et $R_{5ac}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou

$R_{4ac}$ et $R_{5ac}$ ensemble avec l'atome de carbone auquel ils sont attachés forment un hétérocycloalkyle en $C_{3-6}$ dans lequel un des carbones de l'hétérocycloalkyle en $C_{3-6}$ est un centre spiro de sorte qu'un système de cycle spirocyclique est formé par le cycle hétérocycloalkyle en $C_{3-6}$ et un autre cycle cycloalkyle en $C_{3-6}$ ou un cycle hétérocycloalkyle en $C_{3-6}$, et dans lequel l'hétérocycloalkyle en $C_{3-6}$ formé par $R_{4ac}$ et $R_{5ac}$ ensemble avec l'atome de carbone auquel ils sont attachés peuvent être substitués par un ou deux substituants, chaque substituant étant indépendamment choisi dans le groupe composé d'alkyle en $C_{1-3}$ ou d'Oalkyle en $C_{1-3}$ ; ou

$R_{4ac}$ et $R_{5ac}$ ensemble avec l'atome de carbone auquel ils sont attachés forment un hétérocycloalkyle en $C_{3-6}$ comprenant un atome d'azote, dans lequel ledit atome d'azote est substitué par - $S(O)_2R_{29c}$ ; ou

$R_{4bc}$ et $R_{5bc}$ sont chacun indépendamment H, alkyle en $C_{1-6}$, alkylène en $C_{0-2}$cycloalkyle en $C_{3-6}$, alkylène en $C_{0-2}$hétérocycloalkyle en $C_{3-6}$, alkylène en $C_{1-3}$Oalkyle en $C_{1-3}$, alkyle en $C_{1-6}$OH ou halogénoalkyle en $C_{1-6}$,

ou $R_{4bc}$ et $R_{5bc}$ ensemble avec l'atome de carbone auquel ils sont attachés forment un cycle cycloalkyle en $C_{3-6}$ ou hétérocycloalkyle en $C_{3-6}$ ;

$R_{6c}$ est H ou alkyle en $C_{1-3}$ ;

Ar1c est un aryle ou hétéroaryle à 6 chaînons ;

Ar2c est un aryle ou hétéroaryle à 6 chaînons et est attaché à Ar1c en position para par rapport au groupe $A_c$ ;

$R_{10c}$ est H, halogéno, alkyle en $C_{1-3}$, Oalkyle en $C_{1-2}$, halogénoalkyle en $C_{1-2}$, Ohalogénoalkyle en $C_{1-2}$ ou CN ;

$R_{11c}$ est H, F, Cl, $CH_3$, éthyle, $OCH_3$, $CF_3$, $OCF_3$ ou CN ;

$R_{12c}$ est attaché à Ar2c en position méta ou ortho par rapport à Ar1c et $R_{12c}$ est H, halogéno, alkyle en $C_{1-4}$, alcynyle en $C_{2-4}$, C(=O)alkyle en $C_{1-2}$, alkylène en $C_{0-2}$cycloalkyle en $C_{3-5}$, Oalkyle en $C_{1-4}$, alkylène en $C_{1-3}$Oalkyle en $C_{1-3}$, halogénoalkyle en $C_{1-4}$, Ohalogénoalkyle en $C_{1-4}$, CN, Oalkylène en $C_{0-2}$cycloalkyle en $C_{3-5}$, $OCH_2CH_2N(CH_3)_2$, OH, alkyle en $C_{1-4}$OH, $NR_{23c}R_{24c}$, $SO_2CH_3$, $C(O)N(CH_3)_2$, NHC(O)alkyle en $C_{1-3}$, ou un hétérocycloalkyle en $C_{3-6}$ comprenant un azote situé au niveau du point d'attachement à Ar2c, ou $R_{12c}$ ensemble avec un atome d'azote auquel il est attaché forme un N-oxyde ($N^+$-$O^-$) ;

$R_{21c}$ est H, alkyle en $C_{1-5}$, C(O)alkyle en $C_{1-5}$, C(O)Oalkyle en $C_{1-5}$ ;

$R_{22c}$ est H ou $CH_3$ ;

$R_{23c}$ est H ou alkyle en $C_{1-2}$ ;

$R_{24c}$ est H ou alkyle en $C_{1-2}$ ;

$R_{29c}$ est alkyle en $C_{1-3}$, alkylène en $C_{0-2}$cycloalkyle en $C_{3-5}$, lequel cycloalkyle est éventuellement substitué par $CH_3$, ou $CF_3$ ; et

$R_{32c}$ est alkyle en $C_{1-3}$ et $R_{33c}$ est alkyle en $C_{1-3}$ ; ou

$R_{32c}$ et $R_{33c}$ ensemble avec l'atome d'azote auquel ils sont attachés forment un hétérocycloalkyle en $C_{3-5}$ ;
dans lequel :

$R_{1c}$ est $R_{1ac}$ ; et/ou
$R_{4c}$ et $R_{5c}$ sont $R_{4ac}$ et $R_{5ac}$ ; et/ou
$A_c$ est $A_{ac}$ ;
ou un sel et/ou solvate de celui-ci.

**3.** Composé, sel et/ou solvate de celui-ci selon la revendication 1 ou 2, qui est le composé ou un sel et/ou solvate pharmaceutiquement acceptables de celui-ci.

**4.** Sel et solvate pharmaceutiquement acceptables d'un composé de formule (I) selon la revendication 3.

**5.** Sel pharmaceutiquement acceptable d'un composé de formule (I) selon la revendication 3.

**6.** Solvate pharmaceutiquement acceptable d'un composé de formule (I) selon la revendication **3.**

**7.** Composé de formule (I) selon la revendication 1 ou 2.

**8.** Composé, sel et/ou solvate de celui-ci selon l'une quelconque des revendications 1 à 7, dans lesquels le composé de formule (I) est un composé de formule (I-a).

**9.** Composé, sel et/ou solvate de celui-ci selon l'une quelconque des revendications 1 à 7, dans lesquels le composé de formule (I) est un composé de formule (I-b).

**10.** Composé, sel et/ou solvate de celui-ci selon l'une quelconque des revendications 1 à 7, dans lesquels le composé de formule (I) est un composé de formule (I-c).

**11.** Composé, sel et/ou solvate de celui-ci selon l'une quelconque des revendications 8 à 10, dans lesquels $R_{1a}$ est $R_{1aa}$, $R_{1b}$ est $R_{1ab}$ ou $R_{1c}$ est $R_{1ac}$.

**12.** Composé, sel et/ou solvate de celui-ci selon l'une quelconque des revendications 8 à 11, dans lesquels $R_{4a}$ et $R_{5a}$ sont $R_{4aa}$ et $R_{5aa}$, $R_{4b}$ et $R_{5b}$ sont $R_{4ab}$ et $R_{5ab}$ ou $R_{4c}$ et $R_{5c}$ sont $R_{4ac}$ et $R_{5ac}$ .

**13.** Composé, sel et/ou solvate de celui-ci selon l'une quelconque des revendications 8 à 12, dans lesquels $A_a$ est $A_{aa}$, $A_b$ est $A_{ab}$ ou $A_c$ est $A_{ac}$.

**14.** Composé, sel et/ou solvate de celui-ci selon la revendication 1 qui est choisi dans le groupe composé de :

1-(2-(cyclopropanesulfonamido)pyrimidin-4-yl)-*N*-(5-(6-éthoxypyrazin-2-yl)pyridin-2-yl)-4-oxocyclohexanecarboxamide ;
1-(2-(cyclopropanesulfonamido)pyrimidin-4-yl)-*N*-(5-(6-éthoxypyrazin-2-yl)pyridin-2-yl)-4-hydroxycyclohexanecarboxamide ;
1-(2-(cyclopropanesulfonamido)pyrimidin-4-yl)-*N*-(5-(6-éthoxypyrazin-2-yl)pyridin-2-yl)-4-hydroxycyclohexanecarboxamide (diastéréomère 1) ;
1-(2-(cyclopropanesulfonamido)pyrimidin-4-yl)-*N*-(5-(6-éthoxypyrazin-2-yl)pyridin-2-yl)-4-hydroxycyclohexanecarboxamide (diastéréomère 2) ;
1-(2-(cyclopropanesulfonamido)pyrimidin-4-yl)-4-(diméthylamino)-*N*-(5-(6-éthoxypyrazin-2-yl)pyridin-2-yl)cyclohexane-1-carboxamide ;
1-(2-(cyclopropanesulfonamido)pyrimidin-4-yl)-4-(diméthylamino)-*N*-(5-(6-éthoxypyrazin-2-yl)pyridin-2-yl)cyclohexane-1-carboxamide (diastéréomère 1) ;
1-(2-(cyclopropanesulfonamido)pyrimidin-4-yl)-4-(diméthylamino)-*N*-(5-(6-éthoxypyrazin-2-yl)pyridin-2-yl)cyclohexane-1-carboxamide (diastéréomère 2) ;
*N*-(4-(1-((4-(6-éthoxypyrazin-2-yl)-2-fluorobenzyl)amino)propyl)pyrimidin-2-yl)cyclopropanesulfonamide ;
1-(2-(cyclopropanesulfonamido)pyrimidin-4-yl)-*N*-(5-(6-éthoxypyrazin-2-yl)pyridin-2-yl)-4,4-difluorocyclohexane-1-carboxamide ;
8-(2-(cyclopropanesulfonamido)pyrimidin-4-yl)-*N*-(5-(6-éthoxypyrazin-2-yl)pyridin-2-yl)-1,4-dioxaspiro[4.5]décane-8-carboxamide ;
4-(2-((*N*,*N*-diméthylsulfamoyl)amino)pyrimidin-4-yl)-*N*-(5-(6-éthoxypyrazin-2-yl)pyridin-2-yl)tétrahydro-2*H*-pyrane-4-carboxamide ;
4-(2-(cyclopropanesulfonamido)pyrimidin-4-yl)-*N*-(5-(6-éthoxypyrazin-2-yl)pyridin-2-yl)-1-(méthylsulfonyl)pipéridine-4-carboxamide ;
N-(4-(1-(((5-(6-éthoxypyrazin-2-yl)pyridin-2-yl)méthyl)amino)cyclopropyl)pyrimidin-2-yl)cyclopropanesulfonamide ;
N-(4-(1-((4-(6-éthoxypyrazin-2-yl)-2-fluorobenzyl)amino)cyclopropyl)pyrimidin-2-yl)cyclopropanesulfonamide ;
N-(4-(4-(((4-(6-éthoxypyrazin-2-yl)phényl)amino)méthyl)tetrahydro-2H-pyran-4-yl)pyrimidin-2-yl)cyclopropanesulfonamide ;
2-(2-(cyclopropanesulfonamido)pyrimidin-4-yl)-*N*-(5-(6-éthoxypyrazin-2-yl)pyridin-2-yl)-5,8-dioxaspiro[3.4]octane-2-carboxamide ;
1-(2-(cyclopropanesulfonamido)pyrimidin-4-yl)-*N*-(5-(6-éthoxypyrazin-2-yl)pyridin-2-yl)-4-méthoxycyclohexane-1-carboxamide ;
*N*-(4-(1-((4-(6-éthoxypyrazin-2-yl)phényl)amino)propyl)pyrimidin-2-yl)cyclopropanesulfonamidearboxamide ;
4-(2-(cyclopropanesulfonamido)pyrimidin-4-yl)-*N*-(5-(6-éthoxypyrazin-2-yl)pyridin-2-yl)-1-(2-méthoxyacetyl)pipéridine-4-carboxamide ;
4-(2-(cyclopropanesulfonamido)pyrimidin-4-yl)-*N*-(5-(6-éthoxypyrazin-2-yl)pyridin-2-yl)-1-(éthylsulfonyl)pipéridine-4-carboxamide ;
4-(2-(cyclopropanesulfonamido)pyrimidin-4-yl)-1-(cyclopropylsulfonyl)-*N*-(5-(6-éthoxypyrazin-2-yl)pyridin-2-yl) pipéridine-4-carboxamide ;
4-(2-(cyclopropanesulfonamido)pyrimidin-4-yl)-1-(*N*,*N*-diméthylsulfamoyl)-*N*-(5-(6-éthoxypyrazin-2-yl)pyridin-2-yl)pipéridine-4-carboxamide ;
4-(2-(cyclopropanesulfonamido)pyrimidin-4-yl)-*N*-(5-(6-éthoxypyrazin-2-yl)pyridin-2-yl)-1-((trifluorométhyl)sulfonyl)pipéridin-4-carboxamide ;
4-(2-(cyclopropanesulfonamido)pyrimidin-4-yl)-*N*-(5-(6-éthoxypyrazin-2-yl)pyridin-2-yl)-1-((1-méthyl-1*H*-pyrazol-3-yl)sulfonyl)pipéridine-4-carboxamide ;
1-(cyanométhyl)-4-(2-(cyclopropanesulfonamido)pyrimidin-4-yl)-N-(5-(6-éthoxypyrazin-2-yl)pyridin-2-yl)pipéridine-4-carboxamide ;
2-(4-(2-(cyclopropanesulfonamido)pyrimidin-4-yl)-4-((5-(6-éthoxypyrazin-2-yl)pyridin-2-yl)carbamoyl)pipéridin-1-yl)acétate d'éthyle ;
*N*-(5-(6-éthoxypyrazin-2-yl)pyridin-2-yl)-4-(2-(éthylsulfonamido)pyrimidin-4-yl)-1-(2-méthoxyacétyl)pipéridine-4-carboxamide ;
*N*-(5-(6-éthoxypyrazin-2-yl)pyridin-2-yl)-4-(2-(éthylsulfonamido)pyrimidin-4-yl)-1-(méthylsulfonyl)pipéridine-4-carboxamide ;
*N*-(5-(6-éthoxypyrazin-2-yl)pyridin-2-yl)-4-(2-(éthylsulfonamido)pyrimidin-4-yl)-1-(éthylsulfonyl)pipéridine-4-carboxamide ;
1-(cyclopropylsulfonyl)-*N*-(5-(6-éthoxypyrazin-2-yl)pyridin-2-yl)-4-(2-(éthylsulfonamido)pyrimidin-4-yl)pipéridine-4-carboxamide ;
*N*-(5-(6-éthoxypyrazin-2-yl)pyridin-2-yl)-4-(2-(éthylsulfonamido)pyrimidin-4-yl)-1-((1-méthyl-1*H*-pyrazol-3-yl) sulfonyl)pipéridine-4-carboxamide ;
1-(2-(cyclopropanesulfonamido)pyrimidin-4-yl)-N-(5-(6-cyclopropylpyrazin-2-yl)pyridin-2-yl)-4-méthoxycyclohexane-1-carboxamide (diastéréomère 1);
1-(2-(cyclopropanesulfonamido)pyrimidin-4-yl)-N-(5-(6-cyclopropylpyrazin-2-yl)pyridin-2-yl)-4-méthoxycyclohexane-1-carboxamide (diastéréomère 2) ;
1-(2-(cyclopropanesulfonamido)pyrimidin-4-yl)-*N*-(5-(6-éthoxypyrazin-2-yl)pyridin-2-yl)-4-(pyrrolidin-1-yl)cyclohexane-1-carboxamide (diastéréomère 1) ;
1-(2-(cyclopropanesulfonamido)pyrimidin-4-yl)-*N*-(5-(6-éthoxypyrazin-2-yl)pyridin-2-yl)-4-(pyrrolidin-1-yl)cyclohexane-1-carboxamide (diastéréomère 2) ;
4-amino-1-(2-(cyclopropanesulfonamido)pyrimidin-4-yl)-N-(5-(6-éthoxypyrazin-2-yl)pyridin-2-yl)cyclohexane-1-carboxamide (diastéréomère 1) ;
1-(2-(cyclopropanesulfonamido)pyrimidin-4-yl)-N-(5-(6-éthoxypyrazin-2-yl)pyridin-2-yl)-4-morpholincyclohexane-1-carboxamide (diastéréomère 1) ;
1-(2-(cyclopropanesulfonamido)pyrimidin-4-yl)-N-(5-(6-éthoxypyrazin-2-yl)pyridin-2-yl)-4-morpholinocyclohexane-1-carboxamide (diastéréomère 2) ;
1-(2-(cyclopropanesulfonamido)pyrimidin-4-yl)-N-(5-(6-éthoxypyrazin-2-yl)pyridin-2-yl)-4-(méthyl(oxétan-3-yl) amino)cyclohexane-1-carboxamide (diastéréomère 1) ;
1-(2-(cyclopropanesulfonamido)pyrimidin-4-yl)-N-(5-(6- éthoxypyrazin-2-yl)pyridin-2-yl)-4-((2-

méthoxyéthyl) (méthyl)amino)cyclohexane-1-carboxamide (diastéréomère 1) ;
1-(2-(cyclopropanesulfonamido)pyrimidin-4-yl)-N-(5-(6- éthoxypyrazin-2-yl)pyridin-2-yl)-4-((2-méthoxyéthyl) (méthyl)amino)cyclohexane-1-carboxamide (diastéréomère 2) ;
1-(2-(cyclopropanesulfonamido)pyrimidin-4-yl)-4-((2,2-difluoroéthyl) (méthyl)amino)-N-(5-(6-éthoxypyrazin-2-yl)pyridin-2-yl)cyclohexane-1-carboxamide (diastéréomère 1) ;
1-(2-(cyclopropanesulfonamido)pyrimidin-4-yl)-N-(5-(6-éthoxypyrazin-2-yl)pyridin-2-yl)-4-(4-méthylpiperazin-1-yl)cyclohexane-1-carboxamide (diastéréomère 1) ;
1-(2-(cyclopropanesulfonamido)pyrimidin-4-yl)-N-(5-(6-éthoxypyrazin-2-yl)pyridin-2-yl)-4-(4-méthylpiperazin-1-yl)cyclohexane-1-carboxamide (diastéréomère 2) ;
4-(6-(cyclopropanesulfonamido)pyrazin-2-yl)-N-(5-(6-éthoxypyrazin-2-yl)pyridin-2-yl)-1-(méthylsulfonyl)pipéridine-4-carboxamide ;
4-(4-(cyclopropanesulfonamido)pyrimidin-2-yl)-N-(5-(6-cyclopropylpyrazin-2-yl)pyridin-2-yl)-1-(méthylsulfonyl) pipéridine-4-carboxamide ;
4-(4-(cyclopropanesulfonamido)pyrimidin-2-yl)-N-(5-(6-éthoxypyrazin-2-yl)pyridin-2-yl)-1-(méthylsulfonyl)pipéridine-4-carboxamide ;
4-(4-(cyclopropanesulfonamido)pyrimidin-2-yl)-N-(5-(6-cyclopropylpyrazin-2-yl)pyridin-2-yl)-1-(éthylsulfonyl) pipéridine-4-carboxamide ;
4-(4-(cyclopropanesulfonamido)pyrimidin-2-yl)-N-(5-(6-éthoxypyrazin-2-yl)pyridin-2-yl)-1-(éthylsulfonyl)pipéridine-4-carboxamide ;
N-(4-(1-((2-fluoro-4-(pyridin-3-yl)phényl)amino)-2-méthylpropan-2-yl)thiazol-2-yl)cyclopropanesulfonamide ;
*N*-(4-(2-((4-(6-éthoxypyrazin-2-yl)-2-fluorobenzyl)amino)propan-2-yl)thiazol-2-yl)cyclopropanesulfonamide ;
*N*-(4-(2-(((5-(6-éthoxypyrazin-2-yl)pyridin-2-yl)méthyl)amino)propan-2-yl)thiazol-2-yl)cyclopropanesulfonamide ;
et des sels et/ou solvates pharmaceutiquement acceptables de l'un quelconque de ceux-ci.

**15.** Composition pharmaceutique comprenant un composé, un sel et/ou solvate pharmaceutiquement acceptables de celui-ci selon l'une quelconque des revendications 3 à 14.

**16.** Composé, sel et/ou solvate pharmaceutiquement acceptables de celui-ci selon l'une quelconque des revendications 3 à 14 ou composition pharmaceutique selon la revendication 15 pour une utilisation en tant que médicament.

**17.** Composé, sel et/ou solvate pharmaceutiquement acceptables de celui-ci selon l'une quelconque des revendications 3 à 14 ou composition pharmaceutique selon la revendication 15, pour une utilisation dans le traitement d'un cancer hématologique.

**18.** Composé, sel et/ou solvate pharmaceutiquement acceptables de celui-ci selon l'une quelconque des revendications 3 à 14 ou composition pharmaceutique selon la revendication 15, pour une utilisation dans le traitement d'un cancer non hématologique.

**19.** Composé qui est choisi dans le groupe composé :

- d'un composé de formule **(II-a)** :

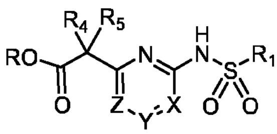

**(II-a)**

dans lequel X, Y et Z sont tels que définis dans la revendication 1, R est H, alkyle en $C_{1-6}$ (par exemple méthyle et éthyle) ou benzyle, $R_1$ est $R_{1aa}$ et/ou $R_4$ et $R_5$ sont $R_{4aa}$ et $R_{5aa}$ ; et R1aa, R4aa et R5aa sont tels que définis dans la revendication 1 ;
et des sels de celui-ci ;

- d'un composé de formule **(XX-a)** :

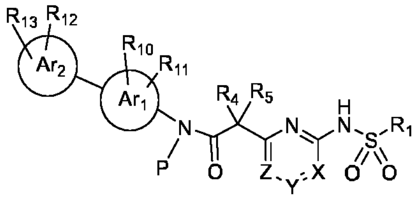

**(XX-a)**

dans lequel X, Y, Z, $R_{10}$, $R_{11}$, $R_{12}$, $R_{13}$, $Ar_1$ et $Ar_2$ sont tels que définis dans la revendication 1, P est un groupe protecteur d'azote tel que para-méthoxybenzyle, $R_1$ est $R_{1aa}$ et/ou $R_4$ et $R_5$ sont $R_{4aa}$ et $R_{5aa}$ ; et R1aa, R4aa et R5aa sont tels que définis dans la revendication 1 ;
et des sels de celui-ci ;

- d'un composé de formule **(XXIV-a)** :

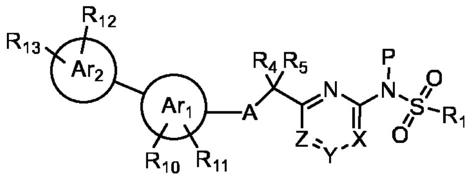

**(XXIV-a)**

dans lequel X, Y, Z, $R_{10}$, $R_{11}$, $R_{12}$, $R_{13}$, A, $Ar_1$ et $Ar_2$ sont tels que définis dans la revendication 1, P est un groupe protecteur d'azote tel que para-méthoxybenzyle, $R_1$ est $R_{1aa}$ et/ou $R_4$ et $R_5$ sont $R_{4aa}$ et $R_{5aa}$ ; et R1aa, R4aa et R5aa sont tels que définis dans la revendication 1 ;
et des sels de celui-ci ;

- d'un composé de formule **(XXXI-a):**

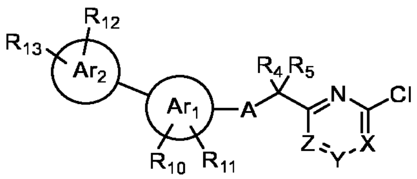

**(XXXI-a) ;**

dans lequel X, Y, Z, $R_{10}$, $R_{11}$, $R_{12}$, $R_{13}$, $Ar_1$ et $A_2$ sont tels que définis dans la revendication 1, A est $A_a$ et/ou $R_4$ et $R_5$ sont $R_{4aa}$ et $R_{5aa}$ ; et R4aa et R5aa sont tels que définis dans la revendication 1 ;
et des sels de celui-ci ;

- d'un composé de formule **(XXXXII-a)** :

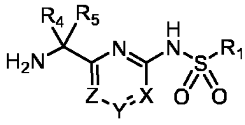

**(XXXXII-a) ;**

dans lequel X, Y et Z sont tels que définis dans la revendication 1, $R_1$ est $R_{1aa}$ et/ou $R_4$ et $R_5$ sont $R_{4aa}$ et $R_{5aa}$; et R1aa, R4aa et R5aa sont tels que définis dans la revendication 1 ;
et des sels de celui-ci ;

- d'un composé de formule **(LI-a)** :

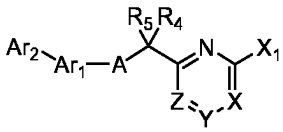

**(LI-a) ;**

dans lequel X, Y, Z, $Ar_1$ et $Ar_2$ sont tels que définis dans la revendication 1, $X_1$ est Cl ou Br, A est $A_{aa}$ et/ou $R_4$ et $R_5$ sont $R_{4aa}$ et $R_{5aa}$. et Aaa, R4aa et R5aa sont tels que définis dans la revendication 1 ;
et des sels de celui-ci ;

- d'un composé de formule **(LXXIII-a)** :

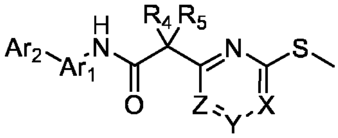

**(LXXIII-a)** ;

dans lequel X, Y, Z, $A_1$, $Ar_2$ sont tels que définis dans la revendication 1, et $R_4$ et $R_5$ sont $R_{4aa}$ et $R_{5aa}$ ; et R4aa et R5aa sont tels que définis dans la revendication 1 ;
et des sels de celui-ci ;

- d'un composé de formule **(LXXIV-a)** :

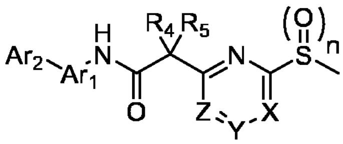

**(LXXIV-a)** n = 1 sulfoxide
n = 2 sulfone ;

dans lequel X, Y, Z, $Ar_1$ et $Ar_2$ sont tels que définis dans la revendication 1, et $R_4$ et $R_5$ sont $R_{4aa}$ et $R_{5aa}$ ; et R4aa et R5aa sont tels que définis dans la revendication 1 ;
et des sels de celui-ci ;

- d'un composé de formule **(LXXXIII-a)** :

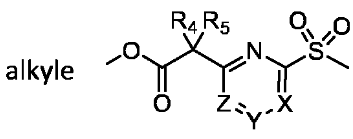

**(LXXXIII-a)** ;

dans lequel X, Y et Z sont tels que définis dans la revendication 1, alkyle est alkyle en $C_{1-4}$ tel que méthyle ou éthyle, par exemple méthyle et $R_4$ et $R_5$ sont $R_{4aa}$ et $R_{5aa}$ ; et R4aa et R5aa sont tels que définis dans la revendication 1 ;
et des sels de celui-ci ;

- d'un composé de formule **(XXXIV-a)** :

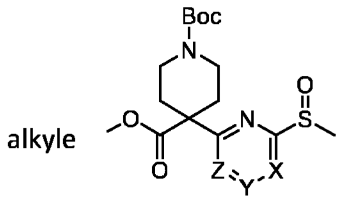

**(XXXIV-a) ;**

dans lequel X, Y et Z sont tels que définis dans la revendication 1, et alkyle est alkyle en $C_{1-4}$ tel que méthyle ou éthyle, par exemple méthyle ;
et des sels de celui-ci ;

- d'un composé de formule **(LVIII-a)** :

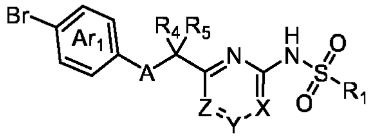

**(LVIII-a) ;**

dans lequel X, Y, Z, et $Ar_1$ sont tels que définis dans la revendication 1, A est $A_a$ et/ou $R_1$ est $R_{1aa}$ et/ou $R_4$ et $R_5$ sont $R_{4aa}$ et $R_{5aa}$ ; et R1aa, R4aa et R5aa sont tels que définis dans la revendication 1 ;
et des sels de celui-ci ;

- d'un composé de formule **(XXXIII-a)** :

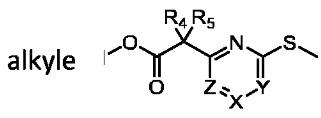

**(XXXIII-a) ;**

dans lequel X, Y et Z sont tels que définis dans la revendication 1, alkyle est alkyle en $C_{1-4}$ tel que méthyle ou éthyle, par exemple méthyle et $R_4$ et $R_5$ are $R_{4aa}$ et $R_{5aa}$; et R4aa et R5aa sont tels que définis dans la revendication 1 ;
et des sels de celui-ci ;

- d'un composé de formule **(II-b)**

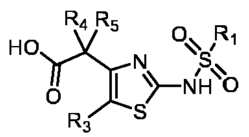

,

dans lequel $R_3$ est tel que défini dans la revendication 1, $R_1$ est $R_{1ab}$ et/ou $R_4$ et $R_5$ sont $R_{4ab}$ et $R_{5ab}$ ; et R1ab, R4ab et R5ab sont tels que définis dans la revendication 1 ;
et des sels de celui-ci ;

- d'un composé de formule **(IV-b)**

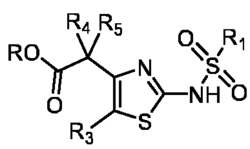

dans lequel $R_3$ est tel que défini dans la revendication 1, R est alkyle en $C_{1-6}$ (par exemple méthyle, éthyle) ou benzyle, $R_1$ est $R_{1ab}$ et/ou $R_4$ et $R_5$ sont $R_{4ab}$ et $R_{5ab}$ ; et R1ab, R4ab et R5ab sont tels que définis dans la

revendication 1 ;
et des sels de celui-ci ;

- d'un composé de formule **(II-c)** :

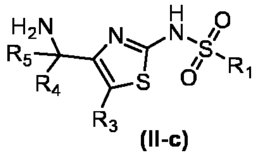

**(II-c)** .

dans lequel $R_3$ est tel que défini dans la revendication 1, $R_1$ est $R_{1ac}$ et/ou $R_4$ et $R_5$ sont $R_{4ac}$ et $R_{5ac}$ ; et R1ac, R4ac et R5ac sont tels que définis dans la revendication 1 ;
et des sels de celui-ci ;

- d'un composé de formule **(VIII-c)** :

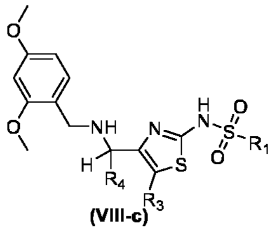

**(VIII-c)** ;

dans lequel $R_3$ et $R_4$ sont tels que définis dans la revendication 1 et $R_1$ est $R_{1ac}$ ; et R1ac sont tels que définis dans la revendication 1 ;
et des sels de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019106156 A
- WO 2019106146 A
- WO 2019179652 A
- WO 2019180244 A
- WO 2020083975 A
- EP 3543232 A
- EP 3492454 A
- EP 3578551 A
- WO 2014170435 A
- WO 0224665 A
- WO 0216318 A
- US 2003158218 A
- CN 104262071
- GB 1575803 A
- GB 155007 A


### Non-patent literature cited in the description


- **SANDOSHAM et al.** *Heterocycles*, 1994, vol. 37 (1), 501-514
- **EVANS, D. R.** ; **GUY, H. I.** Mammalian pyrimidine biosynthesis: fresh insights into an ancient pathway. *J. Biol. Chem.*, 2004, vol. 279, 33035-33038
- **FAIRBANKS, L. D. et al.** Importance of ribonucleotide availability to proliferating T-lymphocytes from healthy humans. Disproportionate expansion of pyrimidine pools and contrasting effects of de novo synthesis inhibitors. *J. Biol. Chem.*, 1995, vol. 270, 29682-29689
- **HIGGINS, M. J. et al.** Regulation of human cytidine triphosphate synthetase 1 by glycogen synthase kinase 3. *J. Biol. Chem.*, 2007, vol. 282, 29493-29503
- **KURSULA, P. et al.** Structure of the synthetase domain of human CTP synthetase, a target for anticancer therapy. *Acta Crystallogr Sect F Struct Biol Cryst Commun.*, 2006, vol. 62, 613-617
- **LIEBERMAN I**. Enzymatic amination of uridine triphosphate to cytidine triphosphate. *The J. Biol. Chem.*, 1956, vol. 222 (2), 765-75
- **MARTIN E. et al.** CTP synthase 1 deficiency in humans reveals its central role in lymphocytes proliferation. *Nature*, 12 June 2014, vol. 510 (7504), 288-92
- *Nature*, 17 July 2014, vol. 511 (7509), 370
- **MCCLUSKEY GD et al.** Exploring the Potent Inhibition of CTP Synthase by Gemcitabine-5'-Triphosphate. *Chembiochem*, 2016, vol. 17, 2240-2249
- **OSTRANDER, D. B. et al.** Effect of CTP synthetase regulation by CTP on phospholipid synthesis in Saccharomyces cerevisiae. *J. Biol. Chem.*, 1998, vol. 273, 18992-19001
- **SAKAMOTO K. et al.** Identification of cytidine-5-triphosphate synthase1-selective inhibitory peptide from random peptide library displayed on T7 phage. *Peptides*, 2017, vol. 94, 56-63
- **SALU et al.** Drug-eluting stents: a new treatment in the prevention of restenosis Part I: experimental studies. *Acta Cardiol*, 2004, vol. 59, 51-61
- **SOUSA J. E. et al.** Drug-Eluting Stents. *Circulation*, 2003, vol. 107, 2274
- **TANG R. et al.** CTP synthase 1, a smooth muscle-sensitive therapeutic target for effective vascular repair. *Arterioscler Thromb Vasc Biol*, 2013, vol. 33 (10), 1-19
- **VAN DEN BERG, A. A. et al.** Cytidine triphosphate (CTP) synthetase activity during cell cycle progression in normal and malignant T-lymphocytic cells. *Eur. J. Cancer*, 1995, vol. 31, 108-112
- **VAN KUILENBURG, A.B.P. et al.** Identification of a cDNA encoding an isoform of human CTP synthetase. *Biochimica et Biophysica Acta*, 2000, 1492548-552